# EUROPÄISCHE PATENTSCHRIFT

(11) **EP 2 847 190 B1**
(45) Veröffentlichungstag und Bekanntmachung des Hinweises auf die Patenterteilung: **06.04.2016**
(21) Anmeldenummer: 13720407.9
(22) Anmeldetag: 03.05.2013
(51) Int. Cl.: C07D 413/14, C07D 401/04, C07D 403/04, C07D 409/14, C07D 413/04, C07D 417/04, C07D 471/04, A61K 38/04

(54) **BICYCLISCH-SUBSTITUIERTE URACILE UND IHRE VERWENDUNG**
BICYCLICALLY SUBSTITUTED URACILS AND THE USE THEREOF
URACILES À SUBSTITUTION BICYCLIQUE ET LEUR UTILISATION

(30) Priorität: 09.05.2012 EP 12167231
(43) Veröffentlichungstag der Anmeldung: 18.03.2015
(60) Teilanmeldung: 16154186.7
(73) Patentinhaber: Bayer Pharma Aktiengesellschaft, 13353 Berlin (DE)
(72) Erfinder: FÜRSTNER, Chantal, 45478 Mülheim/Ruhr (DE); ACKERSTAFF, Jens, 40225 Düsseldorf (DE); STRAUB, Alexander, 42117 Wuppertal (DE); MEIER, Heinrich, 42115 Wuppertal (DE); TINEL, Hanna, 42113 Wuppertal (DE); ZIMMERMANN, Katja, 40489 Düsseldorf (DE); TERSTEEGEN, Adrian, 42111 Wuppertal (DE); ZUBOV, Dmitry, 42857 Remscheid (DE); KAST, Raimund, 42349 Wuppertal (DE); SCHAMBERGER, Jens, 42555 Velbert - Langenberg (DE); SCHÄFER, Martina, 13465 Berlin (DE); BÖRNGEN, Kirsten, 50859 Köln (DE)
(74) Vertreter: BIP Patents
(86) Internationale Anmeldenummer: PCT/EP2013/059286
(87) Internationale Veröffentlichungsnummer: WO 2013/167495

(56) Entgegenhaltungen:
- WO-A2-2007/150011
- US-A1- 2004 102 384
- SAMUEL J. DANISHEFSKY ET AL: "Total synthesis of octosyl acid A. Intramolecular Williamson reaction via a cyclic stannylene derivative", JOURNAL OF THE AMERICAN CHEMICAL SOCIETY, Bd. 110, Nr. 22, 1. Oktober 1988 (1988-10-01), Seiten 7434-7440, XP055066600, ISSN: 0002-7863, DOI: 10.1021/ja00230a024

## Beschreibung

Die vorliegende Anmeldung betrifft neue bicyclisch-substituierte Uracil-Derivate, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten.

Chymase ist eine Chymotrypsin-ähnliche Serinprotease, die als makromolekularer Komplex mit Heparin-Proteoglykanen in sekretorischen Vesikeln von Mastzellen gespeichert wird. Nach einer Aktivierung der Mastzellen wird Chymase in die extrazelluläre Matrix freigesetzt und aktiviert.

Aktivierte Mastzellen spielen eine wichtige Rolle in Wundheilung und inflammatorischen Prozessen, wie z.B. Fibrosierung von Wunden, Angiogenese und kardialem Remodeling (Miyazaki et al., Pharmacol. Ther. 112 (2006), 668-676; Shiota et al., J. Hypertens. 21 (2003), 1823-1825). Eine Erhöhung der Anzahl der Mastzellen wurde beobachtet bei Herzinsuffizienz, Myokardinfarkt und Ischämie, in humanen atherosklerotischen Plaques sowie in abdominalen Aortenaneurysma (Kovanen et al., Circulation 92 (1995), 1084-1088; Libby and Shi, Circulation 115 (2007), 2555-2558; Bacani and Frishman, Cardiol. Rev. 14 (4) (2006), 187-193). Chymasepositive Mastzellen können auch eine wichtige Rolle in dem vaskulären Remodeling der Atemwege bei Asthma und chronisch obstruktiven Lungenerkrankungen spielen. Eine erhöhte Anzahl der Mastzellen wurde in endobronchialen Biopsien von Asthmapatienten gefunden (Zanini et al., J. Allergy Clin. Immunol. 120 (2007), 329-333). Außerdem steht die Chymase im Verdacht, für die Entstehung von vielen Nierenerkrankungen, wie diabetischer Nephropathie und polyzystischer Nierenerkrankung, mitverantwortlich zu sein (Huang et al., J. Am. Soc. Nephrol. 14 (7) (2003), 1738-1747; McPherson et al., J. Am. Soc. Nephrol. 15 (2) (2004), 493-500).

Chymase ist überwiegend beteiligt an der Produktion von Angiotensin II im Herzen, in der Wand der Arterien sowie in der Lunge, wogegen das Angiotensin-konvertierende Enzym für die Entstehung des Peptides im Kreislaufsystem verantwortlich ist (Fleming I., Circ. Res. 98 (2006), 887-896). Darüber hinaus spaltet Chymase eine Reihe von anderen Substraten von pathologischer Bedeutung. Chymase führt zum Abbau von extrazellulären Matrixproteinen, wie Fibronektin, Prokollagen und Vitronektin, und zum Abreißen von fokalen Adhäsionen. Sie bewirkt Aktivierung und Freisetzung von TGFβ aus seiner latenten Form, das eine wichtige Rolle in der Entstehung von Herzhypertrophie und Herzfibrose spielt. Das Enzym wirkt atherogen, indem es Apolipoproteine abbaut und die Aufnahme von Cholesterol durch HDL verhindert. Die Wirkung von Chymase führt zu Freisetzung und Aktivierung von dem Zytokin Interleukin 1 mit seinen pro-inflammatorischen Eigenschaften. Darüber hinaus trägt sie zur Produktion von Endothelin 1 bei (Bacani and Frishman, Cardiol. Rev. 14 (4) (2006), 187-193). Eine Ansammlung von Chymase-positiven Mastzellen hat man in Biopsien von Patienten mit atopischer Dermatitis, Morbus Crohn, chronischer Hepatitis und Leberzirrhose sowie idiopatischer interstitieller Pneumonie gefunden (Dogrell S. A., Expert Opin. Ther. Patents 18 (2008), 485-499).

Die Möglichkeit, Chymase-Inhibitoren für die Therapie unterschiedlicher Krankheiten zu verwenden, wurde in zahlreichen tierexperimentellen Studien nachgewiesen. Inhibition der Chymase kann nützlich sein für die Behandlung des Myokardinfarktes. Jin et al. (Pharmacol. Exp. Ther. 309 (2004), 409-417) zeigten, dass eine Ligatur der Koronararterie im Hund zu ventrikulären Arrhythmien sowie erhöhter Produktion von Angiotensin II und Chymaseaktivität im Herzen geführt hat. Eine intravenöse Gabe des Chymase-Inhibitors TY-501076 reduzierte die Chymaseaktivität sowie die Angiotensin II-Konzentration im Plasma und unterdrückte das Auftreten von Arrhythmien. Positive Wirkung der Chymase-Inhibition wurde in einem in vivo Model für Myokardinfarkt in Hamster gezeigt. Behandlung der Tiere mit dem Chymase-Inhibitor BCEAB reduzierte die Chymaseaktivität, verbesserte die Hämodynamik und reduzierte die Mortalität (Jin et al., Life Sci. 71 (2002), 437-446). Im kardiomyopatischen Syrischen Hamster, wo die Anzahl der Mastzellen im Herzen erhöht ist, hat eine orale Behandlung der Tiere mit dem Chymase-Inhibitor die Herzfibrose um 50 % reduziert (Takai et al., Jpn. J. Pharmacol. 86 (2001), 124-126). In einem Tachykardie-induzierten Herzinsuffizienzmodel im Hund hat die Chymase-Inhibition mit SUN-C82257 zu Reduktion der Anzahl der Mastzellen und der Fibrose im Herzen geführt. Darüber hinaus war die diastolische Funktion des Herzens nach der Behandlung verbessert (Matsumoto et al., Circulation 107 (2003), 2555-2558).
Inhibition von Chymase stellt somit ein wirksames Prinzip in der Behandlung von Herzkreislauferkrankungen, entzündlichen und allergischen sowie unterschiedlichen fibrotischen Erkrankungen dar.
In WO 2007/150011 und WO 2009/049112 wird ein Prozess zur Herstellung von Pyrimidintrionen mit Glycin-Substituenten offenbart. WO 2008/056257 beschreibt Triazindione als GABA-B- Rezeptor Modulatoren zur Behandlung von ZNS-Erkrankungen. In WO 2008/103277 werden verschiedene Stickstoff-Heterocyclen zur Behandlung von Krebs offenbart. US2004/102384 A1 offenbart Uracil-Derivate als Chymase-Hemmer.WO 2009/156182 beschreibt Uracil-Derivate zur Unterdrückung oder Reduzierung der Resistenzbildung bei der Zytostatika-Behandlung.

Die Aufgabe der vorliegenden Erfindung war die Bereitstellung neuer Substanzen, die als Inhibitoren der Chymase wirken und sich als solche zur Behandlung und/oder Prophylaxe von Erkrankungen, insbesondere kardiovaskulären Erkrankungen eignen.

Die vorliegende Erfindung betrifft Verbindungen der allgemeinen Formel (I) 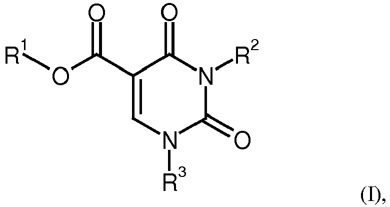 in welcher
- R¹: für Wasserstoff oder (C₁-C₄)-Alkyl steht,
- R²: für eine Gruppe der Formel 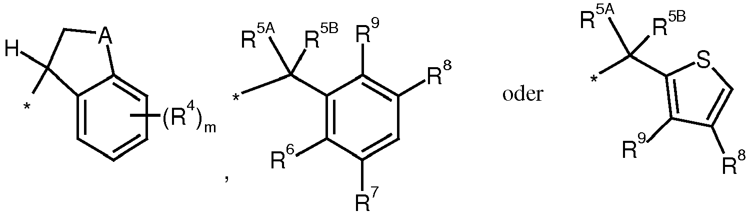 steht, wobei
* für die Anknüpfungsstelle an das Uracil-Stickstoffatom steht,
A für -CH₂- oder Sauerstoff steht,
m für eine Zahl 0, 1 oder 2 steht,
R⁴ für Halogen, Difluormethyl, Trifluormethyl, (C₁-C₄)-Alkyl, Difluormethoxy, Trifluormethoxy oder (C₁-C₄)-Alkoxy steht,
R^{5A} für Wasserstoff oder Deuterium steht,
R^{5B} für Wasserstoff, Deuterium oder (C₁-C₄)-Alkyl steht,
R⁶ für Wasserstoff oder Fluor steht,
R⁷ für Wasserstoff oder Fluor steht,
R⁸ für Halogen, Difluormethyl, Trifluormethyl, (C₁-C₄)-Alkyl oder Nitro steht,
R⁹ für Wasserstoff, Halogen, Difluormethyl, Trifluormethyl, (C₁-C₄)-Alkyl, Nitro oder (C₁-C₄)-Alkylthio steht,
- R³: für eine Gruppe der Formel 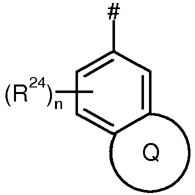 steht, wobei
# für die Anknüpfungsstelle an das Uracil-Stickstoffatom steht, der Ring Q für 5- bis 7-gliedriges Heterocyclyl oder 5- oder 6-gliedriges Heteroaryl steht,
worin 5- bis 7-gliedriges Heterocyclyl und 5- oder 6-gliedriges Heteroaryl mit 1 bis 4 Substituenten unabhängig voneinander ausgewählt aus der Gruppe Halogen, Difluormethyl, Trifluormethyl, Trideuteromethyl, (C₁-C₆)-Alkyl, (C₃-C₇)-Cycloalkyl, Oxo, Hydroxy, (C₁-C₄)-Alkylcarbonyl, (C₁-C₄)-Alkoxycarbonyl, Aminocarbonyl und (C₁-C₄)-Alkylsulfonyl substituiert sein können,
worin (C₁-C₆)-Alkyl und (C₃-C₇)-Cycloalkyl ihrerseits mit 1 bis 3 Substituenten unabhängig voneinander ausgewählt aus der Gruppe Halogen, Cyano, Trifluormethyl, (C₃-C₇)-Cycloalkyl, Hydroxy, (C₁-C₄)-Alkoxy und 4- bis 7-gliedriges Heterocyclyl substituiert sein können,
und
worin zwei an ein Kohlenstoffatom von 5- bis 7-gliedriges Heterocyclyl und 5- oder 6-gliedriges Heteroaryl gebundene (C₁-C₆)-Alkyl-Reste zusammen mit dem Kohlenstoffatom, an das sie gebunden sind, einen 3- bis 6-gliedrigen Carbocyclus bilden können,
R²⁴ für Halogen, (C₁-C₄)-Alkyl oder (C₁-C₄)-Alkoxy steht,
n für eine Zahl 0, 1, 2 oder 3 steht
sowie ihre Salze, Solvate und Solvate der Salze.

Die vorliegende Erfindung betrifft Verbindungen der allgemeinen Formel (I) 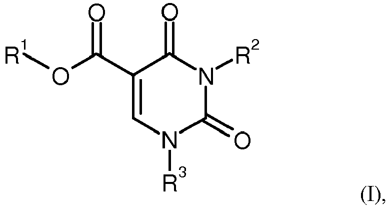 in welcher
- R¹: für Wasserstoff oder (C₁-C₄)-Alkyl steht,
- R²: für eine Gruppe der Formel 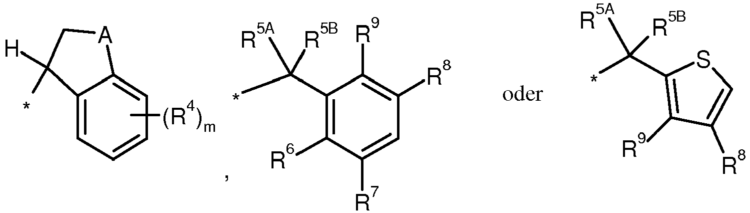 steht, wobei
* für die Anknüpfungsstelle an das Uracil-Stickstoffatom steht,
A für -CH₂-, -CH₂-CH₂-, -O-CH₂-## oder Sauerstoff steht, worin
## für die Anknüpfstelle an den Phenyl-Ring steht,
m für eine Zahl 0, 1 oder 2 steht,
R⁴ für Halogen, Difluormethyl, Trifluormethyl, (C₁-C₄)-Alkyl, Difluormethoxy, Trifluormethoxy oder (C₁-C₄)-Alkoxy steht,
R^{5A} für Wasserstoff oder Deuterium steht,
R^{5B} für Wasserstoff, Deuterium oder (C₁-C₄)-Alkyl steht,
R⁶ für Wasserstoff oder Fluor steht,
R⁷ für Wasserstoff oder Fluor steht,
R⁸ für Halogen, Difluormethyl, Trifluormethyl, (C₁-C₄)-Alkyl oder Nitro steht,
R⁹ für Wasserstoff, Halogen, Difluormethyl, Trifluormethyl, (C₁-C₄)-Alkyl, Nitro oder (C₁-C₄)-Alkylthio steht,
- R³: für eine Gruppe der Formel 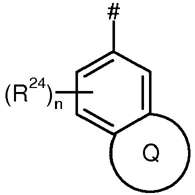 steht, wobei
# für die Anknüpfungsstelle an das Uracil-Stickstoffatom steht, der Ring Q für 5- bis 7-gliedriges Heterocyclyl oder 5- oder 6-gliedriges Heteroaryl steht, worin 5- bis 7-gliedriges Heterocyclyl und 5- oder 6-gliedriges Heteroaryl mit 1 bis 4 Substituenten unabhängig voneinander ausgewählt aus der Gruppe Halogen, Difluormethyl, Trifluormethyl, Trideuteromethyl, (C₁-C₆)-Alkyl, (C₃-C₇)-Cycloalkyl, Oxo, Hydroxy, (C₁-C₄)-Alkylcarbonyl, (C₁-C₄)-Alkoxycarbonyl, Aminocarbonyl und (C₁-C₄)-Alkylsulfonyl substituiert sein können,
worin (C₁-C₆)-Alkyl und (C₃-C₇)-Cycloalkyl ihrerseits mit 1 bis 3 Substituenten unabhängig voneinander ausgewählt aus der Gruppe Halogen, Cyano, Trifluormethyl, (C₃-C₇)-Cycloalkyl, Hydroxy, (C₁-C₄)-Alkoxy und 4- bis 7-gliedriges Heterocyclyl substituiert sein können,
und
worin zwei an ein Kohlenstoffatom von 5- bis 7-gliedriges Heterocyclyl und 5- oder 6-gliedriges Heteroaryl gebundene (C₁-C₆)-Alkyl-Reste zusammen mit dem Kohlenstoffatom, an das sie gebunden sind, einen 3- bis 6-gliedrigen Carbocyclus bilden können,
- R²⁴: für Halogen, (C₁-C₄)-Alkyl oder (C₁-C₄)-Alkoxy steht,
- n: für eine Zahl 0, 1, 2 oder 3 steht
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung, Reinigung oder Lagerung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, *N,N*-Diisopropylethylamin, Monoethanolamin, Diethanolamin, Trisethanolamin, Dimethylaminoethanol, Diethylaminoethanol, Procain, Dicyclohexylamin, Dibenzylamin, *N*-Methylpiperidin, *N*-Methylmorpholin, Arginin, Lysin, Cholin und 1,2-Ethylendiamin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, 18F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper auf beispielsweise metabolischem oder hydrolytischem Wege zu erfindungsgemäßen Verbindungen umgesetzt werden.

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit der jeweils angegebenen Anzahl an Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl, 2-Ethylbutyl, 1,4-Dimethylpentyl, 4,4-Dimethylpentyl und 1,4,4-Trimethylpentyl.

Cycloalkyl steht in Rahmen der Erfindung für einen monocyclischen, gesättigten Alkylrest mit 3 bis 7 Kohlenstoffatomen. Beispielhaft und vorzugsweise seien genannt: Cyclopropyl, Cyclobutyl, Cyclopentyl, Cyclohexyl und Cycloheptyl.

Alkylcarbonyl steht im Rahmen der Erfindung für einen linearen oder verzweigten Alkylrest mit 1 bis 4 Kohlenstoffatomen und einer in 1-Position angebundenen Carbonylgruppe. Beispielhaft und vorzugsweise seien genannt: Methylcarbonyl, Ethylcarbonyl, n-Propylcarbonyl, iso-Propylcarbonyl, n-Butylcarbonyl, iso-Butylcarbonyl und tert.-Butylcarbonyl.

Alkoxy steht im Rahmen der Erfindung für einen linearen oder verzweigten Alkoxyrest 1 bis 4 Kohlenstoffatomen. Beispielhaft und vorzugsweise seien genannt: Methoxy, Ethoxy, *n*-Propoxy, Isopropoxy, *n*-Butoxy und *tert.* -Butoxy.

Alkoxycarbonyl stehen im Rahmen der Erfindung für einen linearen oder verzweigten Alkoxyrest mit 1 bis 4 Kohlenstoffatomen und einer am Sauerstoff angebundenen Carbonylgruppe. Beispielhaft und vorzugsweise seien genannt: Methoxycarbonyl, Ethoxycarbonyl, n-Propoxycarbonyl, Isopropoxycarbonyl und *tert*.-Butoxycarbonyl.

Alkylthio steht im Rahmen der Erfindung für einen linearen oder verzweigten Alkylrest mit 1 bis 4 Kohlenstoffatomen, der über ein Schwefelatom gebunden ist. Beispielhaft und vorzugsweise seien genannt: Methylthio, Ethylthio, *n*-Propylthio, Isopropylthio, 1-Methylpropylthio, *n*-Butylthio, *iso*-Butylthio und *tert*.-Butylthio.

Alkylsulfonyl steht in Rahmen der Erfindung für einen linearen oder verzweigten Alkylrest mit 1 bis 4 Kohlenstoffatomen, der über eine Sulfonylgruppe gebunden ist. Beispielhaft und vorzugsweise seinen genannt: Methylsulfonyl, Ethylsulfonyl, n-Propylsulfonyl, iso-Propylsulfonyl, n-Butylsulfonyl und tert.-Butylsulfonyl.

4- bis 7-gliedriges Heterocyclyl steht im Rahmen der Erfindung für einen monocyclischen, gesättigten Heterocyclus mit insgesamt 4 bis 7 Ringatomen, der ein oder zwei Ring-Heteroatome aus der Reihe N, O, S, SO und/oder SO₂ enthält und über ein Ring-Kohlenstoffatom oder gegebenenfalls ein Ring-Stickstoffatom verknüpft ist. Beispielhaft seien genannt: Azetidinyl, Oxetanyl, Pyrrolidinyl, Pyrazolidinyl, Tetrahydrofuranyl, Piperidinyl, Piperazinyl, Tetrahydropyranyl, Morpholinyl, Thiomorpholinyl. Bevorzugt sind: Azetidinyl, Oxetanyl, Pyrrolidinyl, Tetrahydrofuranyl, Piperidinyl, Piperazinyl, Tetrahydropyranyl und Morpholinyl.

5- bis 7 gliedriges Heterocyclyl steht im Rahmen der Erfindung für einen teilweise ungesättigten Heterocyclus mit insgesamt 5 bis 7 Ringatomen, der 1 bis 3 Ring-Heteroatome aus der Reihe N, O, S und/oder SO₂ enthält und an den Phenylring in R³ annelliert ist. Beispielhaft seien genannt: Dihydropyrrolyl, Dihydroimidazolyl, Dihydrothiazoldioxid, Dihydrooxazolyl, Dihydropyridyl, Tetrahydropyrazinyl und Dihydrooxazinyl.

Heteroaryl steht im Rahmen der Erfindung für einen monocyclischen aromatischen Heterocyclus (Heteroaromaten) mit insgesamt 5 oder 6 Ringatomen, der bis zu drei gleiche oder verschiedene Ring-Heteroatome aus der Reihe N, O und/oder S enthält und an den Phenylring in R³ annelliert ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl und Triazinyl. Bevorzugt sind Pyrazolyl, Imidazolyl, Thiazolyl und Triazolyl.

Halogen schließt im Rahmen der Erfindung Fluor, Chlor, Brom und Iod ein. Bevorzugt sind Chlor oder Fluor.

Eine Oxo-Gruppe steht im Rahmen der Erfindung für ein Sauerstoffatom, das über eine Doppelbindung an ein Kohlenstoff- oder Schwefelatom gebunden ist.

In den Formeln der Gruppe, für die R² und R³ stehen können, steht der Endpunkt der Linie, an dem ein Zeichen * bzw. # bzw. ## steht, nicht für ein Kohlenstoffatom beziehungsweise eine CH₂-Gruppe, sondern ist Bestandteil der Bindung zu dem jeweils bezeichneten Atom, an das R² bzw. R³ gebunden sind.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit einem oder zwei gleichen oder verschiedenen Substituenten ist bevorzugt. Ganz besonders bevorzugt ist die Substitution mit einem Substituenten.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Bevorzugt im Rahmen der vorliegenden Erfindung sind Verbindungen der Formel (I), in welcher
- R¹: für Wasserstoff, Methyl oder Ethyl steht,
- R²: für eine Gruppe der Formel 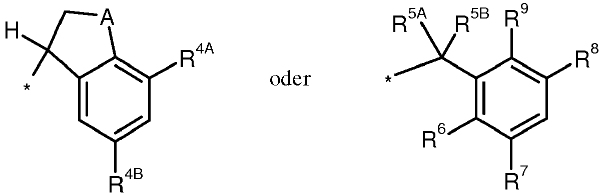 steht, wobei
* für die Anknüpfungsstelle an das Uracil-Stickstoffatom steht,
A für -CH₂- oder Sauerstoff steht,
R^{4A} für Wasserstoff, Fluor, Chlor, Trifluormethyl oder Methyl steht,
R^{4B} für Wasserstoff, Fluor, Chlor, Trifluormethyl oder Methyl steht, mit der Massgabe, dass mindestens einer der Reste R^{4A} und R^{4B} von Wasserstoff verschieden ist,
R^{5A} für Wasserstoff steht,
R^{5B} für Wasserstoff steht,
R⁶ für Wasserstoff steht,
R⁷ für Wasserstoff steht,
R⁸ für Fluor, Chlor, Difluormethyl, Trifluormethyl oder Methyl steht,
R⁹ für Fluor, Chlor, Difluormethyl, Trifluormethyl oder Methyl steht,
- R³: für eine Gruppe der Formel 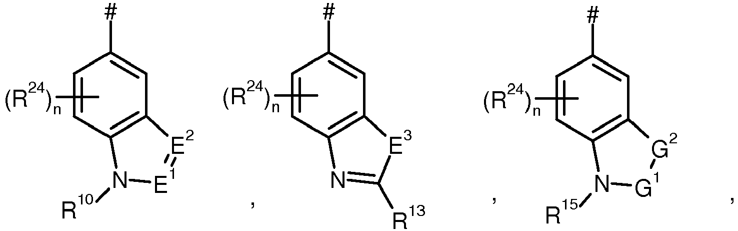 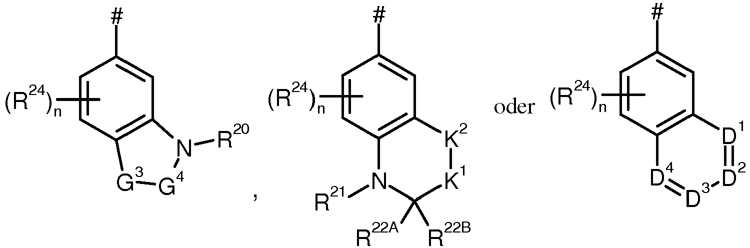 steht, wobei
# für die Anknüpfungsstelle an das Uracil-Stickstoffatom steht,
E¹ für CR¹¹ oder N steht,
worin
R¹¹ für Wasserstoff, (C₁-C₄)-Alkyl, (C₃-C₇)-Cycloalkyl oder Aminocarbonyl steht,
E² für CR¹² oder N steht,
worin
R¹² für Wasserstoff, (C₁-C₄)-Alkyl oder (C₃-C₇)-Cycloalkyl steht,
E³ für NR¹⁴ oder S steht,
worin
R¹⁴ für Wasserstoff, (C₁-C₄)-Alkyl oder (C₃-C₇)-Cycloalkyl steht,
G¹ für C=O oder SO₂ steht,
G² für CR^{16A}R^{16B}, NR¹⁷, O oder S steht,
worin
R^{16A} für Wasserstoff, Fluor, (C₁-C₄)-Alkyl oder Hydroxy steht,
R^{16B} für Wasserstoff, Fluor, Chlor, (C₁-C₄)-Alkyl oder Trifluormethyl steht,
oder
R^{16A} und R^{16B} bilden zusammen mit dem Kohlenstoffatom, an das sie gebunden sind, einen 3- bis 6-gliedrigen Carbocyclus,
R¹⁷ für Wasserstoff, (C₁-C₆)-Alkyl, (C₃-C₇)-Cycloalkyl oder (C₁-C₄)-Alkoxycarbonyl steht, worin (C₁-C₆)-Alkyl mit 1 bis 3 Substituenten unabhängig voneinander ausgewählt aus der Gruppe Fluor, Trifluormethyl, Cyano, (C₃-C₇)-Cycloalkyl, Hydroxy, Trifluormethoxy, (C₁-C₄)-Alkoxy, Azetidinyl, Oxetanyl, Tetrahydrofuranyl und Pyrrolidinyl substituiert sein kann,
G³ für CR^{18A}R^{18B}, NR¹⁹, O oder S steht,
worin
R^{18A} für Wasserstoff, Fluor, (C₁-C₄)-Alkyl oder Hydroxy steht,
R^{18B} für Wasserstoff, Fluor, Chlor, (C₁-C₄)-Alkyl oder Trifluormethyl steht,
oder
R^{18A} und R^{18B} zusammen mit dem Kohlenstoffatom, an das sie gebunden sind, einen 3- bis 6-gliedrigen Carbocyclus bilden,
R¹⁹ für Wasserstoff, (C₁-C₆)-Alkyl, (C₃-C₇)-Cycloalkyl oder (C₁-C₄)-Alkoxycarbonyl steht, worin (C₁-C₆)-Alkyl mit 1 bis 3 Subsituenten unabhängig voneinander ausgewählt aus der Gruppe Fluor, Trifluormethyl, Cyano, (C₃-C₇)-Cycloalkyl, Hydroxy, Trifluormethoxy, (C₁-C₄)-Alkoxy, Azetidinyl, Oxetanyl, Tetrahydrofuranyl und Pyrrolidinyl substituiert sein kann,
G⁴ für CH₂, C=O oder SO₂ steht,
K¹ für CH₂ oder O steht,
K² für CH₂ oder O steht,
mit der Massgabe, dass nur eine der Gruppe K¹ und K² für O steht,
D¹, D², D³ und D⁴ unanhängig voneinander für CR²³ oder N stehen,
worin
R²³ für Wasserstoff, Halogen, (C₁-C₆)-Alkyl oder (C₃-C₇)-Cycloalkyl steht, mit der Massgabe, dass maximal 2 der Gruppen D¹, D², D³ und D⁴ für N stehen,
R²⁴ für Fluor oder Methyl steht,
n für eine Zahl 0 oder 1 steht,
R¹⁰ für (C₁-C₄)-Alkyl oder (C₃-C₇)-Cycloalkyl steht,
worin (C₁-C₄)-Alkyl mit 1 oder 2 Substituenten unabhängig voneinander ausgewählt aus der Gruppe Fluor, Trifluormethyl, Cyclopropyl, Cyclobutyl, Hydroxy, Methoxy, Ethoxy, Azetidinyl, Oxetanyl, Tetrahydrofuranyl und Pyrrolidinyl substituiert sein kann,
R¹³ für Wasserstoff, (C₁-C₄)-Alkyl oder (C₃-C₇)-Cycloalkyl steht,
R¹⁵ für Wasserstoff, (C₁-C₆)-Alkyl oder (C₃-C₇)-Cycloalkyl steht,
worin (C₁-C₆)-Alkyl mit 1 oder 2 Substituenten unabhängig voneinander ausgewählt aus der Gruppe Fluor, Trifluormethyl, Cyclopropyl, Cyclobutyl, Hydroxy, Methoxy, Ethoxy, Azetidinyl, Oxetanyl, Tetrahydrofuranyl und Pyrrolidinyl substituiert sein kann,
R²⁰ für Wasserstoff, (C₁-C₆)-Alkyl, (C₃-C₇)-Cycloalkyl oder (C₁-C₄)-Alkylcarbonyl steht, worin (C₁-C₆)-Alkyl mit 1 oder 2 Substituenten unabhängig voneinander ausgewählt aus der Gruppe Fluor, Trifluormethyl, Cyclopropyl, Cyclobutyl, Hydroxy, Methoxy, Ethoxy, Azetidinyl, Oxetanyl, Tetrahydrofuranyl und Pyrrolidinyl substituiert sein kann,
R²¹ für Wasserstoff, (C₁-C₆)-Alkyl, (C₃-C₇)-Cycloalkyl oder (C₁-C₄)-Alkylsulfonyl steht,
R^{22A} für Wasserstoff oder (C₁-C₄)-Alkyl steht,
R^{22B} für Wasserstoff oder (C₁-C₄)-Alkyl steht,
oder
R^{22A} und R^{22B} bilden zusammen mit dem Kohlenstoffatom, an das sie gebunden sind, Carbonylgruppe,
sowie ihre Salze, Solvate und Solvate der Salze.

Bevorzugt im Rahmen der vorliegenden Erfindung sind Verbindungen der Formel (I), in welcher
- R¹: für Wasserstoff, Methyl oder Ethyl steht,
- R²: für eine Gruppe der Formel 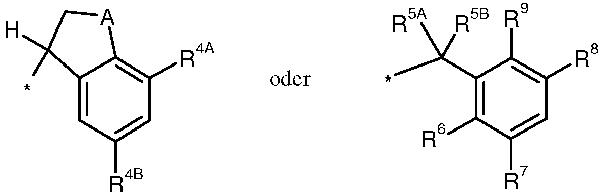 steht, wobei
* für die Anknüpfungsstelle an das Uracil-Stickstoffatom steht,
A für -CH₂-, -CH₂-CH₂-, -O-CH₂-## oder Sauerstoff steht,
worin
## für die Anknüpfstelle an den Phenyl-Ring steht,
R^{4A} für Wasserstoff, Fluor, Chlor, Trifluormethyl oder Methyl steht,
R^{4B} für Wasserstoff, Fluor, Chlor, Trifluormethyl oder Methyl steht, mit der Massgabe, dass mindestens einer der Reste R^{4A} und R^{4B} von Wasserstoff verschieden ist,
R^{5A} für Wasserstoff steht,
R^{5B} für Wasserstoff steht,
R⁶ für Wasserstoff steht,
R⁷ für Wasserstoff steht,
R⁸ für Fluor, Chlor, Difluormethyl, Trifluormethyl oder Methyl steht,
R⁹ für Fluor, Chlor, Difluormethyl, Trifluormethyl oder Methyl steht,
- R³: für eine Gruppe der Formel 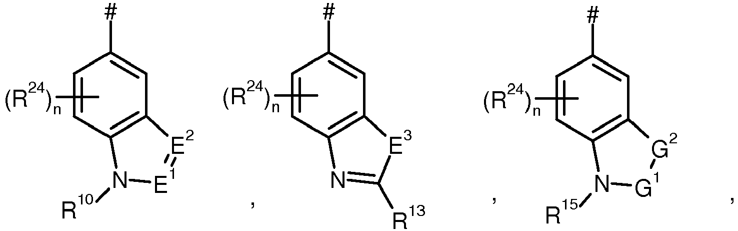 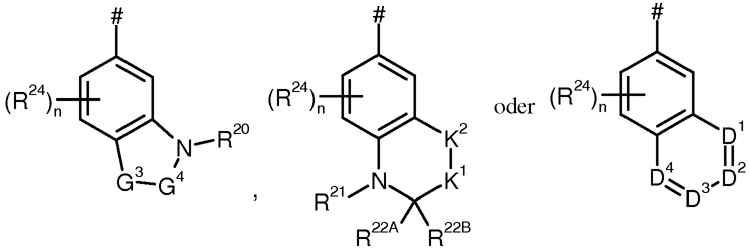 steht, wobei
# für die Anknüpfungsstelle an das Uracil-Stickstoffatom steht,
E¹ für CR¹¹ oder N steht,
worin
R¹¹ für Wasserstoff, (C₁-C₄)-Alkyl, (C₃-C₇)-Cycloalkyl oder Aminocarbonyl steht,
E² für CR¹² oder N steht,
worin
R¹² für Wasserstoff, (C₁-C₄)-Alkyl oder (C₃-C₇)-Cycloalkyl steht,
E³ für NR¹⁴ oder S steht,
worin
R¹⁴ für Wasserstoff, (C₁-C₄)-Alkyl oder (C₃-C₇)-Cycloalkyl steht,
G¹ für C=O oder SO₂ steht,
G² für CR^{16A}R^{16B}, NR¹⁷, O oder S steht,
worin
R^{16A} für Wasserstoff, Fluor, (C₁-C₄)-Alkyl oder Hydroxy steht,
R^{16B} für Wasserstoff, Fluor, Chlor, (C₁-C₄)-Alkyl oder Trifluormethyl steht, oder
R^{16A} und R^{16B} bilden zusammen mit dem Kohlenstoffatom, an das sie gebunden sind, einen 3- bis 6-gliedrigen Carbocyclus,
R¹⁷ für Wasserstoff, (C₁-C₆)-Alkyl, (C₃-C₇)-Cycloalkyl oder (C₁-C₄)-Alkoxycarbonyl steht, worin (C₁-C₆)-Alkyl mit 1 bis 3 Substituenten unabhängig voneinander ausgewählt aus der Gruppe Fluor, Trifluormethyl, Cyano, (C₃-C₇)-Cycloalkyl, Hydroxy, Trifluormethoxy, (C₁-C₄)-Alkoxy, Azetidinyl, Oxetanyl, Tetrahydrofuranyl und Pyrrolidinyl substituiert sein kann,
G³ für CR^{18A}R^{18B}, NR¹⁹, O oder S steht,
worin
R^{18A} für Wasserstoff, Fluor, (C₁-C₄)-Alkyl oder Hydroxy steht,
R^{18B} für Wasserstoff, Fluor, Chlor, (C₁-C₄)-Alkyl oder Trifluormethyl steht,
oder
R^{18A} und R^{18B} zusammen mit dem Kohlenstoffatom, an das sie gebunden sind, einen 3- bis 6-gliedrigen Carbocyclus bilden,
R¹⁹ für Wasserstoff, (C₁-C₆)-Alkyl, (C₃-C₇)-Cycloalkyl oder (C₁-C₄)-Alkoxycarbonyl steht, worin (C₁-C₆)-Alkyl mit 1 bis 3 Subsituenten unabhängig voneinander ausgewählt aus der Gruppe Fluor, Trifluormethyl, Cyano, (C₃-C₇)-Cycloalkyl, Hydroxy, Trifluormethoxy, (C₁-C₄)-Alkoxy, Azetidinyl, Oxetanyl, Tetrahydrofuranyl und Pyrrolidinyl substituiert sein kann,
G⁴ für CH₂, C=O oder SO₂ steht,
K¹ für CH₂ oder O steht,
K² für CH₂ oder O steht, mit der Massgabe, dass nur eine der Gruppe K¹ und K² für O steht,
D¹, D², D³ und D⁴ unanhängig voneinander für CR²³ oder N stehen,
worin
R²³ für Wasserstoff, Halogen, (C₁-C₆)-Alkyl oder (C₃-C₇)-Cycloalkyl steht, mit der Massgabe, dass maximal 2 der Gruppen D¹, D², D³ und D⁴ für N stehen,
R²⁴ für Fluor oder Methyl steht,
n für eine Zahl 0 oder 1 steht,
R¹⁰ für (C₁-C₄)-Alkyl oder (C₃-C₇)-Cycloalkyl steht, worin (C₁-C₄)-Alkyl mit 1 oder 2 Substituenten unabhängig voneinander ausgewählt aus der Gruppe Fluor, Trifluormethyl, Cyclopropyl, Cyclobutyl, Hydroxy, Methoxy, Ethoxy, Azetidinyl, Oxetanyl, Tetrahydrofuranyl und Pyrrolidinyl substituiert sein kann,
R¹³ für Wasserstoff, (C₁-C₄)-Alkyl oder (C₃-C₇)-Cycloalkyl steht,
R¹⁵ für Wasserstoff, (C₁-C₆)-Alkyl oder (C₃-C₇)-Cycloalkyl steht, worin (C₁-C₆)-Alkyl mit 1 oder 2 Substituenten unabhängig voneinander ausgewählt aus der Gruppe Fluor, Trifluormethyl, Cyclopropyl, Cyclobutyl, Hydroxy, Methoxy, Ethoxy, Azetidinyl, Oxetanyl, Tetrahydrofuranyl und Pyrrolidinyl substituiert sein kann,
R²⁰ für Wasserstoff, (C₁-C₆)-Alkyl, (C₃-C₇)-Cycloalkyl oder (C₁-C₄)-Alkylcarbonyl steht, worin (C₁-C₆)-Alkyl mit 1 oder 2 Substituenten unabhängig voneinander ausgewählt aus der Gruppe Fluor, Trifluormethyl, Cyclopropyl, Cyclobutyl, Hydroxy, Methoxy, Ethoxy, Azetidinyl, Oxetanyl, Tetrahydrofuranyl und Pyrrolidinyl substituiert sein kann,
R²¹ für Wasserstoff, (C₁-C₆)-Alkyl, (C₃-C₇)-Cycloalkyl oder (C₁-C₄)-Alkylsulfonyl steht,
R^{22A} für Wasserstoff oder (C₁-C₄)-Alkyl steht,
R^{22B} für Wasserstoff oder (C₁-C₄)-Alkyl steht, oder

- R^{22A} und R^{22B}: bilden zusammen mit dem Kohlenstoffatom, an das sie gebunden sind, eine Carbonylgruppe,
sowie ihre Salze, Solvate und Solvate der Salze.

Besonders bevorzugt im Rahmen der vorliegenden Erfindung sind Verbindungen der Formel (I), in welcher
- R¹: für Wasserstoff steht,
- R²: für eine Gruppe der Formel 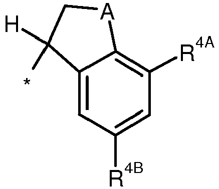 steht, wobei
* für die Anknüpfungsstelle an das Uracil-Stickstoffatom steht,
A für -CH₂- steht,
R^{4A} für Chlor oder Trifluormethyl steht,
R^{4B} für Wasserstoff steht,
- R³: für eine Gruppe der Formel 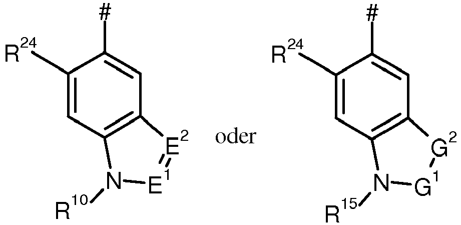 steht, wobei
# für die Anknüpfungsstelle an das Uracil-Stickstoffatom steht,
E¹ für CR¹¹ steht,
worin
R¹¹ für Wasserstoff steht,
E² für N steht,
G¹ für C=O steht,
G² für CR^{16A}R^{16B}, NR¹⁷, O oder S steht,
worin
R^{16A} für Wasserstoff, Fluor, Methyl oder Hydroxy steht,
R^{16B} für Wasserstoff, Fluor, Methyl oder Trifluormethyl steht,
oder
R^{16A} und R^{16B} zusammen mit dem Kohlenstoffatom, an das sie gebunden sind, einen Cyclopropyl-Ring bilden,
R¹⁷ für Wasserstoff, (C₁-C₄)-Alkyl oder (C₃-C₅)-Cycloalkyl steht, worin (C₁-C₄)-Alkyl mit 1 bis 3 Substituenten unabhängig voneinander ausgewählt aus der Gruppe Fluor, Trifluormethyl, Cyano, Cyclopropyl, Cyclobutyl, Hydroxy, Trifluormethoxy, Methoxy, Ethoxy, Azetidinyl, Oxetanyl, Tetrahydrofuranyl und Pyrrolidinyl substituiert sein kann,
- R²⁴: für Wasserstoff oder Fluor steht,
- R¹⁰: für (C₁-C₄)-Alkyl steht,
- R¹⁵: für Wasserstoff, Methyl oder Ethyl steht, worin Methyl und Ethyl mit 1 Substituenten ausgewählt aus der Gruppe Fluor, Trifluormethyl und Cyclopropyl substituiert sein können,
sowie ihre Salze, Solvate und Solvate der Salze.

Bevorzugt sind im Rahmen der vorliegenden Erfindung auch Verbindungen der Formel (I), in welcher
- R¹: für Wasserstoff steht,
- R²: für eine Gruppe der Formel 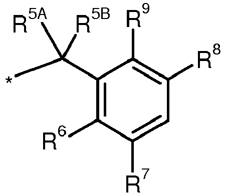 steht, wobei
* für die Anknüpfungsstelle an das Uracil-Stickstoffatom steht,
R^{5A} für Wasserstoff steht,
R^{5B} für Wasserstoff steht,
R⁶ für Wasserstoff steht,
R⁷ für Wasserstoff steht,
R⁸ für Fluor, Chlor oder Trifluormethyl steht,
R⁹ für Fluor, Chlor, Trifluormethyl oder Methyl steht,
R³ für eine Gruppe der Formel 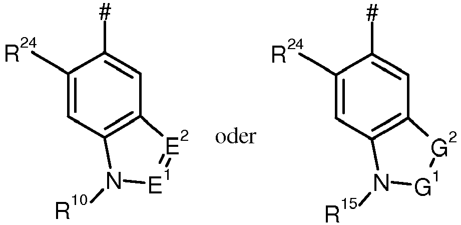 steht, wobei
# für die Anknüpfungsstelle an das Uracil-Stickstoffatom steht,
E¹ für CR¹¹ steht,
worin
R¹¹ für Wasserstoff steht,
E² für N steht,
G¹ für C=O steht,
G² für CR^{16A}R^{16B}, NR¹⁷, O oder S steht,
worin
R^{16A} für Wasserstoff, Fluor, Methyl oder Hydroxy steht,
R^{16B} für Wasserstoff, Fluor, Methyl oder Trifluormethyl steht, oder
R^{16A} und R^{16B} zusammen mit dem Kohlenstoffatom, an das sie gebunden sind, einen Cyclopropyl-Ring bilden,
R¹⁷ für Wasserstoff, (C₁-C₄)-Alkyl oder (C₃-C₅)-Cycloalkyl steht, worin (C₁-C₄)-Alkyl mit 1 bis 3 Substituenten unabhängig voneinander ausgewählt aus der Gruppe Fluor, Trifluormethyl, Cyano, Cyclopropyl, Cyclobutyl, Hydroxy, Trifluormethoxy, Methoxy, Ethoxy, Azetidinyl, Oxetanyl, Tetrahydrofuranyl und Pyrrolidinyl substituiert sein kann,
R²⁴ für Wasserstoff oder Fluor steht,
R¹⁰ für (C₁-C₄)-Alkyl steht,
R¹⁵ für Wasserstoff, Methyl oder Ethyl steht,
worin Methyl und Ethyl mit 1 Substituenten ausgewählt aus der Gruppe Fluor, Trifluormethyl und Cyclopropyl substituiert sein können,
sowie ihre Salze, Solvate und Solvate der Salze.

Bevorzugt sind im Rahmen der vorliegenden Erfindung auch Verbindungen der Formel (I), in welcher
- R¹: für Wasserstoff, Methyl oder Ethyl steht,
- R²: für eine Gruppe der Formel 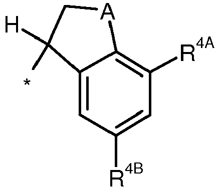 steht, wobei
* für die Anknüpfungsstelle an das Uracil-Stickstoffatom steht,
A für -CH₂- steht,
R^{4A} für Chlor oder Trifluormethyl steht,
R^{4B} für Wasserstoff steht,
steht, sowie ihre Salze, Solvate und Solvate der Salze.

Bevorzugt sind im Rahmen der vorliegenden Erfindung auch Verbindungen der Formel (I), in welcher
- R³: für eine Gruppe der Formel 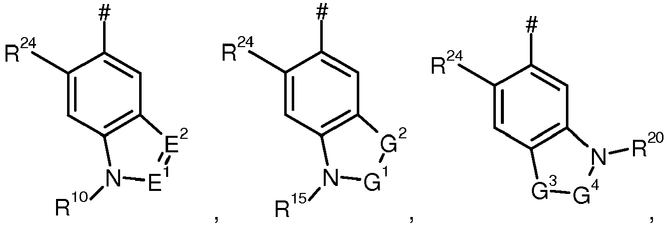 oder 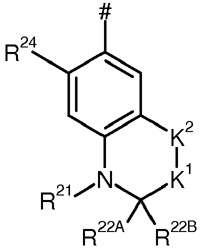 steht, wobei
# für die Anknüpfungsstelle an das Uracil-Stickstoffatom steht,
E¹ für CR¹¹ oder N steht,
worin
R¹¹ für Wasserstoff, Methyl, Ethyl oder Aminocarbonyl steht,
E² für CR¹² oder N steht,
worin
R¹² für Wasserstoff steht,
G¹ für C=O oder SO₂ steht,
G² für CR^{16A}R^{16B}, NR¹⁷, O oder S steht, worin
R^{16A} für Wasserstoff, Fluor, Methyl oder Hydroxy steht,
R^{16B} für Wasserstoff, Fluor, Chlor, Methyl oder Trifluormethyl steht, oder
R^{16A} und R^{16B} bilden zusammen mit dem Kohlenstoffatom, an das sie gebunden sind, einen Cyclopropyl-Ring,
R¹⁷ für Wasserstoff, (C₁-C₄)-Alkyl, Cyclopropyl oder Cyclobutyl steht,
worin (C₁-C₄)-Alkyl mit 1 oder 2 Substituenten unabhängig voneinander ausgewählt aus der Gruppe Fluor, Trifluormethyl, Cyclopropyl, Cyclobutyl, Hydroxy, Azetidinyl und Oxetanyl substituiert sein kann,
- G³: für CR^{18A}R^{18B} steht,
worin
R^{18A} für Wasserstoff, Fluor, Methyl oder Hydroxy steht,
R^{18B} für Wasserstoff, Fluor, Methyl oder Trifluormethyl steht,
- G⁴: für C=O steht,
- K¹: für CH₂ oder O steht,
- K²: für CH₂ steht,
- R²⁴: für Wasserstoff, Fluor oder Methyl steht,
- R¹⁰: für Methyl oder Ethyl steht,
- R¹⁵: für Methyl oder Ethyl steht,
- R²⁰: für Wasserstoff, Methyl, Ethyl oder Methylcarbonyl steht,
- R²¹: für Methyl oder Ethyl steht,
- R^{22A} und R^{22B}: bilden zusammen mit dem Kohlenstoffatom, an das sie gebunden sind, eine Carbonylgruppe,
sowie ihre Salze, Solvate und Solvate der Salze.

Bevorzugt sind im Rahmen der vorliegenden Erfindung auch Verbindungen der Formel (I), in welcher
- R²: für eine Gruppe der Formel 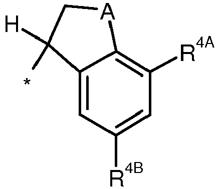 steht, wobei
* für die Anknüpfungsstelle an das Uracil-Stickstoffatom steht,
A für -CH₂- steht,
R^{4A} für Chlor oder Trifluormethyl steht,
R^{4B} für Wasserstoff steht,
und das Kohlenstoffatom, das an das Uracilstickstoffatom gebunden ist, eine R-Konfiguration hat,
sowie ihre Salze, Solvate und Solvate der Salze.

Bevorzugt sind im Rahmen der vorliegenden Erfindung auch Verbindungen der Formel (I), in welcher
- R²: für eine Gruppe der Formel 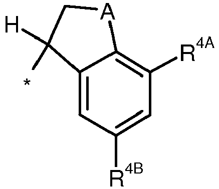 steht, wobei
* für die Anknüpfungsstelle an das Uracil-Stickstoffatom steht,
A für -CH₂- steht,
R^{4A} für Wasserstoff, Fluor, Chlor, Trifluormethyl oder Methyl steht,
R^{4B} für Wasserstoff, Fluor, Chlor, Trifluormethyl oder Methyl steht,
mit der Massgabe, dass mindestens einer der Reste R^{4A} und R^{4B} von Wasserstoff verschieden ist,
und das Kohlenstoffatom, das an das Uracilstickstoffatom gebunden ist, eine R-Konfiguration hat, sowie ihre Salze, Solvate und Solvate der Salze.

Die in den jeweiligen Kombinationen bzw. bevorzugten Kombinationen von Resten im Einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung von erfindungsgemäßen Verbindungen der Formel (I), dadurch gekennzeichnet, dass man

### [A] eine Verbindung der Formel (II)

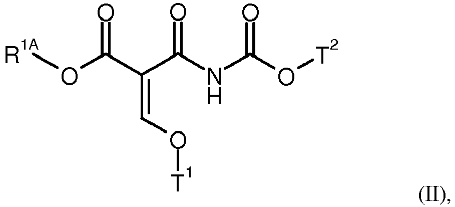

in welcher
- R^{1A}: für (C₁-C₄)-Alkyl steht,
- T¹: für (C₁-C₄)-Alkyl steht,
- T²: für (C₁-C₄)-Alkyl steht,
in einem inerten Lösungsmittel gegebenenfalls in Gegenwart einer geeigneten Base mit einer Verbindung der Formel (III)

H₂N-R³ (III),

in welcher R³ die oben angegebene Bedeutung hat,
zu einer Verbindung der Formel (IV) 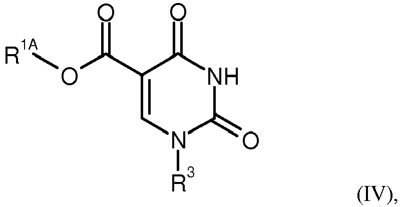 in welcher R^{1A} und R³ jeweils die zuvor angegebenen Bedeutungen haben,
umsetzt, diese anschließend in einem inerten Lösungsmittel in Gegenwart einer geeigneten Base mit einer Verbindung der Formel (V)

X¹-R² (V),

in welcher R² die oben angegebene Bedeutung hat,
und
X¹ für Hydroxy oder eine geeignete Abgangsgruppe, insbesondere Chlor, Brom oder Iod, steht zu einer Verbindung der Formel (I-1) 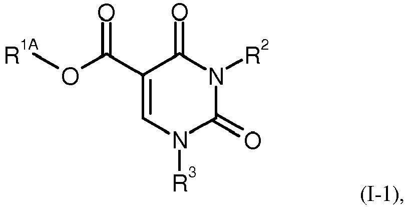 in welcher R^{1A}, R² und R³ jeweils die oben angegebenen Bedeutungen haben, umsetzt
oder

### [B] eine Verbindung der Formel (VI)

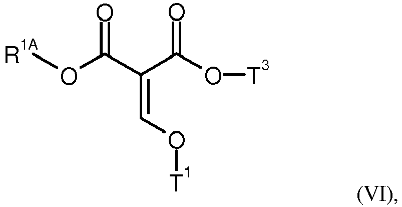

in welcher R^{1A} und T¹ jeweils die oben genannten Bedeutungen haben, und
- T³: für (C₁-C₄)-Alkyl steht,
in einem inerten Lösungsmittel oder auch ohne Lösungsmittel mit einer Verbindung der Formel (III) in eine Verbindung der Formel (VII) 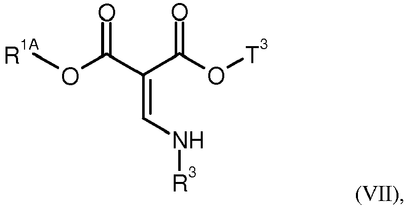 in welcher R^{1A}, R³ und T³ jeweils die oben angegebenen Bedeutungen haben, überführt, diese im Folgenden in einem inerten Lösungsmittel mit Chlorsulfonylisocyanat zu einer Verbindung der Formel (IV) umsetzt und diese anschließend analog zu Verfahren [A] in eine Verbindung der Formel (I-1) überführt,
oder

### [C] eine Verbindung der Formel (VIII)

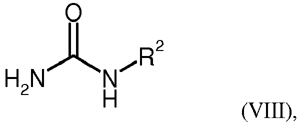

in welcher R² die oben genannte Bedeutung hat,
in einem inerten Lösungsmittel mit einer Verbindung der Formel (IX) 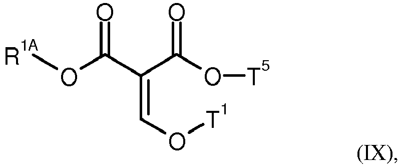 in welcher R^{1A} und T¹ jeweils die oben angegebenen Bedeutungen haben, und
- T⁵: für (C₁-C₄)-Alkyl steht,
umsetzt und in Gegenwart einer geeigneten Base zu einer Verbindung der Formel (X) 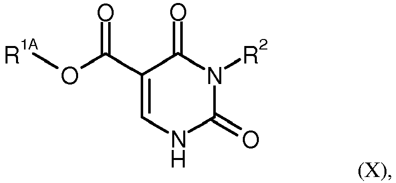 in welcher R^{1A} und R² jeweils die oben genannten Bedeutungen haben,
cyclisiert, und diese anschließend in einem inerten Lösungsmittel in Gegenwart eines geeigneten Katalysators und einer geeigneten Base mit einer Verbindung der Formel (XI) 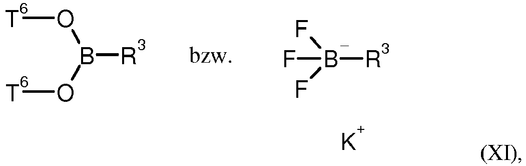 in welcher R³ die oben angegebene Bedeutung hat, und
- T⁶: für Wasserstoff, (C₁-C₄)-Alkyl steht oder beide Reste T⁶ zusammen eine -C(CH₃)₂-C(CH₃)₂- - Brücke bilden,
zu einer Verbindung der Formel (I-1) umsetzt,
oder

### [D] eine Verbindung der Formel (I-1) in einem inerten Lösungsmittel in Gegenwart einer geeigneten Säure oder Base zu einer Verbindung der Formel (I-2)

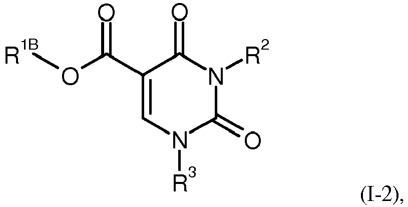

in welcher R² und R³ jeweils die oben genannten Bedeutungen haben, und
- R^{1B}: für Wasserstoff steht,
verseift,
gegebenenfalls vorhandene Schutzgruppen abspaltet und/ oder die Verbindungen der Formeln (I-1) und (I-2) gegebenenfalls mit den entsprechenden (*i*) Lösungsmitteln und/oder (*ii*) Basen oder Säuren in ihre Solvate, Salze und/oder Solvate der Salze überführt.

Die Verbindungen der Formeln (I-1) und (I-2) bilden zusammen die Gruppe der erfindungsgemäßen Verbindungen der Formel (I).

Inerte Lösungsmittel für die Verfahrensschritte (II) + (III) → (IV), (VI) + (III) → (VII) und (VIII) + (IX) → (X) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, 1,2-Dichlorethan, Trichlorethylen oder Chlorbenzol, Alkohole wie Methanol, Ethanol, n-Propanol, iso-Propanol oder n-Butanol, oder andere Lösungsmittel wie Dimethylformamid, Dimethylsulfoxid, *N,N*-Dimethylpropylenharnstoff (DMPU), *N*-Methylpyrrolidinon (NMP), Pyridin, Aceton, 2-Butanon oder Acetonitril. Ebenso ist es möglich, Gemische der genannten Lösungsmittel einzusetzen. Bevorzugt wird Ethanol verwendet.

Als Base für die Verfahrensschritte (II) + (III) → (IV) und (VIII) + (IX) → (X) eignen sich Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium- oder Kalium-bis(trimethylsilyl)amid oder Lithiumdiisopropylamid, oder organische Basen wie Triethylamin, Diisopropylethylamin, 1,5-Diazabicyclo[4.3.0]non-5-en (DBN), 1,8-Diazabicyclo[5.4.0]undec-7-en (DBU) oder 1,4-Diazabicyclo[2.2.2]-octan (DABCO^{®}) oder Phosphazenbasen wie z.B. 1-[N-tert.-Butyl-P,P-di(pyrrolidin-1-yl)phosphorimidoyl]-pyrrolidin oder N"'-tert.-Butyl-N,N,N',N'-tetramethyl-N"-[tris(dimethylamino)-lambda⁵-phosphanyliden]phosphorimidsäuretriamid. Bevorzugt sind Natriumethanolat und Kalium-tert.-butylat.

Die Base wird hierbei im Allgemeinen in einer Menge von 1 bis 5 Mol, bevorzugt in einer Menge von 1.2 bis 3 Mol, bezogen auf 1 Mol der Verbindung der Formel (II) bzw. (IX) eingesetzt.

Die Umsetzungen (II) + (III) → (IV), (VI) + (III) → (VII) und (VIII) + (IX) → (X) erfolgen im Allgemeinen in einem Temperaturbereich von 0°C bis +150°C, bevorzugt bei +20°C bis +120°C, gegebenenfalls in einer Mikrowelle. Die Reaktion kann bei normalem, erhöhtem oder bei erniedrigtem Druck durchgeführt werden (z.B. von 0.5 bis 5 bar). Im Allgemeinen arbeitet man bei Normaldruck.

Für den Fall X¹ = OH erfolgt die Umsetzung (IV) + (V) → (I-1) unter Mitsunobu-Bedingungen [siehe: a) Hughes, D. L. "The Mitsunobu Reaction" Organic Reactions; John Wiley & Sons, Ltd, 1992, vol. 42, p. 335. b) Hughes, D. L. Org. Prep. Proceed. Int. 1996, 28, 127]. Die Mitsunobu-Reaktion erfolgt unter Verwendung von Triphenylphosphin, oder Tri-n-butylphosphin, 1,2-Bis(diphenylphosphino)ethan (DPPE), Diphenyl(2-pyridyl)-phosphin (Ph2P-Py), (*p*-Dimethylaminophenyl)diphenylphosphin (DAP-DP), tris(4-Dimethylaminophenyl)-phosphin (tris-DAP) und eines geeigneten Dialkylazodicarboxylats, wie beispielsweise Diethylazodicarboxylat (DEAD), Diisopropylazodicarboxylat (DIAD), Di-tert-butyl-azodicarboxylat, *N,N,N'N'-*Tetramethylazodicarboxamid (TMAD), 1,1'-(Azodicarbonyl)-dipiperidin (ADDP) oder 4,7-Dimethyl-3,5,7-hexahydro-1,2,4,7-tetrazocin-3,8-dion (DHTD). Bervorzugt werden Triphenylphosphin und Diisopropylazodicarboxylat (DIAD) verwendet.

Inerte Lösungsmittel für die Mitsunobu-Reaktion (IV) + (V) → (I-1) sind beispielsweise Ether wie Tetrahydrofuran, Diethylether, Kohlenwasserstoffe wie Benzol, Toluol, Xylol, Halogenkohlenwasserstoffe wie Dichlormethan, Dichlorethan oder andere Lösungsmittel wie Acetonitril oder Dimethylformamid (DMF). Ebenso ist es möglich, Gemische der genannten Lösungsmittel einzusetzen. Bevorzugt wird THF oder ein Gemisch von THF und DMF verwendet.

Die Mitsunobu-Reaktion (IV) + (V) → (I-1) erfolgt im Allgemeinen in einem Temperaturbereich von -78°C bis +180°C, bevorzugt bei 0°C bis +50°C, gegebenenfalls in einer Mikrowelle. Die Umsetzungen können bei normalem, erhöhtem oder bei erniedrigtem Druck durchgeführt werden (z.B. von 0.5 bis 5 bar).

Im Fall, dass X¹ für eine geeignete Abgangsgruppe steht erfolgt die Umsetzung (IV) + (V) → (I-1) unter Bedingungen einer nucleophilen Substitution. Inerte Lösungsmittel für den Verfahrensschritt (IV) + (V) → (I-1) sind dann beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, 1,2-Dichlorethan, Trichlorethylen oder Chlorbenzol, oder andere Lösungsmittel wie Dimethylformamid, Dimethylsulfoxid, *N,N'-*Dimethylpropylen-harnstoff (DMPU), *N*-Methylpyrrolidinon (NMP), Pyridin, Aceton, 2-Butanon oder Acetonitril. Ebenso ist es möglich, Gemische der genannten Lösungsmittel einzusetzen. Bevorzugt wird Acetonitril, DMF oder Acetonitril im Gemisch mit Dimethylformamid verwendet.

Als Base für den Verfahrensschritt (IV) + (V) → (I-1) eignen sich übliche anorganische Basen. Hierzu gehören insbesondere Alkali- oder Erdalkalicarbonate wie Lithium-, Natrium-, Kalium-, Calcium- oder Cäsiumcarbonat, gegebenenfalls unter Zusatz eines Alkaliiodids wie beispielsweise Kaliumiodid, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium- oder Kalium-bis(trimethylsilyl)amid oder Lithiumdiisopropylamid Bevorzugt werden Kaliumcarbonat mit Kaliumiodid oder Natriumhydrid verwendet.

Die Base wird hierbei im Allgemeinen in einer Menge von 1 bis 5 Mol, bevorzugt in einer Menge von 1.2 bis 3 Mol, bezogen auf 1 Mol der Verbindung der Formel (IV) eingesetzt.

Die Umsetzung (IV) + (V) → (I-1) erfolgt im Allgemeinen in einem Temperaturbereich von 0°C bis +100°C, bevorzugt bei +20°C bis +80°C, gegebenenfalls in einer Mikrowelle. Die Reaktion kann bei normalem, erhöhtem oder bei erniedrigtem Druck durchgeführt werden (z.B. von 0.5 bis 5 bar). Im Allgemeinen arbeitet man bei Normaldruck.

Inerte Lösungsmittel für den Verfahrensschritt (VII) → (IV) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Chlorbenzol, Dimethylformamid, Dimethylsulfoxid, *N,N'-*Dimethylpropylenharnstoff (DMPU), *N*-Methylpyrrolidinon (NMP), Pyridin, Aceton, 2-Butanon oder Acetonitril. Ebenso ist es möglich, Gemische der genannten Lösungsmittel einzusetzen. Bevorzugt wird Toluol verwendet.

Die Umsetzung (VII) → (IV) erfolgt im Allgemeinen in einem Temperaturbereich von 0°C bis +150°C, bevorzugt bei +20°C bis +120°C, gegebenenfalls in einer Mikrowelle. Die Reaktion kann bei normalem, erhöhtem oder bei erniedrigtem Druck durchgeführt werden (z.B. von 0.5 bis 5 bar). Im Allgemeinen arbeitet man bei Normaldruck.

Der Verfahrensschritt (X) + (XI) → (I-1) ähnelt eine in der Literatur als Chan-Lam Kupplung genannte Umsetzung. Inerte Lösungsmittel für den Verfahrensschritt (X) + (XI) → (I-1) sind Ether wie 1,4-Dioxan oder Tetrahydrofuran, Halogenkohlenwasserstoffe wie Dichlormethan, Trichlormethan, 1,2-Dichlorethan, oder andere Lösungsmittel wie Dimethylformamid (DMF), N-Methylpyrrolidon (NMP), Acetonitril oder Dimethylsulfoxid (DMSO). Ebenso ist es möglich, Gemische der genannten Lösungsmittel einzusetzen. Bevorzugt ist ein Gemisch aus Acetonitril und DMSO wenn (XI) einem Boronsäureester oder einem Trifluorborat-Salz entspricht, oder Dichlormethan wenn (XI) eine Boronsäure ist. In manchen Fällen ist die Zugabe von Molekularsieb vorteilhaft.

Als Basen für den Verfahrensschritt (X) + (XI) → (I-1) eignen sich Pyridin, Pyridinderivate wie z. B. DMAP oder organische tertiäre Amine wie z. B. Diisopropylethylamin oder Triethylamin. Bevorzugt wird Triethylamin wenn (XI) einem Boronsäureester oder einem Trifluorborat-Salz entspricht, oder Pyridin wenn (XI) eine Boronsäure ist.

Als Katalysator für den Verfahrensschritt (X) + (XI) → (I-1) eignen sich Kupfer(II)-Salze wie zum Beispiel Kupfer-(II)-Acetat oder Kupfer(II)triflat, bevorzugt ist Kupfer-(II)-Acetat.

Der Verfahrensschritt (X) + (XI) → (I-1) wird an der Luft durchgeführt oder unter einer sauerstoffhaltige Atmosphäre.

Die Reaktion (X) + (XI) → (I-1) wird im Allgemeinen in einem Temperaturbereich von 0°C bis +150°C, bevorzugt bei +20°C bis +80°C durchgeführt.

Die Hydrolyse der Ester-Gruppe R^{1A} der Verbindung (I-1) zu Verbindungen der Formel (I-2) erfolgt indem man die Ester in inerten Lösungsmitteln mit Säuren oder Basen behandelt, wobei bei Letzterem die zunächst entstehenden Salze durch Behandeln mit Säure in die freien Carbonsäuren überführt werden. Im Allgemeinen erfolgt die Ester-Hydrolyse bevorzugt mit Säuren.

Als inerte Lösungsmittel eignen sich für diese Reaktionen Wasser, Diethylether, Tetrahydrofuran, Dioxan oder Glykoldimethylether, oder andere Lösungsmittel wie Acetonitril, Essigsäure, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran oder Acetonitril verwendet. Bei der Hydrolyse von tert-Butylestern wird im Falle der Umsetzung mit Trifluoressigsäure bevorzugt Dichlormethan und im Falle der Umsetzung mit Chlorwasserstoff bevorzugt Tetrahydrofuran, Diethylether oder Dioxan als Lösungsmittel verwendet. Bei der Hydrolyse von anderen Estern unter sauren Bedingungen wird Essigsäure oder ein Gemisch von Essigsäure und Wasser bevorzugt.

Als Basen sind die Alkali- oder Erdalkalihydrogencarbonate wie Natrium- oder Kaliumhydrogencarbonat geeignet. Bevorzugt ist Natriumhydrogencarbonat.

Als Säuren eignen sich für die Esterspaltung im Allgemeinen Schwefelsäure, Chlorwasserstoff/Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische, gegebenenfalls unter Zusatz von Wasser. Bevorzugt sind Chlorwasserstoff oder Trifluoressigsäure im Falle der tert.-Butylester und Salzsäure im Gemisch mit Essigsäure, sowie Schwefelsäure im Gemisch mit Essigsäure und Wasser im Falle der Methylester und Ethylester.

Die Esterspaltung erfolgt im Allgemeinen in einem Temperaturbereich von 0°C bis 180°C, bevorzugt bei +20°C bis 120°C.

Die genannten Umsetzungen können bei normalem, erhöhtem oder bei erniedrigtem Druck durchgeführt werden (z.B. von 0.5 bis 5 bar). Im Allgemeinen arbeitet man jeweils bei Normaldruck.

Die Herstellung der erfindungsgemäßen Verbindungen kann durch die folgenden Syntheseschemata (Schema 1 bis 3) beispielhaft veranschaulicht werden: 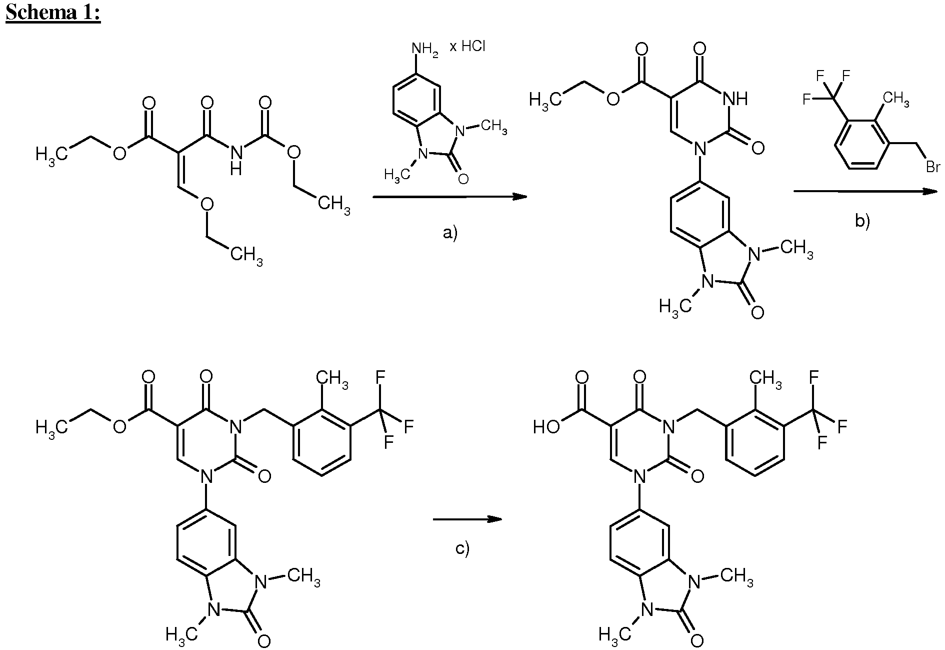 [a): 1. Triethylamin, Ethanol, 80°C; 2. Kalium-*tert*-butylat, 0°C-80°C; b): K₂CO₃, KI, DMF; c): Essigsäure / Salzsäure (2:1), 120°C]. 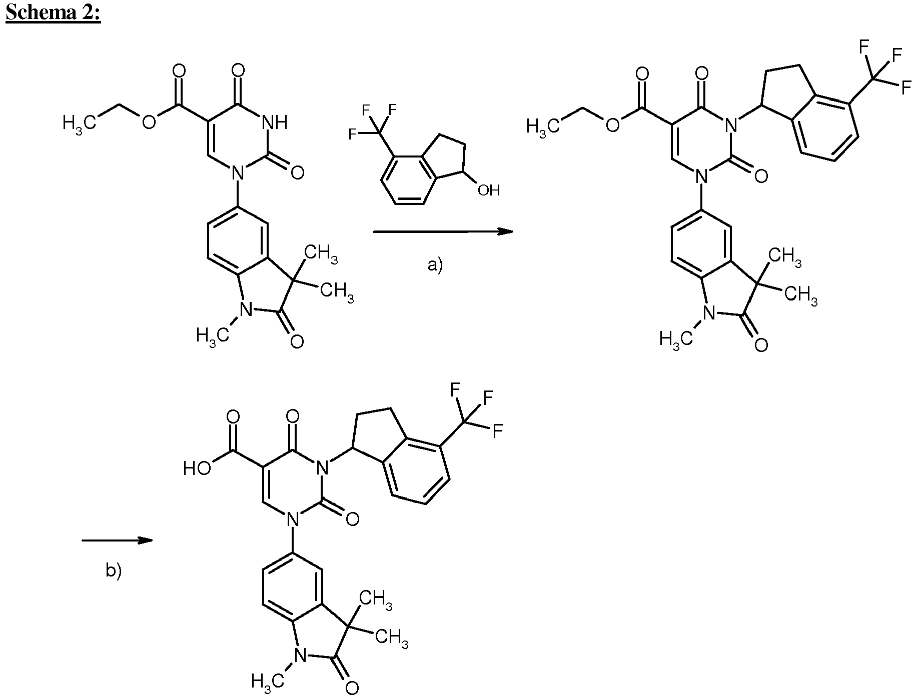 [a): Triphenylphosphin, DIAD, THF / DMF 1:1, 0°C-RT; b): Essigsäure / Salzsäure (2:1), 120°C]. 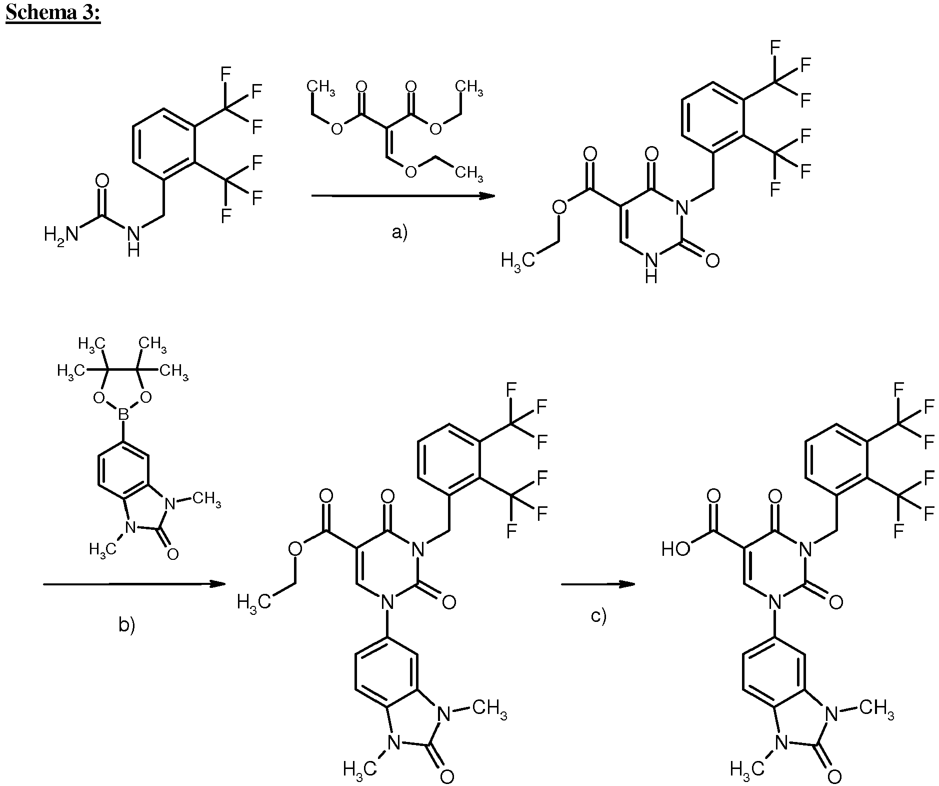 [a): *i*) 140°C, *ii*) Natriumethanolat, Ethanol, 80°C; b): Cu(OAc)₂, NEt₃, CH₃CN, DMSO. Molsieb, 80°C; c): Essigsäure / Salzsäure (2:1), 120°C].

Die Verbindungen der Formeln (II), (III), (V), (VI), (VIII), (IX) und (XI) sind kommerziell erhältlich, literaturbekannt oder können in Analogie zu literaturbekannten Verfahren hergestellt werden.

Weitere erfindungsgemäße Verbindungen können gegebenenfalls auch hergestellt werden durch Umwandlungen von funktionellen Gruppen einzelner Substituenten, insbesondere den unter R³ aufgeführten, ausgehend von den nach obigen Verfahren erhaltenen Verbindungen der Formel (I). Diese Umwandlungen werden wie im vorliegenden experimentellen Teil beschrieben, nach üblichen, dem Fachmann bekannten Methoden durchgeführt, und umfassen beispielsweise Reaktionen wie nukleophile und elektrophile Substitutionen, Oxidationen, Reduktionen, Hydrierungen, Übergangsmetall-katalysierte Kupplungsreaktionen, Eliminierungen, Alkylierung, Aminierung, Veresterung, Esterspaltung, Veretherung, Etherspaltung, Bildung von Carbonamiden, sowie Einführung und Entfernung temporärer Schutzgruppen. Die Umwandlung von funktionellen Gruppen kann durch das folgende Syntheseschema (Schema 4) beispielhaft veranschaulicht werden:

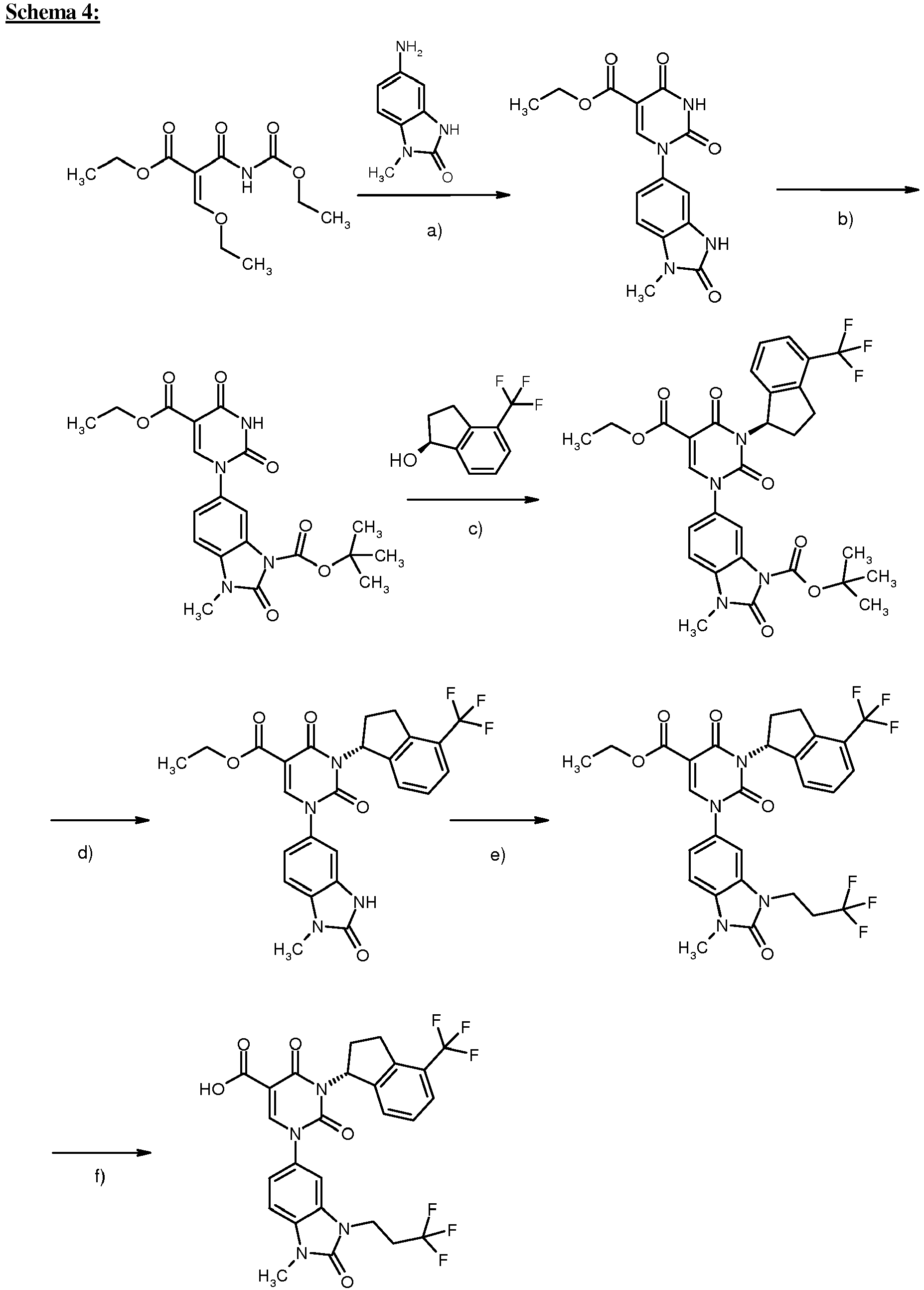

### [a): 1. Ethanol, 80°C 2. Kalium-tert-butylat 80°C; b): (^{t}BuOCO)₂O, DMAP, DMF / CH₂Cl₂, RT; c) Triphenylphosphin, DIAD, THF / DMF 1:1, 0°C-RT; d) CF₃COOH, CH₂Cl₂, RT; e) CF₃CH₂CH₂Br, Cs₂CO₃, KI, DMF, 60°C; f): Essigsäure / Salzsäure (2:1), 120°C].

Die erfindungsgemäßen Verbindungen besitzen wertvolle pharmakologische Eigenschaften und können zur Behandlung und/ oder Prophylaxe von Erkrankungen bei Menschen und Tieren verwendet werden.

Die erfindungsgemäßen Verbindungen stellen Inhibitoren der Chymase dar und eignen sich daher zur Behandlung und/oder Prophylaxe kardiovaskulärer, entzündlicher, allergischer und/ oder fibrotischer Erkrankungen.

Im Sinne der vorliegenden Erfindung sind unter Erkrankungen des Herzkreislauf-Systems beziehungsweise kardiovaskulären Erkrankungen beispielsweise die folgenden Erkrankungen zu verstehen: akute und chronische Herzinsuffizienz, arterielle Hypertonie, koronare Herzerkrankung, stabile und instabile Angina pectoris, myokardiale Ischämie, Myokardinfarkt, Schock, Atherosklerose, Herzhypertrophie, Herzfibrose, atriale und ventrikuläre Arrhythmien, transitorische und ischämische Attacken, Hirnschlag, Präeklampsie, entzündliche kardiovaskuläre Erkrankungen, periphere und kardiale Gefäßerhrankungen, periphere Durchblutungsstörungen, arterielle pulmonale Hypertonie, Spasmen der Koronararterien und peripherer Arterien, Thrombosen, thromboembolische Erkrankungen, Ödembildung wie zum Beispiel pulmonales Ödem, Hirnödem, renales Ödem oder Herzinsuffizienz-bedingtes Ödem, sowie Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), Reperfusionsschäden, arterielle und venöse Thrombosen, Mikroalbuminurie, Herzmuskelschwäche, endotheliale Dysfunktion, periphere und kardiale Gefäßerhrankungen, periphere Durchblutungsstörungen, Herzinsuffizienz-bedingtes Ödem, erhöhte Spiegel von Fibrinogen und von LDL geringer Dichte sowie erhöhte Konzentrationen von Plasminogenaktivator-Inhibitor 1 (PAI-1).

Im Sinne der vorliegenden Erfindung umfasst der Begriff Herzinsuffizienz auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzhlappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz.

Die erfindungsgemäßen Verbindungen sind weiter geeignet für die Prophylaxe und/oder Behandlung der polyzystischen Nierenkrankheit (PCKD) und des Syndroms der inadäquaten ADH-Sekretion (SIADH).

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen.

Im Sinne der vorliegenden Erfindung umfasst der Begriff akute Niereninsuffizienz akute Erscheinungsformen der Nierenerkrankung, des Nierenversagens und/ oder der Niereninsuffizienz mit und ohne Dialysepflicht, wie auch zugrundeliegende oder verwandte Nierenerkrankungen wie renale Hypoperfusion, intradialytische Hypotonie, Volumenmangel (z.B. Dehydratation, Blutverlust), Schock, akute Glomerulonephritis, hämolytischurämisches Syndrom (HUS), vaskuläre Kathastrophe (arterielle oder venöse Thrombose oder Embolie), Cholesterinembolie, akute Bence-Jones-Niere bei Plasmozytom, akute supravesikal oder subvesikale Abflussbehinderungen, immunlogische Nierenerkrankungen wie Nierentransplantatabstoßung, Immunkomplex-induzierte Nierenerkrankungen, tubuläre Dilatation, Hyperphosphatämie und/ oder akute Nierenerkrankungen, die durch die Notwendigkeit zur Dialyse charakterisiert werden können, sowie bei Teilresektionen der Niere, Dehydratation durch forcierte Diurese, unkontrolliertem Blutdruckanstieg mit maligner Hypertonie, Harnwegsobstruktion und -infekt und Amyloidose sowie Systemerkrankungen mit glomerulärer Beteiligung, wie rheumatologisch-immunologische Systemerhrankungen, wie beispielsweise Lupus erythematodes, Nierenarterienthrombose, Nierenvenenthrombose, Analgetikanephropathie und renal-tubuläre Azidose, sowie Röntgen-Kontrastmittel-sowie Medikamenten-induzierte akute interstitielle Nierenerkrankungen.

Im Sinne der vorliegenden Erfindung umfasst der Begriff chronische Niereninsuffizienz chronische Erscheinungsformen der Nierenerkrankung, des Nierenversagens und/ oder der Niereninsuffizienz mit und ohne Dialysepflicht, wie auch zugrundeliegende oder verwandte Nierenerkrankungen wie renale Hypoperfusion, intradialytische Hypotonie, obstruktive Uropathie, Glomerulopathien, glomeruläre und tubuläre Proteinurie, renale Ödeme, Hämaturie, primäre, sekundäre sowie chronische Glomerulonephritis, membranöse und membranoproliferative Glomerulonephritis, Alport-Syndrom, Glomerulosklerose, tubulointerstitielle Erkrankungen, nephropathische Erkrankungen wie primäre und angeborene Nierenerkrankung, Nierenentzündung, immunlogische Nierenerkrankungen wie Nierentransplantatabstoßung, Immunkomplex-induzierte Nierenerkrankungen, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/ oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/ oder die Notwendigkeit zur Dialyse charakterisiert werden können, sowie bei Nierenzellkarzinomen, nach Teilresektionen der Niere, Dehydratation durch forcierte Diurese, unkontrollierter Blutdruckanstieg mit maligner Hypertonie, Harnwegsobstruktion und -infekt und Amyloidose sowie Systemerkrankungen mit glomerulärer Beteiligung, wie rheumatologisch-immunologische Systemerkrankungen, wie beispielsweise Lupus erythematodes, sowie Nierenarterienstenose, Nierenarterienthrombose, Nierenvenenthrombose, Analgetikanephropathie und renal-tubuläre Azidose zu verstehen. Weiterhin Röntgen-Kontrastmittel- sowie Medikamenten-induzierte chronische interstitielle Nierenerkrankungen, Metabolisches Syndrom und Dyslipidämie. Die vorliegende Erfindung umfasst auch die Verwendung der erfindungsgemäßen Verbindungen zur Behandlung und/ oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von pulmonaler arterieller Hypertonie (PAH) und anderen Formen der pulmonalen Hypertonie (PH), der chronischobstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem), der zystischer Fibrose (CF), von akutem Koronarsyndrom (ACS), Herzmuskelentzündungen (Myokarditis) und anderen autoimmune Herzerkrankungen (Perikarditis, Endokarditis, Valvolitis, Aortitis, Kardiomyopathien), kardiogenem Schock, Aneurysmen, Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hautrkrankungen sowie entzündlichen Augenerkrankungen.

Die erfindungsgemäßen Verbindungen können weiterhin verwendet werden zur Behandlung und/ oder Prophylaxe von asthmatischen Erkrankungen unterschiedlicher Schweregrade mit intermittierendem oder persistierendem Verlauf (refraktives Asthma, bronchiales Asthma, allergisches Asthma, intrinsisches Asthma, extrinsisches Asthma, durch Medikamente oder durch Staub induziertes Asthma), von verschiedenen Formen der Bronchitis (chronische Bronchitis, infektiöse Bronchitis, eosinophile Bronchitis), von Bronchiolitis obliterans, Bronchiektasie, Pneumonie, idiopathischer interstitieller Pneumonie, Farmerlunge und verwandten Krankheiten, Hustenund Erkältungskrankheiten (chronischer entzündlicher Husten, iatrogener Husten), Nasenschleimhautentzündungen (einschließlich medikamentöse Rhinitis, vasomotorische Rhinitis und jahreszeitabhängige, allergische Rhinitis, z.B. Heuschnupfen) und von Polypen.

Weiterhin sind die erfindungsgemäßen Verbindungen zur Behandlung und/ oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Kardiomyopathie, Nephropathie, Glomerulonephritis, interstitielle Nierenfibrose, fibrotische Schäden in Folge von Diabetes, Knochenmarksfibrose und ähnliche fibrotische Erkrankungen, Skleroderma, Morphaea, Keloide, hypertrophe Narbenbildung (auch nach chirurgischen Eingriffen), Naevi, diabetische Retinopathie und proliferative Vitroretinopathie.

Weiterhin eignen sich die erfindungsgemäßen Verbindungen zur Bekämpfung postoperativer Narbenbildung, z.B. in Folge von Glaukoma-Operationen.

Des Weiteren können die erfindungsgemäßen Verbindungen ebenfalls kosmetisch bei alternder und verhornender Haut eingesetzt werden.

Darüber hinaus können die erfindungsgemäßen Verbindungen auch eingesetzt werden zur Behandlung und/oder Prophylaxe von Dyslipidämien (Hypercholesterolämie, Hypertriglyceridämie, erhöhte Konzentrationen der postprandialen Plasma-Triglyceride, Hypoalphalipoproteinämie, kombinierte Hyperlipidämien), Nephropathie und Neuropathie), Krebserkrankungen (Hautkrebs, Hirntumore, Brustkrebs, Knochenmarktumore, Leukämien, Liposarcome, Karzinome des Magen-Darm-Traktes, der Leber, Bauchspeicheldrüse, Lunge, Niere, Harnleiter, Prostata und des Genitaltraktes sowie bösartige Tumore des lymphoproliferativen Systems wie z.B. Hodgkin's und Non-Hodgkin's Lymphom), von Erkrankungen des Gastrointestinaltraktes und des Abdomen (Glossitis, Gingivitis, Periodontitis, Oesophagitis, eosinophile Gastroenteritis, Mastocytose, Morbus Crohn, Colitis, Proctitis, Pruritis ani, Diarrhöe, Zöliakie, Hepatitis, chronischer Hepatitis, Leberfibrose, Leberzirrhose, Pankreatitis und Cholecystitis), Hauterkrankungen (allergische Hauterkrankungen, Psoriasis, Akne, Ekzeme, Neurodermitis, vielfältige Formen der Dermatitis, sowie Keratitis, Bullosis, Vasculitis, Cellulitis, Panniculitis, Lupus erythematodes, Erythema, Lymphome, Hautkrebs, Sweet-Syndrom, Weber-Christian-Syndrom, Narbenbildung, Warzenbildung, Frostbeulen), von Erkrankungen des Skelettknochens und der Gelenke sowie der Skelettmuskel (vielfältige Formen der Arthritis, vielfältige Formen der Arthropathien, Sklerodermie sowie von weiteren Erkrankungen mit einer entzündlichen oder immunologischen Komponente, wie beispielsweise paraneoplastisches Syndrom, bei Abstoßungsreaktionen nach Organtransplantationen und zur Wundheilung und Angiogenese insbesondere bei chronischen Wunden.

Die erfindungsgemäßen Verbindungen der Formel (I) eignen sich weiterhin zur Behandlung und/oder Prophylaxe von ophthalmologischen Erkrankungen wie beispielsweise Glaukom, normotensivem Glaukom, Augenhochdruck und deren Kombinationen, von altersbedingter Makuladegeneration (AMD), trockener oder nichtexsudativer AMD, feuchter oder exsudativer oder neovaskulärer AMD, choroidaler Neovascularization (CNV), Netzhautablösung, diabetischer Retinopathie, atrophischen Veränderungen des retinalen Pigmentepithels (RPE), hypertrophischen Veränderungen des retinalen Pigmentepithels (RPE), diabetischem Makulaödem, Netzhautvenenverschluss, choroidalem Netzhautvenenverschluss, Makulaödem, Makulaödem aufgrund von Netzhautvenenverschluss, Angiogenese an der Vorderseite des Auges wie kornealer Angiogenese beispielsweise nach Keratitis, Hornhauttransplantation oder Keratoplastik, korneale Angiogenese aufgrund von Hypoxie (extensives Tragen von Kontaktlinsen), Pterygium conjunctivae, subretinalem Ödem und intraretinalem Ödem.

Desweiteren eignen sich die erfindungsgemäßen Verbindungen der Formel (I) zur Behandlung und/oder Prophylaxe von erhöhten und hohem Augeninnendruck als Folge von traumatischem Hyphaema, periorbitalem Ödem, postoperativer viscoelastischer Retention, intraokularer Entzündung, Anwendung von Kortikosteroiden, Pupillarblock oder idiopathischen Ursachen sowie von erhöhtem Augeninnendruck nach Trabekulektomie und aufgrund von prä-operativen Zusätzen.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung sind die erfindungsgemäßen Verbindungen zur Verwendung in einem Verfahren zur Behandlung und/oder Prophylaxe von Herzinsuffizienz, pulmonaler Hypertonie, chronisch-obstruktiver Lungenerkrankung, Asthma, Niereninsuffizienz, Nephropathien, fibrotischen Erkrankungen der inneren Organe und dermatologischen Fibrosen.

Die erfindungsgemäßen Verbindungen können allein oder bei Bedarf in Kombination mit anderen Wirkstoffen eingesetzt werden. Weiterer Gegenstand der vorliegenden Erfindung sind daher Arzneimittel, enthaltend min-destens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
die Signaltransduktionskaskade inhibierende Verbindungen, beispielhaft und vorzugsweise aus der Gruppe der Kinase-Inhibitoren, insbesondere aus der Gruppe der Tyrosinkinase- und/oder Serin/Threoninkinase-Inhibitoren;
Verbindungen, die den Ab- und Umbau der Extrazellulärmatrix inhibieren, beispielhaft und vorzugsweise Inhibitoren der Matrix-Metalloproteasen (MMPs), insbesondere Inhibitoren von Stromelysin, Kollagenasen, Gelatinasen und Aggrecanasen (hierbei vor allem von MMP-1, MMP-3, MMP-8, MMP-9, MMP-10, MMP-11 und MMP-13) sowie der Metallo-Elastase (MMP-12);
Verbindungen, die die Bindung von Serotonin an dessen Rezeptor blockieren, beispielhaft und vorzugsweise Antagonisten des 5-HT_{2b}-Rezeptors;
organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
NO-unabhängige, jedoch Häm-abhängige Stimulatoren der löslichen Guanylatcyclase, wie insbesondere die in WO 00/06568, WO 00/06569, WO 02/42301 und WO 03/095451 beschriebenen Verbindungen;
NO- und Häm-unabhängige Aktivatoren der löslichen Guanylatcyclase, wie insbesondere die in WO 01/19355, WO 01/19776, WO 01/19778, WO 01/19780, WO 02/070462 und WO 02/070510 beschriebenen Verbindungen; Prostacyclin-Analoga, wie beispielhaft und vorzugsweise Iloprost, Beraprost, Treprostinil oder Epoprostenol; Verbindungen, die die lösliche Epoxidhydrolase (sEH) inhibieren, wie beispielsweise *N,N*'-Dicyclohexylharnstoff, 12-(3-Adamantan-1-yl-ureido)-dodecansäure oder 1-Adamantan-1-yl-3-{5-[2-(2-ethoxyethoxy)ethoxy]-pentyl}-harnstoff;
den Energiestoffwechsel des Herzens beeinflussende Verbindungen, wie beispielhaft und vorzugsweise Etomoxir, Dichloracetat, Ranolazine oder Trimetazidine;
Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil;
antithrombotisch wirkende Mittel, beispielhaft und vorzugsweise aus der Gruppe der Thrombozytenaggregationshemmer, der Antikoagulantien oder der profibrinolytischen Substanzen;
den Blutdruck senkende Wirkstoffe, beispielhaft und vorzugsweise aus der Gruppe der Calcium-Antagonisten, Angiotensin AII-Antagonisten, ACE-Inhibitoren, Vasopeptidase-Inhibitoren, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika;
Vasopressin-Rezeptor-Antagonisten, wie beispielsweise und vorzugsweise Conivaptan, Tolvaptan, Lixivaptan, Mozavaptan, Satavaptan, SR-121463, RWJ 676070 oder BAY 86-8050;
bronchodilatorisch wirkende Mittel, beispielhaft und vorzugsweise aus der Gruppe der beta-adrenergen Rezeptor-Agonisten, wie insbesondere Albuterol, Isoproterenol, Metaproterenol, Terbutalin, Formoterol oder Salmeterol, oder aus der Gruppe der Anticholinergika, wie insbesondere Ipratropiumbromid;
anti-inflammatorisch wirkende Mittel, beispielhaft und vorzugsweise aus der Gruppe der Glucocorticoide, wie insbesondere Prednison, Prednisolon, Methylprednisolon, Triamcinolon, Dexamethason, Beclomethason, Betamethason, Flunisolid, Budesonid oder Fluticason; und/ oder
den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Bei einer bevorzugten Ausführungsform der Erfindung werden die erfindungsgemäßen Verbindungen in Kombination mit einem Kinase-Inhibitor, wie beispielhaft und vorzugsweise Bortezomib, Canertinib, Erlotinib, Gefitinib, Imatinib, Lapatinib, Lestaurtinib, Lonafarnib, Pegaptinib, Pelitinib, Semaxanib, Sorafenib, Regorafenib, Sunitinib, Tandutinib, Tipifarnib, Vatalanib, Fasudil, Lonidamin, Leflunomid, BMS-3354825 oder Y-27632, eingesetzt.

Bei einer bevorzugten Ausführungsform der Erfindung werden die erfindungsgemäßen Verbindungen in Kombination mit einem Serotonin-Rezeptor-Antagonisten, wie beispielhaft und vorzugsweise PRX-08066, eingesetzt.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, DU-176b, Fidexaban, Razaxaban, Fondaparinux, Idraparinux, PMD-3112, YM-150, KFA-1982, EMD-503982, MCM-17, mLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Inhibitor, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095, SB-772077, GSK-269962A oder BA-1049, verabreicht.

Bei einer bevorzugten Ausführungsform der Erfindung werden die erfindungsgemäßen Verbindungen in Kombination mit einem Diuretikum, wie beispielhaft und vorzugsweise Furosemid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. inhalativ, intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer, Aerosole), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale, die intravenöse und die inhalative Applikation.

Die erfindungsgemäßen Verbindungen können in die angeführten Applikationsformen überführt werden. Dies kann in an sich bekannter Weise durch Mischen mit inerten, nicht-toxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich, sofern nicht anders angegeben, jeweils auf das Volumen.

### A. Beispiele

### Abkürzungen:

- Ac: Acetyl
- aq.: wässrig, wässrige Lösung
- br.d: breites Dublett (NMR)
- br.m: breites Multiplett (NMR)
- br.s: breites Singulett (NMR)
- br.t: breites Triplett (NMR)
- c: Konzentration
- cat.: katalytisch
- DC: Dünnschichtchromatographie
- DCI: direkte chemische Ionisation (bei MS)
- dest.: destilliert
- DIAD: Diisopropylzaodicarboxylat
- DIEA: *N,N*-Diisopropylethylamin
- DMAP: 4-*N,N*-Dimethylaminopyridin
- DMF: Dimethylformamid
- DMSO: Dimethylsulfoxid
- DSC: Differential Scanning Thermography
- d. Th.: der Theorie (bei Ausbeute)
- EDC: *N*-(3-dimethylaminopropli)-*N*-ethycarbodiimid-Hydrochlorid
- ee: Enantiomerenüberschuss
- ent: enantiomerenrein, Enantiomer
- eq.: Äquivalent(e)
- ESI: Elektrospray-Ionisation (bei MS)
- Et: Ethyl
- GC-MS: Gaschromatographie-gekoppelte Massenspektrometrie
- h: Stunde(n)
- HATU: *O*-(7-Azabenzotriazol-1-yl)-*N,N,N,'N'*-tetramethyluronium-hexafluorophosphat
- HOBt: 1-Hydroxy-1*H*-benzotriazol-Hydrat
- HPLC: Hochdruck-, Hochleistungsflüssigchromatographie
- konz.: Konzentriert
- LC-MS: Flüssigchromatographie-gekoppelte Massenspektrometrie
- Me: Methyl
- min: Minute(n)
- MPLC: Mitteldruckflüssigchromatographie
- MS: Massenspektrometrie
- MTBE: Methyl-*tert.*-butylether
- NMR: Kernresonanzspektrometrie
- Pd/C: Palladium auf Aktivkohle
- Ph: Phenyl
- PyBOP: Benzotriazol-1-yloxy-tris(pyrrolidino)phosphonium-hexafluorophosphat
- quant.: quantitativ (bei Ausbeute)
- rac: racemisch, Racemat
- RT: Raumtemperatur
- Rₜ: Retentionszeit (bei HPLC)
- Schmp.: Schmelzpunkt
- tBu: *tert.*-Butyl
- tert.: Tertiär
- TFA: Trifluoressigsäure
- TFAA: Trifluoressigsäureanhydrid
- THF: Tetrahydrofuran
- TPPO: Triphenylphosphinoxid
- UV: Ultraviolett-Spektrometrie
- vgl.: vergleiche
- v/v: Volumen zu Volumen-Verhältnis (einer Lösung)

### HPLC-, GC-MS- und LC-MS-Methoden:

Methode 1: Instrument: Waters ACQUITY SQD UPLC System; Säule: Waters Acquity UPLC HSS T3 1.8 µm 50 x 1 mm; Eluent A: 11 Wasser + 0.25 ml 99 %ige Ameisensäure , Eluent B: 11 Acetonitril + 0.25 ml 99 %ige Ameisensäure; Gradient: 0.0 min 90 % A → 1.2 min 5% A → 2.0 min 5 % A Ofen: 50°C; Fluss: 0.40 ml/min; UV-Detektion: 210 - 400 nm.
Methode 2: Gerätetyp MS: Waters (Micromass) Quattro Micro; Gerätetyp HPLC: Agilent 1100 Serie; Säule : Thermo Hypersil GOLD 3 µ 20 x 4 mm; Eluent A: 11 Wasser + 0.5 mal 50 %ige Ameisensäure, Eluent B: 11 Acetonitril + 0.5 mal 50 %ige Ameisensäure; Gradient: 0.0 min 100 % A → 3.0 min 10% A → 4.0 min 10% A → 4.01 min 100 % A (Flow 2.5 ml) → 5.00 min 100 % A; Ofen: 50°C; Fluss: 2 ml/min; UV-Detektion: 210 nm.
Methode 3: Instrument: Micromass Quattro Premier mit Waters UPLC Acquity; Säule: Thermo Hypersil GOLD 1.9 µm 50 x 1 mm; Eluent A: 11 Wasser + 0.5 ml 50 %ige Ameisensäure, Eluent B: 11 Acetonitril + 0.5 mal 50 %ige Ameisensäure; Gradient: 0.0 min 90% A → 0.1 min 90% A → 1.5 min 10% A → 2.2 min 10% A Ofen: 50°C; Fluss: 0.33 ml/min; UV-Detektion: 210 nm.
Methode 4: Instrument: Micromass Quattro Premier mit Waters UPLC Acquity; Säule: Thermo Hypersil GOLD 1.9 µm 50 x 1 mm; Eluent A: 11 Wasser + 0.5 ml 50 %ige Ameisensäure, Eluent B: 11 Acetonitril + 0.5 ml 50 %ige Ameisensäure; Gradient: 0.0 min 97% A → 0.5 min 97% A → 3.2 min 5% A → 4.0 min 5 % A Ofen: 50°C; Fluss: 0.3 ml/min; UV-Detektion: 210 nm.
Methode 5 (LC-MS): Instrument: Waters ACQUITY SQD UPLC System; Säule: Waters Acquity UPLC HSS T3 1.8 µm 30 x 2 mm; Eluent A: 11 Wasser + 0.25 mal 99 %ige Ameisensäure , Eluent B: 11 Acetonitril + 0.25 ml 99 %ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.
Methode 6 (GC-MS): Instrument: Micromass GCT, GC6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).
Methode 7 (präparative HPLC): Säule: Reprosil C18, 10 µm, 250 mm x 30 mm. Eluent A: Ameisensäure 0.1 % in Wasser, Eluent B: Acetonitril; Fluss: 50 ml/min; Programm: 0 bis 6 min: 90%A /10% B; 6 min bis 27 min: Gradient bis 95% B; 27 min bis 38 min 95 %B; 38 min bis 39 min Gradient bis 10 %B; 39 min bis 43 min (Ende): 60%A/ 40 %B. Geringfügige Abweichungen des Gradients sind möglich.
Methode 8 (präparative HPLC): Säule: Reprosil C18, 10 µm, 250 mm x 30 mm. Eluent A: Ameisensäure 0.1 % in Wasser, Eluent B: Methanol; Fluss: 50 ml/min; Programm: 0 bis 4.25 min: 60%A /40% B; 4.25 bis 4.50 min: Gradient bis 60% B; 4.50 min bis 17 min Gradient bis 100 %B; 17 min bis 19.50 min 100 %B; 19.50 min bis 19.75 min Gradient bis 40 %B; 19.75 bis 22 min (Ende): 60%A/ 40 %B. Geringfügige Abweichungen des Gradients sind möglich.
Methode 9 (präparative HPLC): Säule: Sunfire C18, 5 µm, 250 mm x 20 mm. Eluent Methanol / TFA 1% in Wasser 50 / 50; Fluss: 25 ml/min; Detection 210 nm, Temperatur 40°C.
Methode 10 (präparative HPLC): Säule: Sunfire C18, 5 µm, 250 mm x 20 mm. Eluent Acetonitril / TFA 1% in Wasser 55/45; Fluss: 25 ml/min; Detection 210 nm, Temperatur 40°C.
Methode 11: (präparative HPLC): Säule: Reprosil C18, 10 µm, 250 mm x 40 mm. Eluent A: Ameisensäure 0.1 % in Wasser, Eluent B: Acetonitril; Fluss: 50 ml/min. Programm: 0-6 min: 90%A /10% B; 6-40 min: Gradient bis 95% B; 40-53 min: 5% A /95% B; 53.01-54 min: Gradient bis 10% B; 54.01-57 min: 90%A /10%B.
Methode 12 (chirale präparative HPLC): Säule Daicel Chiralpak AD-H 250 mm x 20 mm; Fluss: 20 ml/min; Eluent: *iso*-Propanol / Ethanol / *iso*-hexan 15:15:70 (v/v/v); Detector 230 nm.
Methode 13 (chirale analytische HPLC): Säule Daicel Chiralpak AD-H 5 µm, 250 mm x 4.6 mm; Temperatur 30°C; Fluss: 1 ml/min; Eluent: *iso*-Propanol / Ethanol / *iso*-hexan 15:15:70 (v/v/v); Detector 220 nm.
Methode 14 (chirale analytische HPLC): Säule Daicel Chiralpak AS-H 5 µm, 250 mm x 4.6 mm; Temperatur 30°C; Fluss: 1 ml/min; Eluent: Ethanol / iso-hexan 50:50 versetzt mit 1% Wasser und 0.2% Trifluoressigsäure; Detector 220 nm.
Methode 15 (präparative HPLC): Wie Methode 7 aber mit Säule Chromatorex C18 250 mm x 30 mm.
Methode 16 (chirale präparative HPLC): Säule Daicel Chiralpak AZ-H 250 mm x 20 mm; Fluss: 20 ml/min; Eluent: Ethanol / iso-hexan 50:50 (v/v) versetzt mit 1% Wasser und 0.2% Trifluoressigsäure; Detector 230 nm.
Methode 17 (chirale analytische HPLC): Säule Daicel Chiralpak AZ-H 5 µm, 250 mm x 4.6 mm; Temperatur 40°C; Fluss: 1 ml/min; Eluent: Ethanol / *iso*-hexan 50:50 (v/v) versetzt mit 1% Wasser und 0.2% Trifluoressigsäure; Detector 220 nm.
Methode 18 (chirale präparative HPLC):: Säule Daicel Chiralpak AD-H 250 mm x 20 mm; Fluss: 20 ml/min; Eluent: iso-Propanol / *iso*-hexan 50 : 50 (v/v) versetzt mit 1% Wasser und 0.2% Trifluoressigsäure; Detector 230 nm.
Methode 19 (chirale analytische HPLC): Säule Daicel Chiralpak AD-H 5 µm, 250 mm x 4.6 mm; Temperatur 30°C; Fluss: 1 ml/min; Eluent: *iso*-Propanol / *iso*-hexan 50 : 50 (v/v) versetzt mit 1% Wasser und 0.2% Trifluoressigsäure;; Detector 220 nm.
Methode 20 (chirale präparative HPLC): Säule Daicel Chiralpak AD-H 250 mm x 20 mm; Fluss: 20 ml/min; Eluent: Ethanol / *iso*-hexan 70:30 (v/v) versetzt mit 1% Wasser und 0.2% Trifluoressigsäure; Detector 230 nm.
Methode 21 (chirale analytische HPLC): Säule Daicel Chiralpak AD-H 5 µm, 250 mm x 4.6 mm; Temperatur 40°C; Fluss: 1 ml/min; Eluent: Ethanol / iso-hexan 70 : 30 (v/v) versetzt mit 1% Wasser und 0.2% Trifluoressigsäure; Detector 220 nm.
Methode 22 (präparative HPLC): Säule: Sunfire C18, 5 µm, 250 mm x 20 mm. Eluent Acetonitril/Wasser 60:40; Fluss: 25 ml/min; Detection 210 nM, Temperatur 40°C.
Methode 24 (präparative HPLC): Säule: Sunfire C18, 5 µm, 75 mm x 30 mm. Eluent Acetonitril / 0.05 %iger TFA in Wasser 1 : 99 bis 2.25 min, dann Acetonitril / 1% TFA in Wasser 95:5; Fluss: 60 ml/min; Detection 210 nM, Temperatur 40°C.
Methode 25 (chirale analytische HPLC): Säule Daicel Chiralpak AD-H 5µm, 250 mm x 4.6 mm; Temperatur 30°C; Fluss: 1 ml/min; Eluent: *iso*-Propanol / *iso*-hexan 5 : 95 (v/v); Detector 220 nm.
Methode 26: MS, Instrument: Thermo Fisher-Scientific DSQ; chemische Ionisierung; Reaktantgas NH₃; Quellentemperatur: 200°C; Ionisierungsenergie 70eV.
Methode 27 (chirale analytische HPLC): Säule Daicel Chiralpak AD-H 5µm, 250 mm x 4.6 mm; Temperatur 30°C; Fluss: 1 ml/min; Eluent: iso-Propanol / Ethanol / iso-hexan 25 : 25 : 50 (v/v/v); Detector 220 nm.
Methode 28 (LC-MS): MCW_SQ-HSST3_long Instrument: Waters ACQUITY SQD UPLC System; Säule: Waters Acquity UPLC HSS T3 1.8 µ 50 x 1 mm; Eluent A: 11 Wasser + 0.25 ml 99 %ige Ameisensäure, Eluent B: 11 Acetonitril + 0.25 ml 99 %ige Ameisensäure; Gradient: 0.0 min 95% A → 6.0 min 5% A → 7.5 min 5 % A; Ofen: 50°C; Fluss: 0.35 ml/min; UV-Detektion: 210 - 400 nm.
Methode 29 (chirale präparative HPLC): Säule Daicel Chiralpak IC 5µm, 250 mm x20 mm; Fluss: 20 ml/min; Temperatur 25°C; Detector: 220 nm; Eluent: Acetonitril / MTBE 50: 50 (v/v).
Methode 30 (chirale analytische HPLC): Säule Daicel Chiralpak IC 5µm, 250 mm x4.6 mm; Fluss: 1 ml/min; Temperatur 30°C; Detector: 220 nm; Eluent: Acetonitril / MTBE 50: 50 (v/v).
Methode 31 (chirale präparative HPLC): Säule Daicel Chiralpak IA 5µm, 250 mm x20 mm; Fluss: 20 ml/min; Temperatur 30°C; Detector: 285 nm; Eluent: Acetonitril / MTBE 50: 50 (v/v).
Methode 32 (chirale analytische HPLC): Säule Daicel Chiralpak IA 5µm, 250 mm x4.6 mm; Fluss: 1 ml/min; Temperatur 30°C; Detektor: 285 nm; Eluent: Acetonitril / MTBE 50: 50 (v/v).
Methode 33 (chirale präparative HPLC): Säule Daicel Chiralpak IA 5µm, 250 mm x20 mm; Fluss: 20 ml/min; Temperatur 30°C; Detektor: 285 nm; Eluent: Acetonitril / MTBE 20: 80 (v/v).
Methode 34 (chirale analytische HPLC): Säule Daicel Chiralpak IA 5µm, 250 mm x4.6 mm; Fluss: 1 ml/min; Temperatur 30°C; Detektor: 285 nm; Eluent: Acetonitril / MTBE 50: 50 (v/v).

### Ausgangsverbindungen und Intermediate:

### Beispiel 1A

### 5-Amino-1,3-dimethyl-1,3-dihydro-2H-benzimidazol-2-on Hydrochlorid

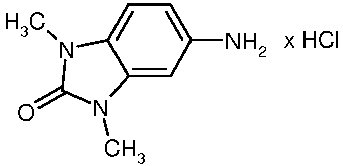

33.2 g (160 mmol) 1,3-Dimethyl-5-nitro-1,3-dihydro-2H-benzimidazol-2-on (Herstellung: siehe WO 2007/120339, Beispiel 2, Seite 33) wurden in 1790 ml Ethanol (nur teilweise gelöst) in Gegenwart von 8.8 g Palladium-Katalysator (10%-ig auf Aktivkohle mit 50% Wasser angefeuchtet) bei RT und 1 atm WasserstoffDruck hydriert. Das Ausgangsmaterial löste sich im Laufe der Reaktion. Nach vollständiger Umsetzung (6 h) wurde der Katalysator durch Filtration über Kieselgur entfernt. Das Filtrat wurde mit 45 ml einer Chlorwasserstoff-Lösung (4N in Dioxan) versetzt, dann am Rotationsverdampfer zur Trockne eingeengt. Der Rückstand wurde dann im HV weiter getrocknet. Man erhielt 31.8 g (91% d. Th.) der Titelverbindung.
LC-MS (Methode 1): Rₜ = 0.18 min; m/z = 178 (M+H)+.
¹H-NMR (400MHz, DMSO-d₆): δ [ppm] = 3.33 (s, 3H), 3.34 (s, 3H), 7.06 - 7.15 (m, 2H), 7.23 (d, 1H), 10.29 (br.s, 3H).

### Beispiel 2A

### Ethyl-1-(1,3-dimethyl-2-oxo-2,3-dihydro-1H-benzimidazol-5-yl)-2,4-dioxo-1,2,3,4-tetrahydropyrimidin-5-carboxylat

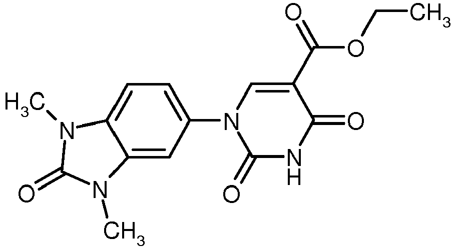

52.80 g (247.1 mmol) der Verbindung aus Beispiel 1A und 64.07 g (247.1 mmol) Ethyl-3-ethoxy-2-[(ethoxycarbonyl)carbamoyl]acrylat (Darstellung siehe: Senda, Shigeo; Hirota, Kosaku; Notani, Jiyoji, Chemical & Pharmaceutical Bulletin (1972), 20(7), 1380-8) wurden in 2 L Ethanol vorgelegt und mit 51.7 ml (370.7 mmol) Triethylamin versetzt. Die entstandene dicke Suspension wurde 1.5 h auf Rückflusstemperatur erhitzt, wobei eine klare Lösung entstand. Nach leichtem Abkühlen (ca. 60°C) wurden 27.73 g (247.1 mmol) Kalium*tert.*-butylat zugegeben. Die Reaktionsmischung wurde wieder auf Rückflußtemperatur erhitzt und 7 h bei dieser Temperatur weitergerührt. Nach Abkühlen auf RT wurde am Rotationsverdampfer ungefähr die Hälfte des Lösungsmittel entfernt. Das aufkonzentrierte Reaktionsgemisch wurde in 7.5 L IN Salzsäure gegossen. Der ausgefallene Feststoff wurde abfiltriert, mit 800 ml Wasser gewaschen und im HV getrocknet. Man erhielt 71.7 g (85 % d. Th.) der Titelverbindung.
LC-MS (Methode 1): Rₜ = 0.63 min; m/z = 345 (M+H)⁺.
¹H-NMR (400MHz, DMSO-d₆): δ [ppm]= 1.22 (t, 2H), 3.30 (s, 3H), 3.37 (s, 3H), 4.17 (q, 2H), 7.19 (dd, 1H), 7.25 (d, 1H), 7.37 (d, 1H), 8.26 (s, 1H).

### Beispiel 3A

### 1,3-Dimethyl-5-(4,4,5,5-tetramethyl-1,3,2-dioxaborolan-2-yl)-1,3-dihydro-2H-benzimidazol-2-on

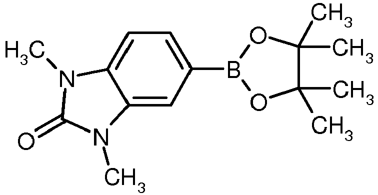

3.16 g 4,4,4',4',5,5,5',5'-Octamethyl-2,2'-bi-1,3,2-dioxaborolan (12.44 mmol) und 43 mg (70%-ige Reinheit, 0.124 mmol) Dibenzoylperoxid wurden in 12 ml Acetonitril bei RT vorgelegt und mit 1.47 g (8.3 mmol) 5-Amino-1,3-dimethyl-1,3-dihydro-2H-benzimidazol-2-on (hergestellt wie unter Beispiel 1A beschrieben, aber ohne Behandlung mit Chlorwasserstoff) und 1.48 ml (12.44 mmol) *tert.*-Butylnitrit versetzt. Die Reaktionsmischung wurde über Nacht bei RT gerührt. Das Lösungsmittel wurde am Rotationsverdampfer entfernt. Der Rückstand wurde in wenig Dichlormethan gelöst, die Lösung mit Diatomeenerde versetzt und am Rotationsverdampfer wieder zur Trockne eingeengt. Der Rückstand wurde über eine Kieselgelkartusche gereinigt (Eluens: Cyclohexan / Essigsäureethylester 2:1 bis 1:1). Die produkthaltigen Fraktionen wurden am Rotationsverdampfer eingeengt. Der Rückstand wurde mit 10 ml Pentan verrührt, der ausgefallene Feststoff abgesaugt, mit Pentan nachgewaschen und im HV getrocknet. Man erhielt 860 mg der Titelverbindung (94 %-ige Reinheit). Durch erneute Kieselgelchromatographie der Mutterlauge konnten zusätzliche 230 mg der Titelverbindung erhalten werden (Gesamtausbeute 43% d. Th.).
LC-MS (Methode 1): Rₜ = 0.95 min; m/z = 289 (M+H)⁺.
¹H-NMR (400MHz, DMSO-d₆): δ [ppm]= 1.30 (s, 12H), 3.33 (s, teilweise unter dem Wassersignal), 3.35 (s, 3H), 7.16 (d, 1H), 7.35 (s, 1H), 7.44 (d, 1H).

### Beispiel 4A

### 1-(2-Chlor-3,6-difluorbenzyl)harnstoff

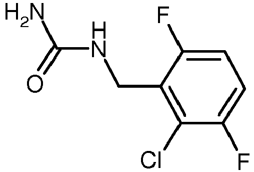

1.50 g (8.44 mmol) 2-Chlor-3,6-difluorbenzylamin und 2.03 g (33.8 mmol) Harnstoff wurden in 4 ml Wasser vorgelegt. Nach Zugabe von 90 µl (ca. 1 mmol) konz. Salzsäure wurde die Reaktionsmischung 3.5 h zum Rückfluss erhitzt. Nach Abkühlung auf RT wurden 100 ml Wasser zugegeben und die Mischung 30 min verrührt. Die ausgefallenen Kristalle wurden abfiltriert, zweimal mit wenig Wasser, dann mit wenig MTBE gewaschen und im HV getrocknet. Man erhielt 1.16 g (62 % d. Th.) der Titelverbindung.
LC-MS (Methode 1): Rₜ = 0.79 min; m/z = 221 (M+H)⁺.
¹H-NMR (400MHz, DMSO-d₆): δ [ppm] = 4.34 (dd, 2H), 5.51 (s, 2H), 6.36 (t, 1H), 7.26 - 7.34 (m, 1H), 7.39 - 7.48 (m, 1H).

### Beispiel 5A

### Ethyl-3-(2-chlor-3,6-difluorbenzyl)-2,4-dioxo-1,2,3,4-tetrahydropyrimidin-5-carboxylat

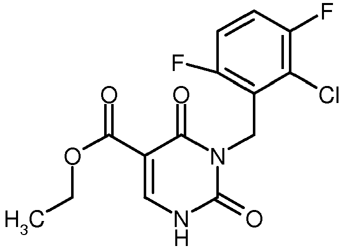

Eine Suspension von 1.16 g (5.25 mmol) 1-(2-Chlor-3,6-difluorbenzyl)harnstoff aus Beispiel 4A und 1.59 mL (7.86 mmol) Diethylethoxymethylenmalonat in 2 ml Ethanol auf 140°C (Badtemperatur) erhitzt und über Nacht bei dieser Temperatur gerührt. Die auf RT abgekühlte Reaktionsmischung wurde in ca. 6 ml Ethanol gelöst, mit 535 mg (7.9 mmol) Natriumethylat versetzt und erneut zum Rückfluss erhitzt. Nach 2 Tagen wurde zusätzlich 0.5 Äquivalent Base hinzugefügt und die Mischung weitere 3 Tage auf Rückflusstemperatur erhitzt. Nach Abkühlung auf RT wurde das Gemisch mit IM Salzsäure angesäuert und zweimal mit Essigsäureethylester extrahiert. Die vereinigten organischen Phasen wurden mit einer gesättigten Natriumchloridlösung gewaschen, über Magnesiumsulfat getrocknet und am Rotationsverdampfer eingeengt. Der Rückstand wurde mit Essigsäureethylester / MTBE 1:1 verrührt. Der Feststoff wurde abfiltriert, mit MTBE gewaschen und im HV getrocknet. Man erhielt 851 mg (45% d. Th.) der Titelverbindung.
LC-MS (Methode 1): Rₜ = 0.79 min; m/z = 345 (M+H)⁺.
¹H-NMR (400MHz, DMSO-d₆): δ [ppm] = 1.22 (t, 3H), 4.16 (q, 2H), 5.13 (s, 2H), 7.20 - 7.29 (m, 1H), 7.38 - 7.46 (m, 1H), 8.20 (s, 1H), 11.94 - 12.05 (m, 1H).

### Beispiel 6A

### 1-(3-Chlor-2-methylbenzyl)harnstoff

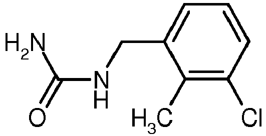

Darstellung und Reinigung der Titelverbindung erfolgten analog zum Beispiel 4A bei einer Reaktionsdauer von 6h. Ausgehend von 2.00 g (12.85 mmol) 3-Chlor-2-methylbenzylamin und 3.08 g (51.40 mmol) Harnstoff erhielt man 2.36 g (92 % d. Th.) der Titelverbindung.
LC-MS (Methode 1): Rₜ = 0.72 min; m/z = 199 (M+H)⁺.
¹H-NMR (400MHz, DMSO-d₆): δ [ppm] = 2.29 (s, 3H), 4.19 (d, 2H), 5.53 (s, 2H), 6.36 (t, 1H), 7.14 - 7.22 (m, 2H), 7.28 - 7.35 (m, 1H).

### Beispiel 7A

### Ethyl-3-(3-chlor-2-methylbenzyl)-2,4-dioxo-1,2,3,4-tetrahydropyrimidin-5-carboxylat

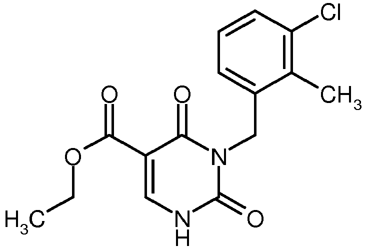

Eine Suspension von 2.36 g (11.88 mmol) 1-(3-Chlor-2-methylbenzyl)harnstoff aus Beispiel 6A und 3.60 mL (17.82 mmol) Diethylethoxymethylenmalonat in 3 ml Ethanol wurde auf 140 °C (Badtemperatur) erhitzt und die nach ca. 3h entstandene Lösung über Nacht bei dieser Temperatur weiter gerührt. Das auf RT abgekühlte Gemisch wurde in 20 ml Ethanol gelöst, mit 1.21 g (17.8 mmol) Natriumethylat versetzt und erneut 1.5 h zum Rückfluss erhitzt. Nach Abkühlung auf RT wurde die Reaktionsmischung in 100 ml eisgekühlte 0.5M Salzsäure getropft. Der ausgefallene Feststoff wurde abfiltriert, mit MTBE gewaschen und im HV getrocknet. Man erhielt 2.20 g (57 % d. Th.) der Titelverbindung.
LC-MS (Methode 1): Rₜ = 0.90 min; m/z = 323 (M+H)⁺.
¹H-NMR (400MHz, DMSO-d₆): δ [ppm] = 1.24 (t, 3H), 2.40 (s, 3H), 4.17 (q, 2H), 4.96 (s, 2H), 6.85 (d, 1H), 7.13 (t, 1H), 7.33 (d, 1H), 8.25 (s, 1H), 12.06 (br. s, 1H).

### Beispiel 8A

### 1-[2,3-Bis(trifluormethyl)phenyl]methanamin

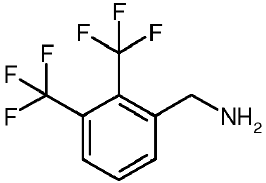

Unter Argon wurden 69.38 mL (69.38 mmol) Boran-THF-Komplex (1.0M) vorgelegt und das Reaktionsgemisch auf 0°C abgekühlt. Anschließend wurde eine Lösung von 5.53 g (23.13 mmol) 2,3-Bis(trifluormethyl)benzonitril (Darstellung siehe: Zhurnal Organicheskoi Khimii 1973, 9(5), 1019-1024, 1046-1050) in 50 mL THF zugetropft und 3 h zum Rückfluss gerührt. Die Reaktionsmischung wurde auf 0°C abgekühlt, mit IN Salzsäure angesäuert und im Vakuum eingeengt. Der Rückstand wurde mit Wasser verdünnt und die wässrige Phase dreimal mit Dichlormethan gewaschen. Anschließend wurde mit IN Natronlauge auf pH 14 gestellt, dreimal mit Dichlormethan extrahiert und die vereinigten organischen Phasen über Natriumsulfat getrocknet, filtriert und eingeengt. Man erhielt 4.07 g (70 % d. Th.) der Titelverbindung.
LC-MS (Methode 1): Rt = 0.49 min; MS (ESIpos): m/z = 244 (M+H)⁺.
¹H-NMR (400 MHz, DMSO-d₆): δ [ppm] = 1.99 (br.s, 2H), 3.90 - 3.97 (m, 2H), 7.83 - 7.92 (m, 2H), 8.17 - 8.23 (m, 1H).

### Beispiel 9A

### 1-[2,3-Bis(trifluormethyl)benzyl]harnstoff

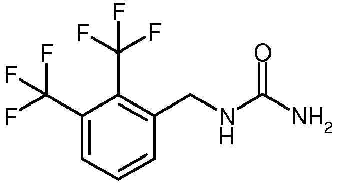

In 1.3 mL Wasser wurden 780 mg (3.21 mmol) 1-[2,3-Bis(trifluormethyl)phenyl]methanamin aus Beispiel 8A sowie 771 mg (12.83 mmol) Harnstoff vorgelegt, mit 34 µL (0.41 mmol) konz. Salzsäure tropfenweise versetzt und die Mischung 3 h zum Rückfluss erhitzt. Anschließend wurde bei RT mit Wasser (100 mL) verdünnt und 30 min gerührt. Der gebildete Feststoff wurde abfiltriert, je zweimal mit Wasser sowie Diethylether gewaschen und im Hochvakuum getrocknet. Man erhielt 541 mg (59 % d.Th.) der Zielverbindung.
LC-MS (Methode 1): Rₜ = 0.85 min; MS (ESIpos): m/z = 287 (M+H)⁺.
¹H-NMR (400 MHz, DMSO-d₆): δ [ppm] = 4.40 - 4.45 (m, 2H), 5.72 (s, 2H), 6.57 - 6.63 (m, 1H), 7.86 - 7.90 (m, 2H), 7.91 - 7.95 (m, 1H).

### Beispiel 10A

### Ethyl-3-[2,3-bis(trifluormethyl)benzyl]-2,4-dioxo-1,2,3,4-tetrahydropyrimidin-5-carboxylat

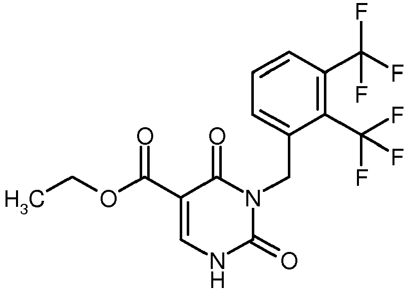

Eine Mischung aus 2.01 g (7.04 mmol) 1-[2,3-Bis(trifluormethyl)benzyl]harnstoff aus Beispiel 9A und 2.13 mL (10.60 mmol) Diethyl(ethoxymethylen)malonat wurde bei 140°C über 4 Tage im Argongegenstrom gerührt. Die Reaktionsmischung wurde anschließend mit Ethanol (20 mL) verdünnt, danach mit 0.72 g (10.60 mmol) Natriumethylat versetzt und weitere 2.5 h zum Rückfluss erhitzt. Die auf RT gebrachte Mischung wurde in eisgekühlte Salzsäure (400 mL, 0.5M) getropft und der entstandene Feststoff abfiltriert. Der Filterrückstand wurde mit MTBE verrührt, abfiltriert und im Hochvakuum getrocknet. Man erhielt 1.92 g (67% d. Th.) der Zielverbindung.
LC-MS (Methode 1): Rₜ = 0.99 min; MS (ESIpos): m/z = 411 (M+H)⁺.
¹H-NMR (400 MHz, DMSO-d₆): δ [ppm] = 1.24 (t, 3H), 4.18 (q, 2H), 5.17 (br.s, 2H), 7.52 (d, 1H), 7.76 - 7.83 (m, 1H), 7.92 - 7.98 (m, 1H), 8.29 (s, 1H), 12.15 (br.s, 1H).

### Beispiel 11A

### N-Benzyl-4-(trifluormethyl)indan-1-amin (Racemat)

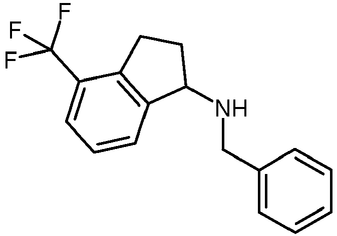

Zu einer Mischung aus 15.40 g (0.075 mol) 4-(Trifluormethyl)-1-indanon sowie 9.78 mL (0.090 mol) Benzylamin in 462 mL Dichlormethan wurden 33.0 mL (0.112 mol) Titan(IV)isopropylat addiert und die Mischung 1 h bei RT gerührt. Anschließend wurden bei 0°C portionsweise 5.65 g (0.149 mol) Natriumborhydrid zugefügt und die Mischung über Nacht bei RT gerührt. Zur Aufarbeitung wurde anschließend unter starker Gasentwicklung Wasser zugetropft. Danach wurde die Mischung mit Wasser sowie Dichlormethan (je 500 mL) weiter verdünnt, die organische Phase über Natriumsulfat getrocknet, filtriert und das Filtrat eingeengt. Das so erhaltene Rohprodukt wurde an Kieselgel chromatographiert (Petrolether/Essigsäureethylester, 10:1). Man erhielt 12.80 g (58 % d.Th.) der Zielverbindung.
LC-MS (Methode 1): Rₜ = 0.80 min; MS (ESIpos): m/z = 292 (M+H)⁺.
¹H-NMR (400 MHz, DMSO-d₆): δ [ppm] = 1.81 - 1.93 (m, 1H), 2.31 - 2.42 (m, 1H), 2.57 - 2.65 (m, 1H), 2.81 - 2.93 (m, 1H), 3.04 - 3.15 (m, 1H), 3.72 - 3.85 (m, 2H), 4.14 - 4.22 (m, 1H), 7.19 - 7.25 (m, 1H), 7.32 (t, 2H), 7.37 - 7.44 (m, 3H), 7.53 (d, 1H), 7.68 (d, 1H).

### Beispiel 12A

### 4-(Trifluormethyl)indan-1-amin (Racemat)

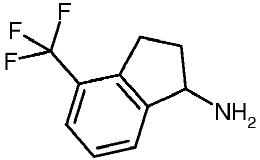

In 230 mL THF wurden 9.70 g (0.032 mol) N-Benzyl-4-(trifluormethyl)indan-1-amin aus Beispiel 11A vorgelegt, anschließend mit 5.00 g Palladium (10 %-ig auf Aktivkohle) versetzt und die Mischung bei RT unter Wasserstoff-Normaldruck über Nacht hydriert. Anschließend wurde die Mischung über Kieselgur filtriert und das Filtrat eingeengt. Man erhielt 6.40 g (98 % d. Th.) Rohprodukt, die ohne weitere Reinigung umgesetzt wurden. LC-MS (Methode 1): Rₜ = 0.56 min; MS (ESIpos): m/z = 202 (M+H)⁺.

### Beispiel 13A

### 1-[4-(Trifluormethyl)-2,3-dihydro-1H-inden-1-yl]harnstoff (Racemat)

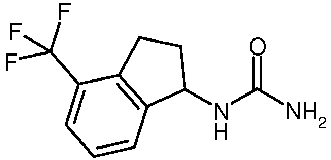

In 25 mL Wasser wurden 6.40 g (0.03 mol) 4-(Trifluormethyl)indan-1-amin aus Beispiel 12A sowie 9.55 g (0.159 mol) Harnstoff vorgelegt, mit 0.34 mL (0.004 mol) konz. Salzsäure tropfenweise versetzt und 3 h zum Rückfluss erhitzt. Die Mischung wurde bei RT mit Wasser (100 mL) verdünnt und 30 min gerührt. Der gebildete Feststoff wurde abfiltriert, mit Wasser gewaschen und im Hochvakuum getrocknet. Das Rohprodukt wurde durch Verrühren mit Diethylether (50 mL) umkristallisiert. Man erhielt 4.60 g (59 % d.Th.) der Zielverbindung.
LC-MS (Methode 1): Rₜ = 0.83 min; MS (ESIpos): m/z = 245 (M+H)⁺.
¹H-NMR (400 MHz, DMSO-d₆): δ [ppm] = 1.71 - 1.82 (m, 1H), 2.39 - 2.49 (m, 1H), 2.84 - 2.96 (m, 1H), 3.00-3.11 (m, 1H), 5.12 (q, 1H), 5.53 (s, 2H), 6.42 (d, 1H), 7.39 - 7.45 (m, 1H), 7.53 (dd, 2H).

### Beispiel 14A

### (S)-4-Trifluormethyl-indan-1-ol

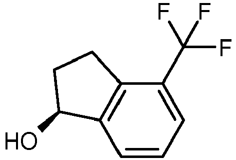

Eine Lösung von 55.7 g (278.3 mmol) 4-Trifluorniethyl-1-indanon, 194 ml (1.391 mol) Triethylamin und 1.60 g (2.50 mmol) RuCl(p-cymene)[(S,S)-TsDPEN] (CAS-Nr: 192139-90-5; IUPAC-Name: (S,S)-N-(p-Toluolsulfonyl)-1,2-diphenylethandiamin(chlor)[1-methyl-4-(propan-2-yl)benzol]-ruthenium(II)) in 258 ml Dichlormethan wurde unter Argon auf 35°C erwärmt und bei dieser Temperatur langsam mit 52.5 ml (1.391 mol) Ameisensäure versetzt (Zugabezeit ca. 40 min). Die Temperatur der Reaktionsmischung stieg dabei auf 42°C. Nach vollständiger Zugabe wurde die Mischung weitere 2 h bei 38°C gerührt. Alle flüchtigen Bestandteile wurden am Rotationsverdampfer und im HV entfernt. Anschließend wurde der Rückstand in wenig Dichlormethan gelöst und über 1 kg Kieselgel gereinigt (Eluens: zuerst 3 Liter Cyclohexan / Essigsäureethylester 5:1 dann 6 Liter Cyclohexan / Essigsäureethylester 1:1). Die geeigneten Fraktionen wurden am Rotationsverdampfer eingeengt und das Produkt im HV getrocknet. Man erhielt 51.2 g (90 % d. Th.) der Titelverbindung.
¹H-NMR (400MHz, DMSO-d₆): δ [ppm]= 1.76 - 1.91 (m, 1H), 2.40 (ddt, 1H), 2.86 (dt, 1H), 3.01 - 3.13 (m, 1H), 5.09 (q, 1H), 5.45 (d, 1H), 7.38 - 7.48 (m, 1H), 7.55 (d, 1H), 7.62 (d, 1H).
Chirale analytische HPLC (Methode 25): Rₜ = 7.49 min; 99 % ee

### Beispiel 15A

### (R)-4-Trifluormethyl-indan-1-ol

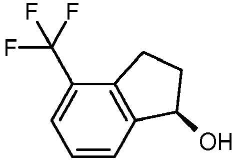

Analog zu Beispiel 14A wurden 5 g (25.0 mmol) 4-Trifluormethyl-1-indanon in Gegenwart von 143 mg (0.225 mmol) RuCl(p-cymene)[(R,R)-TsDPEN] (CAS-Nr: 192139-92-7; IUPAC-Name: (R,R)-N-(p-Toluolsulfonyl)-1,2-diphenylethandiamin(chlor)[1-methyl-4-(propan-2-yl)benzol]-ruthenium(II)) reduziert. Man erhielt 4.60 g (91 % d. Th.) der Titelverbindung.
GC-MS (Methode 6): Rₜ = 3.43 min; MS (CI-pos): m/z = 202 (M)⁺.
¹H-NMR (400MHz, CDCl₃): δ [ppm] = 1.94 (br d, 1H), 1.96 - 2.05 (m, 1H), 2.55 (dddd, 1H), 2.91 - 3.04 (m, 1H), 3.19 - 3.30 (m, 1H), 5.27 (q, 1H), 7.32 - 7.38 (m, 1H), 7.53 (d, 1H), 7.60 (d, 1H).
Chirale analytische HPLC (Methode 25): Rₜ = 6.51 min; ee ca. 96 %.

### Beispiel 16A

### 5-Amino-1-methyl-1,3-dihydro-2H-benzimidazol-2-on

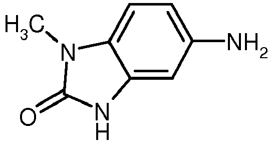

In 78.0 mL einer THF/Methanol-Mischung (1:2) wurden 2.43 g (12.6 mmol) 1-Methyl-5-nitro-1,3-dihydro-2H-benzimidazol-2-on [Synthese beschrieben in US 6,114,532] vorgelegt, danach 134 mg (0.13 mmol) Palladium (10%-ig auf Aktivkohle) zugefügt und die Mischung über Nacht bei Wasserstoff-Normaldruck hydriert. Anschließend wurde die Reaktionsmischung über Kieselgur filtriert, der Rückstand mit THF nachgewaschen und das Filtrat eingeengt. Man erhielt 1.89 g (92% d. Th.) der Zielverbindung.
¹H-NMR (400 MHz, DMSO-d₆): δ [ppm] = 3.16 (s, 3H), 4.66 - 4.71 (m, 2H), 6.25 (dd, 1H), 6.28 (d, 1H), 6.71 (d, 1H), 10.39 (s, 1H).

### Beispiel 17A

### 5-Fluor-1,3-dimethyl-1,3-dihydro-2H-benzimidazol-2-on

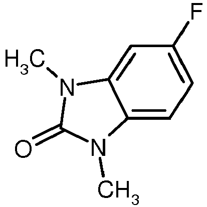

Unter Argon wurden 5.0 mL DMF bei 0°C vorgelegt und mit 318 mg (7.96 mmol) Natriumhydrid (60 %ige Suspension in Mineralöl) versetzt. Anschließend wurde eine Lösung von 881 mg (5.30 mmol) 5-Fluor-1-methyl-1,3-dihydro-2H-benzimidazol-2-on [Synthese beschrieben in US 2010/0305102, Seite 28, Beispiel 26.3] in 5.0 mL DMF zugetropft und die Reaktionsmischung 30 min gerührt. Danach wurden 0.43 mL (6.90 mmol) Iodmethan zugetropft und die Mischung über Nacht bei RT gerührt. Anschließend wurde erneut bei 0°C Natriumhydrid (1.0 eq) zugefügt, weitere 15 min gerührt und schließlich Iodmethan (1.0 eq) zugetropft. Die Reaktionsmischung wurde 2 h bei RT gerührt, anschließend mit Wasser (100 mL) versetzt und mit Essigsäureethylester extrahiert (3x 50 mL). Die vereinigten organischen Phasen wurden mit Wasser sowie gesättigter Natriumchloridlösung gewaschen, über Magnesiumsulfat getrocknet, filtriert und das Filtrat eingeengt. Das so erhaltene Rohprodukt wurde mittels Flash-Kieselgelchromatographie (Cyclohexan/Essigsäureethylester, Gradient 7:1 - 4:1) gereinigt. Man erhielt 672 mg (69% d. Th.) der Zielverbindung.
LC-MS (Methode 1): Rₜ = 0.69 min; MS (ESIpos): m/z = 181 (M+H)⁺.
¹H-NMR (400 MHz, DMSO-d₆): δ [ppm]= 3.33 (s, 3H), 3.3 (s, durch Wassersignal verdeckt), 6.85 - 6.93 (m, 1H), 7.09 - 7.18 (m, 2H).

### Beispiel 18A

### 5-Fluor-1,3-dimethyl-6-nitro-1,3-dihydro-2H-benzimidazol-2-on

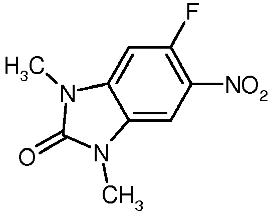

In 3.5 mL THF wurden unter Argon 670 mg (3.72 mmol) 5-Fluor-1,3-dimethyl-1,3-dihydro-2H-benzimidazol-2-on aus Beispiel 17A bei 0°C vorgelegt. Danach wurden 0.24 mL (3.72 mmol) Salpetersäure (65 %ig) zugetropft und die Mischung für 1 h bei 0°C gerührt. Anschließend wurde die Reaktionsmischung auf Eiswasser (50 mL) gegeben, der gebildete Feststoff abfiltriert, mit Wasser (20 mL) gewaschen und anschließend bei 40°C im Hochvakuum getrocknet. Man erhielt 807 mg (92 % d. Th.) der Zielverbindung, die ohne weitere Reinigung umgesetzt wurde.
LC-MS (Methode 1): Rₜ = 0.70 min; MS (ESIpos): m/z = 226 (M+H)⁺.
¹H-NMR (400 MHz, DMSO-d₆): δ [ppm]= 3.38 (s, 3H), 3.40 (s, 3H), 7.52 (d, 1H), 7.99 (d, 1H).

### Beispiel 19A

### 5-Amino-6-fluor-1,3-dimethyl-1,3-dihydro-2H-benzimidazol-2-on

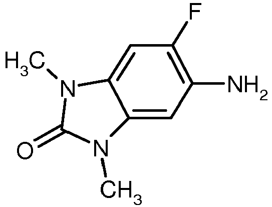

In 22.2 mL einer THF/Methanol-Mischung (1:2) wurden 806 mg (3.58 mmol) 5-Fluor-1,3-dimethyl-6-nitro-1,3-dihydro-2H-benzimidazol-2-on aus Beispiel 18A vorgelegt, danach 38 mg (0.04 mmol) Palladium (10%-ig auf Aktivkohle) zugefügt und die Mischung über Nacht bei Wasserstoff-Normaldruck hydriert. Anschließend wurde die Reaktionsmischung über Kieselgur filtriert, der Rückstand mit Methanol nachgewaschen, das Filtrat eingeengt und im Hochvakuum getrocknet. Man erhielt 668 mg (85 %-ige Reinheit, 81% d. Th.) der Zielverbindung, die ohne weitere Reinigung umgesetzt wurde.
LC-MS (Methode 1): Rₜ = 0.43 min; MS (ESIpos): m/z = 196 (M+H)⁺.
¹H-NMR (400 MHz, DMSO-d₆): δ [ppm]= 3.21 (s, 3H), 3.22 (s, 3H), 4.78 (br.s, 2H), 6.53 (d, 1H), 6.98 (d, 1H).

### Beispiel 20A

### 5-Amino-3-hydroxy-1-methyl-3-(trifluormethyl)-1,3-dihydro-2H-indol-2-on

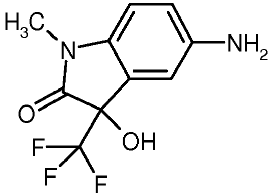

In 20.0 mL Ethanol wurden 2.45 g (8.87 mmol) 3-Hydroxy-1-methyl-5-nitro-3-(trifluormethyl)-1,3-dihydro-2H-indol-2-on [Darstellung siehe: Journal of Heterocyclic Chemistry, 2008, 45, 4, p. 969-973] vorgelegt, danach 600 mg Palladium (10 %-ig auf Aktivkohle) zugefügt und die Mischung 4 h bei Wasserstoff-Normaldruck hydriert. Anschließend wurde die Reaktionsmischung über Kieselgur filtriert, der Rückstand mit Methanol (30 mL) gewaschen und das Filtrat eingeengt. Man erhielt 2.06 g (91 % d. Th.) der Zielverbindung.
LC-MS (Methode 2): Rₜ = 0.97 min; MS (ESIpos): m/z = 247 (M+H)⁺.
¹H-NMR (400 MHz, DMSO-d₆): δ [ppm] = 3.07 (s, 3H), 4.97 - 5.33 (m, 2H), 6.64 (dd, 1H), 6.77 - 6.81 (m, 2H), 7.51 (s, 1H).

### Beispiel 21A

### Ethyl-2,4-dioxo-3-[4-(trifluormethyl)-2,3-dihydro-1H-inden-1-yl]-1,2,3,4-tetrahydropyrimidin-5-carboxylat (Racemat)

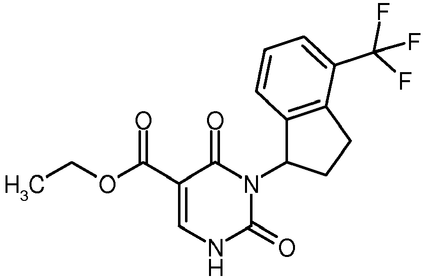

Eine Mischung aus 5.2 g (20 mmol) 1-[4-(Trifluormethyl)-2,3-dihydro-1H-inden-1-yl]harnstoff aus Beispiel 13A und 8.26 mL (41 mmol) Diethyl(ethoxymethylen)malonat wurde bei 140°C für 24 h zum Rückfluss erhitzt (am Anfang kaum rührbar, dann homogen und rührbar). Nach Abkühlen auf RT wurden 47.7 mL Ethanol sowie 2.78 g (41 mmol) Natriumethylat zugefügt und die Mischung weitere 24 h zum Rückfluss erhitzt. Zur Aufarbeitung wurde die Reaktionsmischung im Vakuum eingeengt, mit IM Salzsäure (80 mL) angesäuert und dreimal mit je 80 mL Essigsäureethylester extrahiert. Die veeinigten organischen Phasen wurden über Natriumsulfat getrocknet, filtriert und das Filtrat eingeengt. Der Rückstand wurde an Kieselgel chromatographiert (Petrolether/Essigsäureethylester 3:1 bis 1:3). Man erhielt 4.20 g (56% d. Th.) der Titelverbindung.
LC-MS (Methode 1): Rₜ = 0.94 min; MS (ESIpos): m/z = 369 (M+H)⁺.
¹H-NMR (400 MHz, DMSO-d₆): δ [ppm] = 1.23 (t, 3H), 2.27 - 2.38 (m, 1H), 2.39 - 2.49 (m, 1H), 3.01 - 3.13 (m, 1H), 3.23 - 3.32 (m, 1H), 4.10 - 4.22 (m, 2H), 6.29 - 6.46 (m, 1H), 7.29 - 7.39 (m, 2H), 7.52 (d, 1H), 8.13 - 8.20 (m, 1H), 11.74 - 11.99 (m, 1H).

### Beispiel 22A

### Ethyl-1-(1,3-diethyl-2-oxo-2,3-dihydro-H-benzimidazol-5-yl)-2,4-dioxo-1,2,3,4-tetrahydro-pyrimidin-5-carboxylat

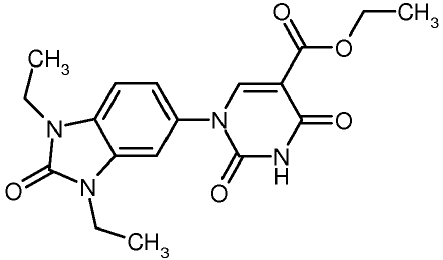

In 31 mL Ethanol wurden 1.00 g (4.13 mmol) 5-Amino-1,3-diethyl-1,3-dihydro-2H-benzimidazol-2-on-Hydrochlorid, 0.63 mL (4.55 mmol) Triethylamin sowie 1.07 g (4.13 mmol) Ethyl-3-ethoxy-2-[(ethoxycarbonyl)carbamoyl]acrylat) (Darstellung siehe: Senda, Shigeo; Hirota, Kosaku; Notani, Jiyoji, Chemical & Pharmaceutical Bulletin (1972), 20(7), 1380-8) vorgelegt und die Mischung 2 h zum Rückfluss erhitzt. Anschließend wurden bei RT 464 mg (4.13 mmol) Kalium-tert.-butylat zugegeben und die Reaktionsmischung über Nacht bei RT gerührt. Danach wurde das Reaktionsgemisch weitere 3 h zum Rückfluss erhitzt. Zur Aufarbeitung wurde bei RT mit Wasser versetzt und mit IN Salzsäure angesäuert. Der ausgefallene Feststoff wurde abgesaugt, je einmal mit Wasser sowie Essigsäureethylester gewaschen und im Vakuum bei 50°C getrocknet. Man erhielt 783 mg (51% d. Th.) der Zielverbindung.
LC-MS (Methode 3): Rₜ = 0.84 min; MS (ESIpos): m/z = 373 (M+H)⁺.
¹H-NMR (400 MHz, DMSO-d₆): δ [ppm] = 1.18 - 1.26 (m, 9H), 3.83 - 3.95 (m, 4H), 4.17 (q, 2H), 7.18 (dd, 1H), 7.31 (d, 1H), 7.44 (d, 1H), 8.30 (s, 1H), 11.68 (s, 1H).

### Beispiel 23A

### 1-Methyl-5-nitro-3-(2,2,2-trifluorethyl)-1,3-dihydro-2H-benzimidazol-2-on

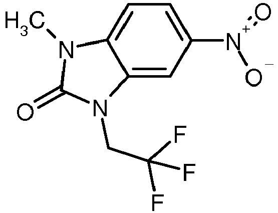

8.00 g (41.4 mmol) 1-Methyl-5-nitro-1,3-dihydro-2H-benzimidazol-2-on [Synthese beschrieben in US 6,114,532] wurden mit 11.45 g (82.8 mmol) Kaliumcarbonat in 600 ml Acetonitril / DMF 2:1 (v/v) vorgelegt und mit 7.48 ml (45.6 mmol) 2,2,2-Trifluorethyl-Trichlormethansulfonat versetzt. Die Reaktionsmischung wurde auf Rüchflusstemperatur erhitzt und über Nacht bei dieser Temperatur gerührt. Nach Abkühlung auf RT wurde das Gemisch in 1.8 L 0.1N Salzsäure gegossen. Der ausgefallene Feststoff wurde abfiltriert und im HV getrocknet. Man erhielt 11.3 g (97 % d. Th.) der Titelverbindung.
¹H-NMR (400MHz, DMSO-d₆): δ [ppm] = 3.44 (s, 3H), 4.97 (q, 2H), 7.44 (d, 1H), 8.14 (dd, 1H), 8.33 (d, 1H).

### Beispiel 24A

### 5-Amino-1-methyl-3-(2,2,2-trifluorethyl)-1,3-dihydro-2H-benzimidazol-2-on

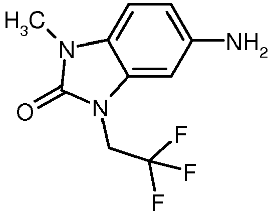

11.3 g (41.06 mmol) der Verbindung aus Beispiel 23A wurden in 623 ml Methanol / Tetrahydrofuran 2:1 (v/v) vorgelegt. 1.66 g Palladium auf Kohle (10 % auf Kohle) und 25.9 g (410.6 mmol) Ammoniumformiat wurden hinzugefügt und das Reaktionsgemisch 4 h bei 70°C verrührt. Nach Abkühlung auf RT wurde der Katalysator abfiltriert und das Filtrat am Rotationsverdampfer von den Lösungsmitteln befreit. Der Rückstand wurde mit 100 ml einer gesättigten Natriumhydrogencarbonatlösung und 400 ml Wasser versetzt. Der gebildete Feststoff wurde abfiltriert, mit 50 ml Wasser gewaschen und in HV getrocknet. Man erhielt 8.90 g (86 %.d. Th.) der Titelverbindung.
LC-MS (Methode 5): Rₜ = 0.41 min; m/z = 246 (M+H)⁺.
¹H-NMR (400MHz, DMSO-d₆): δ [ppm] = 3.25 (s, 3H), 4.63 (q, 2H), 4.89 (br. s, 2H), 6.37 (dd, 1H), 6.48 (s, 1H), 6.85 (d, 1H).

### Beispiel 25A

Ethyl-1-[1-methyl-2-oxo-3-(2,2,2-trifluorethyl)-2,3-dihydro-1H-benzimidazol-5-yl]-2,4-dioxo-1,2,3,4-tetrahydropyrimidin-5-carboxylat

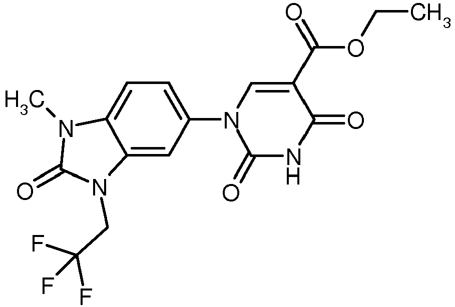

8.90 g (36.3 mmol) der Verbindung aus Beispiel 24A und 9.41 g (36.3 mmol) Ethyl-3-ethoxy-2-[(ethoxycarbonyl)carbamoyl]acrylat (Darstellung siehe: Senda, Shigeo; Hirota, Kosaku; Notani, Jiyoji, Chemical & Pharmaceutical Bulletin (1972), 20(7), 1380-8) wurden in 784 ml Ethanol 1.5 h auf Rückflusstemperatur erhitzt. Nach leichtem Abkühlen (ca. 60°C) wurden 4.07 g (36.3 mmol) Kalium*-tert*.-butylat zugegeben. Die Reaktionsmischung wurde wieder auf Rückflußtemperatur 30 min erhitzt. Nach Abkühlen auf RT wurde das Reaktionsgemisch in 5 L eisgekühlte IN Salzsäure gegossen. Der ausgefallene Feststoff wurde abfiltriert, mit 800 ml Wasser gewaschen und im HV getrocknet. Man erhielt 12.7 g (83 % d. Th.) der Titelverbindung.
LC-MS (Methode 1): Rₜ = 0.70 min; m/z = 413 (M+H)⁺.
¹H-NMR (400MHz, DMSO-d₆): δ [ppm] = 1.22 (t, 3H), 3.40 (s, 3H), 4.17 (q, 2H), 4.78 (q, 2H), 7.25 - 7.30 (m, 1H), 7.31 - 7.36 (m, 1H), 7.52 (s, 1H), 8.26 (s, 1H), 11.71 (s, 1H).

### Beispiel 26A

### Ethyl-2,4-dioxo-1-(2-oxo-2,3-dihydro-1H-benzimidazol-5-yl)-1,2,3,4-tetrahydropyrimidin-5-carboxylat

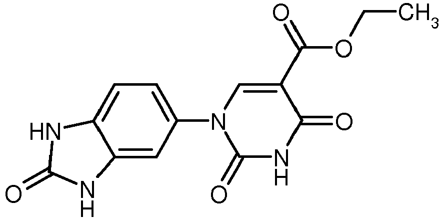

Die Darstellung der Zielverbindung erfolgte analog zum Beispiel 25A unter Verwendung von 1.00 g (6.71 mmol) 5-Amino-1,3-dihydro-2H-benzimidazol-2-on und 1.74 g (6.71 mmol) Ethyl-3-ethoxy-2-[(ethoxycarbonyl)carbamoyl]acrylat. Man erhielt 1.60 g (75 % d. Th.) der Zielverbindung.
LC-MS (Methode 1): Rₜ = 0.46 min; MS (ESIpos): m/z = 317 (M+H)⁺.
¹H-NMR (400 MHz, DMSO-d₆): δ [ppm] = 1.22 (t, 3H), 4.16 (q, 2H), 6.97 - 7.04 (m, 2H), 7.07 - 7.10 (m, 1H), 8.23 (s, 1H), 10.84 - 10.90 (m, 2H), 11.61 (s, 1H).

### Beispiel 27A

### Ethyl-1-(6-methyl-2-oxo-2,3-dihydro-1H-benzimidazol-5-yl)-2,4-dioxo-1,2,3,4-tetrahydropyrimidin-5-carboxylat

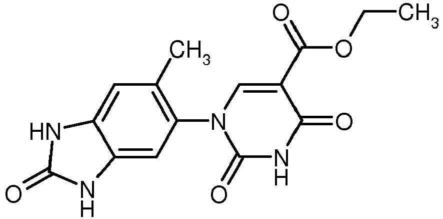

1.59 g (6.13 mmol) Ethyl-3-ethoxy-2-[(ethoxycarbonyl)carbamoyl]acrylat (Darstellung siehe: Senda, Shigeo; Hirota, Kosaku; Notani, Jiyoji, Chemical & Pharmaceutical Bulletin (1972), 20(7), 1380-8) sowie 1.00 g (6.13 mmol) 5-Amino-6-methyl-1,3-dihydro-2H-benzimidazol-2-on wurden in 46 mL Ethanol für 2 h zum Rückfluss erhitzt. Danach wurden bei RT 0.69 g (6.13 mmol) Kalium-tert.-butylat zugefügt und die Reaktionsmischung über Nacht bei RT sowie 1h zum Rückfluss gerührt. Zur Aufarbeitung wurde das Reaktionsgemisch mit Wasser versetzt und mit IN Salzsäure angesäuert. Der entstandene Feststoff wurde abfiltriert, mit Wasser sowie Essigsäureethylester gewaschen und anschließend im Vakuum bei 50°C getrocknet. Man erhielt 1.46 g (72 % d. Th.) der Zielverbindung.
LC-MS (Methode 1): Rₜ = 0.52 min; MS (ESIpos): m/z = 331 (M+H)⁺.
¹H-NMR (400 MHz, DMSO-d₆): δ [ppm] = 1.22 (t, 3H), 2.08 (s, 3H), 4.16 (q, 2H), 6.89 (s, 1H), 7.03 (s, 1H), 8.19 (s, 1H), 10.77 (s, 1H), 10.78 (s, 1H), 11.65 (s, 1H).

### Beispiel 28A

### Ethyl-1-(3-methyl-2-oxo-2,3-dihydro-1,3-benzoxazol-6-yl)-2,4-dioxo-1,2,3,4-tetrahydropyrimidin-5-carboxylat

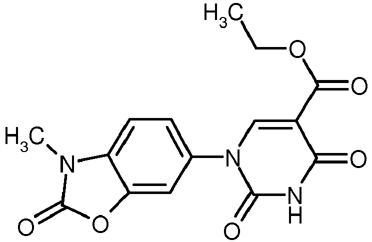

40.0 g (243.7 mmol) 6-Amino-3-methyl-1,3-benzoxazol-2(3H)-on wurden in 2.5 L Ethanol vorgelegt und mit 63.2 g (243.7 mmol) Ethyl-3-ethoxy-2-[(ethoxycarbonyl)carbamoyl]acrylat (Darstellung siehe: Senda, Shigeo; Hirota, Kosaku; Notani, Jiyoji, Chemical & Pharmaceutical Bulletin (1972), 20(7), 1380-8) versetzt. Nach einigen Minuten bildete sich eine dicke Suspension. Diese Mischung wurde 1.5 h auf Rückflusstemperatur erhitzt. Nach leichtem Abkühlen (ca. 60°C) wurden 27.3 g (243.7 mmol) Kalium-*tert.*-butylat zugegeben und das Reaktionsgemisch 4.5 h bei Rückflusstemperatur weiter gerührt. Zur Aufarbeitung wurde die Reaktionssuspension leicht abgekühlt (ca. 60°C) dann in 10 Liter kalte IN Salzsäure eingerührt. Der Feststoff wurde abgesaugt, mit Wasser gewaschen und im Vakuum-Trockenschrank bei 70°C über Nacht getrocknet. Man erhielt 64.0 g (79 % d. Th.) der Ttelverbindung.
LC-MS (Methode 1: Rₜ = 0.59 min; MS (ESIpos): m/z = 332 (M+H)⁺.
¹H-NMR (400MHz, DMSO-d₆): δ [ppm] = 1.22 (t, 3H), 3.38 (s, 3H), 4.17 (q, 2H), 7.38 (s, 2H), 7.59 (s, 1H), 8.26 (s, 1H), 11.69 (s, 1H).

### Beispiel 29A

### 6-Amino-3-ethyl-1,3-benzoxazol-2(3H)-on

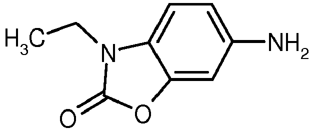

In 32.5 mL Ethanol wurden 1.00 g (4.80 mmol) 3-Ethyl-6-nitro-1,3-benzoxazol-2(3H)-on [Darstellung siehe: WO 2007/120339 A1, 37-38] vorgelegt, danach 51 mg (0.05 mmol) Palladium (10 %-ig auf Aktivkohle) zugefügt und die Mischung über Nacht bei Wasserstoff-Normaldruck hydriert. Anschließend wurde die Reaktionsmischung über Kieselgur filtriert und das Filtrat eingeengt. Der Rückstand wurde in 50.0 mL einer Ethanol/THF-Mischung (1:1) aufgenommen, mit 50 mg (0.05 mmol) Palladium (10 %-ig auf Aktivkohle) versetzt und die Mischung weiter über Nacht bei Wasserstoff-Normaldruck hydriert. Die Reaktionsmischung wurde erneut über Kieselgur filtriert, der Filterkuchen mit Ethanol nachgewaschen und das Filtrat eingeengt. Der Rückstand wurde in Ethanol ausgerührt, der Feststoff abfiltriert und mit Ethanol nachgewaschen. Man erhielt nach Trocknung im Hochvakuum 747 mg der Zielverbindung (83 % d. Th.).
LC-MS (Methode 3): Rt = 0.29 min; MS (ESIpos): m/z = 179 (M+H)⁺.
¹H-NMR (400 MHz, DMSO-d₆): δ [ppm] = 1.21 (t, 3H), 3.74 (q, 2H), 4.99 - 5.05 (m, 2H), 6.42 (dd, 1H), 6.55 (d, 1H), 6.94 (d, 1H).

### Beispiel 30A

### Ethyl-1-(3-ethyl-2-oxo-2,3-dihydro-1,3-benzoxazol-6-yl)-2,4-dioxo-1,2,3,4-tetrahydropyrimidin-5-carboxylat

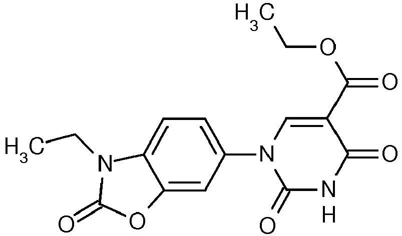

746 mg (4.19 mmol) 6-Amino-3-ethyl-1,3-benzoxazol-2(3H)-on aus Beispiel 29A sowie 1.09 g (4.19 mmol) Ethyl-3-ethoxy-2-[(ethoxycarbonyl)carbamoyl]acrylat wurden in 32 mL Ethanol vorgelegt und die Mischung für 2 h zum Rückfluss erhitzt. Nach Abkühlen auf RT wurden 470 mg (4.19 mmol) Kalium-tert.-butylat zugegeben und das Reaktionsgemisch weiter über Nacht bei RT gerührt. Anschließend wurde die Mischung für 1 h zum Rückfluss erhitzt. Zur Aufarbeitung wurde die Reaktionsmischung bei RT mit Wasser versetzt und mit IM Salzsäure angesäuert. Der entstandene Feststoff wurde abfiltriert, mit Wasser sowie Essigsäureethylester/MTBE (1:1) gewaschen und über Nacht bei 50°C im Vakuum getrocknet. Man erhielt 951 mg (66 % d. Th.) der Zielverbindung.
LC-MS (Methode 1): Rₜ = 0.71 min; MS (ESIpos): m/z = 346 (M+H)⁺.
¹H-NMR (400 MHz, DMSO-d₆): δ [ppm] = 1.22 (t, 3H), 1.28 (t, 3H), 3.90 (q, 2H), 4.17 (q, 2H), 7.36 - 7.41 (m, 1H), 7.43 - 7.47 (m, 1H), 7.59 - 7.62 (m, 1H), 8.28 (s, 1H), 11.70 (s, 1H).

### Beispiel 31 A

### Ethyl-1-(3-methyl-2-oxo-2,3-dihydro-1,3-benzothiazol-6-yl)-2,4-dioxo-1,2,3,4-tetrahydro-pyrimidin-5-carboxylat

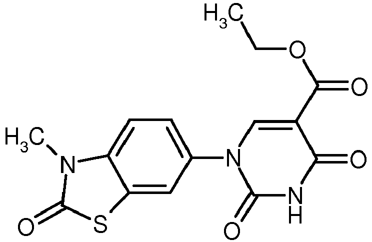

450 mg (2.50 mmol) 6-Amino-3-methyl-1,3-benzothiazol-2(3H)-on (J. Het. Chem. 1992, 29 (5), 1069-1076, Beispiel 8b) sowie 647 mg (2.50 mmol) Ethyl-3-ethoxy-2-[(ethoxycarbonyl)carbamoyl]acrylat wurden in 19 mL Ethanol vorgelegt und die Mischung 2 h zum Rückfluss erhitzt. Nach Abkühlen auf RT wurden 280 mg (2.50 mmol) Kalium-tert.-butylat zugegeben und das Reaktionsgemisch weiter über Nacht bei RT gerührt. Zur Aufarbeitung wurde die Reaktionsmischung mit Wasser verdünnt, mit IM Salzsäure angesäuert und der entstandene Feststoff abfiltriert. Der Feststoff wurde mit Wasser sowie Essigsäureethylester gewaschen und über Nacht im Vakuum bei 50°C getrocknet. Man erhielt 736 mg (85 % d. Th.) der Zielverbindung.
LC-MS (Methode 1): Rₜ = 0.70 min; MS (ESIpos): m/z = 348 (M+H)⁺.
¹H-NMR (400 MHz, DMSO-d₆): δ [ppm] = 1.22 (t, 3H), 3.45 (s, 3H), 4.17 (q, 2H), 7.42 - 7.47 (m, 1H), 7.51 - 7.55 (m, 1H), 7.83 - 7.86 (m, 1H), 8.32 (s, 1H), 11.71 (s, 1H).

### Beispiel 32A

### 1-Methyl-6-nitro-1,3-dihydro-2H-benzimidazol-2-on

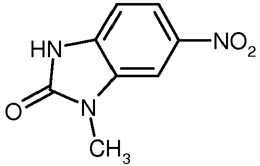

In DMF (9 mL) wurden 500 mg (2.99 mmol) N²-Methyl-4-nitrobenzol-1,2-diamin [Synthese beschrieben in WO 2008/128009, Seite 49] vorgelegt, danach 4.17 mL (0.73 mmol) Triethylamin sowie 2.42 g (15.0 mmol) N,N'-Carbonyldiimidazol zugefügt und die Mischung 5 h bei 100°C gerührt. Anschließend wurde die Reaktionsmischung mit Wasser versetzt und mit IM Salzsäure auf pH 3 eingestellt. Der entstandene Feststoff wurde abfiltriert, mit Wasser nachgewaschen und über Nacht bei 50°C im Vakuum getrocknet. Man erhielt 482 mg (91 %-ige Reinheit 76 % d. Th.) der Zielverbindung. Das Rohprodukt wurde ohne weitere Reinigung umgesetzt.
LC-MS (Methode 3): Rt = 0.71 min; MS (ESIpos): m/z = 194 (M+H)⁺.
¹H-NMR (400 MHz, DMSO-d₆): δ [ppm] = 3.37 (s, 3H), 7.15 (d, 1H), 7.97 - 8.01 (m, 1H), 8.02 - 8.03 (m, 1H), 11.64 (s, 1H).

### Beispiel 33A

### 6-Amino-1-methyl-1,3-dihydro-2H-benzimidazol-2-on

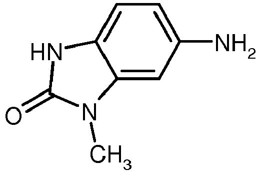

In 31 mL Ethanol wurden 480 mg (2.49 mmol) der Nitroverbindung aus Beispiel 32A vorgelegt, danach 132 mg (0.12 mmol) Palladium (10 %-ig auf Aktivkohle) zugefügt und die Mischung 2 h bei Wasserstoff-Normaldruck hydriert. Anschließend wurde die Reaktionsmischung über Kieselgur filtriert, der Rückstand mit Methanol nachgewaschen und das Filtrat eingeengt. Man erhielt 418 mg (90 %-ige Reinheit 93 % d. Th.) der Zielverbindung. Das Rohprodukt wurde ohne weitere Reinigung umgesetzt.
LC-MS (Methode 2): Rₜ = 0.27 min; MS (ESIpos): m/z = 164 (M+H)⁺.
¹H-NMR (400 MHz, DMSO-d₆): δ [ppm] = 3.16 (s, 3H), 4.72 (s, 2H), 6.23 (dd, 1H), 6.28 - 6.31 (m, 1H), 6.63 (d, 1H), 10.28 (s, 1H).

### Beispiel 34A

### Ethyl-1-(3-methyl-2-oxo-2,3-dihydro-1H-benzimidazol-5-yl)-2,4-dioxo-1,2,3,4-tetrahydro-pyrimidin-5-carboxylat

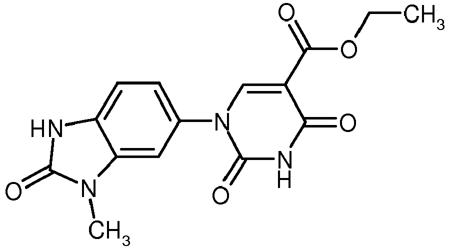

410 mg (2.51 mmol) 6-Amino-1-methyl-1,3-dihydro-2H-benzimidazol-2-on aus Beispiel 33A sowie 651 mg (2.51 mmol) Ethyl-(2E)-3-ethoxy-2-[(ethoxycarbonyl)-carbamoyl]acrylat [Darstellung siehe: Senda, Shigeo; Hirota, Kosaku; Notani, Jiyoji, Chemical & Pharmaceutical Bulletin (1972), 20(7), 1380-1388] wurden in 19 mL Ethanol vorgelegt und die Mischung 2 h zum Rückfluss erhitzt. Anschließend wurden bei RT 282 mg (2.51 mmol) Kalium-tert.-butylat zugegeben und weitere 3 h zum Rückfluss erhitzt. Zur Aufarbeitung wurde das Reaktionsgemisch mit Wasser versetzt und mit IN Salzsäure bis pH3 angesäuert. Der entstandene Feststoff wurde abgesaugt, mit Essigsäureethylester gewaschen und im Vakuum bei 50°C getrocknet. Man erhielt 251 mg (73 %-ige Reinheit, 22 % d. Th.) der Zielverbindung, die ohne weitere Reinigung umgesetzt wurden. Das verbliebene Filtrat wurde dreimal mit Essigsäureethylester extrahiert, die vereinigten organischen Phasen über Magnesiumsulfat getrocknet, filtriert und eingeengt. Der Rückstand wurde in Essigsäureethylester/MTBE-Mischung ausgerührt, der Feststoff abfiltriert und am Hochvakuum getrocknet. Man erhielt so weitere 443 mg (53 % d. Th.) der Zielverbindung.
LC-MS (Methode 1): Rₜ = 0.51 min; MS (ESIpos): m/z = 331 (M+H)⁺.

### Beispiel 35A

### 5-Amino-1-methyl-1,3-dihydro-2H-benzimidazol-2-on

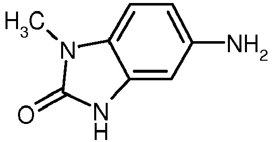

29.5 g (150 mmol) 1-Methyl-5-nitro-1,3-dihydro-2H-benzinlidazol-2-on [Synthese beschrieben in US 6,114,532] wurden in 630 ml Methanol und 315 ml THF vorgelegt, mit 1.62 g Palladium (10 %-ig auf Aktivkohle) versetzt und unter Wasserstoff-Normaldruck bei RT hydriert. Am Ende der Reaktion wurde die Reaktionsmischung über Kieselgur filtriert und das Filtrat am Rotationsverdampfer eingeengt. Der Rückstand wurde mit Diethylether verrührt, abgesaugt und getrocknet. Man erhielt 24.5 g (96 % d. Th.) der Titelverbindung.
LC-MS (Methode 1): Rₜ = 0.16 min; MS (ESIpos): m/z = 164 (M+H)⁺.

### Beispiel 36A

### Ethyl-1-(1-methyl-2-oxo-2,3-dihydro-1H-benzimidazol-5-yl)-2,4-dioxo-1,2,3,4-tetrahydro-pyrimidin-5-carboxylat

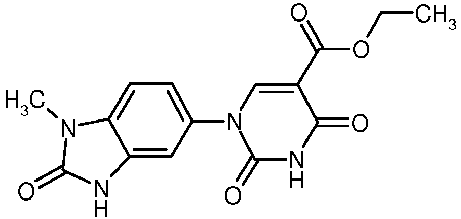

5.00 g (29.3 mmol) 5-Amino-1-methyl-1,3-dihydro-2H-benzimidazol-2-on aus Beispiel 35A und 7.60 g (29.3 mmol) Ethyl-3-ethoxy-2-[(ethoxycarbonyl)carbamoyl]acrylat wurden in 250 mL Ethanol 2 h zum Rückfluss erhitzt. Nach Abkühlen auf RT wurden 3.29 g (29.3 mmol) Kalium-tert.-butylat zugegeben und das Reaktionsgemisch weitere 2.5 h zum Rückfluss erhitzt. Zur Aufarbeitung wurde die Reaktionsmischung bei RT mit 4M Salzsäure angesäuert und mit Wasser verdünnt. Die Mischung wurde im Vakuum teilweise eingeengt und die verbliebene Suspension filtriert. Der Filterrückstand wurde mit Wasser sowie Essigsäureethylester gewaschen und im Vakuum bei 30°C getrocknet. Man erhielt 7.56 g (78 % d. Th.) der Zielverbindung.
LC-MS (Methode 5): Rₜ = 0.52 min; MS (ESIpos): m/z = 331 (M+H)⁺.
¹H-NMR (400 MHz, DMSO-d₆): δ [ppm] = 1.22 (t, 3H), 3.31 (s, 3H), 4.16 (q, 2H), 7.10 - 7.21 (m, 3H), 8.23 (s, 1H), 11.12 (s, 1H), 11.63 (s, 1H).

### Beispiel 37A

### N-[4-(Cyclobutylamino)-3-nitrophenyl]acetamid

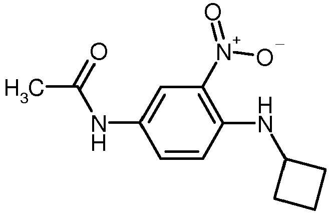

In 40 mL Ethanol wurden 1.00 g (5.04 mmol) N-(4-Fluor-3-nitrophenyl)acetamid (Herstellung siehe: WO2005/72741 Seite 26, Beispiel 117A) sowie 0.86 mL (10.09 mmol) Cyclobutylamin vorgelegt, anschließend 1.40 mL (10.09 mmol) Triethylamin zugefügt und die Reaktionsmischung für 1.5 h bei 140°C in der Mikrowelle gerührt. Zur Aufarbeitung wurde die Mischung im Vakuum eingeengt, der Rückstand mit MTBE verrührt. der gebildete Feststoff abfiltriert und am Hochvakuum getrocknet. Man erhielt 185 mg (69 %-ige Reinheit, 10 % d. Th.) der Zielverbindung. Das verbliebene Filtrat wurde eingeengt, der Rückstand in Essigsäureethylester aufgenommen, je einmal mit Wasser sowie gesättigter Natriumchlorid-Lösung gewaschen, über Magnesiumsulfat getrocknet, filtriert und eingeengt. Man erhielt nach Trocknung im Hochvakuum weitere 1.01 g (78 % d. Th.) der Zielverbindung.
LC-MS (Methode 3): Rₜ = 1.31 min; MS (ESIpos): m/z = 250 (M+H)⁺.
¹H-NMR (400 MHz, DMSO-d₆): δ [ppm] = 1.70 - 1.85 (m, 2H), 1.93 - 2.04 (m, 5H), 2.39 - 2.47 (m, 2H), 4.12 (sxt, 1H), 6.92 (d, 1H), 7.65 (dd, 1H), 7.93 (d, 1H), 8.46 (d, 1H), 9.97 (s, 1H).

### Beispiel 38A

### N-[3-Amino-4-(cyclobutylamino)phenyl]acetamid

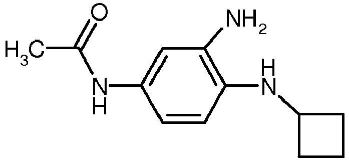

In 96 mL Essigsäureethylester wurden 1.02 g (4.07 mmol) N-[4-(Cyclobutylamino)-3-nitrophenyl]acetamid aus Beispiel 37A vorgelegt, danach 216 mg (0.20 mmol) Palladium (10 %-ig auf Aktivkohle) zugefügt und die Mischung 2 h bei Wasserstoff-Normaldruck hydriert. Anschließend wurde die Reaktionsmischung über Kieselgur filtriert, der Rückstand mit Methanol gewaschen und das Filtrat eingeengt. Man erhielt 870 mg (90 %-ige Reinheit, 87 % d. Th.) der Titelverbindung. Das Rohprodukt wurde ohne weitere Reinigung umgesetzt.
LC-MS (Methode 2): Rₜ = 1.01 min; MS (ESIpos): m/z = 220 (M+H)⁺.
¹H-NMR (400 MHz, DMSO-d₆): δ [ppm] = 1.64 - 1.87 (m, 4H), 1.93 (s, 3H), 2.28 - 2.38 (m, 2H), 3.76 (sxt, 1H), 4.42 (d, 1H), 4.51 - 4.60 (m, 2H), 6.20 (d, 1H), 6.61 (dd, 1H), 6.82 (d, 1H), 9.34 (s, 1H).

### Beispiel 39A

### N-(1-Cyclobutyl-1H-benzimidazol-5-yl)acetamid

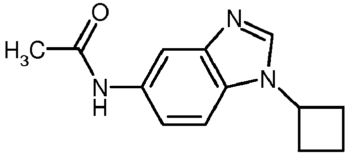

In 25 mL (Diethoxymethoxy)ethan wurden 870 mg (3.96 mmol) N-[3-Amino-4-(cyclobutylamino)phenyl]acetamid aus Beispiel 38A vorgelegt, anschließend 0.43 mL (5.17 mmol) konz. Salzsäure zugetropft und die Reaktionsmischung über Nacht bei RT gerührt. Der ausgefallene Feststoff wurde abgesaugt, mit Essigsäureethylester nachgewaschen und am Hochvakuum getrocknet. Man erhielt 930 mg (100 % d. Th.) der Titelverbindung.
LC-MS (Methode 1): Rₜ = 0.43 min; MS (ESIpos): m/z = 230 (M+H)⁺.
¹H-NMR (400 MHz, DMSO-d₆): δ [ppm] = 1.89 - 2.00 (m, 2H), 2.11 (s, 3H), 2.57 - 2.64 (m, 4H), 5.15 (quin, 1H), 7.59 - 7.64 (m, 1H), 7.86 (d, 1H), 8.38 (d, 1H), 9.66 (s, 1H), 10.53 (s, 1H).

### Beispiel 40A

### 1-Cyclobutyl-1H-benzimidazol-5-amin

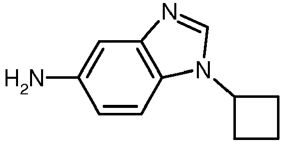

920 mg (4.01 mmol) N-(1-Cyclobutyl-1H-benzimidazol-5-yl)acetamid aus Beispiel 39A wurden in 20 mL einer 1:1-Mischung von IM Salzsäure und Ethanol vorgelegt und das Reaktionsgemisch 1 h bei 120°C gerührt. Das auf RT abgekühlte Reaktionsgemisch wurde eingeengt, in Essigsäureethylester aufgenommen, je einmal mit IN Natronlauge sowie gesättigter Natriumchlorid-Lösung gewaschen, die organische Phase über Magnesiumsulfat getrocknet, filtriert und im Vakuum eingeengt. Man erhielt 593 mg (75 % d. Th.) der Titelverbindung.
LC-MS (Methode 2): Rₜ = 0.89 min; MS (ESIpos): m/z = 189 (M+H)⁺.
¹H-NMR (400 MHz, DMSO-d₆): δ [ppm] = 1.80 - 1.92 (m, 2H), 2.43 - 2.48 (m, teilweise durch DMSO-Signal verdeckt), 4.66 - 4.76 (m, 2H), 4.82 (quin, 1H), 6.59 (dd, 1H), 6.76 (d, 1H), 7.24 (d, 1H), 8.07 (s, 1H).

### Beispiel 41A

### Ethyl-1-(1-cyclobutyl-1H-benzimidazol-5-yl)-2,4-dioxo-1,2,3,4-tetrahydropyrimidin-5-carboxylat

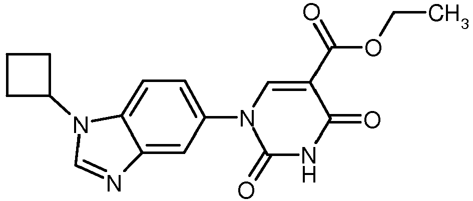

Darstellung und Reinigung der Zielverbindung erfolgten analog zum Beispiel 27A bei einer Reaktionsdauer von 3 h unter Rückfluss. Ausgehend von 590 mg (3.15 mmol) 1-Cyclobutyl-1H-benzimidazol-5-amin aus Beispiel 40A und 817 mg (3.15 mmol) Ethyl-3-ethoxy-2-[(ethoxycarbonyl)carbamoyl]acrylat erhielt man 832 mg (67 % d. Th.) der Titelverbindung.
LC-MS (Methode 1): Rₜ = 0.62 min; MS (ESIpos): m/z = 355 (M+H)⁺.
¹H-NMR (400 MHz, DMSO-d₆): δ [ppm] = 1.18 (t, 3H), 1.86 - 1.97 (m, 2H), 2.55 - 2.58 (m, teilweise durch DMSO-Signal verdeckt), 3.40-3.48 (m, 1H), 4.06 (q, 2H), 5.01 (quin, 1H), 7.16 (dd, 1H), 7.54 (d, 1H), 7.62 (d, 1H), 7.96 (s, 1H), 8.45 (s, 1H).

### Beispiel 42A

### N-Ethyl-2,4-dinitroanilin

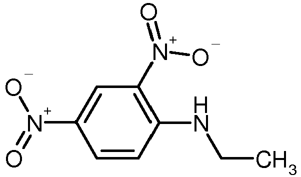

2.00 g (9.87 mmol) 1-Chlor-2,4-dinitrobenzol wurden in 20 mL THF vorgelegt, anschließend bei 0°C 5.92 mL (11.84 mmol) einer 2M Lösung von Ethylamin in THF zugetropft und das Reaktionsgemisch bei RT über Nacht gerührt. Danach wurden bei 0°C erneut 9.86 mL (19.73 mmol) einer 2M Lösung von Ethylamin in THF zugegeben und die Reaktion weitere 5 h bei RT gerührt. Anschließend wurden bei 0°C weitere 4.93 mL (9.86 mmol) einer 2M Lösung von Ethylamin in THF zugegeben und weiter über Nacht gerührt. Zur Aufarbeitung wurde die Reaktionsmischung mit gesättigter Natriumhydrogencarbonat-Lösung versetzt und dreimal mit Essigsäureethylester extrahiert. Die vereinigten organischen Phasen wurden über Magnesiumsulfat getrocknet, filtriert und eingeengt. Der erhaltene Rückstand wurde in MTBE ausgerührt und der ausgefallene Feststoff abgesaugt. Das Filtrat wurde eingeengt und man erhielt eine Gesamtausbeute von 2.29 g (100 % d. Th.) der Titelverbindung.
LC-MS (Methode 1): Rₜ = 0.97 min; MS (ESIpos): m/z = 212 (M+H)⁺.
¹H-NMR (400 MHz, DMSO-d₆): δ [ppm] = 1.23 (t, 3H), 3.49 - 3.58 (m, 2H), 7.23 (d, 1H), 8.26 (dd, 1H), 8.81 - 8.89 (m, 2H).

### Beispiel 43A

### N¹-Ethyl-4-mtrobenzol-1,2-diamin

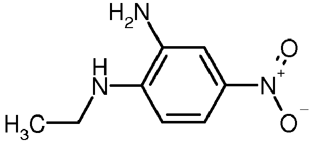

1.20 g (5.68 mmol) N-Ethyl-2,4-dinitroanilin aus Beispiel 42A wurden in 3 mL Acetonitril unter Argon vorgelegt und 64 mg (0.06 mmol) Palladium (10 %-ig auf Aktivkohle) sowie 3.40 mL (24.38 mmol) Triethylamin zugegeben. Das Reaktionsgemisch wurde auf -15°C abgekühlt und mit einer Lösung von 1.03 mL (27.44 mmol) Ameisensäure in 3 mL Acetonitril versetzt. Das Reaktionsgemisch wurde 1 h bei 40°C sowie 2 h bei 60°C gerührt. Zur Aufarbeitung wurde das Reaktionsgemisch bei RT über Kieselgur filtriert, mit Essigsäureethylester/Methanol (1:1) nachgewaschen und das Filtrat eingeengt. Der Rückstand wurde mit Wasser versetzt, der ausgefallene Feststoff abgesaugt, mit Wasser gewaschen und bei 50°C im Vakuum getrocknet. Man erhielt 546 mg (47 % d. Th.) der Titelverbindung.
LC-MS (Methode 1): Rₜ = 0.79 min; MS (ESIpos): m/z = 182 (M+H)⁺.
¹H-NMR (400 MHz, DMSO-d₆): δ [ppm] = 1.23 (t, 3H), 3.18 - 3.26 (m, 2H), 5.09 - 5.20 (m, 2H), 5.87 (t, 1H), 6.46 (d, 1H), 7.39 (d, 2H), 7.52 (dd, 1H).

### Beispiel 44A

### 1-Ethyl-5-nitro-1H-benzimidazol

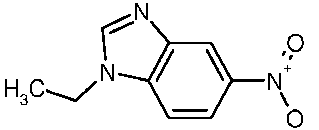

In 19 mL (Diethoxymethoxy)ethan wurden 540 mg (2.98 mmol) N¹-Ethyl-4-nitrobenzol-1,2-diamin aus Beispiel 43A vorgelegt, anschließend 0.32 mL (3.89 mmol) konz. Salzsäure zugetropft und die Reaktionsmischung 2 h bei RT gerührt. Anschließend wurde im Vakuum eingeengt, der Rückstand in MTBE ausgerührt, abfiltriert, mit MTBE gewaschen und getrocknet. Man erhielt 486 mg (54 % d. Th.) der Titelverbindung.
LC-MS (Methode 1): Rt = 0.65 min; MS (ESIpos): m/z = 192 (M+H)⁺.
¹H-NMR (400 MHz, DMSO-d₆): δ [ppm] = 1.46 (t, 3H), 4.42 (q, 2H), 7.97 (d, 1H), 8.26 (d, 1H), 8.60 (d, 1H), 8.83 - 8.90 (m, 1H).

### Beispiel 45A

### 1-Ethyl-1H-benzimidazol-5-amin

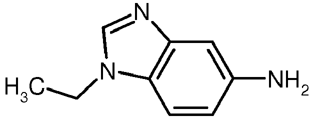

Darstellung und Reinigung der Zielverbindung erfolgten analog zum Beispiel 33A und einer Reaktionsdauer über Nacht. Ausgehend von 485 mg (2.53 mmol) 1-Ethyl-5-nitro-1H-benzimidazol aus Beispiel 44A erhielt man 417 mg (101 % d. Th.) der Titelverbindung.
LC-MS (Methode 2): Rₜ = 0.23 min; MS (ESIpos): m/z = 162 (M+H)⁺.
¹H-NMR (400 MHz, DMSO-d₆): δ [ppm] = 1.47 (t, 3H), 4.36 (q, 2H), 6.85 - 6.96 (m, 2H), 7.64 (d, 1H), 9.16 (s, 1H).

### Beispiel 46A

### Ethyl-1-(1-ethyl-1H-benzimidazol-5-yl)-2,4-dioxo-1,2,3,4-tetrahydropyrimidin-5-carboxylat

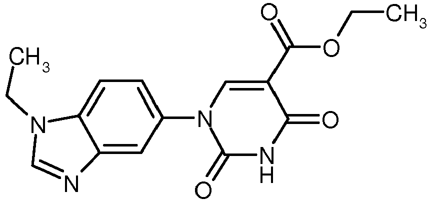

In 19 mL Ethanol wurden 659 mg (2.54 mmol) Ethyl-3-ethoxy-2-[(ethoxycarbonyl)carbamoyl]acrylat sowie 410 mg g (2.54 mmol) 1-Ethyl-1H-benzimidazol-5-amin aus Beispiel 45A vorgelegt und die Mischung 2 h bei Rückfluss gerührt. Danach wurden bei RT 285 mg (2.54 mmol) Kalium-tert.-butylat zugefügt und die Reaktionsmischung 3h zum Rückfluß erhitzt. Zur Aufarbeitung wurde das Reaktionsgemisch mit Wasser versetzt und die Mischung im Vakuum eingeengt. Der Rückstand wurde mit Dichlormethan/Methanol verrührt, filtriert und das Filtrat eingeengt. Der so erhaltene Rückstand wurde in MTBE/Essigsäureethylester verrührt, der Feststoff abfiltriert, mit Essigsäureethylester gewaschen und anschließend im Vakuum bei 50°C getrocknet. Man erhielt 491 mg (59 % d. Th.) der Titelverbindung.
LC-MS (Methode 3): Rₜ = 0.60 min; MS (ESIpos): m/z = 329 (M+H)⁺.
¹H-NMR (400 MHz, DMSO-d₆): δ [ppm] = 1.17 - 1.23 (m, 3H), 1.42 (t, 3H), 4.08 - 4.16 (m, 2H), 4.28 - 4.36 (m, 2H), 7.26 (d, 1H), 7.63 - 7.71 (m, 2H), 8.15 (s, 1H), 8.35 (s, 1H).

### Beispiel 47A

### N-Isopropyl-2,4-dinitroanilin

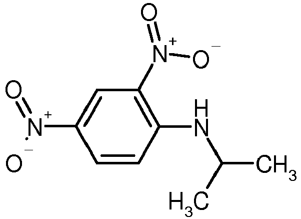

1.00 g (4.93 mmol) 1-Chlor-2,4-dinitrobenzol wurden in 10 mL THF vorgelegt, anschließend 0.84 mL (9.87 mmol) Isopropylamin zugetropft und das Reaktionsgemisch 16 h bei RT gerührt. Zur Aufarbeitung wurde das Gemisch mit gesättigter Natriumhydrogencarbonat-Lösung versetzt, dreimal mit Essigsäureethylester gewaschen, die vereinigten organischen Phasen über Magnesiumsulfat getrocknet, filtriert und eingeengt. Man erhielt 1.13 g (99 % d. Th.) der Titelverbindung.
LC-MS (Methode 3): Rₜ = 1.30 min; MS (ESIpos): m/z = 226 (M+H)⁺.
¹H-NMR (400 MHz, DMSO-d₆): δ [ppm] = 1.31 (d, 6H), 4.02 - 4.15 (m, 1H), 7.27 (d, 1H), 8.27 (dd, 1H), 8.42 (d, 1H), 8.86 (d, 1H).

### Beispiel 48A

### N¹-Isopropyl-4-nitrobenzol-1,2-diamin

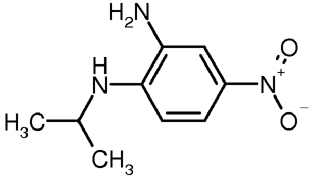

Darstellung und Reinigung der Zielverbindung erfolgten analog zum Beispiel 43A mit einer Reaktionsdauer von 7 h. Ausgehend von 1.13 g (5.01 mmol) N-Isopropyl-2,4-dinitroanilin aus Beispiel 47A erhielt man 708 mg (72 % d. Th.) der Titelverbindung.
LC-MS (Methode 1): Rₜ = 0.88 min; MS (ESIpos): m/z = 196 (M+H)⁺.
¹H-NMR (400 MHz, DMSO-d₆): δ [ppm] = 1.21 (d, 6H), 3.69 - 3.81 (m, 1H), 5.11 - 5.24 (m, 2H), 5.62 (d, 1H), 6.49 (d, 1H), 7.39 (d, 1H), 7.51 (dd, 1H).

### Beispiel 49A

### 1-Isopropyl-5-nitro-1H-benzimidazol

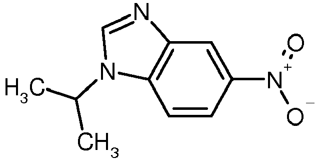

Darstellung der Zielverbindung erfolgte analog zum Beispiel 39A unter Verwendung von 700 mg (3.58 mmol) N¹-Isopropyl-4-nitrobenzol-1,2-diamin aus Beispiel 48A sowie 23 mL (137.49 mmol) (Diethoxymethoxy)ethan. Zur Aufarbeitung wurde die Mischung eingeengt, der Rückstand mit MTBE verrührt, abfiltriert und im Hochvakuum getrocknet. Man erhielt 760 mg der Titelverbindung. Das Rohprodukt wurde ohne weitere Reinigung umgesetzt.
LC-MS (Methode 3): Rₜ = 0.98 min; MS (ESIpos): m/z = 206 (M+H)⁺.
¹H-NMR (400 MHz, DMSO-d₆): δ [ppm] = 1.58 (d, 6H), 4.88 - 4.99 (m, 1H), 8.01 (d, 1H), 8.24 (dd, 1H), 8.60 (d, 1H), 8.94 - 9.01 (m, 1H).

### Beispiel 50A

### 1-Isopropyl-1H-benzimidazol-5-amin

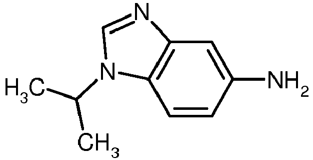

Darstellung und Reinigung der Zielverbindung erfolgten analog zum Beispiel 33A mit einer Reaktionsdauer von 16h. Ausgehend von 750 mg (3.65 mmol) 1-Isopropyl-5-nitro-1H-benzimidazol aus Beispiel 49A erhielt man 612 mg (95 % d. Th.) der Titelverbindung.
LC-MS (Methode 1): Rₜ = 0.23 min; MS (ESIpos): m/z = 176 (M+H)⁺.
¹H-NMR (400 MHz, DMSO-d₆): δ [ppm] = 1.56 (d, 6H), 3.34 (s, durch Wassersignal verdeckt), 4.77-4.90 (m 1H), 6.87 - 6.95 (m, 2H), 7.67 (d, 1H), 9.22 (s, 1H).

### Beispiel 51 A

### Ethyl-1-(1-isopropyl-1H-benzimidazol-5-yl)-2,4-dioxo-1,2,3,4-tetrahydropyrimidin-5-carboxylat

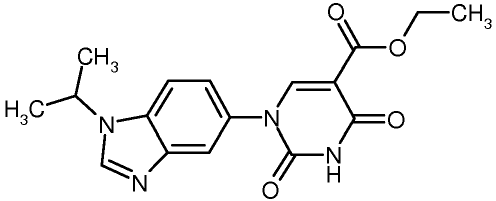

Darstellung und Reinigung der Zielverbindung erfolgten analog zum Beispiel 27A. Ausgehend von 612 mg (3.49 mmol) 1-Isopropyl-1H-benzimidazol-5-amin aus Beispiel 50A und 905 mg (3.49 mmol) Ethyl-3-ethoxy-2-[(ethoxycarbonyl)carbamoyl]acrylat erhielt man 684 mg (57 % d. Th.) der Titelverbindung.
LC-MS (Methode 1): Rₜ = 0.56 min; MS (ESIpos): m/z = 343 (M+H)⁺.
¹H-NMR (400 MHz, DMSO-d₆): δ [ppm] = 1.21 (t, 3H), 1.56 (d, 6H), 4.15 (q, 2H), 4.81 (spt, 1H), 7.32 (d, 1H), 7.71 - 7.79 (m, 2H), 8.26 (s, 1H), 8.47 (s, 1H), 11.66 (br.s, 1H).

### Beispiel 52A

### N-{4-[(Cyclopropylmethyl)amino]-3-nitrophenyl}acetamid

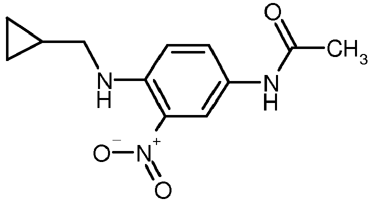

Darstellung und Reinigung der Zielverbindung erfolgten analog zum Beispiel 37A Ausgehend von 1.00 g (5.04 mmol) N-(4-Fluor-3-nitrophenyl)acetamid und 1.04 mL (10.09 mmol) Cyclopropylmethylamin erhielt man 1.34 g der Titelverbindung. Das Rohprodukt wurde ohne weitere Reinigung umgesetzt.
LC-MS (Methode 3): Rₜ = 1.10 min; MS (ESIpos): m/z = 250 (M+H)⁺.
¹H-NMR (400 MHz, DMSO-d₆): δ [ppm] = 0.27 - 0.33 (m, 2H), 0.49 - 0.55 (m, 2H), 1.10 - 1.22 (m, 1H), 2.02 (s, 3H), 3.21 (t, 2H), 7.07 (d, 1H), 7.65 (dd, 1H), 8.09 (t, 1H), 8.46 (d, 1H), 9.96 (s, 1H).

### Beispiel 53A

### N-{3-Amino-4-[(cyclopropylmethyl)amino]phenyl}acetamid

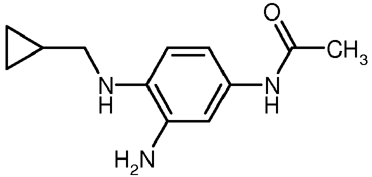

Darstellung und Reinigung der Zielverbindung erfolgten analog zum Beispiel 38A. Ausgehend von 1.10 g (4.41 mmol) N-{4-[(Cyclopropylmethyl)amino]-3-nitrophenyl}acetamid aus Beispiel 52A erhielt man 952 mg (98 % d. Th.) der Titelverbindung.
LC-MS (Methode 2): Rₜ = 0.92 min; MS (ESIpos): m/z = 220 (M+H)⁺.
¹H-NMR (400 MHz, DMSO-d₆): δ [ppm] = 0.17 - 0.23 (m, 2H), 0.42 - 0.52 (m, 2H), 1.01 - 1.13 (m, 1H), 1.93 (s, 3H), 2.83 (t, 2H), 4.22 (t, 1H), 4.50 - 4.65 (m, 2H), 6.31 (d, 1H), 6.64 (dd, 1H), 6.84 (d, 1H), 9.36 (s, 1H).

### Beispiel 54A

### N-[1-(Cyclopropylmethyl)-1H-benzimidazol-5-yl]acetamid

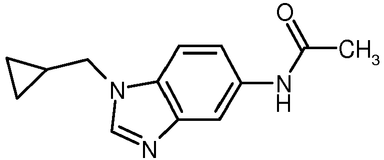

Darstellung und Reinigung der Zielverbindung erfolgten analog zum Beispiel 39A. Ausgehend von 951 mg (4.33 mmol) N-{3-Amino-4-[(cyclopropylmethyl)amino]phenyl}-acetamid aus Beispiel 53A und 28 mL (166.29 mmol) (Diethoxymethoxy)ethan erhielt man 929 mg (84 % d. Th.) der Titelverbindung.
LC-MS (Methode 3): Rₜ = 0.39 min; MS (ESIpos): m/z = 230 (M+H)⁺.
¹H-NMR (400 MHz, DMSO-d₆): δ [ppm] = 0.47 - 0.53 (m, 2H), 0.58 - 0.64 (m, 2H), 1.32 - 1.43 (m, 1H), 2.10 (s, 3H), 4.25 (d, 2H), 7.55 (dd, 1H), 7.87 (d, 1H), 8.30 (d, 1H), 9.22 (s, 1H), 10.34 (s, 1H).

### Beispiel 55A

### 1-(Cyclopropylmethyl)-1H-benzimidazol-5-amin

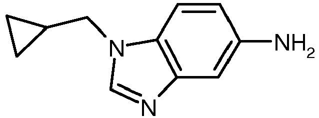

Darstellung und Reinigung der Zielverbindung erfolgten analog zum Beispiel 40A. Ausgehend von 828 mg (3.61 mmol) N-[1-(Cyclopropylmethyl)-1H-benzimidazol-5-yl]acetamid aus Beispiel 54A erhielt man 482 mg (70 % d. Th.) der Titelverbindung.
LC-MS (Methode 2): Rₜ = 0.87 min; MS (ESIpos): m/z = 188 (M+H)⁺.
¹H-NMR (400 MHz, DMSO-d₆): δ [ppm] = 0.34 - 0.40 (m, 2H), 0.48 - 0.55 (m, 2H), 1.19 - 1.30 (m, 1H), 3.96 (d, 2H), 4.71 (br.s, 2H), 6.59 (dd, 1H), 6.77 (d, 1H), 7.27 (d, 1H), 7.97 (s, 1H).

### Beispiel 56A

### Ethyl-1-[1-(cyclopropylmethyl)-1H-benzimidazol-5-yl]-2,4-dioxo-1,2,3,4-tetrahydropyrimidin-5-carboxylat

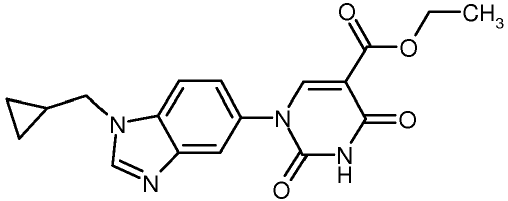

Darstellung der Zielverbindung erfolgte analog zum Beispiel 31A mit einer Reaktionszeit von 5 h unter Verwendung von 547 mg (2.92 mmol) 1-(Cyclopropylmethyl)-1H-benzimidazol-5-amin aus Beispiel 55A sowie 757 mg (2.92 mmol) Ethyl-3-ethoxy-2-[(ethoxycarbonyl)carbamoyl]acrylat. Zur Aufarbeitung wurde die Reaktionsmischung eingeengt, der Rückstand in Essigsäureethylester/Methanol ausgerührt, der Feststoff abgesaugt und am Hochvakuum getrocknet. Man erhielt 1.02 g (98 % d. Th.) der Titelverbindung.
LC-MS (Methode 1): Rₜ = 0.61 min; MS (ESIpos): m/z = 355 (M+H)⁺.
¹H-NMR (400 MHz, DMSO-d₆): δ [ppm] = 0.41 - 0.46 (m, 2H), 0.51 - 0.58 (m, 2H), 1.18 (t, 3H), 1.25 - 1.37 (m, 1H), 4.06 (q, 2H), 4.15 (d, 2H), 7.18 (dd, 1H), 7.53 (d, 1H), 7.67 (d, 1H), 7.97 (s, 1H), 8.33 (s, 1H).

### Beispiel 57A

### 1,3,3-Trimethyl-5-nitro-1,3-dihydro-2H-indol-2-on

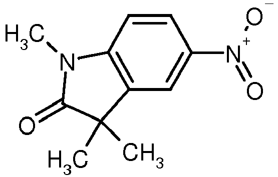

2.44 g (13.96 mmol) 1,3,3-Trimethyl-1,3-dihydro-2H-indol-2-on [Darstellung siehe: Journal of Organic Chemistry, 2000, vol. 65, 24, p. 8317-8325] wurden in 12 mL Essigsäure vorgelegt, anschließend 0.96 mL (13.96 mmol) Salpetersäure (65 %ig) bei RT zugetropft und das Reaktionsgemisch 2 Wochen bei RT gerührt. Die Reaktionsmischung wurde auf Eiswasser gegeben, der ausgefallene Feststoff abgesaugt, mit Wasser nachgewaschen und bei 50°C im Vakuum getrocknet. Man erhielt 2.32 g (72 % d. Th.) der Titelverbindung.
LC-MS (Methode 5): Rₜ = 0.89 min; MS (ESIpos): m/z = 221 (M+H)⁺.
¹H-NMR (400 MHz, DMSO-d₆): δ [ppm] = 1.35 (s, 6H), 3.22 (s, 3H), 7.25 (d, 1H), 8.26 (dd, 1H), 8.33 (d, 1H).

### Beispiel 58A

### 5-Amino-1,3,3-trimethyl-1,3-dihydro-2H-indol-2-on

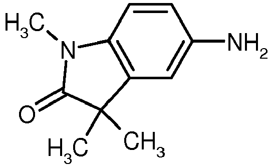

Darstellung und Reinigung der Zielverbindung erfolgten analog zum Beispiel 33A mit einer Reaktionsdauer von 2 Tagen. Ausgehend von 2.32 g (10.56 mmol) 1,3,3-Trimethyl-5-nitro-1,3-dihydro-2H-indol-2-on aus Beispiel 57A erhielt man 1.95 g (93 % d. Th.) der Titelverbindung.
LC-MS (Methode 2): Rₜ = 0.76 min; MS (ESIpos): m/z = 191 (M+H)⁺.
¹H-NMR (400 MHz, DMSO-d₆): δ [ppm] = 1.20 (s, 6H), 3.04 (s, 3H), 4.70 - 4.80 (m, 2H), 6.46 (dd, 1H), 6.58 (d, 1H), 6.67 (d, 1H).

### Beispiel 59A

### Ethyl-2,4-dioxo-1-(1,3,3-trimethyl-2-oxo-2,3-dihydro-1H-indol-5-yl)-1,2,3,4-tetrahydropyrimidin-5-carboxylat

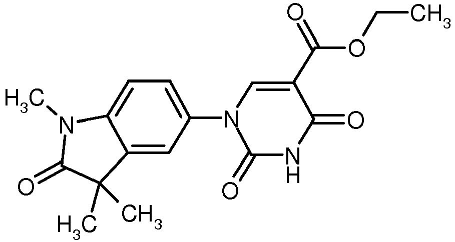

Darstellung und Reinigung der Zielverbindung erfolgten analog zum Beispiel 31A. Ausgehend von 1.95 g (10.26 mmol) 5-Amino-1,3,3-trimethyl-1,3-dihydro-2H-indol-2-on aus Beispiel 58A sowie 2.66 g (10.26 mmol) Ethyl-3-ethoxy-2-[(ethoxycarbonyl)-carbamoyl]acrylat erhielt man 2.84 g (77 % d. Th.) der Titelverbindung.
LC-MS (Methode 2): Rₜ = 1.62 min; MS (ESIpos): m/z = 358 (M+H)⁺.
¹H-NMR (400 MHz, DMSO-d₆): δ [ppm] = 1.22 (t, 3H), 1.29 (s, 6H), 3.17 (s, 3H), 4.17 (q, 2H), 7.13 (d, 1H), 7.40 (dd, 1H), 7.51 (d, 1H), 8.25 (s, 1H), 11.65 - 11.71 (m, 1H).

### Beispiel 60A

### 1'-Methylspiro[cyclopropan-1,3'-indol]-2'(1'H)-on

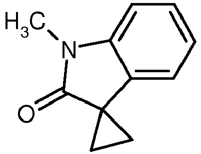

5.43 g (135.89 mmol) Natriumhydrid (60 %ig in Mineralöl) wurden in 40 mL DMF vorgelegt, anschließend wurde bei 0°C eine Lösung von 5.00 g (33.97 mmol) 1-Methyl-1,3-dihydro-2H-indol-2-on in 40 mL DMF zugetropft und die Reaktionsmischung bei RT 30 min gerührt. Anschließend wurden 8.81 mL (101.91 mmol) Dibromethan zugetropft und 1 h bei RT gerührt. Zur Aufarbeitung wurde die Reaktionsmischung mit Wasser versetzt, dreimal mit Essigsäureethylester gewaschen, die vereinigten organischen Phasen über Magnesiumsulfat getrocknet, filtriert und eingeengt. Man erhielt 3.78 g (64 % d. Th.) der Titelverbindung.
LC-MS (Methode 1): Rₜ = 0.76 min; MS (ESIpos): m/z = 174 (M+H)⁺.
¹H-NMR (400 MHz, DMSO-d₆): δ [ppm] = 1.48 - 1.53 (m, 2H), 1.57 - 1.61 (m, 2H), 3.21 (s, 3H), 6.97 - 7.03 (m, 2H), 7.06 (d, 1H), 7.23 - 7.28 (m, 1H).

### Beispiel 61A

### 1'-Methyl-5'-nitrospiro[cyclopropan-1,3'-indol]-2'(1'H)-on

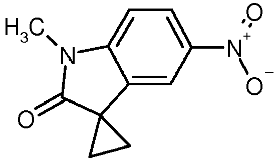

In 40 mL Eisessig wurden 3.77 g (21.79 mmol) 1'-Methylspiro[cyclopropan-1,3'-indol]-2'(1'H)-on aus Beispiel 60A vorgelegt, anschließend 0.90 mL (21.79 mmol) konz. Salpetersäure zugetropft und das Gemisch 2 h bei RT gerührt. Danach wurden weitere 0.45 mL (10.89 mmol) konz. Salpetersäure zugetropft und das Gemisch weitere 1.5 h bei RT gerührt. Zur Aufarbeitung wurde die Mischung auf Eiswasser gegeben, der ausgefallene Feststoff abgesaugt, mit Wasser nachgewaschen und bei 30°C im Vakuum getrocknet. Man erhielt 4.01 g (84 % d. Th.) der Titelverbindung.
GC-MS (Methode 6): Rₜ = 7.21 min; MS (ESIpos): m/z = 219 (M+H)⁺.
¹H-NMR (400 MHz, DMSO-d₆): δ [ppm] = 1.54 - 1.72 (m, 2H), 1.80 - 1.99 (m, 2H), 3.3 (s, teilweise durch Wasser-Signal verdeckt), 7.17 - 7.38 (m, 1H), 7.91 - 8.09 (m, 1H), 8.14 - 8.31 (m, 1H).

### Beispiel 62A

### 5'-Amino-1'-methylspiro[cyclopropan-1,3'-indol]-2'(1'H)-on

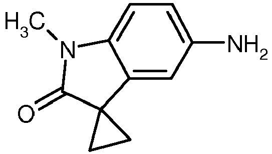

In 11 mL Essigsäureethylester wurden 1.00 g (4.58 mmol) 1'-Methyl-5'-nitrospiro[cyclopropan-1,3'-indol]-2'(1'H)-on aus Beispiel 61A vorgelegt, mit 4.13 g (18.33 mmol) Zinn(II)chlorid-Dihydrat versetzt und 2.5 h zum Rückfluss erhitzt. Die auf RT abgekühlte Reaktionsmischung wurde mit Essigsäureethylester verdünnt und zweimal mit 1 N Salzsäure extrahiert. Die wässrige Phase wurde mit IN Natronlauge auf pH 10 gestellt und viermal mit Dichlormethan extrahiert. Die vereinigten organischen Phasen wurden mit gesättigter Natriumchlorid-Lösung gewaschen, über Magnesiumsulfat getrocknet, filtriert und eingeengt. Man erhielt 375 mg (42 % d. Th.) der Titelverbindung.
LC-MS (Methode 2): Rₜ = 0.73 min; MS (ESIpos): m/z = 189 (M+H)⁺.
¹H-NMR (400 MHz, DMSO-d₆): δ [ppm] = 1.38 - 1.46 (m, 4H), 3.11 (s, 3H), 4.65 - 4.76 (m, 2H), 6.24 (d, 1H), 6.46 (dd, 1H), 6.73 (d, 1H).

### Beispiel 63A

### Ethyl-1-(1'-methyl-2'-oxo-1',2'-dihydrospiro[cyclopropan-1,3'-indol]-5'-yl)-2,4-dioxo-1,2,3,4-tetrahydropyrimidin-5-carboxylat

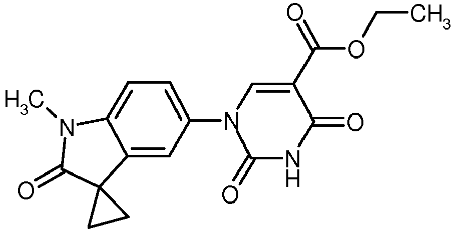

510 mg (1.97 mmol) Ethyl-3-ethoxy-2-[(ethoxycarbonyl)carbamoyl]acrylat sowie 370 mg (1.97 mmol) 5'-Amino-1'-methylspiro[cyclopropan-1,3'-indol]-2'(1'H)-on aus Beispiel 62A wurden in 10 mL Ethanol 45 min zum Rückfluß erhitzt. Danach wurden bei RT 221 mg (1.97 mmol) Kalium-tert.-butylat zugefügt und die Mischung 1.5 h bei RT sowie 1 h zum Rückfluss gerührt. Zur Aufarbeitung wurde die Reaktionsmischung mit Wasser versetzt und mit IN Salzsäure angesäuert. Der entstandene Feststoff wurde abfiltriert, mit Wasser gewaschen und anschließend im Vakuum bei 30°C getrocknet. Man erhielt 557 mg (78 % d. Th.) der Titelverbindung.
LC-MS (Methode 1): Rₜ = 0.69 min; MS (ESIpos): m/z = 356 (M+H)⁺.
¹H-NMR (400 MHz, DMSO-d₆): δ [ppm] = 1.22 (t, 3H), 1.54 - 1.59 (m, 2H), 1.62 - 1.68 (m, 2H), 3.25 (s, 3H), 4.17 (q, 2H), 7.15 - 7.20 (m, 2H), 7.35 - 7.41 (m, 1H), 8.25 (s, 1H), 11.68 (s, 1H).

### Beispiel 64A

### Ethyl-2,4-dioxo-1-(1,3,3-trimethyl-2-oxo-2,3-dihydro-1H-indol-6-yl)-1,2,3,4-tetrahydropyrimidin-5-carboxylat

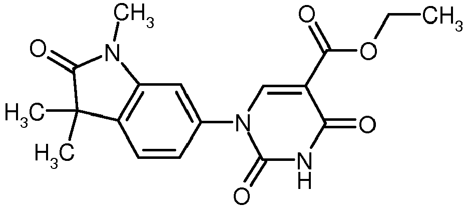

In 10 mL Ethanol wurden 388 mg (1.49 mmol) Ethyl-3-ethoxy-2-[(ethoxycarbonyl)carbamoyl]acrylat sowie 285 mg (1.49 mmol) 6-Amino-1,3,3-trimethyl-1,3-dihydro-2H-indol-2-on [Darstellung siehe: Journal of Medicinal Chemistry, 1989, Vol. 32, (7), 1481-1491] vorgelegt und die Mischung 2 h zum Rückfluss erhitzt. Danach wurden bei RT 167 mg (1.49 mmol) Kalium-tert.-butylat zugefügt und die Mischung 1 h bei RT sowie 15 min bei Rückfluß gerührt. Zur Aufarbeitung wurde die Reaktionsmischung mit Wasser versetzt, mit IN Salzsäure angesäuert und zweimal mit Essigsäureethylester extrahiert. Die vereinigten organischen Phasen wurden über Magnesiumsulftat getrocknet, filtriert und eingeengt. Der Rückstand wurde mit MTBE/Essigsäureethylester ausgerührt, abfiltriert, mit Essigsäureethylester gewaschen und anschließend im Vakuum bei 50°C getrocknet. Der im Filtrat ausgefallene Feststoff wurde abgesaugt und im Vakuum getrocknet. Man erhielt insgesamt 388 mg (68 % d. Th.) der Titelverbindung.
LC-MS (Methode 1): Rₜ = 0.75 min; MS (ESIpos): m/z = 358 (M+H)⁺.
¹H-NMR (400 MHz, DMSO-d₆): δ [ppm] = 1.22 (t, 3H), 1.30 (s, 6H), 3.14 (s, 3H), 4.17 (q, 2H), 7.16 (d, 1H), 7.23 (s, 1H), 7.48 (d, 1H), 8.30 (s, 1H), 11.73 (s, 3H).

### Beispiel 65A

### Ethyl-1-(3,3-dimethyl-2-oxo-2,3-dihydro-1H-indol-6-yl)-2,4-dioxo-1,2,3,4-tetrahydropyrimidin-5-carboxylat

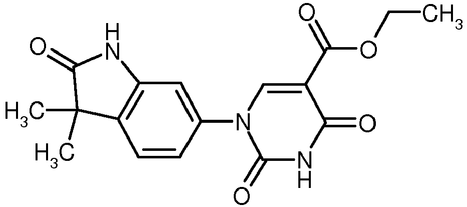

In 14 mL Ethanol wurden 570 mg (2.20 mmol) Ethyl-3-ethoxy-2-[(ethoxycarbonyl)carbamoyl]acrylat sowie 388 mg (2.20 mmol) 6-Amino-3,3-dimethyl-1,3-dihydro-2H-indol-2-on [Darstellung siehe: US 2006/258689, Seite 35] vorgelegt und 2 h zum Rückfluss erhitzt. Anschließend wurden bei RT 247 mg (2.20 mmol) Kalium-tert.-butylat zugefügt und die Mischung 1 h bei RT sowie 1 h bei 60°C gerührt. Zur Aufarbeitung wurde das Reaktionsgemisch mit Wasser versetzt, mit IN Salzsäure angesäuert und zweimal mit Essigsäureethylester extrahiert. Die vereinigten organischen Phasen wurden über Magnesiumsulfat getrocknet, filtriert und eingeengt. Der Rückstand wurde in MTBE/Essigsäureethylester ausgerührt, der entstandene Feststoff abfiltriert, mit Essigsäureethylester gewaschen und anschließend im Vakuum bei 50°C getrocknet. Man erhielt 630 mg (79 % d. Th.) der Titelverbindung.
LC-MS (Methode 1): Rₜ = 0.65 min; MS (ESIpos): m/z = 344 (M+H)⁺.
¹H-NMR (400 MHz, DMSO-d₆): δ [ppm] = 1.22 (t, 3H), 1.28 (s, 6H), 4.16 (q, 2H), 6.96 - 7.01 (m, 1H), 7.04 - 7.09 (m, 1H), 7.42 (d, 1H), 8.27 (s, 1H), 10.58 (s, 1H), 11.65 (s, 1H).

### Beispiel 66A

### Ethyl-1-(1-acetyl-3,3-dimethyl-2,3-dihydro-1H-indol-6-yl)-2,4-dioxo-1,2,3,4-tetrahydropyrimidin-5-carboxylat

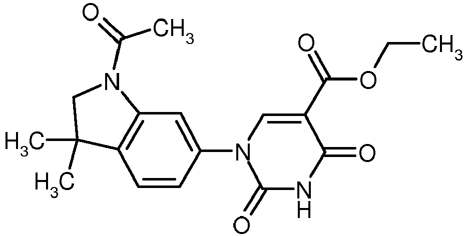

Darstellung und Reinigung der Zielverbindung erfolgten analog zum Beispiel 31A. Ausgehend von 0.81 g (3.96 mmol) 1-(6-Amino-3,3-dimethyl-2,3-dihydro-1H-indol-1-yl)ethanon [Synthese beschrieben in: WO 2006/12374 A1, 2006] sowie 1.06 g (3.96 mmol) Ethyl-3-ethoxy-2-[(ethoxycarbonyl)carbamoyl]acrylat erhielt man 626 mg (40 % d. Th.) der Titelverbindung.
LC-MS (Methode 1): Rₜ = 0.84 min; MS (ESIpos): m/z = 372 (M+H)⁺.
¹H-NMR (400 MHz, DMSO-d₆): δ [ppm] = 1.22 (t, 3H), 1.34 (s, 6H), 2.18 (s, 3H), 3.93 (s, 2H), 4.16 (q, 2H), 7.13 (dd, 1H), 7.38 (d, 1H), 8.05 (d, 1H), 8.23 (s, 1H), 11.64 (br.s, 1H).

### Beispiel 67A

### 5-Brom-1,3,3-trimethyl-1,3-dihydro-2H-indol-2-on

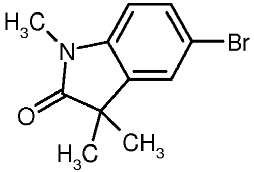

Unter Argon wurden 2.64 g (66 mmol) Natriumhydrid (60 %-ig in Mineralöl) in 25 ml THF suspendiert und auf 0°C gekühlt. Eine Lösung von 4.00 g (18.86 mmol) 5-Brom-1,3-dihydro-2H-indol-2-on in 25 ml DMF wurde tropfenweise hinzugefügt und die Mischung 30 min bei 0°C gerührt. Anschliessend wurden 4.11 ml (66 mmol) Methyliodid langsam dazu zugetropft, die Reaktionsmischung anschliessend auf RT erwärmt und bei dieser Temperatur über Nacht weiter gerührt. Zur Aufarbeitung wurde das Gemisch auf 200 ml IM Salzsäure gegossen und dreimal mit Essigsäureethylester extrahiert. Die vereinigten organischen Phasen wurden mit Wasser, dann einer gesättigten Natriumchloridlösung gewaschen, über Natriumsulfat getrocknet und am Rotationsverdampfer eingeegnt. Der Rückstand wurde in 200 ml Acetonitril gelöst und das Mineralöl mit n-Pentan extrahiert. Die abgetrennte Acetonitril-Phase wurde am Rotationsverdampfer eingeengt und der verbleibende bräunliche Feststoff im HV getrocknet. Man erhielt 4.45 g (84 % d. Th.) der Titelverbindung in 91 %-iger Reinheit.
LC-MS (Methode 3): Rₜ = 1.18 min; m/z = 254, 256 (M+H)⁺.
¹H-NMR (400MHz, DMSO-d₆): δ [ppm]= 1.27 (s, 6H), 3.12 (s, 3H), 6.99 (d, 1H), 7.45 (dd, 1H), 7.60 (d, 1H).

### Beispiel 68A

### 1,3,3-Trimethyl-5-(4,4,5,5-tetramethyl-1,3,2-dioxaborolan-2-yl)-1,3-dihydro-2H-indol-2-on

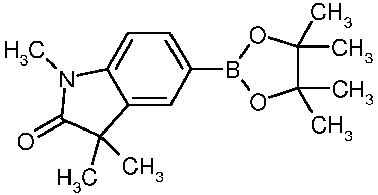

Eine Lösung von 3.45 g (ca. 12.35 mmol) der Verbindung aus Beispiel 67A, 4.71 g (18.5 mmol) 4,4,4',4',5,5,5',5'-Octamethyl-2,2'-bi-1,3,2-dioxaborolan (Bis-pinakolato-dibor) und 2.18 g (22.2 mmol) Kaliumacetat in 60 ml Dioxan wurde entgast und unter Argonatmosphäre gestellt. 1.0 g (1.23 mmol) 1,1'-Bis(diphenylphosphino)ferrocen-palladium(II)dichlorid-Dichlormethankomplex wurden zugegeben und die Mischung über Nacht zum Rückfluss erhitzt. Nach Abkühlen auf RT wurde das Reaktionsgemisch über Celite filtriert, Celite mit Essigsäureethylester gewaschen und das gesamte Filtrat am Rotationsverdampfer eingeengt. Der Rückstand wurde in Dichlormethan auf Diatomeenerde adsorbiert und auf eine Biotage Kieselgelkartusche aufgetragen. Die Kartusche wurde mit Cyclohexan / Essigsäureethylester eluiert. Die produkthaltigen Fraktionen wurden am Rotationsverdampfer eingeengt. Der Rückstand wurde mit 20 ml Diethylether verrührt. Der Feststoff wurde abfiltriert, mit wenig Diethylether gewaschen und im HV getrocknet. Man erhielt so 1.82 g der Titelverbindung. Durch Einengen der Mutterlauge, Verrühren des Rückstands in Pentan und Abfiltrieren des Feststoffs konnte man zusätzlich 1.13 g Produkt erhalten. Geamtausbeute: 79 % d. Th.
LC-MS (Methode 1): Rₜ = 1.09 min; m/z = 302 (M+H)⁺.
¹H-NMR (400MHz, DMSO-d₆): δ [ppm] = 1.27 (s, 6H), 1.29 (s, 12H), 3.14 (s, 3H), 7.03 (d, 1H), 7.58 (br.s, 1H), 7.62 (br d, 1H).

### Beispiel 69A

### Ethyl-1-[3-hydroxy-1-methyl-2-oxo-3-(trifluormethyl)-2,3-dihydro-1H-indol-5-yl]-2,4-dioxo-1,2,3,4-tetrahydropyrimidin-5-carboxylat (Racemat)

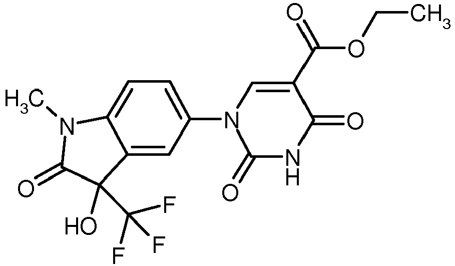

1.00 g (4.06 mmol) 5-Amino-3-hydroxy-1-methyl-3-(trifluormethyl)-1,3-dihydro-2H-indol-2-on aus Beispiel 20A und 1.05 g (4.06 mmol) Ethyl-3-ethoxy-2-[(ethoxycarbonyl)-carbamoyl]acrylat wurden in 100 mL Ethanol 1 h zum Rückfluss erhitzt. Nach Abkühlen auf RT wurden 0.46 mg (4.06 mmol) Kalium-tert.-butylat zugegeben und das Reaktionsgemisch weiter über Nacht bei RT sowie 1 h zum Rückfluss erhitzt. Zur Aufarbeitung wurde die Reaktionsmischung mit IM Salzsäure angesäuert, mit Wasser verdünnt und teilweise eingeengt. Der gebildete Feststoff wurde abfiltriert, mit Wasser gewaschen und über Nacht im Vakuum bei 40°C getrocknet. Man erhielt 1.47 g (88 % d. Th.) der Zielverbindung.
LC-MS (Methode 1): Rₜ = 0.72 min; MS (ESIpos): m/z = 414 (M+H)⁺.
¹H-NMR (400 MHz, DMSO-d₆): δ [ppm] = 1.24 (t, 3H), 3.21 (s, 3H), 4.18 (q, 2H), 7.27 (d, 1H), 7.60 - 7.68 (m, 2H), 7.90 (s, 1H), 8.23 (s, 1H), 11.68 (s, 1H).

### Beispiel 70A

### 3-Hydroxy-1,3-dimethyl-5-nitro-1,3-dihydro-2H-indol-2-on (Racemat)

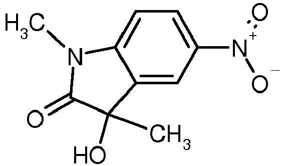

Unter Argon wurden 8.70 g (42.20 mmol) 1-Methyl-5-nitro-1H-indol-2,3-dion [Darstellung siehe: Bioorganic & Medicinal Chemistry, 2006, 14(18), p. 6434-6443] in 200 mL vorgelegt, anschließend bei 0°C 33 mL (46.42 mmol) einer 1.4M Lösung von Magnesiumbromid in Toluol/THF innerhalb von 10 min zugetropft und die Reaktionsmischung 16 h bei RT gerührt. Zur Aufarbeitung wurde das Reaktionsgemisch mit kaltem Wasser versetzt und zweimal mit Dichlormethan extrahiert. Die vereinigten organischen Phasen wurden über Magnesiumsulfat getrocknet, filtriert und eingeengt. Der Rückstand wurde mittels präparativer HPLC (Methode 7) getrennt. Man erhielt 1.41 g (12 % d. Th.) der Titelverbindung.
LC-MS (Methode 5): Rₜ = 0.63 min; MS (ESIpos): m/z = 223 (M+H)⁺.
¹H-NMR (400 MHz, DMSO-d₆): δ [ppm] = 1.46 (s, 3H), 3.19 (s, 3H), 3.22 (s, 1H), 7.24 (d, 1H), 8.19 (d, 1H), 8.30 (dd, 1H).

### Beispiel 71A

### 5-Amino-3-hydroxy-1,3-dimethyl-1,3-dihydro-2H-indol-2-on (Racemat)

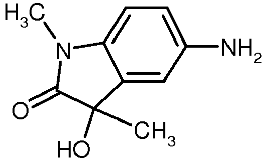

Darstellung und Reinigung der Zielverbindung erfolgten analog zum Beispiel 33A mit einer Reaktionsdauer von 4h. Ausgehend von 1.40 g (6.30 mmol) 3-Hydroxy-1,3-dimethyl-5-nitro-1,3-dihydro-2H-indol-2-on aus Beispiel 70A erhielt man nach zusätzlicher Reinigung mittels präparativer HPLC (Methode 24) 1.15 g (95 % d. Th.) der Titelverbindung.
LC-MS (Methode 2): Rₜ = 0.48 min; MS (ESIpos): m/z = 193 (M+H)⁺.
¹H-NMR (400 MHz, DMSO-d₆): δ [ppm] = 1.39 (s, 3H), 3.11 (s, 3H), 7.08 (d, 1H), 7.25 - 7.32 (m, 2H).

### Beispiel 72A

### Ethyl-1-(3-hydroxy-1,3-dimethyl-2-oxo-2,3-dihydro-1H-indol-5-yl)-2,4-dioxo-1,2,3,4-tetrahydropyrimidin-5-carboxylat (Racemat)

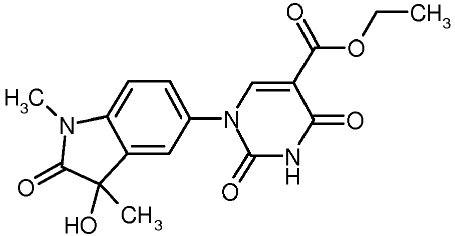

In 15 mL Ethanol wurden 674 mg (2.60 mmol) Ethyl-3-ethoxy-2-[(ethoxycarbonyl)carbamoyl]acrylat sowie 500 mg (2.60 mmol) 5-Amino-3-hydroxy-1,3-dimethyl-1,3-dihydro-2H-indol-2-on aus Beispiel 71A vorgelegt und die Mischung 1.5 h zum Rückfluss erhitzt. Danach wurden bei RT 292 mg (2.60 mmol) Kalium-tert.-butylat zugefügt und die Reaktionsmischung 10 h zum Rückfluss erhitzt. Zur Aufarbeitung wurde das Reaktionsgemisch bei RT mit IN Salzsäure angesäuert und zweimal mit Essigsäureethylester extrahiert. Die vereinigten organischen Phasen wurden mit gesättigter Natriumchlorid-Lösung gewaschen, über Magnesiumsulfat getrocknet, filtriert und eingeengt. Der Rückstand wurde in MTBE/Essigsäureethylester verrührt, der entstandene Feststoff abfiltriert und anschließend im Vakuum bei 30°C getrocknet. Der im Filtrat ausgefallene Feststoff wurde abfiltriert, mit Wasser gewaschen und im Vakuum getrocknet. Man erhielt insgesamt 218 mg (22 % d. Th.) der Titelverbindung.
LC-MS (Methode 5): Rₜ = 0.53 min; MS (ESIpos): m/z = 360 (M+H)⁺.
¹H-NMR (400 MHz, DMSO-d₆): δ [ppm] = 1.23 (t, 3H), 1.41 (s, 3H), 3.14 (s, 3H), 4.18 (q, 2H), 6.10 (s, 1H), 7.11 (d, 1H), 7.43 (d, 1H), 7.52 (s, 1H), 8.20 (s, 1H), 11.67 (s, 1H).

### Beispiel 73A

### Ethyl-1-[3-fluor-1-methyl-2-oxo-3-(trifluormethyl)-2,3-dihydro-1H-indol-5-yl]-2,4-dioxo-1,2,3,4-tetrahydropyrimidin-5-carboxylat (Racemat)

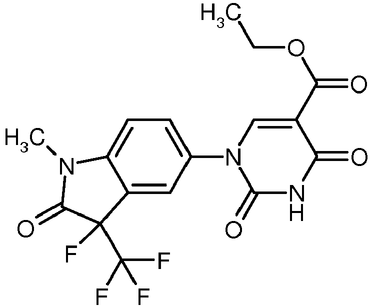

In 4.74 mL Dichlormethan wurden unter Argon 200 mg (0.48 mmol) Ethyl-1-[3-hydroxy-1-methyl-2-oxo-3-(trifluormethyl)-2,3-dihydro-1H-indol-5-yl]-2,4-dioxo-1,2,3,4-tetrahydropyrimi-din-5-carboxylat aus Beispiel 69A bei -78°C vorgelegt. Anschließend wurden 128 µL (0.97 mmol) Diethylaminoschwefeltrifluorid zugetropft, die Mischung auf RT gebracht und weiter über Nacht gerührt. Danach wurde erneut bei -78°C Diethylaminoschwefeltrifluorid (0.5 eq.) zugefügt und für 1 h bei RT gerührt. Zur Aufarbeitung wurde die Mischung mit Dichlormethan verdünnt, je einmal mit gesättigter Natriumhydrogencarbonatlösung sowie gesättigter Natriumchloridlösung gewaschen, über Magnesiumsulfat getrocknet, filtriert und eingeengt. Man erhielt 191 mg (91 % d. Th.) der Titelverbindung.
LC-MS (Methode 5): Rₜ = 0.83 min; MS (ESIpos): m/z = 416 (M+H)⁺.
¹H-NMR (400 MHz, DMSO-d₆): δ [ppm] = 1.23 (t, 3H), 3.26 (s, 3H), 4.18 (q, 2H), 7.40 (d, 1H), 7.77 - 7.83 (m, 1H), 7.91 (s, 1H), 8.34 (s, 1H), 11.72 (s, 1H).

### Beispiel 74A

### Ethyl-1-(1-methyl-1H-benzotriazol-5-yl)-2,4-dioxo-1,2,3,4-tetrahydropyrimidin-5-carboxylat

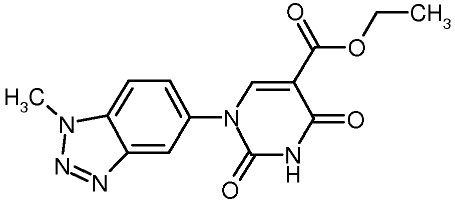

717 mg (2.77 mmol) Ethyl-3-ethoxy-2-[(ethoxycarbonyl)carbamoyl]acrylat sowie 410 mg (2.77 mmol) 1-Methyl-1H-benzotriazol-5-amin [Darstellung siehe: WO 2005/092899, Bsp. 142; Preparation 265] wurden in 21 mL Ethanol für 2 h zum Rückfluss erhitzt. Danach wurden bei RT 311 mg (2.77 mmol) Kalium-tert.-butylat zugefügt und die Reaktionsmischung weitere 3 h zum Rückfluss erhitzt. Zur Aufarbeitung wurde das Reaktionsgemisch mit Wasser versetzt und mit IN Salzsäure angesäuert. Der entstandene Feststoff wurde abfiltriert, mit Essigsäureethylester gewaschen und im Vakuum bei 50°C getrocknet. Man erhielt 659 mg (76 % d. Th.) der Titelverbindung.
LC-MS (Methode 1): Rₜ = 0.59 min; MS (ESIpos): m/z = 316 (M+H)⁺.
¹H-NMR (400 MHz, DMSO-d₆): δ [ppm] = 1.22 (t, 3H), 4.18 (q, 2H), 4.36 (s, 3H), 7.68 (dd, 1H), 7.97 (d, 1H), 8.25 - 8.29 (m, 1H), 8.40 (s, 1H), 11.75 (s, 1H).

### Beispiel 75A

### Ethyl-1-(1-methyl-1H-indazol-5-yl)-2,4-dioxo-1,2,3,4-tetrahydropyrimidin-5-carboxylat

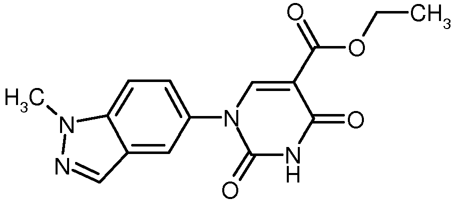

1.76 g (6.79 mmol) Ethyl-3-ethoxy-2-[(ethoxycarbonyl)carbamoyl]acrylat sowie 1.00 g (6.79 mmol) 1-Methyl-1H-indazol-5-amin wurden in 51 mL Ethanol für 2 h zum Rückfluss erhitzt. Danach wurden bei RT 762 mg (6.79 mmol) Kalium-tert.-butylat zugefügt und die Reaktionsmischung weitere 3 h zum Rückfluss erhitzt. Zur Aufarbeitung wurde das Reaktionsgemisch mit Wasser versetzt und mit IN Salzsäure angesäuert. Der entstandene Feststoff wurde abfiltriert, mit Essigsäureethylester/MTBE (1:1) gewaschen und im Vakuum bei 50°C getrocknet. Man erhielt 1.97 g (92 % d. Th.) der Titelverbindung.
LC-MS (Methode 1): Rₜ = 0.62 min; MS (ESIpos): m/z = 315 (M+H)⁺.
¹H-NMR (400 MHz, DMSO-d₆): δ [ppm] = 1.22 (t, 3H), 4.09 (s, 3H), 4.17 (q, 2H), 7.45 - 7.52 (m, 1H), 7.75 (d, 1H), 7.91 (s, 1H), 8.15 (s, 1H), 8.32 (s, 1H), 11.68 (s, 1H).

### Beispiel 76A

### Ethyl-1-(1-methyl-1H-benzimidazol-5-yl)-2,4-dioxo-1,2,3,4-tetrahydropyrimidin-5-carboxylat

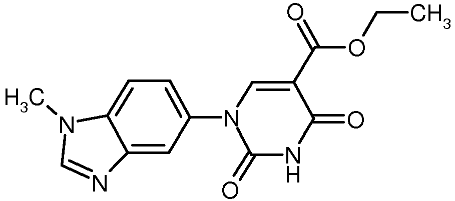

1.76 g (6.79 mmol) Ethyl-3-ethoxy-2-[(ethoxycarbonyl)carbamoyl]acrylat sowie 1.00 g (6.79 mmol) 1-Methyl-1H-benzimidazol-5-amin [Darstellung siehe: US 2008/0090856, Bsp. B23] wurden in 51 mL Ethanol für 2 h zum Rückfluss erhitzt. Danach wurden bei RT 0.76 g (6.79 mmol) Kalium-tert.-butylat zugefügt und die Reak-tionsmischung weitere 3 h zum Rückfluss erhitzt. Zur Aufarbeitung wurde das Reaktionsgemisch mit Wasser versetzt und mit IN Salzsäure angesäuert. Die wässrige Phase wurde eingeengt, mit Dichlormethan/Methanol (1:1) versetzt und der entstandene Feststoff abfiltriert. Das Filtrat wurde eingeengt, mit MTBE/Essigsäureethylester (1:1) versetzt, der entstandene Feststoff abfiltriert, mit Essigsäureethylester gewaschen und anschließend im Vakuum bei 50°C getrocknet. Man erhielt 1.55 g (73 % d. Th.) der Titelverbindung.
LC-MS (Methode 2): Rₜ = 1.00 min; MS (ESIpos): m/z = 315 (M+H)⁺.
¹H-NMR (400 MHz, DMSO-d₆): δ [ppm] = 1.22 (t, 3H), 4.03 (s, 3H), 4.18 (q, 2H), 7.62 - 7.68 (m, 1H), 7.94 - 8.00 (m, 1H), 8.00 - 8.03 (m, 1H), 8.35 (s, 1H), 9.24 (br.s, 1H), 11.73 (s, 1H).

### Beispiel 77A

### 1-Methyl-5-nitro-2-(trichlormethyl)-1H-benzimidazol

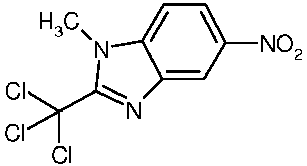

Zu einer auf 0°C gekühlten Suspension von 1.50 g (8.97 mmol) N¹-Methyl-4-nitrobenzol-1,2-diamin in 40.0 mL Eisessig wurden 1.22 mL (9.87 mmol) 2,2,2-Trichloracetimid-säuremethylester getropft und die Mischung 3 h bei RT gerührt. Zur Aufarbeitung wurde die Mischung auf Wasser gegeben, der Feststoff abfiltriert und mit Wasser nachgewaschen. Der Feststoff wurde bei 50°C im Hochvakuum getrocknet. Man erhielt 2.50 g (93 % d. Th.) der Titelverbindung.
LC-MS (Methode 1): Rₜ = 1.06 min; MS (ESIpos): m/z = 296 (M+H)⁺.
¹H-NMR (400 MHz, DMSO-d₆): δ [ppm] = 4.20 (s, 3H), 8.00 (d, 1H), 8.35 (dd, 1H), 8.75 (d, 1H).

### Beispiel 78A

### Ethyl-1-methyl-5-nitro-1H-benzimidazol-2-carboxylat

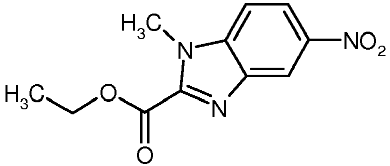

In 24.0 mL Ethanol wurden 2.50 g (8.48 mmol) 1-Methyl-5-nitro-2-(trichlormethyl)-1H-benzimi-dazol aus Beispiel 77A vorgelegt, anschließend 4.75 g (27.98 mmol) Silber(I)nitrat zugefügt und die Mischung über Nacht zum Rückfluss erhitzt. Zur Aufarbeitung wurde die Mischung eingeengt und der Rückstand mit IM Salzsäure sowie Essigsäureethylester versetzt. Danach wurde das Gemisch über Celite filtriert und mit Essigsäureethylester nachgewaschen. Die organische Phase wurde über Mangesiumsulfat getrocknet, filtriert und eingeengt. Der erhaltene Rückstand wurde mit MTBE verrührt, der gebildete Feststoff abfiltriert und mit MTBE nachgewaschen. Der Feststoff wurde im Hochvakuum getrocknet. Man erhielt erhielt 0.32 g (15 % d. Th.) der Titelverbindung.
LC-MS (Methode 5): Rₜ = 0.86 min; MS (ESIpos): m/z = 250 (M+H)⁺.
¹H-NMR (400 MHz, DMSO-d₆): δ [ppm] = 1.39 (t, 3H), 4.15 (s, 3H), 4.44 (q, 2H), 7.99 (d, 1H), 8.28 - 8.35 (m, 1H), 8.69 - 8.74 (m, 1H).

### Beispiel 79A

### 1-Methyl-5-nitro-1H-benzimidazol-2-carboxamid

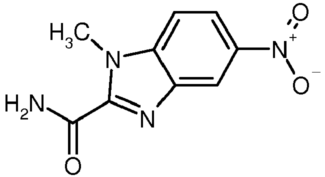

In 10.0 mL THF wurden 745 mg (2.99 mmol) Ethyl-1-methyl-5-nitro-1H-benzimidazol-2-carboxylat aus Beispiel 78A vorgelegt, anschließend 27.4 mL (54.90 mmol) 25 %ige wässrige Ammoniaklösung zugefügt und die Mischung 2.5 h bei RT gerührt. Zur Aufarbeitung wurde das Reaktionsgemisch mit Wasser versetzt, der entstandene Feststoff abfiltriert, mit Wasser nachgewaschen und im Hochvakuum bei 50°C getrocknet. Man erhielt 512 mg (88 %-ige Reinheit, 68 % d. Th.) der Titelverbindung.
LC-MS (Methode 5): Rₜ = 0.64 min; MS (ESIpos): m/z = 221 (M+H)⁺.
¹H-NMR (400 MHz, DMSO-d₆): δ [ppm] = 4.18 (s, 3H), 7.94 (d, 1H), 8.04 (br.s, 1H), 8.28 (dd, 1H), 8.46 (br.s, 1H), 8.60 (d, 1H).

### Beispiel 80A

### 5-Amino-1-methyl-1H-benzimidazol-2-carboxamid

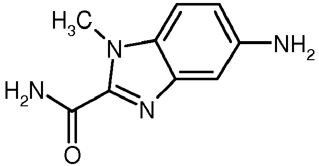

In 16 mL Ethanol wurden 512 mg (2.33 mmol) der Nitroverbindung aus Beispiel 79A vorgelegt, danach 74 mg (0.07 mmol) Palladium (10 %-ig auf Aktivkohle) zugefügt und die Mischung über Nacht bei Wasserstoff-Normaldruck hydriert. Anschließend wurde die Reaktionsmischung über Kieselgur filtriert, der Rückstand mit Ethanol nachgewaschen und das Filtrat eingeengt. Man erhielt 440 mg (90 %-ige Reinheit, 90 % d. Th.) der Zielverbindung.
LC-MS (Methode 5): Rₜ = 0.19 min; MS (ESIpos): m/z = 191 (M+H)⁺.
¹H-NMR (400 MHz, DMSO-d₆): δ [ppm] = 4.02 (s, 3H), 4.88 - 4.96 (m, 2H), 6.73 - 6.77 (m, 1H), 6.77 - 6.81 (m, 1H), 7.31 (d, 1H), 7.64 (br.s, 1H), 8.06 (br.s, 1H).

### Beispiel 81A

### Ethyl-1-(2-carbamoyl-1-methyl-1H-benzimidazol-5-yl)-2,4-dioxo-1,2,3,4-tetrahydropyrimidin-5-carboxylat

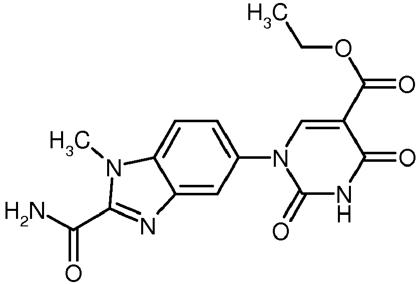

Darstellung und Reinigung der Zielverbindung erfolgte analog zum Beispiel 31A. Ausgehend von 440 mg (2.31 mmol) 5-Amino-1-methyl-1H-benzimidazol-2-carboxamid aus Beispiel 80A und 600 mg (2.31 mmol) Ethyl-3-ethoxy-2-[(ethoxycarbonyl)carbamoyl]-acrylat erhielt man 158 mg (87 %-ige Reinheit 17 % d. Th.) der Titelverbindung. Das Rohprodukt wurde ohne weitere Reinigung umgesetzt.
LC-MS (Methode 1): Rₜ = 0.55 min; MS (ESIpos): m/z = 358 (M+H)⁺.
¹H-NMR (400 MHz, DMSO-d₆): δ [ppm] = 1.22 (t, 3H), 4.13 - 4.21 (m, 5H), 7.46 - 7.53 (m, 1H), 7.80 (d, 1H), 7.89 - 7.93 (m, 2H), 8.31 - 8.37 (m, 2H), 11.69 (s, 1H).

### Beispiel 82A

### Ethyl-1-(1-methyl-1H-benzimidazol-6-yl)-2,4-dioxo-1,2,3,4-tetrahydropyrimidin-5-carboxylat

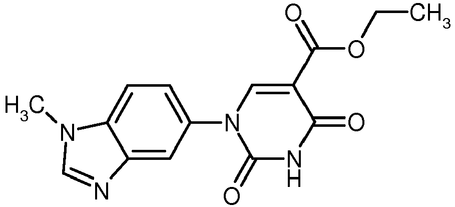

Darstellung und Reinigung der Zielverbindung erfolgte analog zum Beispiel 31A. Ausgehend von 1.00 g (6.79 mmol) 1-Methyl-1H-benzimidazol-6-amin und 1.76 g (6.79 mmol) Ethyl-3-ethoxy-2-[(ethoxycarbonyl)carbamoyl]-acrylat erhielt man 1.03 g (48 % d. Th.) der Titelverbindung.
LC-MS (Methode 1): Rₜ = 0.36 min; MS (ESIpos): m/z = 315 (M+H)⁺.
¹H-NMR (400 MHz, DMSO-d₆): δ [ppm] = 1.23 (t, 3H), 4.01 (s, 3H), 4.19 (q, 2H), 7.61 - 7.67 (m, 1H), 7.90 - 7.96 (m, 1H), 8.15 (s, 1H), 8.37 (s, 1H), 9.29 - 9.37 (m, 1H), 11.80 (s, 1H).

### Beispiel 83A

### 1,3-Dihydro-2,1,3-benzothiadiazol-2,2-dioxid

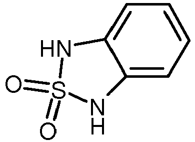

In 14 mL Pyridin wurden 1.00 g (9.25 mmol) 1,2-Phenylendiamin sowie 2.67 g (27.74 mmol) Sulfamid vorgelegt und die Mischung über Nacht bei 130°C gerührt. Zur Aufarbeitung wurde das Reaktionsgemisch im Vakuum eingeengt und der Rückstand mittels Flash-Kieselgelchromatographie (Cyclohexan/Ethylactet-Gradient 7:1, 5:1) getrennt. Man erhielt 659 mg (42 % d. Th.) der Titelverbindung.
LC-MS (Methode 5): Rₜ = 0.51 min; MS (ESIpos): m/z = 171 (M+H)⁺.
¹H-NMR (400 MHz, DMSO-d₆): δ [ppm] = 6.76 - 6.83 (m, 2H), 6.85 - 6.91 (m, 2H), 10.95 (br.s, 2H).

### Beispiel 84A

### 1,3-Dimethyl-1,3-dihydro-2,1,3-benzothiadiazol-2,2-dioxid

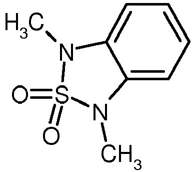

Unter Argon wurden 1.54 g (38.45 mmol) Natriumhydrid (60 %ig in Mineralöl) in 41 mL DMF vorgelegt, anschließend bei 0°C eine Lösung von 2.62 g (15.38 mmol) 1,3-Dihydro-2,1,3-benzothiadiazol-2,2-dioxid aus Beispiel 83A in 5 mL DMF zugetropft und die Reaktionsmischung 30 min bei 0°C gerührt. Danach wurden 2.39 mL (38.45 mmol) Iodmethan zugetropft, die Reaktionsmischung auf RT gebracht und 1 h gerührt. Zur Aufarbeitung wurde bei 0°C mit Wasser (200 mL) versetzt und dreimal mit Essigsäureethylester extrahiert. Die vereinigten organischen Phasen wurden mit Wasser sowie gesättigter Natriumchlorid-Lösung gewaschen, über Magnesiumsulfat getrocknet, filtriert und eingeengt. Der Rückstand wurde in MTBE ausgerührt, der Feststoff abfiltriert, mit MTBE gewaschen und im Hochvakuum getrocknet. Man erhielt 1.89 g (62 % d. Th.) der Titelverbindung. Das Rohprodukt wurde ohne weitere Reinigung umgesetzt.
LC-MS (Methode 1): Rₜ = 0.78 min; MS (ESIpos): m/z = 199 (M+H)⁺.
¹H-NMR (400 MHz, DMSO-d₆): δ [ppm] = 3.23 (s, 6H), 6.98 - 7.05 (m, 4H).

### Beispiel 85A

### 1,3-Dimethyl-5-nitro-1,3-dihydro-2,1,3-benzothiadiazol-2,2-dioxid

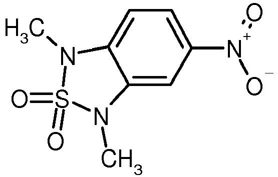

1.88 g (9.52 mmol) 1,3-Dimethyl-1,3-dihydro-2,1,3-benzothiadiazol-2,2-dioxid aus Beispiel 84A wurden in 8 mL Essigsäure vorgelegt, anschließend 0.60 mL (9.52 mmol) konz. Salpetersäure zugetropft und das Reaktionsgemisch 1 h bei RT gerührt. Zur Aufarbeitung wurde das Reaktionsgemisch auf Eiswasser gegeben, der ausgefallene Feststoff abgesaugt, mit Wasser gewaschen und im Vakuum bei 50°C getrocknet. Man erhielt 2.17 g (93 % d. Th.) der Titelverbindung.
LC-MS (Methode 1): Rₜ = 0.81 min; MS (ESIpos): m/z = 244 (M+H)⁺.
¹H-NMR (400 MHz, DMSO-d₆): δ [ppm] = 3.37 - 3.42 (m, 6H), 7.23 (d, 1H), 7.92 (d, 1H), 8.03 (dd, 1H).

### Beispiel 86A

### 1,3-Dimethyl-1,3-dihydro-2,1,3-benzothiadiazol-5-amin-2,2-dioxid

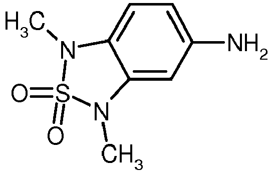

Darstellung und Reinigung der Zielverbindung erfolgten analog zum Beispiel 33A mit einer Reaktionsdauer von 16h. Ausgehend von 2.17 g (8.92 mmol) 1,3-Dimethyl-5-nitro-1,3-dihydro-2,1,3-benzothiadiazol-2,2-dioxid aus Beispiel 85A erhielt man 851 mg (44 % d. Th.) der Titelverbindung.
LC-MS (Methode 1): Rₜ = 0.31 min; MS (ESIpos): m/z = 214 (M+H)⁺.
¹H-NMR (400 MHz, DMSO-d₆): δ [ppm] = 3.07 (s, 3H), 3.10 (s, 3H), 4.89 - 4.99 (m, 2H), 6.18 - 6.24 (m, 2H), 6.70 (d, 1H).

### Beispiel 87A

### Ethyl-1-(1,3-dimethyl-2,2-dioxido-1,3-dihydro-2,1,3-benzothiadiazol-5-yl)-2,4-dioxo-1,2,3,4-tetrahydropyrimidin-5-carboxylat

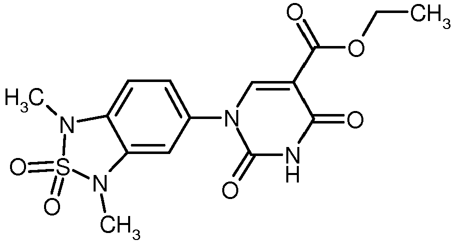

In 30 mL Ethanol wurden 1.03 g (3.99 mmol) Ethyl-3-ethoxy-2-[(ethoxycarbonyl)carbamoyl]acrylat sowie 851 mg (3.99 mmol) 1,3-Dimethyl-1,3-dihydro-2,1,3-benzothiadiazol-5-amin-2,2-dioxid aus Beispiel 86A vorgelegt und 2 h zum Rückfluss erhitzt. Anschließend wurden bei RT 448 mg (3.99 mmol) Kalium-tert.-butylat zugefügt und die Mischung 16 h bei RT sowie 5 h bei Rückfluss gerührt. Zur Aufarbeitung wurde das Reaktionsgemisch mit IN Salzsäure angesäuert, der entstandene Feststoff abfiltriert, mit Wasser sowie Essigsäureethylester gewaschen und anschließend im Vakuum bei 50°C getrocknet. Man erhielt 1.36 g (84 %-ige Reinheit, 75 % d. Th.) der Titelverbindung.
LC-MS (Methode 1): Rₜ = 0.74 min; MS (ESIpos): m/z = 381 (M+H)⁺.
¹H-NMR (400 MHz, DMSO-d₆): δ [ppm] = 1.20 - 1.24 (m, 3H), 3.25 (s, 3H), 3.30 (s, 3H), 4.17 (q, 2H), 7.12 - 7.19 (m, 2H), 7.24 - 7.28 (m, 1H), 8.29 (s, 1H), 11.71 (br.s, 1H).

### Beispiel 88A

### Ethyl-1-(1,3-benzothiazol-6-yl)-2,4-dioxo-1,2,3,4-tetrahydropyrimidin-5-carboxylat

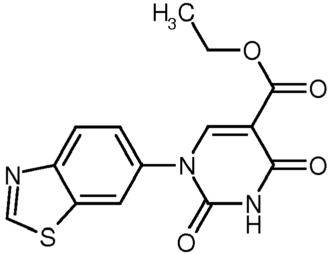

Darstellung der Zielverbindung erfolgte analog zum Beispiel 76A unter Verwendung von 1.00 g (6.65 mmol) 1,3-Benzothiazol-6-amin und 1.72 g (6.65 mmol) Ethyl-3-ethoxy-2-[(ethoxycarbonyl)carbamoyl]acrylat. Zur Aufarbeitung wurde die Reaktionsmischung mit Wasser und IN Salzsäure versetzt, der ausgefallene Feststoff abgesaugt, mit Essigsäureethylester gewaschen und bei 50°C im Vakuum getrocknet. Man erhielt 1.85 g (87 % d. Th.) der Titelverbindung.
LC-MS (Methode 1): Rₜ = 0.61 min; MS (ESIpos): m/z = 318 (M+H)⁺.
¹H-NMR (400 MHz, DMSO-d₆): δ [ppm] = 1.22 (t, 3H), 4.18 (q, 2H), 7.67 (dd, 1H), 8.20 (d, 1H), 8.36 (d, 1H), 8.42 (s, 1H), 9.53 (s, 1H), 11.76 (s, 1H).

### Beispiel 89A

### 1-Methyl-6-nitro-3,4-dihydrochinolin-2(1H)-on

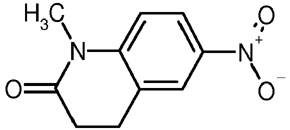

In 148 mL THF wurden unter Argon 1.00 g (5.20 mmol) 6-Nitro-3,4-dihydrochinolin-2(1H)-on [Darstellung siehe: WO 2006/71940, 416] vorgelegt, anschließend 229 mg (5.72 mmol) Natriumhydrid (60 %-ig in Mineralöl) bei 0°C zugefügt und 30 min gerührt. Danach wurden 0.36 mL (5.72 mmol) Iodmethan zugetropft und die Reaktionsmischung über Nacht bei RT gerührt. Zur Aufarbeitung wurde das Reaktionsgemisch mit Essigsäureethylester verdünnt, die organische Phase zweimal mit gesättigter Natriumhydrogencarbonat-Lösung gewaschen, über Magnesiumsulfat getrocknet, filtriert und eingeengt. Der Rückstand wurde mit Ethanol verrührt, der Feststoff abfiltriert, mit Ethanol gewaschen und über Nacht im Hochvakuum getrocknet. Man erhielt 535 mg (50 % d. Th.) der Titelverbindung. Das Rohprodukt wurde ohne weitere Reinigung umgesetzt.
LC-MS (Methode 3): Rₜ = 0.88 min; MS (ESIpos): m/z = 207 (M+H)⁺.
¹H-NMR (400 MHz, DMSO-d₆): δ [ppm] = 2.60 - 2.66 (m, 2H), 3.02 (t, 2H), 3.31 (s, 3H), 7.26 - 7.32 (m, 1H), 8.12 - 8.20 (m, 2H).

### Beispiel 90A

### 6-Amino-1-methyl-3,4-dihydrochinolin-2(1H)-on

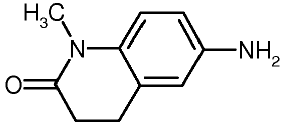

In 36 mL Ethanol wurden 1.10 g (5.33 mmol) der Nitroverbindung aus Beispiel 89A vorgelegt, danach 170 mg (0.16 mmol) Palladium (10 %-ig auf Aktivkohle) zugefügt und die Mischung über Nacht bei Wasserstoff-Normaldruck hydriert. Anschließend wurde die Reaktionsmischung über Kieselgur filtriert, der Rückstand mit Ethanol nachgewaschen und das Filtrat eingeengt. Man erhielt 936 mg (99 % d. Th.) der Zielverbindung. Das Rohprodukt wurde ohne weitere Reinigung umgesetzt.
LC-MS (Methode 2): Rt = 0.73 min; MS (ESIpos): m/z = 177 (M+H)⁺.
¹H-NMR (400 MHz, DMSO-d₆): δ [ppm] = 2.43 (t, 2H), 2.69 (t, 2H), 3.16 (s, 3H), 4.79 - 4.90 (m, 2H), 6.41 - 6.47 (m, 2H), 6.77 (d, 1H).

### Beispiel 91A

### Ethyl-1-(1-methyl-2-oxo-1,2,3,4-tetrahydrochinolin-6-yl)-2,4-dioxo-1,2,3,4-tetrahydropyrimidin-5-carboxylat

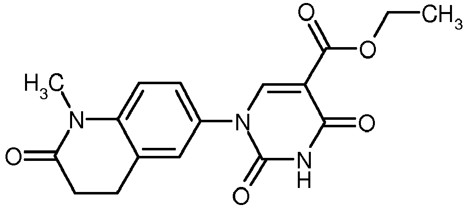

Darstellung und Reinigung der Zielverbindung erfolgte analog zum Beispiel 31A. Ausgehend von 935 mg (5.30 mmol) 6-Amino-1-methyl-3,4-dihydrochinolin-2(1H)-on aus Beispiel 90A [Synthese beschrieben in: WO 2003/72553, Seite 150-151] und 1.37 g (5.30 mmol) Ethyl-3-ethoxy-2-[(ethoxycarbonyl)carbamoyl]acrylat erhielt man 1.33 g (73 % d. Th.) der Titelverbindung.
LC-MS (Methode 1): Rₜ = 0.65 min; MS (ESIpos): m/z = 344 (M+H)⁺.
¹H-NMR (400 MHz, DMSO-d₆): δ [ppm] = 1.22 (t, 3H), 2.56 - 2.61 (m, teilweise durch DMSO-Signal verdeckt), 2.87 - 2.94 (m, 2H), 3.28 (s, 3H), 4.17 (q, 2H), 7.20 (d, 1H), 7.35 - 7.41 (m, 2H), 8.25 (s, 1H), 11.68 (s, 1H).

### Beispiel 92A

### Ethyl-1-(1-methyl-2-oxo-1,4-dihydro-2H-3,1-benzoxazin-6-yl)-2,4-dioxo-1,2,3,4-tetrahydro-pyrimidin-5-carboxylat

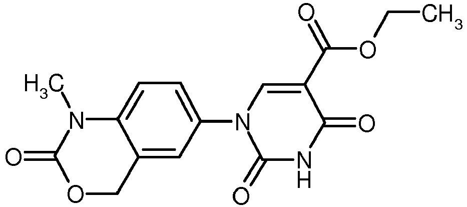

In 20 mL Ethanol wurden 916 mg (3.53 mmol) Ethyl-3-ethoxy-2-[(ethoxycarbonyl)carbamoyl]acrylat sowie 630 mg (3.53 mmol) 6-Amino-1-methyl-1,4-dihydro-2H-3,1-benzoxazin-2-on [Darstellung siehe: WO 2007/93904; p. 22, step 3] vorgelegt und 1 h zum Rückfluss erhitzt. Danach wurden bei RT 397 mg (3.53 mmol) Kalium-tert.-butylat zugefügt und die Reaktionsmischung 16 h bei RT sowie 3 h bei Rückfluss gerührt. Zur Aufarbeitung wurde das Gemisch bei RT mit IN Salzsäure angesäuert, der entstandene Feststoff abfiltriert, mit MTBE gewaschen und anschließend im Vakuum bei 50°C getrocknet. Man erhielt 1.11 g (90 % d. Th.) der Titelverbindung.
LC-MS (Methode 1): Rₜ = 0.58 min; MS (ESIpos): m/z = 346 (M+H)⁺.
¹H-NMR (400 MHz, DMSO-d₆): δ [ppm] = 1.22 (t, 3H), 3.3 (s, teilweise durch Wasser-Signal verdeckt), 4.17 (q, 2H), 5.29 (s, 2H), 7.21 (d, 1H), 7.40 - 7.44 (m, 1H), 7.48 - 7.54 (m, 1H), 8.27 (s, 1H), 11.70 (s, 1H).

### Beispiel 93A

### Ethyl-1-(4-methylchinolin-7-yl)-2,4-dioxo-1,2,3,4-tetrahydropyrimidin-5-carboxylat

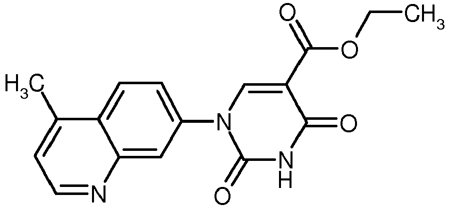

Darstellung und Reinigung der Zielverbindung erfolgten analog zum Beispiel 31A. Ausgehend von 987 mg (5.31 mmol) 4-Methylchinolin-7-amin [Darstellung siehe: Nasr, M. et al., J. Med. Chem. 1988, vol. 31 (7), p. 1347 - 1351] und 1.37 g (5.31 mmol) Ethyl-3-ethoxy-2-[(ethoxycarbonyl)carbamoyl]acrylat erhielt man 745 mg (43 % d. Th.) der Titelverbindung.
LC-MS (Methode 1): Rₜ = 0.63 min; MS (ESIpos): m/z = 326 (M+H)⁺.
¹H-NMR (400 MHz, DMSO-d₆): δ [ppm] = 1.24 (t, 3H), 2.88 (s, 3H), 4.19 (q, 2H), 7.79 (d, 1H), 7.93 (dd, 1H), 8.32 (d, 1H), 8.41 (d, 1H), 8.50 (s, 1H), 9.06 (d, 1H), 11.82 (s, 1H).

### Beispiel 94A

### Ethyl-1-(imidazo[1,2-a]pyridin-7-yl)-2,4-dioxo-1,2,3,4-tetrahydropyrimidin-5-carboxylat

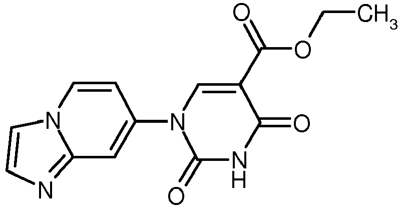

Darstellung und Reinigung der Zielverbindung erfolgten analog zum Beispiel 31A. Ausgehend von 500 mg (3.75 mmol) Imidazo[1,2-a]pyridin-7-amin [Darstellung siehe: Tetrahedron, 2002, vol. 58 (2), p. 295-308] und 973 mg (3.75 mmol) Ethyl-3-ethoxy-2-[(ethoxycarbonyl)carbamoyl]acrylat erhielt man 1.11 g (94% d. Th.) der Titelverbindung.
LC-MS (Methode 1): Rₜ = 0.19 min; MS (ESIpos): m/z = 301 (M+H)⁺.
¹H-NMR (400 MHz, DMSO-d₆): δ [ppm]= 1.24 (t, 3H), 4.21 (q, 2H), 7.60 (d, 1H), 8.21 (d, 2H), 8.39 (s, 1H), 8.48 (s, 1H), 8.96 (d, 1H), 11.91 (s, 1H).

### Beispiel 95A

### Ethyl-1-(6-fluor-1,3-dimethyl-2-oxo-2,3-dihydro-1H-benzimidazol-5-yl)-2,4-dioxo-1,2,3,4-tetrahydropyrimidin-5-carboxylat

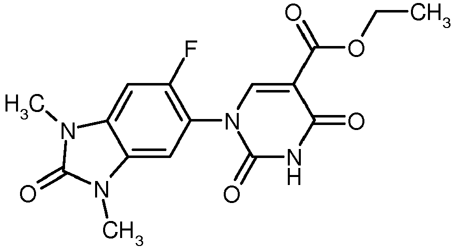

Die Darstellung der Zielverbindung erfolgte analog zum Beispiel 31A unter Verwendung von 660 mg (3.38 mmol) 5-Amino-6-fluor-1,3-dimethyl-1,3-dihydro-2H-benzimidazol-2-on aus Beispiel 19A und 877 mg (3.38 mmol) Ethyl-3-ethoxy-2-[(ethoxycarbonyl)-carbamoyl]acrylat. Das erhaltene Rohprodukt wurde mittels Flash-Kieselgelchromatographie (Dichlormethan/ Methanol-Gradient 54:1 - 20:1) gereinigt und man erhielt 437 mg (36 % d. Th.) der Titelverbindung.
LC-MS (Methode 1): Rₜ = 0.62 min; MS (ESIpos): m/z = 363 (M+H)⁺.
¹H-NMR (400 MHz, DMSO-d₆): δ [ppm]= 1.23 (t, 3H), 3.32 (s, 3H), 3.36 (s, 3H), 4.18 (q, 2H), 7.40 (d, 1H), 7.48 (d, 1H), 8.40 (s, 1H), 11.85 (s, 1H).

### Beispiel 96A

### (3-Chlor-4-methyl-2-thienyl)methanol

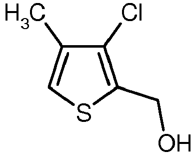

Zu einer Lösung von Boran-Tetrahydrofurankomplex (1M in THF, 3.40 ml, 3.40 mmol) wurden bei RT unter Argon 200 mg (1.13 mmol) 3-Chlor-4-methylthiophen-2-carbonsäure portionsweise hinzugefügt und die Reak-tionsmischung 1h bei RT gerührt. Anschließend wurde das Reaktionsgemisch vorsichtig IN Salzsäure bis zum Ende der Gasentwicklung zugegeben. Die gesamte Mischung wurde per präparativer HPLC (Methode 8) getrennt. Man erhielt 115 mg (62 % d. Th.) der Titelverbindung.
GC-MS (Methode 6): Rₜ = 4.00 min; EI⁺: m/z = 162 (M)⁺
¹H-NMR (400MHz, DMSO-d₆): δ [ppm]= 2.12 (s, 3H), 4.58 (d, 2H), 5.57 (t, 1H), 7.25 (s, 1H).

### Beispiel 97A

### 4,6-Difluorindan-1-ol (Racemat)

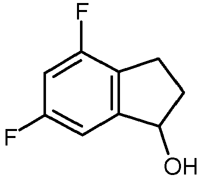

146.3 mg (3.87 mmol) Natriumborhydrid wurden zu einer Lösung von 1.00 g (5.95 mmol) 4,6-Difluor-2,3-dihydro-1H-inden-1-on in 15 ml Ethanol bei RT zugegeben und die Reaktionsmischung über Nacht bei RT gerührt. Das Gemisch wurde mit Essigsäureethylester und Wasser versetzt und gut geschüttelt. Die organische Phase wurde abgetrennt, mit einer gesättigten Ammoniumchloridlösung und einer gesättigten Natriumchlorid-Lösung gewaschen, über Magnesiumsulfat getrocknet und am Rotationsverdampfer vom Lösungsmittel befreit. Der Rückstand wurde kurz am HV getrocknet. Man erhielt 950 mg (94 % d. Th.) der Titelverbindung.
GC-MS (Methode 6): Rₜ = 3.35 min; MS (CI-pos): m/z = 170 (M)⁺.
¹H-NMR (400MHz, DMSO-d₆): δ [ppm] = 1.78 - 1.88 (m, 1H), 2.35 - 2.44 (m, 1H), 2.67 (dt, 1H), 2.90 (ddd, 1H), 5.05 (q, 1H), 5.49 (d, 1H), 6.99 (dd, 1H), 7.04 (td, 1H).

### Beispiel 98A

### 6-Fluor-4-(trifluormethyl)indan-1-ol (Racemat)

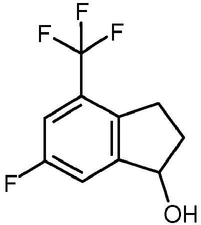

23.3 mg (0.62 mmol) Natriumborhydrid wurden zu einer Lösung von 207 mg (0.95 mmol) 6-Fluor-4-(trifluormethyl)-2,3-dihydro-1H-inden-1-on (Herstellung: siehe US2011/53974, Page 77, Example 61C) in 6 ml Ethanol bei RT zugegeben und die Reaktionsmischung über Nacht bei RT gerührt. Das Gemisch wurde mit Essigsäureethylester und IN Salzsäure versetzt und gut geschüttelt. Die organische Phase wurde abgetrennt, mit IN Salzsäure und dann mit einer gesättigten Natriumchlorid Lösung gewaschen, über Natriumsulfat getrocknet und am Rotationsverdampfer vollständig vom Lösungsmittel befreit. Man erhielt 203 mg (97 % d. Th.) der Titelverbindung.
GC-MS (Methode 6): Rₜ = 3.19 min; MS (CI-pos): m/z = 220 (M)⁺.
¹H-NMR (400MHz, CD₂Cl₂)): δ [ppm]= 1.84 - 1.96 (m, 1H), 2.43 - 2.54 (m, 1H), 2.82 (dt, 1H), 3.02 - 3.14 (m, 1H), 5.14 (t, 1H), 7.17 (d, 1H), 7.21 (d, 1H).

### Beispiel 99A

### 7-(Trifluormethyl)-2,3-dihydro-1-benzofuran-3-ol (Racemat)

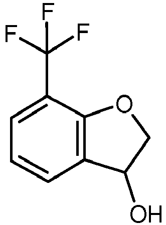

Analog zu Beispiel 98A wurden 388 mg (1.92 mmol) 7-(Trifluormethyl)-2,3-dihydro-1-benzofuran-3-on (Herstellung: siehe US 2011/53974, Page 56, Example 47E) mit Natriumborhydrid reduziert. Man erhielt 210 mg (51 % d. Th.) der Titelverbindung.
GC-MS (Methode 6): Rₜ = 3.80 min; MS (CI-pos): m/z = 204 (M)⁺.
¹H-NMR (400MHz, DMSO-d₆): δ [ppm] = 4.38 (dd, 1H), 4.66 (dd, 1H), 5.33 (dd, 1H), 5.72 - 5.91 (br. m, 1H), 7.07 (t, 1H), 7.52 (d, 1H), 7.66 (d, 1H).

### Beispiel 100A

### 6-Methylindan-1-ol (Racemat)

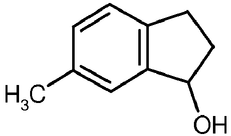

Analog zu Beispiel 97A wurde 1.00 g (6.84 mmol) 6-Methyl-2,3-dihydro-1H-inden-1-on mit Natriumborhydrid reduziert. Man erhielt 950 mg (94 % d. Th.) der Titelverbindung.
GC-MS (Methode 6): Rₜ = 3.89 min; MS (CI-pos): m/z = 148 (M)⁺.
¹H-NMR (400MHz, DMSO-d₆): δ [ppm] = 1.74 (dddd, 1H), 2.25 - 2.35 (m, 1H), 2.64 (dt, 1H), 2.84 (ddd, 1H), 4.99 (q, 1H), 5.14 (d, 1H), 6.99 (br. d, 1H), 7.08 (d, 1H), 7.12 (s, 1H).

### Beispiel 101A

tert-Butyl-6-[5-(ethoxycarbonyl)-2,4-dioxo-3,4-dihydropyrimidin-1(2H)-yl]-3-methyl-2-oxo-2,3-dihydro-1H-benzimidazol-1-carboxylat

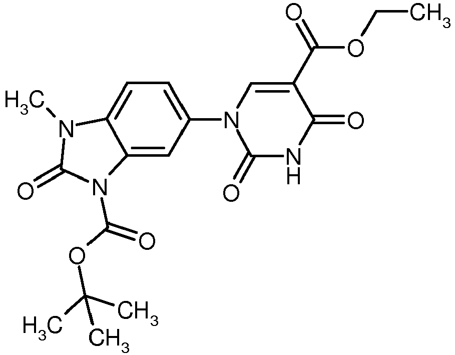

Eine Suspension von 8.00 g (24.2 mmol) der Verbindung aus Beispiel 36A und 30 mg (0.24 mmol) DMAP in 500 ml DMF und 100 ml Dichlormethan wurde bei RT mit 6.12 ml (26.6 mmol) Di-tert-butyldicarbonat versetzt und über Nacht bei RT gerührt. Zur Aufarbeitung wurden 1.6 L Wasser zugegeben und die Mischung dreimal mit Essigsäureethylester extrahiert. Die vereinigten organischen Phasen wurden zweimal mit Wasser gewaschen, über Natriumsulfat getrocknet und am Rotationsverdampfer eingeent. Der Rückstand wurde mit Diethylether verrührt, das ausgefallene Produkt durch Filtration isoliert und im HV getrocknet. Man erhielt 6.00 g (58 % d. Th.) der Titelverbindung.
LC-MS (Methode 4): Rₜ = 1.80 min; m/z = 431 (M+H)⁺.
¹H-NMR (400MHz, DMSO-d₆): δ [ppm] = 1.22 (t, 3H), 1.58 (s, 9H), 4.17 (q, 2H), 7.28 - 7.40 (m, 2H), 7.84 (d, 1H), 8.25 (s, 1H), 11.65 (s, 1H) (Methylgruppe wahrscheinlich unter dem DMSO-Signal).

### Beispiel 102A

### 5-Methoxyindan-1-ol (Racemat)

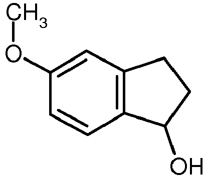

Analog zu Beispiel 97A wurde 1.00 g (6.17 mmol) 5-Methoxy-2,3-dihydro-1H-inden-1-on mit Natriumborhydrid reduziert. Man erhielt 930 mg (80 %-ige Reinheit, 73 % d. Th.) der Titelverbindung.
GC-MS (Methode 6): Rₜ = 4.70 min; MS (CI-pos): m/z = 164 (M)⁺.
¹H-NMR (400MHz, DMSO-d₆): δ [ppm] = 1.71 - 1.82 (m, 1H), 2.25 - 2.34 (m, 1H), 2.61 - 2.72 (m, 1H), 2.83 - 2.93 (m, 1H), 3.72 (s, 3H), 4.97 (q, 1H), 5.05 (d, 1H), 6.71 - 6.76 (m, 1H), 6.77 (br. s, 1H), 7.21 (d, 1H).

### Beispiel 103A

### 4-Methoxyindan-1-ol (Racemat)

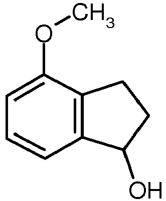

Analog zu Beispiel 97A wurde 1.00 g (6.17 mmol) 4-Methoxy-2,3-dihydro-1H-inden-1-on mit Natriumborhydrid reduziert. Man erhielt 910 mg (90 % d. Th.) der Titelverbindung.
GC-MS (Methode 6): Rₜ = 4.65 min; MS (CI-pos): m/z = 164 (M)⁺.
¹H-NMR (400MHz, DMSO-d₆): δ [ppm] = 1.68 - 1.80 (m, 1H), 2.25 - 2.35 (m, 1H), 2.54 - 2.62 (m, 1H), 2.83 (ddd, 1H), 3.76 (s, 3H), 5.02 (q, 1H), 5.18 (d, 1H), 6.80 (d, 1H), 6.93 (d, 1H), 7.17 (t, 1H).

### Beispiel 104A

### Methyl-(2E)-3-[4-chlor-2-(trifluormethyl)phenyl]acrylat

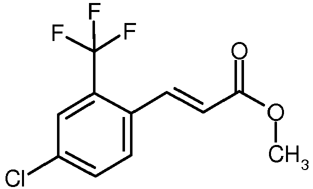

Eine Mischung von 8.00 g (26.1 mmol) 4-Chlor-1-iod-2-(trifluoromethyl)benzol, 3.76 ml (41.8 mmol) Acrylsäuremethylester, 7.47 g (26.9 mmol) Tetra-n-butylammoniumchlorid, 117 mg (0.52 mmol) Palladium(II)acetat und 7.22 g (52.2 mmol) Kaliumcarbonat in 80 ml DMF wurde 3 Tage bei RT gerührt. Das Gemisch wurde mit 1 L Diethylether verdünnt und dreimal mit je 200 ml Wasser gewaschen. Die organische Phase wurde über Natriumsulfat getrocknet und am Rotationsverdampfer eingeengt. Der Rückstand wurde nach einiger Zeit fest. Man erhielt 6.65 g (92 % d. Th.) der Titelverbindung.
¹H-NMR (400MHz, DMSO-d₆): δ [ppm] = 3.76 (s, 3H), 6.81 (d, 1H), 7.79 (dq, 1H), 7.84 (dd, 1H), 7.91 (d, 1H), 8.12 (d, 1H).

### Beispiel 105A

### Methyl-3-[4-chlor-2-(trifluormethyl)phenyl]propanoat

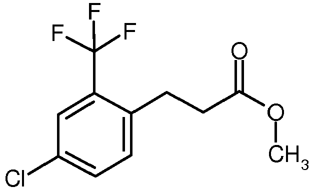

6.65 g (25.1 mmol) der Verbindung aus Beispiel 104A wurden in 250 ml Essigsäureethylester in Gegenwart von 2 g Palladium (10 % auf Kohle) unter Wasserstoff-Normaldruck 2 Tage hydriert. Der Katalysator wurde durch Filtration über Kieselgur abgetrennt und das Filtrat am Rotationsverdampfer eingeengt. Man erhielt 5.26 g der Titelverbindung in ca. 75 %-iger Reinheit (59 % d. Th.).
¹H-NMR (400MHz, DMSO-d₆): δ [ppm] = 2.62 - 2.68 (m, 2H), 3.01 (t, 2H), 3.61 (s, 3H), 7.56 (d, 1H), 7.69 - 7.76 (m, 2H).

### Beispiel 106A

### 3-[4-Chlor-2-(trifluormethyl)phenyl]propansäure

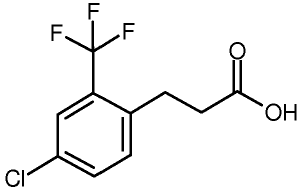

Eine Lösung von 5.26 g (19.7 mmol) der Verbindung aus Beispiel 105A in 150 ml Methanol wurde mit 59.2 ml (59.2 mmol) IM Natronlauge versetzt und 2 h bei RT gerührt. Das Methanol wurde am Rotationsverdampfer entfernt. Der verbleibende wässrige Rückstand wurde mit 600 ml Wasser verdünnt und filtriert. Das Filtrat wurde mit IM Salzsäure angesäuert. Der ausgefallene Feststoff wurde abgesaugt, mit Wasser gewaschen und im HV getrocknet. Man erhielt 4.45 g der Titelverbindung in ca. 90 %-iger Reinheit (80 % d. Th.).
LC-MS (Methode 5): Rₜ = 1.02 min; MS (ESIneg): m/z = 251 (M-H)⁻.
¹H-NMR (400MHz, DMSO-d₆): δ [ppm] = 2.52 - 2.58 (m, 2H, teilweise unter dem DMSO Signal versteckt), 2.97 (t, 2H), 7.56 (d, 1H), 7.68 - 7.76 (m, 2H), 12.32 (br.s, 1H).

### Beispiel 107A

### 6-Chlor-4-(trifluormethyl)indan-1-on

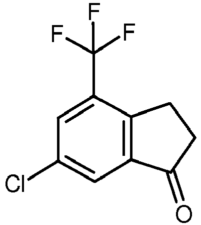

4.08 g (92 %-ige Reinheit, 14.8 mmol) der Verbindung aus Beispiel 106A wurden unter Eiskühlung mit 44 ml Chlorsulfonsäure versetzt und dann bei RT 5 h verrührt. Anschließend wurde die Reaktionsmischung vorsichtig auf 600 g zerstoßenes Eis getropft (sehr exotherm). Das Gemisch wurde dreimal mit Dichlormethan extrahiert. Die vereinigten organischen Phasen wurden zweimal mit einer IM Natriumcarbonatlösung gewaschen, über Natriumsulfat getrocknet und am Rotationsverdampfer eingeengt. Der Rückstand wurde nur kurz im HV getrocknet. Man erhielt 2.38 g der Titelverbindung in ca. 92 %-iger Reinheit (63 % d. Th.).
¹H-NMR (400MHz, DMSO-d₆): δ [ppm] = 2.73 - 2.82 (m, 2H), 3.19 - 3.28 (m, 2H), 7.96 (s, 1H), 8.13 (s, 1H).

### Beispiel 108A

### 6-Chlor-4-(trifluormethyl)indan-1-ol (Racemat)

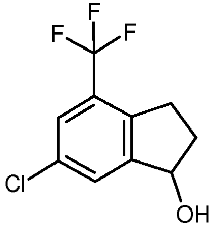

Analog zu Beispiel 98A wurden 2.38 g (10.1 mmol) 6-Chlor-4-(trifluormethyl)indan-1-on aus Beispiel 107A mit Natriumborhydrid reduziert. Man erhielt 1.97 g (82 % d. Th.) der Titelverbindung.
¹H-NMR (400MHz, DMSO-d₆): δ [ppm] = 1.79 - 1.92 (m, 1H), 2.35 - 2.47 (m, 1H), 2.84 (dt, 1H), 2.98 - 3.10 (m, 1H), 5.09 (q, 1H), 5.58 (d, 1H), 7.64 (br. d, 2H).

### Beispiel 109A

### Methyl-(2E)-3-[4-brom-2-(trifluormethyl)phenyl]acrylat

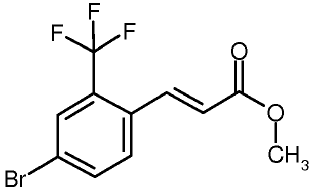

Analog zu Beispiel 104A wurden 8.00 g (22.8 mmol) 4-Brom-1-iod-2-(trifluoromethyl)benzol mit 3.29 ml (36.5 mmol) Acrylsäuremethylester umgesetzt und das Produkt isoliert. Das Rohprodukt wurde über Chromatographie am Kieselgel (Eluent Cyclohexan / Essigsäureethylester 10:1) gereinigt. Man erhielt 5.70 g (81 % d. Th.) der Titelverbindung.
GC-MS (Methode 6): Rₜ = 4.75 min; MS (CI-pos): m/z = 308 /310 (M)⁺.
¹H-NMR (400MHz, DMSO-d₆): δ [ppm] = 3.76 (s, 3H), 6.82 (d, 1H), 7.77 (dq, 1H), 7.94 - 8.07 (m, 3H).

### Beispiel 110A

### Methyl-3-[4-Brom-2-(trifluormethyl)phenyl]propanoat

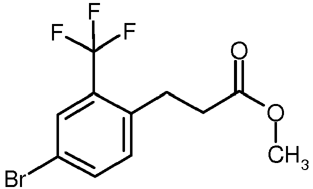

5.70 g (18.4 mmol) der Verbindung aus Beispiel 109A wurden zuerst analog zu Beipiel 105A mit Palladium (10 %-ig auf Kohle) unter Wasserstoff-Normaldruck eingesetzt. Bei der Reaktionskontrolle mittels LC-MS wurde keine Reduktion der Doppelbindung, aber ca. 25 % Debromierung festgestellt. Die Hydrierung wurde unterbrochen, der Katalysator abfiltriert und das Lösungsmittel am Rotationsverdampfer entfernt. Das so zurückgewonnene Edukt (5.0 g) wurde in 30 ml Toluol mit 87 mg (0.16 mmol) [Rh{(S,S)-Phebox-*i*Pr}-(OAc)₂]H₂O (Herstellung: siehe H. Nishiyama et al, Chem. Eur. J. 2006, 12 (1), 63-71, Beispiel 3a) auf 60°C erhitzt und bei dieser Temperatur mit 3.89 ml (24.26 mmol) Methyldiethoxysilan versetzt. Die Mischung wurde 4 h bei 60°C, dann über Nacht bei Rückflusstemperatur weiter gerührt. Nach Abkühlung auf RT wurde das Gemisch mit 50 ml IN Salzsäure versetzt und ml 150 ml Essigsäureethylester extrahiert. Die organische Phase wurde zweimal mit Wasser, zweimal mit einer gesättigten Natriumhydrogencarbonatlösung und einmal mit einer gesättigten Natriumchloridlösung gewaschen, über Natriumsulfat getrocknet und am Rotationsverdampfer eingeengt. Der Rückstand entsprach der Titelverbindung in ca. 80 %-iger Reinheit (5.84 g, 93 % d. Th.) und wurde ohne Reinigung weiter umgesetzt.
GC-MS (Methode 6): Rₜ = 4.42 min; MS (CI-pos): m/z = 310 /312 (M)⁺.
¹H-NMR (400MHz, CD₂Cl₂): δ [ppm] = 2.54 - 2.64 (m, 2H), 3.07 (t, 2H), 3.66 (s, 3H), 7.27 (d, 1H), 7.63 (d, 1H), 7.78 (d, 1H).

### Beispiel 111A

### 3-[4-Brom-2-(trifluormethyl)phenyl]propansäure

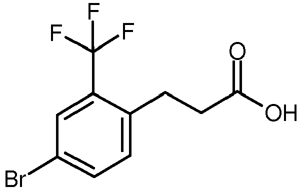

Analog zu Beispiel 106A wurden 5.60 g (18 mmol) der Verbindung aus Beispiel 110A umgesetzt und isoliert. Man erhielt 3.42 g (54 % d.Th.) der Titelverbindung in ca. 85 %-iger Reinheit.
LC-MS (Methode 4): Rₜ = 2.25 min; MS (ESIpos): m/z = 295 / 297 (M-H)⁻.
¹H-NMR (400MHz, DMSO-d₆): δ [ppm] = 2.52 - 2.58 (m, 2H, teilweise unter dem DMSO Signal versteckt), 2.95 (t, 2H), 7.49 (d, 1H), 7.81 - 7.88 (m, 2H), 12.37 (br.s, 1H).

### Beispiel 112A

### 6-Brom-4-(trifluormethyl)indan-1-on

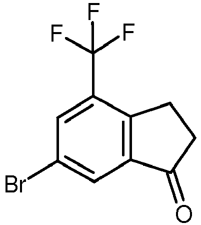

Analog zu Beispiel 107A wurden 3.42 g (85 %-ige Reinheit, 9.8 mmol) der Verbindung aus Beispiele-111A umgesetzt und isoliert. Man erhielt 2.10 g (69 % d.Th.) der Titelverbindung in ca. 90 %-iger Reinheit.
GC-MS (Methode 6): Rₜ = 4.34 min; MS (CI-pos): m/z = 278 /280 (M)⁺.
¹H-NMR (400MHz, CDCl₃): δ [ppm] = 2.75 - 2.82 (m, 2H), 3.23 - 3.31 (m, 2H), 7.96 (s, 1H), 8.05 (s, 1H).

### Beispiel 113A

### 6-Brom-4-(trifluormethyl)indan-1-ol (Racemat)

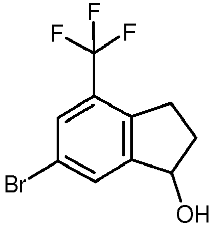

Eine Lösung von 500 mg (1.79 mmol) der Verbindung aus Beispiel 112A in 3.9 ml Ethanol wurde mit 44.0 mg (1.16 mmol) Natriumborhydrid versetzt und über Nacht bei RT gerrührt. 3 ml IN Salzsäure wurden zugegeben, das Gemisch einige Minuten gerührt, dann komplett per HPLC (Methode 7) getrennt. Die produkthaltigen Fraktionen wurden vollständig in vacuo eingeengt und der Rückstand am HV getrocknet. Man erhielt 352 mg (92 % d. Th.) der Titelverbindung.
GC-MS (Methode 6): Rₜ = 4.58 min; MS (CI-pos): m/z = 280 /282 (M)⁺.
¹H-NMR (400MHz, DMSO-d₆): δ [ppm] = 1.79 - 1.90 (m, 1H), 2.35 - 2.46 (m, 1H), 2.82 (dt, 1H), 2.97 - 3.07 (m, 1H), 5.09 (q, 1H), 5.57 (dd, 1H), 7.74 (br.s, 1H), 7.77 (br.s, 1H).

### Beispiel 114A

### 4,6-Dichlorindan-1-ol (Racemat)

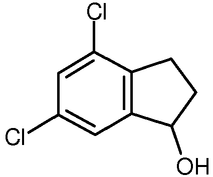

Analog zu Beispiel 98A wurden 1.25 g (6.22 mmol) 4,6-Dichlorindan-1-on mit Natriumborhydrid reduziert und das Produkt isoliert. Man erhielt 1.20 g (95 % d. Th.) der Titelverbindung.
MS (Methode 26 DCI/NH₃): m/z = 202 (M⁺)
¹H-NMR (400MHz, DMSO-d₆): δ [ppm] = 1.75 - 1.90 (m, 1H), 2.30 - 2.44 (m, 1H), 2.64 - 2.78 (m, 1H), 2.85 - 2.98 (m, 1H), 5.09 (q, 1H), 5.53 (d, 1H), 7.32 (s, 1H), 7.44 (d, 1H).

### Beispiel 115A

### 1-[2-Methyl-3-(trifluormethyl)phenyl]ethanol

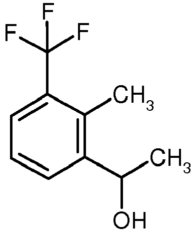

4.25 ml einer Lösung von Methylmagnesiumbromid (3M in Diethylether, 12.75 mmol) wurden tropfenweise auf eine Lösung von 2.00 g (10.6 mmol) 2-Methyl-3-(trifluormethyl)benzaldehyd in 50 ml Diethylether zugegeben, wobei das Reaktionsgemisch sich auf Rückflusstemperatur erwärmte. Nach beendeter Zugabe wurde die Reaktionsmischung eine weitere Stunde zum Rückfluss erhitzt. Nach Abkühlung auf RT wurden kleine Eisstücke zugegeben, dann tropfenweise 6N Salzsäure bis der gebildete Niederschlag sich wieder löste. Die Phasen wurden getrennt. Die wässrige Phase wurde noch einmal mit Diethylether extrahiert. Die vereinigten organischen Phasen wurden über Magnesiumsulfat getrocknet und am Rotationsverdampfer eingeengt. Man erhielt 2.40 g der Titelverbindung (100 % d. Th., enthält laut NMR noch ca. 10 % Diethylether).
¹H-NMR (500MHz, CDCl₃): δ [ppm] = 1.48 (d, 3H), 5.25 (q, 1H), 7.32 (t, 1H), 7.56 (d, 1H), 7.76 (d, 1H).

### Beispiel 116A

### 1-[2-Chlor-3-(trifluormethyl)phenyl]ethanol

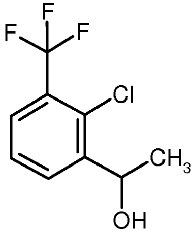

Analog zu Beispiel 115A wurden 2.00 g (9.59 mmol) 2-Methyl-3-(trifluormethyl)benzaldehyd mit Methylmagnesiumbromid umgesetzt. Man erhielt 2.40 g der Titelverbindung (89 % d. Th., enthält laut NMR noch ca. 20 % Diethylether).
¹H-NMR (500MHz, CDCl₃): δ [ppm] = 1.51 (d, 3H), 5.41 (q, 1H), 7.41 (t, 1H), 7.62 (d, 1H), 7.85 (d, 1H).

### Beispiel 117A

### Ethyl-l-(1-ethyl-2-methyl-1H-benzimidazol-5-yl)-2,4-dioxo-1,2,3,4-tetrahydropyrimidin-5-carboxylat

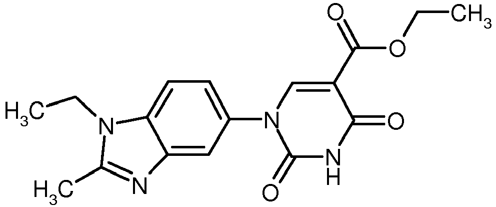

In 30 mL Ethanol wurden 1.04 g (4.03 mmol) Ethyl-3-ethoxy-2-[(ethoxycarbonyl)carbamoyl]-acrylat sowie 1.00 g (4.03 mmol) 1-Ethyl-2-methyl-1H-benzimidazol-5-amin-Dihydrochlorid vorgelegt, anschließend 1.24 mL (8.87 mmol) Triethylamin zugefügt und die Mischung für 2 h zum Rückfluss erhitzt. Danach wurden bei RT 452 mg (4.03 mmol) Kalium-tert.-butylat zugefügt und die Reaktionsmischung zuerst über Nacht bei RT weiter gerührt, anschließend zum Rückfluss erhitzt und bei dieser Temperatur über Nacht gerührt. Zur Aufarbeitung wurde das Reaktionsgemisch mit Wasser versetzt und mit IN Salzsäure angesäuert. Die Mischung wurde zur Trockne eingeengt, der Rückstand mit Dichlormethan/Methanol (1:1) verrührt und filtriert. Das Filtrat wurde erneut eingeengt, der Rückstand mit MTBE/Essigsäureethylester verrührt und der gebildete Feststoff abfiltriert. Nach Trocknung im HV erhielt man 1.07 g (87 %rein, 68 % d. Th.) der Zielverbindung.
LC-MS (Methode 2): Rₜ = 1.06 min; MS (ESIpos): m/z = 343 (M+H)⁺.
¹H-NMR (400 MHz, DMSO-d₆): δ [ppm] = 1.23 (t, 3H), 1.41 (t, 3H), 2.84 (s, 3H), 4.18 (q, 2H), 4.47 (q, 2H), 7.64 - 7.70 (m, 1H), 7.99 - 8.02 (m, 1H), 8.06 (d, 1H), 8.35 (s, 1H), 11.75 (s, 1H).

### Beispiel 118A

### Ethyl-1-(1-cyclohexyl-2-methyl-1H-benzimidazol-5-yl)-2,4-dioxo-1,2,3,4-tetrahydropyrimidin-5-carboxylat

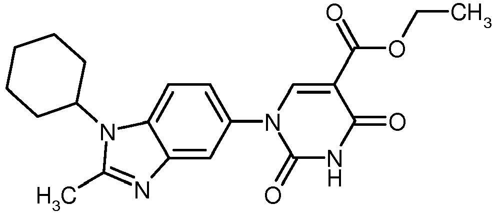

In 25 mL Ethanol wurden 0.86 g (3.31 mmol) Ethyl-3-ethoxy-2-[(ethoxycarbonyl)-carbamoyl]acrylat sowie 1.00 g (3.31 mmol) 1-Cyclohexyl-2-methyl-1H-benzimidazol-5-amin Dihydrochlorid vorgelegt und die Mischung 2 h zum Rückfluss erhitzt. Anschließend wurden bei RT 371 mg (3.31 mmol) Kalium-tert.-butylat zugefügt und die Reaktionsmischung über Nacht bei RT sowie 5 Tage bei Rückfluss gerührt. Zur Aufarbeitung wurde die Reaktionsmischung mit Wasser versetzt, mit IN Salzsäure angesäuert und anschliessend am Rotationsverdampfer eingeengt. Der Rückstand wurde in Dichlormethan/Methanol (1:1) verührt und die unlöslichen Reste abfiltriert. Das Filtrat wurde eingeengt und mit Ethanol versetzt, der gebildete Feststoff abfiltriert und getrocknet. Man erhielt 1.85 g der Titelverbindung als Rohprodukt, das ohne weitere Reinigung umgesetzt wurde. LC-MS (Methode 1): Rₜ = 0.72 min; MS (ESIpos): m/z = 397 (M+H)⁺.

### Beispiel 119A

### Ethyl-1-(4-methyl-3-oxo-3,4-dihydro-2H-1,4-benzoxazin-7-yl)-2,4-dioxo-1,2,3,4-tetrahydropyrimidin-5-carboxylat

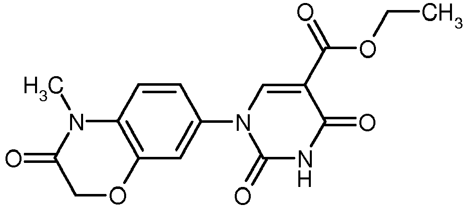

2.00 g (11.2 mmol) 7-Amino-4-methyl-2H-1,4-benzoxazin-3(4H)-on sowie 2.65 g (10.2 mmol) Ethyl-3-ethoxy-2-[(ethoxycarbonyl)carbamoyl]acrylat wurden in 100 mL Ethanol vorgelegt und die Mischung 2 h zum Rückfluss erhitzt. Nach Abkühlen auf RT wurden 1.15 g (10.2 mmol) Kalium-tert.-butylat zugegeben und das Reaktionsgemisch weiter zwei Tage bei RT und anschließend 1h bei Rüchflusstemperatur gerührt. Zur Aufarbeitung wurde die Reaktionsmischung mit Wasser verdünnt und mit IM Salzsäure angesäuert. Der entstandene Feststoff wurde abfiltriert, mit Wasser gewaschen und im HV getrocknet. Man erhielt 2.79 mg (70 % d. Th.) der Titelverbindung.
LC-MS (Methode 3): Rₜ = 0.76 min; MS (ESIpos): m/z = 346 (M+H)⁺.
¹H-NMR (400MHz, DMSO-d₆): δ [ppm] = 1.22 (t, 3H), 4.17 (q, 2H), 4.71 (s, 2H), 7.18 - 7.23 (m, 2H), 7.28 (d, 1H), 8.22 (s, 1H), 11.68 (s, 1H).

### Beispiel 120A

### 8-Chlor-3,4-dihydro-1H-isochromen-4-ol

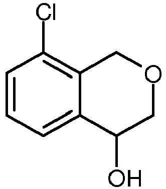

Zu einer Lösung von 270 mg (1.48 mmol) 8-Chlor-1H-isochromen-4(3H)-on (Edukt selber hergestellt, nicht beschrieben in Lit aber käuflich von ACD Anbieter mit Katalog-Nummer und CAS-Nr) in 5 ml Methanol wurden bei RT 224 mg (5.91 mmol) Natriumborhydrid hinzugefügt und das Gemisch 1 h bei RT gerührt. Anschließend wurden 5 ml wässrige IN Salzsäure zugegeben, das Gemisch weitere 10 min gerührt und dann mittels präparativer HPLC (Methode 15) getrennt. Die geeigneten Fraktionen wurden am Rotationsverdampfer bei 130 mbar vom Acetonitril befreit und die verbleibende wässrige Phase wurde dreimal mit Dichlormethan extrahiert. Die vereinigten organischen Phasen wurden über Natriumsulfat getrocknet und am Rotationsverdampfer bei 130 mbar eingeengt. Man erhielt 400 mg der Titelverbindung, die laut NMR noch Acetonitril und Dichlormethan enthält. Sie wurde so für die Herstellung von Beispiel 302 eingesetzt.
LC/MS (Methode 4): Rₜ = 1.69 min; m/z = 167 (M-OH)⁺
¹H-NMR (400MHz, CDCl₃): δ [ppm] = 2. 57 (br. s, 1H), 3.85 (dd, 1H), 4.09 (dd, 1H), 4.56 (br. s., 1H), 4.62 (d, 1H), 4.89 (d, 1H), 7.22 - 7.35 (m, 2H), 7.39 (d, 1H).

### Ausführungsbeispiele:

### Beispiel 1

### Ethyl-1-(1,3-dimethyl-2-oxo-2,3-dihydro-1H-benzimidazol-5-yl)-3-[2-methyl-3-(trifluormethyl)-benzyl]-2,4-dioxo-1,2,3,4-tetrahydropyrimidin-5-carboxylat

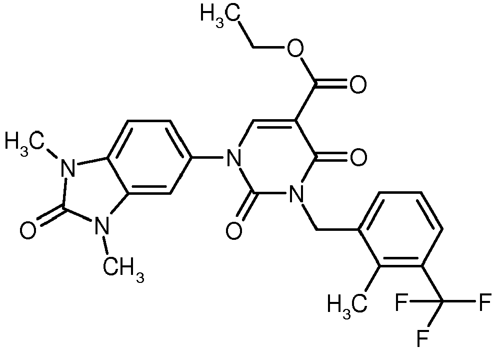

Zu einer Lösung von 14.95 g (43.42 mmol) Ethyl-1-(1,3-dimethyl-2-oxo-2,3-dihydro-1H-benzimidazol-5-yl)-2,4-dioxo-1,2,3,4-tetrahydropyrimidin-5-carboxylat aus Beispiel 2A in DMF (200 ml) gab man 12.00 g (86.84 mmol) Kaliumcarbonat, 12.09 g (47.76 mmol) 2-Methyl-3-(trifluormethyl)benzylbromid sowie 0.721 g (4.34 mmol) Kaliumiodid und ließ die Reaktionsmischung 3 h bei 80 °C rühren. Anschließend wurde das Gemisch auf RT abgekühlt, mit Wasser versetzt und der entstandene Niederschlag abfiltriert. Der Feststoff wurde nacheinander mit Wasser sowie MTBE gewaschen und im Vakuum bei 50°C getrocknet. Man erhielt 21.04 g (94 % d. Th.) der Titelverbindung.
LC-MS (Methode 1): Rₜ = 1.07 min; m/z = 517 (M+H)⁺.
¹H-NMR (400MHz, DMSO-d₆): δ [ppm] = 1.23 (t, 3H), 2.46 (s, 3H), 3.34 (s, 3H), 3.37 (s, 3H), 4.20 (q, 2H), 5.09 (s, 2H), 7.23 - 7.30 (m, 2H), 7.32 - 7.43 (m, 3H), 7.58 - 7.62 (m, 1H), 8.42 (s, 1H).

In Analogie zu Beispiel 1 wurden aus den zuvor beschriebenen 1,2,3,4-Tetrahydropyrimidin-2,4-dion-5-carbonsäureestern (Uracil-5-carbonsäureestern) durch Umsetzung mit den jeweiligen Benzylchloriden oder Benzyl-bromiden in Gegenwart von Kaliumcarbonat und Kaliumiodid die folgenden Benzyl-substituierten Uracil-Verbindungen erhalten. Abweichend können auch 1-3 Äquivalente Kaliumcarbonat und 0.1 bis 2 Äquivalente Kaliumiodid eingesetzt werden. Bei ausreichend löslichen Verbindungen wurde in einigen Fällen Acetonitril als Lösungsmittel verwendet.

### Beispiel 2

### Ethyl-3-[2-chlor-3-(trifluormethyl)benzyl]-1-(1,3-dimethyl-2-oxo-2,3-dihydro-1H-benzimidazol-5-yl)-2,4-dioxo-1,2,3,4-tetrahydropyrimidin-5-carboxylat

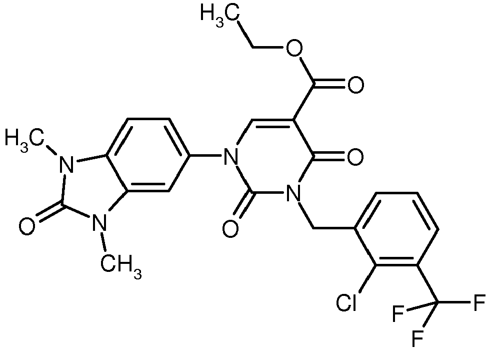

Darstellung und Reinigung der Titelverbindung erfolgten analog zu Beispiel 1. Ausgehend von 200 mg (0.58 mmol) Ethyl-1-(1,3-dimethyl-2-oxo-2,3-dihydro-1H-benzimidazol-5-yl)-2,4-dioxo-1,2,3,4-tetrahydro-pyrimidin-5-carboxylat aus Beispiel 2A und 175 mg (0.64 mmol) 2-Chlor-3-(trifluormethyl)benzylbromid erhielt man 234 mg (73 % d. Th.) der Titelverbindung.
LC-MS (Methode 1): Rₜ = 1.08 min; m/z = 537 (M+H)⁺.
¹H-NMR (400MHz, DMSO-d₆): δ [ppm] = 1.23 (t, 3H), 3.34 (s, 3H), 3.37 (s, 3H), 4.20 (d, 2H), 5.16 (s, 2H), 7.27 (s, 2H), 7.39 - 7.42 (m, 1H), 7.50 - 7.60 (m, 2H), 7.78 - 7.83 (m, 1H), 8.44 (s, 1H).

### Beispiel 3

### Ethyl-3-(2,3-dichlorbenzyl)-1-(1,3-dimethyl-2-oxo-2,3-dihydro-1H-benzimidazol-5-yl)-2,4-dioxo-1,2,3,4-tetrahydropyrimidin-5-carboxylat

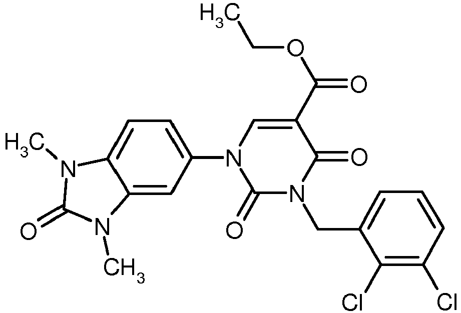

Darstellung und Reinigung der Titelverbindung erfolgten analog zu Beispiel 1 unter Verwendung von Acetonitril als Lösungsmittel. Ausgehend von 200 mg (0.58 mmol) Ethyl-1-(1,3-dimethyl-2-oxo-2,3-dihydro-1H-benzimidazol-5-yl)-2,4-dioxo-1,2,3,4-tetrahydropyrimidin-5-carboxylat aus Beispiel 2A und 125 mg (0.64 mmol) 2,3-Dichlorbenzylchlorid erhielt man 241 mg (79 % d. Th.) der Titelverbindung.
LC-MS (Methode 1): Rₜ = 1.05 min; m/z = 503 (M+H)⁺.
¹H-NMR (400MHz, DMSO-d₆): δ [ppm] = 1.23 (t, 3H), 3.34 (s, 3H), 3.37 (s, 3H), 4.20 (q, 2H), 5.11 (s, 2H), 7.20 - 7.29 (m, 3H), 7.31 - 7.36 (m, 1H), 7.39 - 7.41 (m, 1H), 7.56 - 7.60 (m, 1H), 8.43 (s, 1H).

### Beispiel 4

### Ethyl-1-(1,3-dimethyl-2-oxo-2,3-dihydro-1H-benzimidazol-5-yl)-3-[2-fluor-3-(trifluormethyl)benzyl]-2,4-dioxo-1,2,3,4-tetrahydropyrimidin-5-carboxylat

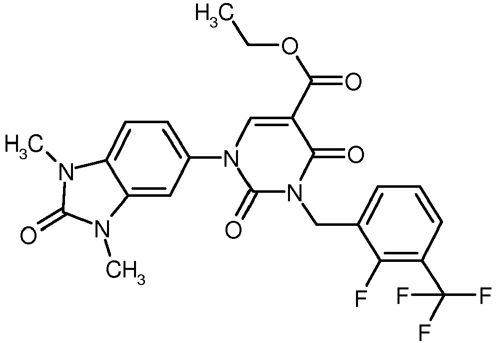

Darstellung und Reinigung der Titelverbindung erfolgten analog zu Beispiel 1. Ausgehend von 200 mg (0.58 mmol) Ethyl-1-(1,3-dimethyl-2-oxo-2,3-dihydro-1H-benzimidazol-5-yl)-2,4-dioxo-1,2,3,4-tetrahydropyrimidin-5-carboxylat aus Beispiel 2A und 164 mg (0.64 mmol) 2-Fluor-3-(trifluormethyl)bromid erhielt man 162 mg (53 % d. Th.) der Titelverbindung.
LC-MS (Methode 1): Rₜ = 1.13 min; m/z = 521 (M+H)⁺.
¹H-NMR (400MHz, DMSO-d₆): δ = 1.23 (t, 3H), 3.33 (s, 3H), 3.34 (s, 3H), 4.20 (d, 2H), 5.15 (s, 2H), 7.19 - 7.31 (m, 2H), 7.31 - 7.44 (m, 2H), 7.68 (d, 2H), 8.40 (s, 1H).

### Beispiel 5

### Ethyl-l-(1,3-dimethyl-2-oxo-2,3-dihydro-1H-benzimidazol-5-yl)-3-[3-fluor-2-(trifluormethyl)-benzyl]-2,4-dioxo-1,2,3,4-tetrahydropyrimidin-5-carboxylat

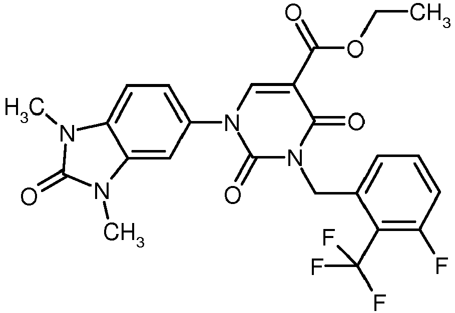

Darstellung und Reinigung der Titelverbindung erfolgten analog zu Beispiel 1. Ausgehend von 179 mg (0.52 mmol) Ethyl-1-(4-methoxyphenyl)-2,4-dioxo-1,2,3,4-tetrahydropyrimidin-5-carboxylat aus Beispiel 2A und 147 mg (0.57 mmol) 3-Fluor-2-trifluorobenzylbromid erhielt man 207 mg (74 % d. Th.) der Titelverbindung. LC-MS (Methode 1): Rₜ = 1.12 min; m/z = 521 (M+H)⁺.
¹H-NMR (400MHz, DMSO-d₆): δ [ppm] = 1.23 (t, 3H), 3.34 (s, 3H), 3.37 (s, 3H), 4.20 (q, 2H), 5.21 (s, 2H), 7.17 - 7.22 (m, 1H), 7.22 - 7.30 (m, 2H), 7.37 - 7.44 (m, 2H), 7.63 - 7.71 (m, 1H), 8.45 (s, 1H).

### Beispiel 6

### Ethyl-3-(2-chlor-3,6-difluorbenzyl)-1-(1,3-dimethyl-2-oxo-2,3-dihydro-1H-benzimidazol-5-yl)-2,4-dioxo-1,2,3,4-tetrahydropyrimidin-5-carboxylat

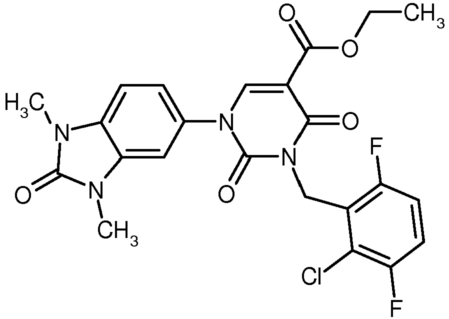

In Acetonitril (2.06 ml) wurden 150 mg (0.43 mmol) der Verbindung aus Beispiel 5A mit 417 mg des Boronsäureesters aus Beispiel 3A (60 %-ige Reinheit, 0.87 mmol) sowie 0.18 ml (1.30 mmol) Triethylamin vorgelegt. Anschließend wurden Molekularsieb (3Ǻ), 118 mg (0.65 mmol) Kupfer(II)acetat sowie 0.13 ml (1.83 mmol) DMSO hinzugefügt und die Reaktionsmischung im geschlossenen Gefäß 3 Tage bei 80°C gerührt. Zur Aufarbeitung wurde die Reaktionsmischung mit Essigsäureethylester versetzt, anschließend zweimal mit Salzsäure (1M), einmal mit gesättigter Natriumhydrogencarbonatlösung und einmal mit gesättigter Natriumchloridlösung gewaschen. Die organische Phase wurde danach über Magnesiumsulfat getrocknet, filtriert und eingeengt. Der Rückstand wurde mit Methanol verrührt, der Feststoff abgesaugt, mit Methanol nachgewaschen und im Vakuum bei 50°C getrocknet. Man erhielt 114 mg (84 %-ige Reinheit, 44 % d. Th.) der Titelverbindung.
LC-MS (Methode 2): Rₜ = 2.06 min; m/z = 505 (M+H)⁺.
¹H-NMR (400MHz, DMSO-d₆): δ [ppm] = 1.22 (t, 3H), 4.16 (q, 2H), 5.21 (s, 2H), 7.16 (d, 1H), 7.22 - 7.31 (m, 2H), 7.34 (s, 1H), 7.38 - 7.48 (m, 1H), 8.36 (s, 1H).

### Beispiel 7

### Ethyl-3-(3-chlor-2-methylbenzyl)-1-(1,3-dimethyl-2-oxo-2,3-dihydro-1H-benzimidazol-5-yl)-2,4-dioxo-1,2,3,4-tetrahydropyrimidin-5-carboxylat

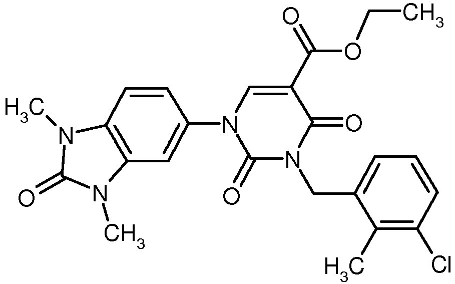

In Acetonitril (4.00 ml) wurden 150 mg (0.46 mmol) der Verbindung aus Beispiel 7A, mit 267 mg des Boronsäureesters aus Beispiel 3A (0.93 mmol) sowie 0.19 ml (1.39 mmol) Triethylamin vorgelegt. Anschließend wurden Molekularsieb (3Ǻ), 126 mg (0.69 mmol) Kupfer(II)acetat sowie 0.13 ml (1.83 mmol) DMSO hinzugefügt und die Reaktionsmischung im geschlossenen Gefäß 1 Tag bei 80°C gerührt. Zur Aufarbeitung wurde das Gemisch mit Essigsäureethylester versetzt, anschließend zweimal mit IM Salzsäure, einmal mit gesättigter Natriumhydrogencarbonatlösung und einmal mit gesättigter Natriumchloridlösung gewaschen. Die organische Phase wurde danach über Magnesiumsulfat getrocknet, filtriert und eingeengt. Der Rückstand wurde mit MTBE verrührt, der Feststoff abgesaugt und im Vakuum bei 50°C getrocknet. Dieser Feststoff wurde mittels präparativer HPLC (Methode 8) gereinigt. Man erhielt 78 mg (35 % d. Th.) der Titelverbindung
LC-MS (Methode 3): Rₜ = 1.34 min; m/z = 483 (M+H)⁺.
¹H-NMR (400MHz, DMSO-d₆): δ [ppm] = 1.23 (t, 3H), 2.41 (s, 3H), 3.34 (s, 3H), 3.37 (s, 3H), 4.19 (q, 2H), 5.05 (s, 2H), 7.05 (d, 1H), 7.17 (t, 1H), 7.22 - 7.30 (m, 2H), 7.35 (d, 1H), 7.41 (d, 1H), 8.40 (s, 1H).

### Beispiel 8

### Ethyl-3-[2,3-bis(trifluormethyl)benzyl]-1-(1,3-dimethyl-2-oxo-2,3-dihydro-1H-benzimidazol-5-yl)-2,4-dioxo-1,2,3,4-tetrahydropyrimidin-5-carboxylat

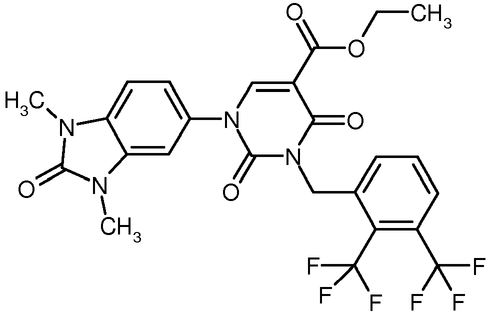

In Acetonitril (4.00 ml) wurden 150 mg (0.37 mmol) der Verbindung aus Beispiel 10A, mit 248 mg des Boronsäureester aus Beispiel 3A (85 %-ige Reinheit, 0.73 mmol) sowie 0.15 ml (1.10 mmol) Triethylamin vorgelegt. Anschließend wurden Molekularsieb (3Å), 100 mg (0.54 mmol) Kupfer(II)acetat sowie 0.13 ml (1.83 mmol) DMSO hinzugefügt und die Reaktionsmischung 3 Tage bei 80°C im geschlossenen Gefäß geschüttelt. Zur Aufarbeitung wurde die Reaktionsmischung mit Essigsäureethylester verdünnt, anschließend zweimal mit IM Salzsäure sowie je einmal mit gesättigter Natriumhydrogencarbonatlösung und gesättigter Natriumchloridlösung gewaschen. Die organische Phase wurde danach über Magnesiumsulfat getrocknet, filtriert und eingeengt. Der Rückstand wurde mittels präparativer HPLC (Methode 8) getrennt. Die produkthaltigen Fraktionen wurden am Rotationsverdampfer teilweise eingeengt. Der dabei ausfallende Feststoff wurde abfiltriert, mit Wasser gewaschen und im Hochvakuum getrocknet. Man erhielt 127 mg (61 % d. Th.) der Titelverbindung.
LC-MS (Methode 1): Rₜ = 1.05 min; m/z = 571 (M+H)⁺.
¹H-NMR (400MHz, DMSO-d₆): δ [ppm] = 1.23 (t, 3H), 3.2-3.4 (2 s, teilweise durch Wasser-Signal verdeckt), 4.20 (q, 2H), 5.25 (br.s, 2H), 7.26 (q, 2H), 7.39 (s, 1H), 7.73 (d, 1H), 7.85 (t, 1H), 7.98 (d, 1H), 8.46 (s, 1H).

### Beispiel 9

### Ethyl-3-(3-chlor-5-fluorbenzyl)-1-(1,3-dimethyl-2-oxo-2,3-dihydro-1H-benzimidazol-5-yl)-2,4-dioxo-1,2,3,4-tetrahydropyrimidin-5-carboxylat

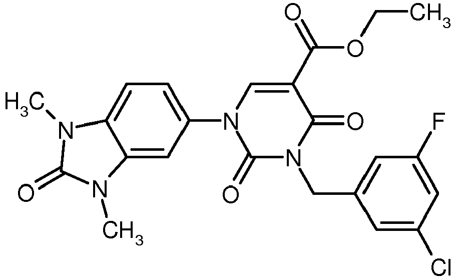

Darstellung und Reinigung der Titelverbindung erfolgten analog zu Beispiel 1. Die Reaktionszeit betrug 1h. Ausgehend von 200 mg (0.58 mmol) Ethyl-1-(1,3-dimethyl-2-oxo-2,3-dihydro-1H-benzimidazol-5-yl)-2,4-dioxo-1,2,3,4-tetrahydropyrimidin-5-carboxylat aus Beispiel 2A und 142 mg (0.63 mmol) 1-(Brommethyl)-3-chlor-5-fluorbenzol erhielt man 255 mg (90 % d. Th.) der Titelverbindung.
LC-MS (Methode 1): Rₜ = 0.97 min; m/z = 487 (M+H)⁺.
¹H-NMR (400MHz, DMSO-d₆): δ = 1.23 (t, 3H), 3.34 (s, 3H), 3.37 (s, 3H), 4.19 (q, 2H), 5.03 (s, 2H), 7.19 (d, 1H), 7.22 - 7.25 (m, 1H), 7.27 (s, 1H), 7.28 - 7.31 (m, 1H), 7.32 - 7.37 (m, 1H), 7.40 (d, 1H), 8.36 (s, 1H).

### Beispiel 10

### Ethyl-1-(1,3-dimethyl-2-oxo-2,3-dihydro-1H-benzimidazol-5-yl)-3-[3-fluor-5-(trifluormethyl)benzyl]-2,4-dioxo-1,2,3,4-tetrahydropyrimidin-5-carboxylat

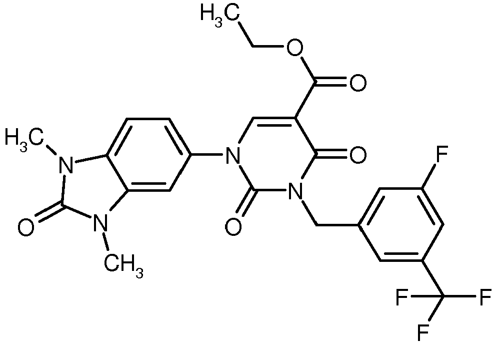

Darstellung und Reinigung der Titelverbindung erfolgten analog zu Beispiel 1. Die Reaktionszeit betrug 1h. Ausgehend von 200 mg (0.58 mmol) Ethyl-1-(1,3-dimethyl-2-oxo-2,3-dihydro-1H-benzimidazol-5-yl)-2,4-dioxo-1,2,3,4-tetrahydropyrimidin-5-carboxylat aus Beispiel 2A und 164 mg (0.63 mmol) 1-(Brommethyl)-3-fluor-5-(trifluormethyl)benzol erhielt man 278 mg (91 % d. Th.) der Titelverbindung.
LC-MS (Methode 1): Rₜ = 1.01 min; m/z = 521 (M+H)⁺.
¹H-NMR (400MHz, DMSO-d₆): δ = 1.23 (s, 3H), 3.31 (s, 3H), 3.37 (s, 3H), 4.20 (q, 2H), 5.12 (s, 2H), 7.23 (dd, 1H), 7.28 (d, 1H), 7.39 (d, 1H), 7.52 (d, 1H), 7.58 - 7.63 (m, 2H), 8.37 (s, 1H).

### Beispiel 11

### Ethyl-1-(1,3-dimethyl-2-oxo-2,3-dihydro-1H-benzimidazol-5-yl)-2,4-dioxo-3-[4-(trifluormethyl)-2,3-dihydro-1H-inden-1-yl]-1,2,3,4-tetrahydropyrimidin-5-carboxylat (Racemat)

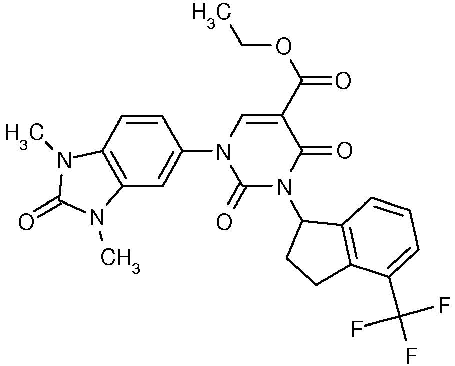

*Methode A:* Darstellung und Reinigung der Titelverbindung erfolgte analog zu Beispiel 8. Die Reaktionszeit betrug 4 Tage. Ausgehend von 300 mg (80 %-ige Reinheit, 0.65 mmol) Ethyl-2,4-dioxo-3-[4-(trifluormethyl)-2,3-dihydro-1H-inden-1-yl]-1,2,3,4-tetrahydropyrimidin-5-carboxylat aus Beispiel 21A und 375 mg (1.30 mmol) 1,3-Dimethyl-5-(4,4,5,5-tetramethyl-1,3,2-dioxaborolan-2-yl)-1,3-dihydro-2H-benzimidazol-2-on aus Beispiel 3A erhielt man nach zusätzlicher Reinigung mittels Flashchromatographie (Dichlormethan/Methanol 98:2) 190 mg (52 % d. Th.) der Titelverbindung.
   LC-MS (Methode 5): Rₜ = 1.08 min; m/z = 529 (M+H)⁺.
   ¹H-NMR (400MHz, DMSO-d₆): δ [ppm] = 1.23 (t, 3H), 2.35 - 2.43 (m, 1H), 2.44 - 2.48 (m, 1H), 3.03 - 3.15 (m, 1H), 3.21 - 3.29 (m, 1H), 3.31 (s, 3H), 3.36 (s, 3H), 4.18 (q, 2H), 6.35 - 6.58 (m, 1H), 7.13 - 7.28 (m, 2H), 7.37 (t, 2H), 7.45 - 7.55 (m, 2H), 8.33 (s, 1H).
*Methode B:* In einem anderen Experiment wurde auf analoger Weise 1.00 g der Verbindung aus Beipiel 21A eingesetzt. Nach der Reinigung durch Flashchromatographie war allerdings das Produkt (1.20 g) nur 63 %-ige Reinheit (entspricht ca. 50 % d. Th.). Dieses wurde direkt per präparativer chiraler HPLC (Methode 12) in die Enantiomeren getrennt: Man erhielt 377 mg (24 % d. Th.) des zuerst eluierenden Enantiomers (siehe Beispiel 12) und 331 mg (21 % d. Th.) des später eluierenden Enantiomers (siehe Beispiel 13)

### Beispiel 12

### Ethyl-1-(1,3-dimethyl-2-oxo-2,3-dihydro-1H-benzimidazol-5-yl)-2,4-dioxo-3-[(1S)-4-(trifluormethyl)-2,3-dihydro-1H-inden-1-yl]-1,2,3,4-tetrahydropyrimidin-5-carboxylat (S-Enantiomer)

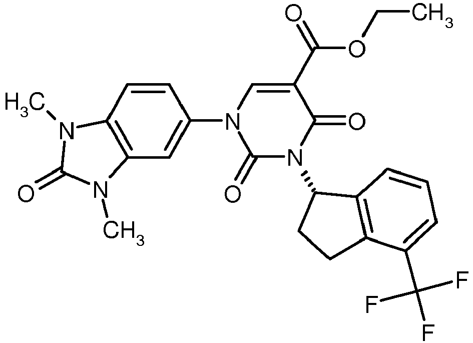

*Methode A:* Zuerst eluierendes Enantiomer (377 mg) aus der Trennung der Verbindung aus Beispiel 11 (*Methode B*) per präparativer HPLC an einer chiralen Phase (Methode 12).
   Chirale HPLC (Methode 13): Rₜ = 9.39 min, 100 % ee.
   Spezifischer optischer Drehwert: α_{D}²⁰=-117.1° (Acetonitril, c = 0.05 g/100 ml).
*Methode B:* Unter Argonatmosphäre wurden 5.68 g (16.49 mmol) der Verbindung aus Beispiel 2A, 4.00 g (19.79 mmol) (1R)-4-(Trifluormethyl)indan-1-ol aus Beispiel 15A und 7.78 g (29.68 mmol) Triphenylphosphin in 200 ml DMF und 100 ml THF vorgelegt und auf 0°C gekühlt. Tropfenweise wurden 5.19 ml (5.33 g, 26.4 mmol) Diisopropylazodicarboxylat hinzugefügt. Das Kühlbad wurde entfernt und die Mischung 2 h bei RT gerührt. Anschließend wurden 25 ml IN Salzsäure zugegeben und die Mischung 15 min gerührt. Zur Aufarbeitung wurden ca. 2 L Essigsäureethylester und 1.33 L verdünnte Salzsäure (ca. 2.5N) zugegeben. Nach Verrühren wurde die organische Phase getrennt, zweimal mit verdünnter Salzsäure, einmal mit einer IN Natriumcarbonatlösung sowie einmal mit einer gesättigten Natriumchloridlösung gewaschen und über Natriumsulfat getrocknet. Die Lösungsmittel wurden am Rotationsverdampfer entfernt. Der Rückstand wurde per präparativer HPLC (Methode 11) gereinigt. Man erhielt 5.15 g der Titelverbindung (59 % d. Th.).
   LC-MS (Methode 1): Rₜ = 1.04 min; m/z = 529 (M+H)⁺.
   ¹H-NMR (400MHz, CD₂Cl₂): δ [ppm]= 1.31 (t, 3H), 2.36 - 2.51 (m, 1H), 2.59 (ddt, 1H), 3.07 -3.20 (m, 1H), 3.39 (s, 3H), 3.40 (s, 3H), 3.42 - 3.54 (m, 1H), 4.29 (q, 2H), 6.57 - 6.68 (br. m, 1H), 6.94 (br.s, 1H), 7.02 (s, 2H), 7.25 - 7.38 (m, 2H), 7.49 (d, 1H), 8.31 (s, 1H).
   Chirale HPLC (Methode 13): Rₜ = 9.39 min, 92 % ee.

### Beispiel 13

### Ethyl-1-(1,3-dimethyl-2-oxo-2,3-dihydro-1H-benzimidazol-5-yl)-2,4-dioxo-3-[(1R)-4-(trifluormethyl)-2,3-dihydro-1H-inden-1-yl]-1,2,3,4-tetrahydropyrimidin-5-carboxylat (R-Enantiomer)

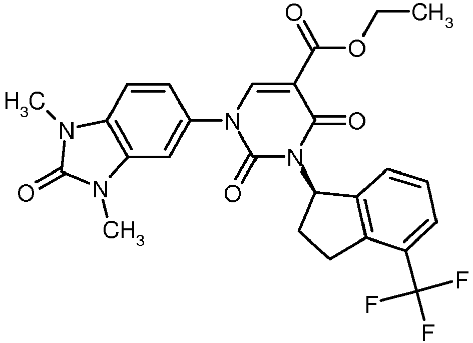

*Methode A:* Zuletzt eluierendes Enantiomer (331 mg) aus der Trennung der Verbindung aus Beispiel 11 (Methode B) per präparativer HPLC an einer chiralen Phase (Methode 12). Chirale HPLC (Methode 13): Rₜ = 11.12 min, 92 % ee.
*Methode B:* 3.05 g (8.86 mmol) Ethyl-1-(1,3-dimethyl-2-oxo-2,3-dihydro-1H-benzimidazol-5-yl)-2,4-dioxo-1,2,3,4-tetrahydropyrimidin-5-carboxylat aus Beispiel 2A, 2.15 g (10.63 mmol) (1S)-4-(Trifluormethyl)indan-1-ol aus Beispiel 14A und 6.97 g (26.6 mmol) Triphenylphosphin wurden unter Argon in THF / DMF 1:1 (1.7 L) vorgelegt und auf -15°C abgekühlt. 3.48 ml (17.71 mmol) Diisopropylazodicarboxylat wurden langsam hinzugefügt. Anschließend wurde die Reaktionsmischung noch 30 min bei RT gerührt. Unter Eiskühlung wurden weitere 0.8 Äquivalente (1.39 ml, 6.86 mmol) Diisopropylazodicarboxylat zugetropft und die Reaktionsmischung 1h bei RT gerührt. Das Reaktionsgemisch wurde auf -40°C abgekühlt, mit IM Salzsäure versetzt, mit Essigsäureethylester verdünnt und einige Minuten kräftig verrührt. Die organische Phase wurde getrennt, zweimal mit IM Natriumcarbonat-Lösung sowie einmal mit gesättigter Natriumchlorid-Lösung gewaschen, über Natriumsulfat getrocknet und am Rotationsverdampfer eingeengt. Der Rückstand wurde mit MTBE versetzt und über Nacht bei RT verrührt, anschließend 20 min bei Eisbadkühlung verrührt. Der ausgefallene Feststoff wurde abgesaugt und mit kaltem MTBE nachgewaschen. Das gesamte Filtrat wurde eingeengt und mittels präparativer HPLC (Methode 7) gereinigt. Man erhielt 2.90 g (62 % d. Th.) der Titelverbindung.
   LC-MS (Methode 1): Rₜ = 1.05 min; m/z = 529 (M+H)⁺.
   ¹H-NMR (400MHz, CD2Cl2): δ = 1.36 (t, 3H), 2.42 - 2.55 (m, 1H), 2.57 - 2.71 (m, 1H), 3.12 - 3.24 (m, 1H), 3.43 (s, 3H), 3.43 - 3.58 (m, 1H), 3.45 (s, 3H), 4.33 (q, 2H), 6.60 - 6.73 (m, 1H), 6.99 (s, 1H), 7.07 (s, 2H), 7.30 - 7.42 (m, 2H), 7.54 (d, 2H), 8.36 (s, 1H).

### Beispiel 14

### Ethyl-1-(1-methyl-2-oxo-2,3-dihydro-1H-benzimidazol-5-yl)-3-[2-methyl-3-(trifluormethyl)-benzyl]-2,4-dioxo-1,2,3,4-tetrahydropyrimidin-5-carboxylat

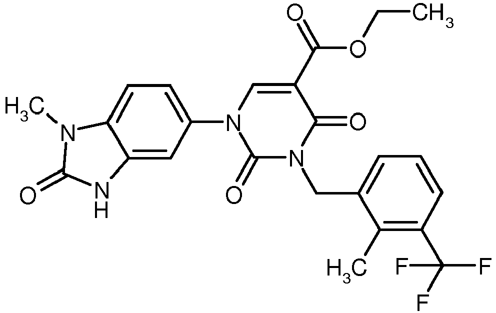

Darstellung und Reinigung der Titelverbindung erfolgten analog zu Beispiel 1. Die Reaktionszeit betrug 1h. Ausgehend von 500 mg (1.51 mmol) Ethyl-1-(1-methyl-2-oxo-2,3-dihydro-1H-benzimidazol-5-yl)-2,4-dioxo-1,2,3,4-tetrahydro-pyrimidin-5-carboxylat aus Beispiel 36A und 421 mg (1.67 mmol) 2-Methyl-3-(trifluormethyl)benzylbromid erhielt man 606 mg (Reinheit ca. 83 %, 66 % d. Th.) der Titelverbindung.
LC-MS (Methode 1): Rₜ = 0.96 min; m/z = 503 (M+H)⁺.
¹H-NMR (400MHz, DMSO-d₆): δ [ppm] = 1.23 (t, 3H), 2.46 (s, 3H), 3.30 (s, teilweise durch Wasser-Signal verdeckt), 4.19 (q, 2H), 5.07 (s, 2H), 7.18 - 7.23 (m, 3H), 7.31 - 7.42 (m, 2H), 7.57 - 7.62 (m, 1H), 8.39 (s, 1H), 11.13 (s, 1H).

### Beispiel 15

### Ethyl-3-(2,3-dichlorbenzyl)-1-(1-methyl-2-oxo-2,3-dihydro-1H-benzimidazol-5-yl)-2,4-dioxo-1,2,3,4-tetrahydropyrimidin-5-carboxylat

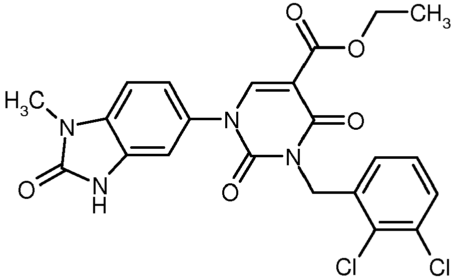

Darstellung und Reinigung der Titelverbindung erfolgten analog zu Beispiel 1. Die Reaktionszeit betrug 5h. Ausgehend von 200 mg (0.61 mmol) Ethyl-1-(1-methyl-2-oxo-2,3-dihydro-1H-benzimidazol-5-yl)-2,4-dioxo-1,2,3,4-tetrahydro-pyrimidin-5-carboxylat aus Beispiel 36A und 130 mg (0.67 mmol) 2,3-Dichlorbenzylchlorid erhielt man nach zusätzlicher Reinigung mittels präparativer HPLC (Methode 8) 40 mg (13 % d. Th.) der Titelverbindung.
LC-MS (Methode 1): Rₜ = 0.94 min; m/z = 489 (M+H)⁺.
¹H-NMR (400MHz, DMSO-d₆): δ [ppm] = 1.23 (t, 3H), 3.30 (s, teilweise durch Wasser-Signal verdeckt), 4.19 (q, 2H), 5.09 (s, 2H), 7.16 - 7.27 (m, 4H), 7.32 (t, 1H), 7.56 - 7.60 (m, 1H), 8.41 (s, 1H), 11.14 (s, 1H).

### Beispiel 16

### Ethyl-3-[2-chlor-3-(trifluormethyl)benzyl]-1-(1-methyl-2-oxo-2,3-dihydro-1H-benzimidazol-5-yl)-2,4-dioxo-1,2,3,4-tetrahydropyrimidin-5-carboxylat

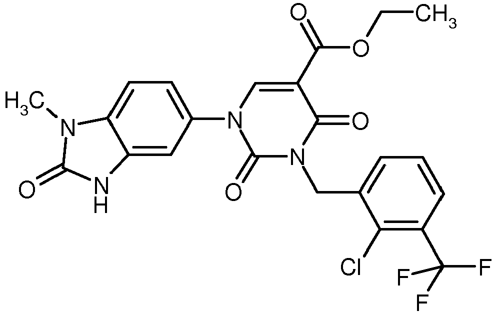

Darstellung und Reinigung der Titelverbindung erfolgten analog zu Beispiel 1. Die Reaktionszeit betrug 5h. Ausgehend von 200 mg (0.61 mmol) Ethyl-1-(1-methyl-2-oxo-2,3-dihydro-1H-benzimidazol-5-yl)-2,4-dioxo-1,2,3,4-tetrahydropyrimidin-5-carboxylat aus Beispiel 36A und 182 mg (0.67 mmol) 2-Chlor-3-(trifluoromethyl)benzylbromid erhielt man nach Reinigung mittels präparativer HPLC (Methode 8) 33 mg (10 % d. Th.) der Titelverbindung.
LC-MS (Methode 1): Rₜ = 0.97 min; m/z = 523 (M+H)⁺.
¹H-NMR (400MHz, DMSO-d₆): δ [ppm] = 1.23 (t, 3H), 3.30 (s, teilweise durch Wasser-Signal verdeckt), 4.20 (q, 2H), 5.14 (s, 2H), 7.19 - 7.23 (m, 3H), 7.48 - 7.55 (m, 1H), 7.58 - 7.62 (m, 1H), 7.78 - 7.82 (m, 1H), 8.42 (s, 1H), 11.14 (s, 1H).

### Beispiel 17

### Ethyl-3-[3-chlor-2-(trifluormethyl)benzyl]-1-(1-methyl-2-oxo-2,3-dihydro-1H-benzimidazol-5-yl)-2,4-dioxo-1,2,3,4-tetrahydropyrimidin-5-carboxylat

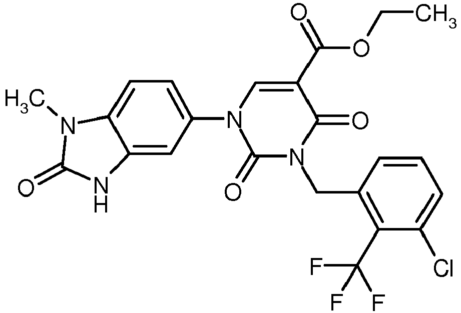

Zu einer Lösung von 0.74 g (2.24 mmol) Ethyl-1-(1-methyl-2-oxo-2,3-dihydro-1H-benzimidazol-5-yl)-2,4-dioxo-1,2,3,4-tetrahydropyrimidin-5-carboxylat aus Beispiel 36A in 28 mL DMF wurden 1.04 g (65 %-ige Reinheit, 2.46 mmol) 1-(Brommethyl)-3-chlor-2-(trifluormethyl)benzol (Herstellung: siehe WO 2004/52858, Seite 149, Beispiel 176), 0.62 g (4.48 mmol) Kaliumcarbonat sowie 0.04 g (0.22 mmol) Kaliumiodid addiert und die Mischung 5 h bei 60°C gerührt. Zur Aufarbeitung wurde das Reaktionsgemisch mit Wasser versetzt und dreimal mit Essigsäureethylester extrahiert. Die vereinigten organischen Phasen wurden mit gesättigter Natriumchloridlösung gewaschen, über Magnesiumsulfat getrocknet, filtriert und eingeengt. Der Rückstand wurde mittels Flash-Kieselgelchromatographie (Dichlormethan/Methanol, 50:1) gereinigt. Man erhielt 0.36 g (29 % d. Th.) der Zielverbindung.
LC-MS (Methode 1): Rₜ = 0.96 min; MS (ESIpos): m/z = 523 (M+H)⁺.
¹H-NMR (400 MHz, DMSO-d₆): δ [ppm] = 1.23 (t, 3H), 3.3 (s, durch DMSO-Signal verdeckt), 4.19 (q, 2H), 5.18 - 5.24 (m, 2H), 7.16 - 7.23 (m, 3H), 7.33 - 7.38 (m, 1H), 7.55 - 7.67 (m, 2H), 8.43 (s, 1H), 11.15 (s, 1H).

### Beispiel 18

### Ethyl-3-[3-chlor-2-(trifluormethyl)benzyl]-1-(3-ethyl-1-methyl-2-oxo-2,3-dihydro-1H-benz-imidazol-5-yl)-2,4-dioxo-1,2,3,4-tetrahydropyrimidin-5-carboxylat

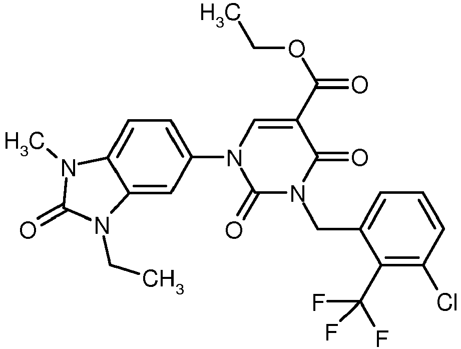

120 mg (0.23 mmol) Ethyl-3-[3-chlor-2-(trifluormethyl)benzyl]-1-(1-methyl-2-oxo-2,3-dihydro-1H-benzimidazol-5-yl)-2,4-dioxo-1,2,3,4-tetrahydropyrimidin-5-carboxylat aus Beispiel 17 wurden in DMF (3 ml) vorgelegt, 39 mg (0.25 mmol) Iodethan, 63 mg (0.46 mmol) Kaliumcarbonat und 4 mg (0.02 mmol) Kaliumiodid wurden zugegeben. Die Reaktionsmischung ließ man 5h bei 60°C rühren. Die auf RT abgekühlte Reaktionsmischung wurde mit Wasser versetzt, der ausgefallene Niederschlag abgesaugt, mit Wasser und MTBE nachgewaschen und im Vakuum bei 50°C getrocknet. Nach zusätzlicher Reinigung mittels Flashchromatographie (Dichlormethan/Methanol 70:1) erhielt man 73 mg (55 % d. Th.) der Titelverbindung.
LC-MS (Methode 1): Rₜ = 1.11 min; m/z = 551 (M+H)⁺.
¹H-NMR (400MHz, DMSO-d₆): δ [ppm] = 1.19 - 1.26 (m, 6H), 3.37 (s, 3H), 3.87 (q, 2H), 4.20 (q, 2H), 5.20 - 5.25 (m, 2H), 7.22 - 7.30 (m, 2H), 7.31 - 7.35 (m, 1H), 7.44 - 7.46 (m, 1H), 7.57 - 7.67 (m, 2H), 8.47 (s, 1H).

### Beispiel 19

### Ethyl-3-[2-chlor-3-(trifluormethyl)benzyl]-1-(3-ethyl-l-methyl-2-oxo-2,3-dihydro-1H-benz-imidazol-5-yl)-2,4-dioxo-1,2,3,4-tetrahydropyrimidin-5-carboxylat

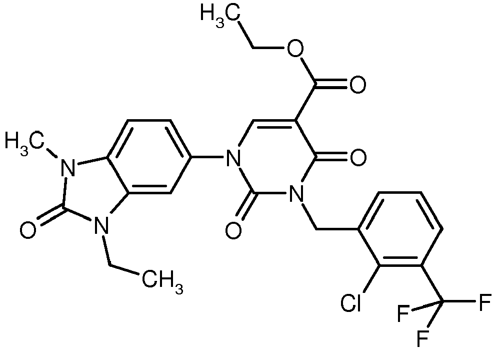

Darstellung und Reinigung der Titelverbindung erfolgten analog zu Beispiel 18. Ausgehend von 90 mg (0.17 mmol) Ethyl-3-[2-chlor-3-(trifluormethyl)benzyl]-1-(1-methyl-2-oxo-2,3-dihydro-1H-benzimidazol-5-yl)-2,4-dioxo-1,2,3,4-tetrahydropyrimidin-5-carboxylat aus Beispiel 16 und 29 mg (0.19 mmol) Iodethan erhielt man 75 mg (77 % d. Th.) der Titelverbindung.
LC-MS (Methode 1): Rₜ = 1.11 min; m/z = 551 (M+H)⁺.
¹H-NMR (400MHz, DMSO-d₆): δ [ppm] = 1.19 - 1.27 (m, 6H), 3.37 (s, teilweise durch Wasser-Signal verdeckt), 3.88 (q, 2H), 4.20 (q, 2H), 5.16 (s, 2H), 7.22 - 7.31 (m, 2H), 7.44 - 7.48 (m, 1H), 7.50 - 7.60 (m, 2H), 7.78 - 7.82 (m, 1H), 8.47 (s, 1H).

### Beispiel 20

### Ethyl-1-(3-ethyl-1-methyl-2-oxo-2,3-dihydro-1H-benzimidazol-5-yl)-3-[2-methyl-3-(trifluor-methyl)benzyl]-2,4-dioxo-1,2,3,4-tetrahydropyrimidin-5-carboxylat

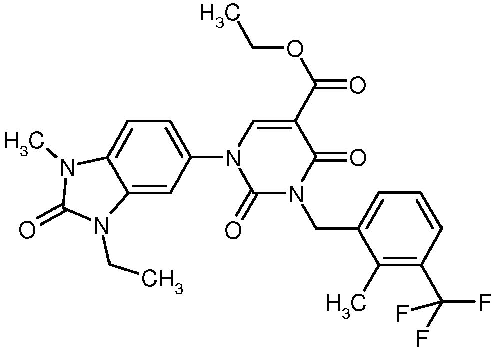

Darstellung und Reinigung der Titelverbindung erfolgten analog zu Beispiel 18. Ausgehend von 214 mg (0.42 mmol) Ethyl-1-(1-methyl-2-oxo-2,3-dihydro-1H-benzimidazol-5-yl)-3-[2-methyl-3-(trifluormethyl)benzyl]-2,4-dioxo-1,2,3,4-tetrahydropyrimidin-5-carboxylat aus Beispiel 14 und 73 mg (0.47 mmol) Iodethan erhielt man 152 mg (65 % d. Th.) der Titelverbindung.
LC-MS (Methode 1): Rₜ = 1.06 min; m/z = 531 (M+H)⁺.
¹H-NMR (400MHz, DMSO-d₆): δ [ppm] = 1.19 - 1.26 (m, 6H), 2.46 (s, teilweise durch DMSO-Signal verdeckt), 3.37 (s, teilweise durch Wasser-Signal verdeckt), 3.87 (q, 2H), 4.20 (q, 2H), 5.09 (s, 2H), 7.23 - 7.30 (m, 2H), 7.33 - 7.39 (m, 2H), 7.47 - 7.49 (m, 1H), 7.59 - 7.62 (m, 1H), 8.44 (s, 1H).

### Beispiel 21

### Ethyl-3-[2-chlor-3-(trifluormethyl)benzyl]-1-(1,3-diethyl-2-oxo-2,3-dihydro-1H-benzimidazol-5-yl)-2,4-dioxo-1,2,3,4-tetrahydropyrimidin-5-carboxylat

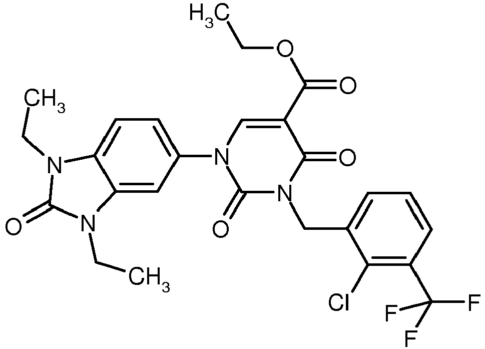

Darstellung und Reinigung der Titelverbindung erfolgten analog zu Beispiel 1. Die Reaktionszeit betrug 5h. Ausgehend von 200 mg (0.54 mmol) Ethyl-1-(1,3-diethyl-2-oxo-2,3-dihydro-1H-benzimidazol-5-yl)-2,4-dioxo-1,2,3,4-tetrahydropyrimidin-5-carboxylat aus Beispiel 22A und 162 mg (0.59 mmol) 2-Chlor-3-(trifluoromethyl)benzylbromid erhielt man 204 mg (66 % d. Th.) der Titelverbindung.
LC-MS (Methode 1): Rₜ = 1.17 min; m/z = 565 (M+H)⁺.
¹H-NMR (400MHz, DMSO-d₆): δ [ppm] = 1.19 - 1.26 (m, 9H), 3.84 - 3.95 (m, 4H), 4.20 (q, 2H), 5.16 (s, 2H), 7.22 - 7.27 (m, 1H), 7.34 (d, 1H), 7.44 - 7.48 (m, 1H), 7.50 - 7.60 (m, 2H), 7.78 - 7.83 (m, 1H), 8.48 (s, 1H).

### Beispiel 22

### Ethyl-1-(1,3-diethyl-2-oxo-2,3-dihydro-1H-benzimidazol-5-yl)-3-[2-methyl-3-(trifluormethyl)-benzyl]-2,4-dioxo-1,2,3,4-tetrahydropyrimidin-5-carboxylat

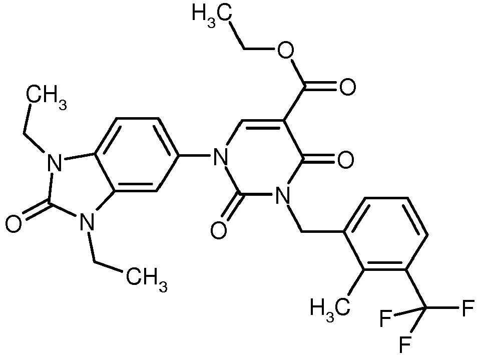

Darstellung und Reinigung der Titelverbindung erfolgten analog zu Beispiel 1. Die Reaktionszeit betrug 5h. Ausgehend von 200 mg (0.54 mmol) Ethyl-1-(1,3-diethyl-2-oxo-2,3-dihydro-1H-benzimidazol-5-yl)-2,4-dioxo-1,2,3,4-tetrahydropyrimidin-5-carboxylat aus Beispiel 22A und 150 mg (0.59 mmol) 2-Methyl-3-(trifluormethyl)benzylbromid erhielt man 174 mg (59 % d. Th.) der Titelverbindung.
LC-MS (Methode 1): Rₜ = 1.16 min; m/z = 545 (M+H)⁺.
¹H-NMR (400MHz, DMSO-d₆): δ [ppm] = 1.23 (d, 9H), 2.46 (s, 3H), 3.83 - 3.95 (m, 4H), 4.20 (q, 2H), 5.09 (s, 2H), 7.22 - 7.26 (m, 1H), 7.31 - 7.41 (m, 3H), 7.46 - 7.49 (m, 1H), 7.58 - 7.62 (m, 1H), 8.46 (s, 1H).

### Beispiel 23

### Ethyl-3-(2,3-dichlorbenzyl)-1-(1,3-diethyl-2-oxo-2,3-dihydro-1H-benzimidazol-5-yl)-2,4-dioxo-1,2,3,4-tetrahydropyrimidin-5-carboxylat

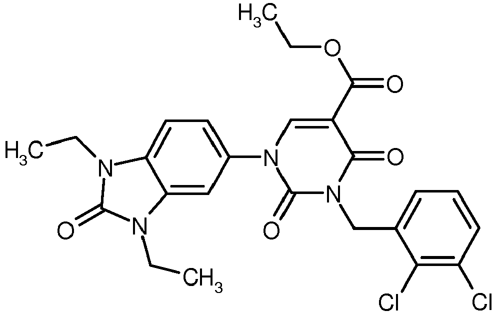

Darstellung und Reinigung der Titelverbindung erfolgten analog zu Beispiel 1. Die Reaktionszeit betrug 5h. Ausgehend von 200 mg (0.53 mmol) Ethyl-1-(1,3-diethyl-2-oxo-2,3-dihydro-1H-benzimidazol-5-yl)-2,4-dioxo-1,2,3,4-tetrahydropyrimidin-5-carboxylat aus Beispiel 22A und 115 mg (0.59 mmol) 2,3-Dichlorbenzylchlorid erhielt man 244 mg (81 % d. Th.) der Titelverbindung.
LC-MS (Methode 1): Rₜ = 1.15 min; m/z = 531 (M+H)⁺.
¹H-NMR (400MHz, DMSO-d₆): δ [ppm] = 1.18 - 1.27 (m, 9H), 3.82 - 3.97 (m, 4H), 4.20 (q, 2H), 5.11 (s, 2H), 7.19 - 7.27 (m, 2H), 7.30 - 7.38 (m, 2H), 7.46 (d, 1H), 7.59 (d, 1H), 8.47 (s, 1H).

### Beispiel 24

### Ethyl-1-(1,3-diethyl-2-oxo-2,3-dihydro-1H-benzimidazol-5-yl)-3-[3-fluor-2-(trifluormethyl)benzyl]-2,4-dioxo-1,2,3,4-tetrahydropyrimidin-5-carboxylat

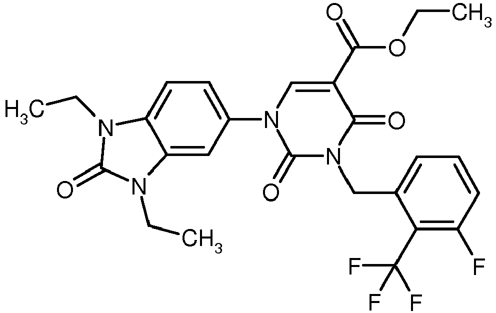

Darstellung und Reinigung der Titelverbindung erfolgten analog zu Beispiel 1. Die Reaktionszeit betrug 5h. Ausgehend von 165 mg (0.44 mmol) Ethyl-1-(1,3-diethyl-2-oxo-2,3-dihydro-1H-benzimidazol-5-yl)-2,4-dioxo-1,2,3,4-tetrahydropyrimidin-5-carboxylat aus Beispiel 22A und 125 mg (0.48 mmol) 1-(Brommethyl)-3-fluor-2-(trifluormethyl)benzol erhielt man 198 mg (82 % d. Th.) der Titelverbindung.
LC-MS (Methode 3): Rₜ = 1.28 min; m/z = 549 (M+H)⁺.
¹H-NMR (400MHz, DMSO-d₆): δ [ppm] = 1.23 (td, 9H), 3.82 - 3.96 (m, 4H), 4.20 (q, 2H), 5.21 (s, 2H), 7.17 - 7.26 (m, 2H), 7.31 - 7.49 (m, 3H), 7.67 (q, 1H), 8.49 (s, 1H).

### Beispiel 25

### Ethyl-3-[3-chlor-2-(trifluormethyl)benzyl]-1-[1-methyl-2-oxo-3-(2,2,2-trifluorethyl)-2,3-dihydro-1H-benzimidazol-5-yl]-2,4-dioxo-1,2,3,4-tetrahydropyrimidin-5-carboxylat

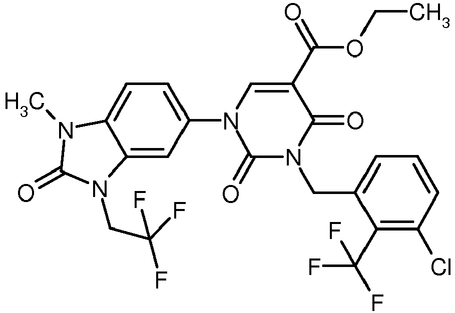

121 mg (0.23 mmol) Ethyl-3-[3-chlor-2-(trifluormethyl)benzyl]-1-(1-methyl-2-oxo-2,3-dihydro-1H-benzimidazol-5-yl)-2,4-dioxo-1,2,3,4-tetrahydropyrimidin-5-carboxylat aus Beispiel 17 wurden in DMF (3 ml) vorgelegt, 76 µL (130 mg, 0.46 mmol) 2,2,2-Trifluoroethyltrichlormethansulfonat, 64 mg (0.46 mmol) Kaliumcarbonat und 4 mg (0.02 mmol) Kaliumiodid wurden zugegeben. Die Reaktionsmischung ließ man 5h bei 60°C rühren. Die auf RT abgekühlte Reaktionsmischung wurde mit Wasser versetzt, der ausgefallene Niederschlag abgesaugt, mit Wasser und MTBE nachgewaschen und im Vakuum bei 50°C getrocknet. Nach zusätzlicher Reinigung mittels Flashchromatographie (Dichlormethan/Methanol 70:1) erhielt man 91 mg (63 % d. Th.) der Titelverbindung.
LC-MS (Methode 1): Rₜ = 1.16 min; m/z = 605 (M+H)⁺.
¹H-NMR (400MHz, DMSO-d₆): δ [ppm] = 1.23 (t, 3H), 3.41 (s, 3H), 4.20 (q, 2H), 4.80 (q, 2H), 5.22 (br.s, 2H), 7.31 - 7.39 (m, 3H), 7.53 (s, 1H), 7.57 - 7.67 (m, 2H), 8.44 (s, 1H).

### Beispiel 26

### Ethyl-3-[2-chlor-3-(trifluormethyl)benzyl]-1-[1-methyl-2-oxo-3-(2,2,2-trifluorethyl)-2,3-dihydro-1H-benzimidazol-5-yl]-2,4-dioxo-1,2,3,4-tetrahydropyrimidin-5-carboxylat

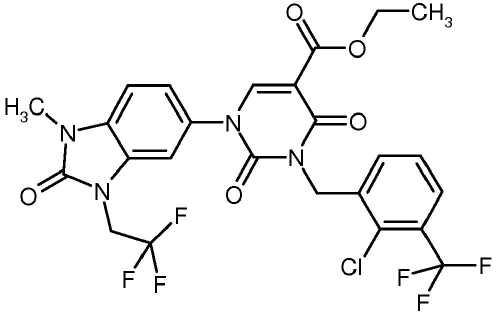

Darstellung und Reinigung der Titelverbindung erfolgten analog zu Beispiel 25. Ausgehend von 89 mg (0.17 mmol) Ethyl-3-[2-chlor-3-(trifluormethyl)benzyl]-1-(1-methyl-2-oxo-2,3-dihydro-1H-benzimidazol-5-yl)-2,4-dioxo-1,2,3,4-tetrahydropyrimidin-5-carboxylat aus Beispiel 16 und 96 mg (0.34 mmol) 2,2,2-Trifluorethyltrichlormethansulfonat erhielt man 80 mg (75 % d. Th.) der Titelverbindung.
LC-MS (Methode 1): Rₜ = 1.16 min; m/z = 605 (M+H)⁺.
¹H-NMR (400MHz, DMSO-d₆): δ [ppm] = 1.23 (t, 3H), 3.41 (s, 3H), 4.21 (q, 2H), 4.80 (q, 2H), 5.15 (s, 2H), 7.31 - 7.39 (m, 2H), 7.50 - 7.56 (m, 2H), 7.57 - 7.61 (m, 1H), 7.78 - 7.82 (m, 1H), 8.43 (s, 1H).

### Beispiel 27

### Ethyl-1-[1-methyl-2-oxo-3-(2,2,2-trifluorethyl)-2,3-dihydro-1H-benzimidazol-5-yl]-3-[2-methyl-3-(trifluormethyl)benzyl]-2,4-dioxo-1,2,3,4-tetrahydropyrimidin-5-carboxylat

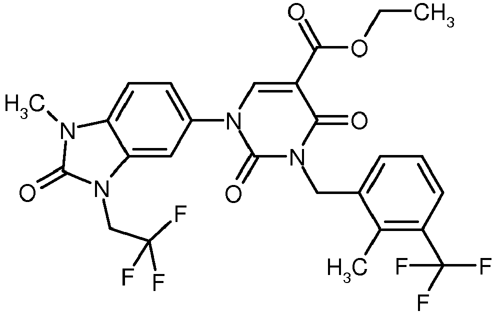

Darstellung und Reinigung der Titelverbindung erfolgten analog zu Beispiel 25. Ausgehend von 133 mg (Reinheit 75 %, 0.19 mmol) Ethyl-1-(1-methyl-2-oxo-2,3-dihydro-1H-benzimidazol-5-yl)-3-[2-methyl-3-(trifluormethyl)benzyl]-2,4-dioxo-1,2,3,4-tetrahydropyrimidin-5-carboxylat aus Beispiel 14 und 111 mg (0.39 mmol) 2,2,2-Trifluorethyltrichlormethansulfonat erhielt man nach Reinigung mittels Flashchromatographie (Dichlormethan/Methanol 100:1) 41 mg (35 % d. Th.) der Titelverbindung.
LC-MS (Methode 1): Rₜ = 1.15 min; m/z = 585 (M+H)⁺.
¹H-NMR (400MHz, DMSO-d₆): δ [ppm] = 1.23 (t, 3H), 2.46 (s, 3H), 3.41 (s, 3H), 4.20 (q, 2H), 4.79 (q, 2H), 5.08 (s, 2H), 7.32 - 7.41 (m, 4H), 7.54 - 7.57 (m, 1H), 7.58 - 7.63 (m, 1H), 8.41 (s, 1H).

### Beispiel 28

### Ethyl-1-[1-methyl-2-oxo-3-(2,2,2-trifluorethyl)-2,3-dihydro-1H-benzimidazol-5-yl]-2,4-dioxo-3-[(1R)-4-(trifluormethyl)-2,3-dihydro-1H-inden-1-yl]-1,2,3,4-tetrahydropyrimidin-5-carboxylat (R-Enantiomer)

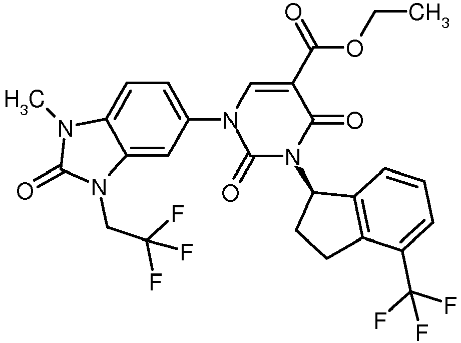

400 mg (0.97 mmol) Ethyl-l-[1-methyl-2-oxo-3-(2,2,2-trifluorethyl)-2,3-dihydro-1H-benzimidazol-5-yl]-2,4-dioxo-1,2,3,4-tetrahydropyrimidin-5-carboxylat aus Beispiel 25A, 235 mg (1.16 mmol) (1S)-4-(Trifluormethyl)indan-1-ol aus Beispiel 14A und 763 mg (2.91 mmol) Triphenylphosphin wurden unter Argon in DMF/THF 1:1 (19.6 ml) vorgelegt, die Reaktionsmischung auf -15°C abgekühlt und mit 0.53 ml (2.71 mmol) Diisopropylazodicarboxylat versetzt.. Man ließ die Reaktionsmischung 30 min bei RT rühren, anschließend wurden unter Eiskühlung weitere 0.2 Äquivalente (38 µL, 0.19 mmol) Diisopropylazodicarboxylat zugetropft und 1 h bei RT gerührt. Das Reaktionsgemisch wurde auf 0°C abgekühlt, mit 1 N Salzsäure versetzt und 15 min bei RT gerührt. Die entstandene Lösung wurde mit Essigsäureethylester extrahiert. Die organische Phase wurde nacheinander zweimal mit 1 N Salzsäure, zweimal mit gesättigter Natriumcarbonatlösung und einmal mit gesättigter Natriumchlorid-Lösung gewaschen, über Magnesiumsulfat getrocknet und eingeengt. Der Rückstand wurde per präparativer HPLC (Methode 7) gereinigt. Man erhielt 370 mg (57 % d. Th.) der Titelverbindung.
LC-MS (Methode 5) Rₜ = 1.17 min; m/z = 597 (M+H)⁺.
¹H-NMR (400MHz, CD₂Cl₂): δ [ppm] = 1.23 (t, 3H), 2.29 - 2.42 (m, 1H), 2.43 - 2.57 (m, 1H), 3.00 - 3.12 (m, 1H), 3.31 - 3.44 (m, 4H), 4.20 (q, 2H), 4.41 (q, 2H), 6.47 - 6.60 (m, 1H), 6.94 - 7.07 (m, 3H), 7.17 - 7.28 (m, 2H), 7.41 (d, 1H), 8.22 (s, 1H).

### Beispiel 29

### Ethyl-3-[2-chlor-3-(trifluormethyl)benzyl]-1-[3-(cyclopropylmethyl)-1-methyl-2-oxo-2,3-dihydro-1H-benzimidazol-5-yl]-2,4-dioxo-1,2,3,4-tetrahydropyrimidin-5-carboxylat

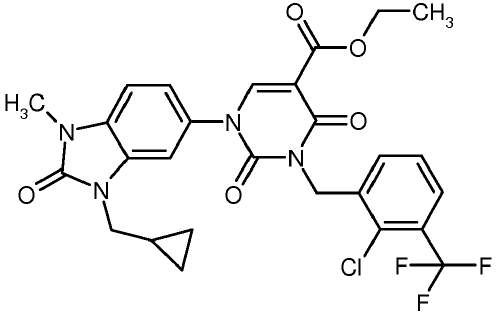

Zu einer Lösung von 90 mg (0.17 mmol) Ethyl-3-[2-chlor-3-(trifluormethyl)benzyl]-1-(1-methyl-2-oxo-2,3-dihydro-1H-benzimidazol-5-yl)-2,4-dioxo-1,2,3,4-tetrahydropyrimidin-5-carboxylat aus Beispiel 16 in DMF (2 ml) gab man 254 mg (0.18 mmol) (Brommethyl)cyclopropan, 47 mg Kaliumcarbonat und 3 mg Kaliumiodid. Anschließend ließ man die Reaktionsmischung 5 h bei 60°C rühren. Nach Abkühlung auf RT wurde Wasser zugegeben und der entstandene Niederschlag abfiltriert. Der Feststoff wurde nacheinander mit Wasser und MTBE gewaschen und im Vakuum bei 50°C getrocknet. Der Feststoff wurde in Dichlormethan gelöst und mittels Flashchromatographie (Dichlormethan/Methanol 70/1) gereinigt. Das erhaltene Produkt wurde im Hochvakuum getrocknet. Man erhielt 67 mg (66 % d. Th.) der Titelverbindung.
LC-MS (Methode 1): Rₜ = 1.17 min; m/z = 577 (M+H)⁺.
¹H-NMR (400MHz, DMSO-d₆): δ = 0.34 - 0.50 (m, 4H), 1.14 - 1.26 (m, 4H), 3.38 (s, 3H), 3.72 (d, 2H), 4.20 (q, 2H), 5.15 (s, 2H), 7.23 - 7.31 (m, 2H), 7.50 - 7.60 (m, 3H), 7.80 (d, 1H), 8.46 (s, 1H).

### Beispiel 30

### Ethyl-1-[3-(cyclopropylmethyl)-1-methyl-2-oxo-2,3-dihydro-1H-benzimidazol-5-yl]-3-[2-methyl-3-(trifluormethyl)benzyl]-2,4-dioxo-1,2,3,4-tetrahydropyrimidin-5-carboxylat

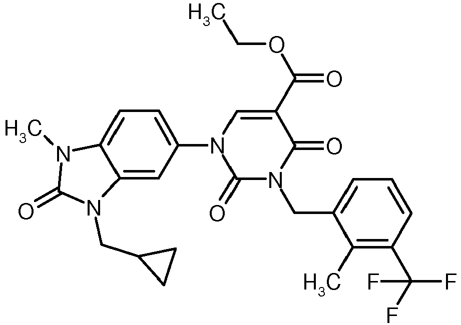

Darstellung und Reinigung der Titelverbindung erfolgten analog zu Beispiel 29. Ausgehend von 133 mg (75 %-ige Reinheit, 0.19 mmol) Ethyl-1-(1-methyl-2-oxo-2,3-dihydro-1H-benzimidazol-5-yl)-3-[2-methyl-3-(trifluormethyl)benzyl]-2,4-dioxo-1,2,3,4-tetrahydropyrimidin-5-carboxylat aus Beispiel 14 und 29 mg (0.18 mmol) (Brommethyl)cyclopropan erhielt man 69 mg (56 % d. Th.) der Titelverbindung.
LC-MS (Methode 1): Rₜ = 1.16 min; m/z = 557 (M+H)⁺.
¹H-NMR (400MHz, DMSO-d₆): δ [ppm] = 0.35 - 0.41 (m, 2H), 0.42 - 0.49 (m, 2H), 1.16 - 1.26 (m, 4H), 2.46 (s, 3H), 3.38 (s, 3H), 3.72 (d, 2H), 4.20 (q, 2H), 5.09 (s, 2H), 7.23 - 7.30 (m, 2H), 7.33 - 7.40 (m, 2H), 7.52 - 7.54 (m, 1H), 7.59 - 7.62 (m, 1H), 8.44 (s, 1H).

### Beispiel 31

### Ethyl-3-[3-chlor-2-(trifluormethyl)benzyl]-1-[3-(cyclopropylmethyl)-1-methyl-2-oxo-2,3-dihydro-1H-benzimidazol-5-yl]-2,4-dioxo-1,2,3,4-tetrahydropyrimidin-5-carboxylat

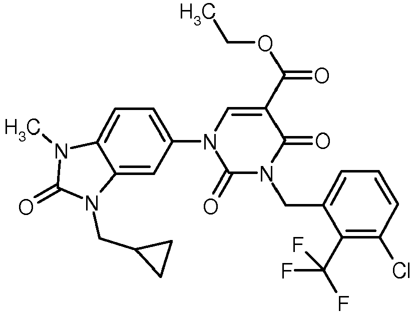

Darstellung und Reinigung der Titelverbindung erfolgten analog zu Beispiel 29. Ausgehend von 120 mg (0.23 mmol) Ethyl-3-[3-chlor-2-(trifluormethyl)benzyl]-1-(1-methyl-2-oxo-2,3-dihydro-1H-benzimidazol-5-yl)-2,4-dioxo-1,2,3,4-tetrahydropyrimidin-5-carboxylat aus Beispiel 17 und 34 mg (0.25 mmol) (Brommethyl)cyclopropan erhielt man 89 mg (62 % d. Th.) der Titelverbindung.
LC-MS (Methode 1): Rₜ = 1.17 min; m/z = 577 (M+H)⁺.
¹H-NMR (400MHz, DMSO-d₆): δ [ppm] = 0.35 - 0.41 (m, 2H), 0.41 - 0.49 (m, 2H), 1.14 - 1.27 (m, 4H), 3.38 (s, 3H), 3.72 (d, 2H), 4.20 (q, 2H), 5.22 (br.s, 2H), 7.22 - 7.30 (m, 2H), 7.31 - 7.36 (m, 1H), 7.49 - 7.52 (m, 1H), 7.57 - 7.67 (m, 2H), 8.47 (s, 1H).

### Beispiel 32

### Ethyl-3-[2-chlor-3-(trifluormethyl)benzyl]-1-[1-methyl-3-(oxetan-2-ylmethyl)-2-oxo-2,3-dihydro-1H-benzimidazol-5-yl]-2,4-dioxo-1,2,3,4-tetrahydropyrimidin-5-carboxylat (Racemat)

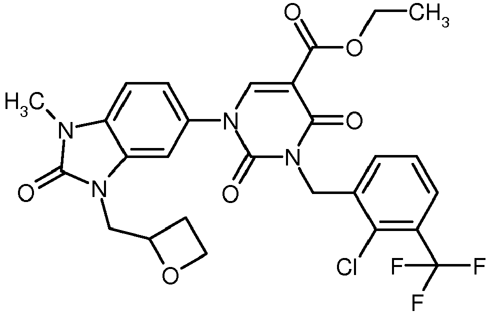

100 mg (0.23 mmol) Ethyl-3-[2-chlor-3-(trifluormethyl)benzyl]-1-(1-methyl-2-oxo-2,3-dihydro-1H-benzimidazol-5-yl)-2,4-dioxo-1,2,3,4-tetrahydropyrimidin-5-carboxylat aus Beispiel 16 wurden in 2.4 mL DMF vorgelegt, 32 mg (0.21 mmol) 2-(Brommethyl)oxetan, 53 mg (0.38 mmol) Kaliumcarbonat und 3 mg (0.02 mmol) Kaliumiodid wurden zugegeben. Die Reaktionsmischung wurde 2 h bei 60°C gerührt. Anschließend wurde bei RT erneut 1 Äquivalent 2-(Brommethyl)oxetan zugefügt und die Reaktionsmischung 2 h bei 80°C gerührt. Zur Aufarbeitung wurde das Reaktionsgemisch mit Wasser versetzt, der ausgefallene Niederschlag abgesaugt, mit Wasser gewaschen und im Vakuum bei 50°C getrocknet. Der Feststoff wurde in Dichlormethan gelöst und mittels Flash-Kieselgelchromatographie (Dichlormethan/Methanol 70:1) gereinigt. Man erhielt 56 mg (50 % d. Th.) der Titelverbindung.
LC-MS (Methode 1): Rₜ = 1.09 min; m/z = 593 (M+H)⁺.
¹H-NMR (400MHz, DMSO-d₆): δ = 1.23 (t, 3H), 2.10 - 2.22 (m, 1H), 2.25 - 2.38 (m, 1H), 3.36 (s, 3H), 3.39 - 3.48 (m, 1H), 3.73 (q, 1H), 3.85 - 3.92 (m, 1H), 3.92 - 3.98 (m, 1H), 4.11 - 4.17 (m, 1H), 4.21 (q, 2H), 5.15 (s, 2H), 7.25 - 7.33 (m, 2H), 7.45 (s, 1H), 7.53 (t, 1H), 7.60 (d, 1H), 7.80 (d, 1H), 8.46 (s, 1H).

### Beispiel 33

### Ethyl-1-(3-cyclobutyl-1-methyl-2-oxo-2,3-dihydro-1H-benzimidazol-5-yl)-3-[2-methyl-3-(trifluormethyl)benzyl]-2,4-dioxo-1,2,3,4-tetrahydropyrimidin-5-carboxylat

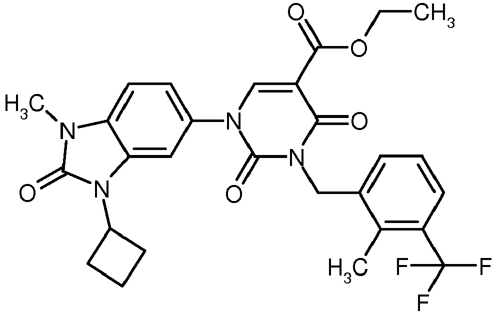

39 µL (0.49 mmol) Cyclobutanol und 130 mg (0.49 mmol) Triphenylphosphin wurden unter Argon in THF (2.5 ml) vorgelegt, 98 µL (0.49 mmol) Diisopropylazodicarboxylat langsam zugetropft und anschließend 100 mg (0.19 mmol) Ethyl-1-(1-methyl-2-oxo-2,3-dihydro-1H-benzimidazol-5-yl)-3-[2-methyl-3-(trifluormethyl)-benzyl]-2,4-dioxo-1,2,3,4-tetrahydropyrimidin-5-carboxylat aus Beispiel 14 zugegeben. Die Reaktionsmischung wurde 16 h bei RT gerührt. Der Ansatz wurde eingeengt und mittels präparativer HPLC (Methode 8) gereinigt. Man erhielt 46 mg (41 % d. Th.) der Titelverbindung.
LC-MS (Methode 1): Rₜ = 1.16 min; m/z = 557 (M+H)⁺.
¹H-NMR (400MHz, DMSO-d₆): δ [ppm] = 1.23 (t, 3H), 1.72 - 1.91 (m, 2H), 2.20 - 2.30 (m, 2H), 2.47 (s, teilweise durch DMSO-Signal verdeckt), 2.75 - 2.87 (m, 2H), 3.31 (s, teilweise durch Wasser-Signal verdeckt), 4.20 (q, 2H), 4.78 - 4.88 (m, 1H), 5.09 (s, 2H), 7.24 - 7.29 (m, 2H), 7.33 - 7.42 (m, 2H), 7.60 (d, 1H), 7.67 (s, 1H), 8.45 (s, 1H).

### Beispiel 34

### Ethyl-1-(3-isopropyl-1-methyl-2-oxo-2,3-dihydro-1H-benzimidazol-5-yl)-3-[2-methyl-3-(trifluormethyl)benzyl]-2,4-dioxo-1,2,3,4-tetrahydropyrimidin-5-carboxylat

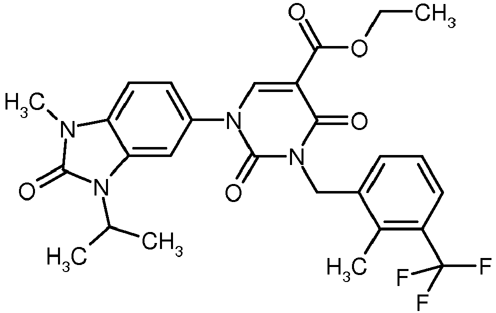

Darstellung und Reinigung der Titelverbindung erfolgten analog zu Beispiel 33. Ausgehend von 100 mg (0.19 mmol) Ethyl-1-(1-methyl-2-oxo-2,3-dihydro-1H-benzimidazol-5-yl)-3-[2-methyl-3-(trifluormethyl)benzyl]-2,4-dioxo-1,2,3,4-tetrahydropyrimidin-5-carboxylat aus Beispiel 14 und 38 µL (0.49 mmol) 2-Propanol erhielt man nach zusätzlicher Reinigung mittels präparativer HPLC (Methode 8) 38 mg (34 % d. Th.) der Titelverbindung.
LC-MS (Methode 1): Rₜ = 1.12 min; m/z = 545 (M+H)⁺.
¹H-NMR (400MHz, DMSO-d₆): δ [ppm] = 1.23 (t, 3H), 1.45 (d, 6H), 2.46 (s, teilweise durch DMSO-Signal verdeckt), 3.31 (s, teilweise durch Wasser-Signal verdeckt), 4.20 (q, 2H), 4.54 - 4.65 (m, 1H), 5.09 (s, 2H), 7.22 - 7.29 (m, 2H), 7.32 - 7.41 (m, 2H), 7.58 - 7.62 (m, 2H), 8.43 (s, 1H).

### Beispiel 35

### Ethyl-1-(3-cyclopropyl-1-methyl-2-oxo-2,3-dihydro-1H-benzimidazol-5-yl)-3-[2-methyl-3-(trifluormethyl)benzyl]-2,4-dioxo-1,2,3,4-tetrahydropyrimidin-5-carboxylat

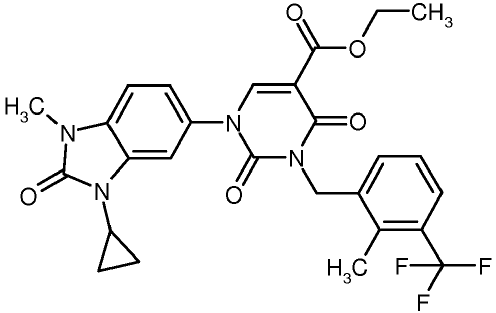

250 mg (0.49 mmol) Ethyl-1-(1-methyl-2-oxo-2,3-dihydro-1H-benzimidazol-5-yl)-3-[2-methyl-3-(trifluormethyl)benzyl]-2,4-dioxo-1,2,3,4-tetrahydropyrimidin-5-carboxylat aus Beispiel 14, 85 mg (0.99 mmol) Cyclopropylboronsäure und 0.41 ml (2.98 mmol) Triethylamin wurden in Dichlormethan (4 ml) vorgelegt. Molekularsieb (3Å) und 271 mg (1.49 mmol) Kupfer(II)acetat wurden zugegeben und die Reaktionsmischung 3 Tage bei RT gerührt. Der Ansatz wurde mit Essigsäureethylester verdünnt, zweimal mit IM Salzsäure, einmal mit gesättigter Natriumhydrogencarbonat-Lösung und einmal mit gesättigter Natriumchlorid-Lösung gewaschen. Die organische Phase wurde über Magnesiumsulfat getrocknet, abfiltriert und eingeengt. Der Rückstand wurde mittels präparativer HPLC (Methode 8) gereinigt. Man erhielt 155 mg (56 % d. Th.) der Titelverbindung.
LC-MS (Methode 1): Rₜ = 1.06 min; m/z = 543 (M+H)⁺.
¹H-NMR (400MHz, DMSO-d₆): δ [ppm] = 0.86 - 0.92 (m, 2H), 0.99 - 1.06 (m, 2H), 1.23 (t, 3H), 2.46 (s, teilweise durch DMSO-Signal verdeckt), 2.87 - 2.95 (m, 1H), 3.31 (s, teilweise durch Wasser-Signal verdeckt), 4.20 (q, 2H), 5.08 (s, 2H), 7.22 - 7.28 (m, 2H), 7.32 - 7.43 (m, 2H), 7.47 (s, 1H), 7.60 (d, 1H), 8.41 (s, 1H).

### Beispiel 36

### Ethyl-1-(3-methyl-2-oxo-2,3-dihydro-1H-benzimidazol-5-yl)-3-[2-methyl-3-(trifluormethyl)-benzyl]-2,4-dioxo-1,2,3,4-tetrahydropyrimidin-5-carboxylat

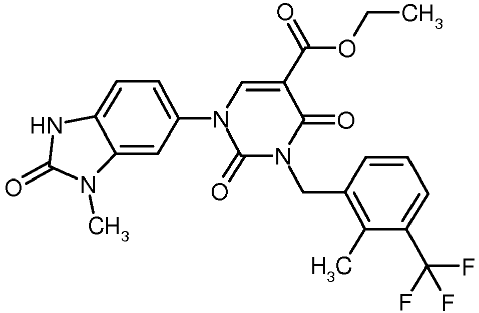

Zu einer Lösung von 250 mg (0.76 mmol) Ethyl-1-(3-methyl-2-oxo-2,3-dihydro-1H-benzimidazol-5-yl)-2,4-dioxo-1,2,3,4-tetrahydropyrimidin-5-carboxylat aus Beispiel 34A in 10 mL DMF wurden 211 mg (0.83 mmol) 1-(Brommethyl)-2-methyl-3-(trifluormethyl)benzol, 209 mg (1.51 mmol) Kaliumcarbonat sowie 13 mg (0.08 mmol) Kaliumiodid addiert und die Mischung 3 h bei 60°C gerührt. Zur Aufarbeitung wurde das Reaktionsgemisch mit Wasser versetzt, der entstandene Niederschlag abgesaugt, mit Wasser sowie MTBE gewaschen und über Nacht bei 50°C im Hochvakuum getrocknet. Man erhielt 42 mg (11 % d. Th.) der Zielverbindung.
LC-MS (Methode 3): Rₜ = 1.19 min; MS (ESIpos): m/z = 503 (M+H)⁺.
¹H-NMR (400 MHz, DMSO-d₆): δ [ppm] = 1.22 (t, 3H), 3.41 (s, 3H), 4.17 (q, 2H), 5.20 (s, 2H), 7.07 - 7.16 (m, 3H), 7.29 - 7.36 (m, 1H), 7.46 (s, 1H), 7.62 (d, 1H), 8.28 (s, 1H), 11.70 (s, 1H).

### Beispiel 37

### Ethyl-3-[2-chlor-3-(trifluormethyl)benzyl]-2,4-dioxo-1-(2-oxo-2,3-dihydro-1H-benzimidazol-5-yl)-1,2,3,4-tetrahydropyrimidin-5-carboxylat

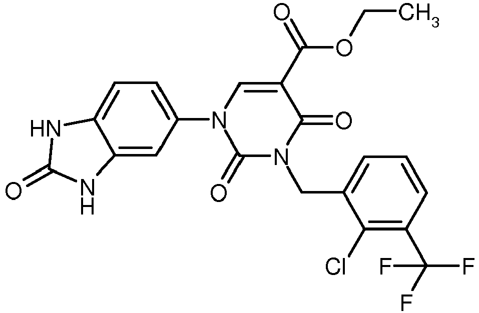

200 mg (0.63 mmol) Ethyl-2,4-dioxo-1-(2-oxo-2,3-dihydro-1H-benzimidazol-5-yl)-1,2,3,4-tetrahydropyrimidin-5-carboxylat aus Beispiel 26A wurden in 8 ml DMF vorgelegt. 190 mg (0.70 mmol) 1-(Brommethyl)-2-chlor-3-(trifluormethyl)benzol, 175 mg (1.27 mmol) Kaliumcarbonat und 10.5 mg (63 µmol) Kaliumiodid wurden zugegeben und die Reaktionsmischung 5 h bei 60°C gerührt. Nach Abkühlung auf RT wurde das Gemisch mit Wasser versetzt. Der Niederschlag wurde abfiltriert, mit wenig Wasser und MTBE gewaschen und im Trockenschrank bei 50°C getrocknet. Das erhaltene Produkt wurde in wenig DMF gelöst und mittels präparativer HPLC (Methode 8) gereinigt. Man erhielt 111 mg (35 % d. Th.) der Titelverbindung.
LC-MS (Methode 3): Rₜ = 1.13 min; m/z = 509 (M+H)⁺.
¹H-NMR (400MHz, DMSO-d₆): δ [ppm] = 1.23 (t, 3H), 4.20 (q, 2H), 5.14 (s, 2H), 7.02 (d, 1H), 7.09 (dd, 1H), 7.15 (s, 1H), 7.51 (t, 1H), 7.59 (d, 1H), 7.79 (d, 1H), 8.41 (s, 1H), 10.88 (d, 2H).

### Beispiel 38

### Ethyl-1-[3-methyl-2-oxo-1-(2,2,2-trifluorethyl)-2,3-dihydro-1H-benzimidazol-5-yl]-3-[2-methyl-3-(trifluormethyl)benzyl]-2,4-dioxo-1,2,3,4-tetrahydropyrimidin-5-carboxylat

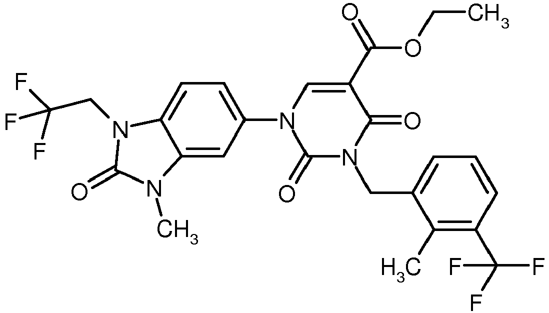

Darstellung und Reinigung der Titelverbindung erfolgten analog zu Beispiel 25. Ausgehend von 91 mg (0.18 mmol) Ethyl-1-(3-methyl-2-oxo-2,3-dihydro-1H-benzimidazol-5-yl)-3-[2-methyl-3-(trifluormethyl)benzyl]-2,4-dioxo-1,2,3,4-tetrahydropyrimidin-5-carboxylat aus Beispiel 36 und 101 mg (0.36 mmol) 2,2,2-Trifluorethyltrichlormethansulfonat erhielt man 57 mg (52 % d. Th.) der Titelverbindung.
LC-MS (Methode 1): Rₜ = 1.11 min; m/z = 585 (M+H)⁺.
¹H-NMR (400MHz, DMSO-d₆): δ [ppm] = 1.23 (t, 3H), 2.46 (s, 3H), 3.38 (s, 3H), 4.19 (q, 2H), 4.86 (q, 2H), 5.09 (s, 2H), 7.28 - 7.41 (m, 3H), 7.44 (d, 1H), 7.50 (d, 1H), 7.61 (d, 1H), 8.47 (s, 1H).

### Beispiel 39

### Ethyl-1-[1-(cyclopropylmethyl)-3-methyl-2-oxo-2,3-dihydro-1H-benzimidazol-5-yl]-3-[2-methyl-3-(trifluormethyl)benzyl]-2,4-dioxo-1,2,3,4-tetrahydropyrimidin-5-carboxylat

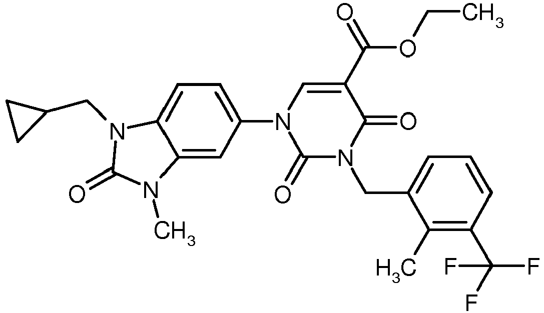

91 mg (0.18 mmol) Ethyl-1-(3-methyl-2-oxo-2,3-dihydro-1H-benzimidazol-5-yl)-3-[2-methyl-3-(trifluormethyl)benzyl]-2,4-dioxo-1,2,3,4-tetrahydropyrimidin-5-carboxylat aus Beispiel 36 wurden analog zu Beispiel 29 mit 26 mg (0.19 mmol) (Brommethyl)cyclopropan umgesetzt. Nach 2h Reaktionszeit wurden zusätzlich 24 mg (0.17 mmol) (Brommethyl)cyclopropan zugegeben und die Reaktionsmischung noch 1h bei 80°C gerührt. Das Produkt wurde durch Zugabe von Wasser gefällt und abfiltriert. Nach zusätzlicher Reinigung mittels Flashchromatographie (Dichlormethan/Methanol 70:1) erhielt man 51 mg (51 % d. Th.) der Titelverbindung.
LC-MS (Methode 1): Rₜ = 1.13 min; m/z = 557 (M+H)⁺.
¹H-NMR (400MHz, DMSO-d₆): δ = 0.36 - 0.42 (m, 2H), 0.42 - 0.50 (m, 2H), 1.14 - 1.20 (m, 1H), 1.23 (t, 3H), 2.46 (s, 3H), 3.35 (s, 3H), 3.77 (d, 2H), 4.19 (q, 2H), 5.09 (s, 2H), 7.24 (dd, 1H), 7.32 - 7.44 (m, 4H), 7.58 - 7.62 (m, 1H), 8.45 (s, 1H).

### Beispiel 40

### Ethyl-1-(3-methyl-2-oxo-2,3-dihydro-1,3-benzoxazol-6-yl)-3-[2-methyl-3-(trifluormethyl)benzyl]-2,4-dioxo-1,2,3,4-tetrahydropyrimidin-5-carboxylat

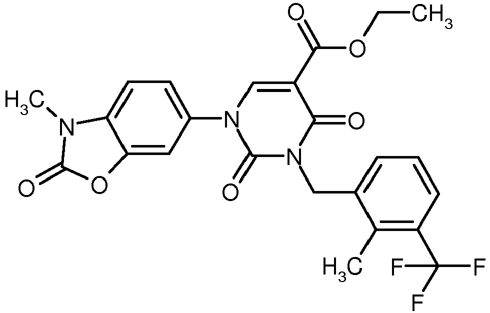

Darstellung und Reinigung der Titelverbindung erfolgten analog zu Beispiel 1 mit 2 h Reaktionszeit. Ausgehend von 200 mg (0.60 mmol) Ethyl-1-(3-methyl-2-oxo-2,3-dihydro-1,3-benzoxazol-6-yl)-2,4-dioxo-1,2,3,4-tetrahydropyrimidin-5-carboxylat aus Beispiel 28A und 168 mg (0.66 mmol) 1-(Brommethyl)-2-methyl-3-(trifluormethyl)benzol erhielt man 288 mg (93 % d. Th.) der Titelverbindung.
LC-MS (Methode 5): Rₜ = 1.10 min; m/z = 504 (M+H)⁺.
¹H-NMR (400MHz, DMSO-d₆): δ [ppm] = 1.23 (t, 3H), 2.46 (s, 3H), 3.38 (s, 3H), 4.20 (q, 2H), 5.07 (s, 2H), 7.31 - 7.42 (m, 3H), 7.43 - 7.48 (m, 1H), 7.58 - 7.62 (m, 1H), 7.63 - 7.66 (m, 1H), 8.44 (s, 1H).

### Beispiel 41

### Ethyl-3-[2-chlor-3-(trifluormethyl)benzyl]-1-(3-methyl-2-oxo-2,3-dihydro-1,3-benzoxazol-6-yl)-2,4-dioxo-1,2,3,4-tetrahydropyrimidin-5-carboxylat

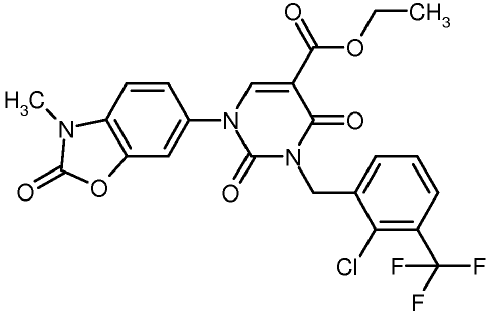

Darstellung und Reinigung der Titelverbindung erfolgten analog zu Beispiel 1. Die Reaktionszeit betrug 2h. Ausgehend von 200 mg (0.60 mmol) Ethyl-1-(3-methyl-2-oxo-2,3-dihydro-1,3-benzoxazol-6-yl)-2,4-dioxo-1,2,3,4-tetrahydropyrimidin-5-carboxylat aus Beispiel 28A und 181 mg (0.66 mmol) 1-(Brommethyl)-2-chlor-3-(trifluormethyl)benzol erhielt man 263 mg (79 % d. Th.) der Titelverbindung.
LC-MS (Methode 5): Rₜ = 1.11 min; m/z = 523 (M+H)⁺.
¹H-NMR (400MHz, DMSO-d₆): δ = 1.23 (t, 3H), 3.38 (s, 3H), 4.20 (q, 2H), 5.14 (s, 2H), 7.38 - 7.48 (m, 2H), 7.53 (t, 1H), 7.59 (d, 1H), 7.64 (s, 1H), 7.80 (d, 1H), 8.47 (s, 1H).

### Beispiel 42

### Ethyl-1-(3-methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carboxylat (R-Enantiomer)

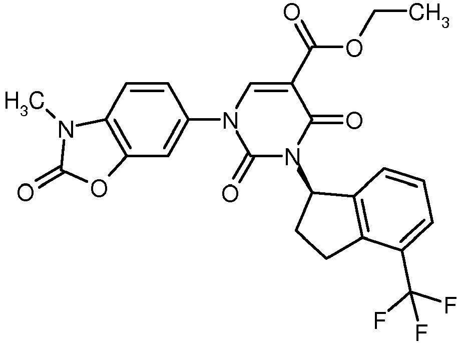

*Methode A:* Eine Lösung von 200 mg (0.60 mmol) Ethyl-1-(3-methyl-2-oxo-2,3-dihydro-1,3-benzoxazol-6-yl)-2,4-dioxo-1,2,3,4-tetrahydropyrimidin-5-carboxylat aus Beispiel 28A und 475 mg (1.81 mmol) Triphenylphosphin wurde unter Argon in THF/DMF 1:1 (7.6 ml) auf -30°C abgekühlt. 238 µl (1.20 mmol) Diisopropylazodicarboxylat wurden zugetropft und anschließend eine Lösung von 146 mg (0.69 mmol (1S)-4-(Trifluormethyl)indan-1-ol aus Beispiel 14A in ca. 1 ml THF zugetropft. Die Reaktionsmischung wurde auf RT erwärmt und 30 min bei RT gerührt. Zur Aufarbeitung wurde das Gemisch auf 0°C abgekühlt, mit 5 ml IM Salzsäure versetzt, auf RT erwärmt und 30 min gerührt. Anschließend wurde mit Essigsäureethylester extrahiert. Die organische Phase wurde zweimal mit IM Salzsäure und einmal mit gesättigter Natriumchlorid-Lösung gewaschen, über Magnesiumsulfat getrocknet und im Vakuum eingeengt. Der Rückstand wurde mit Ethanol ausgerührt, der ausgefallene Feststoff abgesaugt und verworfen. Das Filtrat wurde eingeengt, in wenig Dichlormethan gelöst und mittels Flashchromatographie (DichlormethanMethanol 120:1→ 20:1) gereinigt. Man erhielt 135 mg (43 % d. Th.) der Titelverbindung in ca. 95 %-iger Reinheit.
LC-MS (Methode 1): Rₜ = 1.13 min; m/z = 516 (M+H)⁺.
¹H-NMR (400MHz, DMSO-d₆): δ [ppm] = 1.22 (t, 3H), 2.37 - 2.43 (m, 1H), 2.43 - 2.48 (m, 1H, telweise verdeckt durch DMSO-Signal), 3.03 - 3.14 (m, 1H), 3.22 - 3.30 (m, 1H, teilweise verdeckt durch Wasser-Signal), 3.38 (s, 3H), 4.18 (q, 2H), 6.34 - 6.56 (m, 1H), 7.32 - 7.43 (m, 3H), 7.45 - 7.50 (m, 1H), 7.53 (d, 1H), 7.55 - 7.64 (m, 1H), 8.35 (s, 1H).

In einem analogen Versuch konnte eine Fraktion mit 99 %-iger Reinheit isoliert werden. Für diese Charge wurde der spezifizische optische Drehwert gemessen:
Spezifischer optischer Drehwert: α_{D}²⁰ = +132.9°, (Chloroform, c = 0.395 g/100 ml).

*Methode B:* Eine Lösung von 5.0 g (15.1 mmol) Ethyl-1-(3-methyl-2-oxo-2,3-dihydro-1,3-benzoxazol-6-yl)-2,4-dioxo-1,2,3,4-tetrahydropyrimidin-5-carboxylat aus Beispiel, 6.73 g (25.7 mmol) Triphenylphosphin und 3.66 g (18.1 mmol) (1S)-4-(Trifluormethyl)indan-1-ol aus Beispiel 14A wurde unter Argon in 240 ml DMF/THF 2:1 (v/v) vorgelegt und auf -15°C abgekühlt. 4.76 ml (24.15 mmol) Diisopropylazodicarboxylat wurden langsam so zugetropft, dass die Temperatur der Reaktionsmischung nicht über -10°C anstieg. Am Ende der Zugabe wurde die Mischung noch 1 h bei -10°C gerührt, dann auf RT erwärmt und auf 1.3 L Wasser gegossen. Das Gemisch wurde zweimal mit je 300 ml Essigsäureethylester extrahiert. Die vereinigten organischen Phasen wurden mit einer gesättigten Natriumchloridlösung gewaschen, über Magnesiumsulfat getrocknet und am Rotationsverdampfer vom Lösungsmittel befreit. Der Rückstand (18 g) wurde in zwei chromatographischen Schritten gereinigt: zuerst über eine 200 g Kieselgel-Säule mit Dichlormethan / Aceton 97.5 : 2.5 als Eluent. Die erhaltenen produkthaltigen Fraktionen wurden eingeengt und der Rückstand erneut über eine 200 g Kieselgel-Säule appliziert. Mit 2.5 L Cyclohexan/ Essigsäureethylester 1:1 als Eluent wurden weitere Verunreinigungen eluiert, dann wurde das gewünschte Produkt mit Dichlormethan / Methanol 95 : 5 aus der Säule eluiert. Man erhielt so 3.40 g (44 % d. Th.) der Titelverbindung in 95 %-iger Reinheit (NMR zeigte ca. 5 % Essigsäureethylester). Weitere 920 mg konnten durch erneute Reinigung einer Mischfraktion erhalten werden. Gesamtausbeute: 4.32 g (56 % d. Th.).
LC-MS (Methode 1): Rₜ = 1.15 min; m/z = 516 (M+H)⁺.
¹H-NMR (400MHz, CD₂Cl₂): δ [ppm] = 1.31 (t, 3H), 2.37 - 2.49 (m, 1H), 2.59 (dtd, 1H), 3.14 (dt, 1H), 3.40 (s, 3H), 3.42 - 3.53 (m, 1H), 4.29 (q, 2H), 6.54 - 6.68 (m, 1H), 7.06 (d, 1H), 7.17 (d, 1H), 7.22 (s, 1H), 7.26 - 7.36 (m, 2H), 7.49 (d, 1H), 8.28 (s, 1H).

### Beispiel 43

### Ethyl-1-(3-methyl-2-oxo-2,3-dihydro-1,3-benzoxazol-6-yl)-2,4-dioxo-3-[(1S)-4-(trifluormethyl)-2,3-dihydro-1H-inden-1-yl]-1,2,3,4-tetrahydropyrimidin-5-carboxylat (S-Enantiomer)

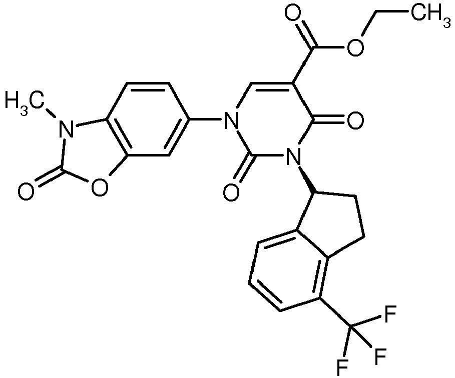

1.00 g (3.02 mmol) der Verbindung aus Beispiel 28A, 732 mg (3.62 mmol) der Verbindung aus Beispiel 15A und 1.35 g (5.13 mmol) Triphenylphosphin wurden in 9 ml THF und 18 ml DMF vorgelegt und bei RT tropfenweise mit 951 µl (4.83 mmol) Diisopropylazodicarboxylat versetzt. Die Reaktionsmischung wurde 1 h bei RT gerührt. Zur Aufarbeitung wurde dem Reaktionsgemisch unter Eiskühlung 5 ml IN Salzsäure hinzugefügt und 10 min gerührt. Das Gemisch wurde dann mit Essigsäureethylester extrahiert. Die vereinigten organischen Phasen wurde zweimal mit IN Salzsäure, zweimal mit einer IM Natriumcarbonatlösung, einmal mit einer gesättigter Natriumchloridlösung gewaschen, über Magnesiumsulfat getrocknet und am Rotationsverdampfer eingeengt. Der Rückstand wurde per präparativer HPLC (Methode 15) gereinigt. Man erhielt 590 mg (38 % d. Th.) der Titelverbindung.
LC-MS (Methode 1): Rₜ = 1.08 min; m/z = 516 (M+H)⁺.
¹H-NMR (400MHz, CD₂Cl₂): δ [ppm] = 1.31 (t, 3H), 2.33 - 2.50 (m, 1H), 2.51 - 2.67 (m, 1H), 3.14 (dt, 1H), 3.39 - 3.52 (m, 1H), 3.40 (s, 3H), 4.29 (q, 2H), 6.55 - 6.68 (m, 1H), 7.06 (d, 1H), 7.18 (d, 1H), 7.22 (s, 1H), 7.26 - 7.35 (m, 2H), 7.49 (d, 1H), 8.28 (s, 1H).
Chirale analytische HPLC (Methode 27): Rₜ = 9.94 min; ca. 93 % ee

### Beispiel 44

### Ethyl-1-(3-ethyl-2-oxo-2,3-dihydro-1,3-benzoxazol-6-yl)-3-[2-methyl-3-(trifluormethyl)benzyl]-2,4-dioxo-1,2,3,4-tetrahydropyrimidin-5-carboxylat

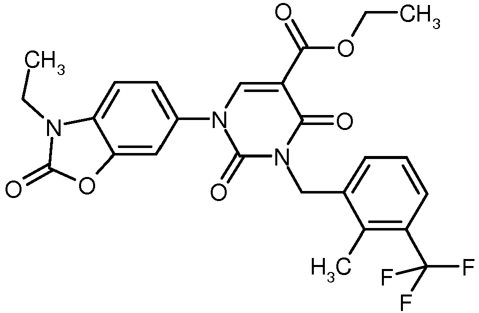

Darstellung und Reinigung der Titelverbindung erfolgten analog zu Beispiel 1. Die Reaktionszeit betrug 5h. Ausgehend von 200 mg (0.58 mmol) Ethyl-1-(3-ethyl-2-oxo-2,3-dihydro-1,3-benzoxazol-6-yl)-2,4-dioxo-1,2,3,4-tetrahydropyrimidin-5-carboxylat aus Beispiel 30A und 161 mg (0.64 mmol) 2-Methyl-3-(trifluormethyl)benzylbromid erhielt man 192 mg (64 % d. Th.) der Titelverbindung.
LC-MS (Methode 1): Rₜ = 1.11 min; m/z = 518 (M+H)⁺.
¹H-NMR (400MHz, DMSO-d₆): δ [ppm] = 1.23 (t, 3H), 1.28 (t, 3H), 2.46 (s, 3H), 3.90 (q, 2H), 4.20 (q, 2H), 5.08 (s, 2H), 7.31 - 7.42 (m, 2H), 7.43 - 7.50 (m, 2H), 7.58 - 7.62 (m, 1H), 7.64 - 7.67 (m, 1H), 8.45 (s, 1H).

### Beispiel 45

### Ethyl-3-(2,3-dichlorbenzyl)-1-(3-ethyl-2-oxo-2,3-dihydro-1,3-benzoxazol-6-yl)-2,4-dioxo-1,2,3,4-tetrahydropyrimidin-5-carboxylat

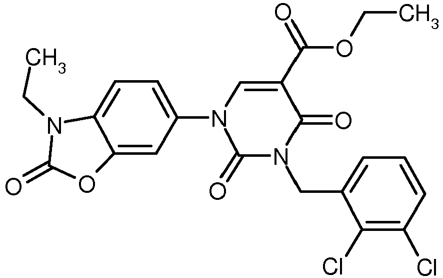

Darstellung und Reinigung der Titelverbindung erfolgten analog zu Beispiel 1. Die Reaktionszeit betrug 5h. Ausgehend von 200 mg (0.58 mmol) Ethyl-1-(3-ethyl-2-oxo-2,3-dihydro-1,3-benzoxazol-6-yl)-2,4-dioxo-1,2,3,4-tetrahydropyrimidin-5-carboxylat aus Beispiel 30A und 124 mg (0.64 mmol) 1,2-Dichlor-3-(chlormethyl)benzol erhielt man 220 mg (75 % d. Th.) der Titelverbindung.
LC-MS (Methode 1): Rₜ = 1.09 min; m/z = 504 (M+H)⁺.
¹H-NMR (400MHz, DMSO-d₆): δ [ppm] = 1.23 (t, 3H), 1.28 (t, 3H), 3.90 (q, 2H), 4.20 (q, 2H), 5.10 (s, 2H), 7.21 - 7.25 (m, 1H), 7.30 - 7.36 (m, 1H), 7.41 - 7.50 (m, 2H), 7.56 - 7.60 (m, 1H), 7.63 - 7.66 (m, 1H), 8.47 (s, 1H).

### Beispiel 46

### Ethyl-3-[2-chlor-3-(trifluormethyl)benzyl]-1-(3-ethyl-2-oxo-2,3-dihydro-1,3-benzoxazol-6-yl)-2,4-dioxo-1,2,3,4-tetrahydropyrimidin-5-carboxylat

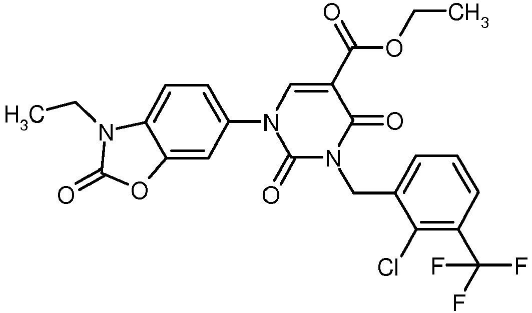

Darstellung und Reinigung der Titelverbindung erfolgten analog zu Beispiel 37. Ausgehend von 200 mg (0.58 mmol) Ethyl-1-(3-ethyl-2-oxo-2,3-dihydro-1,3-benzoxazol-6-yl)-2,4-dioxo-1,2,3,4-tetrahydropyrimidin-5-carboxylat aus Beispiel 30A und 174 mg (0.63 mmol) 1-(Brommethyl)-2-chlor-3-(trifluormethyl)benzol erhielt man 209 mg (67 % d. Th.) der Titelverbindung.
LC-MS (Methode 1): Rₜ = 1.11 min; m/z = 538 (M+H)⁺.
¹H-NMR (400MHz, DMSO-d₆): δ [ppm] = 1.23 (t, 3H), 1.28 (t, 3H), 3.90 (q, 2H), 4.20 (q, 2H), 5.14 (s, 2H), 7.42 - 7.56 (m, 3H), 7.59 (d, 1H), 7.65 (d, 1H), 7.81 (d, 1H), 8.48 (s, 1H).

### Beispiel 47

### Ethyl-1-(3-ethyl-2-oxo-2,3-dihydro-1,3-benzoxazol-6-yl)-3-[3-fluor-2-(trifluormethyl)benzyl]-2,4-dioxo-1,2,3,4-tetrahydropyrimidin-5-carboxylat

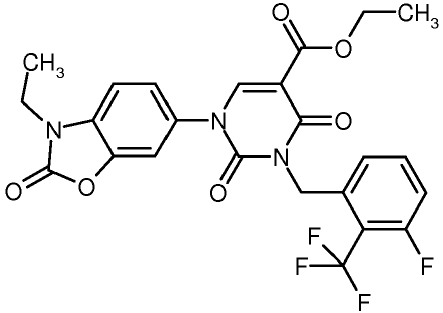

Darstellung und Reinigung der Titelverbindung erfolgten analog zu Beispiel 37. Ausgehend von 200 mg (0.58 mmol) Ethyl-1-(3-ethyl-2-oxo-2,3-dihydro-1,3-benzoxazol-6-yl)-2,4-dioxo-1,2,3,4-tetrahydropyrimidin-5-carboxylat aus Beispiel 30A und 163 mg (0.63 mmol) 1-(Brommethyl)-3-fluor-2-(trifluormethyl)benzol erhielt man 159 mg (52 % d. Th.) der Titelverbindung.
LC-MS (Methode 1): Rₜ = 1.06 min; m/z = 522 (M+H)⁺.
¹H-NMR (400MHz, DMSO-d₆): δ [ppm] = 1.23 (t, 3H), 1.28 (t, 3H), 3.90 (q, 2H), 4.20 (q, 2H), 5.19 (s, 2H), 7.18 - 7.23 (m, 1H), 7.37 - 7.51 (m, 3H), 7.62 - 7.70 (m, 2H), 8.49 (s, 1H).

### Beispiel 48

### Ethyl-1-(3-methyl-2-oxo-2,3-dihydro-1,3-benzothiazol-6-yl)-3-[2-methyl-3-(trifluormethyl)-benzyl]-2,4-dioxo-1,2,3,4-tetrahydropyrimidin-5-carboxylat

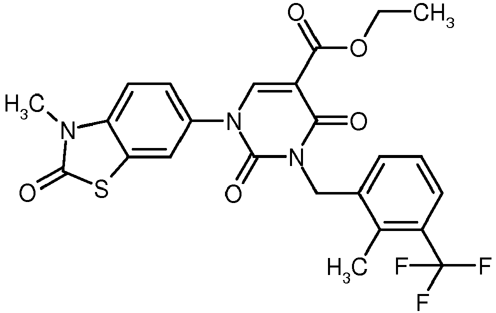

Darstellung und Reinigung der Titelverbindung erfolgten analog zu Beispiel 37. Ausgehend von 500 mg (1.44 mmol) Ethyl-1-(3-methyl-2-oxo-2,3-dihydro-1,3-benzothiazol-6-yl)-2,4-dioxo-1,2,3,4-tetrahydropyrimidin-5-carboxylat aus Beispiel 31A und 400 mg (1.58 mmol) 1-(Brommethyl)-2-methyl-3-(trifluormethyl)benzol erhielt man 392 mg (50 % d. Th.) der Titelverbindung.
LC-MS (Methode 3): Rₜ = 1.11 min; m/z = 520 (M+H)⁺.
¹H-NMR (400MHz, DMSO-d₆): δ [ppm] = 1.23 (t, 3H), 2.46 (s, teilweise durch DMSO-Signal verdeckt), 3.45 (s, 3H), 4.20 (q, 2H), 5.08 (s, 2H), 7.31 - 7.42 (m, 2H), 7.46 (d, 1H), 7.56 - 7.63 (m, 2H), 7.88 - 7.91 (m, 1H), 8.48 (s, 1H).

### Beispiel 49

### Ethyl-3-[2-chlor-3-(trifluormethyl)benzyl]-1-(3-methyl-2-oxo-2,3-dihydro-1,3-benzothiazol-6-yl)-2,4-dioxo-1,2,3,4-tetrahydropyrimidin-5-carboxylat

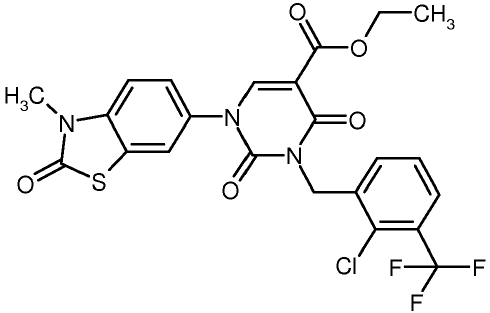

Darstellung und Reinigung der Titelverbindung erfolgten analog zu Beispiel 37. Ausgehend von 184 mg (0.53 mmol) Ethyl-1-(3-methyl-2-oxo-2,3-dihydro-1,3-benzothiazol-6-yl)-2,4-dioxo-1,2,3,4-tetrahydropyrimidin-5-carboxylat aus Beispiel 31A und 159 mg (0.58 mmol) 1-(Brommethyl)-2-chlor-3-(trifluormethyl)benzol erhielt man 216 mg (75 % d. Th.) der Titelverbindung.
LC-MS (Methode 1): Rₜ = 1.11 min; m/z = 540 (M+H)⁺.
¹H-NMR (400MHz, DMSO-d₆): δ [ppm] = 1.24 (t, 3H), 3.45 (s, 3H), 4.20 (q, 2H), 5.15 (s, 2H), 7.46 (d, 1H), 7.49 - 7.55 (m, 1H), 7.56 - 7.61 (m, 2H), 7.78 - 7.82 (m, 1H), 7.88 - 7.90 (m, 1H), 8.51 (s, 1H).

### Beispiel 50

### Ethyl-3-[3-fluor-2-(trifluormethyl)benzyl]-1-(3-methyl-2-oxo-2,3-dihydro-1,3-benzothiazol-6-yl)-2,4-dioxo-1,2,3,4-tetrahydropyrimidin-5-carboxylat

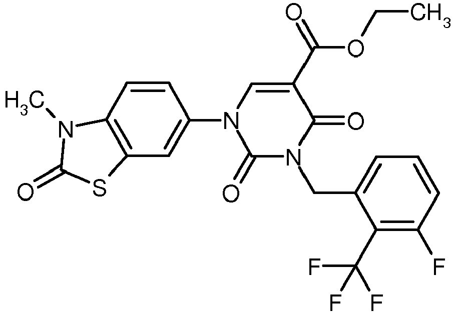

Darstellung und Reinigung der Titelverbindung erfolgten analog zu Beispiel 37. Ausgehend von 200 mg (0.53 mmol) Ethyl-1-(3-methyl-2-oxo-2,3-dihydro-1,3-benzothiazol-6-yl)-2,4-dioxo-1,2,3,4-tetrahydropyrimidin-5-carboxylat aus Beispiel 31A 149 mg (0.58 mmol) 1-(Brommethyl)-3-fluor-2-(trifluormethyl)benzol erhielt man 241 mg (87 % d. Th.) der Titelverbindung.
LC-MS (Methode 1): Rₜ = 1.06 min; m/z = 524 (M+H)⁺.
¹H-NMR (400MHz, DMSO-d₆): δ [ppm] = 1.23 (t, 3H), 3.45 (s, 3H), 4.20 (q, 2H), 5.19 (s, 2H), 7.21 (d, 1H), 7.41 (t, 1H), 7.47 (d, 1H), 7.57 (dd, 1H), 7.66 (q, 1H), 7.88 (d, 1H), 8.52 (s, 1H).

### Beispiel 51

### Ethyl-1-(3-methyl-2-oxo-2,3-dihydro-1,3-benzothiazol-6-yl)-2,4-dioxo-3-[(1R)-4-(trifluormethyl)-2,3-dihydro-1H-inden-1-yl]-1,2,3,4-tetrahydropyrimidin-5-carboxylat (R-Enantiomer)

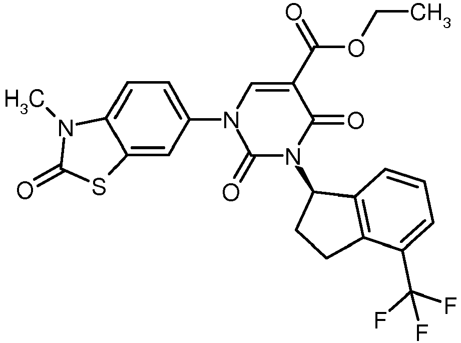

8.00 g (23.03 mmol) Ethyl-1-(3-methyl-2-oxo-2,3-dihydro-1,3-benzothiazol-6-yl)-2,4-dioxo-1,2,3,4-tetrahydro-pyrimidin-5-carboxylat aus Beispiel 31A, 5.12 g (25.33 mmol) (1S)-4-(Trifluormethyl)indan-1-ol aus Beispiel 14A und 10.27 g (39.15 mmol) Triphenylphosphin wurden in 317 ml THF und 317 ml DMF vorgelegt und auf 5°C gekühlt. 7.25 ml (36.85 mmol) Diisopropylazodicarboxylat wurden portionsweise zugegeben. Das Kühlbad wurde entfernt und die Mischung 1 h bei RT gerührt. Zur Aufarbeitung wurden 200 ml IN Salzsäure zugegeben und die Mischung 5 min kräftig gerührt. 400 ml Essigsäureethylester wurden hinzugefügt. Nach 10-minutigem kräftigem Rühren wurde die organische Phase abgetrennt. Die wässrige Phase wurde noch einmal mit 400 ml Essigsäureethylester extrahiert. Die vereinigten organischen Phasen wurden zweimal mit je 100 ml einer gesättigten Natriumcarbonatlösung, dann mit 100 ml einer gesättigten Natriumchloridlösung gewaschen, dann über Natriumsulfat getrocknet und am Rotationsverdampfer eingeengt. Der Rückstand wurde mit 400 ml MTBE versetzt und 30 min unter Eisbadkühlung verrührt. Der ausgefallene Feststoff wurde abgesaugt und zweimal mit kaltem MTBE gewaschen. Die vereinigten Filtrate wurde eingeengt und der Rückstand mittels Flashchromatographie (Cyclohexan/Essigsäureethylester 1:2 → 1:4) gereinigt. Das so erhaltene Produkt wurde aus Acetonitril umkristallisiert und am Hochvakuum getrocknet. Man erhielt 6.3 g (50 % d. Th.) der Titelverbindung.
LC-MS (Methode 1): Rₜ = 1.18 min; m/z = 532 (M+H)⁺.
¹H-NMR (400MHz, CD₂Cl₂): δ [ppm] = 1.31 (t, 3H), 2.37 - 2.49 (m, 1H), 2.53 - 2.65 (m, 1H), 3.08 - 3.20 (m, 1H), 3.40 - 3.52 (m, 1H), 3.45 (s, 3H), 4.29 (q, 2H), 6.56 - 6.68 (m, 1H), 7.09 - 7.18 (m, 1H), 7.25 - 7.36 (m, 3H), 7.44 (s, 1H), 7.47 - 7.54 (m, 1H), 8.29 (s, 1H).

### Beispiel 52

### Ethyl-3-[2-chlor-3-(trifluormethyl)benzyl]-2,4-dioxo-1-(1,3,3-trimethyl-2-oxo-2,3-dihydro-1H-indol-5-yl)-1,2,3,4-tetrahydropyrimidin-5-carboxylat

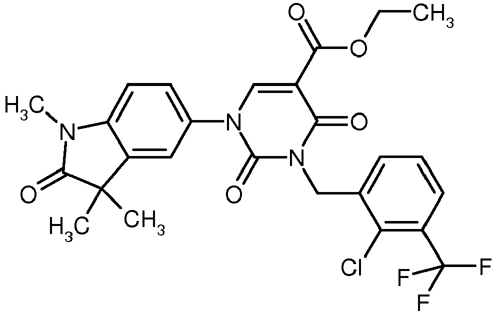

Darstellung und Reinigung der Titelverbindung erfolgten analog zu Beispiel 37. Ausgehend von 200 mg (0.56 mmol) Ethyl-2,4-dioxo-1-(1,3,3-trimethyl-2-oxo-2,3-dihydro-1H-indol-5-yl)-1,2,3,4-tetrahydropyrimidin-5-carboxylat aus Beispiel 59A und 168 mg (0.61 mmol) 1-(Brommethyl)-2-chlor-3-(trifluormethyl)benzol erhielt man 241 mg (77 % d. Th.) der Titelverbindung.
LC-MS (Methode 1): Rₜ = 1.11 min; m/z = 550 (M+H)⁺.
¹H-NMR (400MHz, DMSO-d₆): δ [ppm] = 1.24 (t, 3H), 1.30 (s, 6H), 3.18 (s, 3H), 4.21 (q, 2H), 5.15 (s, 2H), 7.16 (d, 1H), 7.44 - 7.49 (m, 1H), 7.50 - 7.60 (m, 3H), 7.78 - 7.83 (m, 1H), 8.44 (s, 1H).

### Beispiel 53

### Ethyl-3-[2-methyl-3-(trifluormethyl)benzyl]-2,4-dioxo-1-(1,3,3-trimethyl-2-oxo-2,3-dihydro-1H-indol-5-yl)-1,2,3,4-tetrahydropyrimidin-5-carboxylat

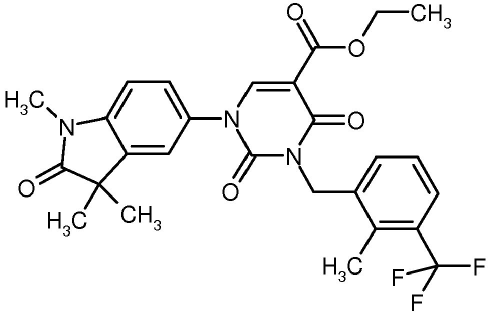

Darstellung und Reinigung der Titelverbindung erfolgten analog zu Beispiel 37. Ausgehend von 500 mg (1.39 mmol) Ethyl-2,4-dioxo-1-(1,3,3-trimethyl-2-oxo-2,3-dihydro-1H-indol-5-yl)-1,2,3,4-tetrahydropyrimidin-5-carboxylat aus Beispiel 59A und 389 mg (1.53 mmol) 1-(Brommethyl)-2-methyl-3-(trifluormethyl)benzol erhielt man 571 mg (77 % d. Th.) der Titelverbindung.
LC-MS (Methode 1): Rₜ = 1.11 min; m/z = 530 (M+H)⁺.
¹H-NMR (400MHz, DMSO-d₆): δ [ppm] = 1.29 (s, 6H), 2.46 (s, 3H), 3.18 (s, 3H), 3.30 (s, 3H), 4.20 (q, 2H), 5.08 (s, 2H), 7.15 (d, 1H), 7.34 - 7.39 (m, 2H), 7.44 - 7.49 (m, 1H), 7.53 - 7.56 (m, 1H), 7.58 - 7.63 (m, 1H), 8.42 (s, 1H).

### Beispiel 54

### Ethyl-3-[3-chlor-2-(trifluormethyl)benzyl]-2,4-dioxo-1-(1,3,3-trimethyl-2-oxo-2,3-dihydro-1H-indol-5-yl)-1,2,3,4-tetrahydropyrimidin-5-carboxylat

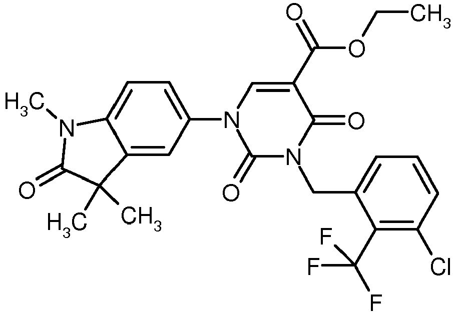

153 mg (0.42 mmol) Ethyl-2,4-dioxo-1-(1,3,3-trimethyl-2-oxo-2,3-dihydro-1H-indol-5-yl)-1,2,3,4-tetrahydro-pyrimidin-5-carboxylat aus Beispiel 59A wurden analog zu Beispiel 37 mit 198 mg (65 %-ige Reinheit 0.47 mmol) 1-(Brommethyl)-3-chlor-2-(trifluormethyl)benzol (Herstellung: siehe WO 2004/52858, Seite 149, Beispiel 176) umgesetzt. Zur Aufarbeitung wurde die auf RT abgekühlte Reaktionsmischung mit Wasser versetzt und zweimal mit Essigsäureethylester extrahiert. Die vereinigten organischen Phasen wurden mit gesättigter Natriumchlorid-Lösung gewaschen, über Magnesiumsulfat getrocknet und eingeengt. Der Rückstand wurde mit MTBE verrührt, der ausgefallene Feststoff abgesaugt, mit MTBE nachgewaschen und an der Hochvakuumpumpe getrocknet. Man erhielt 109 mg (46 % d. Th.) der Titelverbindung.
LC-MS (Methode 1): Rₜ = 1.14 min; m/z = 550 (M+H)⁺.
¹H-NMR (400MHz, DMSO-d₆): δ [ppm] = 1.23 (t, 3H), 1.29 (s, 6H), 3.18 (s, 3H), 4.20 (q, 2H), 5.21 (br.s, 2H), 7.16 (d, 1H), 7.30 - 7.35 (m, 1H), 7.45 (dd, 1H), 7.53 (d, 1H), 7.57 - 7.66 (m, 2H), 8.45 (s, 1H).

### Beispiel 55

### Ethyl-3-[3-fluor-2-(trifluormethyl)benzyl]-2,4-dioxo-1-(1,3,3-trimethyl-2-oxo-2,3-dihydro-1H-indol-5-yl)-1,2,3,4-tetrahydropyrimidin-5-carboxylat

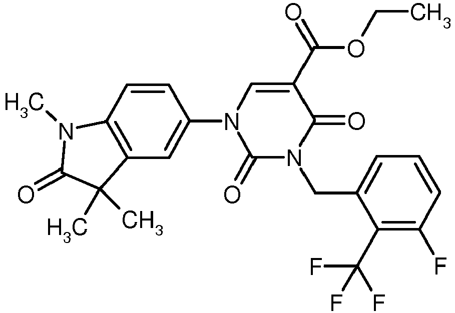

Darstellung und Reinigung der Titelverbindung erfolgten analog zu Beispiel 37. Ausgehend von 200 mg (0.56 mmol) Ethyl-2,4-dioxo-1-(1,3,3-trimethyl-2-oxo-2,3-dihydro-1H-indol-5-yl)-1,2,3,4-tetrahydropyrimidin-5-carboxylat aus Beispiel 59A und 158 mg (0.61 mmol) 1-(Brommethyl)-3-fluor-2-(trifluormethyl)benzol erhielt man 247 mg (80 % d. Th.) der Titelverbindung.
LC-MS (Methode 1): Rₜ = 1.06 min; m/z = 534 (M+H)⁺.
¹H-NMR (400MHz, DMSO-d₆): δ [ppm] = 1.23 (t, 3H), 1.29 (s, 6H), 3.18 (s, 3H), 4.20 (q, 2H), 5.20 (s, 2H), 7.13 - 7.22 (m, 2H), 7.37 - 7.48 (m, 2H), 7.53 (d, 1H), 7.63 - 7.70 (m, 1H), 8.45 (s, 1H).

### Beispiel 56

### Ethyl-3-(2,3-dichlorbenzyl)-2,4-dioxo-1-(1,3,3-trimethyl-2-oxo-2,3-dihydro-1H-indol-5-yl)-1,2,3,4-tetrahydro-pyrimidin-5-carboxylat

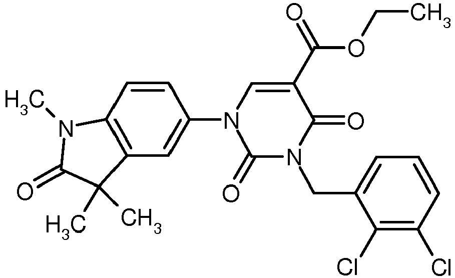

Darstellung und Reinigung der Titelverbindung erfolgten analog zu Beispiel 37. Ausgehend von 200 mg (0.56 mmol) Ethyl-2,4-dioxo-1-(1,3,3-trimethyl-2-oxo-2,3-dihydro-1H-indol-5-yl)-1,2,3,4-tetrahydropyrimidin-5-carboxylat aus Beispiel 59A und 120 mg (0.61 mmol) 1,2-Dichlor-3-(chlormethyl)benzol erhielt man 230 mg (78 % d. Th.) der Titelverbindung.
LC-MS (Methode 1): Rₜ = 1.09 min; m/z = 520 (M+H)⁺.
¹H-NMR (400MHz, DMSO-d₆): δ [ppm] = 1.23 (t, 3H), 1.29 (s, 6H), 3.18 (s, 3H), 4.20 (q, 2H), 7.15 (d, 1H), 7.22 (d, 1H), 7.33 (t, 1H), 7.46 (dd, 1H), 7.54 (d, 1H), 7.58 (d, 1H), 8.43 (s, 1H).

### Beispiel 57

### Ethyl-2,4-dioxo-3-[4-(trifluormethyl)-2,3-dihydro-1H-inden-1-yl]-1-(1,3,3-trimethyl-2-oxo-2,3-dihydro-1H-indol-5-yl)-1,2,3,4-tetrahydropyrimidin-5-carboxylat (Racemat)

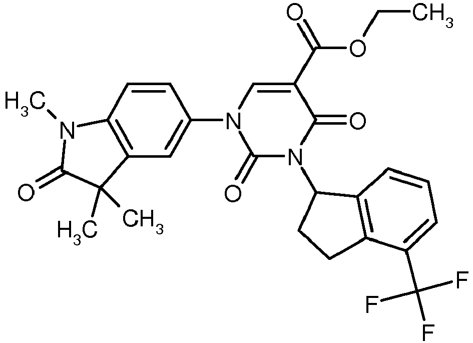

Darstellung und Reinigung der Titelverbindung erfolgten analog zu Beispiel 8 mit einer Reaktionsdauer von 2 Tagen. Ausgehend von 190 mg (0.51 mmol) Ethyl-2,4-dioxo-3-[4-(trifluormethyl)-2,3-dihydro-1H-inden-1-yl]-1,2,3,4-tetrahydropyrimidin-5-carboxylat aus Beispiel 21A und 310 mg (1.03 mmol) 1,3,3-Trimethyl-5-(4,4,5,5-tetramethyl-1,3,2-dioxaborolan-2-yl)-1,3-dihydro-2H-indol-2-on aus Beispiel 68A erhielt man nach zusätzlicher Reinigung mittels Flashchromatographie (Dichlormethan/Methanol 98:2) zusammen 169 mg (60 % d. Th.) der Titelverbindung.
LC-MS (Methode 1): Rₜ = 1.14 min; m/z = 542 (M+H)⁺.
¹H-NMR (400MHz, DMSO-d₆): δ [ppm] = 1.20 - 1.25 (m, 3H), 1.29 (s, 6H), 2.38 - 2.43 (m, 1H), 2.44 - 2.48 (m, 1H, teilweise durch DMSO-Signal verdeckt), 3.03 - 3.13 (m, 1H), 3.17 (s, 3H), 3.23 - 3.29 (m, 1H, teilweise durch Wasser-Signal verdeckt), 4.18 (q, 2H), 6.33 - 6.56 (m, 1H), 7.13 (d, 1H), 7.32 - 7.45 (m, 2H), 7.45 - 7.57 (m, 3H), 8.33 (s, 1H).

### Beispiel 58

### Ethyl-2,4-Dioxo-3-[4-(trifluormethyl)-2,3-dihydro-1H-inden-1-yl]-1-(1,3,3-trimethyl-2-oxo-2,3-dihydro-1H-indol-5-yl)-1,2,3,4-tetrahydropyrimidin-5-carboxylat (R-Enantiomer)

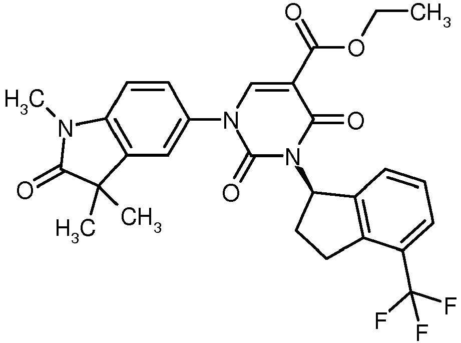

700 mg (1.96 mmol) der Verbindung aus Beispiel 59A, 515 mg (2.55 mmol) (S)-4-Trifluormethyl-indan-1-ol aus Beispiel 14A und 1.54 g (5.88 mmol) Triphenylphosphin wurden in 20 ml THF und 20 ml DMF bei -15°C vorgelegt und tropfenweise mit 1.12 ml (5.68 mmol) Diisopropylazodicarboxylat versetzt. Die Reaktionsmischung wurde 2 h bei RT gerührt. Zur Aufarbeitung wurde das Gemisch wieder auf -15°C gekühlt, mit 30 ml IN Salzsäure versetzt, 10 min bei RT gerührt und dann mit Essigsäureethylester extrahiert. Die organische Phase wurde zweimal mit IN Salzsäure, einmal mit einer IM Natriumcarbonatlösung, einmal mit einer gesättigten Natriumchloridlösung gewaschen, dann über Natriumsulfat getrocknet und am Rotationsverdampfer eingeengt. Der Rückstand wurde per präparativer HPLC (Methode 7) gereinigt. Man erhielt 725 mg (68 % d. Th.) der Titelverbindung.
LC-MS (Methode 5): Rₜ = 1.18 min; m/z = 542 (M+H)⁺.
¹H-NMR (400MHz, CD₂Cl₂): δ [ppm] = 1.31 (t, 3H), 1.35 (s., 3H), 1.36 (s., 3H), 2.37 - 2.50 (m, 1H), 2.58 (dtd, 1H), 3.08 - 3.18 (m, 1H), 3.20 (s, 3H), 3.47 (br.s, 1H), 4.29 (q, 2H), 6.54 - 6.68 (m, 1H), 6.92 (d, 1H), 7.16 (br.s, 1H), 7.21 (d, 1H), 7.26 - 7.36 (m, 2H), 7.49 (d, 1H), 8.29 (s, 1H).

### Beispiel 59

### Ethyl-3-[2-methyl-3-(trifluormethyl)benzyl]-2,4-dioxo-1-(1,3,3-trimethyl-2-oxo-2,3-dihydro-1H-indol-6-yl)-1,2,3,4-tetrahydropyrimidin-5-carboxylat

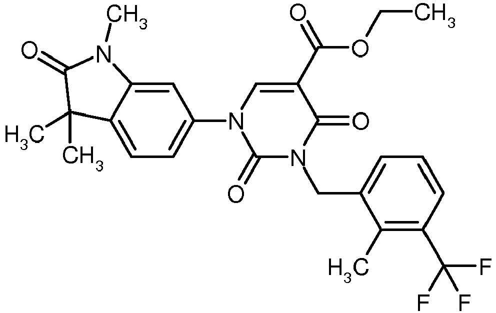

125 mg (0.35 mmol) Ethyl-2,4-dioxo-1-(1,3,3-trimethyl-2-oxo-2,3-dihydro-1H-indol-6-yl)-1,2,3,4-tetrahydro-pyrimidin-5-carboxylat aus Beispiel 64A wurden in 3 ml DMF vorgelegt. 97 mg (0.38 mmol) 1-(Brommethyl)-2-methyl-3-(trifluormethyl)benzol, 97 mg (0.70 mmol) Kaliumcarbonat und 6 mg (0.04 mmol) Kaliumiodid wurden zugegeben und die Reaktionsmischung 2 h bei 60°C gerührt. Nach Abkühlung auf RT wurde das Gemisch mit Wasser versetzt. Der Niederschlag wurde abfiltriert, mit wenig Wasser und Cyclohexan gewaschen und im Trockenschrank bei 50°C getrocknet. Man erhielt 134 mg (90 %-ige Reinheit, 65 % d. Th.) der Titelverbindung.
LC-MS (Methode 1): Rₜ = 1.16 min; m/z = 530 (M+H)⁺.
¹H-NMR (400MHz, DMSO-d₆): δ [ppm] = 1.24 (t, 3H), 1.30 (s, 6H), 2.47 (s, 3H), 3.14 (s, 3H), 4.20 (q, 2H), 5.09 (s, 2H), 7.22 (dd, 1H), 7.27 (d, 1H), 7.31 - 7.41 (m, 2H), 7.51 (d, 1H), 7.60 (d, 1H), 8.47 (s, 1H).

### Beispiel 60

### Ethyl-3-[2-chlor-3-(trifluormethyl)benzyl]-2,4-dioxo-1-(1,3,3-trimethyl-2-oxo-2,3-dihydro-1H-indol-6-yl)-1,2,3,4-tetrahydropyrimidin-5-carboxylat

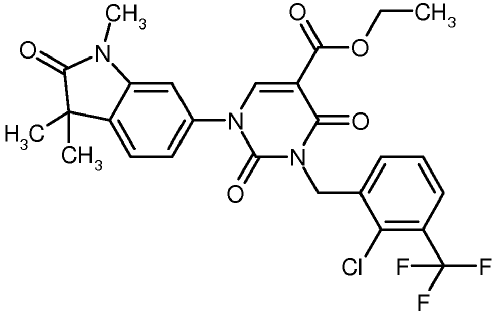

Darstellung und Reinigung der Titelverbindung erfolgten analog zu Beispiel 59. Ausgehend von 125 mg (0.35 mmol) Ethyl-2,4-dioxo-1-(1,3,3-trimethyl-2-oxo-2,3-dihydro-1H-indol-6-yl)-1,2,3,4-tetrahydropyrimidin-5-carboxylat aus Beispiel 64A und 105 mg (0.38 mmol) 1-(Brommethyl)-2-chlor-3-(trifluormethyl)benzol erhielt man 182 mg (85 % d. Th.) der Titelverbindung.
LC-MS (Methode 1): Rₜ = 1.16 min; m/z = 550 (M+H)⁺.
¹H-NMR (400MHz, DMSO-d₆): δ [ppm] = 1.24 (t, 3H), 1.31 (s, 6H), 3.15 (s, 3H), 4.20 (q, 2H), 5.16 (s, 2H), 7.22 (dd, 1H), 7.26 (d, 1H), 7.50 - 7.55 (m, 2H), 7.58 (d, 1H), 7.80 (d, 1H), 8.49 (s, 1H).

### Beispiel 61

### Ethyl-3-[3-chlor-2-(trifluormethyl)benzyl]-2,4-dioxo-1-(1,3,3-trimethyl-2-oxo-2,3-dihydro-1H-indol-6-yl)-1,2,3,4-tetrahydropyrimidin-5-carboxylat

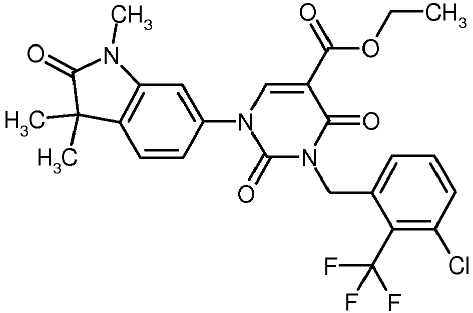

125 mg (0.35 mmol) Ethyl-2,4-dioxo-1-(1,3,3-trimethyl-2-oxo-2,3-dihydro-1H-indol-6-yl)-1,2,3,4-tetrahydro-pyrimidin-5-carboxylat aus Beispiel 64A wurden in DMF (3 ml) vorgelegt. 161 mg (65 %-ige Reinheit, 0.38 mmol) 1-(Brommethyl)-3-chlor-2-(trifluormethyl)benzol, 96 mg (0.70 mmol) Kaliumcarbonat und 6 mg (0.03 mmol) Kaliumiodid wurden zugegeben. Anschließend ließ man die Reaktionsmischung 2h bei 60°C rühren. Das auf RT abgekühlte Gemisch wurde mit Wasser versetzt und zweimal mit Essigsäureethylester extrahiert. Die vereinigten organischen Phasen wurden mit einer gesättigten Natriumchlorid-Lösung gewaschen, über Magnesiumsulfat getrocknet und eingeengt. Der Rückstand wurde mit Cyclohexan/Essigsäureethylester verrührt, der ausgefallene Feststoff abgesaugt und im Vakuum getrocknet. Man erhielt 133 mg (62 % d. Th.) der Titelverbindung.
LC-MS (Methode 1): Rₜ = 1.16 min; m/z = 550 (M+H)⁺.
¹H-NMR (400MHz, DMSO-d₆): δ [ppm] = 1.24 (t, 3H), 1.30 (s, 6H), 3.15 (s, 3H), 4.20 (q, 2H), 5.22 (br.s, 2H), 7.21 (d, 1H), 7.25 (s, 1H), 7.33 (d, 1H), 7.51 (d, 1H), 7.56 - 7.68 (m, 2H), 8.50 (s, 1H).

### Beispiel 62

### Ethyl-3-[2-chlor-3-(trifluormethyl)benzyl]-1-(3-hydroxy-1,3-dimethyl-2-oxo-2,3-dihydro-1H-indol-5-yl)-2,4-dioxo-1,2,3,4-tetrahydropyrimidin-5-carboxylat (Racemat)

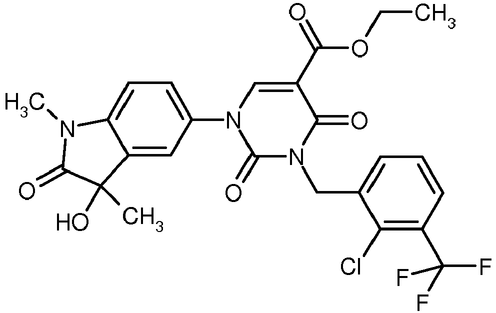

Darstellung und Reinigung der Titelverbindung erfolgten analog zu Beispiel 1. Die Reaktionsdauer betrug 1h. Ausgehend von 105 mg (0.29 mmol) Ethyl-1-(3-hydroxy-1,3-dimethyl-2-oxo-2,3-dihydro-1H-indol-5-yl)-2,4-dioxo-1,2,3,4-tetrahydropyrimidin-5-carboxylat aus Beispiel 72A und 88 mg (0.32 mmol) 1-(Brommethyl)-2-chlor-3-(trifluormethyl)benzol erhielt man 133 mg (74 % d. Th.) der Titelverbindung.
LC-MS (Methode 1): Rₜ = 0.98 min; m/z = 552 (M+H)⁺.
¹H-NMR (400MHz, DMSO-d₆): δ [ppm] = 1.23 (t, 3H), 1.41 (s, 3H), 3.14 (s, 3H), 4.21 (q, 2H), 5.14 (s, 2H), 6.13 (s, 1H), 7.14 (d, 1H), 7.47 - 7.57 (m, 3H), 7.58 - 7.62 (m, 1H), 7.78 - 7.82 (m, 1H), 8.40 (s, 1H).

### Beispiel 63

### Ethyl-1-[3-hydroxy-1-methyl-2-oxo-3-(trifluormethyl)-2,3-dihydro-1H-indol-5-yl]-3-[2-methyl-3-(trifluormethyl)benzyl]-2,4-dioxo-1,2,3,4-tetrahydropyrimidin-5-carboxylat (Racemat)

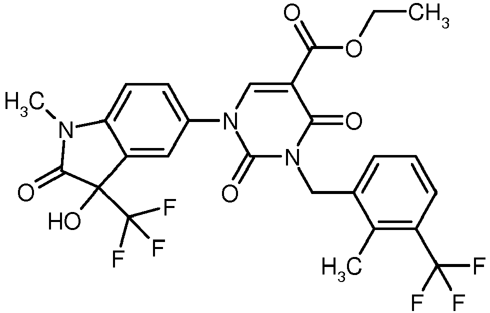

Darstellung und Reinigung der Titelverbindung erfolgten analog zu Beispiel 1. Die Reaktionszeit betrug 45 min. Ausgehend von 200 mg (0.48 mmol) Ethyl-1-[3-hydroxy-1-methyl-2-oxo-3-(trifluormethyl)-2,3-dihydro-1H-indol-5-yl]-2,4-dioxo-1,2,3,4-tetrahydropyrimidin-5-carboxylat aus Beispiel 69A und 134 mg (0.53 mmol) 1-(Brommethyl)-2-methyl-3-(trifluormethyl)benzol erhielt man nach zusätzlicher Reinigung mittels HPLC (Methode 8) 76 mg (26 % d. Th.) der Titelverbindung.
LC-MS (Methode 5): Rₜ = 1.11 min; m/z = 585 (M+H)⁺.
¹H-NMR (400MHz, DMSO-d₆): δ [ppm] = 1.24 (t, 3H), 2.46 (s, 3H), 3.22 (s, 3H), 4.21 (q, 2H), 5.07 (s, 2H), 7.30 (d, 1H), 7.35 (t, 1H), 7.41 (d, 1H), 7.60 (d, 1H), 7.67 - 7.71 (m, 1H), 7.73 (s, 1H), 7.92 (s, 1H), 8.40 (s, 1H).

### Beispiel 64

### Ethyl-1-[3-fluor-1-methyl-2-oxo-3-(trifluormethyl)-2,3-dihydro-1H-indol-5-yl]-3-[2-methyl-3-(trifluormethyl)benzyl]-2,4-dioxo-1,2,3,4-tetrahydropyrimidin-5-carboxylat (Racemat)

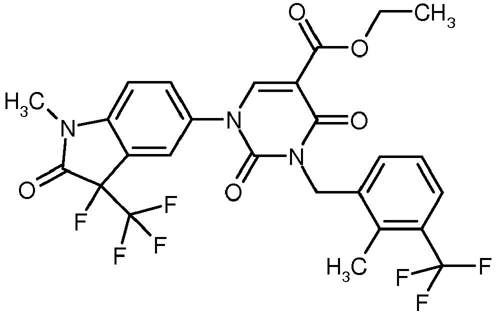

Darstellung und Reinigung der Titelverbindung erfolgten analog zu Beispiel 1. Die Reaktionszeit betrug 45 min. Ausgehend von 90 mg (0.21 mmol) Ethyl-1-[3-fluor-1-methyl-2-oxo-3-(trifluormethyl)-2,3-dihydro-1H-indol-5-yl]-2,4-dioxo-1,2,3,4-tetrahydropyrimidin-5-carboxylat aus Beispiel 73A und 60 mg (0.23 mmol) 1-(Brommethyl)-2-methyl-3-(trifluormethyl)benzol erhielt man nach zusätzlicher Reinigung mittels HPLC (Methode 8) 97 mg (72 % d. Th.) der Titelverbindung.
LC-MS (Methode 5): Rₜ = 1.24 min; m/z = 588 (M+H)⁺.
¹H-NMR (400MHz, DMSO-d₆): δ [ppm] = 1.24 (t, 3H), 2.46 (s, 3H), 3.27 (s, 3H), 4.21 (q, 2H), 5.07 (s, 2H), 7.32 - 7.37 (m, 1H), 7.38 - 7.45 (m, 2H), 7.60 (d, 1H), 7.84 - 7.88 (m, 1H), 7.96 (s, 1H), 8.53 (s, 1H).

### Beispiel 65

### Ethyl-3-[2-chlor-3-(trifluomiethyl)benzyl]-1-(,1'-methyl-2'-oxo-1',2'-dihydrospiro[cyclopropan-1,3'-indol]-5'-yl)-2,4-dioxo-1,2,3,4-tetrahydropyrimidin-5 -carboxylat

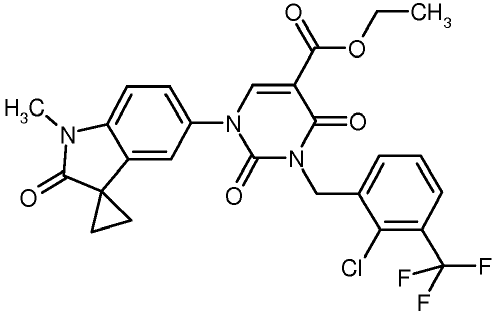

Darstellung und Reinigung der Titelverbindung erfolgten analog zu Beispiel 1. Die Reaktionszeit betrug 1h. Ausgehend von 120 mg (0.33 mmol) Ethyl-1-(1'-methyl-2'-oxo-1',2'-dihydrospiro[cyclopropan-1,3'-indol]-5'-yl)-2,4-dioxo-1,2,3,4-tetrahydropyrimidin-5-carboxylat aus Beispiel 63A und 101 mg (0.37 mmol) 1-(Brommethyl)-2-chlor-3-(trifluormethyl)benzol erhielt man 177 mg (90 % d. Th.) der Titelverbindung.
LC-MS (Methode 1): Rₜ = 1.12 min; m/z = 548 (M+H)⁺.
¹H-NMR (400MHz, DMSO-d₆): δ [ppm] = 1.23 (t, 3H), 1.55 - 1.60 (m, 2H), 1.64 - 1.69 (m, 2H), 3.26 (s, 3H), 4.20 (q, 2H), 5.14 (s, 2H), 7.18 - 7.24 (m, 2H), 7.44 (dd, 1H), 7.49 - 7.58 (m, 2H), 7.78 - 7.82 (m, 1H), 8.44 (s, 1H).

### Beispiel 66

### Ethyl-1-(1'-methyl-2'-oxo-1',2'-dihydrospiro[cyclopropan-1,3'-indol]-5'-yl)-3-[2-methyl-3-(trifluormethyl)benzyl]-2,4-dioxo-1,2,3,4-tetrahydropyrimidin-5-carboxylat

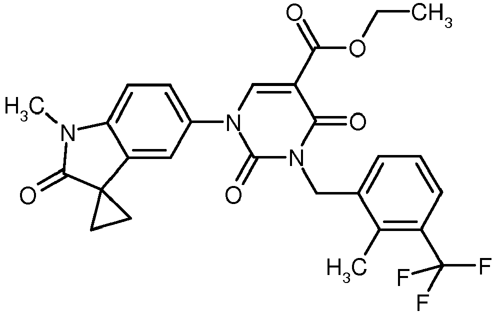

Darstellung und Reinigung der Titelverbindung erfolgten analog zu Beispiel 1. Die Reaktionszeit betrug 1.5h. Ausgehend von 120 mg (0.33 mmol) Ethyl-1-(1'-methyl-2'-oxo-1',2'-dihydrospiro[cyclopropan-1,3'-indol]-5'-yl)-2,4-dioxo-1,2,3,4-tetrahydropyrimidin-5-carboxylat aus Beispiel 63A und 94 mg (0.37 mmol) 1-(Brommethyl)-2-methyl-3-(trifluormethyl)benzol erhielt man 140 mg (77 % d. Th.) der Titelverbindung.
LC-MS (Methode 1): Rₜ = 1.12 min; m/z = 528 (M+H)⁺.
¹H-NMR (400MHz, DMSO-d₆): δ [ppm] = 1.23 (t, 3H), 1.55 - 1.60 (m, 2H), 1.64 - 1.69 (m, 2H), 2.46 (s, teilweise durch DMSO-Signal verdeckt), 3.26 (s, 3H), 4.19 (q, 2H), 5.07 (s, 2H), 7.17 - 7.25 (m, 2H), 7.32 - 7.37 (m, 2H), 7.44 (dd, 1H), 7.57 - 7.63 (m, 1H), 8.42 (s, 1H).

### Beispiel 67

### Ethyl-1-(,1'-methyl-2'-oxo-1',2'-dihydrospiro[cyclopropan-1,3'-indol]-5'-yl)-2,4-dioxo-3-[(1R)-4-(trifluormethyl)-2,3-dihydro-1H-inden-1-yl]-1,2,3,4-tetrahydropyrimidin-5-carboxylat (R-Enantiomer)

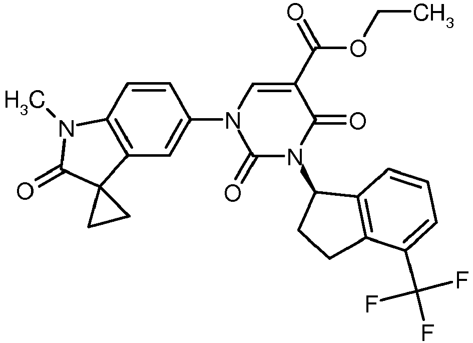

8.00 g (22.5 mmol) Ethyl-1-(1'-methyl-2'-oxo-1',2'-dihydrospiro[cyclopropan-1,3'-indol]-5'-yl)-2,4-dioxo-1,2,3,4-tetrahydropyrimidin-5-carboxylat aus Beispiel 63A, 5.46 g (27.0 mmol) (1S)-4-(Trifluormethyl)indan-1-ol (aus Beispiel 14A) und 10.0 g (38.26 mmol) Triphenylphosphin wurden unter Argon in THF / DMF 1:1 (215 ml) bei RT vorgelegt. Zu diesem Gemisch wurden unter Rühren 7.09 ml (36.02 mmol) Diisopropylazodicarboxylat getropft. Nach 1 h wurden zusätzlich 1.2 g (4.51 mmol) Triphenylphosphin und 0.89 ml (4.51 mmol) Diisopropylazodicarboxylat hinzugefügt. Die Reaktionsmischung wurde weiter 1.5 h bei RT gerührt. Unter Eiskühlung wurde das Gemisch mit 10 ml IM Salzsäure versetzt, 15 min gerührt, dann mit Essigsäureethylester extrahiert. Die vereinigten organischen Phasen wurden zweimal mit IM Salzsäure, dann zweimal mit einer gesättigten Natriumcarbonatlösung und einmal mit einer gesättigter Natriumchlorid-Lösung gewaschen, über Natriumsulfat getrocknet und am Rotationsverdampfer eingeengt. Der Rückstand wurde mit 100 ml MTBE verrührt und über Nacht stehen gelassen. Der gebildete Feststoff wurde abfiltriert und verworfen. Das Filtrat wurde am Rotationsverdampfer eingeengt. Der Rückstand wurde in wenig Dichlormethan aufgenommen und mittels Flashchromatographie (Eluens Cyclohexan / Essigsäureethylester 1:2) gereinigt. Man erhielt 7.81 g (59 % d. Th., 92 %-ige Reinheit laut NMR) der Titelverbindung.
LC-MS (Methode 4): Rₜ = 2.49 min; m/z = 540 (M+H)⁺.
¹H-NMR (400MHz, CD₂Cl₂): δ [ppm] = 1.31 (t, 3H), 1.54 - 1.61 (m, 2H), 1.69 - 1.81 (m, 2H), 2.35 - 2.49 (m, 1H), 2.51 - 2.66 (m, 1H), 3.05 - 3.21 (m, 1H), 3.28 (s, 3H), 3.39 - 3.54 (m, 1H), 4.28 (q, 2H), 6.54 - 6.67 (m, 1H), 6.80 (br.s, 1H), 6.97 (d, 1H), 7.19 (d, 1H), 7.24 - 7.36 (m, 2H), 7.49 (d, 1H), 8.26 (s, 1H).
Spezifischer optischer Drehwert: α_{D}²⁰ = +131.7°, (Chloroform, c = 0.405 g/100 ml).

### Beispiel 68

### Ethyl-3-[2-chlor-3-(trifluormethyl)benzyl]-1-(3,3-dimethyl-2-oxo-2,3-dihydro-1H-indol-6-yl)-2,4-dioxo-1,2,3,4-tetrahydropyrimidin-5-carboxylat

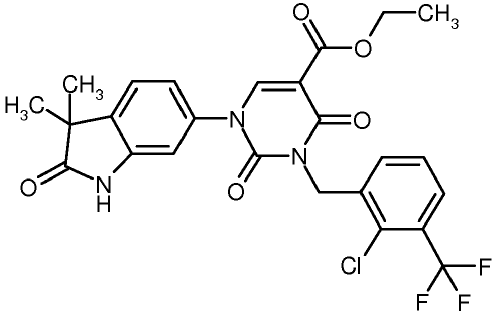

Zu einer Lösung von 629 mg (1.83 mmol) Ethyl-1-(3,3-dimethyl-2-oxo-2,3-dihydro-1H-indol-5-yl)-2,4-dioxo-1,2,3,4-tetrahydropyrimidin-5-carboxylat aus Beispiel 65A in 10 mL DMF wurden 551 mg (2.02 mmol) 1-(Brommethyl)-2-chlor-3-(trifluormethyl)benzol, 506 mg (3.66 mmol) Kaliumcarbonat sowie 30 mg (0.18 mmol) Kaliumiodid addiert und die Mischung 1.5 h bei 60°C gerührt. Zur Aufarbeitung wurde das Reaktionsgemisch mit Wasser versetzt, der entstandene Niederschlag abgesaugt und mit Wasser nachgewaschen. Das so erhaltene Rohprodukt wurde mittels Flash-Kieselgelchromatographie (Dichlormethan/Methanol, 98:2) gereinigt. Man erhielt 371 mg (88 %-ige Reinheit, 33 % d. Th.) der Zielverbindung.
LC-MS (Methode 1): Rₜ = 1.11 min; MS (ESIpos): m/z = 536 (M+H)⁺.
¹H-NMR (400 MHz, DMSO-d₆): δ [ppm] = 1.23 (s, 3H), 1.28 (s, 6H), 4.19 (q, 2H), 5.13 (s, 2H), 7.04 - 7.07 (m, 1H), 7.11 - 7.15 (m, 1H), 7.44 - 7.47 (m, 1H), 7.48 - 7.54 (m, 1H), 7.59 - 7.63 (m, 1H), 7.77 - 7.82 (m, 1H), 8.47 (s, 1H), 10.61 (s, 1H).

### Beispiel 69

### Ethyl-1-(1-acetyl-3,3-dimethyl-2,3-dihydro-1H-indol-6-yl)-3-[2-chlor-3-(trifluormethyl)benzyl]-2,4-dioxo-1,2,3,4-tetrahydropyrimidin-5-carboxylat

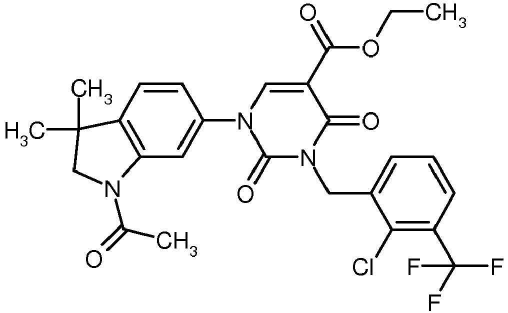

Darstellung und Reinigung der Titelverbindung erfolgten analog zu Beispiel 1. Die Reaktionszeit betrug 2h. Ausgehend von 200 mg (0.53 mmol) Ethyl-1-(1-acetyl-3,3-dimethyl-2,3-dihydro-1H-indol-6-yl)-2,4-dioxo-1,2,3,4-tetrahydropyrimidin-5-carboxylat aus Beispiel 66A und 162 mg (0.59 mmol) 1-(Brommethyl)-2-chlor-3-(trifluormethyl)benzol erhielt man 228 mg (71 % d. Th.) der Titelverbindung.
LC-MS (Methode 1): Rₜ = 1.18 min; m/z = 564 (M+H)⁺.
¹H-NMR (400MHz, DMSO-d₆): δ [ppm] = 1.23 (t, 3H), 1.34 (s, 6H), 2.18 (s, 3H), 3.94 (s, 2H), 4.19 (q, 2H), 5.12 (s, 2H), 7.19 (dd, 1H), 7.40 (d, 1H), 7.50 (t, 1H), 7.60 (d, 1H), 7.79 (d, 1H), 8.14 (s, 1H), 8.41 (s, 1H).

### Beispiel 70

### Ethyl-1-(1-acetyl-3,3-dimethyl-2,3-dihydro-1H-indol-6-yl)-3-[2-methyl-3-(trifluormethyl)benzyl]-2,4-dioxo-1,2,3,4-tetrahydropyrimidin-5-carboxylat

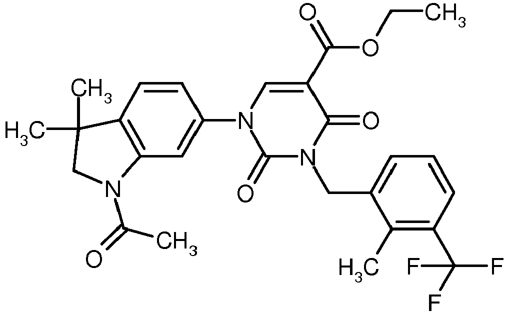

Darstellung und Reinigung der Titelverbindung erfolgten analog zu Beispiel 1. Die Reaktionszeit betrug 2h. Ausgehend von 200 mg (0.53 mmol) Ethyl-1-(1-acetyl-3,3-dimethyl-2,3-dihydro-1H-indol-6-yl)-2,4-dioxo-1,2,3,4-tetrahydropyrimidin-5-carboxylat aus Beispiel 66A und 149 mg (0.59 mmol) 1-(Brommethyl)-2-methyl-3-(trifluormethyl)benzol erhielt man 253 mg (84 % d. Th.) der Titelverbindung.
LC-MS (Methode 1): Rₜ = 1.22 min; m/z = 544 (M+H)⁺.
¹H-NMR (400MHz, DMSO-d₆): δ [ppm] = 1.23 (t, 3H), 1.34 (s, 6H), 2.18 (s, 3H), 2.45 (s, 3H), 3.93 (s, 2H), 4.19 (q, 2H), 5.06 (s, 2H), 7.19 (dd, 1H), 7.33 (t, 1H), 7.40 (d, 2H), 7.59 (d, 1H), 8.13 (d, 1H), 8.39 (s, 1H).

### Beispiel 71

### Ethyl-3-[2-chlor-3-(trifluormethyl)benzyl]-1-(-methyl-1H-benzotriazol-5-yl)-2,4-dioxo-1,2,3,4-tetrahydro-pyrimidin-5-carboxylat

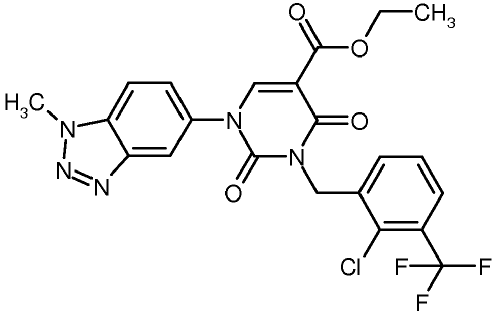

162 mg (0.51 mmol) Ethyl-1-(1-methyl-1H-benzotriazol-5-yl)-2,4-dioxo-1,2,3,4-tetrahydropyrimidin-5-carboxylat aus Beispiel 74A wurden in 6.5 ml DMF vorgelegt. 155 mg (0.56 mmol) 1-(Brommethyl)-2-chlor-3-(trifluormethyl)benzol, 142 mg (1.03 mmol) Kaliumcarbonat und 9 mg (52 µmol) Kaliumiodid wurden zugegeben und die Reaktionsmischung 5 h bei 60°C gerührt. Nach Abkühlung auf RT wurde das Gemisch mit Wasser versetzt. Der Niederschlag wurde abfiltriert, mit wenig Wasser und MTBE gewaschen und im Trockenschrank bei 50°C getrocknet. Man erhielt 149 mg (95 %-ige Reinheit, 54 % d. Th.) der Titelverbindung.
LC-MS (Methode 1): Rₜ = 1.03 min; m/z = 508 (M+H)⁺.
¹H-NMR (400MHz, DMSO-d₆): δ [ppm] = 1.23 (t, 3H), 4.21 (q, 2H), 4.37 (s, 3H), 5.17 (s, 2H), 7.51 - 7.57 (m, 1H), 7.61 - 7.65 (m, 1H), 7.71 - 7.75 (m, 1H), 7.79 - 7.83 (m, 1H), 7.99 - 8.02 (m, 1H), 8.31 - 8.33 (m, 1H), 8.61 (s, 1H).

### Beispiel 72

### Ethyl-1-(1-methyl-1H-benzotriazol-5-yl)-3-[2-methyl-3-(trifluormethyl)benzyl]-2,4-dioxo-1,2,3,4-tetrahydro-pyrimidin-5-carboxylat

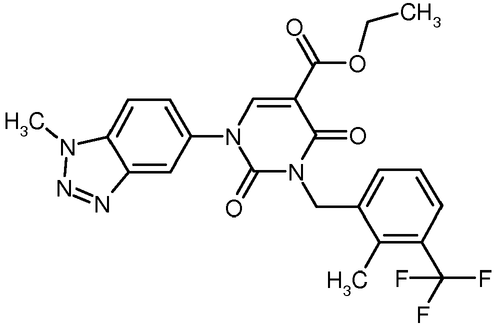

Darstellung und Reinigung der Titelverbindung erfolgten analog zu Beispiel 71. Ausgehend von 162 mg (0.51 mmol) Ethyl-1-(1-methyl-1H-benzotriazol-5-yl)-2,4-dioxo-1,2,3,4-tetrahydropyrimidin-5-carboxylat aus Beispiel 74A und 143 mg (0.56 mmol) 1-(Brommethyl)-2-methyl-3-(trifluormethyl)benzol erhielt man 152 mg (59 % d. Th.) der Titelverbindung.
LC-MS (Methode 1): Rₜ = 1.02 min; m/z = 488 (M+H)⁺.
¹H-NMR (400MHz, DMSO-d₆): δ [ppm] = 1.23 (t, 3H), 2.47 (s, teilweise durch DMSO-Signal verdeckt), 4.20 (q, 2H), 4.37 (s, 3H), 5.10 (s, 2H), 7.33 - 7.39 (m, 1H), 7.40 - 7.45 (m, 1H), 7.58 - 7.64 (m, 1H), 7.73 (dd, 1H), 8.00 (d, 1H), 8.32 - 8.34 (m, 1H), 8.58 (s, 1H).

### Beispiel 73

### Ethyl-3-(2,3-dichlorbenzyl)-1-(1-methyl-1H-benzotriazol-5-yl)-2,4-dioxo-1,2,3,4-tetrahydropyrimidin-5-carboxylat

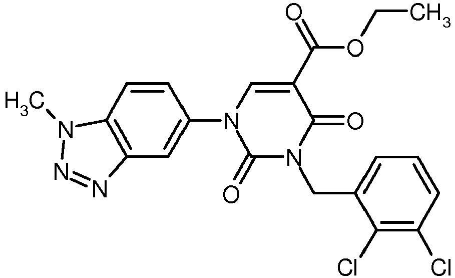

Darstellung und Reinigung der Titelverbindung erfolgten analog zu Beispiel 71. Ausgehend von 162 mg (0.51 mmol) Ethyl-1-(1-methyl-1H-benzotriazol-5-yl)-2,4-dioxo-1,2,3,4-tetrahydropyrimidin-5 -carboxylat aus Beispiel 74A und 136 mg (0.56 mmol) 2,3-Dichlorbenzylbromid erhielt man 188 mg (74 % d. Th.) der Titelverbindung.
LC-MS (Methode 1): Rₜ = 0.99 min; m/z = 474 (M+H)⁺.
¹H-NMR (400MHz, DMSO-d₆): δ [ppm] = 1.23 (t, 3H), 4.20 (q, 2H), 4.37 (s, 3H), 5.12 (s, 2H), 7.27 (d, 1H), 7.35 (t, 1H), 7.59 (d, 1H), 7.72 (d, 1H), 8.00 (d, 1H), 8.32 (s, 1H), 8.59 (s, 1H).

### Beispiel 74

### Ethyl-3-[3-chlor-2-(trifluormethyl)benzyl]-1-(-methyl-1H-benzotriazol-5-yl)-2,4-dioxo-1,2,3,4-tetrahydro-pyrimidin-5-carboxylat

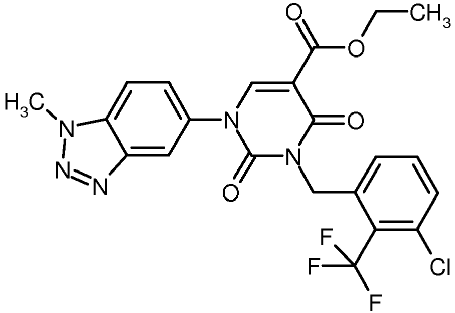

162 mg (0.51 mmol) Ethyl-1-(1-methyl-1H-benzotriazol-5-yl)-2,4-dioxo-1,2,3,4-tetrahydropyrimidin-5-carboxylat aus Beispiel 74A wurden in DMF (6 ml) vorgelegt, 238 mg (65 %-ige Reinheit, 0.56 mmol) 1-(Brommethyl)-3-chlor-2-(trifluormethyl)benzol, 142 mg (1.03 mmol) Kaliumcarbonat und 8 mg (0.05 mmol) Kaliumiodid wurden zugegeben. Anschließend ließ man die Reaktionsmischung 5 h bei 60°C rühren. Das auf RT abgekühlte Gemisch wurde mit Wasser versetzt und zweimal mit Essigsäureethylester extrahiert. Die vereinigten organischen Phasen wurden mit einer gesättigten Natriumchlorid-Lösung gewaschen, über Magnesiumsulfat getrocknet, abfiltriert und eingeengt. Der Rückstand wurde mit Cyclohexan/Essigsäureethylester verrührt, der ausgefallene Feststoff abgesaugt und im Vakuum getrocknet. Man erhielt 115 mg (43 % d. Th.) der Titelverbindung.
LC-MS (Methode 1): Rₜ = 1.02 min; m/z = 508 (M+H)⁺.
¹H-NMR (400MHz, DMSO-d₆): δ [ppm] = 1.23 (t, 3H), 4.21 (q, 2H), 4.37 (s, 3H), 5.23 (br.s, 2H), 7.36 - 7.41 (m, 1H), 7.58 - 7.67 (m, 2H), 7.70 - 7.75 (m, 1H), 8.00 (d, 1H), 8.30 - 8.33 (m, 1H), 8.62 (s, 1H).

### Beispiel 75

### Ethyl-1-(1-methyl-1H-indazol-5-yl)-3-[2-methyl-3-(trifluormethyl)benzyl]-2,4-dioxo-1,2,3,4-tetrahydro-pyrimidin-5-carboxylat

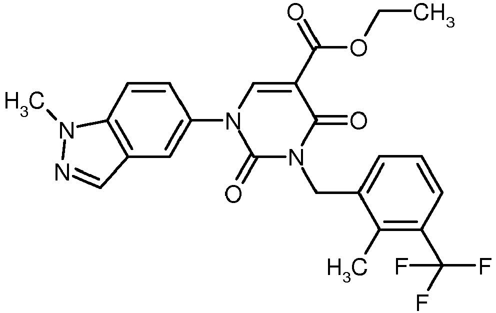

Darstellung und Reinigung der Titelverbindung erfolgten analog zu Beispiel 1. Die Reaktionszeit betrug 2h. Ausgehend von 200 mg (0.63 mmol) Ethyl-1-(1-methyl-1H-indazol-5-yl)-2,4-dioxo-1,2,3,4-tetrahydro-pyrimidin-5-carboxylat aus Beispiel 75A und 177 mg (0.70 mmol) 1-(Brommethyl)-2-methyl-3-(trifluormethyl)benzol erhielt man 254 mg (80 % d. Th.) der Titelverbindung.
LC-MS (Methode 1): Rₜ = 1.11 min; m/z = 487 (M+H)⁺.
¹H-NMR (400MHz, DMSO-d₆): δ [ppm] = 1.22 (t, 3H), 2.46 (s, 3H), 4.10 (s, 3H), 4.19 (q, 2H), 5.09 (s, 2H), 7.32 - 7.44 (m, 2H), 7.54 (d, 1H), 7.60 (d, 1H), 7.78 (d, 1H), 7.98 (s, 1H), 8.18 (s, 1H), 8.49 (s, 1H).

### Beispiel 76

### Ethyl-1-(1-methyl-1H-indazol-5-yl)-2,4-dioxo-3-[(1R)-4-(trifluormethyl)-2,3-dihydro-1H-inden-1-yl]-1,2,3,4-tetrahydropyrimidin-5-carboxylat (R-Enantiomer)

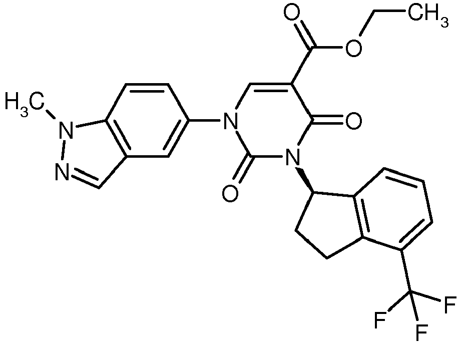

200 mg (0.63 mmol) Ethyl-1-(1-methyl-1H-indazol-5-yl)-2,4-dioxo-1,2,3,4-tetrahydropyrimidin-5-carboxylat aus Beispiel 75A und 500 mg (1.90 mmol) Triphenylphosphin wurden unter Argon in THF/DMF 1:1 (8.4 ml) vorgelegt und auf -30°C abgekühlt. 257 mg (1.27 mmol) Diisopropylazodicarboxylat und eine Lösung von 154 mg (0.76 mmol (1S)-4-(Trifluormethyl)indan-1-ol aus Beispiel 14A in 1 ml THF wurden zugetropft. Die Reak-tionsmischung wurde 16 h bei RT gerührt. Zur Aufarbeitung wurde das Reaktionsgemisch auf - 40°C abgekühlt, mit IM Salzsäure versetzt, auf RT erwärmt und mit Essigsäureethylester extrahiert. Die organische Phase wurde nacheinander zweimal mit IM Salzsäure und einmal mit gesättigter Natriumchlorid-Lösung gewaschen, über Magnesiumsulfat getrocknet und eingeengt. Der Rückstand wurde mittels HPLC (Methode 8) gereinigt. Man erhielt 142 mg (43 % d. Th.) der Titelverbindung.
LC-MS (Methode 1): Rₜ = 1.11 min; m/z = 499 (M+H)⁺.
¹H-NMR (400MHz, CDCl₃): δ [ppm] = 1.36 (t, 3H), 2.40 - 2.52 (m, 1H), 2.53 - 2.61 (m, 1H, teilweise verdeckt durch DMSO-Signal), 3.08 - 3.19 (m, 1H), 3.45 - 3.58 (m, 1H), 4.11 (s, 3H), 4.35 (q, 2H), 6.61 - 6.77 (m, 1H), 7.23 - 7.33 (m, 3H, teilweise verdeckt durch CDCl₃-Signal), 7.44 - 7.53 (m, 2H), 7.69 (s, 1H), 8.04 (s, 1H), 8.37 (s, 1H).
Spezifischer optischer Drehwert: α_{D}²⁰ = +146.6°, (Chloroform, c = 0.405 g/100 ml).

### Beispiel 77

### Ethyl-1-(1-methyl-1H-benzimidazol-5-yl)-3-[2-methyl-3-(trifluormethyl)benzyl]-2,4-dioxo-1,2,3,4-tetrahydro-pyrimidin-5-carboxylat

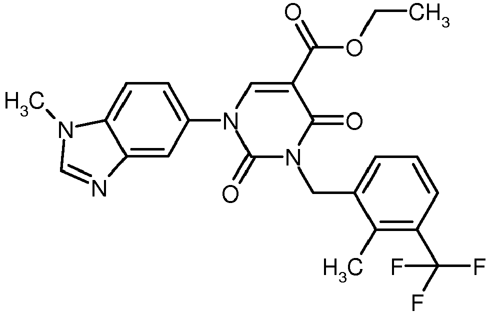

1.00 g (3.18 mmol) Ethyl-1-(1-methyl-1H-benzimidazol-5-yl)-2,4-dioxo-1,2,3,4-tetrahydropyrimidin-5-carboxylat aus Beispiel 76A wurden in DMF (8 ml) vorgelegt, 886 mg (3.50 mmol) 1-(Brommethyl)-2-methyl-3-(trifluomethyl)benzol, 879 mg (6.36 mmol) Kaliumcarbonat und 53 mg (0.32 mmol) Kaliumiodid wurden zugegeben. Anschließend ließ man die Reaktionsmischung 5h bei 60°C rühren. Das auf RT abgekühlte Gemisch wurde mit Wasser versetzt, der Niederschlag abgesaugt, mit Wasser sowie Ethanol/MTBE gewaschen und im Vakuum bei 50 °C getrocknet. Man erhielt 1.06 g (68 % d. Th.) der Titelverbindung.
LC-MS (Methode 1): Rₜ = 0.93 min; m/z = 487 (M+H)⁺.
¹H-NMR (400MHz, DMSO-d₆): δ [ppm] = 1.23 (t, 3H), 2.46 (s, 3H), 3.89 (s, 3H), 4.19 (q, 2H), 5.09 (s, 2H), 7.32 - 7.46 (m, 3H), 7.60 (d, 1H), 7.71 (d, 1H), 7.89 (d, 1H), 8.33 (s, 1H), 8.46 (s, 1H).

### Beispiel 78

### Ethyl-3-[3-chlor-2-(trifluormethyl)benzyl]-1-(1-methyl-1H-benzimidazol-5-yl)-2,4-dioxo-1,2,3,4-tetrahydro-pyrimidin-5-carboxylat

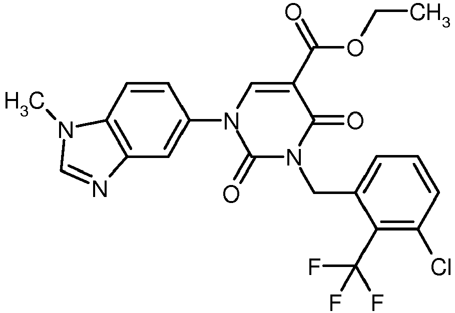

Darstellung und Reinigung der Titelverbindung erfolgten analog zu Beispiel 77. Ausgehend von 200 mg (0.63 mmol) Ethyl-1-(1-methyl-1H-benzimidazol-5-yl)-2,4-dioxo-1,2,3,4-tetrahydropyrimidin-5-carboxylat aus Beispiel 76A und 295 mg (65 %-ige Reinheit, 0.70 mmol) 1-(Brommethyl)-3-chlor-2-(trifluormethyl)benzol erhielt man 82 mg (26 % d. Th.) der Titelverbindung.
LC-MS (Methode 1): Rₜ = 0.97 min; m/z = 507 (M+H)⁺.
¹H-NMR (400MHz, DMSO-d₆): δ [ppm] = 1.23 (t, 3H), 3.89 (s, 3H), 4.20 (q, 2H), 5.23 (s, 2H), 7.36 (d, 1H), 7.41 - 7.46 (m, 1H), 7.57 - 7.67 (m, 2H), 7.70 (d, 1H), 7.87 (d, 1H), 8.32 (s, 1H), 8.49 (s, 1H).

### Beispiel 79

### Ethyl-3-[2-chlor-3-(trifluormethyl)benzyl]-1-(1-methyl-1H-benzimidazol-5-yl)-2,4-dioxo-1,2,3,4-tetrahydro-pyrimidin-5-carboxylat

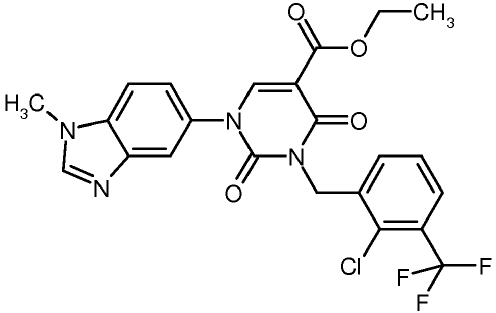

Darstellung der Titelverbindung erfolgten analog zu Beispiel Beispiel 77. Ausgehend von 200 mg (0.63 mmol) Ethyl-1-(1-methyl-1H-benzimidazol-5-yl)-2,4-dioxo-1,2,3,4-tetrahydropyrimidin-5-carboxylat aus Beispiel 76A und 191 mg (0.70 mmol) 1-(Brommethyl)-2-chlor-3-(trifluormethyl)benzol erhielt man nach zusätzlicher Reinigung mittels präparativer HPLC (Methode 8) 153 g (47 % d. Th.) der Titelverbindung.
LC-MS (Methode 1): Rₜ = 0.97 min; m/z = 507 (M+H)⁺.
¹H-NMR (400MHz, DMSO-d₆): δ [ppm] = 1.23 (t, 3H), 3.89 (s, 3H), 4.20 (q, 2H), 5.16 (s, 2H), 7.44 (dd, 1H), 7.50 - 7.56 (m, 1H), 7.59 - 7.63 (m, 1H), 7.71 (d, 1H), 7.78 - 7.83 (m, 1H), 7.88 (d, 1H), 8.33 (s, 1H), 8.49 (s, 1H).

### Beispiel 80

### Ethyl-1-(1-ethyl-1H-benzimidazol-5-yl)-3-[2-methyl-3-(trifluormethyl)benzyl]-2,4-dioxo-1,2,3,4-tetrahydro-pyrimidin-5-carboxylat

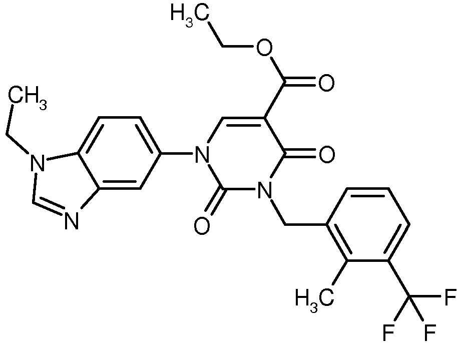

122.5 mg (0.37 mmol) Ethyl-1-(1-ethyl-1H-benzimidazol-5-yl)-2,4-dioxo-1,2,3,4-tetrahydropyrimidin-5-carboxylat aus Beispiel 46A und 103 mg (0.41 mmol) 1-(Brommethyl)-2-methyl-3-(trifluormethyl)benzol wurden in DMF (4 ml) vorgelegt und mit 103 mg (0.74 mmol) Kaliumcarbonat sowie 6 mg (0.04 mmol) Kaliumiodid versetzt. Die Reaktionsmischung wurde 5 h bei 60°C gerührt, anschließend auf RT gebracht und mit Wasser versetzt. Der ausgefallene Niederschlag wurde abgesaugt, mit Wasser und MTBE gewaschen und im Vakuum bei 50°C über Nacht getrocknet. Man erhielt 38 mg (19 % d. Th.) der Titelverbindung.
LC-MS (Methode 1): Rₜ = 0.97 min; m/z = 501 (M+H)⁺.
¹H-NMR (400MHz, DMSO-d₆): δ [ppm] = 1.23 (t, 3H), 1.43 (t, 3H), 2.46 (s, 3H), 4.19 (q, 2H), 4.33 (q, 2H), 5.09 (s, 2H), 7.31 - 7.46 (m, 3H), 7.60 (d, 1H), 7.76 (d, 1H), 7.89 (d, 1H), 8.40 (s, 1H), 8.48 (s, 1H), 8.48 (s, 1H).

### Beispiel 81

### Ethyl-1-(2-carbamoyl-1-methyl-1H-benzimidazol-5-yl)-3-[2-methyl-3-(trifluormethyl)benzyl]-2,4-dioxo-1,2,3,4-tetrahydropyrimidin-5-carboxylat

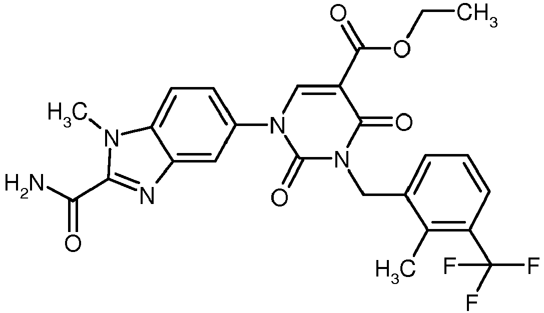

158 mg (0.44 mmol) Ethyl-1-(2-carbamoyl-1-methyl-1H-benzimidazol-5-yl)-2,4-dioxo-1,2,3,4-tetrahydro-pyrimidin-5-carboxylat aus Beispiel 81A wurden in DMF (3 ml) vorgelegt und mit 123 mg (0.48 mmol) 1-(Brommethyl)-2-methyl-3-(trifluormethyl)benzol, 122 mg (0.88 mmol) Kaliumcarbonat und 7 mg (0.04 mmol) Kaliumiodid versetzt. Man ließ die Reaktionsmischung 1 h bei 80°C rühren. Das abgekühlte Gemisch wurde mit Wasser versetzt, der ausgefallene Feststoff abfiltriert und mit Wasser gewaschen. Das Filtrat wurde zweimal mit Dichlormethan extahiert, die vereinigten organischen Phasen über Magnesiumsulfat getrocknet, abfiltriert und eingeengt. Der Rückstand wurde zusammen mit dem vorher isolierten Feststoff vereinigt und mittels präparativer (Methode 8) gereinigt. Man erhielt 131 mg (54 % d. Th.) der Titelverbindung.
LC-MS (Methode 1): Rₜ = 0.98 min; m/z = 530 (M+H)⁺.
¹H-NMR (400MHz, DMSO-d₆): δ [ppm] = 1.23 (t, 3H), 2.47 (s, teilweise durch DMSO-Signal verdeckt), 4.16 (s, 3H), 4.20 (q, 2H), 5.10 (s, 2H), 7.33 - 7.39 (m, 1H), 7.39 - 7.44 (m, 1H), 7.53 - 7.63 (m, 2H), 7.82 (d, 1H), 7.92 (br.s, 1H), 7.96 - 8.00 (m, 1H), 8.32 (br. s, 1H), 8.50 (s, 1H).

### Beispiel 82

### Ethyl-3-[2-chlor-3-(trifluormethyl)benzyl]-1-(1-methyl-1H-benzimidazol-6-yl)-2,4-dioxo-1,2,3,4-tetrahydro-pyrimidin-5-carboxylat

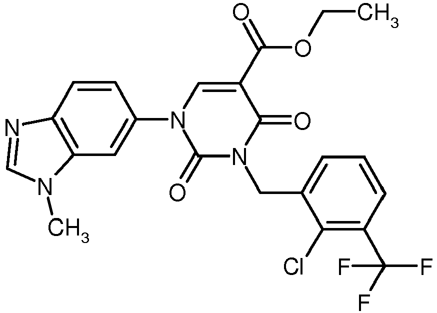

Darstellung und Reinigung der Titelverbindung erfolgten analog zu Beispiel 80. Die Reaktionszeit betrug 1h. Ausgehend von 150 mg (0.47 mmol) Ethyl-1-(1-methyl-1H-benzimidazol-6-yl)-2,4-dioxo-1,2,3,4-tetrahydro-pyrimidin-5-carboxylat aus Beispiel 82A und 143 mg (0.52 mmol) 1-(Brommethyl)-2-chlor-3-(trifluormethyl)benzol erhielt man nach zusätzlicher Reinigung mittels Flashchromatographie (Dichlormethan/Methanol 98:2) 110 mg (44 % d. Th.) der Titelverbindung.
LC-MS (Methode 1): Rₜ = 0.92 min; m/z = 507 (M+H)⁺.
¹H-NMR (400MHz, DMSO-d₆): δ [ppm] = 1.23 (t, 3H), 3.87 (s, 3H), 4.20 (q, 2H), 5.17 (s, 2H), 7.37 (dd, 1H), 7.54 (t, 1H), 7.61 (d, 1H), 7.77 (d, 1H), 7.81 (d, 1H), 7.84 (d, 1H), 8.34 (s, 1H), 8.52 (s, 1H).

### Beispiel 83

### Ethyl-1-(1-ethyl-2-methyl-1H-benzimidazol-5-yl)-3-[3-fluor-2-(trifluormethyl)benzyl]-2,4-dioxo-1,2,3,4-tetrahydropyrimidin-5-carboxylat

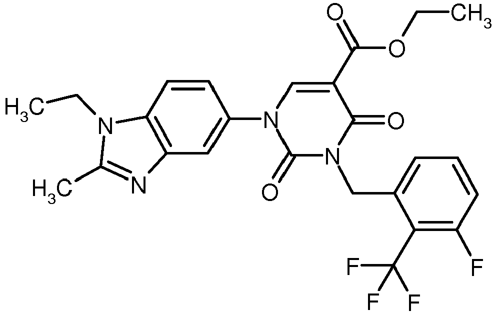

200 mg (0.58 mmol) Ethyl-1-(1-ethyl-2-methyl-1H-benzimidazol-5-yl)-2,4-dioxo-1,2,3,4-tetrahydropyrimidin-5-carboxylat aus 117A wurden in DMF (7 ml) vorgelegt und mit 165 mg (0.64 mmol) 1-(Brommethyl)-3-fluor-2-(trifluormethyl)benzol, 161 mg (1.17 mmol) Kaliumcarbonat und 10 mg (0.06 mmol) Kaliumiodid versetzt. Man ließ die Reaktionsmischung 5 h bei 60°C rühren. Das abgekühlte Gemisch wurde mit Wasser versetzt, der ausgefallene Feststoff abfiltriert und mit Wasser gewaschen. Der Feststoff wurde in Dichlormethan gelöst und mittels Flash-Kieselgelchromatographie (Dichlormethan/Methanol, 30:1) gereinigt. Man erhielt 153 mg (50 % d. Th.) der Titelverbindung.
LC-MS (Methode 1): Rₜ = 0.85 min; m/z = 519 (M+H)⁺.
¹H-NMR (400MHz, DMSO-d₆): δ [ppm] = 1.22 (t, 3H), 1.32 (t, 3H), 2.58 (s, 3H), 4.19 (q, 2H), 4.28 (q, 2H), 5.21 (s, 2H), 7.22 (d, 1H), 7.33 (d, 1H), 7.41 (t, 1H), 7.61 - 7.70 (m, 2H), 7.70 - 7.75 (m, 1H), 8.44 - 8.50 (m, 1H).

### Beispiel 84

### Ethyl-3-[2-chlor-3-(trifluormethyl)benzyl]-1-(1-ethyl-2-methyl-1H-benzimidazol-5-yl)-2,4-dioxo-1,2,3,4-tetrahydropyrimidin-5-carboxylat

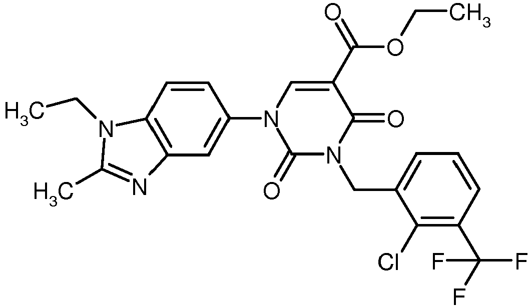

Darstellung und Reinigung der Titelverbindung erfolgten analog zu Beispiel 83. Ausgehend von 200 mg (0.58 mmol) Ethyl-1-(1-ethyl-2-methyl-1H-benzimidazol-5-yl)-2,4-dioxo-1,2,3,4-tetrahydropyrimidin-5-carboxylat aus Beispiel 117A und 175 mg (0.63 mmol) 1-(Brommethyl)-2-chlor-3-(trifluormethyl)benzol erhielt man 114 mg (36 % d. Th.) der Titelverbindung.
LC-MS (Methode 1): Rₜ = 0.89 min; m/z = 535 (M+H)⁺.
¹H-NMR (400MHz, DMSO-d₆): δ [ppm] = 1.23 (t, 3H), 1.31 (t, 3H), 2.58 (s, 3H), 4.19 (q, 2H), 4.27 (q, 2H), 5.16 (s, 2H), 7.35 (dd, 1H), 7.53 (t, 1H), 7.60 (d, 1H), 7.65 (d, 1H), 7.73 (d, 1H), 7.80 (d, 1H), 8.47 (s, 1H).

### Beispiel 85

### Ethyl-1-(1-cyclohexyl-2-methyl-1H-benzimidazol-5-yl)-3-[3-fluor-2-(trifluormethyl)benzyl]-2,4-dioxo-1,2,3,4-tetrahydropyrimidin-5-carboxylat

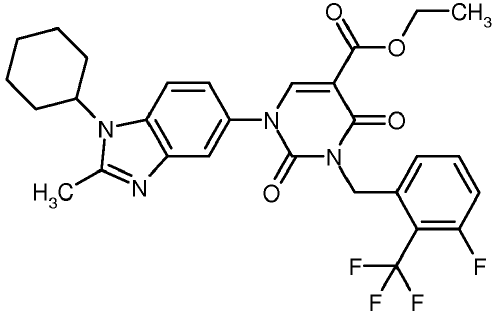

Darstellung und Reinigung der Titelverbindung erfolgten analog zu Beispiel 83. Ausgehend von 200 mg (0.50 mmol) Ethyl-1-(1-cyclohexyl-2-methyl-1H-benzimidazol-5-yl)-2,4-dioxo-1,2,3,4-tetrahydropyrimidin-5-carboxylat aus Beispiel 118A und 142 mg (0.55 mmol) 1-(Brommethyl)-3-fluor-2-(trifluormethyl)benzol erhielt man 90 mg (30 % d. Th.) der Titelverbindung.
LC-MS (Methode 1): Rₜ = 1.05 min; m/z = 573 (M+H)⁺.
¹H-NMR (400MHz, DMSO-d₆): δ [ppm] = 1.23 (t, 3H), 1.34 - 1.57 (m, 3H), 1.67 - 1.75 (m, 1H), 1.82 - 1.92 (m, 4H), 2.10 - 2.23 (m, 2H), 2.60 (s, 3H), 4.19 (q, 2H), 4.26 - 4.37 (m, 1H), 5.20 (s, 2H), 7.22 (d, 1H), 7.29 (dd, 1H), 7.36 - 7.45 (m, 1H), 7.62 - 7.70 (m, 1H), 7.71 (d, 1H), 7.83 (d, 1H), 8.47 (s, 1H).

### Beispiel 86

### Ethyl-3-[2-chlor-3-(trifluormethyl)benzyl]-1-(1-isopropyl-1H-benzimidazol-5-yl)-2,4-dioxo-1,2,3,4-tetrahydro-pyrimidin-5-carboxylat

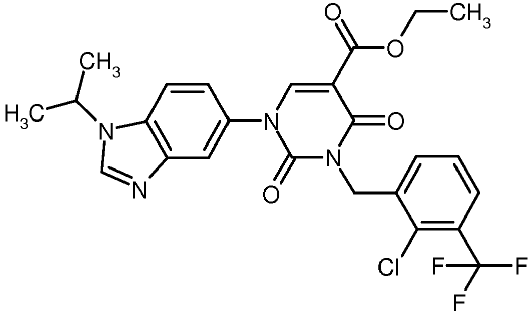

Darstellung der Titelverbindung erfolgte analog zu Beispiel 83. Ausgehend von 200 mg (0.58 mmol) Ethyl-1-(1-isopropyl-1H-benzimidazol-5-yl)-2,4-dioxo-1,2,3,4-tetrahydropyrimidin-5-carboxylat aus Beispiel 51A und 175 mg (0.64 mmol) 1-(Brommethyl)-2-chlor-3-(trifluormethyl)benzol erhielt man nach Reinigung mittels Flashchromatographie (Dichlormethan/Methanol 50:1) 64 mg (19 % d. Th.) der Titelverbindung.
LC-MS (Methode 1): Rₜ = 1.05 min; m/z = 535 (M+H)⁺.
¹H-NMR (400MHz, DMSO-d₆): δ [ppm] = 1.23 (t, 3H), 1.56 (d, 6H), 4.19 (q, 2H), 4.82 (spt, 1H), 5.16 (s, 2H), 7.42 (dd, 1H), 7.54 (t, 1H), 7.61 (d, 1H), 7.80 (d, 2H), 7.88 (d, 1H), 8.49 (s, 1H), 8.51 (s, 1H).

### Beispiel 87

### Ethyl-3-(2,3-dichlorbenzyl)-1-(1-isopropyl-1H-benzimidazol-5-yl)-2,4-dioxo-1,2,3,4-tetrahydropyrimidin-5-carboxylat

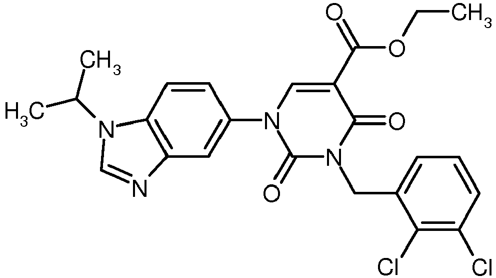

Darstellung der Titelverbindung erfolgte analog zu Beispiel 83. Ausgehend von 200 mg (0.58 mmol) Ethyl-1-(1-isopropyl-1H-benzimidazol-5-yl)-2,4-dioxo-1,2,3,4-tetrahydropyrimidin-5-carboxylat aus Beispiel 51A und 154 mg (0.64 mmol) 1-(Brommethyl)-2,3-dichlorbenzol erhielt man nach Reinigung mittels Flashchromatographie (Dichlormethan/Methanol 50:1) 83 mg (28 % d. Th.) der Titelverbindung.
LC-MS (Methode 1): Rₜ = 1.02 min; m/z = 501 (M+H)⁺.
¹H-NMR (400MHz, DMSO-d₆): δ [ppm] = 1.23 (t, 3H), 1.56 (d, 6H), 4.19 (q, 2H), 4.82 (spt, 1H), 5.11 (s, 2H), 7.26 (d, 1H), 7.34 (t, 1H), 7.42 (dd, 1H), 7.58 (d, 1H), 7.79 (d, 1H), 7.88 (d, 1H), 8.49 (s, 2H).

### Beispiel 88

### Ethyl-3-[2-chlor-3-(trifluormethyl)benzyl]-1-(-cyclobutyl-1H-benzimidazol-5-yl)-2,4-dioxo-1,2,3,4-tetrahydropyrimidin-5-carboxylat

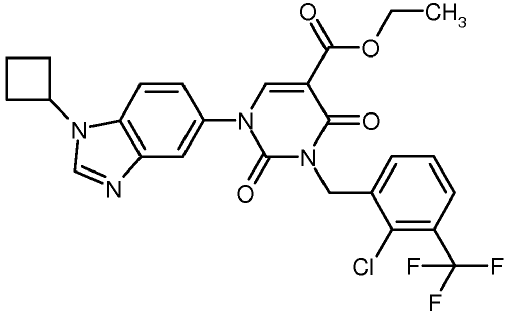

Darstellung der Titelverbindung erfolgte analog zu Beispiel 83 unter Verwendung von 200 mg (0.56 mmol) Ethyl-1-(1-cyclobutyl-1H-benzimidazol-5-yl)-2,4-dioxo-1,2,3,4-tetrahydropyrimidin-5-carboxylat aus Beispiel 41A und 169 mg (0.62 mmol) 1-(Brommethyl)-2-chlor-3-(trifluormethyl)benzol. Zur Aufarbeitung wurde das Reaktionsgemisch mit Wasser versetzt, der Niederschlag abgesaugt, mit Wasser sowie MTBE gewaschen und über Nacht bei 50°C im Vakuum getrocknet. Der Feststoff wurde mittels Flashchromatographie (Dichlormethan/Methanol 70:1) gereinigt. Die produkthaltigen Fraktionen wurden eingeengt, der Rückstand in Ethanol ausgerührt, abfiltriert, mit Ethanol gewaschen und im Hochvakuum getrocknet. Man erhielt 141 mg (42 % d. Th.) der Titelverbindung.
LC-MS (Methode 1): Rₜ = 1.06 min; m/z = 547 (M+H)⁺.
¹H-NMR (400MHz, DMSO-d₆): δ [ppm] = 1.23 (t, 3H), 1.86 - 1.96 (m, 2H), 2.56 (s, 4H, teilweise durch DMSO-Signal verdeckt), 4.20 (q, 2H), 5.04 (quin, 1H), 5.16 (s, 2H), 7.42 (dd, 1H), 7.53 (t, 1H), 7.62 (d, 1H), 7.74 (d, 1H), 7.81 (d, 1H), 7.89 (d, 1H), 8.50 (s, 1H), 8.55 (s, 1H).

### Beispiel 89

Ethyl-3-[2-chlor-3-(trifluormethyl)benzyl]-1-(1-methyl-1H-indazol-5-yl)-2,4-dioxo-1,2,3,4-tetrahydro-pyrimidin-5-carboxylat

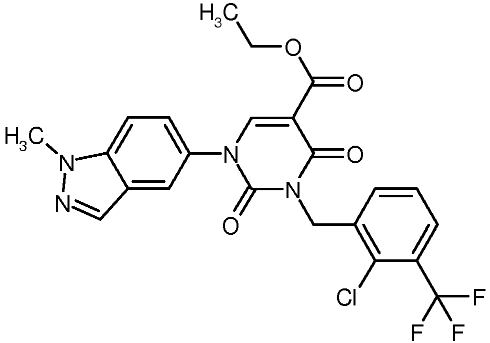

Darstellung und Reinigung der Titelverbindung erfolgten analog zu Beispiel 80. Ausgehend von 200 mg (0.63 mmol) Ethyl-1-(1-methyl-1H-indazol-5-yl)-2,4-dioxo-1,2,3,4-tetrahydropyrimidin-5-carboxylat aus Beispiel 75A und 191 mg (0.70 mmol) 1-(Brommethyl)-2-chlor-3-(trifluormethyl)benzol erhielt man 228 mg (67 % d. Th.) der Titelverbindung.
LC-MS (Methode 1): Rₜ = 1.11 min; m/z = 507 (M+H)⁺.
¹H-NMR (400MHz, DMSO-d₆): δ [ppm] = 1.24 (t, 3H), 4.10 (s, 3H), 4.20 (q, 2H), 5.16 (s, 2H), 7.49 - 7.57 (m, 2H), 7.62 (d, 1H), 7.74 - 7.84 (m, 2H), 7.98 (d, 1H), 8.18 (s, 1H), 8.52 (s, 1H).

### Beispiel 90

### Ethyl-1-(1-ethyl-1H-benzimidazol-5-yl)-3-[3-fluor-2-(trifluormethyl)benzyl]-2,4-dioxo-1,2,3,4-tetrahydropyrimidin-5-carboxylat

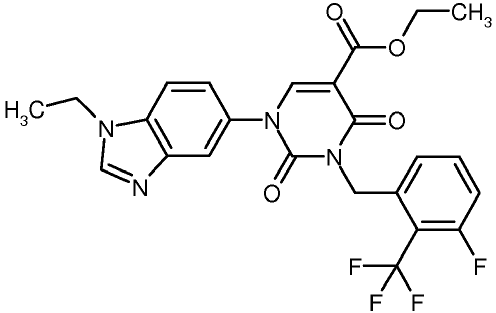

Darstellung und Reinigung der Titelverbindung erfolgten analog zu Beispiel 1. Ausgehend von 122.5 mg (0.37 mmol) Ethyl-1-(1-ethyl-1H-benzimidazol-5-yl)-2,4-dioxo-1,2,3,4-tetrahydropyrimidin-5-carboxylat aus Beispiel 46A und 105 mg (0.41 mmol) 1-(Brommethyl)-3-fluor-2-(trifluormethyl)benzol erhielt man 73 mg (35 % d. Th.) der Titelverbindung.
LC-MS (Methode 1): Rₜ = 0.92 min; m/z = 505 (M+H)⁺.
¹H-NMR (400MHz, DMSO-d₆): δ [ppm] = 1.23 (t, 3H), 1.43 (t, 3H), 4.20 (q, 2H), 4.33 (q, 2H), 5.21 (br. s, 2H), 7.22 (d, 1H), 7.36 - 7.46 (m, 2H), 7.67 (q, 1H), 7.76 (d, 1H), 7.84 - 7.90 (m, 1H), 8.40 (s, 1H), 8.51 (s, 1H).

### Beispiel 91

### Ethyl-3-[2-chlor-3-(trifluormethyl)benzyl]-1-(1-ethyl-1H-benzimidazol-5-yl)-2,4-dioxo-1,2,3,4-tetrahydropyrimidin-5-carboxylat

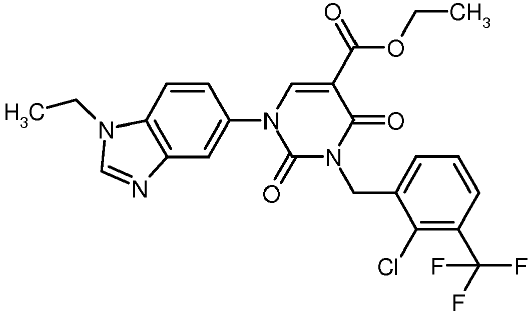

Darstellung der Titelverbindung erfolgte analog zu Beispiel 80. Ausgehend von 122.5 mg (0.37 mmol) Ethyl-1-(1-ethyl-1H-benzimidazol-5-yl)-2,4-dioxo-1,2,3,4-tetrahydropyrimidin-5-carboxylat aus Beispiel 46A und 112 mg (0.41 mmol) 1-(Brommethyl)-2-chlor-3-(trifluormethyl)benzol erhielt man nach zusätzlicher Reinigung mittels Flashchromatographie (Dichlormethan/Methanol 30:1) 52 mg (27 % d. Th.) der Titelverbindung.
LC-MS (Methode 1): Rₜ = 1.01 min; m/z = 521 (M+H)⁺.
¹H-NMR (400MHz, DMSO-d₆): δ [ppm] = 1.23 (t, 3H), 1.43 (t, 3H), 4.20 (q, 2H), 4.34 (q, 2H), 5.16 (s, 2H), 7.43 (dd, 1H), 7.53 (t, 1H), 7.61 (d, 1H), 7.76 (d, 1H), 7.81 (d, 1H), 7.88 (d, 1H), 8.40 (s, 1H), 8.50 (s, 1H).

### Beispiel 92

### Ethyl-3-[3-chlor-2-(trifluormethyl)benzyl]-1-[1-(cyclopropylmethyl)-1H-benzimidazol-5-yl]-2,4-dioxo-1,2,3,4-tetrahydropyrimidin-5-carboxylat

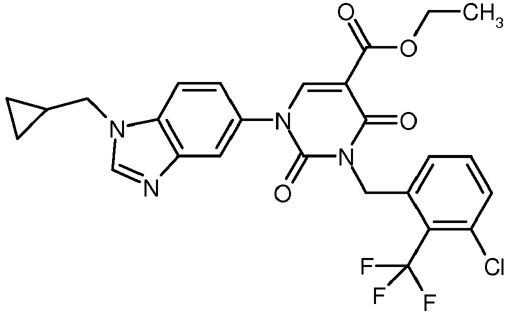

200 mg (0.56 mmol) Ethyl-l-[1-(cyclopropylmethyl)-1H-benzimidazol-5-yl]-2,4-dioxo-1,2,3,4-tetrahydropyrimidin-5-carboxylat aus Beispiel 56A wurden in 7.1 ml DMF vorgelegt. 156 mg (1.13 mmol) Kaliumcarbonat, 9 mg (0.05 mmol) Kaliumiodid und 261 mg (65 %-ige Reinheit, 0.62 mmol) 1-(Brommethyl)-3-chlor-2-(trifluormethyl)benzol wurde zugegeben und die Mischung 5 h auf 60°C erhitzt. Das abgekühlte Reaktionsgemisch wurde mit Wasser versetzt, zweimal mit Essigsäureethylester extrahiert, die vereinigten organischen Phasen mit gesättigter Natriumchlorid-Lösung gewaschen, über Magnesiumsulfat getrocknet und eingeengt. Der Rückstand wurde in Ethanol verrührt, der ausgefallene Feststoff abgesaugt und an der Hochvakuumpumpe getrocknet. Man erhielt 137 mg (44 % d. Th.) der Titelverbindung. Das Filtrat wurde eingeengt und der Rückstand mittels Flashchromatographie (Dichlormethan/Methanol 50:1) gereinigt. Damit konnten zusätzlich 56 mg der Titelverbindung isoliert werden (Gesamtausbeute 61 % d. Th.).
LC-MS (Methode 3): Rₜ = 1.29 min; m/z = 547 (M+H)⁺.
¹H-NMR (400MHz, DMSO-d₆): δ [ppm] = 0.40 - 0.48 (m, 2H), 0.51 - 0.58 (m, 2H), 1.23 (t, 3H), 1.27 - 1.37 (m, 1H), 4.14 - 4.24 (m, 4H), 5.23 (s, 2H), 7.36 (d, 1H), 7.42 (dd, 1H), 7.56 - 7.68 (m, 2H), 7.81 (d, 1H), 7.87 (d, 1H), 8.43 (s, 1H), 8.53 (s, 1H).

### Beispiel 93

### Ethyl-1-(1-isopropyl-1H-benzimidazol-5-yl)-3-[2-methyl-3-(trifluormethyl)benzyl]-2,4-dioxo-1,2,3,4-tetrahydropyrimidin-5-carboxylat

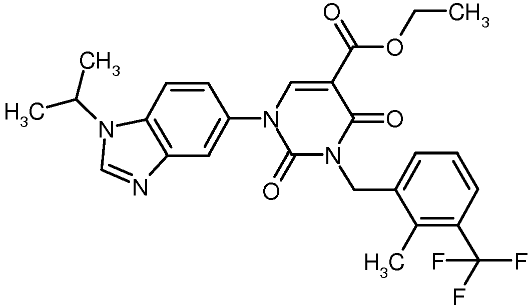

Darstellung der Titelverbindung erfolgte analog zu Beispiel 80. Ausgehend von 200 mg (0.58 mmol) Ethyl-1-(1-isopropyl-1H-benzimidazol-5-yl)-2,4-dioxo-1,2,3,4-tetrahydropyrimidin-5-carboxylat aus Beispiel 51A und 162 mg (0.64 mmol) 1-(Brommethyl)-2-methyl-3-(trifluormethyl)benzol erhielt man nach zusätzlicher Reinigung mittels Flashchromatographie (Dichlormethan/Methanol 50:1) 90 mg (29 % d. Th.) der Titelverbindung.
LC-MS (Methode 1): Rₜ = 1.04 min; m/z = 515 (M+H)⁺.
¹H-NMR (400MHz, DMSO-d₆): δ [ppm] = 1.22 (t, 3H), 1.56 (d, 6H), 2.46 (s, 3H), 4.19 (q, 2H), 4.82 (spt, 1H), 5.09 (s, 2H), 7.32 - 7.46 (m, 3H), 7.60 (d, 1H), 7.80 (d, 1H), 7.89 (d, 1H), 8.48 (d, 2H).

### Beispiel 94

### Ethyl-1-(1,3-dimethyl-2,2-dioxido-1,3-dihydro-2,1,3-benzothiadiazol-5-yl)-3-[2-methyl-3-(trifluormethyl)benzyl]-2,4-dioxo-1,2,3,4-tetrahydropyrimidin-5-carboxylat

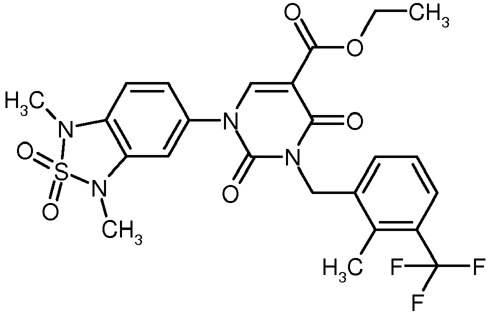

Darstellung und Reinigung der Titelverbindung erfolgten analog zu Beispiel 1. Die Reaktionsdauer betrug 2h. Ausgehend von 160 mg (0.42 mmol) Ethyl-1-(1,3-dimethyl-2,2-dioxido-1,3-dihydro-2,1,3-benzothiadiazol-5-yl)-2,4-dioxo-1,2,3,4-tetrahydropyrimidin-5-carboxylat aus Beispiel 87A und 117 mg (0.46 mmol) 1-(Brommethyl)-2-methyl-3-(trifluormethyl)benzol erhielt man 195 mg (84 % d. Th.) der Titelverbindung.
LC-MS (Methode 1): Rₜ = 1.10 min; m/z = 553 (M+H)⁺.
¹H-NMR (400MHz, DMSO-d₆): δ [ppm] = 1.23 (t, 3H), 2.46 (s, 3H), 3.26 (s, 3H), 3.30 (s, 3H), 4.20 (q, 2H), 5.08 (s, 2H), 7.15 (d, 1H), 7.23 (dd, 1H), 7.29 (d, 1H), 7.31 - 7.39 (m, 2H), 7.58 - 7.63 (m, 1H), 8.45 (s, 1H).

### Beispiel 95

### Ethyl-3-[3-chlor-2-(trifluormethyl)benzyl]-1-(1,3-dimethyl-2,2-dioxido-1,3-dihydro-2,1,3-benzothiadiazol-5-yl)-2,4-dioxo-1,2,3,4-tetrahydropyrimidin-5-carboxylat

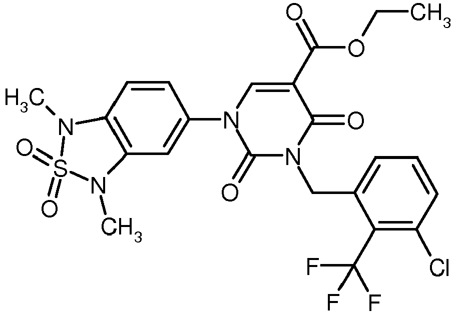

Darstellung und Reinigung der Titelverbindung erfolgten analog zu Beispiel 1. Die Reaktionsdauer betrug 2h. Ausgehend von 160 mg (0.42 mmol) Ethyl-1-(1,3-dimethyl-2,2-dioxido-1,3-dihydro-2,1,3-benzothiadiazol-5-yl)-2,4-dioxo-1,2,3,4-tetrahydropyrimidin-5-carboxylat aus Beispiel 87A und 194 mg (65 %-ige Reinheit, 0.46 mmol) 1-(Brommethyl)-3-chlor-2-(trifluormethyl)benzol (Herstellung: siehe WO 2004/52858, Seite 149, Beispiel 176) erhielt man nach zusätzlicher Reinigung mittels Flashchromatographie (Dichlormethan/Methanol 250:1) 120 mg (50 % d. Th.) der Titelverbindung.
LC-MS (Methode 1): Rₜ = 1.11 min; m/z = 573 (M+H)⁺.
¹H-NMR (400MHz, DMSO-d₆): δ [ppm] = 1.23 (t, 3H), 3.27 (s, 3H), 3.30 (s, 3H), 4.20 (q, 2H), 5.21 (br.s, 2H), 7.17 (d, 1H), 7.21 (dd, 1H), 7.26 (d, 1H), 7.32 (d, 1H), 7.56 - 7.67 (m, 2H), 8.49 (s, 1H).

### Beispiel 96

### Ethyl-3-[2-chlor-3-(trifluormethyl)benzyl]-1-(1,3-dimethyl-2,2-dioxido-1,3-dihydro-2,1,3-benzothiadiazol-5-yl)-2,4-dioxo-1,2,3,4-tetrahydropyrimidin-5-carboxylat

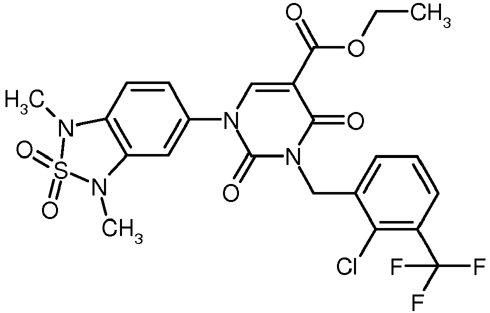

Darstellung und Reinigung der Titelverbindung erfolgten analog zu Beispiel 1. Ausgehend von 160 mg (0.42 mmol) Ethyl-1-(1,3-dimethyl-2,2-dioxido-1,3-dihydro-2,1,3-benzothiadiazol-5-yl)-2,4-dioxo-1,2,3,4-tetrahydropyrimidin-5-carboxylat aus Beispiel 87A und 126 mg (0.46 mmol) 1-(Brommethyl)-2-chlor-3-(trifluormethyl)benzol erhielt man 167 mg (69 % d. Th.) der Titelverbindung.
LC-MS (Methode 1): Rₜ = 1.11 min; m/z = 573 (M+H)⁺.
¹H-NMR (400MHz, DMSO-d₆): δ [ppm] = 1.23 (t, 3H), 3.27 (s, 3H), 3.31 (s, 3H), 4.20 (q, 2H), 5.15 (s, 2H), 7.16 (d, 1H), 7.22 (dd, 1H), 7.27 (d, 1H), 7.49 - 7.60 (m, 2H), 7.80 (d, 1H), 8.48 (s, 1H).

### Beispiel 97

### Ethyl-1-(1,3-dimethyl-2,2-dioxido-1,3-dihydro-2,1,3-benzothiadiazol-5-yl)-2,4-dioxo-3-[(R)-4-(trifluormethyl)-2,3-dihydro-1H-inden-1-yl]-1,2,3,4-tetrahydropyrimidin-5-carboxylat (R-Enantiomer)

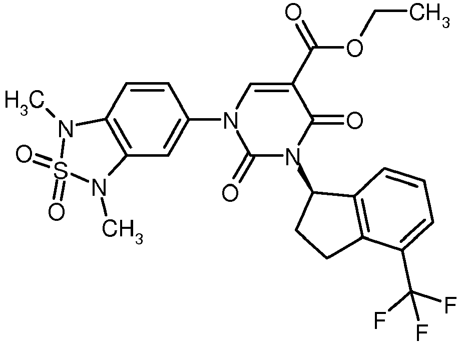

Darstellung und Reinigung der Titelverbindung erfolgten analog zu Beispiel 42 *(Methode A).* Ausgehend von 200 mg (0.52 mmol) Ethyl-1-(1,3-dimethyl-2,2-dioxido-1,3-dihydro-2,1,3-benzothiadiazol-5-yl)-2,4-dioxo-1,2,3,4-tetrahydropyrimidin-5-carboxylat aus Beispiel 87A und 127 mg (0.63 mmol (1S)-4-(Trifluormethyl)indan-1-ol aus Beispiel 14A erhielt man 149 mg (50 % d. Th.) der Titelverbindung.
LC-MS (Methode 1): Rₜ = 1.15 min; m/z = 565 (M+H)⁺.
¹H-NMR (400MHz, DMSO-d₆): δ [ppm] = 1.22 (t, 3H), 2.35 - 2.43 (m, 1H), 2.43 - 2.48 (m, 1H, teilweise verdeckt durch DMSO-Signal), 3.03 - 3.15 (m, 1H), 3.22 - 3.27 (m, 4H), 3.29 (s, 3H), 4.17 (q, 2H), 6.31 - 6.59 (m, 1H), 7.09 - 7.31 (m, 3H), 7.36 (t, 1H), 7.47 (d, 1H), 7.53 (d, 1H), 8.37 (s, 1H).

### Beispiel 98

### Ethyl-1-(1,3-benzothiazol-6-yl)-3-[2-methyl-3-(trifluormethyl)benzyl]-2,4-dioxo-1,2,3,4-tetrahydropyrimidin-5-carboxylat

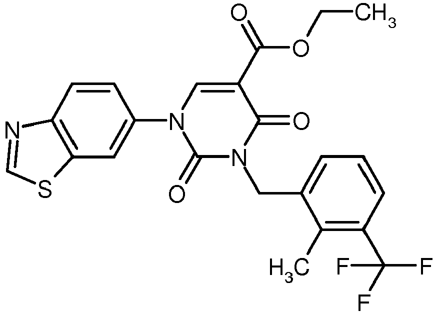

Darstellung und Reinigung der Titelverbindung erfolgten analog zu Beispiel 80. Ausgehend von 200 mg (0.63 mmol) Ethyl-1-(1,3-benzothiazol-6-yl)-2,4-dioxo-1,2,3,4-tetrahydropyrimidin-5-carboxylat aus Beispiel 88A und 175 mg (0.69 mmol) 1-(Brommethyl)-2-methyl-3-(trifluormethyl)benzol erhielt man 204 mg (65 % d. Th.) der Titelverbindung.
LC-MS (Methode 1): Rₜ = 1.08 min; m/z = 490 (M+H)⁺.
¹H-NMR (400MHz, DMSO-d₆): δ [ppm] = 1.23 (t, 3H), 2.46 (s, 3H), 4.20 (q, 2H), 5.10 (s, 2H), 7.36 (t, 1H), 7.42 (d, 1H), 7.60 (d, 1H), 7.73 (dd, 1H), 8.22 (d, 1H), 8.41 (d, 1H), 8.59 (s, 1H), 9.54 (s, 1H).

### Beispiel 99

### Ethyl-1-(4-methylchinolin-7-yl)-3-[2-methyl-3-(trifluormethyl)benzyl]-2,4-dioxo-1,2,3,4-tetrahydropyrimidin-5-carboxylat

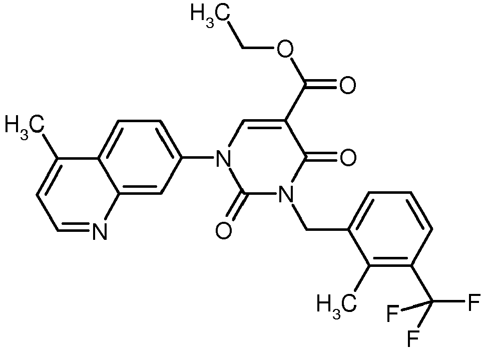

Darstellung und Reinigung der Titelverbindung erfolgten analog zu Beispiel 80. Ausgehend von 200 mg (0.61 mmol) Ethyl-1-(4-methylchinolin-7-yl)-2,4-dioxo-1,2,3,4-tetrahydropyrimidin-5-carboxylat aus Beispiel 93A und 171 mg (0.67 mmol) 1-(Brommethyl)-2-methyl-3-(trifluormethyl)benzol erhielt man 230 mg (75 % d. Th.) der Titelverbindung.

LC-MS (Methode 1): Rₜ = 1.13 min; m/z = 498 (M+H)⁺.
¹H-NMR (400MHz, DMSO-d₆): δ [ppm] = 1.24 (t, 3H), 2.47 (s, 3H), 2.74 (s, 3H), 4.21 (q, 2H), 5.11 (s, 2H), 7.33 - 7.39 (m, 1H), 7.43 - 7.47 (m, 1H), 7.48 - 7.51 (m, 1H), 7.59 - 7.63 (m, 1H), 7.77 - 7.81 (m, 1H), 8.22 - 8.27 (m, 2H), 8.62 (s, 1H), 8.85 (d, 1H).

### Beispiel 100

### Ethyl-1-(1-methyl-2-oxo-1,4-dihydro-2H-3,1-benzoxazin-6-yl)-3-[2-methyl-3-(trifluormethyl)benzyl]-2,4-dioxo-1,2,3,4-tetrahydropyrimidin-5-carboxylat

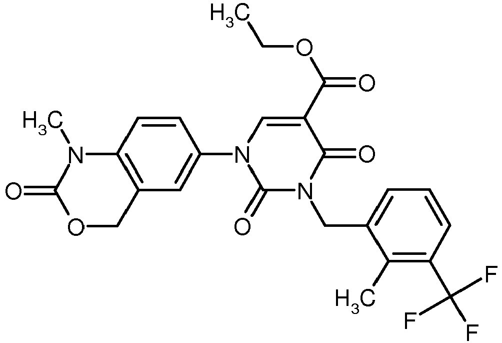

Darstellung und Reinigung der Titelverbindung erfolgten analog zu Beispiel 1. Die Reaktionszeit betrug ca. 16h. Ausgehend von 200 mg (0.57 mmol) Ethyl-1-(1-methyl-2-oxo-1,4-dihydro-2H-3,1-benzoxazin-6-yl)-2,4-dioxo-1,2,3,4-tetrahydropyrimidin-5-carboxylat aus Beispiel 92A und 161 mg (0.63 mmol) 1-(Brommethyl)-2-methyl-3-(trifluormethyl)benzol erhielt man 255 mg (85 % d. Th.) der Titelverbindung.
LC-MS (Methode 1): Rₜ = 1.07 min; m/z = 518 (M+H)⁺.
¹H-NMR (400MHz, DMSO-d₆): δ [ppm] = 1.23 (t, 3H), 2.46 (s, 3H), 3.30 (s, teilweise durch Wasser-Signal verdeckt), 4.20 (q, 2H), 5.07 (s, 2H), 5.30 (s, 2H), 7.24 (d, 1H), 7.30 - 7.41 (m, 2H), 7.47 (d, 1H), 7.54 - 7.62 (m, 2H), 8.44 (s, 1H).

### Beispiel 101

### Ethyl-1-(1-methyl-2-oxo-1,4-dihydro-2H-3,1-benzoxazin-6-yl)-2,4-dioxo-3-[(1R)-4-(trifluormethyl)-2,3-dihydro-1H-inden-1-yl]-1,2,3,4-tetrahydropyrimidin-5-carboxylat

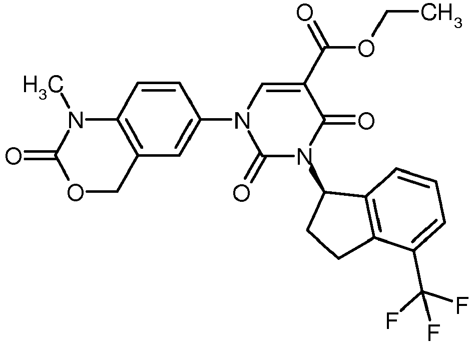

Darstellung und Reinigung der Titelverbindung erfolgten analog zu Beispiel 67. 200 mg (0.56 mmol) Ethyl-1-(1-methyl-2-oxo-1,4-dihydro-2H-3,1-benzoxazin-6-yl)-2,4-dioxo-1,2,3,4-tetrahydropyrimidin-5-carboxylat aus Beispiel 92A und 140 mg (0.69 mmol (1S)-4-(Trifluormethyl)indan-1-ol aus Beispiel 14A erhielt man nach Reinigung mittels HPLC (Methode 8) 160 mg (51 % d. Th.) der Titelverbindung.
LC-MS (Methode 1): Rₜ = 1.07 min; m/z = 530 (M+H)⁺.
¹H-NMR (400MHz, CDCl₃): δ [ppm] = 1.36 (t, 3H), 2.37 - 2.48 (m, 1H), 2.53 - 2.60 (m, 1H), 3.08 - 3.19 (m, 1H), 3.39 (s, 3H), 3.45 - 3.58 (m, 1H), 4.36 (q, 2H), 5.21 (s, 2H), 6.61 - 6.73 (m, 1H), 7.03 (d, 1H), 7.13 (s, 1H), 7.26 (d, 3H, teilweise verdeckt durch CHCl₃-Signal), 7.47 (d, 1H), 8.26 - 8.30 (m, 1H).
Spezifischer optischer Drehwert: α_{D}²⁰ = +124.4°, (Chloroform c = 0.360 g/100 ml).

### Beispiel 102

### Ethyl-3-[2-chlor-3-(trifluormethyl)benzyl]-1-(1-methyl-2-oxo-1,2,3,4-tetrahydrochinolin-6-yl)-2,4-dioxo-1,2,3,4-tetrahydropyrimidin-5-carboxylat

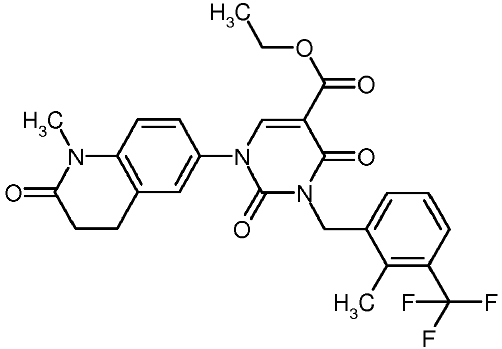

Darstellung und Reinigung der Titelverbindung erfolgten analog zu Beispiel 80. Ausgehend von 200 mg (0.58 mmol) Ethyl-1-(1-methyl-2-oxo-1,2,3,4-tetrahydrochinolin-6-yl)-2,4-dioxo-1,2,3,4-tetrahydropyrimidin-5-carboxylat aus Beispiel 91A und 162 mg (0.64 mmol) 1-(Brommethyl)-2-methyl-3-(trifluormethyl)benzol erhielt man 267 mg (89 % d. Th.) der Titelverbindung.
LC-MS (Methode 1): Rₜ = 1.06 min; m/z = 516 (M+H)⁺.
¹H-NMR (400MHz, DMSO-d₆): δ [ppm] = 1.23 (t, 3H), 2.46 (s, 3H), 2.58 (t, 2H), 2.92 (t, 2H), 3.28 (s, 3H), 4.19 (q, 2H), 5.08 (s, 2H), 7.22 (d, 1H), 7.31 - 7.39 (m, 2H), 7.40 - 7.46 (m, 2H), 7.60 (d, 1H), 8.41 (s, 1H).

### Beispiel 103

### Ethyl-1-(1-methyl-2-oxo-1,2,3,4-tetrahydrochinolin-6-yl)-2,4-dioxo-3-[(1R)-4-(trifluormethyl)-2,3-dihydro-1H-inden-1-yl]-1,2,3,4-tetrahydropyrimidin-5-carboxylat (R-Enantiomer)

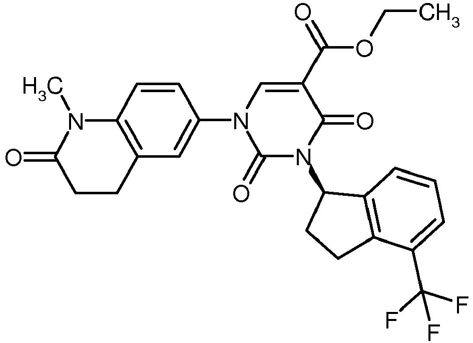

200 mg (0.58 mmol) Ethyl-1-(1-methyl-2-oxo-1,2,3,4-tetrahydrochinolin-6-yl)-2,4-dioxo-1,2,3,4-tetrahydropyrimidin-5-carboxylat aus Beispiel 91A und 475 mg (1.81 mmol) Triphenylphosphin wurden unter Argon in THF/DMF 1:1 (7.6 ml) vorgelegt. 235 mg (1.16 mmol) Diisopropylazodicarboxylat wurde zugetropft und anschließend 141 mg (0.69 mmol (1S)-4-(Trifluormethyl)indan-1-ol aus Beispiel 14A zugegeben. Die Reaktionsmischung wurde 16 h bei RT gerührt. Zur Aufarbeitung wurde der Ansatz mit IM Salzsäure versetzt, mit Essigsäureethylester verdünnt und Phasen getrennt. Die organische Phase wurde nacheinander zweimal mit IM Salzsäure, einmal mit gesättigter Natriumchlorid-Lösung gewaschen, über Magnesiumsulfat getrocknet, abfiltriert und eingeengt. Der Rückstand wurde mittels präparativer HPLC (Methode 8) gereinigt. Man erhielt 125 mg (40 % d. Th.) der Titelverbindung.
LC-MS (Methode 1): Rₜ = 1.09 min; m/z = 528 (M+H)⁺.
¹H-NMR (400MHz, CDCl₃): δ [ppm] = 1.36 (t, 3H), 2.38 - 2.50 (m, 1H), 2.53 - 2.61 (m, 1H, teilweise verdeckt durch DMSO-Signal), 2.67 (t, 2H), 2.94 (t, 2H), 3.08 - 3.19 (m, 1H), 3.36 (s, 3H), 3.46 - 3.58 (m, 1H), 4.36 (q, 2H), 6.62 - 6.74 (m, 1H), 7.05 (d, 1H), 7.13 (s, 1H), 7.18 - 7.23 (m, 1H), 7.24 - 7.30 (m, 2H), 7.47 (d, 1H), 8.29 (s, 1H).
Spezifischer optischer Drehwert: α_{D}²⁰ = +128.5°, (Chloroform, c = 0.415 g/100 ml).

### Beispiel 104

### Ethyl-1-(6-fluor-1,3-dimethyl-2-oxo-2,3-dihydro-1H-benzimidazol-5-yl)-2,4-dioxo-3-[(1R)-4-(trifluormethyl)-2,3-dihydro-1H-inden-1-yl]-1,2,3,4-tetrahydropyrimidin-5-carboxylat (R-Enantiomer)

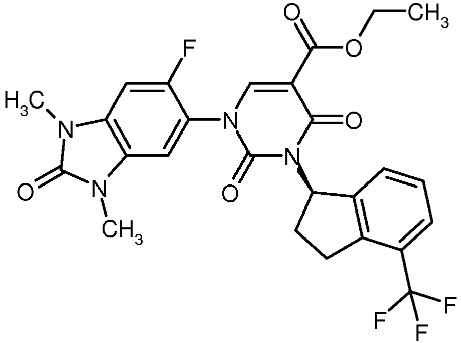

200 mg (0.55 mmol) Ethyl-1-(6-fluor-1,3-dimethyl-2-oxo-2,3-dihydro-1H-benzimidazol-5-yl)-2,4-dioxo-1,2,3,4-tetrahydropyrimidin-5-carboxylat aus Beispiel 95A und 434 mg (1.66 mmol) Triphenylphosphin wurden unter Argon in THF/DMF 1:1 (7.3 ml) vorgelegt und auf -30°C abgekühlt. 218 µl (1.10 mmol) Diisopropylazodicarboxylat, dann eine Lösung von 134 mg (0.66 mmol (1S)-4-(Trifluormethyl)indan-1-ol aus Beispiel 14A in 3 ml THF wurden dazu getropft. Die Reaktionsmischung wurde auf RT erwärmt und 30 min bei RT gerührt. Zur Aufarbeitung wurde die Reaktionsmischung auf 0°C abgekühlt, mit 5 ml IM Salzsäure versetzt, dann bei RT mit Essigsäureethylester extrahiert. Die organische Phase wurde nacheinander zweimal mit IM Salzsäure, einmal mit gesättigter Natriumchlorid-Lösung gewaschen, über Magnesiumsulfat getrocknet und eingeengt. Der Rückstand wurde mit Ethanol ausgerührt, der ausgefallene Feststoff abgesaugt und verworfen. Das Filtrat wurde am Rotationsverdampfer eingeengt, in wenig Dichlormethan gelöst und mittels Flashchromatographie (Eluens Dichlormethan/Methanol 120:1→ 20:1) gereinigt. Das erhaltene Produkt wurde im HV getrocknet, dann in 10 ml Cyclohexan / Essigsäureethylester 1:1 verrührt. Der Feststoff wurde abfiltriert und im HV getrocknet. Man erhielt 146 mg (47 % d. Th.) der Titelverbindung.
LC-MS (Methode 1): Rₜ = 1.10 min; m/z = 547 (M+H)⁺.
¹H-NMR (400MHz, DMSO-d₆): δ [ppm] = 1.22 (t, 3H), 2.30 - 2.42 (m, 1H), 2.52 - 2.53 (m, 1H, teilweise verdeckt durch DMSO-Signal), 3.04 - 3.15 (m, 1H), 3.22 - 3.30 (m, 1H), 3.32 (s, 3H), 3.35 (s, 3H), 4.19 (q, 2H), 6.37 - 6.57 (m, 1H), 7.33 - 7.50 (m, 4H), 7.54 (d, 1H), 8.48 (s, 1H).

### Beispiel 105

### Ethyl-3-[(3-chlor-4-methyl-2-thienyl)methyl]-1-(1,3-dimethyl-2-oxo-2,3-dihydro-1H-benzimidazol-5-yl)-2,4-dioxo-1,2,3,4-tetrahydropyrimidin-5-carboxylat

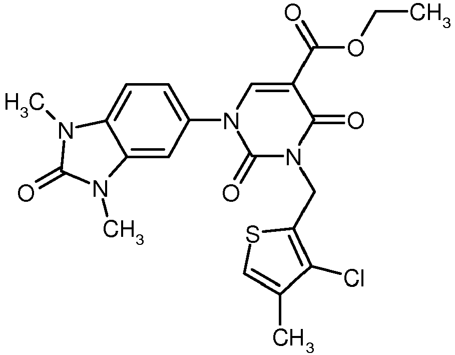

45 µl (0.23 mmol) Diisopropylazodicarboxylat wurden zu einer unter Argon vorgelegten Lösung von 33 mg (0.20 mmol) (3-Chlor-4-methyl-2-thienyl)methanol aus Beispiel 96A und 74 mg (0.28 mmol) in 2 ml wasserfreiem THF bei RT zugetropft. Nach 5 min wurden 65 mg (0.18 mmol) Ethyl-1-(1,3-dimethyl-2-oxo-2,3-dihydro-1H-benzimidazol-5-yl)-2,4-dioxo-1,2,3,4-tetrahydropyrimidin-5-carboxylat aus Beispiel 2A zugegeben und die Reaktionsmischung über Nacht bei RT gerührt. Zur Aufarbeitung wurden 3 Tropfen IN Salzsäure zugegeben und die gesamte Reaktionsmischung mittels präparativen HPLC (Methode 8) getrennt. Die produkthaltigen Fraktionen wurden am Rotationsverdampfer eingeengt und der Rückstand in Diethylether verrührt. Der Feststoff wurde abgesaugt und im Hochvakuum getrocknet. Man erhielt 26 mg (26 % d. Th.) der Titelverbindung.
LC-MS (Methode 1): Rₜ = 1.00 min; m/z = 489 (M+H)⁺.
¹H-NMR (400MHz, DMSO-d₆): δ [ppm] = 1.23 (t, 3H), 2.12 (s., 3H), 3.30 (s., 3H, teilweise verdeckt durch Wasser-Signal), 3.37 (s, 3H), 4.19 (q, 2H), 5.19 (s., 2H), 7.14 - 7.23 (m, 1H), 7.24 - 7.32 (m, 2H), 7.38 (s, 1H), 8.36 (s, 1H).

### Beispiel 106

### Ethyl-3-(4,6-difluor-2,3-dihydro-1H-inden-1-yl)-1-(1,3-dimethyl-2-oxo-2,3-dihydro-1H-benzimidazol-5-yl)-2,4-dioxo-1,2,3,4-tetrahydropyrimidin-5-carboxylat (Racemat)

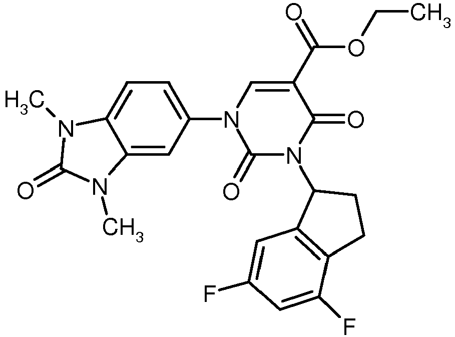

229 µl (1.16 mmol) Diisopropylazodicarboxylat wurden zu einer unter Argon bei -40°C vorgelegten Lösung von 200 mg (0.58 mmol) Ethyl-1-(1,3-dimethyl-2-oxo-2,3-dihydro-1H-benzimidazol-5-yl)-2,4-dioxo-1,2,3,4-tetrahydropyrimidin-5-carboxylat aus Beispiel 2A und 457 mg (1.74 mmol) Triphenylphosphin in 16 ml THF / DMF 1:1 zugetropft. 128 mg (1.16 mmol) 4,6-Difluorindan-1-ol aus Beispiel 97A wurden zugegeben. Die Reaktionsmischung wurde auf RT erwärmt und über Nacht weiter gerührt. Zur Aufarbeitung wurden unter Eiskühlung 5 ml IN Salzsäure zugegeben, die Mischung 15 min weiter gerührt dann mit Essigsäureethylester extrahiert. Die organische Phase wurde zweimal mit IN Salzsäure, zweimal mit einer gesättigten Natriumhydrogencarbonatlösung, dann mit einer gesättigten Natriumchloridlösung gewaschen, dann über Natriumsulfat getrocknet und am Rotationsverdampfer eingeengt. Der Rückstand wurde mittels präparativen HPLC (Methode 8) gereinigt. Man erhielt 178 mg (61 % d. Th.) der Titelverbindung.
LC-MS (Methode 1): Rₜ = 1.01 min; m/z = 497 (M+H)⁺.
¹H-NMR (400MHz, DMSO-d₆): δ [ppm] = 1.22 (t, 3H), 2.35 - 2.48 (m, 2H), 2.84 - 2.96 (m, 1H), 3.02 - 3.16 (m, 1H), 3.31 (s, 3H), 3.37 (s, 3H), 4.18 (q, 2H), 6.25 - 6.55 (m, 1H), 6.93 - 7.08 (m, 2H), 7.13 - 7.30 (m, 2H), 7.31 - 7.45 (m, 1H), 8.33 (s, 1H).

### Beispiel 107

### Ethyl-1-(1,3-dimethyl-2-oxo-2,3-dihydro-1H-benzimidazol-5-yl)-3-(6-methyl-2,3-dihydro-1H-inden-1-yl)-2,4-dioxo-1,2,3,4-tetrahydropyrimidin-5-carboxylat (Racemat)

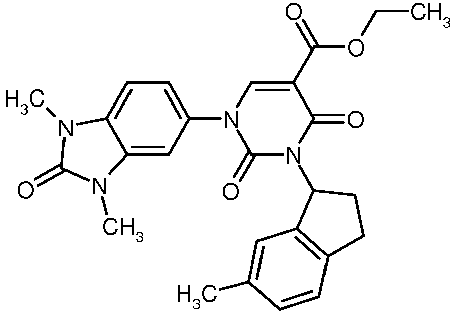

Darstellung und Reinigung der Titelverbindung erfolgten analog zu Beispiel 106. Ausgehend von 200 mg (0.58 mmol) Ethyl-1-(1,3-dimethyl-2-oxo-2,3-dihydro-1H-benzimidazol-5-yl)-2,4-dioxo-1,2,3,4-tetrahydropyrimidin-5-carboxylat aus Beispiel 2A und 112 mg (0.75 mmol) 6-Methylindan-1-ol aus Beispiel 100A erhielt man 130 mg (47 % d. Th.) der Titelverbindung.
LC-MS (Methode 1): Rₜ = 1.02 min; m/z = 475 (M+H)⁺.
¹H-NMR (400MHz, DMSO-d₆): δ [ppm] = 1.21 (t, 3H), 2.25 (s, 3H), 2.31 - 2.43 (m, 2H), 2.79 - 2.91 (m, 1H), 3.04 - 3.18 (m, 1H), 3.31 (s, 3H), 3.37 (s, 3H), 4.17 (q, 2H), 6.24 - 6.51 (m, 1H), 6.93 - 7.01 (m, 2H), 7.09 (d, 1H), 7.14 - 7.29 (m, 2H), 7.31 - 7.47 (m, 1H), 8.31 (s, 1H).

### Beispiel 108

### Ethyl-3-(4,6-difluor-2,3-dihydro-1H-inden-1-yl)-1-(3-methyl-2-oxo-2,3-dihydro-1,3-benzothiazol-6-yl)-2,4-dioxo-1,2,3,4-tetrahydropyrimidin-5-carboxylat (Racemat)

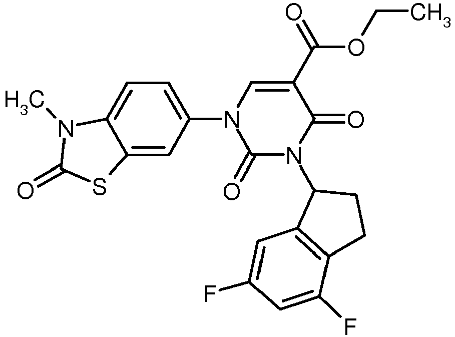

Darstellung der Titelverbindung erfolgte analog zu Beispiel 103 aber mit einer Reaktionszeit von 1 h, ausgehend von 200 mg (0.57 mmol) Ethyl-1-(3-methyl-2-oxo-2,3-dihydro-1,3-benzothiazol-6-yl)-2,4-dioxo-1,2,3,4-tetrahydropyrimidin-5-carboxylat aus Beispiel 31A und 127 mg (0.74 mmol) 4,6-Difluorindan-1-ol aus Beispiel 97A. Das Produkt wurde per präparativer HPLC (Methode 7) gereinigt. Man erhielt 173 mg (57 % d. Th.) der Titelverbindung.
LC-MS (Methode 1): Rₜ = 1.10 min; m/z = 500 (M+H)⁺.
¹H-NMR (400MHz, DMSO-d₆): δ [ppm] = 1.23 (t, 3H), 2.35 - 2.48 (m, 2H), 2.84 - 2.96 (m, 1H), 3.00 - 3.15 (m, 1H), 3.44 (s, 3H), 4.18 (q, 2H), 6.27 - 6.52 (m, 1H), 6.93 - 7.07 (m, 2H), 7.39 - 7.65 (m, 2H), 7.76 - 7.92 (m, 1H), 8.40 (s, 1H).

### Beispiel 109

### Ethyl-3-(6-methyl-2,3-dihydro-1H-inden-1-yl)-1-(3-methyl-2-oxo-2,3-dihydro-1,3-benzothiazol-6-yl)-2,4-dioxo-1,2,3,4-tetrahydropyrimidin-5-carboxylat (Racemat)

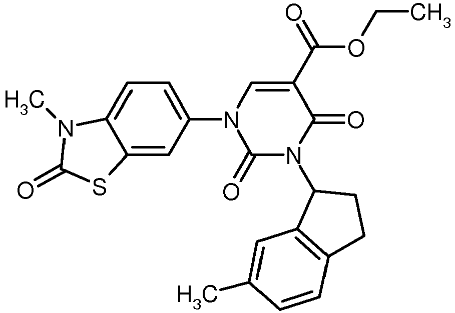

Darstellung und Reinigung der Titelverbindung erfolgten analog zu Beispiel 108. Ausgehend von 200 mg (0.57 mmol) Ethyl-1-(3-methyl-2-oxo-2,3-dihydro-1,3-benzothiazol-6-yl)-2,4-dioxo-1,2,3,4-tetrahydropyrimidin-5-carboxylat aus Beispiel 31A und 111 mg (0.74 mmol) 6-Methylindan-1-ol aus Beispiel 100A erhielt man 131 mg (47 % d. Th.) der Titelverbindung.
LC-MS (Methode 1): Rₜ = 1.11 min; m/z = 478 (M+H)⁺.
¹H-NMR (400MHz, DMSO-d₆): δ [ppm] = 1.22 (t, 3H), 2.24 (s, 3H), 2.31 - 2.43 (m, 2H), 2.79 - 2.91 (m, 1H), 3.01 - 3.17 (m, 1H), 3.44 (s, 3H), 4.17 (q, 2H), 6.21 - 6.51 (m, 1H), 6.91 - 7.02 (m, 2H), 7.09 (d, 1H), 7.42 (d, 1H), 7.48 - 7.63 (m, 1H), 7.77 - 7.92 (m, 1H), 8.38 (s, 1H).

### Beispiel 110

### Ethyl-1-(1,3-dimethyl-2-oxo-2,3-dihydro-1H-benzimidazol-5-yl)-3-[6-fluor-4-(trifluormethyl)-2,3-dihydro-1H-inden-1-yl]-2,4-dioxo-1,2,3,4-tetrahydropyrimidin-5-carboxylat (Racemat)

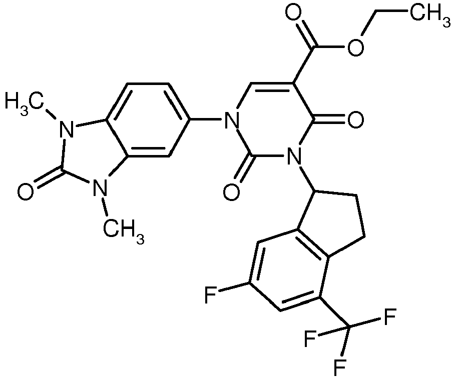

Darstellung und Reinigung der Titelverbindung erfolgten analog zu Beispiel 108. Ausgehend von 60 mg (0.17 mmol) Ethyl-1-(1,3-dimethyl-2-oxo-2,3-dihydro-1H-benzimidazol-5-yl)-2,4-dioxo-1,2,3,4-tetrahydropyrimidin-5-carboxylat aus Beispiel 2A und 50 mg (0.22 mmol) 6-Fluor-4-(trifluormethyl)indan-1-ol aus Beispiel 98A erhielt man 68 mg (71 % d. Th.) der Titelverbindung.
LC-MS (Methode 4): Rₜ = 2.38 min; m/z = 547 (M+H)⁺.
¹H-NMR (400MHz, CD₂Cl₂): δ [ppm] = 1.23 (t, 3H), 2.33 - 2.46 (m, 1H), 2.48 - 2.60 (m, 1H), 2.95 - 3.07 (m, 1H), 3.26 - 3.40 (m, 7H), 4.21 (q, 2H), 6.47 - 6.57 (m, 1H), 6.86 (s, 1H), 6.92 - 7.01 (m, 3H), 7.08 - 7.17 (m, 1H), 8.24 (s, 1H).

### Beispiel 111

### tert.-Butyl-6-[5-(ethoxycarbonyl)-2,4-dioxo-3-[(1R)-4-(trifluormethyl)-2,3-dihydro-1H-inden-1-yl]-3,4-dihydropyrimidin-1(2H)-yl]-3-methyl-2-oxo-2,3-dihydro-1H-benzimidazol-1-carboxylat (R-Enantiomer)

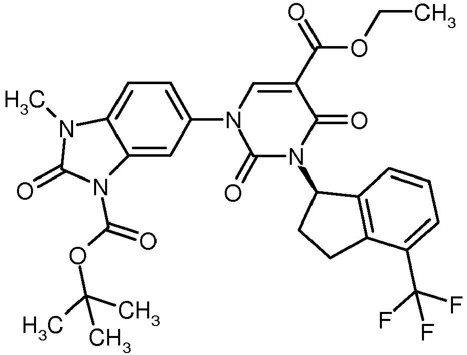

Darstellung und Reinigung der Titelverbindung erfolgten analog zu Beispiel 108 mit anfänglicher Eisbadkühlung. Ausgehend von 2.50 g (5.80 mmol) tert.-Butyl-6-[5-(ethoxycarbonyl)-2,4-dioxo-3,4-dihydropyrimidin-1(2H)-yl]-3-methyl-2-oxo-2,3-dihydro-1H-benzimidazol-1-carboxylat aus Beispiel 101A und 1.29 g (6.39 mmol) 4-(Trifluormethyl)indan-1-ol (S-Enantiomer) aus Beispiel 14A erhielt man 2.29 g (61 % d. Th.) der Titelverbindung.
LC-MS (Methode 1): Rₜ = 1.24 min; m/z = 615 (M+H)⁺.
¹H-NMR (400MHz, DMSO-d₆): δ [ppm] = 1.21 (t, 3H), 1.56 (s, 9H), 2.35 - 2.43 (m, 1H), 2.43 - 2.48 (m, 1H), 3.02 - 3.14 (m, 1H), 3.21 - 3.30 (m, 1H), 3.32 (br.s, 3H), 4.18 (q, 2H), 6.33 - 6.59 (m, 1H), 7.26 - 7.45 (m, 3H), 7.46 - 7.58 (m, 2H), 7.77 - 7.96 (m, 1H), 8.32 (s, 1H).

### Beispiel 112

### Ethyl-1-(1-Methyl-2-oxo-2,3-dihydro-1H-benzimidazol-5-yl)-2,4-dioxo-3-[4-(trifluormethyl)-2,3-dihydro-1H-inden-1-yl]-1,2,3,4-tetrahydropyrimidin-5-carboxylat (R-Enantiomer)

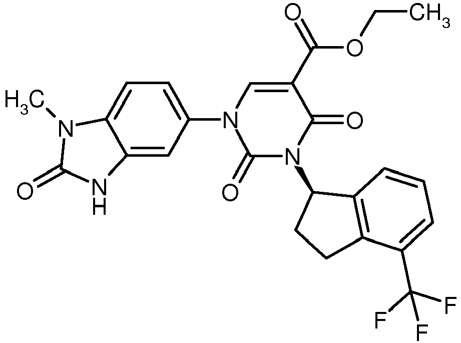

2.29 g (3.73 mmol) der Verbindung aus Beispiel 111 wurden 1 h in 50 ml Dichlormethan und 50 ml Trifluoressigsäure bei RT gerührt. Die Reaktionsmischung wurde am Rotationsverdampfer zur Trockne eingeengt. Der Rückstand wurde mit Essigsäureethylester und einer IM Natriumcarbonatlösung versetzt. Die organische Phase wurde getrennt, mit einer gesättigten Natriumchloridlösung gewaschen, über Natriumsulfat getrocknet und am Rotationsverdampfer eingeengt. Der Rückstand wurde im HV getrocknet. Man erhielt 1.66 g (84 % d. Th) der Titelverbindung.
LC-MS (Methode 1): Rₜ = 1.03 min; m/z = 515 (M+H)⁺.
¹H-NMR (400MHz, DMSO-d₆): δ [ppm]= 1.22 (t, 3H), 2.34 - 2.55 (m, 2H), 3.01 - 3.15 (m, 1H), 3.21 - 3.33 (m, 1H), 3.30 (s, 3H), 4.17 (q, 2H), 6.46 (br. m., 1H), 7.06 - 7.23 (m, 2H), 7.36 (t, 1H), 7.45 - 7.55 (m, 2H), 8.31 (s, 1H), 11.12 (br.s, 1H).

### Beispiel 113

### Ethyl-1-(3-ethyl-1-methyl-2-oxo-2,3-dihydro-1H-benzimidazol-5-yl)-2,4-dioxo-3-[(1R)-4-(trifluormethyl)-2,3-dihydro-1H-inden-1-yl]-1,2,3,4-tetrahydropyrimidin-5-carboxylat (R-Enantiomer)

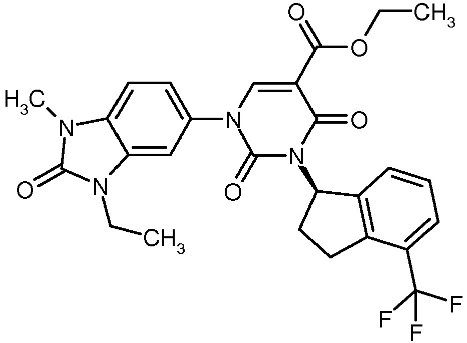

100 mg (0.19 mmol) Ethyl-1-(1-methyl-2-oxo-2,3-dihydro-1H-benzimidazol-5-yl)-2,4-dioxo-3-[4-(trifluormethyl)-2,3-dihydro-1H-inden-l-yl]-1,2,3,4-tetrahydropyrimidin-5-carboxylat (R-Enantiomer) aus Beispiel 112 wurde in DMF (3 ml) vorgelegt. 36 mg (0.23 mmol) Iodethan und 126 mg (0.38 mmol) Cäsiumcarbonat wurden zugegeben. Die Reaktionsmischung ließ man 1 h bei 60°C rühren. Die auf RT abgekühlte Reaktionsmischung wurde filtriert und das Filtrat per präparativer HPLC (Methode 7) gereinigt. Man erhielt 77 mg (72 % d. Th.) der Titelverbindung.
LC-MS (Methode 4): Rₜ = 2.40 min; m/z = 543 (M+H)⁺.
¹H-NMR (400MHz, CD₂Cl₂): δ [ppm] = 1.22 (t, 6H), 2.31 - 2.45 (m, 1H), 2.45 - 2.56 (m, 1H), 2.99 - 3.12 (m, 1H), 3.32 (s, 3H), 3.35 - 3.43 (m, 1H), 3.82 (q, 2H), 4.20 (q, 2H), 6.48 - 6.59 (m, 1H), 6.88 (s, 1H), 6.91 - 6.98 (m, 2H), 7.17 - 7.29 (m, 2H), 7.41 (d, 1H), 8.24 (s, 1H).

### Beispiel 114

### Ethyl-1-(3-isopropyl-1-methyl-2-oxo-2,3-dihydro-1H-benzimidazol-5-yl)-2,4-dioxo-3-[(1R)-4-(trifluormethyl)-2,3-dihydro-1H-inden-1-yl]-1,2,3,4-tetrahydropyrimidin-5-carboxylat (R-Enantiomer)

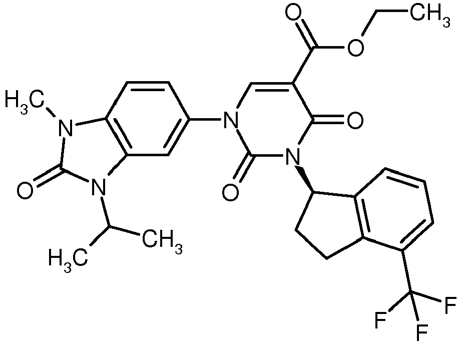

Darstellung und Reinigung der Titelverbindung erfolgten analog zu Beispiel 113. Ausgehend von 200 mg (0.30 mmol) Ethyl-1-(1-methyl-2-oxo-2,3-dihydro-1H-benzimidazol-5-yl)-2,4-dioxo-3-[4-(trifluormethyl)-2,3-dihydro-1H-inden-1-yl]-1,2,3,4-tetrahydropyrimidin-5-carboxylat (R-Enantiomer) aus Beispiel 112 und 79 mg (0.46 mmol) 2-Iodpropan erhielt man 125 mg (57 % d. Th.) der Titelverbindung.
LC-MS (Methode 1): Rₜ = 1.17 min; m/z = 557 (M+H)⁺.
¹H-NMR (400MHz, CD₂Cl₂): δ [ppm] = 1.23 (t, 3H), 1.40 - 1.44 (m, 6H), 2.31 - 2.43 (m, 1H), 2.45 - 2.57 (m, 1H), 3.00 - 3.12 (m, 1H), 3.30 (s, 3H), 3.34 - 3.46 (m, 1H), 4.20 (q, 2H), 4.49 - 4.59 (m, 1H), 6.47 - 6.60 (m, 1H), 6.93 (s, 3H), 7.17 - 7.28 (m, 2H), 7.41 (d, 1H), 8.23 (s, 1H).

### Beispiel 115

### Ethyl-1-[1-methyl-2-oxo-3-(3,3,3-trifluor-2-hydroxypropyl)-2,3-dihydro-1H-benzimidazol-5-yl]-2,4-dioxo-3-[(1R)-4-(trifluormethyl)-2,3-dihydro-1H-inden-1-yl]-1,2,3,4-tetrahydropyrimidin-5-carboxylat (Diastereomerengemisch)

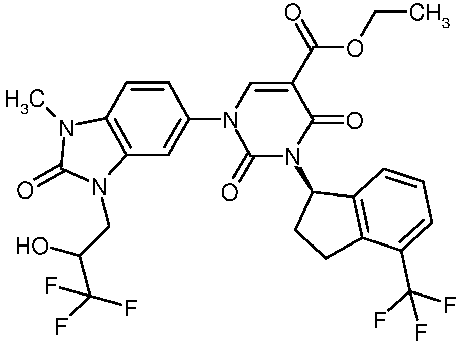

Darstellung und Reinigung der Titelverbindung erfolgten analog zu Beispiel 113 mit einer Reaktionszeit von 16h. Ausgehend von 250 mg (0.48 mmol) Ethyl-1-(1-methyl-2-oxo-2,3-dihydro-1H-benzimidazol-5-yl)-2,4-dioxo-3-[4-(trifluormethyl)-2,3-dihydro-1H-inden-1-yl]-1,2,3,4-tetrahydropyrimidin-5-carboxylat (R-Enantiomer) aus Beispiel 112 und 112 mg (0.58 mmol) 3-Brom-1,1,1-trifluorpropan-2-ol (Racemat) erhielt man 186 mg (57 % d. Th.) der Titelverbindung.
LC-MS (Methode 1): Rₜ = 1.10 min; m/z = 627 (M+H)⁺.
¹H-NMR (400MHz, CD₂Cl₂): δ [ppm] = 1.23 (t, 3H), 2.28 - 2.43 (m, 1H), 2.44 - 2.57 (m, 1H), 2.98 - 3.12 (m, 1H), 3.33 - 3.44 (m, 4H), 4.00 - 4.10 (m, 1H), 4.11 - 4.24 (m, 3H), 4.25 - 4.47 (m, 2H), 6.47 - 6.60 (m, 1H), 6.94 - 7.06 (m, 3H), 7.17 - 7.29 (m, 2H), 7.41 (d, 1H), 8.21 (s, 1H).

### Beispiel 116

### Ethyl-1-[1-methyl-2-oxo-3-(3,3,3-trifluorpropyl)-2,3-dihydro-1H-benzimidazol-5-yl]-2,4-dioxo-3- [(1R)-4-(trifluormethyl)-2,3-dihydro-1H-inden-1-yl]-1,2,3,4-tetrahydropyrimidin-5-carboxylat (R-Enantiomer)

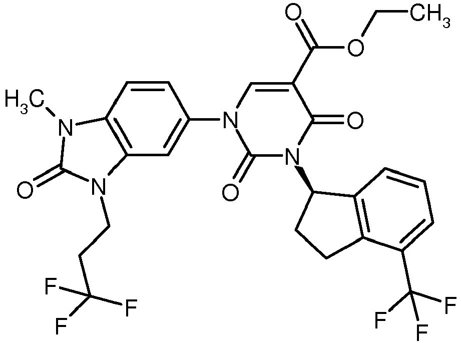

250 mg (0.48 mmol) Ethyl-1-(1-methyl-2-oxo-2,3-dihydro-1H-benzimidazol-5-yl)-2,4-dioxo-3-[4-(trifluormethyl)-2,3-dihydro-1H-inden-1-yl]-1,2,3,4-tetrahydropyrimidin-5-carboxylat (R-Enantiomer) aus Beispiel 112, 317 mg (0.97 mmol) Cäsiumcarbonat, 2 mg (12 µmol) Kaliumiodid und 103 mg (0.58 mmol) 3-Brom-1,1,1-trifluorpropan wurden in 7.5 ml DMF bei 60°C gerührt. Da die Umsetzung nach 16 h unzureichend war, wurden nach 16 h und noch mal nach 40 h zusätzlich je 1 eq. Cäsiumcarbonat und 3-Brom-1,1,1-trifluorpropan hinzugefügt und die Mischung über Nacht bei 60°C gerührt. Anschließend wurde die auf RT abgekühlte Reaktionsmischung mit Essigsäureethylester verdünnt und zweimal mit 1 N Salzsäure gewaschen. Die organische Phase wurde über Natriumsulfat getrocknet und das Lösungsmittel am Rotationsverdampfer entfernt. Der Rückstand wurde in MTBE verrührt und der entstandene Feststoff abgesaugt. Der Feststoff ensprach unreagiertem Edukt (88 mg). Das Filtrat wurde eingeengt und der Rückstand per präparativer HPLC (Methode 7) gereinigt. Man erhielt 106 mg (35 % d. Th.) der Titelverbindung.
LC-MS (Methode 5): Rt = 1.19 min; m/z = 611 (M+H)+.
¹H-NMR (400MHz, DMSO-d₆): δ [ppm] = 1.22 (t, 3H), 2.36 - 2.44 (m, 1H), 2.44 - 2.48 (m, 1H), 2.69 - 2.82 (m, 2H), 3.03 - 3.15 (m, 1H), 3.23 - 3.30 (m, 1H), 3.37 (s, 3H), 4.10 (t, 2H), 4.18 (q, 2H), 6.36 - 6.55 (m, 1H), 7.17 - 7.32 (m, 2H), 7.37 (t, 1H), 7.45 - 7.51 (m, 2H), 7.51 - 7.56 (m, 1H), 8.35 (s, 1H).

### Beispiel 117

### Ethyl-1-(3-cyclopropyl-1-methyl-2-oxo-2,3-dihydro-1H-benzimidazol-5-yl)-2,4-dioxo-3-[(1R)-4-(trifluormethyl)-2,3-dihydro-1H-inden-1-yl]-1,2,3,4-tetrahydropyrimidin-5-carboxylat (R-Enantiomer)

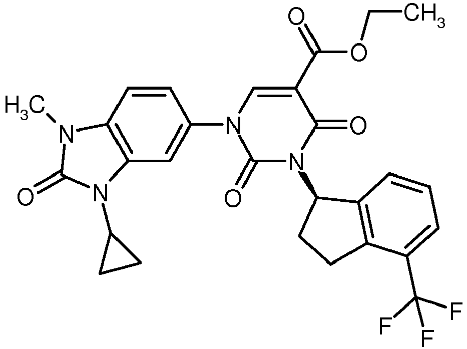

Eine Mischung aus 100 mg (0.19 mmol) der Verbindung aus Beispiel 112, 33.4 mg (0.39 mmol) Cyclopropylboronsäure, 24 mg (0.19 mmol) Kupfer(I)acetat, 41.2 mg (0.39 mmol) Natriumcarbonat, 31 µl (0.39 mmol) Pyridin in 2 ml Toluol wurde 6 h bei 70°C verrührt. Anschließend wurde die auf RT abgekühlte Reaktionsmischung mit Essigsäureethylester verdünnt und zweimal mit 1 N Salzsäure gewaschen. Die organische Phase wurde über Natriumsulfat getrocknet und das Lösungsmittel am Rotationsverdampfer entfernt. Der Rückstand wurde durch präparative HPLC (Methode 8) gereinigt. Man erhielt 90 mg (84 % d. Th.) der Titelverbindung.
LC-MS (Methode 1): Rₜ = 1.09 min; m/z = 555 (M+H)⁺.
¹H-NMR (400MHz, CD₂Cl₂): δ [ppm] = 0.92 - 1.01 (m, 2H), 1.04 - 1.11 (m, 2H), 1.31 (t, 3H), 2.39 - 2.51 (m, 1H), 2.53 - 2.65 (m, 1H), 2.86 (br. spt, 1H), 3.08 - 3.21 (m, 1H), 3.36 (s, 3H), 3.42 - 3.55 (m, 1H), 4.29 (q, 2H), 6.55 - 6.68 (m, 1H), 6.96 - 7.05 (m, 2H), 7.11 - 7.16 (m, 1H), 7.27 - 7.32 (m, 1H), 7.33 (d, 1H), 7.49 (d, 1H), 8.32 (s, 1H).

### Beispiel 118

### Ethyl-1-[3-(cyanmethyl)-1-methyl-2-oxo-2,3-dihydro-1H-benzimidazol-5-yl]-2,4-dioxo-3-[(1R)-4-(trifluormethyl)-2,3-dihydro-1H-inden-1-yl]-1,2,3,4-tetrahydropyrimidin-5-carboxylat (R-Enantiomer)

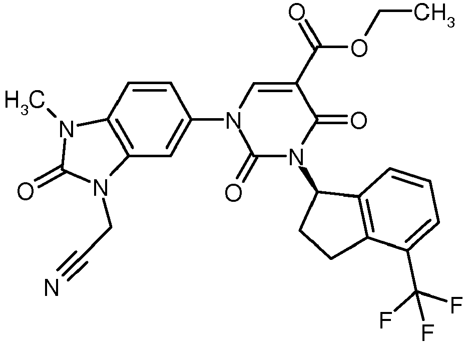

200 mg (0.38 mmol) Ethyl-1-(1-methyl-2-oxo-2,3-dihydro-1H-benzimidazol-5-yl)-2,4-dioxo-3-[4-(trifluormethyl)-2,3-dihydro-1H-inden-1-yl]-1,2,3,4-tetrahydropyrimidin-5-carboxylat (R-Enantiomer) aus Beispiel 112 wurden in Acetonitril (3.7 ml) vorgelegt, 93 mg (0.77 mmol) Bromacetonitril, 161 mg (1.16 mmol) Kaliumcarbonat wurden zugegeben. Die Reaktionsmischung ließ man 2 h bei 70°C rühren. Die auf RT abgekühlte Reaktionsmischung wurde mit 3 ml 1 N Salzsäure versetzt und 10 min gerührt. Das gesamte Gemisch wurde direkt per präparativer HPLC (Methode 7) getrennt. Man erhielt 180 mg (83 % d. Th.) der Titelverbindung.
LC-MS (Methode 1): Rₜ = 1.09 min; m/z = 554 (M+H)⁺.
¹H-NMR (400MHz, DMSO-d₆): δ [ppm] = 1.22 (t, 3H), 2.38 - 2.47 (m, 2H), 3.03 - 3.15 (m, 1H), 3.23 - 3.28 (m, 1H), 3.40 (s, 3H), 4.17 (q, 2H), 5.13 (s, 2H), 6.35 - 6.56 (m, 1H), 7.26 - 7.40 (m, 3H), 7.46 - 7.61 (m, 3H), 8.36 (s, 1H).

### Beispiel 119

### Ethyl-1-(3-methyl-2-oxo-2,3-dihydro-1,3-benzothiazol-6-yl)-2,4-dioxo-3-[7-(trifluormethyl)-2,3-dihydro-1-benzofur-3-yl]-1,2,3,4-tetrahydropyrimidin-5-carboxylat (Racemat)

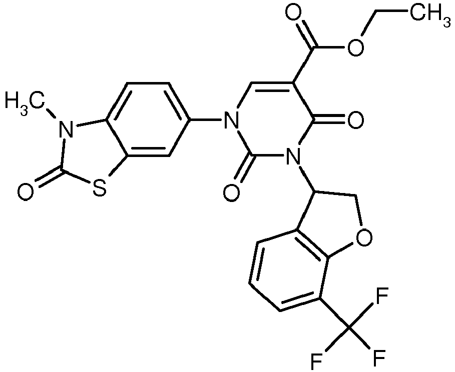

Darstellung und Reinigung der Titelverbindung erfolgten analog zu Beispiel 108. Ausgehend von 71 mg (0.20 mmol) Ethyl-1-(3-methyl-2-oxo-2,3-dihydro-1,3-benzothiazol-6-yl)-2,4-dioxo-1,2,3,4-tetrahydropyrimidin-5-carboxylat aus Beispiel 31A und 50 mg (0.24 mmol) 7-(Trifluormethyl)-2,3-dihydro-1-benzofuran-3-ol (Racemat) aus Beispiel 99A erhielt man 35 mg (31 % d. Th.) der Titelverbindung.
LC-MS (Methode 4): Rₜ = 2.35 min; m/z = 534 (M+H)⁺.
¹H-NMR (400MHz, CD₂Cl₂): δ [ppm] = 1.24 (t, 3H), 3.37 (s, 3H), 4.22 (q, 2H), 4.69 - 4.75 (m, 1H), 4.79 (t, 1H), 6.75 - 6.82 (m, 1H), 6.87 (t, 1H), 7.05 (d, 1H), 7.22 (dd, 1H), 7.30 (d, 1H), 7.33 - 7.38 (m, 2H), 8.21 (s, 1H).

### Beispiel 120

### Ethyl-1-(1,3-dimethyl-2-oxo-2,3-dihydro-1H-benzimidazol-5-yl)-3-(5-methoxy-2,3-dihydro-1H-inden-1-yl)-2,4-dioxo-1,2,3,4-tetrahydropyrimidin-5-carboxylat (Racemat)

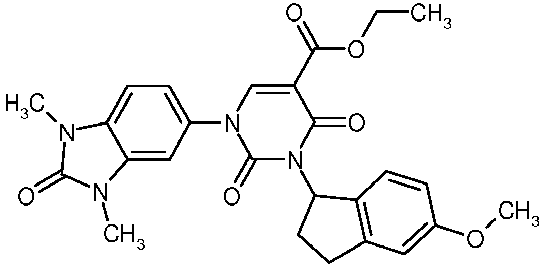

Unter Argonatmosphäre wurden 200 mg (0.58 mmol) der Verbindung aus Beispiel 2A und 457 mg (1.74 mmol) Triphenylphosphin in 8 ml DMF und 8 ml THF vorgelegt und auf -40°C gekühlt. Tropfenweise wurden 229 µl (1.16 mmol) Diisopropylazodicarboxylat hinzugefügt, dann 155 mg (80 %-ige Reinheit, 0.76 mmol) der Verbindung aus Beispiel 102A. Das Kühlbad wurde entfernt und die Mischung über Nacht bei RT gerührt. Anschließend wurden 25 ml IN Salzsäure zugegeben und die Mischung weiter 15 min gerührt. Zur Aufarbeitung wurden zum Reaktionsgemisch unter Eiskühlung 5 ml IN Salzsäure zugegeben, die Mischung 15 min weiter gerührt dann mit Essigsäureethylester extrahiert. Die organische Phase wurde zweimal mit IN Salzsäure, zweimal mit einer gesättigten Natriumhydrogencarbonatlösung, dann mit einer gesättigten Natriumchloridlösung gewaschen, dann über Natriumsulfat getrocknet und am Rotationsverdampfer eingeengt. Der Rückstand wurde per präparativer HPLC (Methode 7) gereinigt. Man erhielt 89 mg (30 % d. Th.) der Titelverbindung
LC-MS (Methode 1): Rₜ = 0.96 min; m/z = 491 (M+H)⁺.
¹H-NMR (400MHz, DMSO-d₆): δ [ppm]= 1.22 (t, 3H), 2.24 - 2.48 (m, 2H), 2.80 - 2.96 (m, 1H), 3.09 - 3.21 (m, 1H), 3.31 (s, 3H), 3.36 (s, 3H), 3.72 (s, 3H), 4.17 (q, 2H), 6.25 - 6.48 (m, 1H), 6.69 (dd, 1H), 6.78 (s, 1H), 7.04 (d, 1H), 7.10 - 7.29 (m, 2H), 7.37 (br.s, 1H), 8.30 (s, 1H).

### Beispiel 121

### 1-(1,3-Dimethyl-2-oxo-2,3-dihydro-1H-benzimidazol-5-yl)-3-[2-methyl-3-(trifluormethyl)benzyl]-2,4-dioxo-1,2,3,4-tetrahydropyrimidin-5-carbonsäure

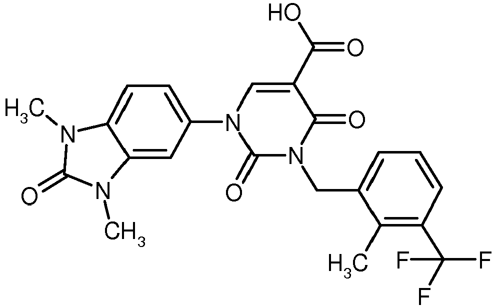

5.60 g (10.84 mmol) Ethyl-1-(1,3-dimethyl-2-oxo-2,3-dihydro-1H-benzimidazol-5-yl)-3-[2-methyl-3-(trifluormethyl)-benzyl]-2,4-dioxo-1,2,3,4-tetrahydropyrimidin-5-carboxylat aus Bei-spiel 1 wurden in 78 ml Eisessig und 39 ml konz. Salzsäure vorgelegt und bei 120°C für 1h gerührt. Anschließend versetzte man die auf RT abgekühlte Mischung mit Wasser und saugte den ausgefallenen Niederschlag ab. Der Feststoff wurde nacheinander mit Wasser sowie MTBE gewaschen und anschließend bei 50°C im Vakuum getrocknet. Man erhielt 5.11 g (96 % d. Th.) der Titelverbindung.
LC-MS (Methode 1): Rₜ = 0.98 min; m/z = 489 (M+H)⁺.
¹H-NMR (400MHz, DMSO-d₆): δ [ppm] = 2.47 (s, 3H), 3.31 (s, 3H), 3.37 (s, 3H), 5.11 (s, 2H), 7.22 - 7.30 (m, 2H), 7.33 - 7.43 (m, 3H), 7.59 - 7.63 (m, 1H), 8.45 (s, 1H), 12.73 (br.s, 1H).

### Beispiel 122

### 3-[2,3-Bis(trifluormethyl)benzyl]-1-(1,3-dimethyl-2-oxo-2,3-dihydro-1H-benzimidazol-5-yl)-2,4-dioxo-1,2,3,4-tetrahydropyrimidin-5-carbonsäure

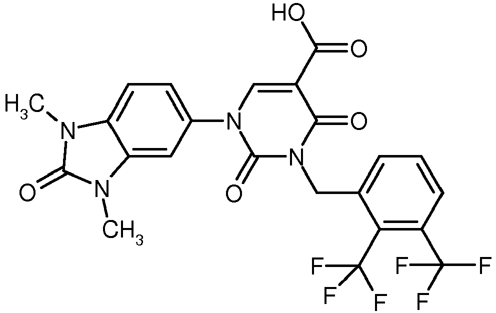

Darstellung und Reinigung der Titelverbindung erfolgten analog zu Beispiel 121. Ausgehend von 114 mg (0.20 mmol) Ethyl-3-[2,3-bis(trifluormethyl)benzyl]-1-(1,3-dimethyl-2-oxo-2,3-dihydro-1H-benzimidazol-5-yl)-2,4-dioxo-1,2,3,4-tetrahydropyrimidin-5-carboxylat aus Beispiel 8 erhielt man 92 mg (85 % d. Th.) der Titelverbindung.
LC-MS (Methode 1): Rₜ = 1.01 min; m/z = 543 (M+H)⁺.
¹H-NMR (400MHz, DMSO-d₆): δ [ppm] = 3.34 (s, 3H), 3.37 (s, 3H), 5.27 (m, 2H), 7.22 - 7.30 (m, 2H), 7.37 - 7.40 (m, 1H), 7.73 - 7.77 (m, 1H), 7.82 - 7.88 (m, 1H), 7.96 - 8.00 (m, 1H), 8.47 (s, 1H), 12.73 (br.s, 1H).

### Beispiel 123

### 3-[2-Chlor-3-(trifluormethyl)benzyl]-1-(1,3-dimethyl-2-oxo-2,3-dihydro-1H-benzimidazol-5-yl)-2,4-dioxo-1,2,3,4-tetrahydropyrimidin-5-carbonsäure

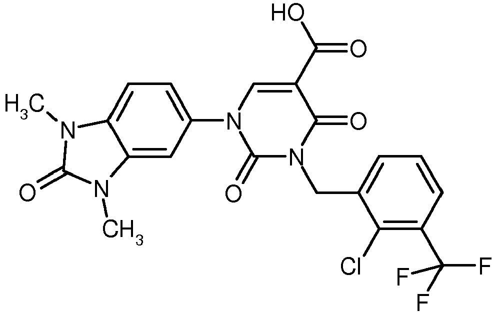

Darstellung und Reinigung der Titelverbindung erfolgten analog zu Beispiel 121. Ausgehend von 200 mg (0.37 mmol) Ethyl-3-[2-chlor-3-(trifluormethyl)benzyl]-1-(1,3-dimethyl-2-oxo-2,3-dihydro-1H-benzimidazol-5-yl)-2,4-dioxo-1,2,3,4-tetrahydropyrimidin-5-carboxylat aus Beispiel 2 erhielt man 67 mg (88 % d. Th.) der Titelverbindung.
LC-MS (Methode 3): Rₜ = 1.21 min; m/z = 509 (M+H)⁺.
¹H-NMR (400MHz, DMSO-d₆): δ [ppm] = 3.34 (s, 3H), 3.37 (s, 3H), 5.18 (s, 2H), 7.22 - 7.30 (m, 2H), 7.38 - 7.41 (m, 1H), 7.51 - 7.57 (m, 1H), 7.58 - 7.63 (m, 1H), 7.78 - 7.83 (m, 1H), 8.47 (s, 1H), 12.72 (br.s, 1H).

### Beispiel 124

### 3-(2,3-Dichlorbenzyl)-1-(1,3-dimethyl-2-oxo-2,3-dihydro-1H-benzimidazol-5-yl)-2,4-dioxo-1,2,3,4-tetrahydropyrimidin-5-carbonsäure

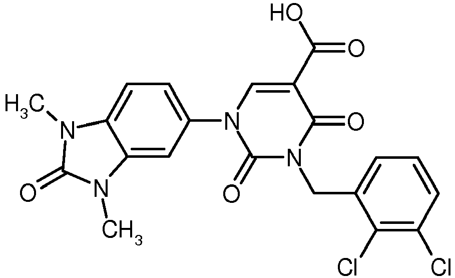

Darstellung und Reinigung der Titelverbindung erfolgten in Analogie zu Beispiel 121. Ausgehend von 200 mg (0.40 mmol) Ethyl-3-(2,3-dichlorbenzyl)-1-(1,3-dimethyl-2-oxo-2,3-dihydro-1H-benzimidazol-5-yl)-2,4-dioxo-1,2,3,4-tetrahydropyrimidin-5-carboxylat aus Beispiel 3 erhielt man 147 mg (78 % d. Th.) der Titelverbindung.
LC-MS (Methode 1): Rₜ = 1.00 min; m/z = 475 (M+H)⁺.
¹H-NMR (400MHz, DMSO-d₆): δ [ppm] = 3.34 (s, 3H), 3.37 (s, 3H), 5.13 (s, 2H), 7.22 - 7.30 (m, 3H), 7.31 - 7.37 (m, 1H), 7.38 - 7.41 (m, 1H), 7.57 - 7.61 (m, 1H), 8.45 (s, 1H), 12.72 (br.s, 1H).

### Beispiel 125

### 1-(1,3-Dimethyl-2-oxo-2,3-dihydro-1H-benzimidazol-5-yl)-3-[3-fluor-2-(trifluormethyl)benzyl]-2,4-dioxo-1,2,3,4-tetrahydropyrimidin-5-carbonsäure

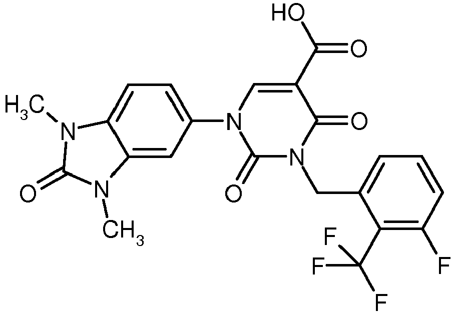

Darstellung und Reinigung der Titelverbindung erfolgten in Analogie zu Beispiel 121. Ausgehend von 170 mg (0.33 mmol) Ethyl-1-(1,3-dimethyl-2-oxo-2,3-dihydro-1H-benzimidazol-5-yl)-3-[3-fluor-2-(trifluormethyl)-benzyl]-2,4-dioxo-1,2,3,4-tetrahydropyrimidin-5-carboxylat aus Beispiel 5 erhielt man 141 mg (87 % d. Th.) der Titelverbindung.
LC-MS (Methode 1): Rₜ = 0.98 min; m/z = 493 (M+H)⁺.
¹H-NMR (400MHz, DMSO-d₆): δ [ppm] = 3.34 (s, 3H), 3.37 (s, 3H), 5.22 (s, 2H), 7.19 - 7.30 (m, 3H), 7.36 - 7.45 (m, 2H), 7.63 - 7.71 (m, 1H), 8.47 (s, 1H), 12.71 (br.s, 1H).

### Beispiel 126

### 1-(1,3-Dimethyl-2-oxo-2,3-dihydro-1H-benzimidazol-5-yl)-3-[2-fluor-3-(trifluormethyl)benzyl]-2,4-dioxo-1,2,3,4-tetrahydropyrimidin-5-carbonsäure

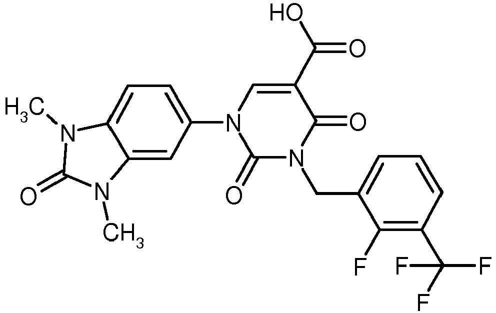

Darstellung und Reinigung der Titelverbindung erfolgten in Analogie zu Beispiel 121. Ausgehend von 161 mg (0.31 mmol) Ethyl-1-(1,3-dimethyl-2-oxo-2,3-dihydro-1H-benzimidazol-5-yl)-3-[2-fluor-3-(trifluormethyl)-benzyl]-2,4-dioxo-1,2,3,4-tetrahydropyrimidin-5-carboxylat aus Beispiel 4 erhielt man 115 mg (76 % d. Th.) der Titelverbindung.
LC-MS (Methode 1): Rₜ = 0.98 min; m/z = 493 (M+H)⁺.
¹H-NMR (400MHz, DMSO-d₆): δ [ppm] = 3.34 (s, 3H), 3.36 - 3.39 (m, 3H), 5.17 (s, 2H), 7.21 - 7.29 (m, 2H), 7.38 (s, 2H), 7.65 - 7.74 (m, 2H), 8.42 (s, 1H), 12.72 (br.s, 1H).

### Beispiel 127

### 3-(2-Chlor-3,6-difluorbenzyl)-1-(1,3-dimethyl-2-oxo-2,3-dihydro-1H-benzimidazol-5-yl)-2,4-dioxo-1,2,3,4-tetrahydropyrimidin-5-carbonsäure

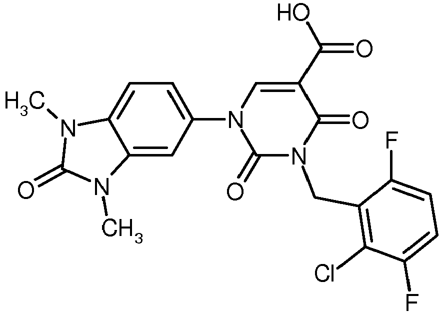

Darstellung und Reinigung der Titelverbindung erfolgten in Analogie zu Beispiel 121 mit 30 min Reaktionszeit. Ausgehend von 110 mg (0.22 mmol) Ethyl-3-(2-chlor-3,6-difluorbenzyl)-1-(1,3-dimethyl-2-oxo-2,3-dihydro-1H-benzimidazol-5-yl)-2,4-dioxo-l,2,3,4-tetrahydropyrimidin-5-carboxylat aus Beispiel 6 erhielt man 80 mg (76 % d. Th.) der Titelverbindung.
LC-MS (Methode 1): Rₜ = 0.93 min; m/z = 477 (M+H)⁺.
¹H-NMR (400MHz, DMSO-d₆): δ [ppm] = 3.2-3.4 (2 s, durch Wasser-Signal verdeckt), 5.24 (s, 2H), 7.14 - 7.19 (m, 1H), 7.23 - 7.32 (m, 2H), 7.32 - 7.36 (m, 1H), 7.40 - 7.48 (m, 1H), 8.39 (s, 1H), 12.74 (br.s, 1H).

### Beispiel 128

### 3-(3-Chlor-2-methylbenzyl)-1-(1,3-dimethyl-2-oxo-2,3-dihydro-1H-benzimidazol-5-yl)-2,4-dioxo-1,2,3,4-tetrahydropyrimidin-5-carbonsäure

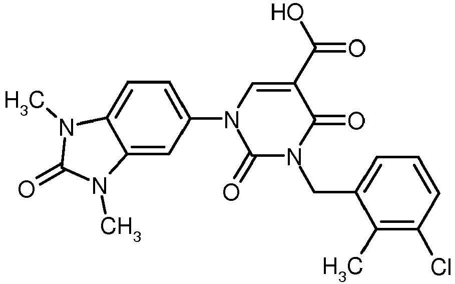

Darstellung und Reinigung der Titelverbindung erfolgten in Analogie zu Beispiel 121 mit einer Reaktionszeit von 30 min. Ausgehend von 75 mg (0.16 mmol) Ethyl-3-(3-chlor-2-methylbenzyl)-1-(1,3-dimethyl-2-oxo-2,3-dihydro-1H-benzimidazol-5-yl)-2,4-dioxo-1,2,3,4-tetrahydropyrimidin-5-carboxylat aus Beispiel 7 erhielt man 62 mg (87 % d. Th.) der Titelverbindung.
LC-MS (Methode 1): Rₜ = 0.96 min; m/z = 455 (M+H)⁺.
¹H-NMR (400MHz, DMSO-d₆): δ [ppm] = 2.41 (s, 3H), 3.34 (s, 3H), 3.37 (s, 3H), 5.08 (s, 2H), 7.06 - 7.09 (m, 1H), 7.17 (t, 1H), 7.22 - 7.29 (m, 2H), 7.33 - 7.37 (m, 1H), 7.39 - 7.42 (m, 1H), 8.44 (s, 1H), 12.73 (br.s, 1H).

### Beispiel 129

### 3-(3-Chlor-5-fluorbenzyl)-1-(1,3-dimethyl-2-oxo-2,3-dihydro-1H-benzimidazol-5-yl)-2,4-dioxo-1,2,3,4-tetrahydropyrimidin-5-carbonsäure

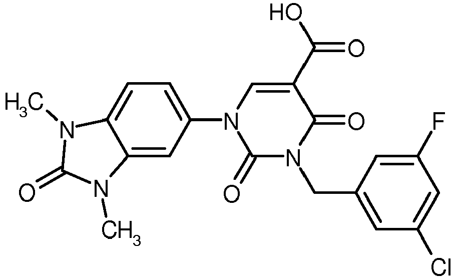

Darstellung und Reinigung der Titelverbindung erfolgte analog zu Beispiel 121. Die Reaktionszeit betrug 45 min. Ausgehend von 244 mg (0.50 mmol) Ethyl-3-(3-chlor-5-fluorbenzyl)-1-(1,3-dimethyl-2-oxo-2,3-dihydro-1H-benzimidazol-5-yl)-2,4-dioxo-1,2,3,4-tetrahydropyrimidin-5-carboxylat aus Beispiel 9 erhielt man 198 mg (85 % d. Th.) der Titelverbindung.
LC-MS (Methode 1): Rₜ = 0.98 min; m/z = 459 (M+H)⁺.
¹H-NMR (400MHz, DMSO-d₆): δ [ppm] = 3.31 (s, 3H), 3.37 (s, 3H), 5.02 - 5.09 (m, 2H), 7.19 - 7.33 (m, 4H), 7.33 - 7.38 (m, 1H), 7.39 (s, 1H), 8.39 (s, 1H), 12.73 (s, 1H).

### Beispiel 130

### 1-(1,3-Dimethyl-2-oxo-2,3-dihydro-1H-benzimidazol-5-yl)-3-[3-fluor-5-(trifluormethyl)benzyl]-2,4-dioxo-1,2,3,4-tetrahydropyrimidin-5-carbonsäure

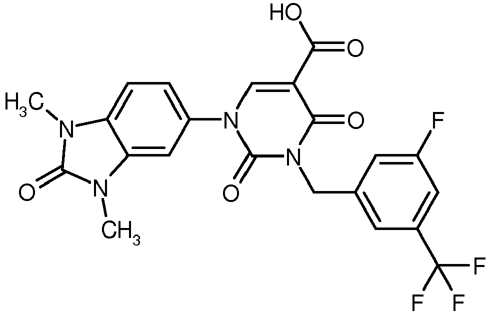

Darstellung und Reinigung der Titelverbindung erfolgten analog zu Beispiel 121. Die Reaktionszeit betrug 45 min. Ausgehend von 268 mg (0.51 mmol) Ethyl-1-(1,3-dimethyl-2-oxo-2,3-dihydro-1H-benzimidazol-5-yl)-3-[3-fluor-5-(trifluormethyl)benzyl]-2,4-dioxo-1,2,3,4-tetrahydropyrimidin-5-carboxylat aus Beispiel 10 erhielt man 215 mg (84 % d. Th.) der Titelverbindung.
LC-MS (Methode 1): Rₜ = 1.00 min; m/z = 493 (M+H)⁺.
¹H-NMR (400MHz, DMSO-d₆): δ [ppm] = 3.31 (s, 3H), 3.37 (s, 3H), 5.15 (s, 2H), 7.23 (dd, 1H), 7.28 (d, 1H), 7.38 (d, 1H), 7.54 (d, 1H), 7.58 - 7.65 (m, 2H), 8.40 (s, 1H), 12.73 (s, 1H).

### Beispiel 131

### 1-(1,3-Dimethyl-2-oxo-2,3-dihydro-1H-benzimidazol-5-yl)-2,4-dioxo-3-[4-(trifluormethyl)-2,3-dihydro-1H-inden-1-yl]-1,2,3,4-tetrahydropyrimidin-5-carbonsäure (Racemat)

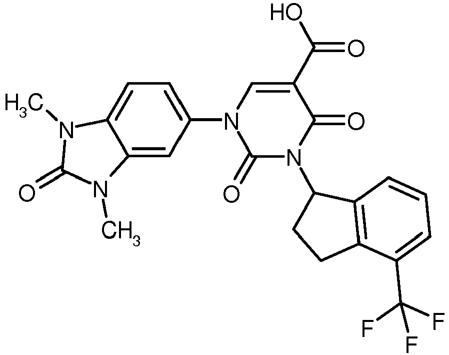

103 mg (0.19 mmol) Ethyl-1-(1,3-dimethyl-2-oxo-2,3-dihydro-1H-benzimidazol-5-yl)-2,4-dioxo-3-[4-(trifluormethyl)-2,3-dihydro-1H-inden-1-yl]-1,2,3,4-tetrahydropyrimidin-5-carboxylat aus Beispiel 11 wurden in Acetonitril/Wasser 1.5:1 (2.5 ml) vorgelegt, mit 36 mg (0.43 mmol) Natriumhydrogencarbonat versetzt und 4 h bei 80°C gerührt. Das abgekühlte Reaktionsgemisch wurde mit IN Salzsäure angesäuert, zweimal mit Essigsäureethylester extrahiert, die vereinigten organischen Phasen über Magnesiumsulfat getrocknet, abfiltriert und eingeengt. Der Rückstand wurde mittels HPLC (Methode 7) getrennt. Die Produktfraktionen wurden am Rotationsverdampfer fast vollständig eingeengt, der dabei ausgefallene Feststoff abfiltriert und an der Hochvakuumpumpe getrocknet. Man erhielt 32 mg (33 % d. Th.) der Titelverbindung.
LC-MS (Methode 1): Rₜ = 1.01 min; m/z = 501 (M+H)⁺.
¹H-NMR (400MHz, DMSO-d₆): δ [ppm] = 2.39 - 2.46 (m, 1H), 2.46 - 2.48 (m, 1H, teilweise verdeckt durch DMSO-Signal), 3.04 - 3.16 (m, 1H), 3.23 - 3.29 (m, 1H, teilweise verdeckt durch Wasser-Signal), 3.31 (s, 3H), 3.35 - 3.38 (m, 3H), 6.36 - 6.60 (m, 1H), 7.13 - 7.29 (m, 2H), 7.31 - 7.42 (m, 2H), 7.49 - 7.57 (m, 2H), 8.38 (s, 1H), 12.70 (br.s, 1H).

### Beispiel 132

### 1-(1,3-Dimethyl-2-oxo-2,3-dihydro-1H-benzimidazol-5-yl)-2,4-dioxo-3-[(1R)-4-(trifluormethyl)-2,3-dihydro-1H-inden-1-yl]-1,2,3,4-tetrahydropyrimidin-5-carbonsäure (R-Enantiomer)

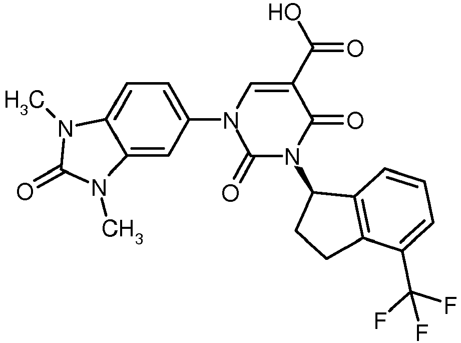

4.20 g (7.79 mmol) Ethyl-1-(1,3-dimethyl-2-oxo-2,3-dihydro-1H-benzimidazol-5-yl)-2,4-dioxo-3-[4-(trifluormethyl)-2,3-dihydro-1H-inden-1-yl]-1,2,3,4-tetrahydropyrimidin-5-carboxylat aus Beispiel 13 wurden mit 40 ml Eisessig und 20 ml konz. Salzsäure 1h bei Rückflusstemperatur gerührt. Die Reaktionsmischung wurde auf RT abgekühlt, dann mit 300 ml Wasser verdünnt. Der ausgefallene Feststoff wurde abgesaugt, mit wenig Wasser gewaschen und im HV getrocknet. Der so erhaltene Feststoff wurde mit 45 ml Toluol verrührt. Er löste sich zuerst vollständig, aber nach einigen Minuten bildete sich ein kristalliner Feststoff. Die Mischung wurde auf 0°C gekühlt und bei dieser Temperatur 30 min gerührt. Anschließend wurde der Feststoff abfiltriert, mit 5 ml Toluol gewaschen und im HV getrocknet. Man erhielt 3.17 g (81 % d. Th.) der Titelverbindung.
LC-MS (Methode 1): Rₜ = 1.06 min; m/z = 501 (M+H)⁺.
¹H-NMR (400MHz, DMSO-d₆): δ [ppm]= 2.38 - 2.46 (m, 1H), 2.46 - 2.60 (m, 1H teilweise unter DMSO-Signal versteckt), 3.10 (dt, 1H), 3.23 - 3.35 (m, 1H teilweise unter DMSO-Signal versteckt), 3.31 (s, 4H), 3.36 (s, 3H), 6.36 - 6.60 (m, 1H), 7.12 - 7.30 (m, 2H), 7.31 - 7.43 (m, 2H), 7.48 - 7.58 (m, 2H), 8.38 (s, 1H), 12.71 (br.s, 1H).
¹H-NMR (400MHz, CD₂Cl₂): δ [ppm]= 2.42 - 2.53 (m, 1H), 2.60 - 2.72 (m, 1H), 3.11 - 3.25 (m, 1H), 3.39 (s, 3H), 3.41 (s, 3H), 3.45 - 3.55 (m, 1H), 6.59 - 6.71 (m, 1H), 6.94 (br. s, 1H), 7.04 (s, 2H), 7.28 - 7.41 (m, 2H), 7.54 (d, 1H), 8.57 (s, 1H), 12.45 (br. S, 1H).

In einem analogen Versuch konnte eine Fraktion mit 99 % Reinheit isoliert werden. Für diese Charge wurde der spezifizische optische Drehwert gemessen:
Spezifischer optische Drehwert: αD²⁰ = +110.6°, (Methanol, c = 0.405 g/100 ml).

Eine Röntgenstrukturanalyse im Komplex mit Chymase bestätigte für dieses Enantiomer die R-Konfiguration.

### Beispiel 133

### 1-(1,3-Dimethyl-2-oxo-2,3-dihydro-1H-benzimidazol-5-yl)-2,4-dioxo-3-[(1S)-4-(trifluormethyl)-2,3-dihydro-1H-inden-1-yl]-1,2,3,4-tetrahydropyrimidin-5-carbonsäure (S-Enantiomer)

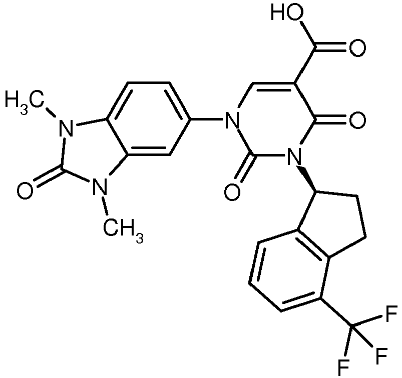

5.10 g (9.65 mmol) der Verbindung aus Beispiel 12 wurden in 50 ml Eisessig und 25 ml konz. Salzsäure 15 min bei Rückflusstemperatur gerührt. Nach Abkühlung auf RT wurde das Gemisch mit 5 ml Acetonitril verdünnt und portionsweise per präparativer HPLC (Methode 7) getrennt. Man erhielt 4.5 g (93 % d. Th.) der Titelverbindung.
LC-MS (Methode 1): Rₜ = 1.02 min; m/z = 501 (M+H)⁺.
¹H-NMR (400MHz, CD₂Cl₂): δ [ppm]= 2.33 - 2.46 (m, 1H), 2.58 (dtd, 1H), 3.04 - 3.16 (m, 1H), 3.30 (s, 3H), 3.33 (s, 3H), 3.36 - 3.47 (m, 1H), 6.50 - 6.66 (m, 1H), 6.86 (br.s, 1H), 6.95 (br. s, 2H), 7.20 - 7.33 (m, 2H), 7.46 (d, 1H), 8.49 (s, 1H), 12.38 (br.s, 1H).

### Beispiel 134

### 1-(6-Fluor-1,3-dimethyl-2-oxo-2,3-dihydro-1H-benzimidazol-5-yl)-2,4-dioxo-3-[(1R)-4-(trifluormethyl)-2,3-dihydro-1H-inden-1-yl]-1,2,3,4-tetrahydropyrimidin-5-carbonsäure (R-Enantiomer)

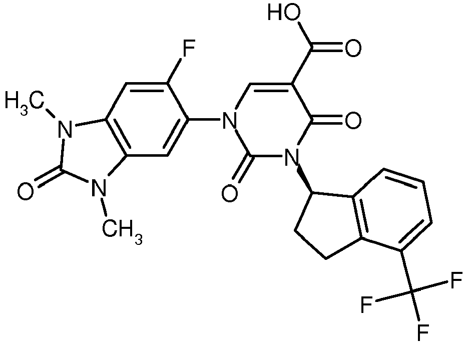

Darstellung und Reinigung der Titelverbindung erfolgten analog zu Beispiel 121 mit 45 min Reaktionszeit. Ausgehend von 120 mg (0.22 mmol) Ethyl-1-(6-fluor-1,3-dimethyl-2-oxo-2,3-dihydro-1H-benzimidazol-5-yl)-2,4-dioxo-3-[4-(trifluormethyl)-2,3-dihydro-1H-inden-l-yl]-1,2,3,4-tetrahydropyrimidin-5-carboxylat aus Beispiel 104 erhielt man nach zusätzlicher Reinigung mittels HPLC (Methode 8) 92 mg (80 % d. Th.) der Titelverbindung.
LC-MS (Methode 5): Rₜ = 1.09 min; m/z = 519 (M+H)⁺.
¹H-NMR (400MHz, CD₂Cl₂): δ [ppm] = 2.31 - 2.43 (m, 1H), 2.51 - 2.62 (m, 1H), 3.03 - 3.14 (m, 1H), 3.28 (s, 3H), 3.29 (s, 3H), 3.34 - 3.47 (m, 1H), 6.50 - 6.58 (m, 1H), 6.76 - 6.84 (m, 2H), 7.21 - 7.27 (m, 2H), 7.41 - 7.47 (m, 1H), 8.41 (s, 1H), 12.31 (s, 1H).

### Beispiel 135

### 3-[2-Chlor-3-(trifluormethyl)benzyl]-1-(1,3-diethyl-2-oxo-2,3-dihydro-1H-benzimidazol-5-yl)-2,4-dioxo-1,2,3,4-tetrahydropyrimidin-5-carbonsäure

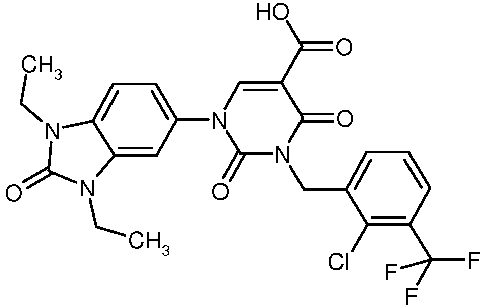

Darstellung und Reinigung der Titelverbindung erfolgten in Analogie zu Beispiel 121. Ausgehend von 170 mg (0.30 mmol) Ethyl-3-[2-chlor-3-(trifluormethyl)benzyl]-1-(1,3-diethyl-2-oxo-2,3-dihydro-1H-benzimidazol-5-yl)-2,4-dioxo-1,2,3,4-tetrahydropyrimidin-5-carboxylat aus Beispiel 21 erhielt man 133 mg (82 % d. Th.) der Titelverbindung.
LC-MS (Methode 1): Rₜ = 1.20 min; m/z = 537 (M+H)⁺.
¹H-NMR (400MHz, DMSO-d₆): δ [ppm] = 1.22 (t, 6H), 3.82 - 3.96 (m, 4H), 5.18 (s, 2H), 7.21 - 7.27 (m, 1H), 7.34 (d, 1H), 7.43 - 7.48 (m, 1H), 7.50 - 7.63 (m, 2H), 7.77 - 7.84 (m, 1H), 8.50 (s, 1H), 12.71 (br.s, 1H).

### Beispiel 136

### 1-(1,3-Diethyl-2-oxo-2,3-dihydro-1H-benzimidazol-5-yl)-3-[2-methyl-3-(trifluormethyl)benzyl]-2,4-dioxo-1,2,3,4-tetrahydropyrimidin-5-carbonsäure

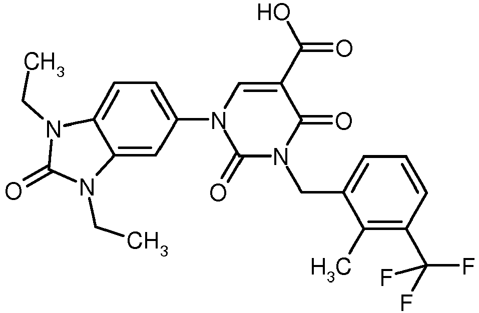

Darstellung und Reinigung der Titelverbindung erfolgten in Analogie zu Beispiel 121. Ausgehend von 170 mg (0.31 mmol) Ethyl-1-(1,3-diethyl-2-oxo-2,3-dihydro-1H-benzimidazol-5-yl)-3-[2-methyl-3-(trifluormethyl)-benzyl]-2,4-dioxo-1,2,3,4-tetrahydropyrimidin-5-carboxylat aus Beispiel 22 erhielt man 144 mg (89 % d. Th.) der Titelverbindung.
LC-MS (Methode 1): Rₜ = 1.10 min; m/z = 517 (M+H)⁺.
¹H-NMR (400MHz, DMSO-d₆): δ [ppm] = 1.22 (t, 6H), 2.47 (s, 3H), 3.84 - 3.95 (m, 4H), 5.12 (s, 2H), 7.22 - 7.26 (m, 1H), 7.33 (s, 3H), 7.46 - 7.48 (m, 1H), 7.59 - 7.63 (m, 1H), 8.49 (s, 1H), 12.72 (br.s, 1H).

### Beispiel 137

### 3-(2,3-Dichlorbenzyl)-1-(1,3-diethyl-2-oxo-2,3-dihydro-1H-benzimidazol-5-yl)-2,4-dioxo-1,2,3,4-tetrahydro-pyrimidin-5-carbonsäure

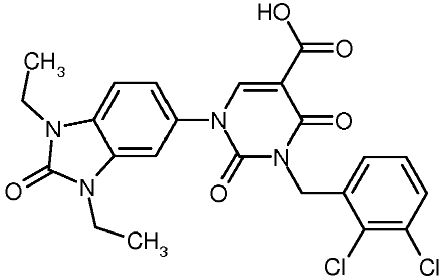

Darstellung und Reinigung der Titelverbindung erfolgten in Analogie zu Beispiel 121. Ausgehend von 244 mg (0.46 mmol) Ethyl-3-(2,3-dichlorbenzyl)-1-(1,3-diethyl-2-oxo-2,3-dihydro-1H-benzimidazol-5-yl)-2,4-dioxo-1,2,3,4-tetrahydropyrimidin-5-carboxylat aus Beispiel 23 erhielt man 188 mg (81 % d. Th.) der Titelverbindung.
LC-MS (Methode 1): Rₜ = 1.08 min; m/z = 503 (M+H)⁺.
¹H-NMR (400MHz, DMSO-d₆): δ [ppm] = 1.22 (t, 6H), 3.83 - 3.95 (m, 4H), 5.14 (s, 2H), 7.21 - 7.27 (m, 2H), 7.31 - 7.37 (m, 2H), 7.45 - 7.47 (m, 1H), 7.57 - 7.61 (m, 1H), 8.49 (s, 1H), 12.71 (br.s, 1H).

### Beispiel 138

### 1-(1,3-Diethyl-2-oxo-2,3-dihydro-1H-benzimidazol-5-yl)-3-[3-fluor-2-(trifluormethyl)benzyl]-2,4-dioxo-1,2,3,4-tetrahydropyrimidin-5-carbonsäure

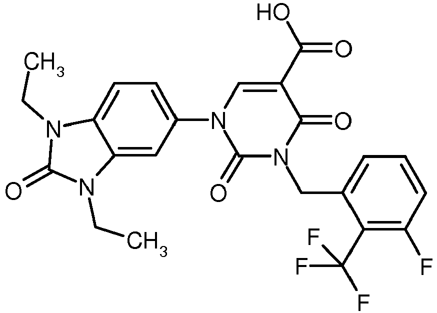

Darstellung und Reinigung der Titelverbindung erfolgten in Analogie zu Beispiel 121. Ausgehend von 167 mg (0.30 mmol) Ethyl-1-(1,3-diethyl-2-oxo-2,3-dihydro-1H-benzimidazol-5-yl)-3-[3-fluor-2-(trifluormethyl)-benzyl]-2,4-dioxo-1,2,3,4-tetrahydropyrimidin-5-carboxylat aus Beispiel 24 erhielt man 96 mg (61 % d. Th.) der Titelverbindung.
LC-MS (Methode 3): Rₜ = 1.24 min; m/z = 521 (M+H)⁺.
¹H-NMR (400MHz, DMSO-d₆): δ [ppm] = 1.22 (t, 6H), 3.83 - 3.96 (m, 4H), 5.23 (s, 2H), 7.19 - 7.26 (m, 2H), 7.31 - 7.37 (m, 1H), 7.37 - 7.48 (m, 2H), 7.63 - 7.72 (m, 1H), 8.50 (s, 1H), 12.71 (br.s, 1H).

### Beispiel 139

### 3-[3-Chlor-2-(trifluormethyl)benzyl]-1-(3-ethyl-1-methyl-2-oxo-2,3-dihydro-1H-benzimidazol-5-yl)-2,4-dioxo-1,2,3,4-tetrahydropyrimidin-5-carbonsäure

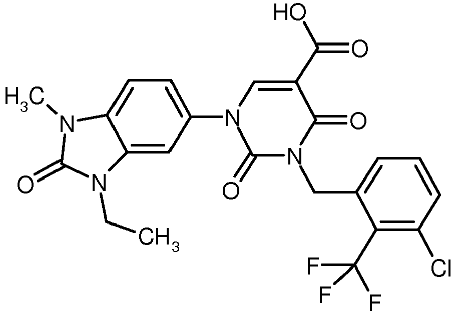

Darstellung und Reinigung der Titelverbindung erfolgten in Analogie zu Beispiel 121. Ausgehend von 73 mg (0.13 mmol) Ethyl-3-[3-chlor-2-(trifluormethyl)benzyl]-1-(3-ethyl-1-methyl-2-oxo-2,3-dihydro-1H-benzimidazol-5-yl)-2,4-dioxo-1,2,3,4-tetrahydropyrimidin-5-carboxylat aus Beispiel 18 erhielt man 50 mg (69 % d. Th.) der Titelverbindung.
LC-MS (Methode 1): Rₜ = 1.08 min; m/z = 523 (M+H)⁺.
¹H-NMR (400MHz, DMSO-d₆): δ [ppm] = 1.22 (t, 3H), 3.37 (s, 3H), 3.88 (q, 2H), 5.21 - 5.27 (m, 2H), 7.21 - 7.30 (m, 2H), 7.33 - 7.37 (m, 1H), 7.43 - 7.46 (m, 1H), 7.58 - 7.68 (m, 2H), 8.49 (s, 1H), 12.72 (br.s, 1H).

### Beispiel 140

### 3-[2-Chlor-3-(trifluormethyl)benzyl]-1-(3-ethyl-1-methyl-2-oxo-2,3-dihydro-1H-benzimidazol-5-yl)-2,4-dioxo-1,2,3,4-tetrahydropyrimidin-5-carbonsäure

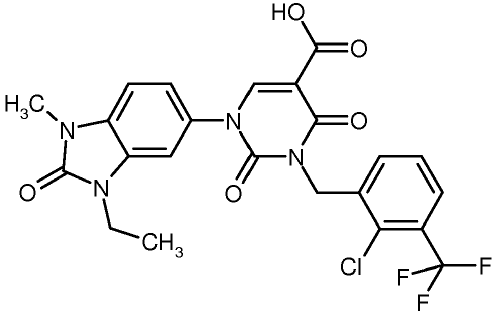

Darstellung und Reinigung der Titelverbindung erfolgten in Analogie zu Beispiel 121. Ausgehend von 75 mg (0.14 mmol) Ethyl-3-[2-chlor-3-(trifluormethyl)benzyl]-1-(3-ethyl-1-methyl-2-oxo-2,3-dihydro-1H-benzimidazol-5-yl)-2,4-dioxo-1,2,3,4-tetrahydropyrimidin-5-carboxylat aus Beispiel 19 erhielt man 35 mg (49 % d. Th.) der Titelverbindung.
LC-MS (Methode 1): Rₜ = 1.02 min; m/z = 523 (M+H)⁺.
¹H-NMR (400MHz, DMSO-d₆): δ [ppm] = 1.22 (t, 3H), 3.37 (s, 3H), 3.88 (q, 2H), 5.18 (s, 2H), 7.22 - 7.30 (m, 2H), 7.44 - 7.47 (m, 1H), 7.51 - 7.57 (m, 1H), 7.58 - 7.62 (m, 1H), 7.79 - 7.83 (m, 1H), 8.49 (s, 1H), 12.73 (br.s, 1H).

### Beispiel 141

### 1-(3-Ethyl-1-methyl-2-oxo-2,3-dihydro-1H-benzimidazol-5-yl)-3-[2-methyl-3-(trifluormethyl)-benzyl]-2,4-dioxo-1,2,3,4-tetrahydropyrimidin-5-carbonsäure

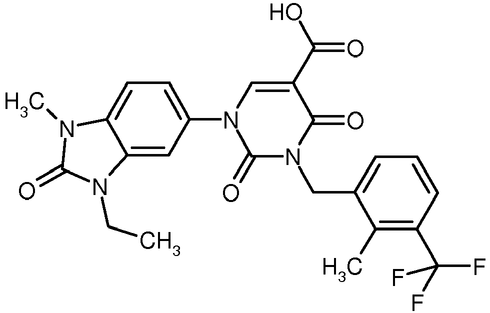

Darstellung und Reinigung der Titelverbindung erfolgten in Analogie zu Beispiel 121. Ausgehend von 53 mg (0.10 mmol) Ethyl-1-(3-ethyl-1-methyl-2-oxo-2,3-dihydro-1H-benzimidazol-5-yl)-3-[2-methyl-3-(trifluormethyl)benzyl]-2,4-dioxo-1,2,3,4-tetrahydropyrimidin-5-carboxylat aus Beispiel 20 erhielt man 23 mg (46 % d. Th.) der Titelverbindung.
LC-MS (Methode 1): Rₜ = 1.06 min; m/z = 503 (M+H)⁺.
¹H-NMR (400MHz, DMSO-d₆): δ [ppm] = 1.22 (t, 3H), 2.47 (s, 3H), 3.37 (s, 3H), 3.87 (q, 2H), 5.11 (s, 2H), 7.22 - 7.30 (m, 2H), 7.33 - 7.43 (m, 2H), 7.46 - 7.49 (m, 1H), 7.59 - 7.64 (m, 1H), 8.48 (s, 1H), 12.74 (br.s, 1H).

### Beispiel 142

### 3-[3-Chlor-2-(trifluormethyl)benzyl]-1-[1-methyl-2-oxo-3-(2,2,2-trifluorethyl)-2,3-dihydro-1H-benzimidazol-5-yl]-2,4-dioxo-1,2,3,4-tetrahydropyrimidin-5-carbonsäure

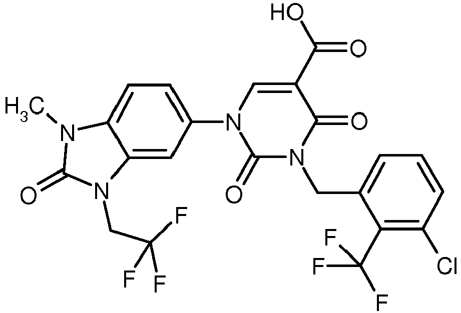

Darstellung und Reinigung der Titelverbindung erfolgten in Analogie zu Beispiel 121. Ausgehend von 90 mg (0.15 mmol) Ethyl-3-[3-chlor-2-(trifluormethyl)benzyl]-1-[1-methyl-2-oxo-3-(2,2,2-trifluorethyl)-2,3-dihydro-1H-benzimidazol-5-yl]-2,4-dioxo-1,2,3,4-tetrahydropyrimidin-5-carboxylat aus Beispiel 25 erhielt man 55 mg (61 % d. Th.) der Titelverbindung.
LC-MS (Methode 1): Rₜ = 1.12 min; m/z = 577 (M+H)⁺.
¹H-NMR (400MHz, DMSO-d₆): δ [ppm] = 3.41 (s, 3H), 4.81 (q, 2H), 5.22 - 5.26 (m, 2H), 7.30 - 7.39 (m, 3H), 7.51 - 7.55 (m, 1H), 7.57 - 7.67 (m, 2H), 8.46 (s, 1H), 12.74 (br.s, 1H).

### Beispiel 143

### 3-[2-Chlor-3-(trifluormethyl)benzyl]-1-[1-methyl-2-oxo-3-(2,2,2-trifluorethyl)-2,3-dihydro-1H-benzimidazol-5-yl]-2,4-dioxo-1,2,3,4-tetrahydropyrimidin-5-carbonsäure

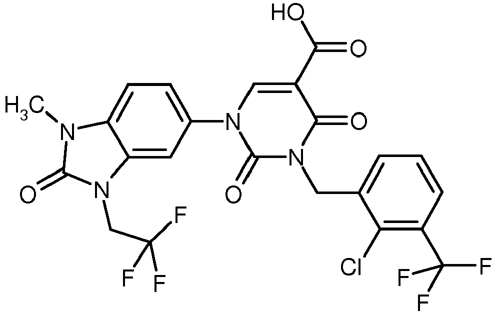

Darstellung und Reinigung der Titelverbindung erfolgten in Analogie zu Beispiel 121. Ausgehend von 80 mg (0.13 mmol) Ethyl-3-[2-chlor-3-(trifluormethyl)benzyl]-1-[1-methyl-2-oxo-3-(2,2,2-trifluorethyl)-2,3-dihydro-1H-benzimidazol-5-yl]-2,4-dioxo-1,2,3,4-tetrahydropyrimidin-5-carboxylat aus Beispiel 26 erhielt man 46 mg (57 % d. Th.) der Titelverbindung.
LC-MS (Methode 1): Rₜ = 1.12 min; m/z = 577 (M+H)⁺.
¹H-NMR (400MHz, DMSO-d₆): δ [ppm] = 3.41 (s, 3H), 4.80 (q, 2H), 5.17 (s, 2H), 7.31 - 7.38 (m, 2H), 7.50 - 7.56 (m, 2H), 7.59 - 7.63 (m, 1H), 7.79 - 7.83 (m, 1H), 8.45 (s, 1H), 12.75 (br.s, 1H).

### Beispiel 144

### 1-[1-Methyl-2-oxo-3-(2,2,2-trifluorethyl)-2,3-dihydro-1H-benzimidazol-5-yl]-3-[2-methyl-3-(trifluormethyl)benzyl]-2,4-dioxo-1,2,3,4-tetrahydropyrimidin-5-carbonsäure

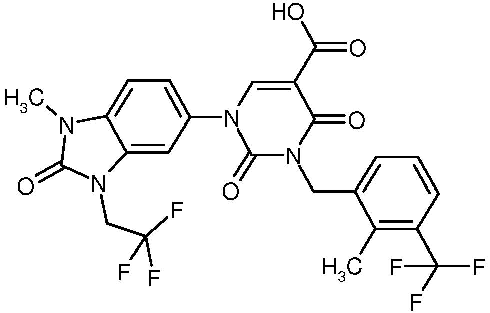

Darstellung und Reinigung der Titelverbindung erfolgten in Analogie zu Beispiel 121. Ausgehend von 41 mg (0.07 mmol) Ethyl-1-[1-methyl-2-oxo-3-(2,2,2-trifluorethyl)-2,3-dihydro-1H-benzimidazol-5-yl]-3-[2-methyl-3-(trifluormethyl)benzyl]-2,4-dioxo-1,2,3,4-tetrahydro-5-carboxylat aus Beispiel 27 erhielt man 25 mg (63 % d. Th.) der Titelverbindung.
LC-MS (Methode 3): Rₜ = 1.33 min; m/z = 557 (M+H)⁺.
¹H-NMR (400MHz, DMSO-d₆): δ [ppm] = 2.47 (s, 3H), 3.41 (s, 3H), 4.80 (q, 2H), 5.11 (s, 2H), 7.31 - 7.43 (m, 4H), 7.55 (s, 1H), 7.58 - 7.63 (m, 1H), 8.43 (s, 1H), 12.76 (br.s, 1H).

### Beispiel 145

### 3-[2-Chlor-3-(trifluormethyl)benzyl]-1-[3-(cyclopropylmethyl)-1-methyl-2-oxo-2,3-dihydro-1H-benzimidazol-5-yl]-2,4-dioxo-1,2,3,4-tetrahydropyrimidin-5-carbonsäure

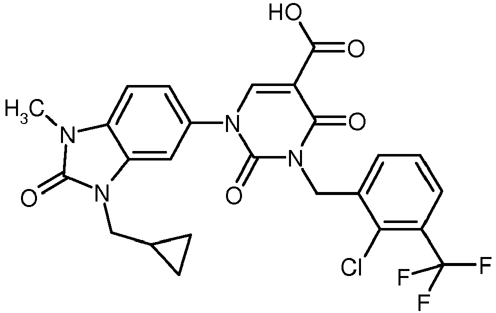

Darstellung und Reinigung der Titelverbindung erfolgten in Analogie zu Beispiel 121, ausgehend von 65 mg (0.11 mmol) Ethyl-3-[2-chlor-3-(trifluormethyl)benzyl]-1-[3-(cyclopropylmethyl)-1-methyl-2-oxo-2,3-dihydro-1H-benzimidazol-5-yl]-2,4-dioxo-1,2,3,4-tetrahydropyrimidin-5-carboxylat aus Beispiel 29. Das erhaltene Rohprodukt wurde zusätzlich mittels präparativer HPLC (Methode 22) gereinigt. Man erhielt 23 mg (62 % d. Th.) der Titelverbindung.
LC-MS (Methode 1): Rₜ = 1.08 min; m/z = 549 (M+H)⁺.
¹H-NMR (400MHz, DMSO-d₆): δ [ppm] = 0.35 - 0.49 (m, 4H), 1.14 - 1.26 (m, 1H), 2.5 (s, durch DMSO-Signal verdeckt), 3.38 (s, 3H), 3.72 (d, 2H), 5.18 (s, 2H), 7.22 - 7.31 (m, 2H), 7.50 - 7.57 (m, 2H), 7.58 - 7.63 (m, 1H), 7.79 - 7.83 (m, 1H), 8.48 (s, 1H), 12.73 (br.s, 1H).

### Beispiel 146

### 1-[3-(Cyclopropylmethyl)-1-methyl-2-oxo-2,3-dihydro-1H-benzimidazol-5-yl]-3-[2-methyl-3-(trifluormethyl)benzyl]-2,4-dioxo-1,2,3,4-tetrahydropyrimidin-5-carbonsäure

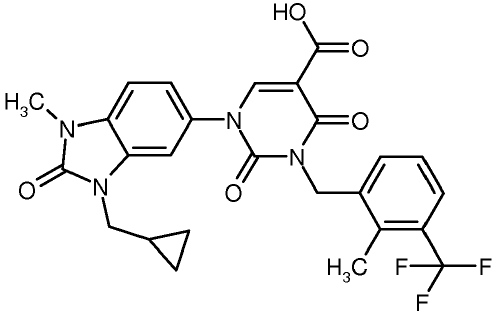

Darstellung und Reinigung der Titelverbindung erfolgte analog zu Beispiel 121. Ausgehend von 69 mg (0.12 mmol) Ethyl-1-[3-(cyclopropylmethyl)-1-methyl-2-oxo-2,3-dihydro-1H-benzimidazol-5-yl]-3-[2-methyl-3-(trifluormethyl)benzyl]-2,4-dioxo-1,2,3,4-tetrahydropyrimidin-5-carboxylat aus Beispiel 30 erhielt man nach zusätzlicher Reinigung mittels präparativer HPLC (Methode 10) 29 mg (90 %ig, 40 % d. Th.) der Titelverbindung.
LC-MS (Methode 1): Rₜ = 1.08 min; m/z = 529 (M+H)⁺.
¹H-NMR (400MHz, DMSO-d₆): δ [ppm] = 0.35 - 0.41 (m, 2H), 0.42 - 0.49 (m, 2H), 1.15 - 1.25 (m, 1H), 2.47 (s, 3H), 3.38 (s, verdeckt durch DMSO-Signal), 3.72 (d, 2H), 5.12 (s, 2H), 7.23 - 7.30 (m, 2H), 7.33 - 7.43 (m, 2H), 7.51 - 7.54 (m, 1H), 7.59 - 7.63 (m, 1H), 8.47 (s, 1H), 12.73 (br.s, 1H).

### Beispiel 147

### 3-[3-Chlor-2-(trifluormethyl)benzyl]-1-[3-(cyclopropylmethyl)-1-methyl-2-oxo-2,3-dihydro-1H-benzimidazol-5-yl]-2,4-dioxo-1,2,3,4-tetrahydropyrimidin-5-carbonsäure

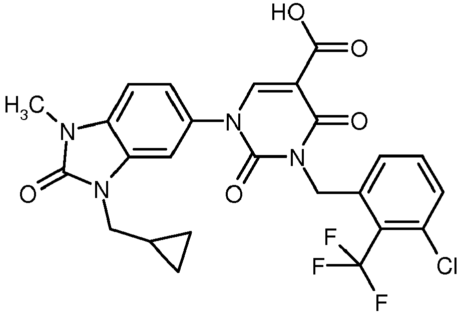

Darstellung und Reinigung der Titelverbindung erfolgten in Analogie zu Beispiel 121. Ausgehend von 115 mg (0.23 mmol) Ethyl-3-[3-chlor-2-(trifluormethyl)benzyl]-1-[3-(cyclopropylmethyl)-1-methyl-2-oxo-2,3-dihydro-1H-benzimidazol-5-yl]-2,4-dioxo-1,2,3,4-tetrahydropyrimidin-5-carboxylat aus Beispiel 31 erhielt man 92 mg (84 % d. Th.) der Titelverbindung.
LC-MS (Methode 1): Rₜ = 1.13 min; m/z = 549 (M+H)⁺.
¹H-NMR (400MHz, DMSO-d₆): δ [ppm] = 0.35 - 0.41 (m, 2H), 0.41 - 0.49 (m, 2H), 1.15 - 1.25 (m, 1H), 3.4 (s, verdeckt durch Wasser-Signal), 3.72 (d, 2H), 5.25 (br.s, 2H), 7.22 - 7.31 (m, 2H), 7.32 - 7.38 (m, 1H), 7.49 - 7.52 (m, 1H), 7.57 - 7.68 (m, 2H), 8.48 (s, 1H), 12.73 (br.s, 1H).

### Beispiel 148

### 3-[2-Chlor-3-(trifluormethyl)benzyl]-1-[1-methyl-3-(oxetan-2-ylmethyl)-2-oxo-2,3-dihydro-1H-benzimidazol-5-yl]-2,4-dioxo-1,2,3,4-tetrahydropyrimidin-5-carbonsäure (Racemat)

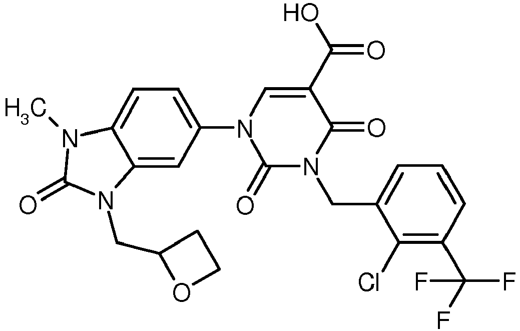

Darstellung und Reinigung der Titelverbindung erfolgten in Analogie zu Beispiel 121. Ausgehend von 56 mg (0.09 mmol) Ethyl-3-[2-chlor-3-(trifluormethyl)benzyl]-1-[1-methyl-3-(oxetan-2-ylmethyl)-2-oxo-2,3-dihydro-1H-benzimidazol-5-yl]-2,4-dioxo-1,2,3,4-tetrahydropyrimidin-5-carboxylat aus Beispiel 32 erhielt man 10 mg (18 % d. Th.) der Titelverbindung.
LC-MS (Methode 3): Rₜ = 1.24 min; m/z = 565 (M+H)⁺.
¹H-NMR (400MHz, DMSO-d₆): δ [ppm] = 2.11 - 2.21 (m, 1H), 2.26 - 2.36 (m, 1H), 3.36 (s, 3H), 3.72 (q, 1H), 3.84 - 3.91 (m, 1H), 3.93 - 3.99 (m, 1H), 4.11 - 4.18 (m, 1H), 5.09 - 5.20 (m, 3H), 7.25 - 7.32 (m, 2H), 7.43 - 7.46 (m, 1H), 7.50 - 7.57 (m, 1H), 7.60 - 7.65 (m, 1H), 7.78 - 7.84 (m, 1H), 8.48 (s, 1H), 12.74 (br.s, 1H).

### Beispiel 149

1-(3-Cyclobutyl-1-methyl-2-oxo-2,3-dihydro-1H-benzimidazol-5-yl)-3-[2-methyl-3-(trifluor-methyl)benzyl]-2,4-dioxo-1,2,3,4-tetrahydropyrimidin-5-carbonsäure

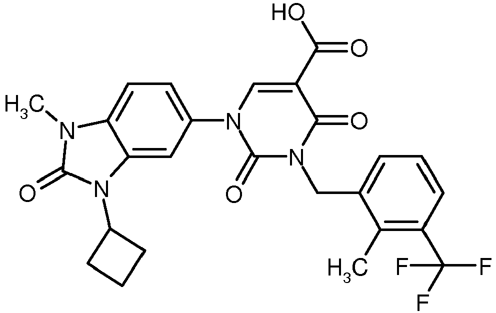

Darstellung und Reinigung der Titelverbindung erfolgten in Analogie zu Beispiel 121 mit 5.5 h Reaktionszeit bei 60°C. Ausgehend von 33 mg (0.06 mmol) Ethyl-1-(3-cyclobutyl-1-methyl-2-oxo-2,3-dihydro-1H-benzimidazol-5-yl)-3-[2-methyl-3-(trifluormethyl)benzyl]-2,4-dioxo-1,2,3,4-tetrahydropyrimidin-5-carboxylat aus Beispiel 33 erhielt man 18 mg (57 % d. Th.) der Titelverbindung.
LC-MS (Methode 1): Rₜ = 1.10 min; m/z = 529 (M+H)⁺.
¹H-NMR (400MHz, DMSO-d₆): δ [ppm] = 1.73 - 1.90 (m, 2H), 2.21 - 2.31 (m, 2H), 2.47 (s, teilweise durch DMSO-Signal verdeckt), 2.75 - 2.87 (m, 2H), 3.34 (s, teilweise durch Wasser-Signal verdeckt), 4.78 - 4.89 (m, 1H), 5.12 (s, 2H), 7.24 - 7.29 (m, 2H), 7.34 - 7.39 (m, 1H), 7.40 - 7.44 (m, 1H), 7.59 - 7.63 (m, 1H), 7.64 - 7.67 (m, 1H), 8.49 (s, 1H), 12.73 (br.s, 1H).

### Beispiel 150

### 1-(3-Cyclopropyl-1-methyl-2-oxo-2,3-dihydro-1H-benzimidazol-5-yl)-3-[2-methyl-3-(trifluor-methyl)benzyl]-2,4-dioxo-1,2,3,4-tetrahydropyrimidin-5-carbonsäure

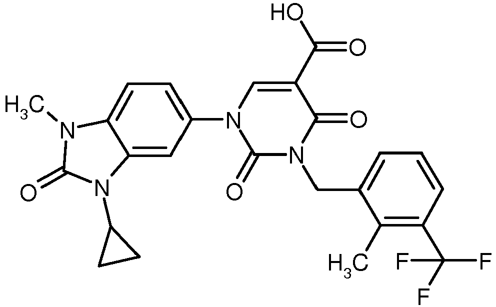

Darstellung und Reinigung der Titelverbindung erfolgten in Analogie zu Beispiel 121 mit 2 h Reaktionszeit bei 60°C. Ausgehend von 141 mg (0.26 mmol) Ethyl-1-(3-cyclopropyl-1-methyl-2-oxo-2,3-dihydro-1H-benzimidazol-5-yl)-3-[2-methyl-3-(trifluormethyl)benzyl]-2,4-dioxo-1,2,3,4-tetrahydropyrimidin-5-carboxylat aus Beispiel 35 erhielt man 107 mg (80 % d. Th.) der Titelverbindung.
LC-MS (Methode 5): Rₜ = 1.07 min; m/z = 515 (M+H)⁺.
¹H-NMR (400MHz, DMSO-d₆): δ [ppm] = 0.86 - 0.92 (m, 2H), 0.99 - 1.05 (m, 2H), 2.47 (s, teilweise durch DMSO-Signal verdeckt), 2.88 - 2.95 (m, 1H), 3.31 (s, teilweise durch Wasser-Signal verdeckt), 5.11 (s, 2H), 7.25 (s, 2H), 7.33 - 7.39 (m, 1H), 7.40 - 7.47 (m, 2H), 7.59 - 7.63 (m, 1H), 8.45 (s, 1H), 12.72 (br.s, 1H).

### Beispiel 151

### 1-(3-Isopropyl-1-methyl-2-oxo-2,3-dihydro-1H-benzimidazol-5-yl)-3-[2-methyl-3-(trifluormethyl)-benzyl]-2,4-dioxo-1,2,3,4-tetrahydropyrimidin-5-carbonsäure

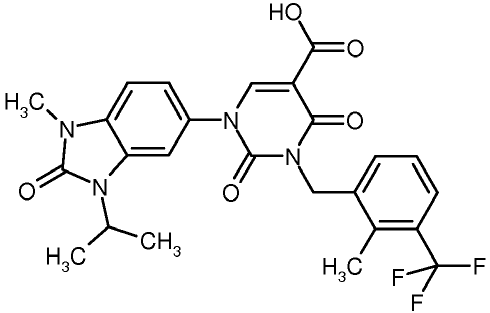

Darstellung und Reinigung der Titelverbindung erfolgten in Analogie zu Beispiel 121 mit 5.5 h Reaktionszeit bei 60°C. Ausgehend von 33 mg (0.06 mmol) Ethyl-1-(3-isopropyl-1-methyl-2-oxo-2,3-dihydro-1H-benzimidazol-5-yl)-3-[2-methyl-3-(trifluormethyl)benzyl]-2,4-dioxo-1,2,3,4-tetrahydropyrimidin-5-carboxylat aus Beispiel 34 erhielt man 25 mg (76 % d. Th.) der Titelverbindung.
LC-MS (Methode 1): Rₜ = 1.06 min; m/z = 517 (M+H)⁺.
¹H-NMR (400MHz, DMSO-d₆): δ [ppm] = 1.44 (d, 6H), 2.47 (s, teilweise durch DMSO-Signal verdeckt), 3.35 (s, teilweise durch Wasser-Signal verdeckt), 4.55 - 4.64 (m, 1H), 5.12 (s, 2H), 7.21 - 7.28 (m, 2H), 7.33 - 7.43 (m, 2H), 7.58 - 7.63 (m, 2H), 8.47 (s, 1H), 12.73 (br.s, 1H).

### Beispiel 152

### 1-[3-Methyl-2-oxo-1-(2,2,2-trifluorethyl)-2,3-dihydro-1H-benzimidazol-5-yl]-3-[2-methyl-3-(trifluormethyl)benzyl]-2,4-dioxo-1,2,3,4-tetrahydropyrimidin-5-carbonsäure

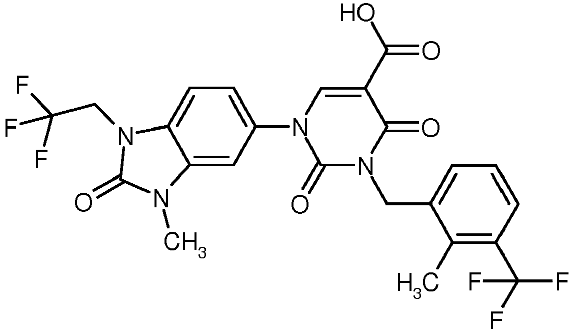

Darstellung und Reinigung der Titelverbindung erfolgten in Analogie zu Beispiel 121. Ausgehend von 57 mg (0.09 mmol) Ethyl-1-[3-methyl-2-oxo-1-(2,2,2-trifluorethyl)-2,3-dihydro-1H-benzimidazol-5-yl]-3-[2-methyl-3-(trifluorniethyl)benzyl]-2,4-dioxo-1,2,3,4-tetrahydropyrimidin-5-carboxylat aus Beispiel 38 erhielt man 48 mg (83 % d. Th.) der Titelverbindung.
LC-MS (Methode 1): Rₜ = 1.08 min; m/z = 557 (M+H)⁺.
¹H-NMR (400MHz, DMSO-d₆): δ [ppm] = 2.47 (s, 3H), 3.37 (s, 3H), 4.85 (q, 2H), 5.11 (s, 2H), 7.30 (dd, 1H), 7.33 - 7.45 (m, 3H), 7.47 - 7.50 (m, 1H), 7.59 - 7.63 (m, 1H), 8.49 (s, 1H), 12.73 (br.s, 1H).

### Beispiel 153

### 1-[1-(Cyclopropylmethyl)-3-methyl-2-oxo-2,3-dihydro-1H-benzimidazol-5-yl]-3-[2-methyl-3-(trifluormethyl)benzyl]-2,4-dioxo-1,2,3,4-tetrahydropyrimidin-5-carbonsäure

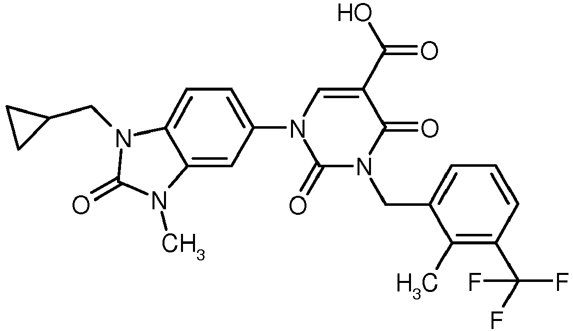

Darstellung und Reinigung der Titelverbindung erfolgten analog zu Beispiel 121. Ausgehend von 52 mg (0.09 mmol) Ethyl-1-[1-(cyclopropylmethyl)-3-methyl-2-oxo-2,3-dihydro-1H-benzimidazol-5-yl]-3-[2-methyl-3-(trifluormethyl)benzyl]-2,4-dioxo-1,2,3,4-tetrahydropyrimidin-5-carboxylat aus Beispiel 39 erhielt man nach Reinigung mittels HPLC (Methode 8) und zusätzlicher Feinreinigung der Verbindung mittels HPLC (Methode 23) 19 mg (37 % d. Th.) der Titelverbindung.
LC-MS (Methode 1): Rₜ = 1.09 min; m/z = 529 (M+H)⁺.
¹H-NMR (400MHz, DMSO-d₆): δ [ppm] = 0.36 - 0.42 (m, 2H), 0.44 - 0.50 (m, 2H), 1.15 - 1.25 (m, 1H), 2.47 (s, 3H), 3.35 (br.s, 3H, teilweise verdeckt durch Wasser-Signal), 3.77 (d, 2H), 5.11 (s, 2H), 7.24 (dd, 1H), 7.33 - 7.43 (m, 4H), 7.61 (d, 1H), 8.47 (s, 1H), 12.72 (s, 1H).

### Beispiel 154

### 3-[2-Chlor-3-(trifluormethyl)benzyl]-1-(1-methyl-1H-benzotriazol-5-yl)-2,4-dioxo-1,2,3,4-tetrahydro-pyrimidin-5-carbonsäure

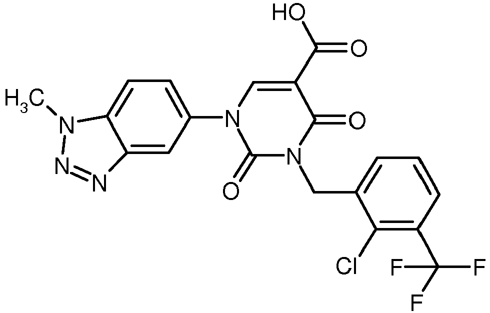

Darstellung und Reinigung der Titelverbindung erfolgten in Analogie zu Beispiel 121. Ausgehend von 149 mg (0.29 mmol) Ethyl-3-[2-chlor-3-(trifluormethyl)benzyl]-1-(1-methyl-1H-benzotriazol-5-yl)-2,4-dioxo-1,2,3,4-tetrahydropyrimidin-5-carboxylat aus Beispiel 71 erhielt man 115 mg (80 % d. Th.) der Titelverbindung.
LC-MS (Methode 1): Rₜ = 1.03 min; m/z = 480 (M+H)⁺.
¹H-NMR (400MHz, DMSO-d₆): δ [ppm] = 4.37 (s, 3H), 5.19 (s, 2H), 7.51 - 7.58 (m, 1H), 7.62 - 7.67 (m, 1H), 7.73 (dd, 1H), 7.79 - 7.84 (m, 1H), 8.00 (d, 1H), 8.30 - 8.33 (m, 1H), 8.62 (s, 1H), 12.76 (br.s, 1H).

### Beispiel 155

### 1-(1-Methyl-1H-benzotriazol-5-yl)-3-[2-methyl-3-(trifluormethyl)benzyl]-2,4-dioxo-1,2,3,4-tetrahydro-pyrimidin-5-carbonsäure

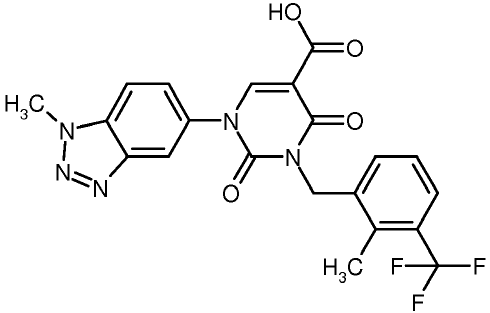

Darstellung und Reinigung der Titelverbindung erfolgten in Analogie zu Beispiel 121. Ausgehend von 151 mg (0.31 mmol) Ethyl-1-(1-methyl-1H-benzotriazol-5-yl)-3-[2-methyl-3-(trifluormethyl)benzyl]-2,4-dioxo-1,2,3,4-tetrahydropyrimidin-5-carboxylat aus Beispiel 72 erhielt man 124 mg (86 % d. Th.) der Titelverbindung.
LC-MS (Methode 1): Rₜ = 1.02 min; m/z = 460 (M+H)⁺.
¹H-NMR (400MHz, DMSO-d₆): δ [ppm] = 2.47 (s, 3H), 4.37 (s, 3H), 5.12 (s, 2H), 7.33 - 7.40 (m, 1H), 7.42 - 7.48 (m, 1H), 7.59 - 7.64 (m, 1H), 7.70 - 7.76 (m, 1H), 8.00 (d, 1H), 8.31 - 8.35 (m, 1H), 8.61 (s, 1H), 12.75 (br.s, 1H).

### Beispiel 156

### 3-(2,3-Dichlorbenzyl)-1-(-methyl-1H-benzotriazol-5-yl)-2,4-dioxo-1,2,3,4-tetrahydropyrimidin-5-carbonsäure

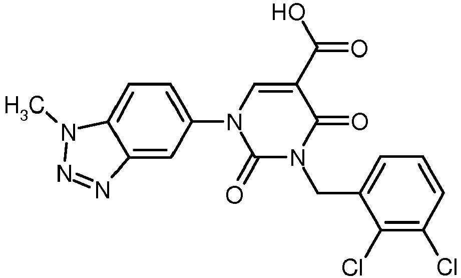

Darstellung und Reinigung der Titelverbindung erfolgten in Analogie zu Beispiel 121. Ausgehend von 188 mg (0.40 mmol) Ethyl-3-(2,3-dichlorbenzyl)-1-(1-methyl-1H-benzotriazol-5-yl)-2,4-dioxo-1,2,3,4-tetrahydro-pyrimidin-5-carboxylat aus Beispiel 73 erhielt man 143 mg (80 % d. Th.) der Titelverbindung.
LC-MS (Methode 1): Rₜ = 1.00 min; m/z = 446 (M+H)⁺.
¹H-NMR (400MHz, DMSO-d₆): δ [ppm] = 4.37 (s, 3H), 5.14 (s, 2H), 7.27 - 7.31 (m, 1H), 7.32 - 7.38 (m, 1H), 7.56 - 7.62 (m, 1H), 7.70 - 7.74 (m, 1H), 8.00 (d, 1H), 8.30 - 8.33 (m, 1H), 8.61 (s, 1H), 12.75 (br.s, 1H).

### Beispiel 157

### 3-[3-Chlor-2-(trifluormethyl)benzyl]-1-(1-methyl-1H-benzotriazol-5-yl)-2,4-dioxo-1,2,3,4-tetrahydro-pyrimidin-5-carbonsäure

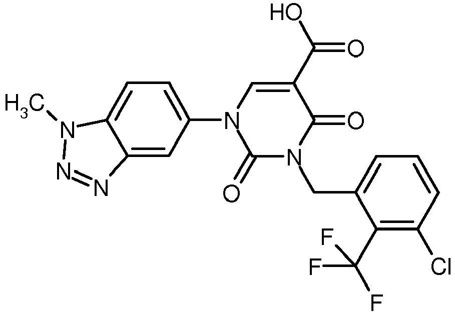

Darstellung und Reinigung der Titelverbindung erfolgten in Analogie zu Beispiel 121. Ausgehend von 115 mg (0.31 mmol) Ethyl-3-[3-chlor-2-(trifluormethyl)benzyl]-1-(1-methyl-1H-benzotriazol-5-yl)-2,4-dioxo-1,2,3,4-tetrahydropyrimidin-5-carboxylat aus Beispiel 74 erhielt man 92 mg (84 % d. Th.) der Titelverbindung. LC-MS (Methode 1): Rₜ = 1.03 min; m/z = 480 (M+H)⁺.
¹H-NMR (400MHz, DMSO-d₆): δ [ppm] = 4.37 (s, 3H), 5.22 - 5.28 (m, 2H), 7.37 - 7.42 (m, 1H), 7.58 - 7.68 (m, 2H), 7.69 - 7.74 (m, 1H), 8.00 (d, 1H), 8.29 - 8.33 (m, 1H), 8.63 (s, 1H), 12.75 (br.s, 1H).

### Beispiel 158

### 1-(1-Methyl-1H-indazol-5-yl)-3-[2-methyl-3-(trifluormethyl)benzyl]-2,4-dioxo-1,2,3,4-tetrahydro-pyrimidin-5-carbonsäure

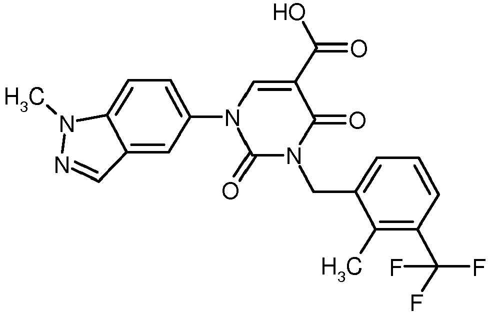

Darstellung und Reinigung der Titelverbindung erfolgten in Analogie zu Beispiel 121. Ausgehend von 254 mg (0.52 mmol) Ethyl-1-(1-methyl-1H-indazol-5-yl)-3-[2-methyl-3-(trifluormethyl)benzyl]-2,4-dioxo-1,2,3,4-tetrahydropyrimidin-5-carboxylat aus Beispiel 75 erhielt man 212 mg (88 % d. Th.) der Titelverbindung. LC-MS (Methode 1): Rₜ = 1.04 min; m/z = 459 (M+H)⁺.
¹H-NMR (400MHz, DMSO-d₆): δ [ppm] = 2.47 (s, 3H), 4.10 (s, 3H), 5.12 (s, 2H), 7.33 - 7.39 (m, 1H), 7.41 - 7.46 (m, 1H), 7.52 - 7.56 (m, 1H), 7.59 - 7.63 (m, 1H), 7.76 - 7.80 (m, 1H), 7.97 - 7.99 (m, 1H), 8.16 - 8.19 (m, 1H), 8.52 (s, 1H), 12.72 (br.s, 1H).

### Beispiel 159

### 3-[2-Chlor-3-(trifluormethyl)benzyl]-1-(1-methyl-1H-benzimidazol-6-yl)-2,4-dioxo-1,2,3,4-tetrahydro-pyrimidin-5-carbonsäure

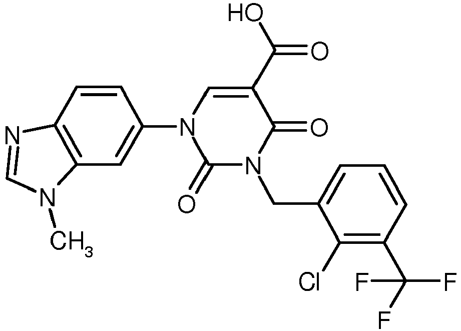

94 mg (0.18 mmol) Ethyl-3-[2-chlor-3-(trifluormethyl)benzyl]-1-(1-methyl-1H-benzimidazol-6-yl)-2,4-dioxo-1,2,3,4-tetrahydropyrimidin-5-carboxylat aus Beispiel 82 wurden in 1.5 ml Eisessig / konz. Salzsäure 2:1 (v/v) 30 min auf 120 °C gerührt. Das abgekühlte Reaktionsgemisch wurde mit Wasser versetzt, zweimal mit Dichlormethan extrahiert, die vereinigten organischen Phasen über Magnesiumsulfat getrocknet und eingeengt. Der Rückstand wurde mit Essigsäureethylester verrührt, der ausgefallene Feststoff abgesaugt und bei 50°C im Vakuum getrocknet. Man erhielt 88 mg (98 % d. Th.) der Titelverbindung.
LC-MS (Methode 3): Rₜ = 1.12 min; m/z = 479 (M+H)⁺.
¹H-NMR (400MHz, DMSO-d₆): δ [ppm] = 4.00 (s, 3H), 5.19 (s, 2H), 7.54 (t, 1H), 7.59 - 7.67 (m, 2H), 7.81 (d, 1H), 7.92 (d, 1H), 8.10 (s, 1H), 8.56 (s, 1H), 9.13 (s, 1H).

### Beispiel 160

### 1-(1-Methyl-1H-benzimidazol-5-yl)-3-[2-methyl-3-(trifluormethyl)benzyl]-2,4-dioxo-1,2,3,4-tetrahydro-pyrimidin-5-carbonsäure

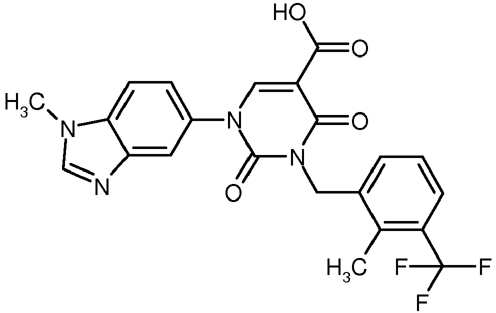

Darstellung und Reinigung der Titelverbindung erfolgten in Analogie zu Beispiel 121. Ausgehend von 170 mg (0.35 mmol) Ethyl-1-(1-methyl-1H-benzimidazol-5-yl)-3-[2-methyl-3-(trifluormethyl)benzyl]-2,4-dioxo-1,2,3,4-tetrahydropyrimidin-5-carboxylat aus Beispiel 77 erhielt man 124 mg (77 % d. Th.) der Titelverbindung.
LC-MS (Methode 1): Rₜ = 0.90 min; m/z = 459 (M+H)⁺.
¹H-NMR (400MHz, DMSO-d₆): δ [ppm] = 2.47 (s, 3H), 3.89 (s, 3H), 5.12 (s, 2H), 7.33 - 7.40 (m, 1H), 7.41 - 7.46 (m, 2H), 7.59 - 7.63 (m, 1H), 7.71 (d, 1H), 7.86 - 7.89 (m, 1H), 8.33 (s, 1H), 8.50 (s, 1H), 12.72 (br.s, 1H).

### Beispiel 161

### 3-[3-Chlor-2-(trifluormethyl)benzyl]-1-(1-methyl-1H-benzimidazol-5-yl)-2,4-dioxo-1,2,3,4-tetra-hydropyrimidin-5-carbonsäure

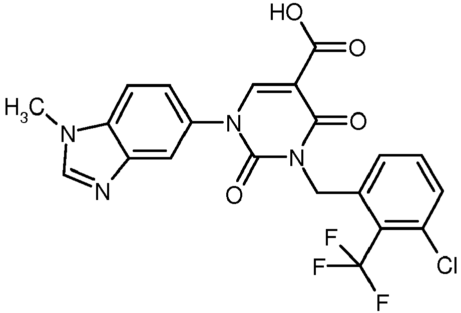

Darstellung und Reinigung der Titelverbindung erfolgten in Analogie zu Beispiel 121. Ausgehend von 82 mg (0.16 mmol) Ethyl-3-[3-chlor-2-(trifluormethyl)benzyl]-1-(1-methyl-1H-benzimidazol-5-yl)-2,4-dioxo-1,2,3,4-tetrahydropyrimidin-5-carboxylat aus Beispiel 78 erhielt man 52 mg (66 % d. Th.) der Titelverbindung.
LC-MS (Methode 1): Rₜ = 0.93 min; m/z = 479 (M+H)⁺.
¹H-NMR (400MHz, DMSO-d₆): δ [ppm] = 3.89 (s, 3H), 5.25 (br.s, 2H), 7.35 - 7.46 (m, 2H), 7.58 - 7.68 (m, 2H), 7.71 (d, 1H), 7.84 - 7.88 (m, 1H), 8.33 (s, 1H), 8.51 (s, 1H), 12.70 (br.s, 1H).

### Beispiel 162

### 3-[2-Chlor-3-(trifluormethyl)benzyl]-1-(1-methyl-1H-benzimidazol-5-yl)-2,4-dioxo-1,2,3,4-tetrahydro-pyrimidin-5-carbonsäure

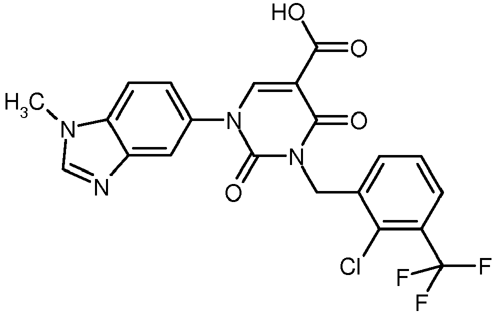

Darstellung und Reinigung der Titelverbindung erfolgten in Analogie zu Beispiel 121. Ausgehend von 120 mg (0.16 mmol) Ethyl-3-[2-chlor-3-(trifluormethyl)benzyl]-1-(1-methyl-1H-benzimidazol-5-yl)-2,4-dioxo-1,2,3,4-tetrahydropyrimidin-5-carboxylat aus Beispiel 79 erhielt man 83 mg (74 % d. Th.) der Titelverbindung.
LC-MS (Methode 1): Rₜ = 0.89 min; m/z = 479 (M+H)⁺.
¹H-NMR (400MHz, DMSO-d₆): δ [ppm] = 3.89 (s, 3H), 5.18 (s, 2H), 7.42 - 7.47 (m, 1H), 7.51 - 7.57 (m, 1H), 7.61 - 7.65 (m, 1H), 7.71 (d, 1H), 7.79 - 7.83 (m, 1H), 7.86 - 7.89 (m, 1H), 8.33 (s, 1H), 8.51 (s, 1H), 12.70 (br.s, 1H).

### Beispiel 163

### 1-(2-Carbamoyl-1-methyl-1H-benzimidazol-5-yl)-3-[2-methyl-3-(trifluormethyl)benzyl]-2,4-dioxo-1,2,3,4-tetrahydropyrimidin-5-carbonsäure

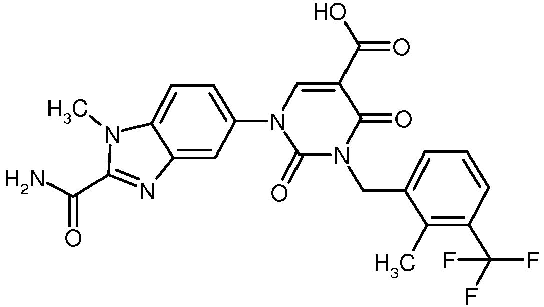

Darstellung und Reinigung der Titelverbindung erfolgten in Analogie zu Beispiel 121 mit einer Reaktionszeit von 3.5 h bei 60°C. Ausgehend von 116 mg (0.21 mmol) Ethyl-1-(2-carbamoyl-1-methyl-1H-benzimidazol-5-yl)-3-[2-methyl-3-(trifluormethyl)benzyl]-2,4-dioxo-1,2,3,4-tetrahydropyrimidin-5-carboxylat aus Beispiel 81 erhielt man nach zusätzlicher Reinigung mittels präparativer HPLC (Methode 9) 40 mg (36 % d. Th.) der Titelverbindung.
LC-MS (Methode 1): Rₜ = 0.96 min; m/z = 502 (M+H)⁺.
¹H-NMR (400MHz, DMSO-d₆): δ [ppm] = 2.47 (s, teilweise durch DMSO-Signal verdeckt), 4.16 (s, 3H), 5.13 (s, 2H), 7.34 - 7.40 (m, 1H), 7.42 - 7.47 (m, 1H), 7.54 - 7.58 (m, 1H), 7.59 - 7.64 (m, 1H), 7.83 (d, 1H), 7.90 - 7.94 (m, 1H), 7.96 - 7.99 (m, 1H), 8.30 - 8.35 (m, 1H), 8.53 (s, 1H), 12.73 (br.s, 1H).

### Beispiel 164

### 1-(1-Ethyl-2-methyl-1H-benzimidazol-5-yl)-3-[3-fluor-2-(trifluormethyl)benzyl]-2,4-dioxo-1,2,3,4-tetrahydro-pyrimidin-5-carbonsäure

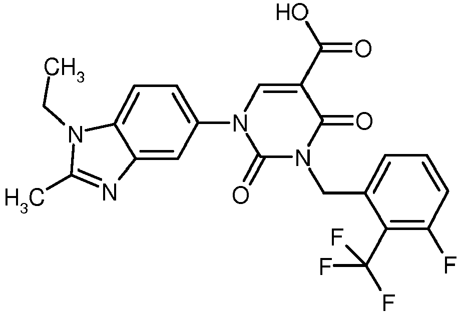

153 mg (0.30 mmol) Ethyl-1-(1-ethyl-2-methyl-1H-benzimidazol-5-yl)-3-[3-fluor-2-(trifluormethyl)benzyl]-2,4-dioxo-1,2,3,4-tetrahydropyrimidin-5-carboxylat aus Beispiel 83 wurden in 2.1 ml Eisessig und 1.1 ml konz. Salzsäure vorgelegt und bei 120°C für 1h gerührt. Anschließend versetzte man die auf RT abgekühlte Mischung mit Wasser und extrahierte dreimal mit Dichlormethan. Die vereinigten organischen Phasen wurden über Magnesiumsulfat getrocknet, filtriert und eingeengt. Um Rückstände von Essigsäure zu entfernen wurde der Rückstand mit Methanol/Dichlormethan verrührt, erneut eingeengt und im Vakuum getrocknet. Man erhielt 120 mg (81 % d. Th.) der Titelverbindung.
LC-MS (Methode 3): Rₜ = 1.02 min; m/z = 491 (M+H)⁺.
¹H-NMR (400MHz, DMSO-d₆): δ [ppm] = 1.36 (t, 3H), 2.72 (s, 3H), 4.38 (q, 2H), 5.23 (s, 2H), 7.23 - 7.28 (m, 1H), 7.38 - 7.45 (m, 1H), 7.51 - 7.57 (m, 1H), 7.64 - 7.71 (m, 1H), 7.86 - 7.93 (m, 2H), 8.54 (s, 1H), 12.71 (br.s, 1H).

### Beispiel 165

### 1-(1-Ethyl-1H-benzimidazol-5-yl)-3-[3-fluor-2-(trifluormethyl)benzyl]-2,4-dioxo-1,2,3,4-tetra-hydropyrimidin-5-carbonsäure

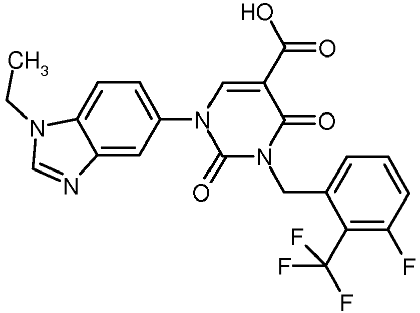

Darstellung und Reinigung der Titelverbindung erfolgten in Analogie zu Beispiel 121. Ausgehend von 73 mg (0.15 mmol) Ethyl-1-(1-ethyl-1H-benzimidazol-5-yl)-3-[3-fluor-2-(trifluormethyl)benzyl]-2,4-dioxo-1,2,3,4-tetrahydropyrimidin-5-carboxylat aus Beispiel 90 erhielt man 16 mg (23 % d. Th.) der Titelverbindung.
LC-MS (Methode 1): Rₜ = 0.89 min; m/z = 477 (M+H)⁺.
¹H-NMR (400MHz, DMSO-d₆): δ [ppm] = 1.43 (t, 3H), 4.33 (q, 2H), 5.23 (s, 2H), 7.21 - 7.27 (m, 1H), 7.37 - 7.45 (m, 2H), 7.63 - 7.71 (m, 1H), 7.76 (d, 1H), 7.84 - 7.88 (m, 1H), 8.40 (s, 1H), 8.49 (s, 1H), 12.70 (br.s, 1H).

### Beispiel 166

### 3-[2-Chlor-3-(trifluormethyl)benzyl]-1-(1-ethyl-1H-benzimidazol-5-yl)-2,4-dioxo-1,2,3,4-tetrahydropyrimidin-5-carbonsäure

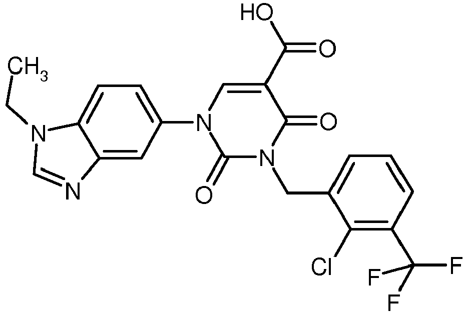

53 mg (0.10 mmol) Ethyl-3-[2-chlor-3-(trifluormethyl)benzyl]-1-(1-ethyl-1H-benzimidazol-5-yl)-2,4-dioxo-1,2,3,4-tetrahydropyrimidin-5-carboxylat aus Beispiel 91 wurden in 0.7 ml Eisessig und 0.4 ml konz. Salzsäure vorgelegt und bei 120°C für 1h gerührt. Anschließend versetzte man die auf RT abgekühlte Mischung mit Wasser und extrahierte dreimal mit Dichlormethan. Die vereinigten organischen Phasen wurden über Magnesiumsulfat getrocknet, filtriert und eingeengt. Der Rückstand wurde mittels präparativer HPLC (Methode 8) gereinigt. Man erhielt 37 mg (75 % d. Th.) der Titelverbindung.
LC-MS (Methode 1): Rₜ = 0.94 min; m/z = 493 (M+H)⁺.
¹H-NMR (400MHz, DMSO-d₆): δ [ppm] = 1.43 (t, 3H), 4.33 (q, 2H), 5.18 (s, 2H), 7.40 - 7.45 (m, 1H), 7.51 - 7.57 (m, 1H), 7.60 - 7.64 (m, 1H), 7.76 (d, 1H), 7.79 - 7.83 (m, 1H), 7.86 - 7.88 (m, 1H), 8.40 (s, 1H), 8.48 (s, 1H).

### Beispiel 167

### 1-(1-Ethyl-1H-benzimidazol-5-yl)-3-[2-methyl-3-(trifluormethyl)benzyl]-2,4-dioxo-1,2,3,4-tetrahydro-pyrimidin-5-carbonsäure

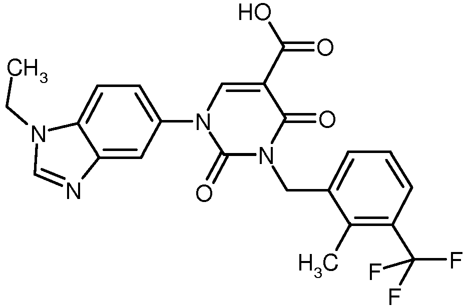

Darstellung und Reinigung der Titelverbindung erfolgten in Analogie zu Beispiel 166. Ausgehend von 38 mg (0.08 mmol) Ethyl-1-(1-ethyl-1H-benzimidazol-5-yl)-3-[2-methyl-3-(trifluormethyl)benzyl]-2,4-dioxo-1,2,3,4-tetrahydropyrimidin-5-carboxylat aus Beispiel 80 erhielt man 9 mg (25 % d. Th.) der Titelverbindung.
LC-MS (Methode 1): Rₜ = 0.94 min; m/z = 473 (M+H)⁺.
¹H-NMR (400MHz, DMSO-d₆): δ [ppm] = 1.43 (t, 3H), 2.47 (s, 3H), 4.33 (q, 2H), 5.12 (s, 2H), 7.33 - 7.39 (m, 1H), 7.40 - 7.45 (m, 2H), 7.59 - 7.63 (m, 1H), 7.76 (d, 1H), 7.86 - 7.88 (m, 1H), 8.40 (s, 1H), 8.46 (s, 1H).

### Beispiel 168

### 3-[2-Chlor-3-(trifluormethyl)benzyl]-1-(1-ethyl-2-methyl-1H-benzimidazol-5-yl)-2,4-dioxo-1,2,3,4-tetrahydro-pyrimidin-5-carbonsäure

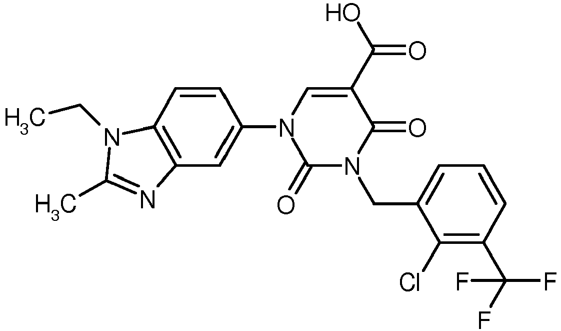

Darstellung und Reinigung der Titelverbindung erfolgten in Analogie zu Beispiel 166. Ausgehend von 114 mg (0.21 mmol) Ethyl-3-[2-chlor-3-(trifluormethyl)benzyl]-1-(1-ethyl-2-methyl-1H-benzimidazol-5-yl)-2,4-dioxo-1,2,3,4-tetrahydropyrimidin-5-carboxylat aus Beispiel 84 erhielt man 93 mg (83 % d. Th.) der Titelverbindung.
LC-MS (Methode 3): Rₜ = 1.09 min; m/z = 507 (M+H)⁺.
¹H-NMR (400MHz, DMSO-d₆): δ [ppm] = 1.34 (t, 3H), 2.66 (s, 3H), 4.33 (q, 2H), 5.18 (s, 2H), 7.43 - 7.49 (m, 1H), 7.51 - 7.57 (m, 1H), 7.61 - 7.65 (m, 1H), 7.76 - 7.85 (m, 3H), 8.51 (s, 1H), 12.70 (br.s, 1H).

### Beispiel 169

### 1-(1-Cyclohexyl-2-methyl-1H-benzimidazol-5-yl)-3-[3-fluor-2-(trifluormethyl)benzyl]-2,4-dioxo-1,2,3,4-tetrahydropyrimidin-5-carbonsäure

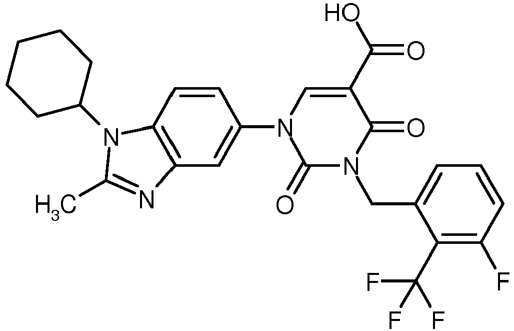

Darstellung und Reinigung der Titelverbindung erfolgten in Analogie zu Beispiel 166. Ausgehend von 90 mg (0.15 mmol) Ethyl-1-(1-cyclohexyl-2-methyl-1H-benzimidazol-5-yl)-3-[3-fluor-2-(trifluormethyl)benzyl]-2,4-dioxo-1,2,3,4-tetrahydropyrimidin-5-carboxylat aus Beispiel 85 erhielt man 74 mg (81 % d. Th.) der Titelverbindung.
LC-MS (Methode 1): Rₜ = 1.01 min; m/z = 545 (M+H)⁺.
¹H-NMR (400MHz, DMSO-d₆): δ [ppm] = 1.38 - 1.57 (m, 3H), 1.67 - 1.74 (m, 1H), 1.84 - 1.94 (m, 4H), 2.12 - 2.25 (m, 2H), 2.68 (s, 3H), 4.33 - 4.43 (m, 1H), 5.23 (s, 2H), 7.25 (d, 1H), 7.36 - 7.46 (m, 2H), 7.64 - 7.71 (m, 1H), 7.77 - 7.82 (m, 1H), 7.92 - 8.01 (m, 1H), 8.51 (s, 1H), 12.70 (br.s, 1H).

### Beispiel 170

### 1-(1-Isopropyl-1H-benzimidazol-5-yl)-3-[2-methyl-3-(trifluormethyl)benzyl]-2,4-dioxo-1,2,3,4-tetrahydro-pyrimidin-5-carbonsäure

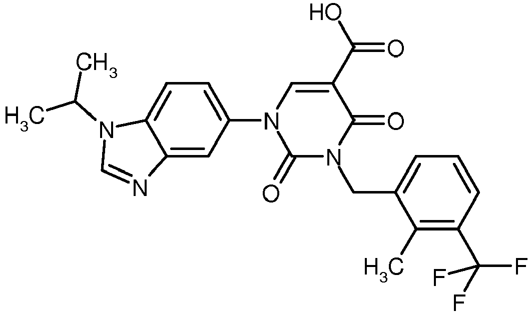

91 mg (0.18 mmol) Ethyl-1-(1-isopropyl-1H-benzimidazol-5-yl)-3-[2-methyl-3-(trifluormethyl)benzyl]-2,4-dioxo-1,2,3,4-tetrahydropyrimidin-5-carboxylat aus Beispiel 93 wurden in 1.3 ml Eisessig und 0.6 ml konz. Salzsäure vorgelegt und bei 120°C für 1h gerührt. Anschließend versetzte man die auf RT abgekühlte Mischung mit Wasser und extrahierte dreimal mit Dichlormethan. Die vereinigten organischen Phasen wurden über Magnesiumsulfat getrocknet, filtriert und eingeengt. Der Rückstand wurde mit Methanol verrührt, der Feststoff abfiltriert und im Vakuum getrocknet. Das Filtrat wurde erneut eingeengt und der Rückstand im Vakuum getrocknet. Man erhielt insgesamt 61 mg (70 % d. Th.) der Titelverbindung.
LC-MS (Methode 1): Rₜ = 1.01 min; m/z = 487 (M+H)⁺.
¹H-NMR (400MHz, DMSO-d₆): δ [ppm] = 1.56 (d, 6H), 2.47 (s, 3H), 4.78 - 4.87 (m, 1H), 5.12 (s, 2H), 7.33 - 7.39 (m, 1H), 7.40 - 7.46 (m, 2H), 7.59 - 7.63 (m, 1H), 7.80 (d, 1H), 7.87 - 7.90 (m, 1H), 8.48 - 8.52 (m, 2H), 12.71 (br.s, 1H).

### Beispiel 171

### 3-[2-Chlor-3-(trifluormethyl)benzyl]-1-(1-isopropyl-1H-benzimidazol-5-yl)-2,4-dioxo-1,2,3,4-tetrahydro-pyrimidin-5-carbonsäure

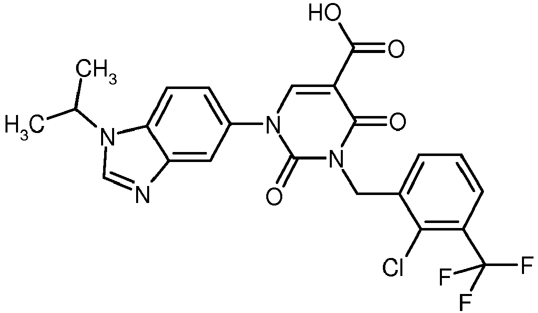

65 mg (0.12 mmol) Ethyl-3-[2-chlor-3-(trifluormethyl)benzyl]-1-(1-isopropyl-1H-benzimidazol-5-yl)-2,4-dioxo-1,2,3,4-tetrahydropyrimidin-5-carboxylat aus Beispiel 86 wurden in 0.9 ml Eisessig und 0.4 ml konz. Salzsäure vorgelegt und bei 120°C für 1h gerührt. Anschließend versetzte man die auf RT abgekühlte Mischung mit Wasser und extrahierte dreimal mit Dichlormethan. Die vereinigten organischen Phasen wurden über Magnesiumsulfat getrocknet, filtriert und eingeengt. Der Rückstand wurde mit Methanol verrührt, der Feststoff abfiltriert, mit Methanol gewaschen und im Vakuum getrocknet. Das Filtrat wurde eingeengt und der Rückstand im Vakuum getrocknet. Man erhielt insgesamt 44 mg (71 % d. Th.) der Titelverbindung.
LC-MS (Methode 1): Rₜ = 1.01 min; m/z = 507 (M+H)⁺.
¹H-NMR (400MHz, DMSO-d₆): δ [ppm] = 1.55 (s, 3H), 1.57 (s, 3H), 4.78 - 4.87 (m, 1H), 5.18 (s, 2H), 7.39 - 7.45 (m, 1H), 7.51 - 7.57 (m, 1H), 7.61 - 7.66 (m, 1H), 7.78 - 7.83 (m, 2H), 7.86 - 7.89 (m, 1H), 8.50 (s, 1H), 8.52 (s, 1H), 12.70 (br.s, 1H).

### Beispiel 172

### 3-(2,3-Dichlorbenzyl)-1-(1-isopropyl-1H-benzimidazol-5-yl)-2,4-dioxo-1,2,3,4-tetrahydro-pyrimidin-5-carbonsäure

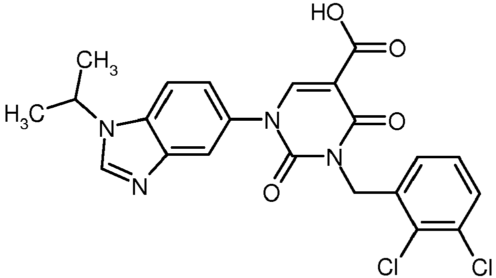

Darstellung und Reinigung der Titelverbindung erfolgten in Analogie zu Beispiel 171. Ausgehend von 83 mg (0.17 mmol) Ethyl-3-(2,3-dichlorbenzyl)-1-(1-isopropyl-1H-benzimidazol-5-yl)-2,4-dioxo-1,2,3,4-tetrahydro-pyrimidin-5-carboxylat aus Beispiel 87 erhielt man 59 mg (72 % d. Th.) der Titelverbindung.
LC-MS (Methode 1): Rₜ = 0.99 min; m/z = 473 (M+H)⁺.
¹H-NMR (400MHz, DMSO-d₆): δ [ppm] = 1.56 (d, 6H), 4.78 - 4.86 (m, 1H), 5.14 (s, 2H), 7.26 - 7.30 (m, 1H), 7.32 - 7.38 (m, 1H), 7.40 - 7.44 (m, 1H), 7.57 - 7.61 (m, 1H), 7.80 (d, 1H), 7.86 - 7.89 (m, 1H), 8.48 - 8.52 (m, 2H), 12.70 (br.s, 1H).

### Beispiel 173

### 3-[3-Chlor-2-(trifluormethyl)benzyl]-1-[1-(cyclopropylmethyl)-1H-benzimidazol-5-yl]-2,4-dioxo-1,2,3,4-tetrahydropyrimidin-5-carbonsäure

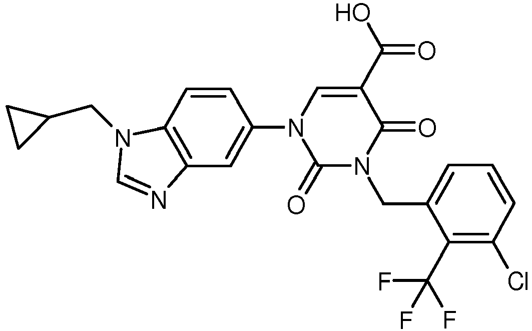

Darstellung und Reinigung der Titelverbindung erfolgten in Analogie zu Beispiel 171. Ausgehend von 135 mg (0.24 mmol) Ethyl-3-[3-chlor-2-(trifluormethyl)benzyl]-1-[1-(cyclopropylmethyl)-1H-benzimidazol-5-yl]-2,4-dioxo-1,2,3,4-tetrahydropyrimidin-5-carboxylat aus Beispiel 92 erhielt man 59 mg (45 % d. Th.) der Titelverbindung.
LC-MS (Methode 1): Rₜ = 1.00 min; m/z = 519 (M+H)⁺.
¹H-NMR (400MHz, DMSO-d₆): δ [ppm] = 0.41 - 0.48 (m, 2H), 0.52 - 0.58 (m, 2H), 1.27 - 1.36 (m, 1H), 4.17 (d, 2H), 5.25 (s, 2H), 7.36 - 7.44 (m, 2H), 7.58 - 7.68 (m, 2H), 7.81 (d, 1H), 7.85 - 7.89 (m, 1H), 8.43 (s, 1H), 8.53 (s, 1H), 12.70 (br.s, 1H).

### Beispiel 174

### 3-[2-Chlor-3-(trifluormethyl)benzyl]-1-(1-cyclobutyl-1H-benzimidazol-5-yl)-2,4-dioxo-1,2,3,4-tetrahydro-pyrimidin-5-carbonsäure

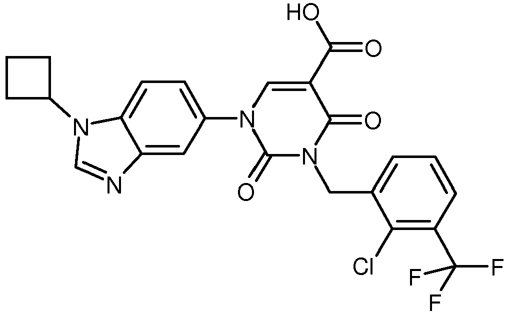

Darstellung und Reinigung der Titelverbindung erfolgten in Analogie zu Beispiel 171. Ausgehend von 153 mg (0.25 mmol) Ethyl-3-[2-chlor-3-(trifluormethyl)benzyl]-1-(1-cyclobutyl-1H-benzimidazol-5-yl)-2,4-dioxo-1,2,3,4-tetrahydropyrimidin-5-carboxylat aus Beispiel 88 erhielt man 90 mg (68 % d. Th.) der Titelverbindung.
LC-MS (Methode 1): Rₜ = 1.02 min; m/z = 519 (M+H)⁺.
¹H-NMR (400MHz, DMSO-d₆): δ [ppm] = 1.86 - 1.96 (m, 2H), 2.5 - 2.6 (m, teilweise durch DMSO-Signal verdeckt), 4.99 - 5.09 (m, 1H), 5.18 (s, 2H), 7.40 - 7.45 (m, 1H), 7.50 - 7.57 (m, 1H), 7.60 - 7.66 (m, 1H), 7.75 (d, 1H), 7.79 - 7.83 (m, 1H), 7.87 - 7.90 (m, 1H), 8.51 (s, 1H), 8.55 (s, 1H), 12.71 (br.s, 1H).

### Beispiel 175

### 3-[2-Chlor-3-(trifluorniethyl)benzyl]-1-(1-methyl-1H-indazol-5-yl)-2,4-dioxo-1,2,3,4-tetrahydro-pyrimidin-5-carbonsäure

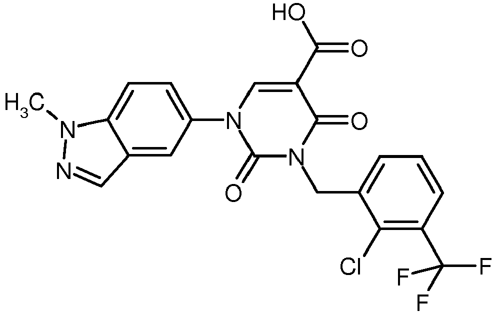

Darstellung und Reinigung der Titelverbindung erfolgten in Analogie zu Beispiel 121. Ausgehend von 228 mg (0.45 mmol) Ethyl-3-[2-chlor-3-(trifluormethyl)benzyl]-1-(1-methyl-1H-indazol-5-yl)-2,4-dioxo-1,2,3,4-tetrahydropyrimidin-5-carboxylat aus Beispiel 89 erhielt man 170 mg (77 % d. Th.) der Titelverbindung.
LC-MS (Methode 1): Rₜ = 1.04 min; m/z = 479 (M+H)⁺.
¹H-NMR (400MHz, DMSO-d₆): δ [ppm] = 4.10 (s, 3H), 5.18 (s, 2H), 7.51 - 7.57 (m, 2H), 7.61 - 7.66 (m, 1H), 7.76 - 7.83 (m, 2H), 7.96 - 7.99 (m, 1H), 8.18 (s, 1H), 8.54 (s, 1H), 12.71 (br.s, 1H).

### Beispiel 176

### 1-(1-Methyl-1H-indazol-5-yl)-2,4-dioxo-3-[(1R)-4-(trifluormethyl)-2,3-dihydro-1H-inden-1-yl]-1,2,3,4-tetrahydropyrimidin-5-carbonsäure (R-Enantiomer)

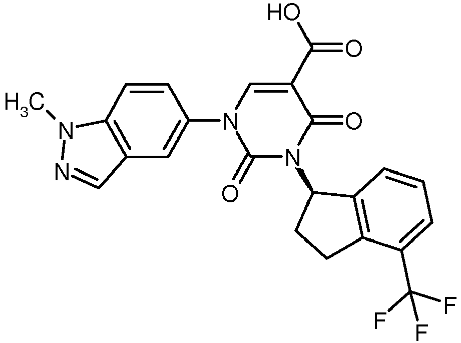

Darstellung und Reinigung der Titelverbindung erfolgten analog zu Beispiel 121 mit 45 min Reaktionszeit. Ausgehend von 119 mg (0.24 mmol) Ethyl-1-(1-methyl-1H-indazol-5-yl)-2,4-dioxo-3-[4-(trifluormethyl)-2,3-dihydro-1H-inden-l-yl]-1,2,3,4-tetrahydropyrimidin-5-carboxylat aus Beispiel 76 erhielt man 81 mg (71 % d. Th.) der Titelverbindung.
LC-MS (Methode 1): Rₜ = 1.08 min; m/z = 471 (M+H)⁺.
¹H-NMR (400MHz, CDCl₃): δ [ppm] = 2.45 - 2.54 (m, 1H), 2.65 - 2.72 (m, 1H), 3.13 - 3.24 (m, 1H), 3.48 - 3.61 (m, 1H), 4.12 (s, 3H), 6.62 - 6.71 (m, 1H), 7.28 - 7.35 (m, 3H), 7.48 - 7.56 (m, 2H), 7.70 (s, 1H), 8.06 (s, 1H), 8.63 (s, 1H), 12.53 (s, 1H).

### Beispiel 177

### 1-(1,3-Dimethyl-2,2-dioxido-1,3-dihydro-2,1,3-benzothiadiazol-5-yl)-3-[2-methyl-3-(trifluor-methyl)benzyl]-2,4-dioxo-1,2,3,4-tetrahydropyrimidin-5-carbonsäure

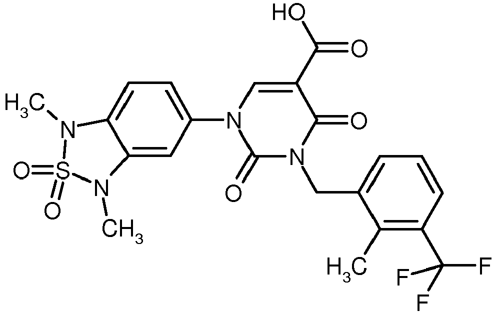

Darstellung und Reinigung der Titelverbindung erfolgten in Analogie zu Beispiel 121. Ausgehend von 195 mg (0.35 mmol) Ethyl-1-(1,3-dimethyl-2,2-dioxido-1,3-dihydro-2,1,3-benzothiadiazol-5-yl)-3-[2-methyl-3-(trifluormethyl)benzyl]-2,4-dioxo-1,2,3,4-tetrahydropyrimidin-5-carboxylat aus Beispiel 94 erhielt man 153 mg (79 % d. Th.) der Titelverbindung.
LC-MS (Methode 1): Rₜ = 1.11 min; m/z = 525 (M+H)⁺.
¹H-NMR (400MHz, DMSO-d₆): δ [ppm] = 2.47 (s, 3H), 3.26 (s, 3H), 3.30 (s, teilweise durch Wasser-Signal verdeckt), 5.10 (s, 2H), 7.13 - 7.17 (m, 1H), 7.20 - 7.24 (m, 1H), 7.26 - 7.30 (m, 1H), 7.32 - 7.42 (m, 2H), 7.59-7.63 (m, 1H), 8.48 (s, 1H), 12.74 (br.s, 1H).

### Beispiel 178

### 3-[3-Chlor-2-(trifluormethyl)benzyl]-1-(1,3-dimethyl-2,2-dioxido-1,3-dihydro-2,1,3-benzothia-diazol-5-yl)-2,4-dioxo-1,2,3,4-tetrahydropyrimidin-5-carbonsäure

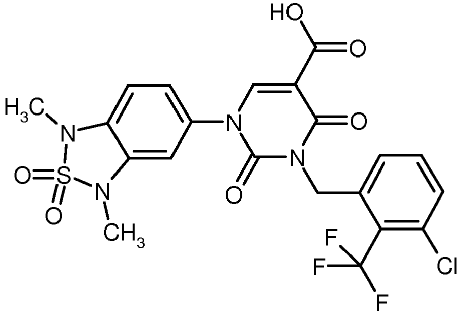

Darstellung und Reinigung der Titelverbindung erfolgten in Analogie zu Beispiel 121. Ausgehend von 120 mg (0.21 mmol) Ethyl-3-[3-chlor-2-(trifluormethyl)benzyl]-1-(1,3-dimethyl-2,2-dioxido-1,3-dihydro-2,1,3-benzothiadiazol-5-yl)-2,4-dioxo-1,2,3,4-tetrahydropyrimidin-5-carboxylat aus Beispiel 95 erhielt man 98 mg (85 % d. Th.) der Titelverbindung.
LC-MS (Methode 1): Rₜ = 1.11 min; m/z = 545 (M+H)⁺.
¹H-NMR (400MHz, DMSO-d₆): δ [ppm] = 3.27 (s, teilweise durch DMSO-Signal verdeckt), 3.30 (s, 3H), 5.23 (s, 2H), 7.13 - 7.18 (m, 1H), 7.18 - 7.23 (m, 1H), 7.24 - 7.27 (m, 1H), 7.31 - 7.36 (m, 1H), 7.56 - 7.67 (m, 2H), 8.50 (s, 1H), 12.73 (br.s, 1H).

### Beispiel 179

### 3-[2-Chlor-3-(trifluormethyl)benzyl]-1-(1,3-dimethyl-2,2-dioxido-1,3-dihydro-2,1,3-benzo-thiadiazol-5-yl)-2,4-dioxo-1,2,3,4-tetrahydropyrimidin-5-carbonsäure

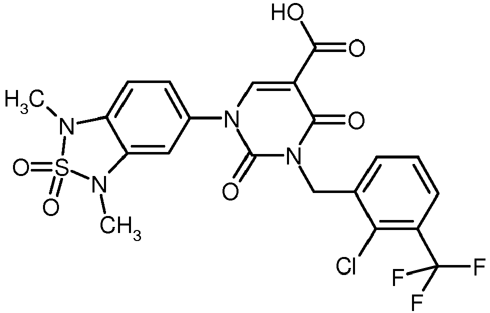

Darstellung und Reinigung der Titelverbindung erfolgten in Analogie zu Beispiel 121. Ausgehend von 167 mg (0.29 mmol) Ethyl-3-[2-chlor-3-(trifluormethyl)benzyl]-1-(1,3-dimethyl-2,2-dioxido-1,3-dihydro-2,1,3-benzothiadiazol-5-yl)-2,4-dioxo-1,2,3,4-tetrahydropyrimidin-5-carboxylat aus Beispiel 96 erhielt man 129 mg (80 % d. Th.) der Titelverbindung.
LC-MS (Methode 1): Rₜ = 1.12 min; m/z = 545 (M+H)⁺.
¹H-NMR (400MHz, DMSO-d₆): δ [ppm] = 3.27 (s, 3H), 3.30 (s, 3H), 5.17 (s, 2H), 7.13 - 7.18 (m, 1H), 7.20 - 7.24 (m, 1H), 7.25 - 7.28 (m, 1H), 7.50 - 7.56 (m, 1H), 7.56 - 7.61 (m, 1H), 7.79 - 7.83 (m, 1H), 8.49 (s, 1H), 12.74 (br.s, 1H).

### Beispiel 180

### 1-(1,3-Dimethyl-2,2-dioxido-1,3-dihydro-2,1,3-benzothiadiazol-5-yl)-2,4-dioxo-3-[(1R)-4-(trifluormethyl)-2,3-dihydro-1H-inden-1-yl]-1,2,3,4-tetrahydropyrimidin-5-carbonsäure (R-Enantiomer)

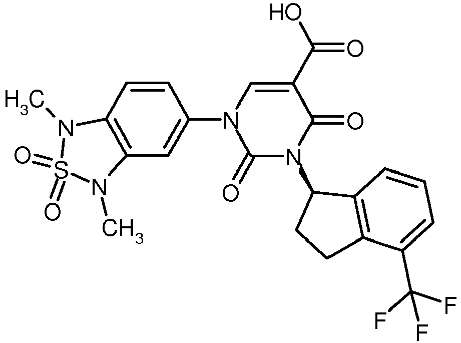

Darstellung und Reinigung der Titelverbindung erfolgten analog zu Beispiel 121. Ausgehend von 130 mg (0.23 mmol) Ethyl-1-(1,3-dimethyl-2,2-dioxido-1,3-dihydro-2,1,3-benzothiadiazol-5-yl)-2,4-dioxo-3-[4-(trifluor-methyl)-2,3-dihydro-1H-inden-1-yl]-1,2,3,4-tetrahydropyrimidin-5-carboxylat aus Beispiel 97 erhielt man nach zusätzlicher Reinigung mittels präparativer HPLC (Methode 8) 50 mg (40 % d. Th.) der Titelverbindung.
LC-MS (Methode 4): Rₜ = 2.44 min; m/z = 537 (M+H)⁺.
¹H-NMR (400MHz, CD₂Cl₂): δ [ppm] = 2.32 - 2.44 (m, 1H), 2.51 - 2.63 (m, 1H), 3.04 - 3.16 (m, 1H), 3.20 (s, 3H), 3.23 (s, 3H), 3.36 - 3.47 (m, 1H), 6.51 - 6.60 (m, 1H), 6.65 (s, 1H), 6.75 (d, 1H), 6.89 (d, 1H), 7.21 - 7.30 (m, 2H), 7.42 - 7.49 (m, 1H), 8.44 (s, 1H).

### Beispiel 181

### 3-[2-Chlor-3-(trifluormethyl)benzyl]-1-(1-methyl-2-oxo-2,3-dihydro-1H-benzimidazol-5-yl)-2,4-dioxo-1,2,3,4-tetrahydropyrimidin-5-carbonsäure

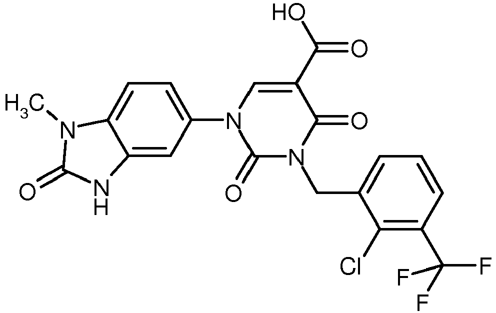

Darstellung und Reinigung der Titelverbindung erfolgten in Analogie zu Beispiel 121. Ausgehend von 20 mg (0.04 mmol) Ethyl-3-[2-chlor-3-(trifluormethyl)benzyl]-1-(1-methyl-2-oxo-2,3-dihydro-1H-benzimidazol-5-yl)-2,4-dioxo-1,2,3,4-tetrahydropyrinidin-5-carboxylat aus Beispiel 16 erhielt man 14 mg (71 % d. Th.) der Titelverbindung.
LC-MS (Methode 3): Rₜ = 1.16 min; m/z =495 (M+H)⁺.
¹H-NMR (400MHz, DMSO-d₆): δ [ppm] = 3.30 (s, 3H), 5.16 (s, 2H), 7.17 - 7.23 (m, 3H), 7.49 - 7.55 (m, 1H), 7.60 - 7.64 (m, 1H), 7.78 - 7.83 (m, 1H), 8.44 (s, 1H), 11.14 (s, 1H), 12.69 (br.s, 1H).

### Beispiel 182

### 1-(1-Methyl-2-oxo-2,3-dihydro-1H-benzimidazol-5-yl)-3-[2-methyl-3-(trifluormethyl)benzyl]-2,4-dioxo-1,2,3,4-tetrahydropyrimidin-5-carbonsäure

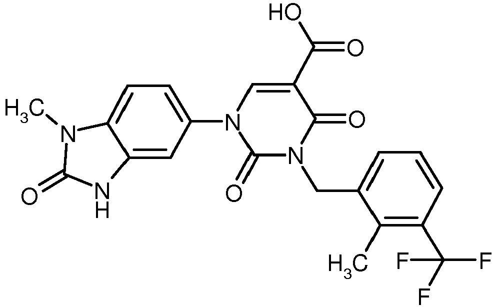

Darstellung und Reinigung der Titelverbindung erfolgten in Analogie zu Beispiel 121. Ausgehend von 129 mg (0.26 mmol) Ethyl-1-(1-methyl-2-oxo-2,3-dihydro-1H-benzimidazol-5-yl)-3-[2-methyl-3-(trifluormethyl)-benzyl]-2,4-dioxo-1,2,3,4-tetrahydropyrinidin-5-carboxylat aus Beispiel 14 erhielt man 113 mg (93 % d. Th.) der Titelverbindung.
LC-MS (Methode 1): Rₜ = 0.94 min; m/z = 475 (M+H)⁺.
¹H-NMR (400MHz, DMSO-d₆): δ [ppm] = 2.46 (s, 3H), 3.32 (s, 3H), 5.10 (s, 2H), 7.17 - 7.23 (m, 3H), 7.32 - 7.38 (m, 1H), 7.40 - 7.45 (m, 1H), 7.58 - 7.63 (m, 1H), 8.43 (s, 1H), 11.14 (s, 1H), 12.70 (br.s, 1H).

### Beispiel 183

### 3-(2,3 -Dichlorbenzyl)-1-(1-methyl-2-oxo-2,3-dihydro-1H-benzimidazol-5-yl)-2,4-dioxo-1,2,3,4-tetrahydropyrimidin-5-carbonsäure

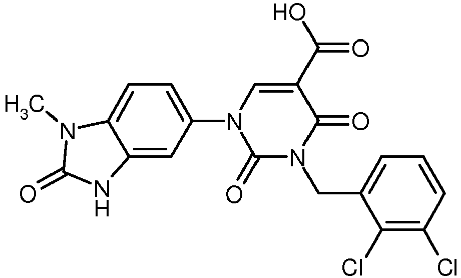

Darstellung und Reinigung der Titelverbindung erfolgten in Analogie zu Beispiel 121. Ausgehend von 30 mg (0.06 mmol) Ethyl-3-(2,3-dichlorbenzyl)-1-(1-methyl-2-oxo-2,3-dihydro-1H-benzimidazol-5-yl)-2,4-dioxo-1,2,3,4-tetrahydropyrimidin-5-carboxylat aus Beispiel 15 erhielt man 22 mg (73 % d. Th.) der Titelverbindung.
LC-MS (Methode 1): Rₜ = 0.91 min; m/z = 461 (M+H)⁺.
¹H-NMR (400MHz, DMSO-d₆): δ [ppm] = 3.30 (s, teilweise verdeckt durch Wasser-Signal), 5.12 (s, 2H), 7.20 (s, 3H), 7.25 - 7.29 (m, 1H), 7.30 - 7.36 (m, 1H), 7.56 - 7.61 (m, 1H), 8.43 (s, 1H), 11.14 (s, 1H), 12.69 (br.s, 1H).

### Beispiel 184

### Ethyl-1-(6-methyl-2-oxo-2,3-dihydro-1H-benzimidazol-5-yl)-3-[2-methyl-3-(trifluormethyl)benzyl]-2,4-dioxo-1,2,3,4-tetrahydropyrimidin-5-carboxylat

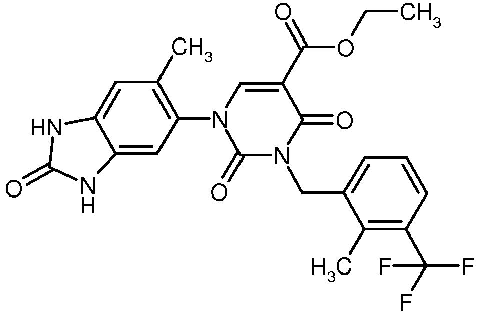

Darstellung und Reinigung der Titelverbindung erfolgten analog zu Beispiel 1. Ausgehend von 200 mg (0.60 mmol) Ethyl-1-(6-methyl-2-oxo-2,3-dihydro-1H-benzimidazol-5-yl)-2,4-dioxo-1,2,3,4-tetrahydropyrimidin-5-carboxylat aus Beispiel 27A und 168 mg (0.66 mmol) 1-(Brommethyl)-2-methyl-3-(trifluormethyl)benzol erhielt man nach zusätzlicher Umkristallisation aus Ethanol 207 mg (62 % d. Th.) der Titelverbindung.
LC-MS (Methode 1): Rₜ = 0.98 min; m/z = 503 (M+H)⁺.
¹H-NMR (400MHz, DMSO-d₆): δ [ppm] = 1.22 (t, 3H), 2.09 (s, 3H), 2.45 (s, 3H), 4.18 (q, 2H), 5.08 (d, 2H), 6.89 (s, 1H), 7.12 (s, 1H), 7.30 - 7.36 (m, 2H), 7.57 - 7.62 (m, 1H), 8.37 (s, 1H), 10.80 (s, 1H).

### Beispiel 185

### Ethyl-3-[2-chlor-3-(trifluormethyl)benzyl]-1-(6-methyl-2-oxo-2,3-dihydro-1H-benzimidazol-5-yl)-2,4-dioxo-1,2,3,4-tetrahydropyrimidin-5-carboxylat

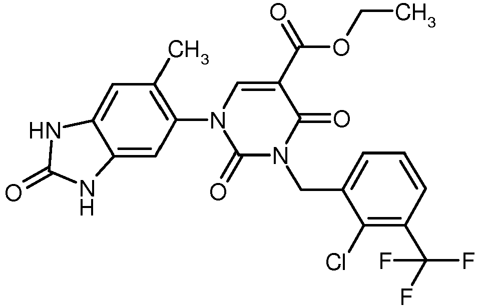

Die Darstellung der Titelverbindung erfolgte analog zu Beispiel 37. Ausgehend von 200 mg (0.60 mmol) Ethyl-1-(6-methyl-2-oxo-2,3-dihydro-1H-benziniidazol-5-yl)-2,4-dioxo-1,2,3,4-tetrahydropyrimidin-5-carboxylat aus Beispiel 27A und 182 mg (0.66 mmol) 1-(Brommethyl)-2-chlor-3-(trifluormethyl)benzol erhielt man nach Reinigung mittels präparativer HPLC (Methode 8) 178 mg (56 % d. Th.) der Titelverbindung.
LC-MS (Methode 1): Rₜ = 1.01 min; m/z = 523 (M+H)⁺.
¹H-NMR (400MHz, DMSO-d₆): δ [ppm] = 1.23 (t, 3H), 2.10 (s, 3H), 4.19 (q, 2H), 5.15 (s, 2H), 6.89 (s, 1H), 7.11 (s, 1H), 7.49 - 7.57 (m, 2H), 7.77 - 7.83 (m, 1H), 8.39 (s, 1H), 10.80 (s, 2H).

### Beispiel 186

### 3-[2-Chlor-3-(trifluormethyl)benzyl]-2,4-dioxo-1-(2-oxo-2,3-dihydro-1H-benzimidazol-5-yl)-1,2,3,4-tetrahydropyrimidin-5-carbonsäure

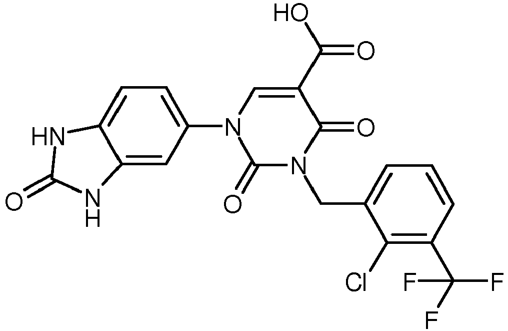

Darstellung und Reinigung der Titelverbindung erfolgten analog zu Beispiel 121 mit einer Reaktionszeit von 1h. Ausgehend von 99 mg (0.19 mmol) Ethyl-3-[2-chlor-3-(trifluormethyl)benzyl]-2,4-dioxo-1-(2-oxo-2,3-dihydro-1H-benzimidazol-5-yl)-1,2,3,4-tetrahydropyrimidin-5-carboxylat aus Beispiel 37 erhielt man 79 mg (85 % d. Th.) der Titelverbindung.
LC-MS (Methode 1): Rₜ = 0.89 min; m/z = 481 (M+H)⁺.
¹H-NMR (400MHz, DMSO-d₆): δ [ppm] = 5.16 (s, 2H), 7.00 - 7.04 (m, 1H), 7.07 - 7.11 (m, 1H), 7.13 - 7.16 (m, 1H), 7.49 - 7.55 (m, 1H), 7.59 - 7.64 (m, 1H), 7.78 - 7.83 (m, 1H), 8.43 (s, 1H), 10.87 - 10.92 (m, 2H), 12.68 (br.s, 1H).

### Beispiel 187

### 1-(3-Methyl-2-oxo-2,3-dihydro-1,3-benzoxazol-6-yl)-3-[2-methyl-3-(trifluormethyl)benzyl]-2,4-dioxo-1,2,3,4-tetrahydropyrimidin-5-carbonsäure

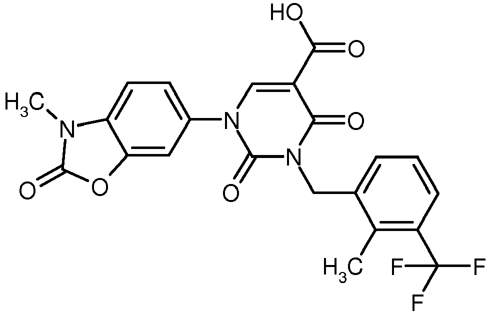

Darstellung und Reinigung der Titelverbindung erfolgten analog zu Beispiel 121 mit einer Reaktionszeit von 1h. Ausgehend von 280 mg (0.55 mmol) Ethyl-1-(3-methyl-2-oxo-2,3-dihydro-1,3-benzoxazol-6-yl)-3-[2-methyl-3-(trifluormethyl)benzyl]-2,4-dioxo-1,2,3,4-tetrahydropyrimidin-5-carboxylat aus Beispiel 40 erhielt man 186 mg (69 % d. Th.) der Titelverbindung.
LC-MS (Methode 5): Rₜ = 1.06 min; m/z = 476 (M+H)⁺.
¹H-NMR (400MHz, DMSO-d₆): δ [ppm] = 2.47 (s, 3H), 3.38 (s, 3H), 5.10 (s, 2H), 7.32 - 7.47 (m, 4H), 7.58 - 7.66 (m, 2H), 8.47 (s, 1H), 12.74 (br.s, 1H).

### Beispiel 188

### 3-[2-Chlor-3-(trifluormethyl)benzyl]-1-(3-methyl-2-oxo-2,3-dihydro-1,3-benzoxazol-6-yl)-2,4-dioxo-1,2,3,4-tetrahydropyrimidin-5-carbonsäure

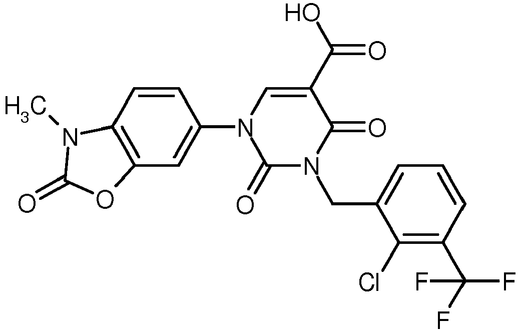

Darstellung und Reinigung der Titelverbindung erfolgten analog zu Beispiel 121 mit einer Reaktionszeit von 1h. Ausgehend von 250 mg (0.47 mmol) Ethyl-3-[2-chlor-3-(trifluormethyl)benzyl]-1-(3-methyl-2-oxo-2,3-dihydro-1,3-benzoxazol-6-yl)-2,4-dioxo-1,2,3,4-tetrahydropyrimidin-5-carboxylat aus Beispiel 41 erhielt man 220 mg (91 % d. Th.) der Titelverbindung.
LC-MS (Methode 5): Rₜ = 1.07 min; m/z = 496 (M+H)⁺.
¹H-NMR (400MHz, DMSO-d₆): δ [ppm] = 3.38 (s, 3H), 5.16 (s, 2H), 7.38 - 7.47 (m, 2H), 7.50 - 7.56 (m, 1H), 7.58 - 7.64 (m, 2H), 7.78 - 7.83 (m, 1H), 8.48 (s, 1H), 12.74 (br.s, 1H).

### Beispiel 189

### 1-(3-Methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carbonsäure (R-Enantiomer)

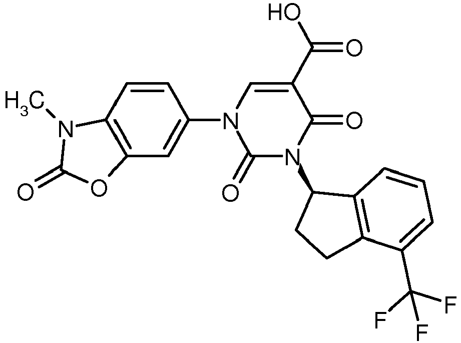

3.40 g (6.60 mmol) der Verbindung aus Beispiel 42 wurden in 44 ml Eisessig und 22 ml konzentrierter Salzsäure 1 h bei Rückflusstemperatur gerührt. Nach leichter Abkühlung (ca. 60 °C) wurde das Gemisch vollständig im Vakuum eingeengt. Der amorphe Rückstand wurde mit 50 mal Isopropanol versetzt und 15 min zum Rückfluss erhitzt, wobei sich ein Feststoff bildete. Die Suspension wurde anschließend auf 10°C gekühlt und dann der Feststoff abgesaugt. Der Feststoff wurde zweimal mit je 15 mal Isopropanol gewaschen, abgesaugt und im HV getrocknet. Man erhielt 2.53 g (79 % d. Th.) der Titelverbindung.
LC-MS (Methode 1): Rₜ = 1.12 min; m/z = 488 (M+H)⁺.
Chirale analytische HPLC (Methode 14): Rₜ= 13.3 min; ca. 99 % ee
¹H-NMR (400MHz, CD₂Cl₂): δ [ppm]= 2.40 - 2.52 (m, 1H), 2.59 - 2.72 (m, 1H), 3.12 - 3.25 (m, 1H), 3.41 (s, 3H), 3.44 - 3.56 (m, 1H), 6.58 - 6.69 (m, 1H), 7.04 - 7.11 (m, 1H), 7.15 - 7.21 (m, 1H), 7.24 (br.s, 1H), 7.29 - 7.38 (m, 2H), 7.53 (s, 1H), 8.54 (s, 1H), 12.39 (br. s, 1H).
Spezifischer Drehwert α_{D}²⁰ = +135.3° (Methanol, c = 0.43).
In einem analogen Experiment wurde der spezifischer Drehwert des Produkts in Chloroform gemessen: α_{D} ²⁰ = +159.5° (Chloroform, c = 0.395).
Eine Röntgenstrukturanalyse im Komplex mit Chymase bestätigte für dieses Enantiomer die R-Konfiguration.

### Beispiel 190

### 1-(3-Methyl-2-oxo-2,3-dihydro-1,3-benzoxazol-6-yl)-2,4-dioxo-3-[(1S)-4-(trifluormethyl)-2,3-dihydro-1H-inden-l-yl]-1,2,3,4-tetrahydropyrimidin-5-carbonsäure (S-Enantiomer)

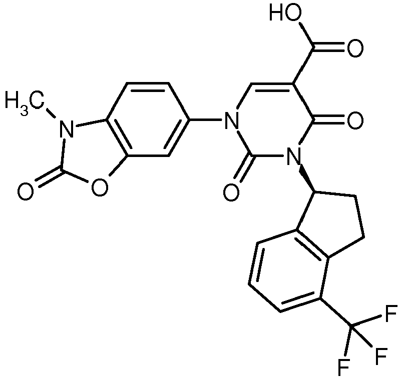

420 mg (0.80 mmol) der Verbindung aus Beispiel 43 wurden in 7.7 ml Eisessig / konz. Salzsäure 2:1 (v/v) 1 h zum Rückfluss erhitzt. Anschließend wurde das Reaktionsgemisch am Rotationsverdampfer eingeengt und der Rückstand am HV getrocknet. Man erhielt 390 mg (96 % d. Th.) der Titelverbindung.
LC-MS (Methode 1): Rₜ = 1.09 min; m/z = 488 (M+H)⁺.
¹H-NMR (400MHz, CD₂Cl₂): δ [ppm]= 2.37 - 2.53 (m, 1H), 2.66 (dtd, 1H), 3.10 - 3.26 (m, 1H), 3.41 (s, 3H), 3.44 - 3.55 (m, 1H), 6.58 - 6.71 (m, 1H), 7.08 (d, 1H), 7.19 (br. d, 1H), 7.24 (br. s, 1H), 7.30 - 7.38 (m, 2H), 7.50 - 7.59 (m, 1H), 8.55 (s, 1H).
Chirale analytische HPLC (Methode 14): Rₜ = 9.97 min, ca. 95 % ee.
Spezifischer Drehwert: α_{D}²⁰ = -122.5° (c = 0.5, Methanol).

### Beispiel 191

### 3-(2,3-Dichlorbenzyl)-1-(3-ethyl-2-oxo-2,3-dihydro-1,3-benzoxazol-6-yl)-2,4-dioxo-1,2,3,4-tetrahydropyrimidin-5-carbonsäure

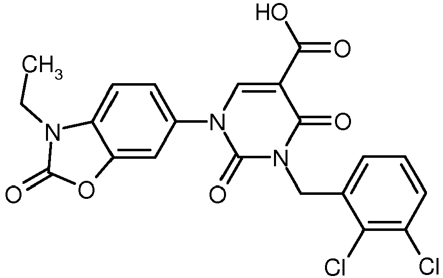

Darstellung und Reinigung der Titelverbindung erfolgten in Analogie zu Beispiel 121. Ausgehend von 210 mg (0.42 mmol) Ethyl-3-(2,3-dichlorbenzyl)-1-(3-ethyl-2-oxo-2,3-dihydro-1,3-benzoxazol-6-yl)-2,4-dioxo-1,2,3,4-tetrahydropyrimidin-5-carboxylat aus Beispiel 45 erhielt man 180 mg (89 % d. Th.) der Titelverbindung.
LC-MS (Methode 1): Rₜ = 1.08 min; m/z = 476 (M+H)⁺.
¹H-NMR (400MHz, DMSO-d₆): δ [ppm] = 1.28 (t, 3H), 3.90 (q, 2H), 5.12 (s, 2H), 7.23 - 7.28 (m, 1H), 7.31 - 7.36 (m, 1H), 7.47 (s, 2H), 7.57 - 7.61 (m, 1H), 7.62 - 7.65 (m, 1H), 8.48 (s, 1H), 12.70 (br.s, 1H).

### Beispiel 192

### 1-(3-Ethyl-2-oxo-2,3-dihydro-1,3-benzoxazol-6-yl)-3-[2-methyl-3-(trifluormethyl)benzyl]-2,4-dioxo-1,2,3,4-tetrahydropyrimidin-5-carbonsäure

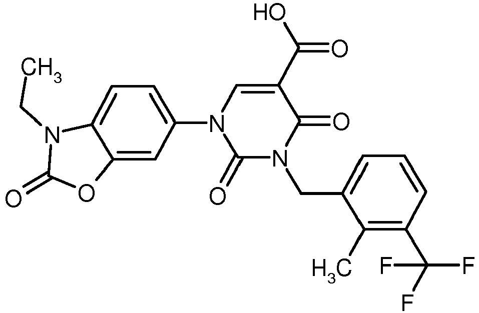

Darstellung und Reinigung der Titelverbindung erfolgten in Analogie zu Beispiel 121. Ausgehend von 185 mg (0.36 mmol) Ethyl-1-(3-ethyl-2-oxo-2,3-dihydro-1,3-benzoxazol-6-yl)-3-[2-methyl-3-(trifluormethyl)benzyl]-2,4-dioxo-1,2,3,4-tetrahydropyrimidin-5-carboxylat aus Beispiel 44 erhielt man 159 mg (90 % d. Th.) der Titelverbindung.
LC-MS (Methode 1): Rₜ = 1.10 min; m/z = 490 (M+H)⁺.
¹H-NMR (400MHz, DMSO-d₆): δ [ppm] = 1.28 (t, 3H), 2.47 (s, 3H), 3.90 (q, 2H), 5.10 (s, 2H), 7.32 - 7.38 (m, 1H), 7.39 - 7.50 (m, 3H), 7.59 - 7.63 (m, 1H), 7.64 - 7.66 (m, 1H), 8.48 (s, 1H), 12.72 (br.s, 1H).

### Beispiel 193

### 3-[2-Chlor-3-(trifluormethyl)benzyl]-1-(3-ethyl-2-oxo-2,3-dihydro-1,3-benzoxazol-6-yl)-2,4-dioxo-1,2,3,4-tetrahydropyrimidin-5-carbonsäure

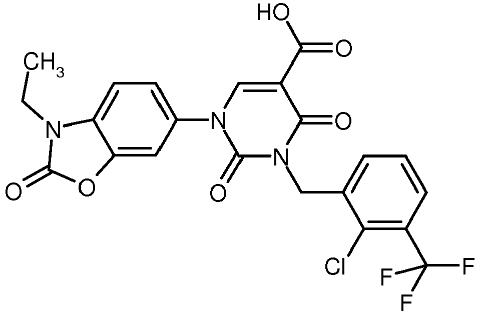

Darstellung und Reinigung der Titelverbindung erfolgten in Analogie zu Beispiel 121. Ausgehend von 200 mg (0.37 mmol) Ethyl-3-[2-chlor-3-(trifluormethyl)benzyl]-1-(3-ethyl-2-oxo-2,3-dihydro-1,3-benzoxazol-6-yl)-2,4-dioxo-1,2,3,4-tetrahydropyrimidin-5-carboxylat aus Beispiel 46 erhielt man 165 mg (85 % d. Th.) der Titelverbindung.
LC-MS (Methode 1): Rₜ = 1.10 min; m/z = 510 (M+H)⁺.
¹H-NMR (400MHz, DMSO-d₆): δ [ppm] = 1.28 (d, 3H), 3.90 (q, 2H), 5.17 (s, 2H), 7.42 - 7.56 (m, 3H), 7.58 - 7.65 (m, 2H), 7.78 - 7.83 (m, 1H), 8.49 (s, 1H), 12.73 (br.s, 1H).

### Beispiel 194

### 1-(3-Ethyl-2-oxo-2,3-dihydro-1,3-benzoxazol-6-yl)-3-[3-fluor-2-(trifluormethyl)benzyl]-2,4-dioxo-1,2,3,4-tetrahydropyrimidin-5-carbonsäure

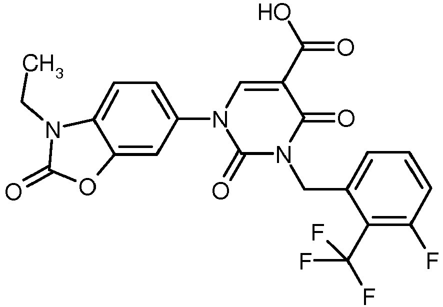

Darstellung und Reinigung der Titelverbindung erfolgten in Analogie zu Beispiel 121. Ausgehend von 150 mg (0.29 mmol) Ethyl-1-(3-ethyl-2-oxo-2,3-dihydro-1,3-benzoxazol-6-yl)-3-[3-fluor-2-(trifluormethyl)benzyl]-2,4-dioxo-1,2,3,4-tetrahydropyrimidin-5-carboxylat aus Beispiel 47 erhielt man 105 mg (73 % d. Th.) der Titelverbindung.
LC-MS (Methode 1): Rₜ = 1.05 min; m/z = 494 (M+H)⁺.
¹H-NMR (400MHz, DMSO-d₆): δ [ppm] = 1.28 (t, 3H), 3.90 (q, 2H), 5.21 (s, 2H), 7.19 - 7.25 (m, 1H), 7.38 - 7.51 (m, 3H), 7.61 - 7.70 (m, 2H), 8.50 (s, 1H), 12.72 (br.s, 1H).

### Beispiel 195

### 1-(3-Methyl-2-oxo-2,3-dihydro-1,3-benzothiazol-6-yl)-3-[2-methyl-3-(trifluormethyl)benzyl]-2,4-dioxo-1,2,3,4-tetrahydropyrimidin-5-carbonsäure

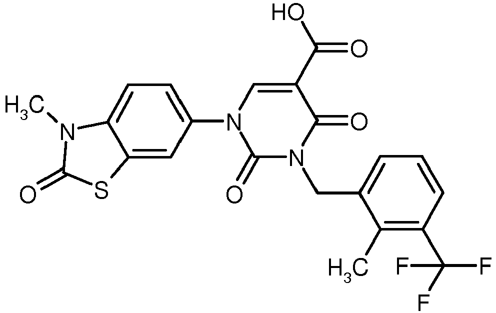

Darstellung und Reinigung der Titelverbindung erfolgten in Analogie zu Beispiel 121. Ausgehend von 390 mg (0.75 mmol) Ethyl-1-(3-methyl-2-oxo-2,3-dihydro-1,3-benzothiazol-6-yl)-3-[2-methyl-3-(trifluormethyl)-benzyl]-2,4-dioxo-1,2,3,4-tetrahydropyrimidin-5-carboxylat aus Beispiel 48 erhielt man 314 mg (81 % d. Th.) der Titelverbindung.
LC-MS (Methode 3): Rₜ = 1.33 min; m/z = 492 (M+H)⁺.
¹H-NMR (400MHz, DMSO-d₆): δ [ppm] = 2.47 (s, 3H), 3.45 (s, 3H), 5.10 (s, 2H), 7.32 - 7.38 (m, 1H), 7.39 - 7.43 (m, 1H), 7.46 (d, 1H), 7.60 (s, 2H), 7.89 (d, 1H), 8.52 (s, 1H), 12.73 (br.s, 1H).

### Beispiel 196

### 3-[2-Chlor-3-(trifluormethyl)benzyl]-1-(3-methyl-2-oxo-2,3-dihydro-1,3-benzothiazol-6-yl)-2,4-dioxo-1,2,3,4-tetrahydropyrimidin-5-carbonsäure

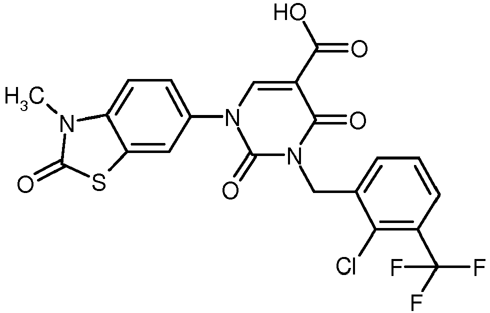

Darstellung und Reinigung der Titelverbindung erfolgten in Analogie zu Beispiel 121. Ausgehend von 216 mg (0.40 mmol) Ethyl-3-[2-chlor-3-(trifluormethyl)benzyl]-1-(3-methyl-2-oxo-2,3-dihydro-1,3-benzothiazol-6-yl)-2,4-dioxo-1,2,3,4-tetrahydropyrimidin-5-carboxylat aus Beispiel 49 erhielt man 155 mg (72 % d. Th.) der Titelverbindung.
LC-MS (Methode 3): Rₜ = 1.33 min; m/z = 512 (M+H)⁺.
¹H-NMR (400MHz, DMSO-d₆): δ [ppm] = 3.45 (s, 3H), 5.17 (s, 2H), 7.46 (d, 1H), 7.49 - 7.64 (m, 3H), 7.78 - 7.84 (m, 1H), 7.89 (d, 1H), 8.53 (s, 1H), 12.73 (br.s, 1H).

### Beispiel 197

### 3-[3-Fluor-2-(trifluormethyl)benzyl]-1-(3-methyl-2-oxo-2,3-dihydro-1,3-benzothiazol-6-yl)-2,4-dioxo-1,2,3,4-tetrahydropyrimidin-5-carbonsäure

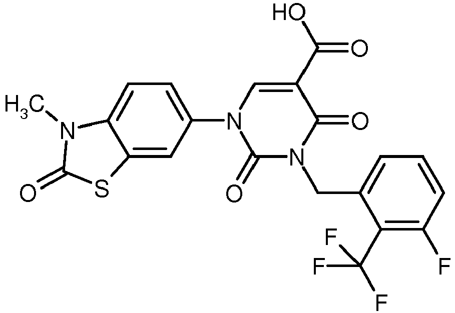

Darstellung und Reinigung der Titelverbindung erfolgten in Analogie zu Beispiel 121. Ausgehend von 241 mg (0.46 mmol) Ethyl-3-[3-fluor-2-(trifluormethyl)benzyl]-1-(3-methyl-2-oxo-2,3-dihydro-1,3-benzothiazol-6-yl)-2,4-dioxo-1,2,3,4-tetrahydropyrimidin-5-carboxylat aus Beispiel 50 erhielt man 180 mg (73 % d. Th.) der Titelverbindung.
LC-MS (Methode 3): Rₜ = 1.27 min; m/z = 496 (M+H)⁺.
¹H-NMR (400MHz, DMSO-d₆): δ [ppm] = 3.45 (s, 3H), 5.21 (s, 2H), 7.19 - 7.25 (m, 1H), 7.37 - 7.49 (m, 2H), 7.55 - 7.60 (m, 1H), 7.62 - 7.70 (m, 1H), 7.86 - 7.90 (m, 1H), 8.53 (s, 1H), 12.73 (br.s, 1H).

### Beispiel 198

### 1-(1,3-Benzothiazol-6-yl)-3-[2-methyl-3-(trifluormethyl)benzyl]-2,4-dioxo-1,2,3,4-tetrahydro-pyrimidin-5-carbonsäure

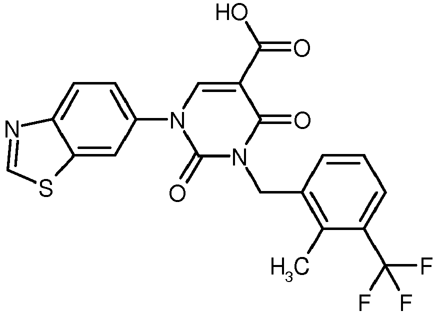

Darstellung und Reinigung der Titelverbindung erfolgten in Analogie zu Beispiel 121. Ausgehend von 204 mg (0.41 mmol) Ethyl-1-(1,3-benzothiazol-6-yl)-3-[2-methyl-3-(trifluormethyl)benzyl]-2,4-dioxo-1,2,3,4-tetrahydropyrimidin-5-carboxylat aus Beispiel 98 erhielt man 160 mg (82 % d. Th.) der Titelverbindung.
LC-MS (Methode 1): Rₜ = 1.07 min; m/z = 462 (M+H)⁺.
¹H-NMR (400MHz, DMSO-d₆): δ [ppm] = 2.47 (teilweise durch DMSO-Signal verdeckt), 5.12 (s, 2H), 7.33 - 7.39 (m, 1H), 7.42 - 7.47 (m, 1H), 7.58 - 7.64 (m, 1H), 7.70 - 7.75 (m, 1H), 8.22 (d, 1H), 8.39 - 8.43 (m, 1H), 8.61 (s, 1H), 9.54 (s, 1H), 12.75 (br.s, 1H).

### Beispiel 199

### 1-[3-Hydroxy-1-methyl-2-oxo-3-(trifluormethyl)-2,3-dihydro-1H-indol-5-yl]-3-[2-methyl-3-(trifluormethyl)benzyl]-2,4-dioxo-1,2,3,4-tetrahydropyrimidin-5-carbonsäure (Racemat)

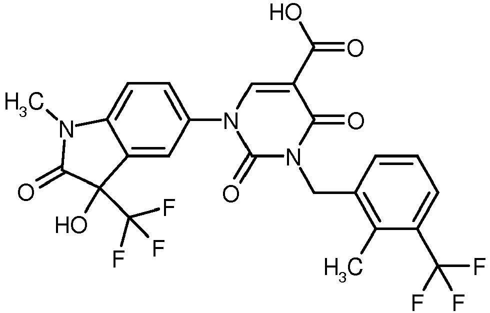

Darstellung und Reinigung der Titelverbindung erfolgten in Analogie zu Beispiel 121 mit einer Reaktionszeit von 20 min. Ausgehend von 74 mg (0.12 mmol) Ethyl-1-[3-hydroxy-1-methyl-2-oxo-3-(trifluormethyl)-2,3-dihydro-1H-indol-5-yl]-3-[2-methyl-3-(trifluormethyl)benzyl]-2,4-dioxo-1,2,3,4-tetrahydropyrimidin-5-carboxylat aus Beispiel 63 erhielt man 37 mg (53 % d. Th.) der Titelverbindung.
LC-MS (Methode 5): Rₜ = 1.09 min; m/z = 558 (M+H)⁺.
¹H-NMR (400MHz, DMSO-d₆): δ [ppm] = 2.46 (s, 3H), 3.22 (s, 3H), 5.10 (s, 2H), 7.29 (d, 1H), 7.32 - 7.38 (m, 1H), 7.41 - 7.45 (m, 1H), 7.58 - 7.63 (m, 1H), 7.66 - 7.73 (m, 2H), 7.92 (s, 1H), 8.44 (s, 1H), 12.74 (br. s, 1H).

### Beispiel 200

### 1-[3-Fluor-1-methyl-2-oxo-3-(trifluormethyl)-2,3-dihydro-1H-indol-5-yl]-3-[2-methyl-3-(trifluormethyl)benzyl]-2,4-dioxo-1,2,3,4-tetrahydropyrimidin-5-carbonsäure Racemat)

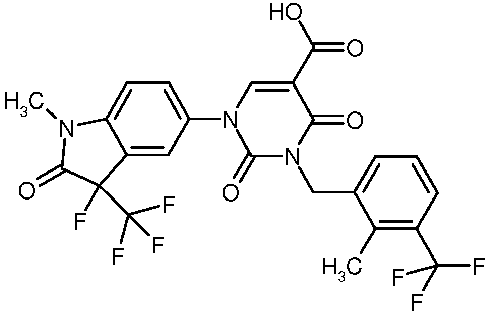

Darstellung und Reinigung der Titelverbindung erfolgten in Analogie zu Beispiel 121. Ausgehend von 85 mg (0.14 mmol) Ethyl-1-[3-fluor-1-methyl-2-oxo-3-(trifluormethyl)-2,3-dihydro-1H-indol-5-yl]-3-[2-methyl-3-(trifluormethyl)benzyl]-2,4-dioxo-1,2,3,4-tetrahydropyrimidin-5-carboxylat aus Beispiel 64 erhielt man 66 mg (82 % d. Th.) der Titelverbindung.
LC-MS (Methode 1): Rₜ = 1.18 min; m/z = 560 (M+H)⁺.
¹H-NMR (400MHz, DMSO-d₆): δ [ppm] = 2.46 (s, teilweise durch DMSO-Signal verdeckt), 3.26 (s, teilweise durch Wasser-Signal verdeckt), 5.10 (s, 2H), 7.31 - 7.38 (m, 1H), 7.39 - 7.45 (m, 2H), 7.60 (d, 1H), 7.82 - 7.88 (m, 1H), 7.95 (s, 1H), 8.57 (s, 1H), 12.75 (br.s, 1H).

### Beispiel 201

### 3-[2-Chlor-3-(trifluormethyl)benzyl]-1-(3-hydroxy-1,3-dimethyl-2-oxo-2,3-dihydro-1H-indol-5-yl)-2,4-dioxo-1,2,3,4-tetrahydropyrimidin-5-carbonsäure (Racemat)

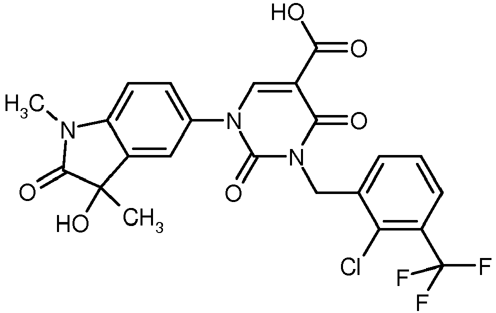

Darstellung und Reinigung der Titelverbindung erfolgten in Analogie zu Beispiel 121 mit einer Reaktionszeit von 45 min. Ausgehend von 124 mg (0.22 mmol) Ethyl-3-[2-chlor-3-(trifluormethyl)benzyl]-1-(3-hydroxy-1,3-dimethyl-2-oxo-2,3-dihydro-1H-indol-5-yl)-2,4-dioxo-1,2,3,4-tetrahydropyrimidin-5-carboxylat aus Beispiel 62 erhielt man 54 mg (50 % d. Th.) der Titelverbindung, sowie 24 mg (22 % d. Th.) von Beispiel 202 beschrieben ist. Analytische Daten für die Titelverbindung:
LC-MS (Methode 1): Rₜ = 0.95 min; m/z = 524 (M+H)⁺.
¹H-NMR (400MHz, DMSO-d₆): δ [ppm] = 1.41 (s, 3H), 3.14 (s, 3H), 5.16 (s, 2H), 6.12 (s, 1H), 7.14 (d, 1H), 7.47 - 7.57 (m, 3H), 7.59 - 7.64 (m, 1H), 7.78 - 7.83 (m, 1H), 8.42 (s, 1H), 12.73 (br. s, 1H).

### Beispiel 202

### 1-(3-Chlor-1,3-dimethyl-2-oxo-2,3-dihydro-1H-indol-5-yl)-3-[2-chlor-3-(trifluormethyl)benzyl]-2,4-dioxo-1,2,3,4-tetrahydropyrimidin-5-carbonsäure

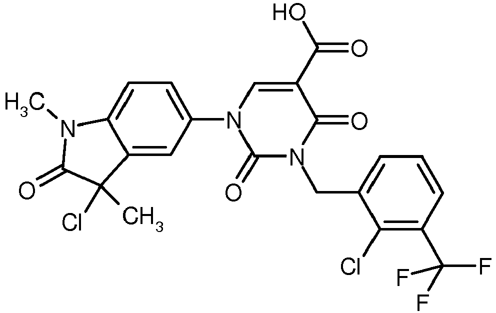

Die Titelverbindung (24 mg) wurde bei der Synthese von Beispiel 201 isoliert.
LC-MS (Methode 1): Rₜ = 1.09 min; m/z = 542 (M+H)⁺.
¹H-NMR (400MHz, DMSO-d₆): δ [ppm] = 1.87 (s, 3H), 3.22 (s, 3H), 5.17 (s, 2H), 7.26 (d, 1H), 7.51 - 7.56 (m, 1H), 7.57 - 7.64 (m, 2H), 7.77 - 7.84 (m, 2H), 8.48 (s, 1H), 12.75 (br. s, 1H).

### Beispiel 203

### 3-[2-Methyl-3-(trifluormethyl)benzyl]-2,4-dioxo-1-(2-oxo-2,3-dihydro-1H-benzimidazol-5-yl)-1,2,3,4-tetrahydropyrimidin-5-carbonsäure

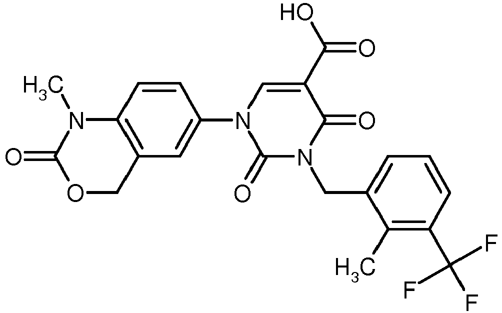

Darstellung und Reinigung der Titelverbindung erfolgten analog zu Beispiel 121 mit einer Reaktionszeit von 15 min. Ausgehend von 265 mg (0.51 mmol) Ethyl-1-(1-methyl-2-oxo-1,4-dihydro-2H-3,1-benzoxazin-6-yl)-3-[2-methyl-3-(trifluormethyl)benzyl]-2,4-dioxo-1,2,3,4-tetrahydropyrimidin-5-carboxylat aus Beispiel 100 erhielt man nach Reinigung mittels präparativer HPLC (Methode 8) 121 mg (46 % d. Th.) der Titelverbindung.
LC-MS (Methode 1): Rₜ = 1.03 min; m/z = 490 (M+H)⁺.
¹H-NMR (400MHz, DMSO-d₆): δ [ppm] = 2.47 (s, 3H), 3.3 (durch Wasser-Signal verdeckt), 5.10 (s, 2H), 5.30 (s, 2H), 7.21 - 7.26 (m, 1H), 7.31 - 7.43 (m, 2H), 7.45 - 7.48 (m, 1H), 7.53 - 7.62 (m, 2H), 8.46 (s, 1H), 12.72 (br.s, 1H).

### Beispiel 204

### 1-(1-Methyl-2-oxo-1,4-dihydro-2H-3,1-benzoxazin-6-yl)-2,4-dioxo-3-[(1R)-4-(trifluormethyl)-2,3-dihydro-1H-inden-1-yl]-1,2,3,4-tetrahydropyrimidin-5-carbonsäure (R-Enantiomer)

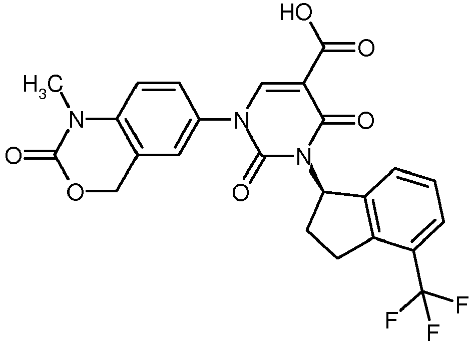

Darstellung und Reinigung der Titelverbindung erfolgten analog zu Beispiel 121 mit 45 min Reaktionszeit. Ausgehend von 135 mg (0.25 mmol) Ethyl-1-(1-methyl-2-oxo-1,4-dihydro-2H-3,1-benzoxazin-6-yl)-2,4-dioxo-3-[4-(trifluormethyl)-2,3-dihydro-1H-inden-1-yl]-1,2,3,4-tetrahydropyrimidin-5-carboxylat aus Beispiel 101 erhielt man 81 mg (59 % d. Th.) der Titelverbindung.
LC-MS (Methode 1): Rₜ = 1.04 min; m/z = 502 (M+H)⁺.
¹H-NMR (400MHz, CDCl₃): δ [ppm] = 2.39 - 2.52 (m, 1H), 2.64 - 2.71 (m, 1H), 3.12 - 3.25 (m, 1H), 3.41 (s, 3H), 3.47 - 3.61 (m, 1H), 5.22 (s, 2H), 6.60 - 6.70 (m, 1H), 7.05 (d, 1H), 7.15 (s, 1H), 7.28 - 7.36 (m, 3H), 7.50 - 7.57 (m, 1H), 8.53 (s, 1H), 12.18 - 12.70 (m, 2H).

### Beispiel 205

### 3-[2-Methyl-3-(trifluormethyl)benzyl]-2,4-dioxo-1-(1,3,3-trimethyl-2-oxo-2,3-dihydro-1H-indol-6-yl)-1,2,3,4-tetrahydropyrimidin-5-carbonsäure

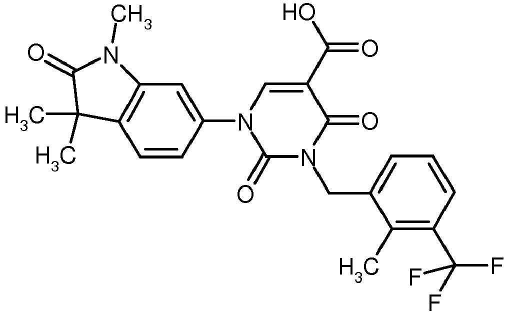

Darstellung und Reinigung der Titelverbindung erfolgten in Analogie zu Beispiel 121 mit einer Reaktionszeit von 45 min. Ausgehend von 127 mg (0.24 mmol) Ethyl-3-[2-methyl-3-(trifluormethyl)benzyl]-2,4-dioxo-1-(1,3,3-trimethyl-2-oxo-2,3-dihydro-1H-indol-6-yl)-1,2,3,4-tetrahydropyrimidin-5-carboxylat aus Beispiel 59 erhielt man nach zusätzlicher Reinigung mittels präparativer HPLC (Methode 8) 76 mg (63 % d. Th.) der Titelverbindung.
LC-MS (Methode 1): Rₜ = 1.08 min; m/z = 502 (M+H)⁺.
¹H-NMR (400MHz, DMSO-d₆): δ [ppm] = 1.30 (s, 6H), 2.47 (s, 3H), 3.14 (s, 3H), 5.11 (s, 2H), 7.19 - 7.24 (m, 1H), 7.25 - 7.28 (m, 1H), 7.32 - 7.43 (m, 2H), 7.51 (d, 1H), 7.61 (d, 1H), 8.48 (s, 1H), 12.74 (br.s, 1H).

### Beispiel 206

### 3-[2-Chlor-3-(trifluormethyl)benzyl]-2,4-dioxo-1-(1,3,3-trimethyl-2-oxo-2,3-dihydro-1H-indol-6-yl)-1,2,3,4-tetrahydropyrimidin-5-carbonsäure

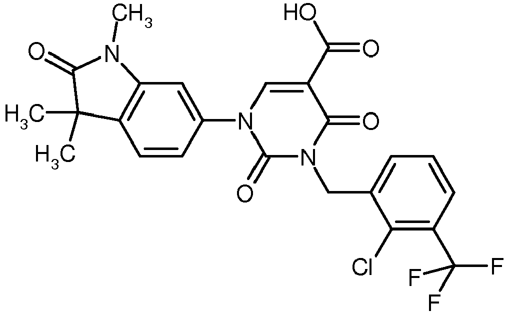

Darstellung und Reinigung der Titelverbindung erfolgten in Analogie zu Beispiel 121 mit einer Reaktionszeit von 2 h, wobei zur Aufarbeitung MTBE durch Cyclohexan ersetzt wurde. Ausgehend von 125 mg (0.35 mmol) Ethyl-3-[2-chlor-3-(trifluormethyl)benzyl]-2,4-dioxo-1-(1,3,3-trimethyl-2-oxo-2,3-dihydro-1H-indol-6-yl)-1,2,3,4-tetrahydropyrimidin-5-carboxylat aus Beispiel 60 erhielt man 134 mg (65 % d. Th.) der Titelverbindung.
LC-MS (Methode 1): Rₜ = 1.08 min; m/z = 522 (M+H)⁺.
¹H-NMR (400MHz, DMSO-d₆): δ [ppm] = 1.30 (s, 6H), 3.15 (s, 3H), 5.17 (s, 2H), 7.19 - 7.24 (m, 1H), 7.24 - 7.27 (m, 1H), 7.48 - 7.56 (m, 2H), 7.58 - 7.62 (m, 1H), 7.78 - 7.83 (m, 1H), 8.49 (s, 1H), 12.74 (br.s, 1H).

### Beispiel 207

### 3-[3-Chlor-2-(trifluormethyl)benzyl]-2,4-dioxo-1-(1,3,3-trimethyl-2-oxo-2,3-dihydro-1H-indol-6-yl)-1,2,3,4-tetrahydropyrimidin-5-carbonsäure

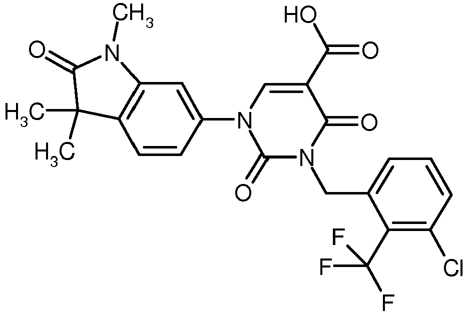

Darstellung und Reinigung der Titelverbindung erfolgten in Analogie zu Beispiel 121 mit einer Reaktionszeit von 45 min. Ausgehend von 122 mg (0.22 mmol) Ethyl-3-[3-chlor-2-(trifluormethyl)benzyl]-2,4-dioxo-1-(1,3,3-trimethyl-2-oxo-2,3-dihydro-1H-indol-6-yl)-1,2,3,4-tetrahydropyrimidin-5-carboxylat aus Beispiel 61 erhielt man 87 mg (71 % d. Th.) der Titelverbindung.
LC-MS (Methode 1): Rₜ = 1.12 min; m/z = 522 (M+H)⁺.
¹H-NMR (400MHz, DMSO-d₆): δ [ppm] = 1.30 (s, 6H), 3.15 (s, 3H), 5.24 (br.s, 2H), 7.19 - 7.23 (m, 1H), 7.23 - 7.26 (m, 1H), 7.33 - 7.37 (m, 1H), 7.51 (d, 1H), 7.57 - 7.67 (m, 2H), 8.50 (s, 1H), 12.73 (br.s, 1H).

### Beispiel 208

### Ethyl-3-[2-chlor-3-(trifluormethyl)benzyl]-1-(1-ethyl-3,3-dimethyl-2-oxo-2,3-dihydro-1H-indol-6-yl)-2,4-dioxo-1,2,3,4-tetrahydropyrimidin-5-carboxylat

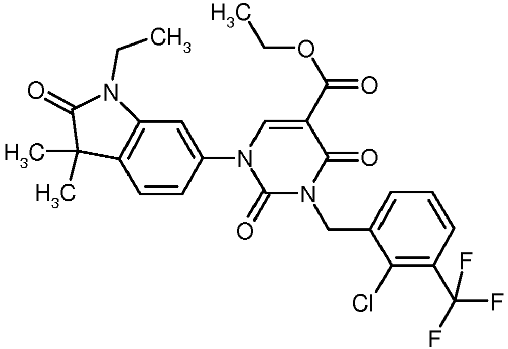

371 mg (0.69 mmol) Ethyl-3-[2-chlor-3-(trifluormethyl)benzyl]-1-(3,3-dimethyl-2-oxo-2,3-dihydro-1H-indol-6-yl)-2,4-dioxo-1,2,3,4-tetrahydropyrimidin-5-carboxylat aus Beispiel 68 wurden unter Argon in THF (5 ml) bei 0°C vorgelegt und mit 29 mg (60 % Gehalt, 0.72 mmol) Natriumhydrid versetzt. Das Gemisch wurde 30 min bei RT gerührt und anschließend erneut auf 0°C gekühlt. Eine Lösung von 113 mg (0.72 mmol) Iodethan in 1 ml THF wurde zugetropft. Die Reaktionsmischung ließ man 2 Tage bei RT rühren. Zur Aufarbeitung wurde das gemisch mit Wasser versetzt und zweimal mit Essigsäureethylester extrahiert. Die vereinigten organischen Phasen wurden über Magnesiumsulfat getrocknet und eingeengt. Der Rückstand wurde mittels präparativer HPLC (Methode 7) gereinigt. Man erhielt 50 mg (12 % d. Th.) der Titelverbindung.
LC-MS (Methode 1): Rₜ = 1.37 min; m/z = 564 (M+H)⁺.
¹H-NMR (400MHz, DMSO-d₆): δ [ppm] = 1.16 (t, 3H), 1.24 (t, 3H), 1.30 (s, 6H), 3.70 (q, 2H), 4.20 (q, 2H), 5.16 (s, 2H), 7.21 (dd, 1H), 7.31 (d, 1H), 7.49 - 7.60 (m, 3H), 7.78 - 7.82 (m, 1H), 8.52 (s, 1H).

### Beispiel 209

### 1-(4-Methylchinolin-7-yl)-3-[2-methyl-3-(trifluormethyl)benzyl]-2,4-dioxo-1,2,3,4-tetrahydro-pyrimidin-5-carbonsäure Hydrochlorid

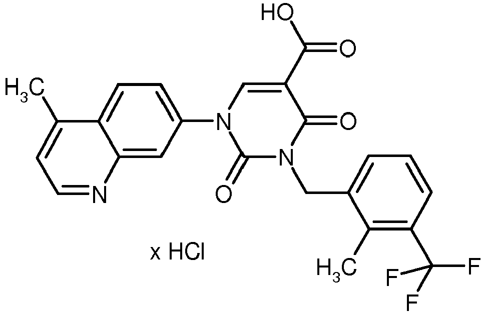

Darstellung und Reinigung der Titelverbindung erfolgten in Analogie zu Beispiel 121. Ausgehend von 200 mg (0.40 mmol) Ethyl-1-(4-methylchinolin-7-yl)-3-[2-methyl-3-(trifluormethyl)benzyl]-2,4-dioxo-1,2,3,4-tetrahydropyrimidin-5-carboxylat aus Beispiel 99 erhielt man 173 mg (91 % d. Th.) der Titelverbindung.
LC-MS (Methode 3): Rₜ = 1.25 min; m/z = 470 (M+H)⁺.
¹H-NMR (400MHz, DMSO-d₆): δ [ppm] = 2.48 (s, 3H), 2.80 (s, 3H), 3.80 (br.s, 1H), 5.14 (s, 2H), 7.34 - 7.40 (m, 1H), 7.46 - 7.51 (m, 1H), 7.59 - 7.65 (m, 2H), 7.84 - 7.90 (m, 1H), 8.28 - 8.36 (m, 2H), 8.68 (s, 1H), 8.93 - 8.98 (m, 1H), 12.75 (br.s, 1H).

### Beispiel 210

### 3-[2-Chlor-3-(trifluormethyl)benzyl]-1-(3,3-dimethyl-2,3-dihydro-1H-indol-6-yl)-2,4-dioxo-1,2,3,4-tetrahydropyrimidin-5-carbonsäure

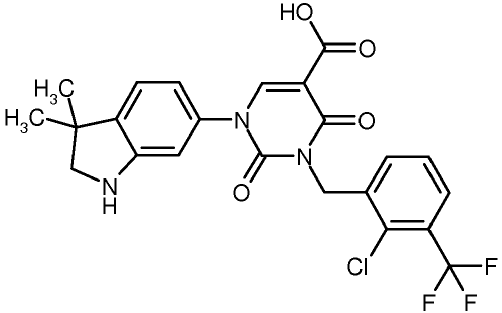

228 mg (0.40 mmol) der Verbindung aus Beispiel 69 wurden in 4.4 ml Eisessig / konz. Salzsäure 2:1 (v/v) 1 h bei 120 °C (Badtemperatur) gerührt. Nach Abkühlung auf RT wurde das Gemisch mit Wasser versetzt und dreimal mit Dichlormethan extrahiert. Die vereinigten organischen Phasen wurden über Magnesiumsulfat getrocknet und am Rotationsverdampfer eingeengt. Der Rückstand wurde in MTBE verrührt, der gebildete Feststoff abfiltriert, mit wenig MTBE gewaschen und im HV getrocknet. Man erhielt 160 mg (80 % d. Th.) der Titelverbindung.
LC-MS (Methode 5): Rₜ = 1.23 min; m/z = 494 (M+H)⁺.
¹H-NMR (400MHz, DMSO-d₆): δ [ppm]= 1.25 (s, 6H), 3.25 (s, 2H), 5.15 (s, 2H), 5.88 (br.s, 1H), 6.59 (s, 1H), 6.65 (d, 1H), 7.09 (d, 1H), 7.47 - 7.55 (m, 1H), 7.56 - 7.61 (m, 1H), 7.80 (d, 1H), 8.36 (s, 1H), 12.67 (br.s, 1H).

### Beispiel 211

### 1-(1-Acetyl-3,3-dimethyl-2,3-dihydro-1H-indol-6-yl)-3-[2-chlor-3-(trifluormethyl)benzyl]-2,4-dioxo-1,2,3,4-tetrahydropyrimidin-5-carbonsäure

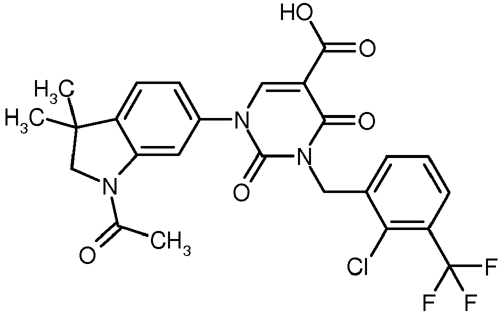

In THF (1.4 ml) wurden 160 mg (0.32 mmol) der Verbindung aus Beispiel 210 vorgelegt, anschließend 90 µL (0.65 mmol) Triethylamin sowie 34 µL (0.36 mmol) Essigsäureanhydrid hinzugefügt und die Mischung über Nacht bei RT gerührt. Danach wurde die Reaktionsmischung mit IM Salzsäure versetzt und dreimal mit Dichlormethan extrahiert. Die vereinigten organischen Phasen wurden über Magnesiumsulfat getrocknet, filtriert und eingeengt. Der Rückstand wurde mit Methanol verrührt, der Feststoff abfiltriert und im Vakuum getrocknet. Man erhielt 85 mg (47 % d. Th.) der Titelverbindung.
LC-MS (Methode 1): Rₜ = 1.13 min; m/z = 536 (M+H)⁺.
¹H-NMR (400MHz, DMSO-d₆): δ [ppm] = 1.34 (s, 6H), 2.18 (s, 3H), 3.94 (s, 2H), 5.15 (s, 2H), 7.16 - 7.21 (m, 1H), 7.38 - 7.43 (m, 1H), 7.48 - 7.54 (m, 1H), 7.61 - 7.65 (m, 1H), 7.77 - 7.82 (m, 1H), 8.13 - 8.16 (m, 1H), 8.42 (s, 1H), 12.69 (br.s, 1H).

### Beispiel 212

### 1-(3,3-Dimethyl-2,3-dihydro-1H-indol-6-yl)-3-[2-methyl-3-(trifluormethyl)benzyl]-2,4-dioxo-1,2,3,4-tetrahydropyrimidin-5-carbonsäure

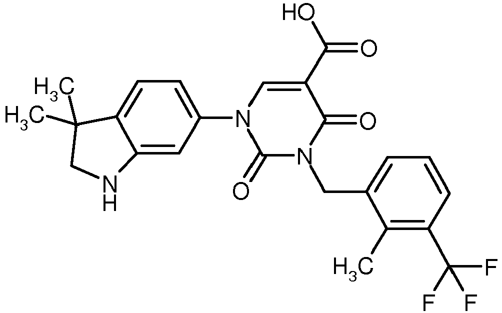

Analog zu Beispiel 210 wurden 253 mg (0.47 mmol) der Verbindung aus Beispiel 70 hydrolysiert und das Produkt gereinigt. Man erhielt 174 mg (77 % d. Th.) der Titelverbindung.
LC-MS (Methode 5): Rₜ = 1.23 min; m/z = 474 (M+H)⁺.
¹H-NMR (400MHz, DMSO-d₆): δ [ppm]= 1.24 (s, 6H), 2.46 (s, 3H), 3.24 (s, 2H), 5.09 (s, 2H), 5.86 (br.s, 1H), 6.58 (s, 1H), 6.64 (d, 1H), 7.08 (d, 1H), 7.26 - 7.45 (m, 2H), 7.60 (d, 1H), 8.35 (s, 1H), 12.68 (br.s, 1H).

### Beispiel 213

### 1-(1-Acetyl-3,3-dimethyl-2,3-dihydro-1H-indol-6-yl)-3-[2-methyl-3-(trifluormethyl)benzyl]-2,4-dioxo-1,2,3,4-tetrahydropyrimidin-5-carbonsäure

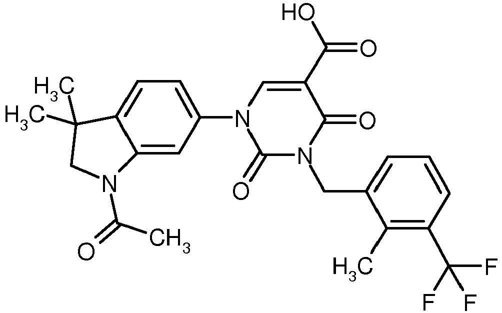

Darstellung und Reinigung der Titelverbindung erfolgten analog zu Beispiel 211. Ausgehend von 174 mg (0.36 mmol) 1-(3,3-Dimethyl-2,3-dihydro-1H-indol-6-yl)-3-[2-methyl-3-(trifluormethyl)benzyl]-2,4-dioxo-1,2,3,4-tetrahydropyrimidin-5-carbonsäure aus Beispiel 212 erhielt man 135 mg (70 % d. Th.) der Titelverbindung.
LC-MS (Methode 5): Rₜ = 1.18 min; m/z = 516 (M+H)⁺.
¹H-NMR (400MHz, DMSO-d₆): δ [ppm] = 1.34 (s, 6H), 2.18 (s, 3H), 2.46 (s, 3H), 3.93 (s, 2H), 5.09 (s, 2H), 7.16 - 7.21 (m, 1H), 7.31 - 7.37 (m, 1H), 7.38 - 7.44 (m, 1H), 7.57 - 7.62 (m, 1H), 8.12 - 8.15 (m, 1H), 8.40 (s, 1H), 12.69 (br.s, 1H).

### Beispiel 214

### 1-(1-Methyl-2-oxo-1,2,3,4-tetrahydrochinolin-6-yl)-3-[2-methyl-3-(trifluormethyl)benzyl]-2,4-dioxo-1,2,3,4-tetrahydropyrimidin-5-carbonsäure

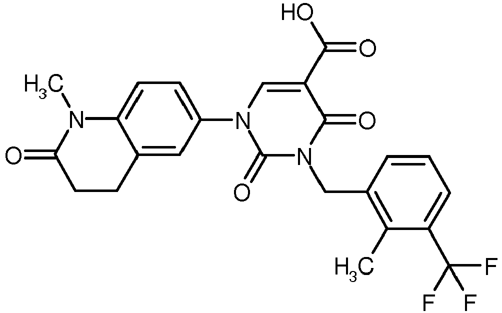

Darstellung und Reinigung der Titelverbindung erfolgten analog zu Beispiel 121. Ausgehend von 267 mg (0.51 mmol) Ethyl-1-(1-methyl-2-oxo-1,2,3,4-tetrahydrochinolin-6-yl)-3-[2-methyl-3-(trifluormethyl)benzyl]-2,4-dioxo-1,2,3,4-tetrahydropyrimidin-5-carboxylat aus Beispiel 102 erhielt man 218 mg (83 % d. Th.) der Titelverbindung.
LC-MS (Methode 3): Rₜ = 1.28 min; m/z = 488 (M+H)⁺.
¹H-NMR (400MHz, DMSO-d₆): δ [ppm] = 2.47 (s, 3H), 2.56 - 2.61 (m, 2H), 2.89 - 2.94 (m, 2H), 3.28 (s, 3H), 5.10 (s, 2H), 7.20 - 7.24 (m, 1H), 7.32 - 7.46 (m, 4H), 7.58 - 7.62 (m, 1H), 8.43 (s, 1H), 12.72 (br.s, 1H).

### Beispiel 215

### 1-(1-Methyl-2-oxo-1,2,3,4-tetrahydrochinolin-6-yl)-2,4-dioxo-3-[(1R)-4-(trifluormethyl)-2,3-dihydro-1H-inden-1-yl]-1,2,3,4-tetrahydropyrimidin-5-carbonsäure (R-Enantiomer)

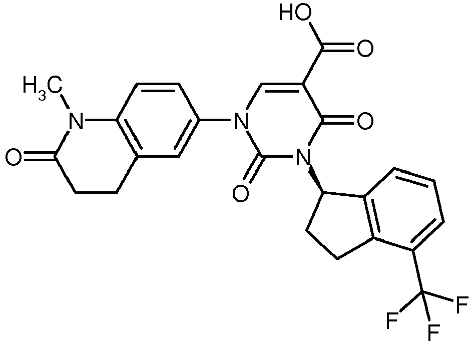

Darstellung und Reinigung der Titelverbindung erfolgten analog zu Beispiel 121 mit 45 min Reaktionszeit. Ausgehend von 83 mg (0.15 mmol) Ethyl-1-(1-methyl-2-oxo-1,2,3,4-tetrahydrochinolin-6-yl)-2,4-dioxo-3-[4-(trifluormethyl)-2,3-dihydro-1H-inden-l-yl]-1,2,3,4-tetrahydropyrimidin-5-carboxylat aus Beispiel 103 erhielt man 39 mg (46 % d. Th.) der Titelverbindung.
LC-MS (Methode 1): Rₜ = 1.10 min; m/z = 500 (M+H)⁺.
¹H-NMR (400MHz, CDCl₃): δ [ppm] = 2.42 - 2.52 (m, 1H), 2.63 - 2.66 (m, 1H, teilweise durch DMSO-Signal verdeckt), 2.69 (t, 2H), 2.95 (t, 2H), 3.12 - 3.20 (m, 1H, , 3.37 (s, 3H), 3.48 - 3.60 (m, 1H), 6.60 - 6.71 (m, 1H), 7.06 (d, 1H), 7.14 (s, 1H), 7.21 (d, 1H), 7.28 - 7.34 (m, 2H), 7.50 - 7.56 (m, 1H), 8.55 (s, 1H), 12.49 (s, 1H).

### Beispiel 216

### Ethyl-3-(2-methyl-3-nitrobenzyl)-1-(4-methyl-3-oxo-3,4-dihydro-2H-1,4-benzoxazin-7-yl)-2,4-dioxo-1,2,3,4-tetrahydropyrimidin-5-carboxylat

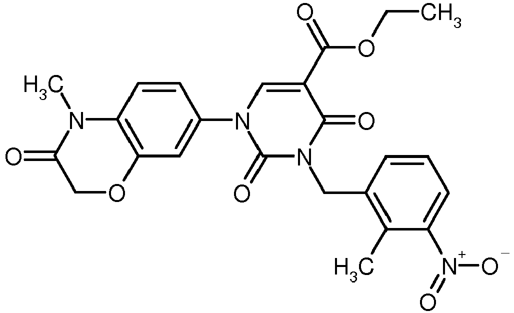

200 mg (0.58 mmol) Ethyl-1-(4-methyl-3-oxo-3,4-dihydro-2H-1,4-benzoxazin-7-yl)-2,4-dioxo-1,2,3,4-tetrahydropyrimidin-5-carboxylat aus Beispiel 119A wurden in Acetonitril (7.5 ml) vorgelegt. 108 mg (0.58 mmol) 2-Methyl-3-nitrobenzylchlorid, 160 mg (1.16 mmol) Kaliumcarbonat und 48 mg (0.29 mmol) Kaliumsodid wurden zugegeben und die Mischung 41 h bei 60°C gerührt. Das auf RT abgekühlte Gemisch wurde komplett durch präparative HPLC (Methode 8) getrennt und das isolierte Produkt im HV getrocknet. Man erhielt 218 mg (75 % d. Th.) der Titelverbindung.
LC-MS (Methode 3): Rₜ = 1.13 min; m/z = 495 (M+H)⁺.
¹H-NMR (400MHz, DMSO-d₆): δ [ppm] = 1.22 (t, 3H), 2.41 (s, 3H), 4.21 (q, 2H), 3.30 (s, teilweise durch Wassersignal verdeckt, 3H), 4.71 (s, 2H), 5.06 (s, 2H), 7.22 - 7.32 (m, 3H), 7.36 (t, 1H), 7.41 (d, 1H), 7.72 (d, 1H), 8.38 (s, 1H).

### Beispiel 217

### 1-(3-Isopropyl-1-methyl-2-oxo-2,3-dihydro-1H-benzimidazol-5-yl)-2,4-dioxo-3-[(1R)-4-(trifluormethyl)-2,3-dihydro-1H-inden-1-yl]-1,2,3,4-tetrahydropyrimidin-5-carbonsäure (R-Enantiomer)

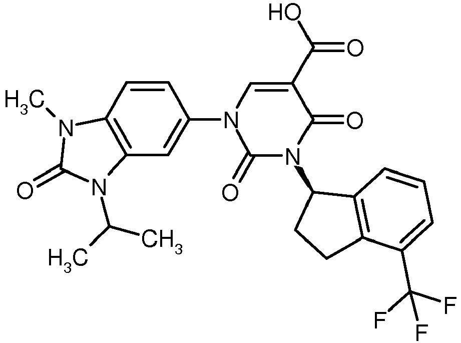

122 mg (0.22 mmol) der Verbindung aus Beispiel 114 wurden 1 h in 3.8 ml Eisessig / konz. Salzsäure 2:1 (v/v) auf 120°C (Badtemperatur) erhitzt. Nach Abkühlen auf RT wurden 30 ml Wasser zugegeben und das ausgefallene Produkt abgesaugt. Der Feststoff wurde mit Wasser gewaschen und im HV getrocknet. Man erhielt 107 mg (91 % d. Th.) der Titelverbindung.
LC-MS (Methode 3): Rₜ = 2.43 min; m/z = 529 (M+H)⁺.
¹H-NMR (400MHz, CD₂Cl₂): δ [ppm] = 1.42 (d, 3H), 1.43 (d, 3H), 2.34 - 2.46 (m, 1H), 2.52 - 2.64 (m, 1H), 3.04 - 3.16 (m, 1H), 3.31 (s, 3H), 3.37 - 3.50 (m, 1H), 4.54 (sept, 1H), 6.51 - 6.62 (m, 1H), 6.88 - 7.01 (m, 3H), 7.21 - 7.31 (m, 2H), 7.46 (d, 1H), 8.49 (s, 1H), 12.29 (br. s, 1H).

### Beispiel 218

### 3-[2-Chlor-3-(trifluormethyl)benzyl]-2,4-dioxo-1-(1,3,3-trimethyl-2-oxo-2,3-dihydro-1H-indol-5-yl)-1,2,3,4-tetrahydropyrimidin-5-carbonsäure

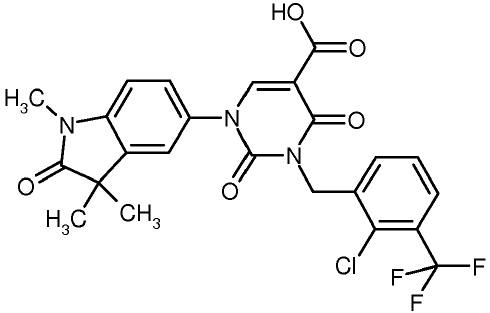

Darstellung und Reinigung der Titelverbindung erfolgten in Analogie zu Beispiel 121. Ausgehend von 200 mg (0.36 mmol) Ethyl-3-[2-chlor-3-(trifluormethyl)benzyl]-2,4-dioxo-1-(1,3,3-trimethyl-2-oxo-2,3-dihydro-1H-indol-5-yl)-1,2,3,4-tetrahydropyrimidin-5-carboxylat aus Beispiel 52 erhielt man 161 mg (83 % d. Th.) der Titelverbindung.
LC-MS (Methode 3): Rₜ = 1.29 min; m/z = 522 (M+H)⁺.
¹H-NMR (400MHz, DMSO-d₆): δ [ppm] = 1.30 (s, 6H), 3.18 (s, 3H), 5.17 (s, 2H), 7.15 (d, 1H), 7.43 - 7.48 (m, 1H), 7.50 - 7.56 (m, 2H), 7.57 - 7.62 (m, 1H), 7.78 - 7.83 (m, 1H), 8.46 (s, 1H), 12.73 (br.s, 1H).

### Beispiel 219

### 3-[2-Methyl-3-(trifluormethyl)benzyl]-2,4-dioxo-1-(1,3,3-trimethyl-2-oxo-2,3-dihydro-1H-indol-5-yl)-1,2,3,4-tetrahydropyrimidin-5-carbonsäure

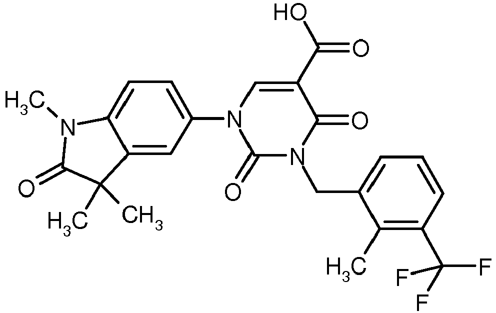

Darstellung und Reinigung der Titelverbindung erfolgten in Analogie zu Beispiel 121. Ausgehend von 200 mg (0.38 mmol) Ethyl-3-[2-methyl-3-(trifluormethyl)benzyl]-2,4-dioxo-1-(1,3,3-trimethyl-2-oxo-2,3-dihydro-1H-indol-5-yl)-1,2,3,4-tetrahydropyrimidin-5-carboxylat aus Beispiel 53 erhielt man 153 mg (80 % d. Th.) der Titelverbindung.
LC-MS (Methode 1): Rₜ = 1.07 min; m/z = 502 (M+H)⁺.
¹H-NMR (400MHz, DMSO-d₆): δ [ppm] = 1.29 (s, 6H), 2.47 (s, 3H), 3.17 (s, 3H), 5.11 (s, 2H), 7.15 (d, 1H), 7.32 - 7.42 (m, 2H), 7.46 (dd, 1H), 7.54 (d, 1H), 7.58 - 7.64 (m, 1H), 8.45 (s, 1H), 12.73 (br.s, 1H).

### Beispiel 220

### 3-[3-Chlor-2-(trifluormethyl)benzyl]-2,4-dioxo-1-(1,3,3-trimethyl-2-oxo-2,3-dihydro-1H-indol-5-yl)-1,2,3,4-tetrahydropyrimidin-5-carbonsäure

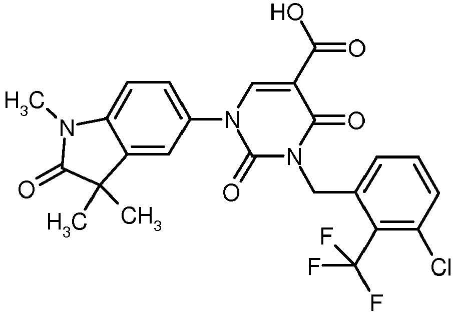

Darstellung und Reinigung der Titelverbindung erfolgten in Analogie zu Beispiel 121. Ausgehend von 109 mg (0.20 mmol) Ethyl-3-[3-chlor-2-(trifluormethyl)benzyl]-2,4-dioxo-1-(1,3,3-trimethyl-2-oxo-2,3-dihydro-1H-indol-5-yl)-1,2,3,4-tetrahydropyrimidin-5-carboxylat aus Beispiel 54 erhielt man 83 mg (79 % d. Th.) der Titelverbindung.
LC-MS (Methode 1): Rₜ = 1.10 min; m/z = 522 (M+H)⁺.
¹H-NMR (400MHz, DMSO-d₆): δ [ppm] = 1.29 (s, 6H), 3.17 (s, 3H), 5.24 (br.s, 2H), 7.15 (d, 1H), 7.32 - 7.37 (m, 1H), 7.42 - 7.47 (m, 1H), 7.50 - 7.54 (m, 1H), 7.57 - 7.67 (m, 2H), 8.47 (s, 1H), 12.71 (br.s, 1H).

### Beispiel 221

### 3-[3-Fluor-2-(trifluormethyl)benzyl]-2,4-dioxo-1-(1,3,3-trimethyl-2-oxo-2,3-dihydro-1H-indol-5-yl)-1,2,3,4-tetrahydropyrimidin-5-carbonsäure

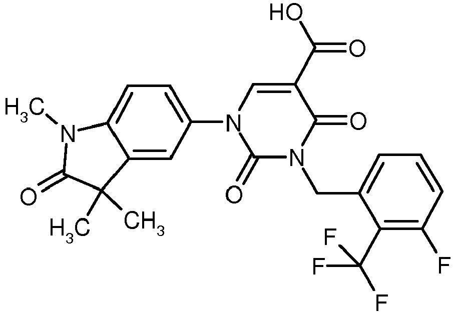

Darstellung und Reinigung der Titelverbindung erfolgten in Analogie zu Beispiel 121. Ausgehend von 230 mg (0.43 mmol) Ethyl-3-[3-fluor-2-(trifluormethyl)benzyl]-2,4-dioxo-1-(1,3,3-trimethyl-2-oxo-2,3-dihydro-1H-indol-5-yl)-1,2,3,4-tetrahydropyrimidin-5-carboxylat aus Beispiel 55 erhielt man 193 mg (85 % d. Th.) der Titelverbindung.
LC-MS (Methode 1): Rₜ = 1.02 min; m/z = 506 (M+H)⁺.
¹H-NMR (400MHz, DMSO-d₆): δ [ppm] = 1.29 (s, 6H), 3.17 (s, 3H), 5.22 (s, 2H), 7.15 (d, 1H), 7.18 - 7.24 (m, 1H), 7.38 - 7.43 (m, 1H), 7.43 - 7.47 (m, 1H), 7.52 - 7.54 (m, 1H), 7.63 - 7.70 (m, 1H), 8.46 (s, 1H), 12.72 (br.s, 1H).

### Beispiel 222

### 3-(2,3-Dichlorbenzyl)-2,4-dioxo-1-(1,3,3-trimethyl-2-oxo-2,3-dihydro-1H-indol-5-yl)-1,2,3,4-tetrahydropyrimidin-5-carbonsäure

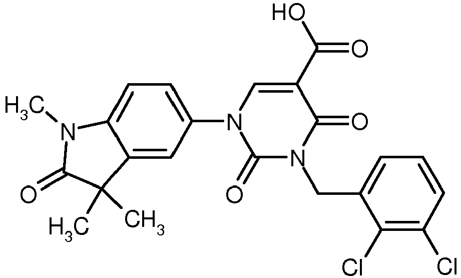

Darstellung und Reinigung der Titelverbindung erfolgten in Analogie zu Beispiel 121. Ausgehend von 200 mg (0.39 mmol) Ethyl-3-(2,3-dichlorbenzyl)-2,4-dioxo-1-(1,3,3-trimethyl-2-oxo-2,3-dihydro-1H-indol-5-yl)-1,2,3,4-tetrahydropyrimidin-5-carboxylat aus Beispiel 56 erhielt man 173 mg (90 % d. Th.) der Titelverbindung.
LC-MS (Methode 1): Rₜ = 1.05 min; m/z = 488 (M+H)⁺.
¹H-NMR (400MHz, DMSO-d₆): δ [ppm] = 1.29 (s, 6H), 3.17 (s, 3H), 5.13 (s, 2H), 7.15 (d, 1H), 7.22 - 7.27 (m, 1H), 7.34 (t, 1H), 7.45 (dd, 1H), 7.54 (d, 1H), 7.57 - 7.61 (m, 1H), 8.45 (s, 1H), 12.72 (br.s, 1H).

### Beispiel 223

### 2,4-Dioxo-3-[4-(trifluormethyl)-2,3-dihydro-1H-inden-1-yl]-1-(1,3,3-trimethyl-2-oxo-2,3-dihydro-1H-indol-5-yl)-1,2,3,4-tetrahydropyrimidin-5-carbonsäure (Racemat)

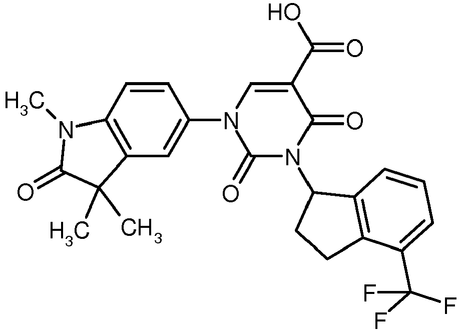

Darstellung und Reinigung der Titelverbindung erfolgte analog zu Beispiel 131. Ausgehend von 507 mg (0.93 mmol) Ethyl-2,4-dioxo-3-[4-(trifluormethyl)-2,3-dihydro-1H-inden-1-yl]-1-(1,3,3-trimethyl-2-oxo-2,3-dihydro-1H-indol-5-yl)-1,2,3,4-tetrahydropyrimidin-5-carboxylat aus Beispiel 57 erhielt man nach Reinigung mittels HPLC (Methode 8) 131 mg (26 % d. Th.) der Titelverbindung.
LC-MS (Methode 5): Rₜ = 1.15 min; m/z = 514 (M+H)⁺.
¹H-NMR (400MHz, DMSO-d₆): δ [ppm] = 1.29 (br.s, 6H), 2.38 - 2.47 (m, 1H), 2.46-2.48 (m, 1H, verdeckt durch DMSO-Signal), 3.03 - 3.14 (m, 1H), 3.17 (s, 3H), 3.20 - 3.27 (m, 1H, teilweise verdeckt durch Wasser-Signal), 6.34 - 6.60 (m, 1H), 7.08 - 7.18 (m, 1H), 7.33 - 7.46 (m, 2H), 7.47 - 7.58 (m, 3H), 8.38 (s, 1H), 12.69 (br. s, 1H).

### Beispiel 224

### 2,4-Dioxo-3-[(1R)-4-(trifluormethyl)-2,3-dihydro-1H-inden-1-yl]-1-(1,3,3-trimethyl-2-oxo-2,3-dihydro-1H-indol-5-yl)-1,2,3,4-tetrahydropyrimidin-5-carbonsäure (R-Enantiomer)

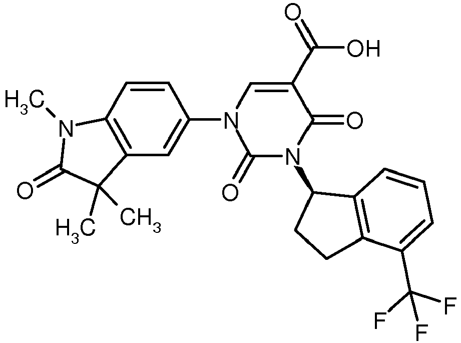

Analog zu Beispiel 217 wurden 147 mg (0.27 mmol) der Verbindung aus Beispiel 58 hydrolysiert und das Produkt isoliert. Man erhielt 120 mg (84 % d. Th.) der Titelverbindung.
LC-MS (Methode 1): Rₜ = 1.14 min; m/z = 514 (M+H)⁺.
¹H-NMR (400MHz, DMSO-d₆): δ [ppm] = 1.29 (br.s, 6H), 2.39 - 2.46 (m, 1H), 2.46-2.60 (m, 1H, verdeckt durch DMSO-Signal), 3.04 - 3.18 (m, 1H), 3.17 (s, 3H), 3.22 - 3.36 (m, 1H teilweise verdeckt durch Wasser-Signal), 6.34 - 6.61 (br. m, 1H), 7.13 (d, 1H), 7.33 - 7.46 (m, 2H), 7.47 - 7.57 (m, 3H), 8.38 (s, 1H), 12.69 (br. s, 1H).
α_{D}²⁰ [Chloroform, c = 0.385] = +130.1°.

### Beispiel 225

### 3-[2-Chlor-3-(trifluomiethyl)benzyl]-1-(1'-methyl-2'-oxo-1',2'-dihydrospiro[cyclopropan-1,3'-indol]-5'-yl)-2,4-dioxo-1,2,3,4-tetrahydropyrimidin-5-carbonsäure

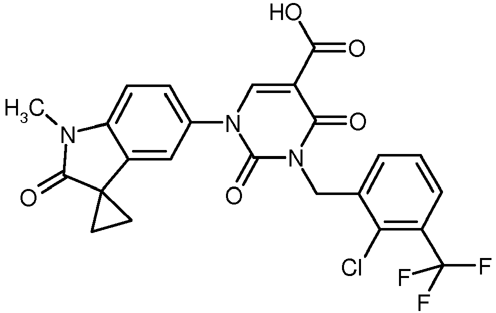

Darstellung und Reinigung der Titelverbindung erfolgten analog zu Beispiel 131. Ausgehend von 147 mg (0.26 mmol) Ethyl-3-[2-chlor-3-(trifluormethyl)benzyl]-1-(1'-methyl-2'-oxo-1',2'-dihydrospiro[cyclopropan-1,3'-indol]-5'-yl)-2,4-dioxo-1,2,3,4-tetrahydropyrimidin-5-carboxylat aus Beispiel 65 erhielt man nach Reinigung mittels HPLC (Methode 7) 30 mg (21 % d. Th.) der Titelverbindung.
LC-MS (Methode 1): Rₜ = 1.08 min; m/z = 520 (M+H)⁺.
¹H-NMR (400MHz, DMSO-d₆): δ [ppm] = 1.55 - 1.60 (m, 2H), 1.65 - 1.70 (m, 2H), 3.26 (s, 3H), 5.16 (s, 2H), 7.20 - 7.23 (m, 2H), 7.41 - 7.46 (m, 1H), 7.50 - 7.55 (m, 1H), 7.56 - 7.60 (m, 1H), 7.78 - 7.83 (m, 1H), 8.46 (s, 1H), 12.73 (br.s, 1H).

### Beispiel 226

### 1-(1'-Methyl-2'-oxo-1',2'-dihydrospiro[cyclopropan-1',3'-indol]-5'-yl)-3-[2-methyl-3-(trifluor-methyl)benzyl]-2,4-dioxo-1,2,3,4-tetrahydropyrimidin-5-carbonsäure

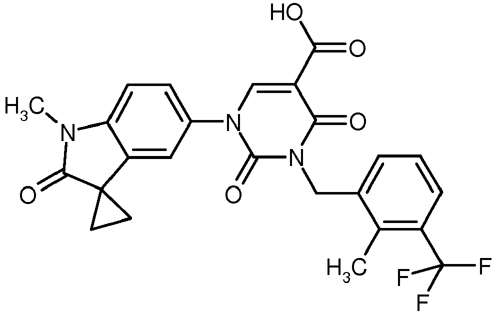

Darstellung und Reinigung der Titelverbindung erfolgten analog zu Beispiel 131. Ausgehend von 130 mg (0.24 mmol) Ethyl-1-(1'-methyl-2'-oxo-1',2'-dihydrospiro[cyclopropan-1,3'-indol]-5'-yl)-3-[2-methyl-3-(trifluormethyl)benzyl]-2,4-dioxo-1,2,3,4-tetrahydropyrimidin-5-carboxylat aus Beispiel 66 erhielt man nach Reinigung mittels HPLC (Methode 7) 27 mg (22 % d. Th.) der Titelverbindung.
LC-MS (Methode 1): Rₜ = 1.08 min; m/z = 500 (M+H)⁺.
¹H-NMR (400MHz, DMSO-d₆): δ [ppm] = 1.55 - 1.60 (m, 2H), 1.65 - 1.69 (m, 2H), 2.46 (s, 3H), 3.26 (s, 3H), 5.10 (s, 2H), 7.18 - 7.24 (m, 2H), 7.32 - 7.40 (m, 2H), 7.42 - 7.46 (m, 1H), 7.59 - 7.63 (m, 1H), 8.45 (s, 1H), 12.73 (br.s, 1H).

### Beispiel 227

### 1-(1'-Methyl-2'-oxo-1',2'-dihydrospiro[cyclopropan-1',3'-indol]-5'-yl)-2,4-dioxo-3-[(1R)-4-(trifluorniethyl)-2,3-dihydro-1H-inden-1-yl]-1,2,3,4-tetrahydropyrimidin-5-carbonsäure (R-Enantiomer)

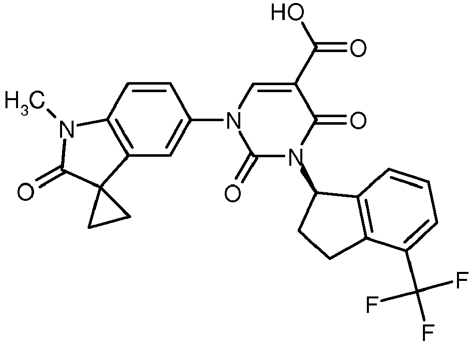

7.81 g (92 %-ige Reinheit, 13.31 mmol) Ethyl-1-(1'-methyl-2'-oxo-r,2'-dihydrospiro[cyclopropan-1,3'-indol]-5'-yl)-2,4-dioxo-3-[4-(trifluormethyl)-2,3-dihydro-1H-inden-1-yl]-1,2,3,4-tetrahydropyrimidin-5-carboxylat aus Beispiel 67 wurden in 117 ml eines Gemisches aus Essigsäure/Wasser/ konz. Schwefelsäure (12:8:1) 2.5 h bei 120°C gerührt. Das abgekühlte Reaktionsgemisch wurde mit Wasser versetzt, der ausgefallene Feststoff abgesaugt, mit Wasser nachgewaschen und am Hochvakuum getrocknet. Die Mutterlauge wurde zweimal mit Dichlormethan extrahiert. Die vereinigten organischen Phasen wurden über Natriumsulfat getrocknet und eingeengt. Der Rückstand wurde zusammen mit dem vorher isolierten Feststoff mittels HPLC (Methode 7) gereinigt. Das isolierte Produkt (95 %-ige Reinheit) wurde in siedendem 2-Propanol gelöst und die Lösung über Nacht abgekühlt. Der gebildete Feststoff wurde abgesaugt, mit 2-Propanol nachgewaschen und anschließend am Hochvakuum getrocknet. Man erhielt 5.22 g (74 % d. Th.) der Titelverbindung.
LC-MS (Methode 1): Rₜ = 1.08 min; m/z = 512 (M+H)⁺.
¹H-NMR (400MHz, CD₂Cl₂): δ [ppm] = 1.46 - 1.53 (m, 2H), 1.62 - 1.69 (m, 2H), 2.31 - 2.44 (m, 1H), 2.50 - 2.63 (m, 1H), 3.04 - 3.14 (m, 1H), 3.20 (s, 3H), 3.35 - 3.48 (m, 1H), 6.50 - 6.60 (m, 1H), 6.71 (br.s, 1H), 6.90 (d, 1H), 7.08 - 7.16 (m, 1H), 7.20 - 7.29 (m, 2H), 7.42 - 7.49 (m, 1H), 8.44 (s, 1H).

### Beispiel 228

### 3-[(3-Chlor-4-methyl-2-thienyl)methyl]-1-(1,3-dimethyl-2-oxo-2,3-dihydro-1H-benzimidazol-5-yl)-2,4-dioxo-1,2,3,4-tetrahydropyrimidin-5-carbonsäure

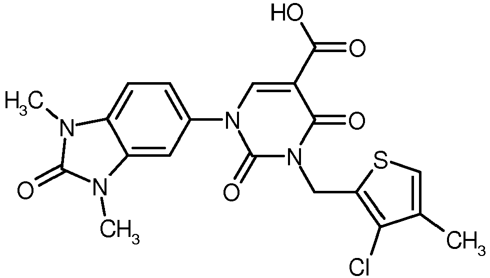

22 mg (43 µmol) der Verbindung aus Beispiel 105wurden 4 h in 1 ml Eisessig / konz. Salzsäure 2:1 auf 120°C (Badtemperatur) erhitzt. Nach Abkühlen auf RT wurden 10 ml Wasser zugegeben und das ausgefallene Produkt abgesaugt. Der Feststoff wurde mit Diethylether verrührt, wieder abgesaugt und im HV getrocknet. Man erhielt 15 mg (74 % d. Th.) der Titelverbindung.
LC-MS (Methode 1): Rₜ = 0.97 min; m/z = 461 (M+H)⁺.
¹H-NMR (400MHz, DMSO-d₆): δ [ppm] = 2.12 (s, 3H), 3.33 (s, 3H), 3.37 (s, 3H), 5.21 (s, 2H), 7.20 (dd, 1H), 7.27 (d, 1H), 7.30 (s, 1H), 7.37 (d, 1H), 8.37 (s, 1H), 12.74 (br. s, 1H).

### Beispiel 229

### 1-(3-Methyl-2-oxo-2,3-dihydro-1,3-benzothiazol-6-yl)-2,4-dioxo-3-[(1R)-4-(trifluoromethyl)-2,3-dihydro-1H-inden-1-yl]-1,2,3,4-tetrahydropyrimidin-5-carbonsäure (R-Enantiomer)

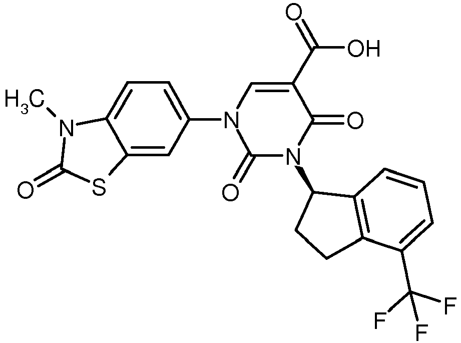

6.20 g (11.3 mmol) der Verbindung aus Beispiel 51 wurden 1 h in 150 ml Eisessig / konz. Salzsäure 2:1 auf 120°C (Badtemperatur) erhitzt. Nach Abkühlen auf RT wurden die Reaktionsmischung in 1 L Eis-Wasser gegossen. Das ausgefallene Produkt wurde abgesaugt. Der Feststoff wurde mit Diethylether verrührt, wieder abgesaugt und im HV getrocknet. Man erhielt 5.04 g (88 % d. Th.) der Titelverbindung.
LC-MS (Methode 5): Rₜ = 1.14 min; m/z = 504 (M+H)⁺.
¹H-NMR (400MHz, CD₂Cl₂): δ [ppm] = 2.39 - 2.53 (m, 1H), 2.60 - 2.72 (m, 1H), 3.12 - 3.24 (m, 1H), 3.42 - 3.56 (m, 4H), 6.58 - 6.71 (m, 1H), 7.15 (d, 1H), 7.26 - 7.38 (m, 3H), 7.45 (s, 1H), 7.50 - 7.58 (m, 1H), 8.55 (s, 1H).
Für weitere Chargen der Titelverbindung, die analog hergestellt worden sind, sind folgende zusätzliche analytische Daten erhoben worden:
α_{D}²⁰ [Chloroform, c = 0.365] = +148.6°.

### Beispiel 230

### 1-(1,3-Dimethyl-2-oxo-2,3-dihydro-1H-benzimidazol-5-yl)-3-(5-methoxy-2,3-dihydro-1H-inden-1-yl)-2,4-dioxo-1,2,3,4-tetrahydropyrimidin-5-carbonsäure (Racemat)

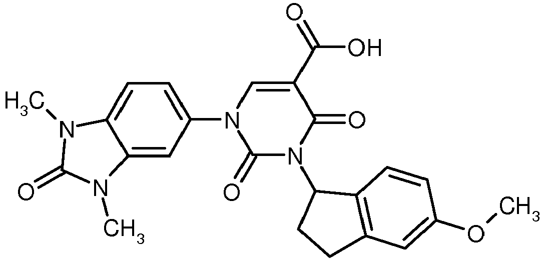

86 mg (0.18 mmol) der Verbindung aus Beispiel 120 und 49 mg (0.58 mmol) Natriumhydrogencarbonat wurden in 2 ml Acetonitril und 2 ml Wasser 6 h zum Rückfluss erhitzt. Nach Abkühlung auf RT wurde das Gemisch durch Zugabe von IN Salzsäure angesäuert und direkt über die präparative HPLC (Methode 7) getrennt. Man erhielt 24 mg (29 % d. Th.) der Titelverbindung.
LC-MS (Methode 1): Rₜ = 0.90 min; m/z = 463 (M+H)⁺.
¹H-NMR (400MHz, DMSO-d₆): δ [ppm] = 2.31 - 2.47 (m, 2H), 2.83 - 2.95 (m, 1H), 3.09 - 3.22 (m, 1H), 3.34 (s, 6H), 3.72 (s, 3H), 6.29 - 6.47 (m, 1H), 6.67 - 6.74 (m, 1H), 6.79 (s, 1H), 7.08 (d, 1H), 7.13 - 7.21 (m, 1H), 7.22 - 7.30 (m, 1H), 7.37 (s, 1H), 8.38 (s, 1H), 12.74 (br. s, 1H).

### Beispiel 231

### 3-(4,6-Difluor-2,3-dihydro-1H-inden-1-yl)-1-(1,3-dimethyl-2-oxo-2,3-dihydro-1H-benzimidazol-5-yl)-2,4-dioxo-1,2,3,4-tetrahydropyrimidin-5-carbonsäure (Racemat)

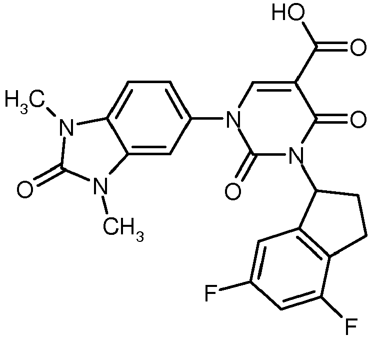

Analog zu Beispiel 217 wurden 173 mg (0.35 mmol) der Verbindung aus Beispiel 106 hydrolysiert. Man erhielt 130 mg (80 % d. Th.) der Titelverbindung.
LC-MS (Methode 5): Rₜ = 0.99 min; m/z = 469 (M+H)⁺.
¹H-NMR (400MHz, DMSO-d₆): δ [ppm] = 2.38 - 2.48 (m, 2H, teilweise verdeckt durch DMSO-Signal), 2.84 - 2.98 (m, 1H), 3.02 - 3.18 (m, 1H), 3.34 (br.s, 3H), 6.22 - 6.60 (m, 1H), 7.03 (t, 2H), 7.12 - 7.29 (m, 2H), 7.31 - 7.43 (m, 1H), 8.38 (s, 1H), 12.67 (br. s, 1H).

### Beispiel 232

### 1-(1,3-Dimethyl-2-oxo-2,3-dihydro-1H-benzimidazol-5-yl)-3-(6-methyl-2,3-dihydro-1H-inden-1-yl)-2,4-dioxo-1,2,3,4-tetrahydropyrimidin-5-carbonsäure (Racemat)

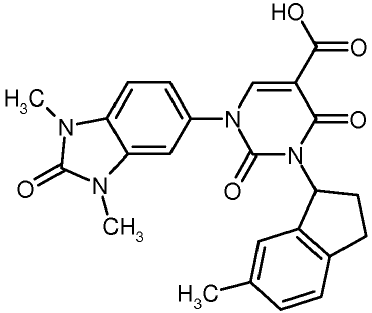

127 mg (0.27 mmol) der Verbindung aus Beispiel 107 wurden in 2.5 ml Acetonitril vorgelegt. 74 mg (0.88 mmol) Natriumhydrogencarbonat sowie 2.5 ml Wasser wurden hinzugefügt und die Mischung 6 h zum Rückfluss erhitzt. Nach Abkühlung auf RT wurde das Gemisch mit IN Salzsäure angesäuert und komplett per präparativer HPLC (Methode 7) getrennt. Man erhielt 78 mg (65 % d. Th.) der Titelverbindung.
LC-MS (Methode 1): Rₜ = 0.98 min; m/z = 447 (M+H)⁺.
¹H-NMR (400MHz, DMSO-d₆): δ [ppm] = 2.25 (s, 3H), 2.35 - 2.45 (m, 2H), 2.82 - 2.93 (m, 1H), 3.04 - 3.18 (m, 1H), 3.31 (s, 3H), 3.36 (s, 3H), 6.23 - 6.54 (m, 1H), 6.96 - 7.03 (m, 2H), 7.10 (d, 1H), 7.16 - 7.30 (m, 2H), 7.33 - 7.45 (m, 1H), 8.39 (s, 1H), 12.73 (br. s, 1H).

### Beispiel 233

### Ethyl-3-(4-methoxy-2,3-dihydro-1H-inden-1-yl)-1-(3-methyl-2-oxo-2,3-dihydro-1,3-benzothiazol-6-yl)-2,4-dioxo-1,2,3,4-tetrahydropyrimidin-5-carboxylat (Racemat)

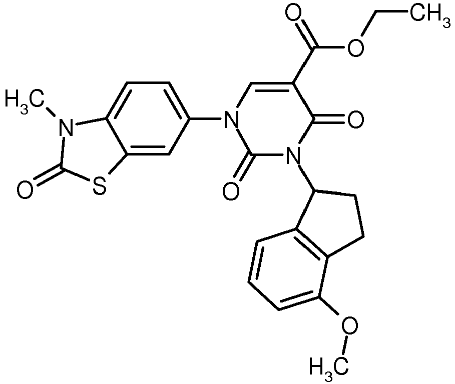

Unter Argon wurde eine Lösung von 200 mg (0.58 mmol) der Verbindung aus Beispiel 31A und 453 mg (1.73 mmol) Triphenylphosphin in 15.8 ml THF / DMF 1:1 (v/v) tropfenweise mit 227 µl (1.15 mmol) Diisopropylazodicarboxylat versetzt. Dann wurden 123 mg (0.75 mmol) der Verbindung aus Beispiel 103A hinzugefügt und das Gemisch 1 h bei RT gerührt. Unter Eiskühlung wurden 2 ml IN Salzsäure zugefügt, die Mischung 15 min weiter gerührt und anschließend per präparativer HPLC (Methode 7) getrennt. Man erhielt 118 mg (41 % d. Th.) der Titelverbindung.
LC-MS (Methode 1): Rₜ = 1.05 min; m/z = 494 (M+H)⁺.
¹H-NMR (400MHz, DMSO-d₆): δ [ppm]= 1.22 (t, 3H), 2.26 - 2.46 (m, 2H), 2.73 - 2.85 (m, 1H), 2.95 - 3.10 (m, 1H), 3.44 (s, 3H), 3.77 (s, 3H), 4.09 - 4.27 (m, 2H), 6.25 - 6.57 (m, 1H), 6.74 (d, 1H), 6.78 (d, 1H), 7.12 (t, 1H), 7.35 - 7.64 (m, 2H), 7.83 (br.s, 1H), 8.38 (s, 1H).

### Beispiel 234

### 3-(4-Methoxy-2,3-dihydro-1H-inden-1-yl)-1-(3-methyl-2-oxo-2,3-dihydro-1,3-benzothiazol-6-yl)-2,4-dioxo-1,2,3,4-tetrahydropyrimidin-5-carbonsäure (Racemat)

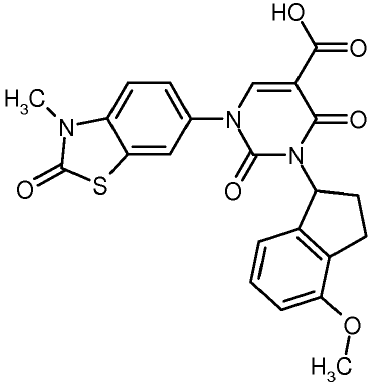

115 mg (0.23 mmol) der Verbindung aus Beispiel 233 wurden in 7.2 ml Eisessig / konz. Salzsäure 2:1 (v/v) 1 h zum Rückfluss erhitzt. Nach Abkühlung auf RT wurde das ganze Reaktionsgemisch per präparativer HPLC (Methode 7) getrennt. Man erhielt 42 mg (39 % d. Th.) der Titelverbindung.
LC-MS (Methode 1): Rₜ = 1.03 min; m/z = 466 (M+H)⁺.
¹H-NMR (400MHz, CD₂Cl₂): δ [ppm] = 2.26 - 2.38 (m, 1H), 2.44 - 2.56 (m, 1H), 2.78 - 2.89 (m, 1H), 3.07 - 3.19 (m, 1H), 3.38 (s, 3H), 3.75 (s, 3H), 6.46 - 6.58 (m, 1H), 6.62 - 6.73 (m, 2H), 7.02 - 7.14 (m, 2H), 7.18 - 7.28 (m, 1H), 7.37 (br.s, 1H), 8.44 (s, 1H).

### Beispiel 235

### 3-(4,6-Difluor-2,3-dihydro-1H-inden-1-yl)-1-(3-methyl-2-oxo-2,3-dihydro-1,3-benzothiazol-6-yl)-2,4-dioxo-1,2,3,4-tetrahydropyrimidin-5-carbonsäure (Racemat)

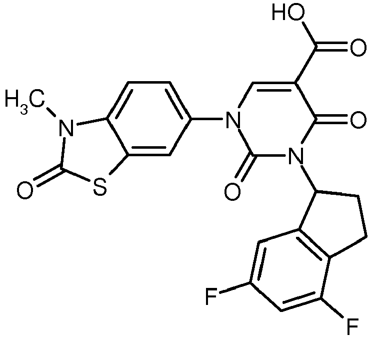

170 mg (0.34 mmol) der Verbindung aus Beispiel 108 wurden in 7 ml Eisessig und 3.5 ml konz. Salzsäure 1 h zum Rückfluss erhitzt. Nach Abkühlung auf RT wurde das Reaktionsgemisch per präparativer HPLC (Methode 7) gereinigt. Man erhielt 133 mg (83 % d. Th.) der Titelverbindung.
LC-MS (Methode 1): Rₜ = 1.07 min; m/z = 472 (M+H)⁺.
¹H-NMR (400MHz, CD₂Cl₂): δ [ppm] = 2.32 - 2.45 (m, 1H), 2.52 - 2.64 (m, 1H), 2.84 - 2.97 (m, 1H), 3.14 - 3.26 (m, 1H), 3.38 (s, 3H), 6.44 - 6.56 (m, 1H), 6.58 - 6.70 (m, 2H), 7.07 (d, 1H), 7.23 (d, 2H), 7.37 (br.s, 1H), 8.46 (s, 1H).

### Beispiel 236

### 3-(6-Methyl-2,3-dihydro-1H-inden-1-yl)-1-(3-methyl-2-oxo-2,3-dihydro-1,3-benzothiazol-6-yl)-2,4-dioxo-1,2,3,4-tetrahydropyrimidin-5-carbonsäure (Racemat)

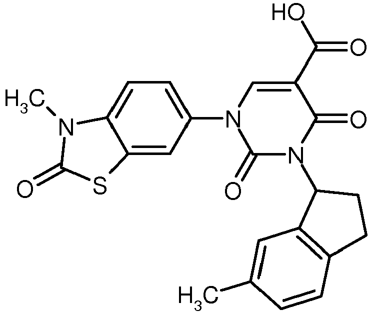

127 mg (0.27 mmol) der Verbindung aus Beispiel 109 wurden analog zu Beispiel 232 unter alkalischen Bedingungen hydrolysiert und das Produkt gereinigt. Man erhielt 56 mg (47 % d. Th.) der Titelverbindung.
LC-MS (Methode 1): Rₜ = 1.10 min; m/z = 450 (M+H)⁺.
¹H-NMR (400MHz, CD₂Cl₂): δ [ppm] = 2.22 (s, 3H), 2.28 - 2.39 (m, 1H), 2.43 - 2.55 (m, 1H), 2.82 - 2.94 (m, 1H), 3.12 - 3.24 (m, 1H), 3.38 (s, 3H), 6.44 - 6.55 (m, 1H), 6.86 (s, 1H), 6.98 (d, 1H), 7.02 - 7.12 (m, 2H), 7.24 (d, 1H), 7.38 (br.s, 1H), 8.45 (s, 1H).

### Beispiel 237

### Ethyl-3-[6-chlor-4-(trifluormethyl)-2,3-dihydro-1H-inden-1-yl]-1-(1,3-dimethyl-2-oxo-2,3-dihydro-1H-benzimidazol-5-yl)-2,4-dioxo-1,2,3,4-tetrahydropyrimidin-5-carboxylat (Racemat)

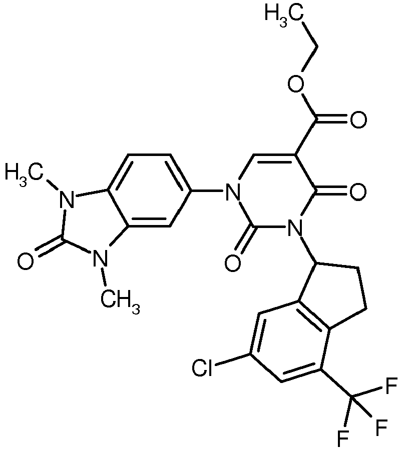

Analog zu Beispiel 233 wurden 200 mg (0.58 mmol) der Verbindung aus Beispiel 2A mit 179 mg (0.76 mmol) 6-Chlor-4-(trifluormethyl)indan-1-ol aus Beispiel 108A umgesetzt und das Produkt isoliert. Man erhielt 260 mg (69 % d. Th.) der Titelverbindung in 87 %-iger Reinheit.
LC-MS (Methode 1): Rₜ = 1.14 min; m/z = 563 (M+H)⁺.
¹H-NMR (400MHz, DMSO-d₆): δ [ppm]= 1.22 (br. t, 3H), 2.36 - 2.55 (m, 2H, teilweise vom DMSO-Signal verdeckt), 3.00 - 3.14 (m, 1H), 3.14 - 3.29 (m, 1H), 3.31 (s, 3H), 3.37 (s, 3H), 4.13 - 4.25 (m, 2H), 6.29 - 6.54 (m, 1H), 7.18 - 7.31 (m, 2H), 7.39 (br.s, 1H), 7.59 (s, 1H), 7.68 (br.s, 1H), 8.34 (s, 1H).

### Beispiel 238

### 3-[6-Chlor-4-(trifluormethyl)-2,3-dihydro-1H-inden-1-yl]-1-(1,3-dimethyl-2-oxo-2,3-dihydro-1H-benzimidazol-5-yl)-2,4-dioxo-1,2,3,4-tetrahydropyrimidin-5-carbonsäure (Racemat)

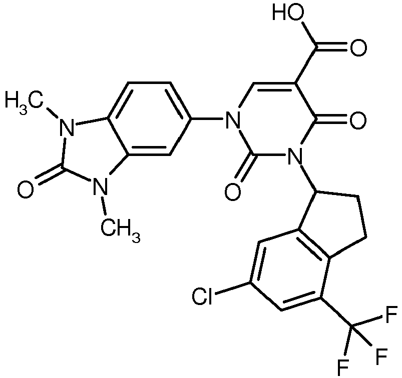

Analog zu Beispiel 217 wurden 260 mg (0.46 mmol) der Verbindung aus Beispiel 237 hydrolysiert und das Produkt isoliert. Man erhielt 200 mg (79 % d. Th.) der Titelverbindung.
LC-MS (Methode 1): Rₜ = 1.09 min; m/z = 535 (M+H)⁺.
¹H-NMR (400MHz, CD₂Cl₂): δ [ppm] = 2.35 - 2.46 (m, 1H), 2.58 (s, 1H), 3.00 - 3.12 (m, 1H), 3.31 (s, 3H), 3.33 (s, 3H), 3.35 - 3.44 (m, 1H), 6.49 - 6.60 (m, 1H), 6.87 (s, 1H), 6.96 (s, 2H), 7.27 (s, 1H), 7.45 (s, 1H), 8.50 (s, 1H).

### Beispiel 239

### Ethyl-3-[6-brom-4-(trifluormethyl)-2,3-dihydro-1H-inden-1-yl]-1-(1,3-dimethyl-2-oxo-2,3-dihydro-1H-benzimidazol-5-yl)-2,4-dioxo-1,2,3,4-tetrahydropyrimidin-5-carboxylat (Racemat)

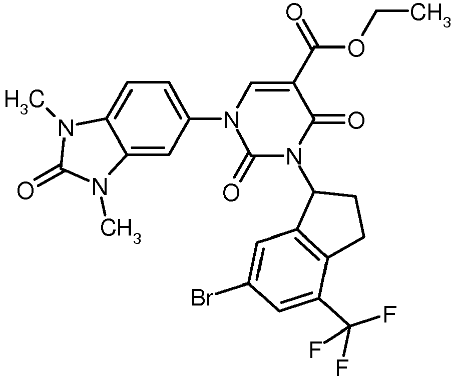

Analog zu Beispiel 233 wurden 226 mg (0.66 mmol) der Verbindung aus Beispiel 2A mit 240 mg (0.85 mmol) 6-Brom-4-(trifluormethyl)indan-1-ol aus Beispiel 113A umgesetzt und das Produkt isoliert. Man erhielt 230 mg (58 % d. Th.) der Titelverbindung.
LC-MS (Methode 1): Rₜ = 1.17 min; m/z = 607 / 609 (M+H)⁺.
¹H-NMR (400MHz, CD₂Cl₂): δ [ppm]= 1.23 (t, 2H), 2.28 - 2.43 (m, 1H), 2.52 (dtd, 1H), 3.00 (dt, 1H), 3.31 (s, 3H), 3.33 (s, 2H), 3.29 - 3.41 (m, 1H, teilweise von den Methyl-Signalen verdeckt), 4.21 (q, 2H), 6.42 - 6.65 (m, 1H), 6.88 (br.s, 1H), 6.96 (s, 2H), 7.40 (s, 1H), 7.54 (s, 1H), 8.24 (s, 1H).

### Beispiel 240

### 3-[6-Brom-4-(trifluormethyl)-2,3-dihydro-1H-inden-1-yl]-1-(1,3-dimethyl-2-oxo-2,3-dihydro-1H-benzimidazol-5-yl)-2,4-dioxo-1,2,3,4-tetrahydropyrimidin-5-carbonsäure (Racemat)

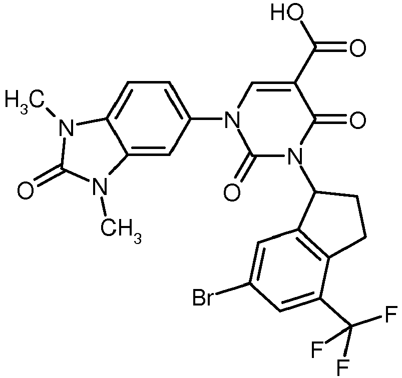

Analog zu Beispiel 217 wurden 52 mg (86 µmol) der Verbindung aus Beispiel 238 hydrolysiert und das Produkt isoliert. Man erhielt 23 mg (46 % d. Th.) der Titelverbindung.
LC-MS (Methode 5): Rₜ = 1.15 min; m/z = 579 (M+H)⁺.
¹H-NMR (400MHz, CD₂Cl₂): δ [ppm] = 2.41 - 2.54 (m, 1H), 2.61 - 2.74 (m, 1H), 3.06 - 3.18 (m, 1H), 3.39 (s, 3H), 3.42 (s, 3H), 3.43 - 3.51 (m, 1H), 6.57 - 6.69 (m, 1H), 6.95 (s, 1H), 7.05 (s, 2H), 7.50 (s, 1H), 7.67 (s, 1H), 8.58 (s, 1H),

### Beispiel 241

### 1-[1-Methyl-2-oxo-3-(2,2,2-trifluorethyl)-2,3-dihydro-1H-benzimidazol-5-yl]-2,4-dioxo-3-[(1R)-4-(trifluormethyl)-2,3-dihydro-1H-inden-1-yl]-1,2,3,4-tetrahydropyrimidin-5-carbonsäure (R-Enantiomer)

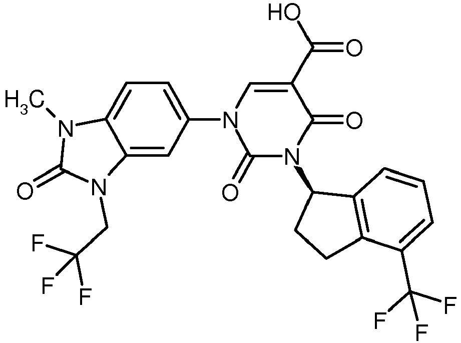

Analog zu Beispiel 217 wurden 370 mg (0.62 mmol) der Verbindung aus Beispiel 28 hydrolysiert und das Produkt isoliert. Man erhielt 314 mg (89 % d. Th.) der Titelverbindung.
LC-MS (Methode 1): Rₜ = 1.15 min; m/z = 569 (M+H)⁺.
¹H-NMR (400MHz, CD₂Cl₂): δ [ppm] = 2.32 - 2.46 (m, 1H), 2.51 - 2.65 (m, 1H), 3.03 - 3.17 (m, 1H), 3.36 (s, 3H), 3.40 - 3.48 (m, 1H), 4.41 (q, 2H), 6.51 - 6.63 (m, 1H), 6.96 (s, 1H), 7.00 - 7.09 (m, 2H), 7.21 - 7.30 (m, 2H), 7.46 (d, 1H), 8.48 (s, 1H).

### Beispiel 242

### 1-(1,3-Dimethyl-2-oxo-2,3-dihydro-1H-benzimidazol-5-yl)-3-[6-fluor-4-(trifluormethyl)-2,3-dihydro-1H-inden-1-yl]-2,4-dioxo-1,2,3,4-tetrahydropyrimidin-5-carbonsäure (Racemat)

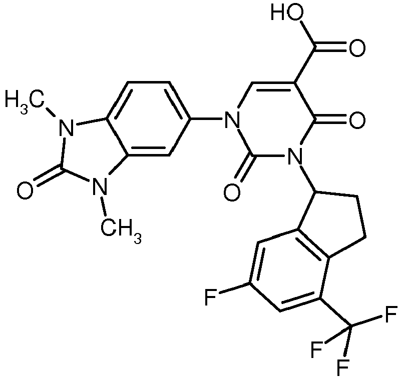

Analog zu Beispiel 217 wurden 63 mg (115 µmol) der Verbindung aus Beispiel 110 hydrolysiert und das Produkt isoliert. Man erhielt 47 mg (78 % d. Th.) der Titelverbindung.
LC-MS (Methode 1): Rₜ = 1.09 min; m/z = 519 (M+H)⁺.
¹H-NMR (400MHz, CD₂Cl₂): δ [ppm] = 2.44 - 2.57 (m, 1H), 2.63 - 2.76 (m, 1H), 3.07 - 3.19 (m, 1H), 3.39 (s, 3H), 3.42 (s, 3H), 3.43 - 3.50 (m, 1H), 6.56 - 6.68 (m, 1H), 6.94 (s, 1H), 7.01 - 7.10 (m, 3H), 7.23 - 7.30 (m, 1H), 8.58 (s, 1H), 12.36 (br. s, 1H).

### Beispiel 243

### 1-(1-Methyl-2-oxo-2,3-dihydro-1H-benzimidazol-5-yl)-2,4-dioxo-3-[(1R)-4-(trifluormethyl)-2,3-dihydro-1H-inden-1-yl]-1,2,3,4-tetrahydropyrimidin-5-carbonsäure (R-Enantiomer)

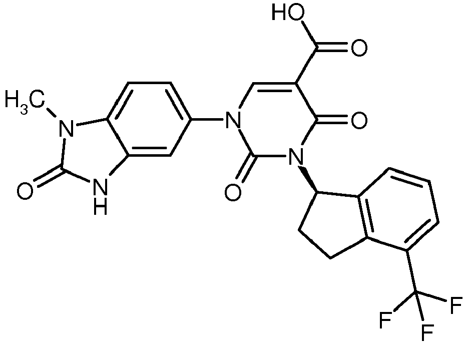

Analog zu Beispiel 217 wurden 600 mg (1.17 mmol) der Verbindung aus Beispiel 112 hydrolysiert (Reaktionszeit 4h) und das Produkt isoliert. Man erhielt 540 mg (89 % d. Th.) der Titelverbindung.
LC-MS (Methode 3): Rₜ = 2.20 min; m/z = 487 (M+H)⁺.
¹H-NMR (400MHz, CD₂Cl₂): δ [ppm] = 2.32 - 2.45 (m, 1H), 2.51 - 2.64 (m, 1H), 3.02 - 3.17 (m, 1H), 3.31 (s, 3H), 3.36 - 3.47 (m, 1H), 6.52 - 6.61 (m, 1H), 6.96 (s, 3H), 7.21 - 7.31 (m, 2H), 7.42 - 7.50 (m, 1H), 8.14 (s, 1H), 8.47 (s, 1H), 12.36 (br. s, 1H).

### Beispiel 244

### 1-(3-Ethyl-1-methyl-2-oxo-2,3-dihydro-1H-benzimidazol-5-yl)-2,4-dioxo-3-[(1R)-4-(trifluormethyl)-2,3-dihydro-1H-inden-1-yl]-1,2,3,4-tetrahydropyrimidin-5-carbonsäure (R-Enantiomer)

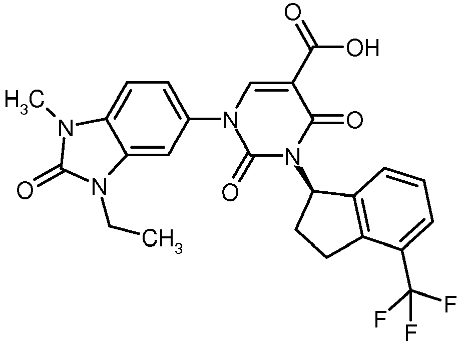

Analog zu Beispiel 217 wurden 73 mg (0.14 mmol) der Verbindung aus Beispiel 113 hydrolysiert und das Produkt isoliert. Man erhielt 58 mg (82 % d. Th.) der Titelverbindung.
LC-MS (Methode 3): Rₜ = 2.36 min; m/z = 515 (M+H)⁺.
¹H-NMR (400MHz, CD₂Cl₂): δ [ppm] = 1.22 (t, 3H), 2.33 - 2.45 (m, 1H), 2.52 - 2.64 (m, 1H), 3.04 - 3.17 (m, 1H), 3.33 (s, 3H), 3.37 - 3.48 (m, 1H), 3.83 (q, 2H), 6.51 - 6.61 (m, 1H), 6.87 (s, 1H), 6.92 - 7.01 (m, 2H), 7.21 - 7.31 (m, 2H), 7.46 (d, 1H), 8.49 (s, 1H), 12.35 (br. s, 1H).

### Beispiel 245

### 1-(3-Methyl-2-oxo-2,3-dihydro-1,3-benzothiazol-6-yl)-2,4-dioxo-3-[7-(trifluormethyl)-2,3-dihydro-1-benzofur-3-yl]-1,2,3,4-tetrahydropyrimidin-5-carbonsäure (Racemat)

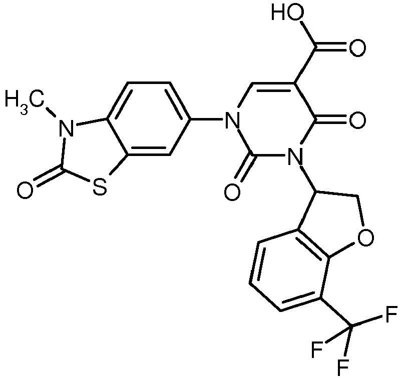

Analog zu Beispiel 234 wurden 32 mg (60 µmol) der Verbindung aus Beispiel 119 hydrolysiert und das Produkt isoliert. Man erhielt 19 mg (63 % d. Th.) der Titelverbindung.
LC-MS (Methode 5): Rₜ = 1.04 min; m/z = 506 (M+H)⁺.
¹H-NMR (400MHz, CD₂Cl₂): δ [ppm] = 3.38 (s, 3H), 4.74 - 4.88 (m, 2H), 6.79 (dd, 1H), 6.91 (t, 1H), 7.07 (d, 1H), 7.21 (dd, 1H), 7.33 (d, 1H), 7.37 (d, 1H), 7.41 (d, 1H), 8.47 (s, 1H), 11.67 - 12.36 (br.s., 1H).

### Beispiel 246

### 1-[1-Methyl-2-oxo-3-(3,3,3-trifluor-2-hydroxypropyl)-2,3-dihydro-1H-benzimidazol-5-yl]-2,4-dioxo-3-[(1R)-4-(trifluormethyl)-2,3-dihydro-1H-inden-1-yl]-1,2,3,4-tetrahydropyrimidin-5-carbonsäure (Diatereomerengemisch)

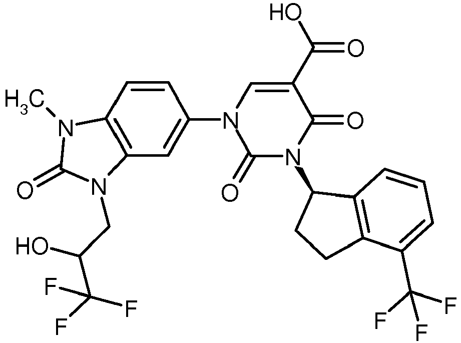

Analog zu Beispiel 217 wurden 180 mg (0.29 mmol) der Verbindung aus Beispiel 115 hydrolysiert und das Produkt isoliert. Man erhielt 152 mg (83 % d. Th.) der Titelverbindung.
LC-MS (Methode 1): Rₜ = 1.11 min; m/z = 599 (M+H)⁺.
¹H-NMR (400MHz, CD₂Cl₂): δ [ppm] = 2.32 - 2.46 (m, 1H), 2.52 - 2.65 (m, 1H), 3.03 - 3.16 (m, 1H), 3.37 (s, 3H), 3.42 - 3.51 (m, 1H), 3.99 - 4.08 (m, 1H), 4.16 (d, 1H), 4.22 - 4.37 (m, 2H), 6.50 - 6.64 (m, 1H), 7.03 (d, 3H), 7.20 - 7.32 (m, 2H), 7.46 (d, 1H), 8.47 (s, 1H), 12.29 (br. s, 1H).

### Beispiel 247

### 1-[1-Methyl-2-oxo-3-(3,3,3-trifluorpropyl)-2,3-dihydro-1H-benzimidazol-5-yl]-2,4-dioxo-3-[(1R)-4-(trifluormethyl)-2,3-dihydro-1H-inden-1-yl]-1,2,3,4-tetrahydropyrimidin-5-carbonsäure (R-Enantiomer)

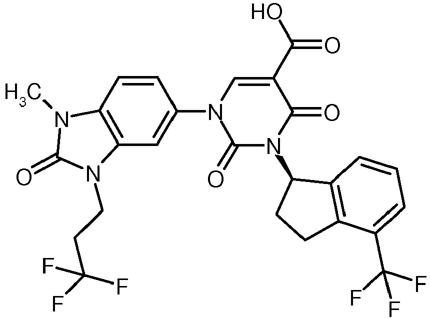

Analog zu Beispiel 217 wurden 160 mg (0.26 mmol) der Verbindung aus Beispiel 116 hydrolysiert und das Produkt isoliert. Man erhielt 140 mg (91 % d. Th.) der Titelverbindung.
LC-MS (Methode 5): Rₜ = 1.16 min; m/z = 583 (M+H)⁺.
¹H-NMR (400MHz, CD₂Cl₂): δ [ppm] = 2.47 - 2.58 (m, 1H), 2.60 - 2.77 (m, 3H), 3.17 - 3.29 (m, 1H), 3.47 (s, 3H), 3.49 - 3.61 (m, 1H), 4.16 (t, 2H), 6.63 - 6.76 (m, 1H), 7.00 (s, 1H), 7.08 - 7.16 (m, 2H), 7.35 - 7.44 (m, 2H), 7.59 (d, 1H), 8.61 (s, 1H), 12.46 (br. s, 1H).

### Beispiel 248

### 1-(3-Cyclopropyl-1-methyl-2-oxo-2,3-dihydro-1H-benzimidazol-5-yl)-2,4-dioxo-3-[(1R)-4-(trifluormethyl)-2,3-dihydro-1H-inden-1-yl]-1,2,3,4-tetrahydropyrimidin-5-carbonsäure (R-Enantiomer)

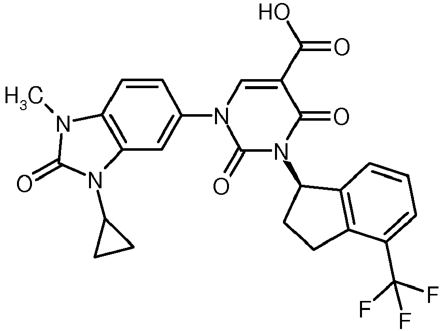

Analog zu Beispiel 234 wurden 160 mg (0.26 mmol) der Verbindung aus Beispiel 117 hydrolysiert. Das Reaktionsgemisch wurde mit 5 ml Acetonitril verdünnt und per präparativer HPLC (Methode 7) gereinigt. Man erhielt 140 mg (91 % d. Th.) der Titelverbindung.
LC-MS (Methode 1): Rₜ = 1.07 min; m/z = 527 (M+H)⁺.
¹H-NMR (400MHz, CD₂Cl₂): δ [ppm] = 0.84 - 0.93 (m, 2H), 0.95 - 1.03 (m, 2H), 2.34 - 2.48 (m, 1H), 2.52 - 2.64 (m, 1H), 2.73 - 2.83 (m, 1H), 3.05 - 3.16 (m, 1H), 3.28 (s, 3H), 3.36 - 3.49 (m, 1H), 6.51 - 6.63 (m, 1H), 6.89 - 6.99 (m, 2H), 7.06 (s, 1H), 7.21 - 7.32 (m, 2H), 7.46 (d, 1H), 8.49 (s, 1H).

### Beispiel 249

### Ethyl-3-(4,6-dichlor-2,3-dihydro-1H-inden-1-yl)-1-(3-methyl-2-oxo-2,3-dihydro-1,3-benzothiazol-6-yl)-2,4-dioxo-1,2,3,4-tetrahydropyrimidin-5-carbonxylat (Racemat)

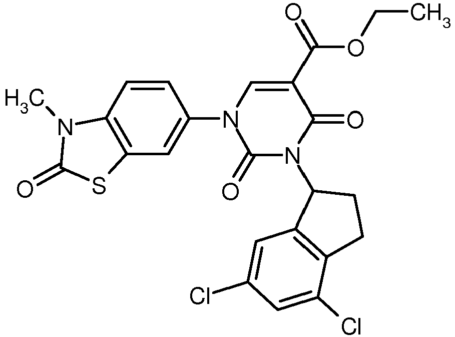

Unter Argon wurden 101 mg (0.29 mmol) der Verbindung aus Beispiel 31A, 71 mg (0.35 mmol) 4,6-Dichlorindan-1-ol aus Beispiel 114A und 137 mg (0.52 mmol) Triphenylphosphin in 8 ml THF/ DMF 1:1 (v/v) vorgelegt und mit 97 µl (0.49 mmol) Diisopropylazodicarboxylat tropfenweise versetzt. Das Gemisch wurde 1 h bei RT gerührt. Unter Eiskühlung wurden 2 ml IN Salzsäure zugefügt, die Mischung 15 min weiter gerührt und anschließend komplett über präparative HPLC (Methode 7) gereinigt. Man erhielt 101 mg (65 % d. Th.) der Titelverbindung.
LC-MS (Methode 1): Rₜ = 1.20 min; m/z = 532 (M+H)⁺.
¹H-NMR (400MHz, CD₂Cl₂): δ [ppm]= 1.36 (t, 3H), 2.37 - 2.52 (m, 1H), 2.63 (dtd, 1H), 2.93 - 3.08 (m, 1H), 3.25 - 3.40 (m, 1H), 3.51 (s, 3H), 4.34 (q, 2H), 6.65 (br.s, 1H), 7.09 (s, 1H), 7.19 (d, 1H), 7.29 (s, 1H), 7.36 (d, 1H), 7.50 (br.s, 1H), 8.34 (s, 1H).

### Beispiel 250

### 3-(4,6-Dichlor-2,3-dihydro-1H-inden-1-yl)-1-(3-methyl-2-oxo-2,3-dihydro-1,3-benzothiazol-6-yl)-2,4-dioxo-1,2,3,4-tetrahydropyrimidin-5-carbonsäure (Racemat)

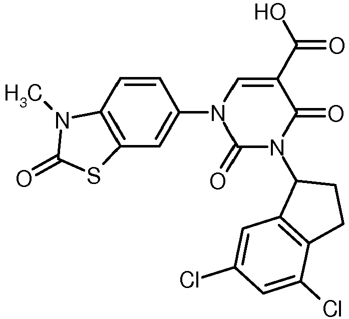

Analog zu Beispiel 217 wurden 106 mg (0.20 mmol) der Verbindung aus Beispiel 249 hydrolysiert und das Produkt isoliert. Man erhielt 74 mg (73 % d. Th.) der Titelverbindung.
LC-MS (Methode 1): Rₜ = 1.17 min; m/z = 505 (M+H)⁺.
¹H-NMR (500MHz, CD₂Cl₂): δ [ppm] = 2.37 - 2.51 (m, 1H), 2.60 - 2.70 (m, 1H), 2.95 - 3.06 (m, 1H), 3.24 - 3.36 (m, 1H), 3.47 (s, 3H), 6.54 - 6.71 (m, 1H), 7.07 (s, 1H), 7.12 - 7.20 (m, 1H), 7.29 (s, 1H), 7.31 - 7.38 (m, 1H), 7.41 - 7.54 (m, 1H), 8.56 (s, 1H).

### Beispiel 251

### Ethyl-1-(1,3-Dimethyl-2-oxo-2,3-dihydro-1H-benzimidazol-5-yl)-3-{1-[2-methyl-3-(trifluormethyl)-phenyl]ethyl}-2,4-dioxo-1,2,3,4-tetrahydropyrimidin-5-carboxylat (Racemat)

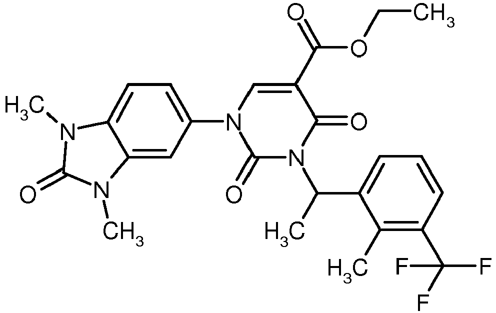

Unter Argon wurden 250 mg (0.73 mmol) der Verbindung aus Beispiel 2A, 198 mg (90 %-ige Reinheit, 0.87 mmol) 1-[2-Methyl-3-(trifluormethyl)phenyl]ethanol aus Beispiel 115A und 324 mg (1.23 mmol) Triphenylphosphin in 6.5 ml THF / DMF 1:2 (v/v) vorgelegt und mit 229 µl (1.16 mmol) Diisopropylazodicarboxylat tropfenweise versetzt. Das Gemisch wurde 1 h bei RT gerührt. Unter Eiskühlung wurde 1 ml IN Salzsäure zugefügt, die Mischung 10 min weiter gerührt und anschließend per präparativer HPLC (Methode 7) gereinigt. Man erhielt 153 mg (40 % d. Th.) der Titelverbindung.
LC-MS (Methode 1): Rₜ = 1.05 min; m/z = 531 (M+H)⁺.
¹H-NMR (400MHz, CD₂Cl₂): δ [ppm]= 1.30 (t, 3H), 1.87 (d, 3H), 2.34 (s, 3H), 3.38 (s, 3H), 3.40 (s, 3H), 4.27 (q, 2H), 6.30 (q, 1H), 6.90 (d, 1H), 6.95 - 7.07 (m, 2H), 7.31 (t, 1H), 7.58 (d, 1H), 7.92 (d, 1H), 8.28 (s, 1H).

### Beispiel 252

### 1-(1,3-Dimethyl-2-oxo-2,3-dihydro-1H-benzimidazol-5-yl)-3-{1-[2-methyl-3-(trifluormethyl)phenyl]ethyl}-2,4-dioxo-1,2,3,4-tetrahydropyrimidin-5-carbonsäure (Racemat)

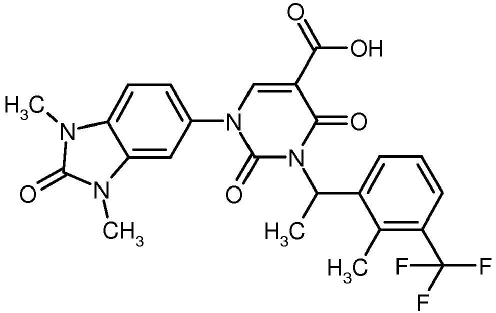

Analog zu Beispiel 217 wurden 140 mg (0.26 mmol) der Verbindung aus Beispiel 251 hydrolysiert und das Produkt isoliert. Man erhielt 79 mg (58 % d. Th.) der Titelverbindung.
LC-MS (Methode 1): Rₜ = 1.08 min; m/z = 503 (M+H)⁺.
¹H-NMR (400MHz, CD₂Cl₂): δ [ppm]= 1.92 (d, 3H), 2.35 (s, 3H), 3.38 (s, 3H), 3.41 (s, 3H), 6.35 (q, 1H), 6.90 (d, 1H), 6.97 - 7.05 (m, 2H), 7.34 (t, 1H), 7.62 (d, 1H), 7.93 (d, 1H), 8.53 (s, 1H), 12.5 (br.s, 1H).
Durch präparative HPLC an einer chiralen Phase (Methode 16) wurde das Produkt in seine Enantiomeren getrennt: siehe Beispiele 253 und 254.

### Beispiel 253

### 1-(1,3-Dimethyl-2-oxo-2,3-dihydro-1H-benzimidazol-5-yl)-3-{1-[2-methyl-3-(trifluormethyl)phenyl]ethyl}-2,4-dioxo-1,2,3,4-tetrahydropyrimidin-5-carbonsäure (Enantiomer 1)

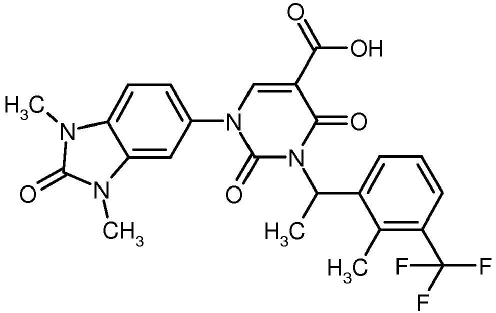

Zuerst eluierendes Enantiomer aus der präparative Trennung von 65 mg der Verbindung aus Beispiel 252 nach Methode 16. Nach Trocknen im HV erhielt man 25 mg der Titelverbindung.
Chirale analytische HPLC (Methode 17): Rₜ = 10.6 min

### Beispiel 254

### 1-(1,3-Dimethyl-2-oxo-2,3-dihydro-1H-benzimidazol-5-yl)-3-{1-[2-methyl-3-(trifluormethyl)phenyl]ethyl}-2,4-dioxo-1,2,3,4-tetrahydropyrimidin-5-carbonsäure (Enantiomer 2)

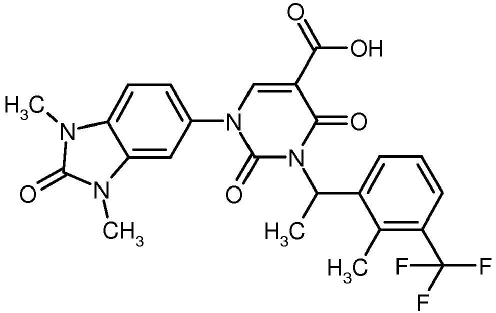

Zuletzt eluierendes Enantiomer aus der präparative Trennung von 65 mg der Verbindung aus Beispiel 252 nach Methode 16. Nach Trocknen im HV erhielt man 28 mg der Titelverbindung.
Chirale analytische HPLC (Methode 17): Rₜ = 11.5 min

### Beispiel 255

### Ethyl-3-{1-[2-chlor-3-(trifluormethyl)phenyl]ethyl}-1-(3-methyl-2-oxo-2,3-dihydro-1,3-benzoxazol-6-yl)-2,4-dioxo-1,2,3,4-tetrahydropyrimidin-5-carboxylat (Racemat)

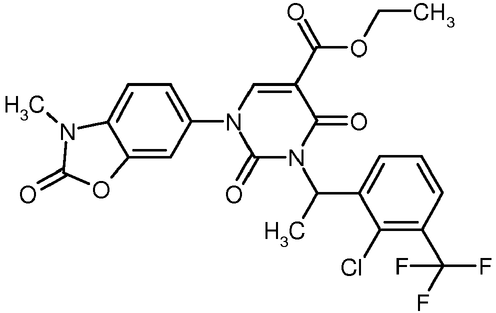

Analog zu Beispiel 251 wurden 500 mg (1.51 mmol) der Verbindung aus Beispiel 28A mit 508 mg (80 %-ige Reinheit, 1.81 mmol) 1-[2-Chlor-3-(trifluormethyl)phenyl]ethanol aus Beispiel 116A umgesetzt und das Produkt gereinigt. Man erhielt 435 mg (54 % d. Th.) der Titelverbindung.
LC-MS (Methode 4): Rₜ = 2.38 min; m/z = 538 (M+H)⁺.
¹H-NMR (500MHz, CD₂Cl₂): δ [ppm]= 1.22 - 1.35 (m, 3H), 1.87 (d, 3H), 3.40 (s, 3H), 4.26 (q, 2H), 6.30 (q, 1H), 7.05 (d, 1H), 7.11 - 7.17 (m, 1H), 7.20 (d, 1H), 7.42 (t, 1H), 7.67 (d, 1H), 7.97 (d, 1H), 8.23 (s, 1H).

### Beispiel 256

### 3-{1-[2-Chlor-3-(trifluormethyl)phenyl]ethyl}-1-(3-methyl-2-oxo-2,3-dihydro-1,3-benzoxazol-6-yl)-2,4-dioxo-1,2,3,4-tetrahydropyrimidin-5-carbonsäure (Racemat)

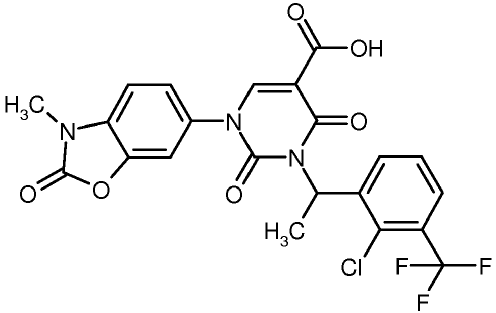

Analog zu Beispiel 217 wurden 400 mg (0.74 mmol) der Verbindung aus Beispiel 255 hydrolysiert und das Produkt isoliert. Man erhielt 320 mg (84 % d. Th.) der Titelverbindung.
LC-MS (Methode 1): Rₜ = 1.04 min; m/z = 510 (M+H)⁺.
¹H-NMR (400MHz, CD₂Cl₂): δ [ppm]= 1.91 (d, 3H), 3.41 (s, 3H), 6.37 (q, 1H), 7.05 - 7.09 (m, 1H), 7.15 (dd, 1H), 7.21 (d, 1H), 7.45 (t, 1H), 7.71 (d, 1H), 7.97 (d, 1H), 8.50 (s, 1H), 12.37 (br. s, 1H).
Durch präparative HPLC an einer chiralen Phase (Methode 18) wurde das Produkt in seine Enantiomeren getrennt: siehe Beispiele 257 und 258.

### Beispiel 257

### 3-{1-[2-Chlor-3-(trifluormethyl)phenyl]ethyl}-1-(3-methyl-2-oxo-2,3-dihydro-1,3-benzoxazol-6-yl)-2,4-dioxo-1,2,3,4-tetrahydropyrimidin-5-carbonsäure (Enantiomer 1)

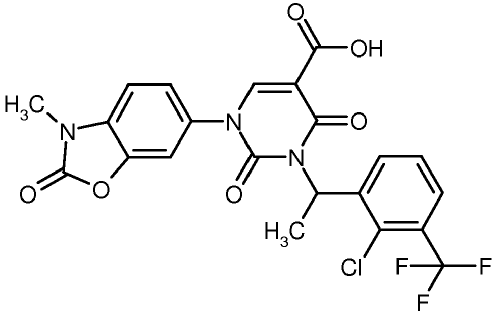

Zuerst eluierendes Enantiomer aus der präparative Trennung von 300 mg der Verbindung aus Beispiel 256 nach Methode 18. Nach Trocknen im HV erhielt man 129 mg der Titelverbindung.
Chirale analytische HPLC (Methode 19): Rt = 7.4 min

### Beispiel 258

### 3-{1-[2-Chlor-3-(trifluormethyl)phenyl]ethyl}-1-(3-methyl-2-oxo-2,3-dihydro-1,3-benzoxazol-6-yl)-2,4-dioxo-1,2,3,4-tetrahydropyrimidin-5-carbonsäure (Enantiomer 2)

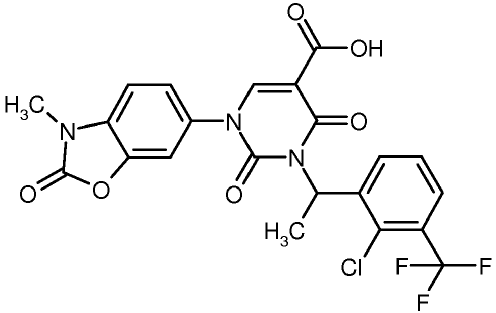

Zuletzt eluierendes Enantiomer aus der präparative Trennung von 300 mg der Verbindung aus Beispiel 256 nach Methode 18. Nach Trocknen im HV erhielt man 128 mg der Titelverbindung.
Chirale analytische HPLC (Methode 19): 16.6 min

### Beispiel 259

### Ethyl-1-(3-Methyl-2-oxo-2,3-dihydro-1,3-benzoxazol-6-yl)-3-{1-[2-methyl-3-(trifluormethyl)phenyl]ethyl}-2,4-dioxo-1,2,3,4-tetrahydropyrimidin-5-carboxylat (Racemat)

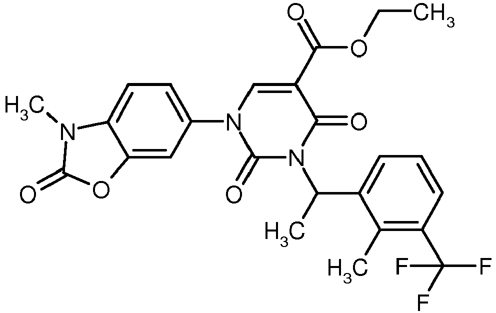

Analog zu Beispiel 251 wurden 500 mg (1.51 mmol) der Verbindung aus Beispiel 28A mit 411 mg (90 %-ige Reinheit, 1.81 mmol) 1-[2-Methyl-3-(trifluormethyl)phenyl]ethanol aus Beispiel 115A umgesetzt und das Produkt gereinigt. Man erhielt 285 mg (36 % d. Th.) der Titelverbindung.
LC-MS (Methode 1): Rₜ = 1.09 min; m/z = 518 (M+H)⁺.
¹H-NMR (500MHz, CD₂Cl₂): δ [ppm]= 1.30 (t, 3H), 1.86 (d, 3H), 2.33 (s, 3H), 3.40 (s, 3H), 4.27 (q, 2H), 6.29 (q, 1H), 7.04 (d, 1H), 7.10 - 7.15 (m, 1H), 7.18 (d, 1H), 7.31 (t, 1H), 7.58 (d, 1H), 7.91 (d, 1H), 8.24 (s, 1H).

### Beispiel 260

### 1-(3-Methyl-2-oxo-2,3-dihydro-1,3-benzoxazol-6-yl)-3-{1-[2-methyl-3-(trifluormethyl)phenyl]ethyl}-2,4-dioxo-1,2,3,4-tetrahydropyrimidin-5-carbonsäure (Racemat)

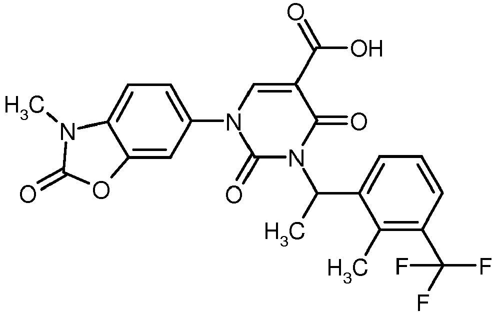

Analog zu Beispiel 217 wurden 260 mg (0.50 mmol) der Verbindung aus Beispiel 259 hydrolysiert und das Produkt isoliert. Man erhielt 200 mg (81 % d. Th.) der Titelverbindung.
LC-MS (Methode 1): Rₜ = 1.07 min; m/z = 490 (M+H)⁺.
¹H-NMR (400MHz, CD₂Cl₂): δ [ppm]= 1.91 (d, 3H), 2.34 (s, 3H), 3.41 (s, 3H), 6.35 (q, 1H), 7.07 (d, 1H), 7.14 (dd, 1H), 7.20 (d, 1H), 7.34 (t, 1H), 7.62 (d, 1H), 7.92 (d, 1H), 8.51 (s, 1H), 12.43 (br. s, 1H).
Durch präparative HPLC an einer chiralen Phase (Methode 20) konnte das Produkt in seine Enantiomeren getrennt werden: siehe Beispiele 261 und 262.

### Beispiel 261

### 1-(3-Methyl-2-oxo-2,3-dihydro-1,3-benzoxazol-6-yl)-3-{1-[2-methyl-3-(trifluormethyl)phenyl]ethyl}-2,4-dioxo-1,2,3,4-tetrahydropyrimidin-5-carbonsäure (Enantiomer 1)

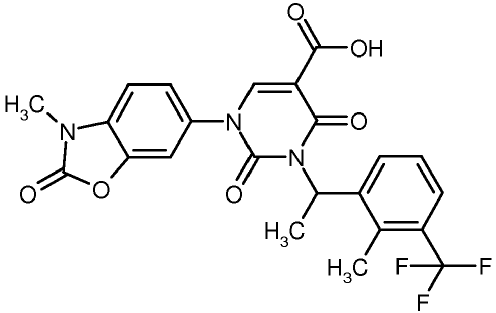

Zuerst eluierendes Enantiomer aus der präparative Trennung von 190 mg der Verbindung aus Beispiel 256 nach Methode 20. Nach Trocknen im HV erhielt man 80 mg der Titelverbindung.
Chirale analytische HPLC (Methode 21): Rₜ = 6.61 min

### Beispiel 262

### 1-(3-Methyl-2-oxo-2,3-dihydro-1,3-benzoxazol-6-yl)-3-{1-[2-methyl-3-(trifluormethyl)phenyl]ethyl}-2,4-dioxo-1,2,3,4-tetrahydropyrimidin-5-carbonsäure (Enantiomer 2)

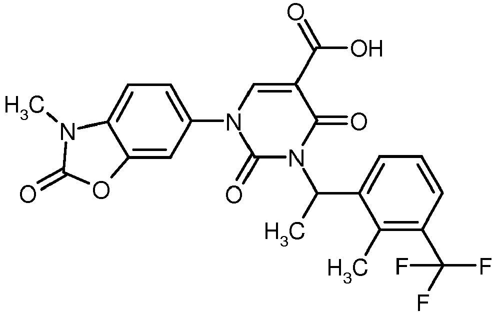

Zuletzt eluierendes Enantiomer aus der präparative Trennung von 190 mg der Verbindung aus Beispiel 256 nach Methode 20. Nach Trocknen im HV erhielt man 82 mg der Titelverbindung.
Chirale analytische HPLC (Methode 21): Rₜ = 10.6 min.

### Beispiel 263

### 1-(6-Methyl-2-oxo-2,3-dihydro-1H-benzimidazol-5-yl)-3-[2-methyl-3-(trifluormethyl)benzyl]-2,4-dioxo-1,2,3,4-tetrahydropyrimidin-5-carbonsäure

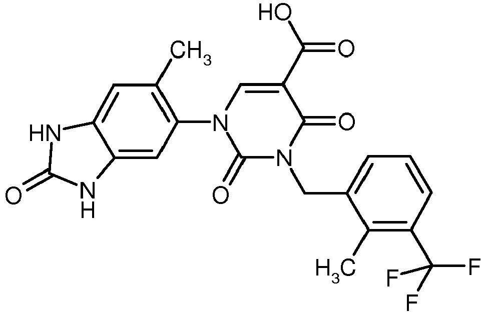

Darstellung und Reinigung der Titelverbindung erfolgten in Analogie zu Beispiel 121 ausgehend von 130 mg (0.26 mmol) Ethyl-1-(6-methyl-2-oxo-2,3-dihydro-1H-benzimidazol-5-yl)-3-[2-methyl-3-(trifluormethyl)-benzyl]-2,4-dioxo-1,2,3,4-tetrahydropyrimidin-5-carboxylat aus Beispiel 184. Das erhaltene Rohprodukt wurde mittels präparativer HPLC (Methode 8) gereinigt. Die eingeengten Produktfraktionen wurden mit Dichlormethan verrührt, der Feststoff abfiltriert und im Vakuum getrocknet. So erhielt man 67 mg (51 % d. Th.) der Titelverbindung.
LC-MS (Methode 1): Rₜ = 0.95 min; m/z = 475 (M+H)⁺.
¹H-NMR (400MHz, DMSO-d₆): δ [ppm] = 2.09 (s, 3H), 2.46 (s, 3H), 5.04 - 5.17 (m, 2H), 6.88 (s, 1H), 7.10 (s, 1H), 7.36 (s, 2H), 7.58 - 7.62 (m, 1H), 8.37 (s, 1H), 10.77 - 10.83 (m, 2H), 12.72 (br.s, 1H).

### Beispiel 264

### 3-[2-Chlor-3-(trifluormethyl)benzyl]-1-(6-methyl-2-oxo-2,3-dihydro-1H-benzimidazol-5-yl)-2,4-dioxo-1,2,3,4-tetrahydropyrimidin-5-carbonsäure

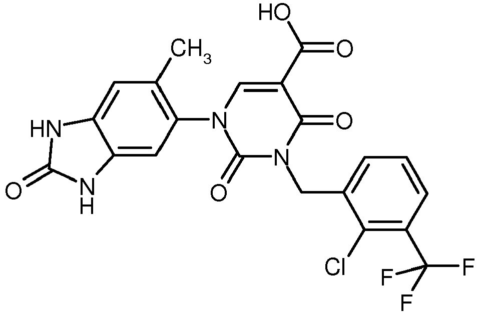

Darstellung und Reinigung der Titelverbindung erfolgten in Analogie zu Beispiel 121 mit einer Reaktionszeit von 1.5h. Ausgehend von 150 mg (0.29 mmol) Ethyl-3-[2-chlor-3-(trifluormethyl)benzyl]-1-(6-methyl-2-oxo-2,3-dihydro-1H-benzimidazol-5-yl)-2,4-dioxo-1,2,3,4-tetrahydropyrimidin-5-carboxylat aus Beispiel 185 erhielt man 126 mg (84 % d. Th.) der Titelverbindung.
LC-MS (Methode 1): Rₜ = 0.96 min; m/z = 495 (M+H)⁺.
¹H-NMR (400MHz, DMSO-d₆): δ [ppm] = 2.10 (s, 3H), 5.17 (s, 2H), 6.88 (s, 1H), 7.10 (s, 1H), 7.50 - 7.60 (m, 2H), 7.78 - 7.83 (m, 1H), 8.39 (s, 1H), 10.80 (s, 2H), 12.69 (br.s, 1H).

### Beispiel 265

### 3-[2-Chlor-3-(trifluormethyl)benzyl]-1-(1-ethyl-3,3-dimethyl-2-oxo-2,3-dihydro-1H-indol-6-yl)-2,4-dioxo-1,2,3,4-tetrahydropyrimidin-5-carbonsäure

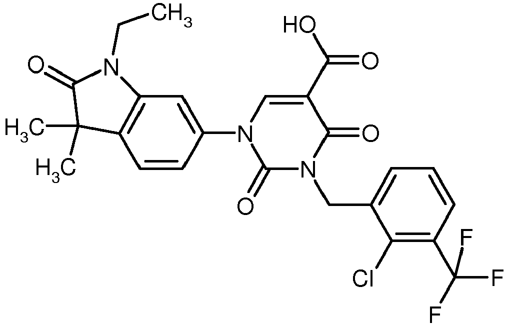

Darstellung und Reinigung der Titelverbindung erfolgten in Analogie zu Beispiel 121 mit einer Reaktionszeit von 30 min. Ausgehend von 50 mg (0.09 mmol) Ethyl-3-[2-chlor-3-(trifluormethyl)benzyl]-1-(1-ethyl-3,3-dimethyl-2-oxo-2,3-dihydro-1H-indol-6-yl)-2,4-dioxo-1,2,3,4-tetrahydropyrimidin-5-carboxylat aus Beispiel 208 erhielt man 26 mg (54 % d. Th.) der Titelverbindung.
LC-MS (Methode 3): Rₜ = 1.37 min; m/z = 536 (M+H)⁺.
¹H-NMR (400MHz, DMSO-d₆): δ [ppm] = 1.11 - 1.19 (m, 3H), 1.30 (s, 6H), 3.65 - 3.75 (m, 2H), 5.18 (s, 2H), 7.17 - 7.23 (m, 1H), 7.31 (s, 1H), 7.48 - 7.57 (m, 2H), 7.57 - 7.63 (m, 1H), 7.77 - 7.85 (m, 1H), 8.52 (s, 1H), 12.73 (br.s, 1H).

### Beispiel 266

### Ethyl-1-(4-methyl-3-oxo-3,4-dihydro-2H-1,4-benzoxazin-7-yl)-3-[2-methyl-3-(trifluormethyl)benzyl]-2,4-dioxo-1,2,3,4-tetrahydropyrimidin-5-carboxylat

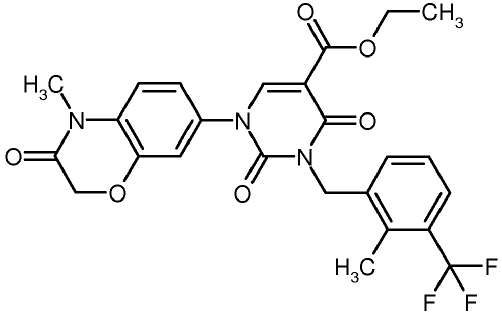

Darstellung und Reinigung erfolgten analog zu Beispiel 216 ausgehend von 200 mg (0.58 mmol) Ethyl-1-(4-methyl-3-oxo-3,4-dihydro-2H-1,4-benzoxazin-7-yl)-2,4-dioxo-1,2,3,4-tetrahydropyrimidin-5-carboxylat aus Beispiel 119A und 147 mg (0.58 mmol) 2-Methyl-3-(trifluormethyl)benzylbromid. Man erhielt 168 mg (53 % d. Th.) der Titelverbindung.
LC-MS (Methode 3): Rₜ = 1.26 min; m/z = 518 (M+H)⁺.
¹H-NMR (400MHz, DMSO-d₆): δ [ppm] = 1.22 (t, 3H), 2.46 (s, 3H), 3.30 (s, teilweise durch Wassersignal verdeckt, 3H), 4.20 (q, 2H), 4.71 (s, 2H), 5.06 (s, 2H), 7.22 - 7.32 (m, 3H), 7.32-7.41 (m, 2H), 7.59 (d, 1H), 8.39 (s, 1H).

### Beispiel 267

### 3-(2-Methyl-3-nitrobenzyl)-1-(4-methyl-3-oxo-3,4-dihydro-2H-1,4-benzoxazin-7-yl)-2,4-dioxo-1,2,3,4-tetrahydropyrimidin-5-carbonsäure

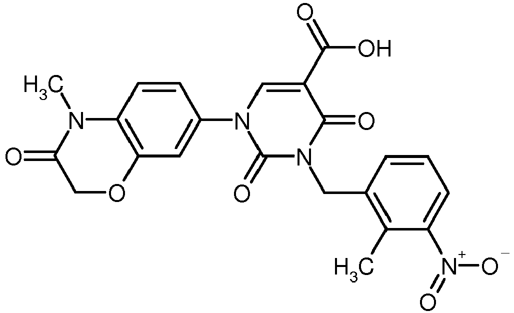

185 mg (0.58 mmol) der Verbindung aus Beispiel 216 wurden in 5 ml Eisessig und 2.5 ml konzentrierter Salzsäure gelöst und 6 h bei 60°C gerührt. Nach Abkühlen auf RT wurden 75 ml Wasser hinzugefügt. Der ausgefallene Feststoff wurde abfiltriert, mit Wasser gewaschen und im HV getrocknet. Man erhielt 129 mg (70 % d. Th.) der Titelverbindung.
LC-MS (Methode 1): Rₜ = 0.96 min; m/z = 467 (M+H)⁺.
¹H-NMR (400MHz, DMSO-d₆): δ [ppm] = 2.41 (s, 3H), 3.30 (s, teilweise durch Wassersignal verdeckt, 3H), 4.72 (s, 2H), 5.10 (s, 2H), 7.22 - 7.32 (m, 3H), 7.37 (t, 1H), 7.44 (d, 1H), 7.72 (d, 1H), 8.41 (s, 1H), 12.71 (br. s, 1H).

### Beispiel 268

### 1-(4-Methyl-3-oxo-3,4-dihydro-2H-1,4-benzoxazin-7-yl)-3-[2-methyl-3-(trifluormethyl)benzyl]-2,4-dioxo-1,2,3,4-tetrahydropyrimidin-5-carbonsäure

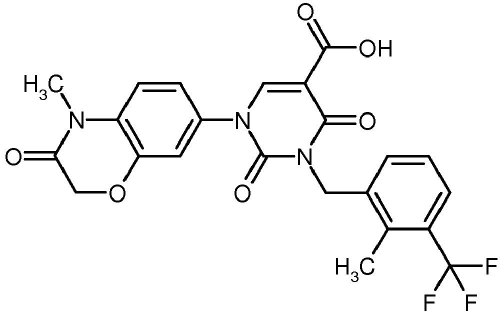

130 mg (0.25 mmol) der Verbindung aus Beispiel 267 wurden in 5 ml Eisessig und 2.5 ml konzentrierter Salzsäure gelöst und 6 h bei 60°C gerührt. Nach Abkühlen auf RT wurden 75 ml Wasser hinzugefügt. Der ausgefallene Feststoff wurde abfiltriert, mit Wasser gewaschen und im HV getrocknet. Man erhielt 109 mg (89 % d. Th.) der Titelverbindung.
LC-MS (Methode 1): Rₜ = 1.09 min; m/z = 490 (M+H)⁺.
¹H-NMR (400MHz, DMSO-d₆): δ [ppm] = 2.48 (s, 3H), 3.30 (s, teilweise durch Wassersignal verdeckt, 3H), 4.72 (s, 2H), 5.10 (s, 2H), 7.22 - 7.42 (m, 5H), 7.60 (d, 1H), 8.42 (s, 1H), 12.70 (br. s, 1H).

### Beispiel 269

### Ethyl-3-(5-chlor-1,2,3,4-tetrahydronaphthalen-1-yl)-1-(1,3-dimethyl-2-oxo-2,3-dihydro-1H-benzimidazol-5-yl)-2,4-dioxo-1,2,3,4-tetrahydropyrimidin-5-carboxylat (Racemat)

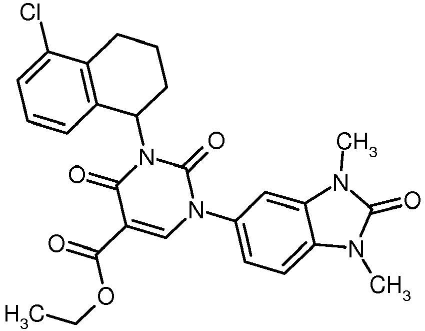

400 mg (1.16 mmol) Ethyl-1-(1,3-dimethyl-2-oxo-2,3-dihydro-1H-benzimidazol-5-yl)-2,4-dioxo-1,2,3,4-tetrahydropyrimidin-5-carboxylat (Beispiel 2A), 255 mg (1.39 mmol) 5-Chlor-1,2,3,4-tetrahydronaphthalen-1-ol und 518 mg (1.98 mmol) Triphenylphosphin wurden in 5 ml THF und 10 ml DMF gelöst. 376 mg (1.86 mmol) DIAD wurden zugegeben und die Mischung 2 h bei RT gerührt. Die Reaktionsmischung wurde mit wenig 1 M wässriger Salzsäure versetzt und komplett mittels präparativer HPLC (Methode 15) getrennt. Man erhielt 510 mg (86 % d. Th.) der Titelverbindung.
LC/MS (Methode 1): Rₜ= 1.09; m/z = 509 (M+H)⁺
¹H-NMR (400MHz, CD₂Cl₂): δ [ppm]= 1.23 (t, 3H), 1.61 - 1.80 (m, 1H), 1.92 - 2.12 (m, 2H), 2.24 -2.44 (m, 1H), 2.53-2.72 (m, 1H), 2.94 (br. d, 1H), 3.30 (s, 3H), 3.32 (s, 3H), 4.21 (br. q, 2H), 6.16 (br. s., 1H), 6.85 (d, 2H), 6.89 - 7.02 (m, 3H), 7.13 (d, 1H), 8.25 (s, 1H).

### Beispiel 270

### 3-(5-Chlor-1,2,3,4-tetrahydronaphthalen-1-yl)-1-(1,3-dimethyl-2-oxo-2,3-dihydro-1H-benzimidazol-5-yl)-2,4-dioxo-1,2,3,4-tetrahydropyrimidin-5-carbonsäure (Racemat)

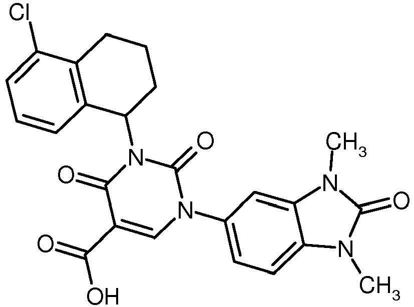

490 mg (0.96 mmol) Ethyl-3-(5-chlor-1,2,3,4-tetrahydronaphthalen-1-yl)-1-(1,3-dimethyl-2-oxo-2,3-dihydro-1H-benzimidazol-5-yl)-2,4-dioxo-1,2,3,4-tetrahydropyrimidin-5-carboxylat aus Beispiel 269 wurden in 3 ml konz. Salzsäure und 7 ml Eisessig bei Rückflusstemperatur gerührt. Nach vollständiger Umsetzung wurde das Reaktionsgemisch abgekühlt und direkt mittels präparativer HPLC (Methode 15) getrennt. Man erhielt 369 mg (80 % d. Th.) der Titelverbindung.
LC/MS (Methode 1): Rₜ= 1.10 min; m/z = 481 (M+H)⁺
¹H-NMR (400MHz, CD₂Cl₂): δ [ppm] = 1.74 - 1.91 (m, 1H), 2.07 - 2.24 (m, 2H), 2.44 (q, 1H), 2.63 - 2.84 (m, 1H), 3.05 (d, 1H), 3.39 (s, 3H), 3.41 (s, 3H), 6.27 (br. s., 1H), 6.84 - 6.98 (m, 2H), 6.98 - 7.15 (m, 3H), 7.25 (d, 1H), 8.59 (s, 1H), 12.5 (br. s, 1H).

### Beispiel 271

### Methyl-3-(5-chlor-1,2,3,4-tetrahydronaphthalen-1-yl)-1-(1,3-dimethyl-2-oxo-2,3-dihydro-1H-benzimidazol-5-yl)-2,4-dioxo-1,2,3,4-tetrahydropyrimidin-5-carboxylat (Racemat)

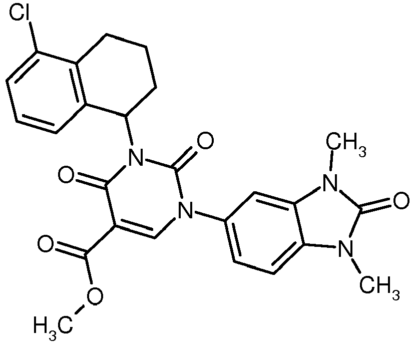

Zu einer Lösung von 300 mg (0.62 mmol) 3-(5-Chlor-1,2,3,4-tetrahydronaphthalen-1-yl)-1-(1,3-dimethyl-2-oxo-2,3-dihydro-1H-benzimidazol-5-yl)-2,4-dioxo-1,2,3,4-tetrahydropyrimidin-5-carbonsäure aus Beispiel 270 in 5 ml Methanol wurden 680 µl (9.36 mmol) Thionylchlorid zugegeben. Das Gemisch wurde 7 Stunden bei Rückflusstemperatur gerührt, dann am Rotationsverdampfer eingeengt. Der Rückstand wurde im Hochvakuum getrocknet. Man erhielt 302 mg (94 % d. Th.) der Titelverbindung.
LC/MS (Methode 28): Rₜ = 3.10 min; m/z = 495 (M+H)⁺

### Beispiel 272

### Methyl-3-(5-chlor-1,2,3,4-tetrahydronaphthalen-1-yl)-1-(1,3-dimethyl-2-oxo-2,3-dihydro-1H-benzimidazol-5-yl)-2,4-dioxo-1,2,3,4-tetrahydropyrimidin-5-carboxylat (Enantiomer 1)

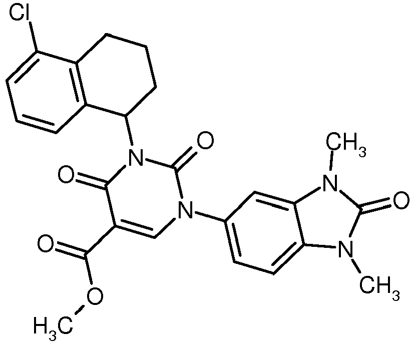

Zuerst eluierendes Enantiomer (56 mg) aus der Trennung von 300 mg der racemischen Substanz aus Beispiel 271 mittels präparativen HPLC an einer chiralen Phase (Methode 29).
Chirale analytische HPLC (Methode 30): Rₜ = 6.14 min, >99 % ee.
Um Lösungsmittelsverunreinigungen zu entfernen wurde das erhaltene Produkt noch mittels präparativer HPLC (Methode 15) gereinigt. Man erhielt 49 mg der Titelverbindung.
¹H-NMR (400 MHz, CD₂Cl₂): δ [ppm] = 1.72 - 1.87 (m, 1H), 2.01 - 2.20 (m, 2H), 2.33 - 2.50 (m, 1H), 2.62 - 2.79 (m, 1H), 3.03 (d, 1H), 3.38 (s, 3H), 3.41 (s, 3H), 3.82 (br. s., 3H), 6.23 (br. s., 1H), 6.85 - 7.13 (m, 5H), 7.21 (d, 1H), 8.36 (s, 1H).

### Beispiel 273

### Methyl-3-(5-chlor-1,2,3,4-tetrahydronaphthalen-1-yl)-1-(1,3-dimethyl-2-oxo-2,3-dihydro-1H-benzimidazol-5-yl)-2,4-dioxo-1,2,3,4-tetrahydropyrimidin-5-carboxylat (Enantiomer 2)

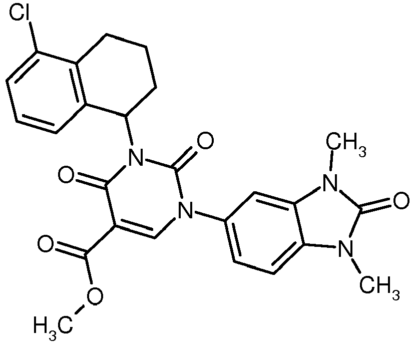

Zuletzt eluierendes Enantiomer (92 mg) aus der Trennung von 300 mg der racemischen Substanz aus Beispiel 271 mittels präparativen HPLC an einer chiralen Phase (Methode 29).
Chirale analytische HPLC (Methode 30): Rₜ = 7.29 min, 97 % ee.
Um Lösungsmittelsverunreinigungen zu entfernen wurde das erhaltene Produkt noch mittels präparativer HPLC (Methode 15) gereinigt. Man erhielt 68 mg der Titelverbindung.
¹H-NMR (400 MHz, CD₂Cl₂): δ [ppm]= 1.73 - 1.88 (m, 1H), 2.01 - 2.21 (m, 2H), 2.33 - 2.51 (m, 1H), 2.62 - 2.80 (m, 1H), 3.03 (d, 1H), 3.38 (s, 3H), 3.41 (s, 3H), 3.82 (br. s., 3H), 6.24 (br. s., 1H), 6.84 - 7.11 (m, 5H), 7.21 (d, 1H), 8.36 (s, 1H).

### Beispiel 274

### 3-(5-Chlor-1,2,3,4-tetrahydronaphthalen-1-yl)-1-(1,3-dimethyl-2-oxo-2,3-dihydro-1H-benzimidazol-5-yl)-2,4-dioxo-1,2,3,4-tetrahydropyrimidin-5-carbonsäure (Enantiomer 1)

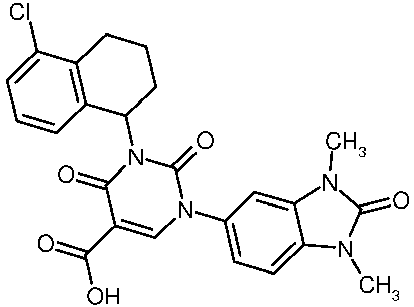

47 mg (0.10 mmol) Methyl-3-(5-chlor-1,2,3,4-tetrahydronaphthalen-1-yl)-1-(1,3-dimethyl-2-oxo-2,3-dihydro-1H-benzimidazol-5-yl)-2,4-dioxo-1,2,3,4-tetrahydropyrimidin-5-carboxylat (*Enantiomer 1)* aus Beispiel 272 wurden in 2 ml Eisessig / konz. Salzsäure (2:1 v/v) 2h bei Rückflusstemperatur gerührt. Das Reaktionsgemisch wurde am Rotationsverdampfer eingeengt und der Rückstand in Acetonitril/Wasser gelöst und lyophilisiert. Man erhielt 38 mg (76 % d. Th.) der Titelverbindung.
LC/MS (Methode 1): Rₜ= 1.08 min; m/z = 481 (M+H)⁺

### Beispiel 275

### 3-(5-Chlor-1,2,3,4-tetrahydronaphthalen-1-yl)-1-(1,3-dimethyl-2-oxo-2,3-dihydro-1H-benzimidazol-5-yl)-2,4-dioxo-1,2,3,4-tetrahydropyrimidin-5-carbonsäure (Enantiomer 2)

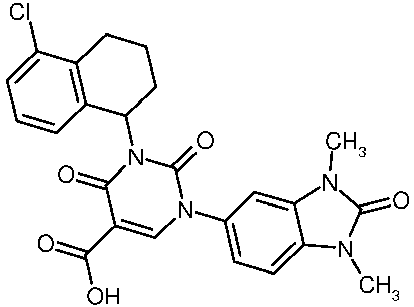

66 mg (0.13 mmol) Methyl-3-(5-chlor-1,2,3,4-tetrahydronaphthalen-1-yl)-1-(1,3-dimethyl-2-oxo-2,3-dihydro-1H-benzimidazol-5-yl)-2,4-dioxo-1,2,3,4-tetrahydropyrimidin-5-carboxylat *(Enantiomer 2)* aus Beispiel 273 wurden in 2 ml Eisessig / konz. Salzsäure (2:1 v/v) 2h bei Rückflusstemperatur gerührt. Das Reaktionsgemisch wurde am Rotationsverdampfer eingeengt und der Rückstand in Acetonitril/Wasser gelöst und lyophilisiert. Man erhielt 62 mg (87 % d. Th.) der Titelverbindung.
LC/MS (Methode 1): Rₜ= 1.08 min; m/z = 481 (M+H)⁺

### Beispiel 276

### Ethyl-1-(1,3-dimethyl-2-oxo-2,3-dihydro-1H-benzimidazol-5-yl)-2,4-dioxo-3-[5-(trifluormethyl)-1,2,3,4-tetrahydronaphthalen-1-yl]-1,2,3,4-tetrahydropyrimidin-5-carboxylat (Racemat)

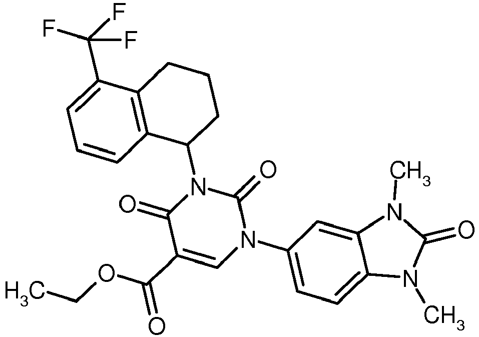

225 mg (0.66 mmol) Ethyl-1-(1,3-dimethyl-2-oxo-2,3-dihydro-1H-benzimidazol-5-yl)-2,4-dioxo-1,2,3,4-tetrahydropyrimidin-5-carboxylat (Beispiel 2A), 170 mg (0.79 mmol) 5-(Trifluormethyl)-1,2,3,4-tetrahydronaphthalen-1-ol und 292 mg (1.11 mmol) Triphenylphosphin in 3 ml THF und 6 ml DMF wurden bei RT mit 212 mg (1.05 mmol) DIAD versetzt. Es wurde 2h bei RT gerührt, dann mit wenig IM wässriger Salzsäure versetzt, mit DMSO verdünnt und mittels präparativer HPLC (Methode 15) gereinigt. Man erhielt 136 mg (38 % d.Th.) der Titelverbindung.
LC/MS (Methode 1): Rₜ= 1.14 min; m/z = 543 (M+H)⁺
¹H-NMR (400MHz, CD₂Cl₂): δ [ppm] = 1.23 (t, 3H), 1.65 - 1.80 (m, 1H), 1.99 - 2.11 (m, 2H), 2.25 - 2.41 (m, 1H), 2.74 - 2.92 (m, 1H), 2.99 (d, 1H), 3.30 (s, 3H), 3.32 (s, 3H), 4.21 (q, 2H), 6.20 (br. s., 1H), 6.78 - 7.00 (m, 3H), 7.09 - 7.18 (m, 2H), 7.40 (t, 1H), 8.25 (s, 1H).

### Beispiel 277

### 1-(1,3-Dimethyl-2-oxo-2,3-dihydro-1H-benzimidazol-5-yl)-2,4-dioxo-3-[5-(trifluormethyl)-1,2,3,4-tetrahydronaphthalen-1-yl]-1,2,3,4-tetrahydropyrimidin-5-carbonsäure (Racemat)

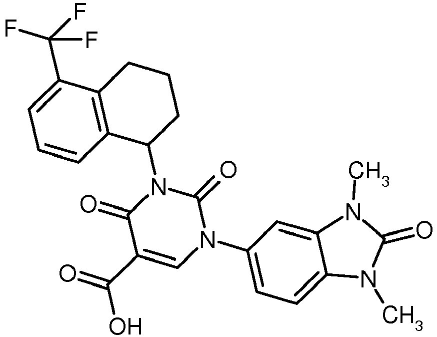

120 mg (0.22 mmol) Ethyl-1-(1,3-dimethyl-2-oxo-2,3-dihydro-1H-benzimidazol-5-yl)-2,4-dioxo-3-[5-(trifluormethyl)-1,2,3,4-tetrahydronaphthalen-1-yl]-1,2,3,4-tetrahydropyrimidin-5-carboxylat aus Beispiel 276 wurden mit 2 ml konz. wässriger Salzsäure und 4 ml Eisessig 2 h bei Rückflusstemperatur gerührt. Die Reaktionsmischung wurde mit 5 ml Acetonitril verdünnt und mittels präparativer HPLC (Methode 15) gereinigt. Man erhielt 54 mg (47 % d.Th.) der Titelverbindung.
LC/MS (Methode 1): Rₜ = 1.12 min; m/z = 515 (M+H)⁺
¹H-NMR (400MHz, CD₂Cl₂): δ [ppm] = 1.74 - 1.91 (m, 1H), 2.12 - 2.24 (m, 2H), 2.35 - 2.50 (m, 1H), 2.85 - 2.99 (m, 1H), 3.04 - 3.15 (m, 1H), 3.39 (s, 3H), 3.41 (s, 3H), 6.32 (br. s., 1H), 6.86 - 6.97 (m, 1H), 6.98 - 7.11 (m, 2H), 7.16 - 7.29 (m, 2H), 7.52 (d, 1H), 8.59 (s, 1H), 12.47 (br. s, 1H).

### Beispiel 278

### Methyl-1-(1,3-dimethyl-2-oxo-2,3-dihydro-1H-benzimidazol-5-yl)-2,4-dioxo-3-[5-(trifluormethyl)-1,2,3,4-tetrahydronaphthalen-1-yl]-1,2,3,4-tetrahydropyrimidin-5-carboxylat (Racemat)

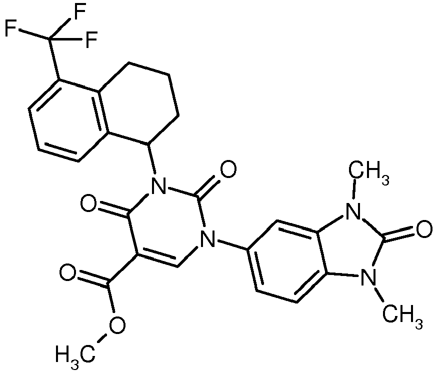

45 mg (0.09 mmol) 1-(1,3-Dimethyl-2-oxo-2,3-dihydro-1H-benzimidazol-5-yl)-2,4-dioxo-3-[5-(trifluor-methyl)-1,2,3,4-tetrahydronaphthalen-1-yl]-1,2,3,4-tetrahydropyrimidin-5-carbonsäure aus Beispiel 277 wurden in 5 ml Methanol gelöst, mit 100 µl (1.31 mmol) Thionylchlorid versetzt und 5 h bei Rückflusstemperatur gerührt. Die Reaktionslösung wurde im Vakuum eingeengt und der Rückstand im Hochvakuum getrocknet. Man erhielt 46 mg (92 % d. Th.) der Titelverbindung.
LC/MS (Methode 28): Rₜ=3.25 min; m/z = 529 (M+H)⁺
¹H-NMR (400MHz, CD₂Cl₂): δ [ppm]= 1.70 - 1.90 (m, 1H), 2.07 - 2.19 (m, 2H), 2.31 - 2.49 (m, 1H), 2.83 - 3.00 (m, 1H), 3.07 (d, 1H), 3.40 (s, 3H), 3.42 (s, 3H), 3.82 (s, 3H), 6.27 (br. s., 1H), 6.91 - 6.99 (m, 1H), 7.00 - 7.11 (m, 2H), 7.21 (d, 2H), 7.48 (t, 1H), 8.37 (s, 1H).

### Beispiel 279

### Methyl-1-(1,3-dimethyl-2-oxo-2,3-dihydro-1H-benzimidazol-5-yl)-2,4-dioxo-3-[5-(trifluormethyl)-1,2,3,4-tetrahydronaphthalen-1-yl]-1,2,3,4-tetrahydropyrimidin-5-carboxylat (Enantiomer 1)

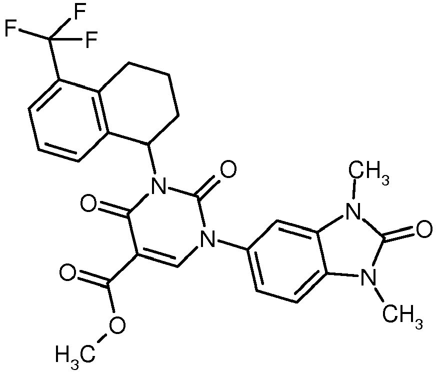

Zuerst eluierendes Enantiomer (17 mg) aus der Trennung von 45 mg der racemischen Substanz aus Beispiel 278 mittels präparativen HPLC an einer chiralen Phase (Methode 31).
Chirale analytische HPLC (Methode 32): Rₜ = 4.14 min, >99 % ee.
LC/MS (Methode 1): Rₜ= 1.06 min; m/z = 529 (M+H)⁺

### Beispiel 280

### Methyl-1-(1,3-dimethyl-2-oxo-2,3-dihydro-1H-benzimidazol-5-yl)-2,4-dioxo-3-[5-(trifluormethyl)-1,2,3,4-tetrahydronaphthalen-1-yl]-1,2,3,4-tetrahydropyrimidin-5-carboxylat (Enantiomer 2)

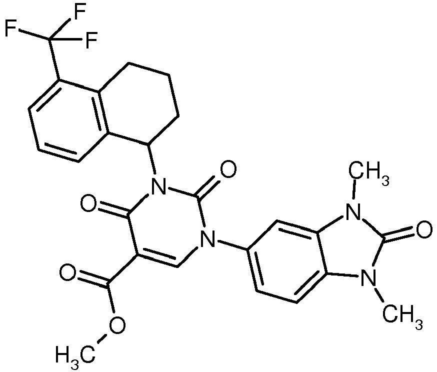

Zuletzt eluierendes Enantiomer (19 mg) aus der Trennung von 45 mg der racemischen Substanz aus Beispiel 278 mittels präparativen HPLC an einer chiralen Phase (Methode 31).
Chirale analytische HPLC (Methode 32): Rₜ = 4.68 min, 98 % ee.
LC/MS (Methode 1): Rₜ = 1.06 min; m/z = 529 (M+H)+

### Beispiel 281

### 1-(1,3-Dimethyl-2-oxo-2,3-dihydro-1H-benzimidazol-5-yl)-2,4-dioxo-3-[5-(trifluormethyl)-1,2,3,4-tetrahydronaphthalen-1-yl]-1,2,3,4-tetrahydropyrimidin-5-carbonsäure (Enantiomer 1)

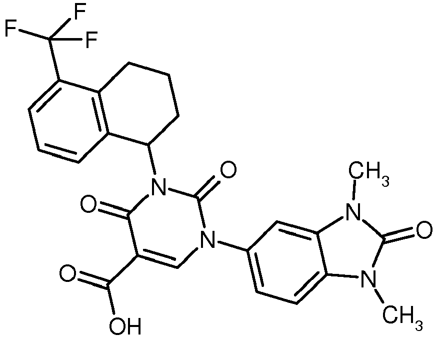

14 mg (0.03 mmol) der Verbindung aus Beispiel 279 wurden in 1.75 ml Eisessig / konz. Salzsäure 2:1 (v/v) 2 h bei Rückflusstemperatur gerührt. Das Reaktionsgemisch wurde im Vakuum eingeengt, der Rückstand in Acetonitril und Wasser gelöst und lyophilisiert. Man erhielt 7 mg (48 % d.Th.) der Titelverbindung.
LC/MS (Methode 1): Rₜ: 1.10 min; m/z = 515 (M+H)⁺

### Beispiel 282

### 1-(1,3-Dimethyl-2-oxo-2,3-dihydro-1H-benzimidazol-5-yl)-2,4-dioxo-3-[5-(trifluormethyl)-1,2,3,4-tetrahydronaphthalen-1-yl]-1,2,3,4-tetrahydropyrimidin-5-carbonsäure (Enantiomer 2)

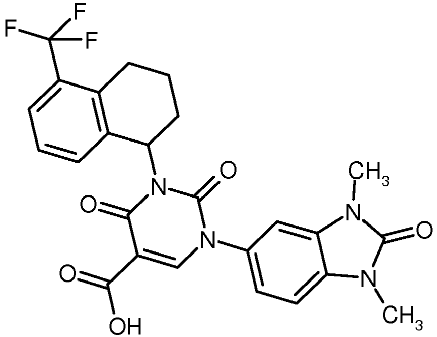

16 mg (0.03 mmol) der Verbindung aus Beispiel 280 wurden in 2 ml eines Gemisches aus Eisessig / konz. Salzsäure 2:1 (v/v) 2 h bei Rückflusstemperatur gerührt. Das Reaktionsgemisch wurde im Vakuum eingeengt, der Rückstand in Acetonitril und Wasser gelöst und lyophilisiert. Man erhielt 13 mg (76 % d.Th.) der Titelverbindung.
LC/MS (Methode 1): Rₜ= 1.10 min; m/z = 515 (M+H)⁺

### Beispiel 283

### Ethyl-3-(5-chlor-1,2,3,4-tetrahydronaphthalen-1-yl)-1-(3-methyl-2-oxo-2,3-dihydro-1,3-benzoxazol-6-yl)-2,4-dioxo-1,2,3,4-tetrahydropyrimidin-5-carboxylat (Racemat)

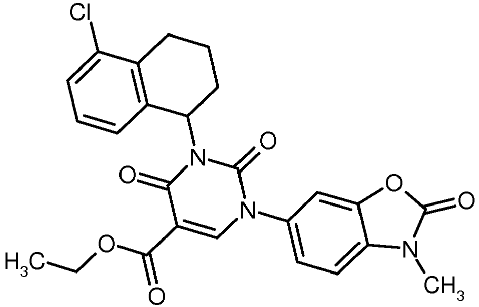

400 mg (1.21 mmol) Ethyl-1-(3-methyl-2-oxo-2,3-dihydro-1,3-benzoxazol-6-yl)-2,4-dioxo-1,2,3,4-tetrahydropyrimidin-5-carboxylat aus Beispiel 28A, 265 mg (1.45 mmol) 5-Chlor-1,2,3,4-tetrahydronaphthalen-1-ol und 538 mg (2.05 mmol) Triphenylphosphin wurden in 5 ml THF und 10 ml DMF bei RT vorgelegt. 391 mg (1.93 mmol) DIAD wurden zugegeben und das Reaktionsgemisch 2 h bei RT gerührt. Nach Zugabe von wenig IM wässriger Salzsäure wurde das Gemisch in DMSO gelöst und mittels präparativer HPLC (Methode 11) gereinigt. Man erhielt 300 mg (47 % d.Th.) der Titelverbindung.
LC/MS (Methode 1): Rₜ= 1.13 min; m/z = 496 (M+H)⁺
¹H-NMR (400MHz, CD₂Cl₂): δ [ppm]= 1.24 (t, 3H), 1.64 - 1.81 (m, 1H), 1.93 - 2.11 (m, 2H), 2.21 - 2.42 (m, 1H), 2.51 - 2.71 (m, 1H), 2.94 (d, 1H), 3.32 (s, 3H), 4.21 (q, 2H), 6.14 (br. s., 1H), 6.83 (d, 1H), 6.97 (t, 2H), 7.13 (d, 3H), 8.22 (s, 1H).

### Beispiel 284

### 3-(5-Chlor-1,2,3,4-tetrahydronaphthalen-1-yl)-1-(3-methyl-2-oxo-2,3-dihydro-1,3-benzoxazol-6-yl)-2,4-dioxo-1,2,3,4-tetrahydropyrimidin-5-carbonsäure (Racemat)

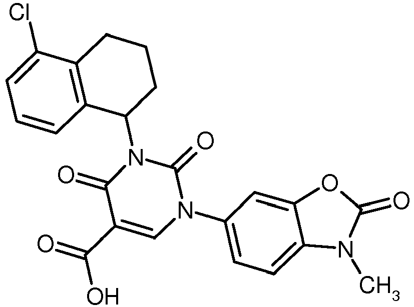

270 mg (0.54 mmol) Ethyl-3-(5-chlor-1,2,3,4-tetrahydronaphthalen-1-yl)-1-(3-methyl-2-oxo-2,3-dihydro-1,3-benzoxazol-6-yl)-2,4-dioxo-1,2,3,4-tetrahydropyrimidin-5-carboxylat aus Beispiel 283 wurden in 2 ml konz. Salzsäure und 4 ml Eisessig bei Rückflusstemperatur gerührt. Nach Abkühlung wurde das Gemisch mittels präparativer HPLC (Methode 15) gereinigt. Man erhielt 200 mg (79 % d.Th) der Titelverbindung.
LC/MS (Methode 1): Rₜ =1.14 min; m/z = 468 (M+H)⁺
¹H-NMR (400MHz, CD₂Cl₂): δ [ppm] = 1.73 - 1.90 (m, 1H), 2.03 - 2.23 (m, 2H), 2.31 - 2.51 (m, 1H), 2.63 - 2.80 (m, 1H), 3.05 (d, 1H), 3.41 (s, 3H), 6.27 (br. s., 1H), 6.89 (d, 1H), 7.08 (t, 2H), 7.14 - 7.29 (m, 3H), 8.56 (s, 1H), 12.40 (br. s., 1H).

### Beispiel 285

### Methyl-3-(5-chlor-1,2,3,4-tetrahydronaphthalen-1-yl)-1-(3-methyl-2-oxo-2,3-dihydro-1,3-benzoxazol-6-yl)-2,4-dioxo-1,2,3,4-tetrahydropyrimidin-5-carboxylat (Racemat)

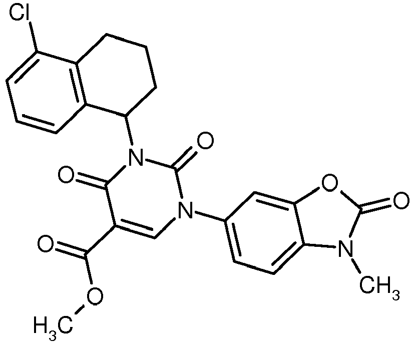

50 mg (0.11 mmol) der Verbindung aus Beispiel 284 wurden in 5 ml Methanol gelöst und mit 117 µl (1.60 mmol) Thionylchlorid versetzt. Das Gemisch wurde 5 h bei Rückflusstemperatur gerührt, dann am Rotationsverdampfer eingeengt und der Rückstand im Hochvakuum getrocknet. Man erhielt 51 mg (90 % d. Th.) der Titelverbindung.
LC/MS (Methode 4): Rₜ = 2.42 min; m/z = 482 (M+H)⁺
¹H-NMR (400MHz, CD₂Cl₂): δ [ppm] = 1.80 (q, 1H), 2.01 - 2.21 (m, 2H), 2.30 - 2.50 (m, 1H), 2.63 - 2.79 (m, 1H), 3.03 (d, 1H), 3.41 (s, 3H), 3.83 (s, 3H), 6.06 - 6.41 (m, 1H), 6.91 (d, 1H), 7.03 - 7.11 (m, 2H), 7.14 - 7.26 (m, 3H), 8.33 (s, 1H).

### Beispiel 286

### Methyl-3-(5-chlor-1,2,3,4-tetrahydronaphthalen-1-yl)-1-(3-methyl-2-oxo-2,3-dihydro-1,3-benzoxazol-6-yl)-2,4-dioxo-1,2,3,4-tetrahydropyrimidin-5-carboxylat (Enantiomer 1)

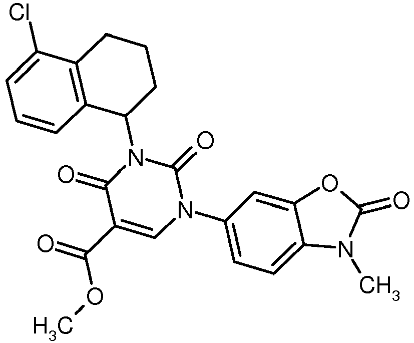

Zuerst eluierendes Enantiomer aus der Trennung von 152 mg racemischen Methyl-3-(5-chlor-1,2,3,4-tetrahydronaphthalen-1-yl)-1-(3-methyl-2-oxo-2,3-dihydro-1,3-benzoxazol-6-yl)-2,4-dioxo-1,2,3,4-tetrahydropyrimidin-5-carboxylat (Beispiel 285) mittels präparativer HPLC an einer chiralen Phase (Methode 29). Chirale analytische HPLC (Methode 30): Rₜ = 4.44 min, >99 % ee.

Um Lösungsmittelsverunreinigungen zu entfernen wurde das erhaltene Produkt noch mittels präparativer HPLC (Methode 15) gereinigt. Man erhielt 34 mg der Titelverbindung.
LC/MS (Methode 1): Rₜ = 1.08 min; m/z = 482 (M+H)⁺

### Beispiel 287

### Methyl-3-(5-chlor-1,2,3,4-tetrahydronaphthalen-1-yl)-1-(3-methyl-2-oxo-2,3-dihydro-1,3-benzoxazol-6-yl)-2,4-dioxo-1,2,3,4-tetrahydropyrimidin-5-carboxylat (Enantiomer 2)

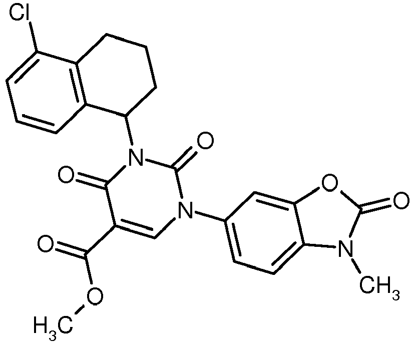

Zuletzt eluierendes Enantiomer aus der Trennung von 152 mg racemischen Methyl-3-(5-chlor-1,2,3,4-tetrahydronaphthalen-1-yl)-1-(3-methyl-2-oxo-2,3-dihydro-1,3-benzoxazol-6-yl)-2,4-dioxo-1,2,3,4-tetrahydropyrimidin-5-carboxylat (Beispiel 285) mittels präparativer HPLC an einer chiralen Phase (Methode 29). Chirale analytische HPLC (Methode 30): Rt = 5.87 min, 99 % ee.

Um Lösungsmittelsverunreinigungen zu entfernen wurde das erhaltene Produkt noch mittels präparativer HPLC (Methode 15) gereinigt. Man erhielt 27 mg der Titelverbindung.
LC/MS (Methode 1): Rₜ = 1.08 min; m/z = 482 (M+H)⁺

### Beispiel 288

### 3-(5-Chlor-1,2,3,4-tetrahydronaphthalen-1-yl)-1-(3-methyl-2-oxo-2,3-dihydro-1,3-benzoxazol-6-yl)-2,4-dioxo-1,2,3,4-tetrahydropyrimidin-5-carbonsäure (Enantiomer 1)

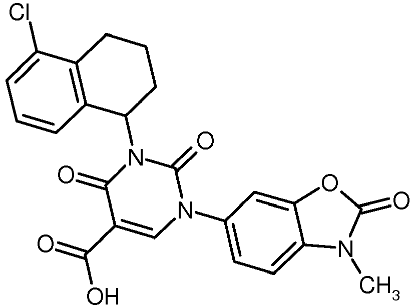

32 mg (66 µmol) der Verbindung aus Beispiel 286 wurden in 2 ml Eisessig / konz. Salzsäure 2:1 (v/v) 2 h bei Rückflusstemperatur gerührt. Das Gemisch wurde einrotiert, in Acetonitril und Wasser gelöst und lyophilisiert. Man erhielt 33 mg (92 % rein, 97 % d.Th.) der Titelverbindung.
LC/MS (Methode 1): Rₜ = 1.12 min; m/z = 468 (M+H)⁺

### Beispiel 289

### 3-(5-Chlor-1,2,3,4-tetrahydronaphthalen-1-yl)-1-(3-methyl-2-oxo-2,3-dihydro-1,3-benzoxazol-6-yl)-2,4-dioxo-1,2,3,4-tetrahydropyrimidin-5-carbonsäure (Enantiomer 2)

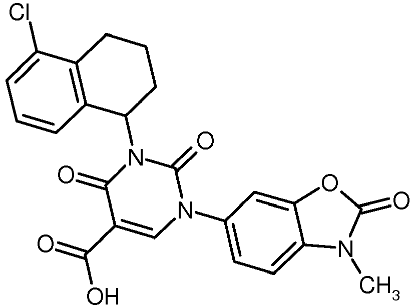

25 mg (0.052 mmol) der Verbindung aus Beispiel 287 wurden in 1.6 ml Eisessig / konz. Salzsäure 2:1 (v/v) 2 h bei Rückflusstemperatur gerührt. Das Gemisch wurde im Vakuum eingeengt, der Rückstand in Acetonitril und Wasser gelöst und lyophilisiert. Man erhielt 24 mg (93 % d.Th.) der Titelverbindung.
LC/MS (Methode 1): Rₜ= 1.12 min; m/z = 468 (M+H)⁺

### Beispiel 290

### Ethyl-1-(3-methyl-2-oxo-2,3-dihydro-1,3-benzoxazol-6-yl)-2,4-dioxo-3-[5-(trifluormethyl)-1,2,3,4-tetrahydronaphthalen-1-yl]-1,2,3,4-tetrahydropyrimidin-5-carboxylat (Racemat)

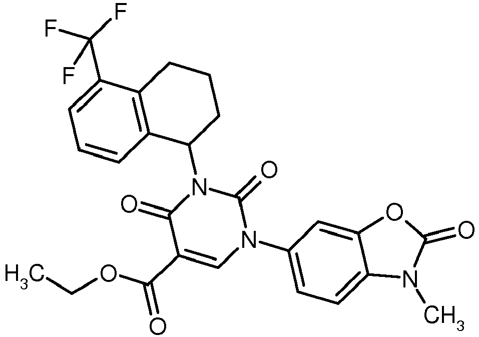

217 mg (0.66 mmol) Ethyl-1-(3-methyl-2-oxo-2,3-dihydro-1,3-benzoxazol-6-yl)-2,4-dioxo-1,2,3,4-tetrahydropyrimidin-5-carboxylat (Beispiel 28A), 170 mg (0.79 mmol) 5-(Trifluormethyl)-1,2,3,4-tetrahydronaphthalen-1-ol und 292 mg (1.11 mmol) Triphenylphosphin wurden in 3 ml THF und 6 ml DMF bei RT vorgelegt. 212 mg (1.05 mmol) DIAD wurden zugegeben und das Gemisch 2 h bei RT gerührt. Nach Zugabe von wenig IM wässriger Salzsäure wurde das Gemisch in DMSO gelöst und mittels präparativer HPLC (Methode 15) gereinigt. Man erhielt 114 mg (33 % d.Th.) der Titelverbindung.
LC/MS (Methode 1): Rₜ= 1.18 min; m/z = 530 (M+H)⁺
¹H-NMR (400MHz, CD₂Cl₂): δ [ppm]= 1.24 (t, 3H), 1.65 - 1.80 (m, 1H), 1.98 - 2.11 (m, 2H), 2.21- 2.39 (d, 1H), 2.73 - 2.91 (d, 1H), 2.93 - 3.04 (m, 1H), 3.32 (s, 3H), 4.22 (q, 2H), 6.19 (br. s., 1H), 6.97 (d, 1H), 7.03 - 7.20 (m, 4H), 7.37 - 7.44 (m, 1H), 8.22 (s, 1H).

### Beispiel 291

### 1-(3-Methyl-2-oxo-2,3-dihydro-1,3-benzoxazol-6-yl)-2,4-dioxo-3-[5-(trifluormethyl)-1,2,3,4-tetrahydronaphthalen-1-yl]-1,2,3,4-tetrahydropyrimidin-5-carbonsäure (Racemat)

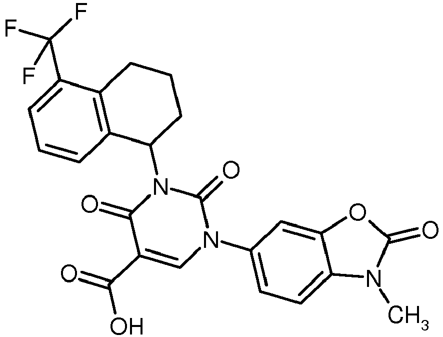

95 mg (0.18 mmol) der Verbindung aus Beispiel 290 wurden mit 2 ml konz. Salzsäure und 4 ml Eisessig 2 h bei Rückflusstemperatur gerührt. Nach Abkühlung wurde das Gemisch mit 5 ml Acetonitril verdünnt und mittels präparativer HPLC (Methode 15) gereinigt. Man erhielt 83 mg (92 % d.Th.) der Titelverbindung.
LC/MS (Methode 1): Rₜ= 1.15 min; m/z = 502 (M+H)⁺
¹H-NMR (400MHz, CD₂Cl₂): δ [ppm]= 1.65 - 1.81 (m, 1H), 2.01 - 2.17 (m, 2H), 2.25 - 2.39 (m, 1H), 2.76 - 2.92 (m, 1H), 2.96 - 3.10 (m, 1H), 3.33 (s, 3H), 6.23 (br. s., 1H), 6.99 (d, 1H), 7.04 - 7.22 (m, 4H), 7.44 (d, 1H), 8.48 (s, 1H), 12.30 (br. s, 1H).

### Beispiel 292

### Methyl-1-(3-methyl-2-oxo-2,3-dihydro-1,3-benzoxazol-6-yl)-2,4-dioxo-3-[5-(trifluormethyl)-1,2,3,4-tetrahydronaphthalen-1-yl]-1,2,3,4-tetrahydropyrimidin-5-carboxylat (Racemat)

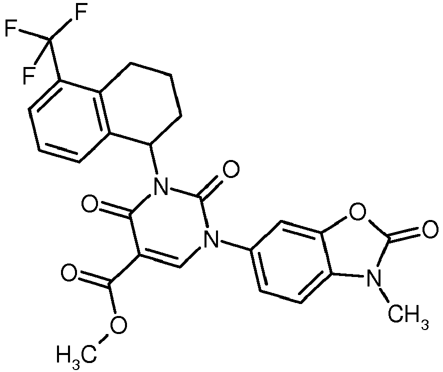

60 mg (0.12 mmol) 1-(3-Methyl-2-oxo-2,3-dihydro-1,3-benzoxazol-6-yl)-2,4-dioxo-3-[5-(trifluormethyl)-1,2,3,4-tetrahydronaphthalen-1-yl]-1,2,3,4-tetrahydropyrimidin-5-carbonsäure aus Beispiel 291 wurden in 5 ml Methanol gelöst und mit 131 µl (1.80 mmol) Thionylchlorid versetzt. Das Gemisch wurde 7 h bei Rückflusstemperatur gerührt, dann am Rotationsverdampfer eingeengt und der Rückstand im Hochvakuum getrocknet. Man erhielt 60 mg (77 % d. Th.) der Titelverbindung in 79 % Reinheit.
LC/MS (Methode 28): Rₜ= 3.40 min; m/z = 516 (M+H)⁺

### Beispiel 293

### Methyl-1-(3-methyl-2-oxo-2,3-dihydro-1,3-benzoxazol-6-yl)-2,4-dioxo-3-[5-(trifluormethyl)-1,2,3,4-tetrahydronaphthalen-1-yl]-1,2,3,4-tetrahydropyrimidin-5-carboxylat (Enantiomer 1)

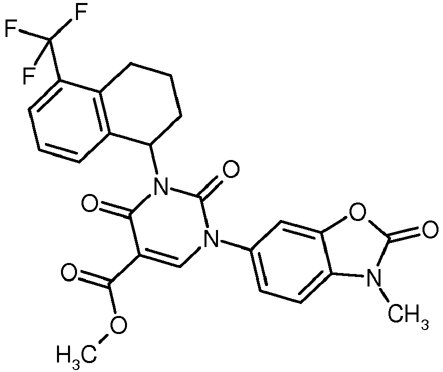

60 mg (0.12 mmol) racemisches Methyl-1-(3-methyl-2-oxo-2,3-dihydro-1,3-benzoxazol-6-yl)-2,4-dioxo-3-[5-(trifluormethyl)-1,2,3,4-tetrahydronaphthalen-1-yl]-1,2,3,4-tetrahydropyrimidin-5-carboxylat aus Beispiel 292 wurden in 3 ml Acetonitril und 1 ml Ethanol gelöst und auf einer Daicel Chiralpak IC-Säule mit 40 % Acetonitril und 60 % MTBE getrennt. Teilweise fand eine Umesterung zum Ethylester statt. Man erhielt als erste eluierende Fraktion 14.5 mg der Titelverbindung.
Chirale analytische HPLC (Methode 30): Rt = 3.99 min, 99 % ee.
LC/MS (Methode 1): Rₜ= 1.11 min; m/z = 516 (M+H)⁺
¹H-NMR (400MHz, CD₂Cl₂): δ [ppm]= 1.64 - 1.80 (m, 1H), 1.99 - 2.10 (m, 2H), 2.23 - 2.36 (m, 1H), 2.75 - 2.90 (m, 1H), 2.94 - 3.04 (m, 1H), 3.32 (s, 3H), 3.75 (s, 3H), 6.18 (br. s., 1H), 6.97 (d, 1H), 7.03 - 7.22 (m, 4H), 7.37 - 7.45 (m, 1H), 8.25 (s, 1H).

Als zweite eluierende Fraktion wurde eine Mischung aus dem Epimer der Titelverbindung und den beiden Enantiomeren des entsprechenden Ethylesters (43 mg) erhalten. Diese Mischung wurde nicht weiter gereinigt.

### Beispiel 294

### 1-(3-Methyl-2-oxo-2,3-dihydro-1,3-benzoxazol-6-yl)-2,4-dioxo-3-[5-(trifluormethyl)-1,2,3,4-tetrahydronaphthalen-1-yl]-1,2,3,4-tetrahydropyrimidin-5-carbonsäure (Enantiomer 1)

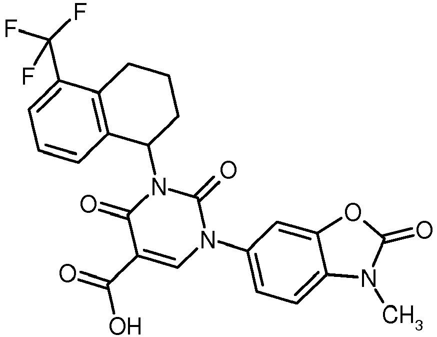

12 mg (0.02 mmol) Methyl-1-(3-methyl-2-oxo-2,3-dihydro-1,3-benzoxazol-6-yl)-2,4-dioxo-3-[5-(trifluormethyl)-1,2,3,4-tetrahydronaphthalen-1-yl]-1,2,3,4-tetrahydropyrimidin-5-carboxylat aus Beispiel 293 wurden mit 0.4 ml eines Gemisches aus Eisessig und konz. Salzsäure im Verhältnis 2:1 2 h bei Rückflusstemperatur gerührt. Das Gemisch wurde eingeengt, der Rückstand in Acetonitril/Wasser gelöst und anschließend lyophilisiert. Man erhielt 11 mg (91 % d.Th.) der Titelverbindung.
LC/MS (Methode 1): Rₜ= 1.12 min; m/z = 502 (M+H)⁺

### Beispiel 295

### Ethyl-1-(3-methyl-2-oxo-2,3-dihydro-1,3-benzothiazol-6-yl)-2,4-dioxo-3-[5-(trifluormethyl)-1,2,3,4-tetrahydronaphthalen-1-yl]-1,2,3,4-tetrahydropyrimidin-5-carboxylat (Racemat)

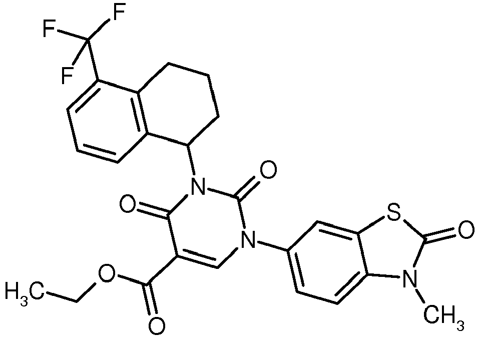

227 mg (0.66 mmol) Ethyl-1-(3-methyl-2-oxo-2,3-dihydro-1,3-benzothiazol-6-yl)-2,4-dioxo-1,2,3,4-tetrahydropyrimidin-5-carboxylat (Beispiel 31A), 170 mg (0.79 mmol) 5-(Trifluormethyl)-1,2,3,4-tetrahydronaphthalen-1-ol und 292.2 mg (1.11 mmol) Triphenylphosphin wurden in 3 ml THF und 6 ml DMF bei RT vorgelegt. 206 µl (1.05 mmol) DIAD wurden zugegeben und die Reaktionsmischung 2 h bei RT gerührt. Nach Zugabe von wenig IM wässriger Salzsäure wurde das Gemisch in DMSO gelöst und mittels präparativer HPLC (Methode 15) getrennt. Man erhielt 196 mg (52 % d.Th.) der Titelverbindung.
LC/MS (Methode 1): Rₜ = 1.23 min; m/z = 546 (M+H)⁺
¹H-NMR (400MHz, CD₂Cl₂): δ [ppm] = 1.24 (t, 3H), 1.65 - 1.80 (m, 1H), 1.99 - 2.11 (m, 2H), 2.23 - 2.38 (m, 1H), 2.75 - 2.90 (m, 1H), 2.94 - 3.05 (m, 1H), 3.37 (s, 3H), 4.22 (q, 2H), 6.20 (br. s., 1H), 7.05 (d, 1H), 7.12 (d, 2H), 7.15 - 7.28 (m, 1H), 7.29 - 7.46 (m, 2H), 8.23 (s, 1H).

### Beispiel 296

### 1-(3-Methyl-2-oxo-2,3-dihydro-1,3-benzothiazol-6-yl)-2,4-dioxo-3-[5-(trifluormethyl)-1,2,3,4-tetrahydronaphthalen-1-yl]-1,2,3,4-tetrahydropyrimidin-5-carbonsäure (Racemat)

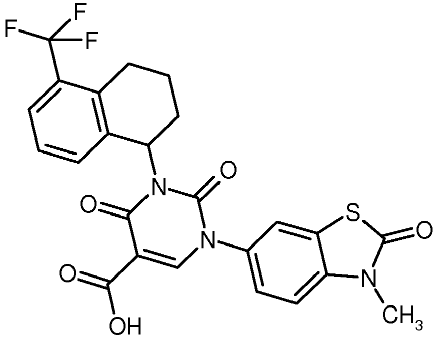

120 mg (0.21 mmol) der Verbindung aus Beispiel 295 wurden in 2 ml Eisessig und 4 ml konz. Salzsäure 2 h bei Rückflusstemperatur gerührt. Das Reaktionsgemisch wurde abgekühlt, mit 5 ml Acetonitril verdünnt und mittels präparativer HPLC (Methode 15) gereinigt. Man erhielt 85 mg (78 % d. Th.) der Titelverbindung.
LC/MS (Methode 1): Rₜ=1.18 min; m/z = 518 (M+H)⁺
¹H-NMR (400MHz, CD₂Cl₂): δ [ppm]= 1.74 - 1.90 (m, 1H), 2.10 - 2.25 (m, 2H), 2.40 (q, 1H), 2.83 - 2.99 (m, 1H), 3.05 - 3.15 (m, 1H), 3.46 (s, 3H), 6.32 (br. s., 1H), 7.11 - 7.20 (m, 2H), 7.21 - 7.35 (m, 2H), 7.44 (br. s., 1H), 7.52 (d, 1H), 8.57 (s, 1H), 12.38 (br. s, 1H).

### Beispiel 297

### Methyl-1-(3-methyl-2-oxo-2,3-dihydro-1,3-benzothiazol-6-yl)-2,4-dioxo-3-[5-(trifluormethyl)-1,2,3,4-tetrahydronaphthalen-1-yl]-1,2,3,4-tetrahydropyrimidin-5-carboxylat (Racemat)

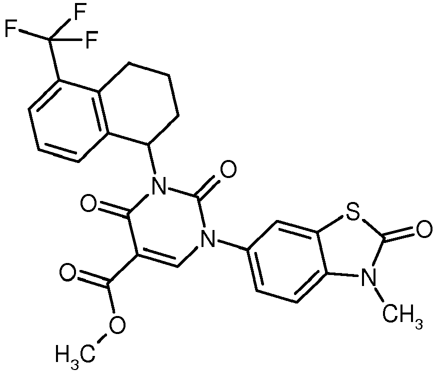

70 mg (0.14 mmol) der Verbindung aus Beispiel 296 wurden in 5 ml Methanol gelöst und mit 148 µl (2.03 mmol) Thionylchlorid versetzt. Das Gemisch wurde 7 h bei Rückflusstemperatur gerührt, dann am Rotationsverdampfer eingeengt und der Rückstand im Hochvakuum getrocknet. Man erhielt 70 mg der Titelverbindung in 75 % Reinheit (72 % d.Th.).
LC/MS (Methode 28): Rₜ=3.59 min; m/z = 532 (M+H)⁺

### Beispiel 298

### Methyl-1-(3-methyl-2-oxo-2,3-dihydro-1,3-benzothiazol-6-yl)-2,4-dioxo-3-[5-(trifluormethyl)-1,2,3,4-tetrahydronaphthalen-1-yl]-1,2,3,4-tetrahydropyrimidin-5-carboxylat (Enantiomer 1)

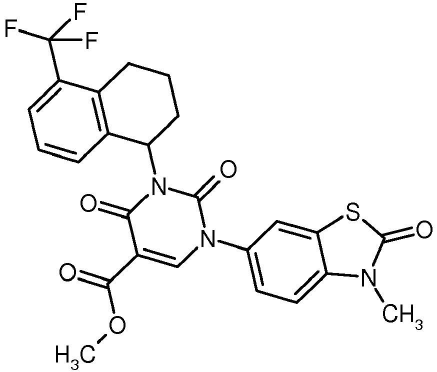

Zuerst eluierendes Enantiomer (24 mg) aus der Trennung von 70 mg der racemischen Substanz aus Beispiel 297 mittels präparativen HPLC an einer chiralen Phase (Methode 33).
Chirale analytische HPLC (Methode 34): Rt = 6.03 min, 99 % ee.
LC/MS (Methode 1): Rₜ= 1.16 min; m/z = 532 (M+H)⁺
¹H-NMR (400MHz, CD₂Cl₂): δ [ppm] = 1.72 - 1.88 (m, 1H), 2.05 - 2.20 (m, 2H), 2.30 - 2.47 (m, 1H), 2.83 - 2.98 (m, 1H), 3.01 - 3.14 (m, 1H), 3.45 (s, 3H), 3.83 (s, 3H), 6.18 - 6.37 (m, 1H), 7.05 - 7.37 (m, 4H), 7.48 (d, 2H), 8.34 (s, 1H).

### Beispiel 299

### Methyl-1-(3-methyl-2-oxo-2,3-dihydro-1,3-benzothiazol-6-yl)-2,4-dioxo-3-[5-(trifluormethyl)-1,2,3,4-tetrahydronaphthalen-1-yl]-1,2,3,4-tetrahydropyrimidin-5-carboxylat (Enantiomer 2)

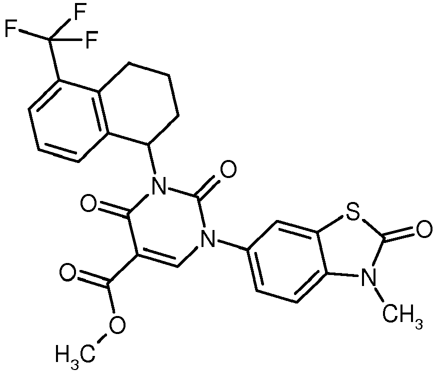

Zuletzt eluierendes Enantiomer (29 mg) aus der Trennung von 70 mg der racemischen Substanz aus Beispiel 297 mittels präparativen HPLC an einer chiralen Phase (Methode 33).
Chirale analytische HPLC (Methode 34): Rt = 7.37 min 99 % ee
LC/MS (Methode 1): Rₜ= 1.16 min; m/z = 532 (M+H)⁺
¹H-NMR (400MHz, CD₂Cl₂): δ [ppm] = 1.71 - 1.87 (m, 1H), 2.06 - 2.20 (m, 2H), 2.30 - 2.46 (m, 1H), 2.82 - 2.99 (m, 1H), 3.01 - 3.12 (m, 1H), 3.45 (s, 3H), 3.83 (s, 3H), 6.28 (br. s., 1H), 7.13 (d, 1H), 7.16 - 7.35 (m, 3H), 7.38 - 7.57 (m, 2H), 8.34 (s, 1H).

### Beispiel 300

### 1-(3-Methyl-2-oxo-2,3-dihydro-1,3-benzothiazol-6-yl)-2,4-dioxo-3-[5-(trifluormethyl)-1,2,3,4-tetrahydronaphthalen-1-yl]-1,2,3,4-tetrahydropyrimidin-5-carbonsäure (Enantiomer 1)

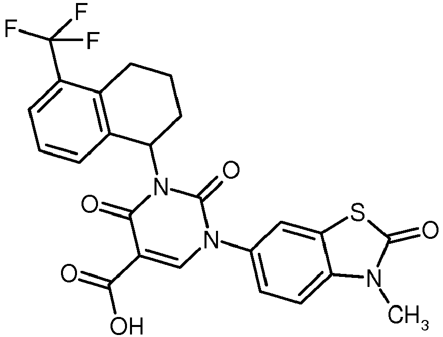

22 mg (0.04 mmol) der Verbindung aus Beispiel 298 wurden mit 2 ml Eisessig/ konz. Salzsäure im Verhältnis 2:1 (v/v) 2 h bei Rückflusstemperatur gerührt. Das Gemisch wurde am Rotationsverdampfer eingeengt, der Rückstand in Acetonitril/Wasser gelöst und anschließend lyophilisiert. Man erhielt 16 mg (75 % d.Th.) der Titelverbindung.
LC/MS (Methode 1): Rₜ= 1.16 min; m/z = 518 (M+H)⁺
¹H-NMR (400MHz, CD₂Cl₂): δ [ppm] = 1.74 - 1.90 (m, 1H), 2.11 - 2.24 (m, 2H), 2.31 - 2.46 (m, 1H), 2.84 - 3.00 (m, 1H), 3.04 - 3.15 (m, 1H), 3.46 (s, 3H), 6.32 (br. s., 1H), 7.10 - 7.21 (m, 2H), 7.21 - 7.37 (m, 4H), 7.38 -7.49 (m, 1H), 7.52 (d, 1H), 8.58 (s, 1H), 12.38 (br.s, 1H).

### Beispiel 301

### 1-(3-Methyl-2-oxo-2,3-dihydro-1,3-benzothiazol-6-yl)-2,4-dioxo-3-[5-(trifluormethyl)-1,2,3,4-tetrahydronaphthalen-1-yl]-1,2,3,4-tetrahydropyrimidin-5-carbonsäure (Enantiomer 2)

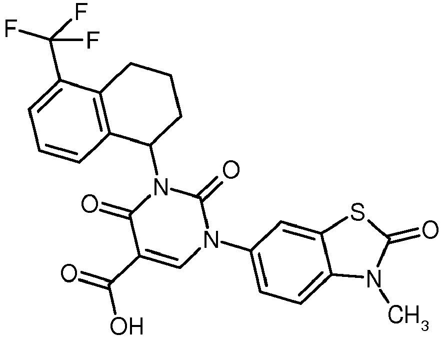

27 mg (0.05 mmol) der Verbindung aus Beispiel 299 wurden in 2.5 ml Eisessig/konz. Salzsäure 2 h bei Rückflusstemperatur gerührt. Das Gemisch wurde am Rotationsverdampfer eingeengt und der Rückstand in Acetonitril/Wasser gelöst und lyophilisiert. Man erhielt 22 mg (81 % d.Th.) der Titelverbindung.
LC/MS (Methode 1): Rₜ = 1.19 min; m/z = 518 (M+H)⁺

### Beispiel 302

### Ethyl-3-(8-chlor-3,4-dihydro-1H-isochromen-4-yl)-1-(3-methyl-2-oxo-2,3-dihydro-1,3-benzoxazol-6-yl)-2,4-dioxo-1,2,3,4-tetrahydropyrimidin-5-carboxylat (Racemat)

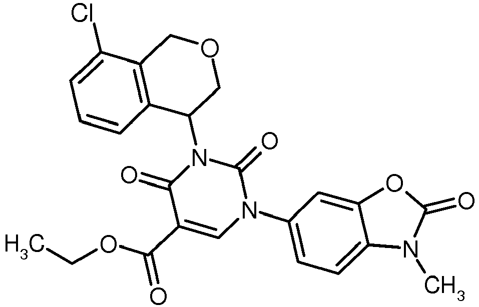

Unter Argon wurden 149.5 mg (0.45 mmol) Ethyl-1-(3-methyl-2-oxo-2,3-dihydro-1,3-benzoxazol-6-yl)-2,4-dioxo-1,2,3,4-tetrahydropyrimidin-5-carboxylat aus Beispiel 28A, 100.0 mg (0.54 mmol) 8-Chlor-3,4-dihydro-1H-isochromen-4-ol (Beispiel 120A) und 201.3 mg (0.77 mmol) Triphenylphosphin in 4.8 ml DMF und 2.4 ml THF gelöst. 146.0 mg (0.72 mmol) DIAD wurden hinzugetropft und die Reaktionsmischung wurde bei RT gerührt. Nach 2 h wurden 5 ml wässrige IM Salzsäure zugegeben und das Gemisch mittels präparativer HPLC (Methode 15) getrennt. Man erhielt 59.0 mg (26 % d.Th.) der Titelverbindung.
LC/MS (Methode 1): Rₜ= 1.02min; m/z = 498 (M+H)⁺
¹H-NMR (400MHz, CD₂Cl₂): δ [ppm] = 1.31 (t, 3H), 3.41 (s, 3H), 4.08 - 4.15 (m, 1H), 4.22 - 4.35 (m, 3H), 4.74 (d, 1H), 4.93 (d, 1H), 6.26 - 6.38 (m, 1H), 7.00 (d, 1H), 7.03 - 7.08 (m, 1H), 7.13 - 7.26 (m, 4H), 8.31 (s, 1H).

### Beispiel 303

### 3-(8-Chlor-3,4-dihydro-1H-isochromen-4-yl)-1-(3-methyl-2-oxo-2,3-dihydro-1,3-benzoxazol-6-yl)-2,4-dioxo-1,2,3,4-tetrahydropyrimidin-5-carbonsäure (Racemat)

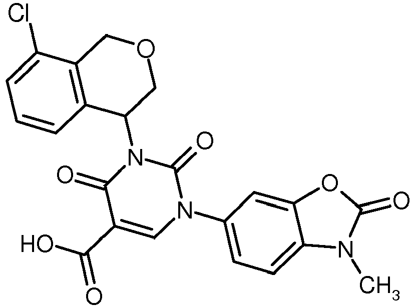

50 mg (0.10 mmol) der Verbindung aus Beispiel 302 wurden 50 min in 6.7 ml eines Gemisches aus konz. Salzsäure / Eisessig 1:2 auf Rückflusstemperatur erhitzt. Nach Abkühlung auf RT wurde das gesamte Gemisch mittels präparativer HPLC (Methode 15) getrennt. Man erhielt 10 mg (21 % d. Th.) der Titelverbindung.
LC/MS (Methode 1): Rₜ= 0.98 min; m/z = 470 (M+H)⁺
¹H-NMR (400MHz, CD₂Cl₂): δ [ppm]= 3.41 (s, 3H), 4.17 (dd, 1H), 4.22 - 4.30 (m, 1H), 4.76 (d, 1H), 4.94 (d, 1H), 6.28 - 6.40 (m, 1H), 6.99 (d, 1H), 7.08 (d, 1H), 7.19 (t, 2H), 7.28 (d, 1H), 8.57 (s, 1H).

### B. Bewertung der pharmakologischen Wirksamkeit

Die pharmakologische Wirkung der erfindungsgemäßen Verbindungen kann in den nachstehend beschriebenen Assays gezeigt werden:

**Abkürzungen:**

| | |
|---|---|
| Abz-HPFHL-Lys(Dnp)-NH₂ | 1-[N-(3-Aminobenzoyl)histidylprolylphenylalanylhistidylleucyl-N⁶-(2,4-dinitrophenyl)lysin |
| AMC | 7-Amido-4-methylcumarin |
| BNP | brain natriuretic peptide |
| BSA | bovine serum albumin |
| CHAPS | 3-[(3-Cholamidopropyl)dimethylammonio]-1-propansulfonat |
| HEPES | *N*-(2-Hydroxyethyl)piperazin-*N'*-2-ethansulfonsäure |
| IC | Inhibitionskonzentration |
| MeOSuc | Methoxysuccinyl |
| NADP | Nikotinamid-Adenin-Dinukleotid-Phosphat |
| PBS | Phosphat-gepufferte Kochsalz-Lösung |
| PEG | Polyethylenglykol |
| v/v | Volumen zu Volumen-Verhältnis (einer Lösung) |
| w/v | Gewicht zu Volumen-Verhältnis (einer Lösung) |

### B-1. Enzymatischer Chymase-Assay

Als Enzymquelle wird rekombinante humane Chymase (exprimiert in HEK293 Zellen) oder Chymase aufgereinigt aus Hamsterzungen benutzt. Als Substrat für Chymase wird Abz-HPFHL-Lys(Dnp)-NH₂ benutzt. Für den Assay werden 1 µl einer 50-fach konzentrierten Lösung von Testsubstanz in DMSO, 24 µl Enzymlösung (Verdünnung 1:80.000 human oder 1:4.000 Hamster) und 25 µl Substratlösung (finale Konzentration 10 µM) in Assaypuffer (Tris 50 mM (pH 7.5), Natriumchlorid 150 mM, BSA 0.10 %, Chaps 0.10 %, Glutathion 1 mM, EDTA 1 mM) in einer weißen 384-Loch Mikrotiterplatte (Greiner Bio-One, Frickenhausen, Deutschland) zusammengegeben. Die Reaktion wird 60 min bei 32 Grad inkubiert und die Fluoreszenz-Emission bei 465 nm nach Anregung mit 340 nm wird in einem Fluoreszenz-Reader z.B. Tecan Ultra (Tecan, Männedorf, Schweiz) gemessen.

Eine Testverbindung wird auf der gleichen Mikrotiterplatte in 10 verschiedenen Konzentrationen von 30 µM bis 1 nM in Doppelbestimmung getestet. Die Daten werden normalisiert (Enzymreaktion ohne Inhibitor = 0 % Inhibition, alle Assaykomponenten ohne Enzym = 100 % Inhibition) und IC₅₀-Werte mit einer hauseigenen Software kalkuliert. Verbindungen im Sinne der Erfindung, die in diesem Assay getestet wurden, hemmten die Chymaseaktivität mit einem IC₅₀-Wert kleiner 10 µM.

Für die erfindungsgemäßen Verbindungen repräsentative IC₅₀-Werte sind in den folgenden Tabellen 1 und 2 wiedergegeben:

**Tabelle 1:**

| **Beispiel Nr.** | **Hamster Chymase IC 50 [nM]** | **Beispiel Nr.** | **Hamster Chymase IC 50 [nM]** | **Beispiel Nr.** | **Hamster Chymase IC 50 [nM]** |
|---|---|---|---|---|---|
| 1 | 8 | 117 | 3 | 191 | 16 |
| 2 | 7 | 118 | 6 | 192 | 5 |
| 3 | 9 | 119 | 280 | 193 | 8 |
| 4 | 64 | 120 | 1025 | 194 | 13 |
| 5 | 20 | 121 | 3 | 195 | 4 |
| 8 | 33 | 122 | 2 | 196 | 6 |
| 9 | 1500 | 123 | 4 | 197 | 10 |
| 10 | 1600 | 124 | 7 | 198 | 54 |
| 13 | 5 | 125 | 6 | 199 | 8 |
| 14 | 10 | 126 | 10 | 200 | 4 |
| 15 | 330 | 127 | 34 | 201 | 7 |
| 16 | 14 | 128 | 7 | 202 | 4 |
| 18 | 10 | 129 | 450 | 203 | 20 |
| 20 | 8 | 130 | 350 | 204 | 39 |
| 21 | 5 | 131 | 4 | 205 | 3 |
| 22 | 6 | 132 | 2 | 206 | 3 |
| 25 | 7 | 133 | 465 | 207 | 4 |
| 27 | 5 | 134 | 2 | 209 | 13 |
| 28 | 4 | 135 | 4 | 211 | 20 |
| 33 | 4 | 136 | 2 | 213 | 18 |
| 34 | 7 | 137 | 4 | 214 | 20 |
| 35 | 6 | 138 | 4 | 215 | 26 |
| 37 | 700 | 139 | 2 | 216 | 183 |
| 40 | 15 | 140 | 1 | 217 | 1 |
| 41 | 23 | 141 | 2 | 218 | 4 |
| 42 | 7 | 142 | 1 | 219 | 5 |
| 43 | 643 | 143 | 2 | 220 | 6 |
| 44 | 18 | 144 | 2 | 221 | 10 |
| 45 | 50 | 145 | 2 | 222 | 12 |
| 47 | 35 | 146 | 1 | 223 | 3 |
| 48 | 17 | 147 | 2 | 224 | 2 |
| 49 | 17 | 148 | 4 | 225 | 4 |
| 50 | 31 | 149 | 2 | 226 | 3 |
| 51 | 120 | 150 | 5 | 227 | 2 |
| 52 | 16 | 151 | 2 | 228 | 14 |
| 53 | 30 | 152 | 19 | 229 | 4 |
| 55 | 39 | 153 | 4 | 230 | 170 |
| 56 | 67 | 154 | 4 | 231 | 21 |
| 62 | 44 | 155 | 5 | 232 | 6 |
| 63 | 37 | 156 | 12 | 233 | 470 |
| 64 | 19 | 157 | 6 | 234 | 270 |
| 65 | 19 | 158 | 10 | 235 | 9 |
| 66 | 30 | 159 | 92 | 236 | 5 |
| 67 | 4 | 160 | 32 | 238 | 45 |
| 75 | 82 | 161 | 53 | 239 | 490 |
| 76 | 41 | 162 | 58 | 240 | 67 |
| 77 | 170 | 163 | 28 | 241 | 2 |
| 78 | 140 | 164 | 34 | 242 | 40 |
| 79 | 210 | 165 | 40 | 243 | 6 |
| 81 | 65 | 166 | 62 | 244 | 2 |
| 82 | | 167 | 91 | 245 | 67 |
| 83 | 220 | 168 | 49 | 246 | 1 |
| 86 | 140 | 169 | 370 | 247 | 1 |
| 89 | 84 | 170 | 20 | 248 | 2 |
| 94 | 62 | 171 | 17 | 249 | 200 |
| 95 | 100 | 172 | 27 | 250 | 37 |
| 96 | 80 | 173 | 110 | 251 | 420 |
| 97 | 33 | 174 | 44 | 252 | 190 |
| 99 | 64 | 175 | 8 | 253 | 1500 |
| 101 | 24 | 176 | 29 | 254 | 84 |
| 103 | 27 | 177 | 30 | 255 | 500 |
| 104 | 2 | 178 | 16 | 256 | 170 |
| 105 | 64 | 179 | 10 | 257 | 540 |
| 106 | 56 | 180 | 7 | 258 | 190 |
| 107 | 29 | 181 | 4 | 259 | 430 |
| 108 | 76 | 182 | 4 | 260 | 130 |
| 109 | 24 | 183 | 10 | 261 | 110 |
| 110 | 150 | 184 | 170 | 262 | 2100 |
| 111 | 20 | 185 | 140 | 263 | 38 |
| 112 | | 186 | 23 | 264 | 31 |
| 113 | 6 | 187 | 4 | 265 | 2 |
| 114 | 7 | 188 | 4 | 266 | 59 |
| 115 | 10 | 189 | 3 | 267 | 16 |
| 116 | 20 | 190 | 140 | 268 | 18 |

**Tabelle 2:**

| **Beispiel Nr.** | **Hamster Chymase IC 50 [nM]** | **Beispiel Nr.** | **Hamster Chymase IC 50 [nM]** | **Beispiel Nr.** | **Hamster Chymase IC 50 [nM]** |
|---|---|---|---|---|---|
| 269 | 14 | 281 | 1 | 293 | 12 |
| 270 | 6 | 282 | 88 | 294 | 2 |
| 271 | 23 | 283 | 40 | 295 | 8 |
| 272 | 11 | 284 | 11 | 296 | 3 |
| 273 | 1100 | 285 | 42 | 297 | |
| 274 | 2 | 286 | 37 | 298 | 6 |
| 275 | 2300 | 287 | 4500 | 299 | 120 |
| 276 | 4 | 288 | 14 | 300 | 2 |
| 277 | 2 | 289 | 970 | 301 | 33 |
| 278 | 5 | 290 | 8 | 302 | 19 |
| 279 | 4 | 291 | 4 | 303 | 9 |
| 280 | 250 | 292 | | | |

### B-2. Kontraktionsmessung an isolierten Aortenringen vom Hamster

Männliche Syrische Hamster (120-150 g) wurden mit Kohlendioxid euthanisiert. Die Aorta wurde präpariert und in eiskalten Krebs-Henseleit-Puffer gelegt. (Zusammensetzung in mmol/l: Natriumchlorid 112, Kaliumchlorid 5.9, Calciumchlorid 2.0, Magnesiumchlorid 1.2, Natriumdihydrogenphosphat 1.2, Natriumhydrogencarbonat 25, Glucose 11.5). Die Aorta wurde in 2 mm lange Ringe geschnitten, in ein Organbad gefüllt mit 5 mL Krebs-Henseleit Puffer übertragen und an einen Myographen (DMT, Dänemark) angeschlossen. Der Puffer wurde auf 37°C gewärmt und mit 95 % Sauerstoff, 5 % Kohlendioxid begast. Um die isometrische Muskelkontraktion zu messen, wurden die Aortenringe zwischen zwei Haken montiert. Einer der Haken war mit einem Druckaufnehmer verbunden. Der zweite Haken war beweglich und erlaubte eine präzise Einstellung der Vorlast nach einem von Mulvany und Halpern beschriebenen Protokoll (Circulation Research 1977; 41:19-26).

Vor jedem Experiment wurde die Ansprechbarkeit des Präparates durch Zugabe von kaliumhaltiger Krebs-Henseleit-Lösung (50 mmol/l KCl) überprüft. Mit einem künstlichen Peptid Angiotensin 1-18 wurde eine Kontraktion der Aortenringe induziert. Das Angiotensin 1-18 wird unabhängig von ACE zu Angiotensin II umgewandelt. Anschließend wurden die Aortenringe mit der Testsubstanz 20 Min inkubiert und die Kontraktionsmessung wiederholt. Die Chymase- Inhibition wird als Reduktion der von Angiotensin 1-18 induzierten Kontraktion dargestellt.

### B-3. Isoprenalin-induziertes Herzfibrosemodell im Hamster

Für die Versuche wurden männliche Syrische Hamster mit einem Körpergewicht von 130-160 g verwendet. Herzhypertrophie und Herzfibrose wurden durch eine tägliche subkutane Injektion von 20 mg/kg Isoprenalin über 7 Tage induziert. Die Testsubstanz wurde den Tieren oral 2 Stunden vor der Injektion des Isoprenalins appliziert. Kontrollgruppen wurden entsprechend mit Lösungsmitteln subkutan und oral behandelt. Am Ende des Versuches wurden die Herzen entnommen, gewogen und fixiert. Das fibrotische Gewebe wurde auf den histologischen Schnitten aus den Herzen mit Hilfe der Siriusrot-Färbung markiert. Anschließend wurde die fibrotische Fläche planimetrisch bestimmt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.
Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5 %-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96 %), 400 mg Rhodigel^{®} (Xanthan gum der Firma FMC, Pennsylvania, USA) und 99 g Wasser.
Einer Einzeldosis von 100 mg der erfindungsgemäßen Verbindung entsprechen 10 mL orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Natriumchlorid-Lösung, Glucoselösung 5 % und/oder PEG 400-Lösung 30 %) gelöst. Die Lösung wird steril filtriert und in sterile und pyrogenfreie Injektionsbehältnisse abgefüllt.

## Patentansprüche

1. Verbindung der Formel (I) 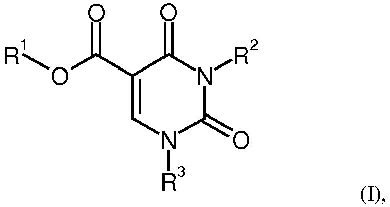 in welcher
R¹ für Wasserstoff oder (C₁-C₄)-Alkyl steht,
R² für eine Gruppe der Formel 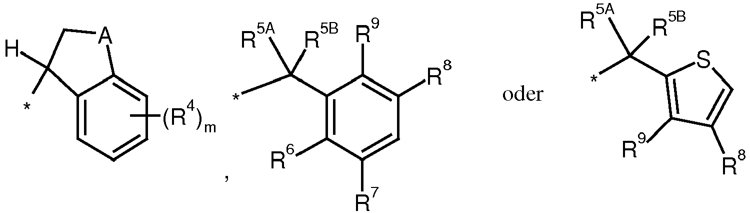 steht, wobei
* für die Anknüpfungsstelle an das Uracil-Stickstoffatom steht,
A für -CH₂-, -CH₂-CH₂-, -O-CH₂-## oder Sauerstoff steht,
worin
## für die Anknüpfstelle an den Phenyl-Ring steht,
m für eine Zahl 0, 1 oder 2 steht,
R⁴ für Halogen, Difluormethyl, Trifluormethyl, (C₁-C₄)-Alkyl, Difluormethoxy, Trifluormethoxy oder (C₁-C₄)-Alkoxy steht,
R^{5A} für Wasserstoff oder Deuterium steht,
R^{5B} für Wasserstoff, Deuterium oder (C₁-C₄)-Alkyl steht,
R⁶ für Wasserstoff oder Fluor steht,
R⁷ für Wasserstoff oder Fluor steht,
R⁸ für Halogen, Difluormethyl, Trifluormethyl, (C₁-C₄)-Alkyl oder Nitro steht,
R⁹ für Wasserstoff, Halogen, Difluormethyl, Trifluormethyl, (C₁-C₄)-Alkyl, Nitro oder (C₁-C₄)-Alkylthio steht,
R³ für eine Gruppe der Formel 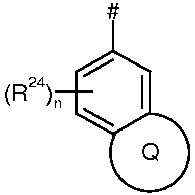 steht, wobei
# für die Anknüpfungsstelle an das Uracil-Stickstoffatom steht,
der Ring Q für 5- bis 7-gliedriges Heterocyclyl oder 5- oder 6-gliedriges Heteroaryl steht,
worin 5- bis 7-gliedriges Heterocyclyl und 5- oder 6-gliedriges Heteroaryl mit 1 bis 4 Substituenten unabhängig voneinander ausgewählt aus der Gruppe Halogen, Difluormethyl, Trifluormethyl, Trideuteromethyl, (C₁-C₆)-Alkyl, (C₃-C₇)-Cycloalkyl, Oxo, Hydroxy, (C₁-C₄)-Alkylcarbonyl, (C₁-C₄)-Alkoxycarbonyl, Aminocarbonyl und (C₁-C₄)-Alkylsulfonyl substituiert sein können,
worin (C₁-C₆)-Alkyl und (C₃-C₇)-Cycloalkyl ihrerseits mit 1 bis 3 Substituenten unabhängig voneinander ausgewählt aus der Gruppe Halogen, Cyano, Trifluormethyl, (C₃-C₇)-Cycloalkyl, Hydroxy, (C₁-C₄)-Alkoxy und 4- bis 7-gliedriges Heterocyclyl substituiert sein können,
und
worin zwei an ein Kohlenstoffatom von 5- bis 7-gliedriges Heterocyclyl und 5- oder 6-gliedriges Heteroaryl gebundene (C₁-C₆)-Alkyl-Reste zusammen mit dem Kohlenstoffatom, an das sie gebunden sind, einen 3- bis 6-gliedrigen Carbocyclus bilden können,
R²⁴ für Halogen, (C₁-C₄)-Alkyl oder (C₁-C₄)-Alkoxy steht,
n für eine Zahl 0, 1, 2 oder 3 steht
sowie ihre Salze, Solvate und Solvate der Salze.

2. Verbindung der Formel (I) nach Anspruch 1, in welcher
R¹ für Wasserstoff, Methyl oder Ethyl steht,
R² für eine Gruppe der Formel 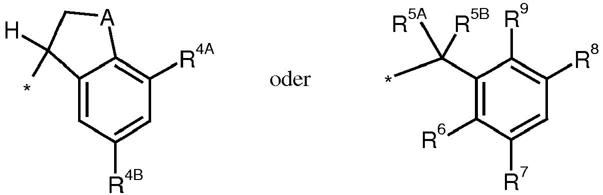 steht, wobei
* für die Anknüpfungsstelle an das Uracil-Stickstoffatom steht,
A für -CH₂-, -CH₂-CH₂-, -O-CH₂-## oder Sauerstoff steht,
worin
## für die Anknüpfstelle an den Phenyl-Ring steht,
R^{4A} für Wasserstoff, Fluor, Chlor, Trifluormethyl oder Methyl steht,
R^{4B} für Wasserstoff, Fluor, Chlor, Trifluormethyl oder Methyl steht, mit der Massgabe, dass mindestens einer der Reste R^{4A} und R^{4B} von Wasserstoff verschieden ist,
R^{5A} für Wasserstoff steht,
R^{5B} für Wasserstoff steht,
R⁶ für Wasserstoff steht,
R⁷ für Wasserstoff steht,
R⁸ für Fluor, Chlor, Difluormethyl, Trifluormethyl oder Methyl steht,
R⁹ für Fluor, Chlor, Difluormethyl, Trifluormethyl oder Methyl steht,
R³ für eine Gruppe der Formel 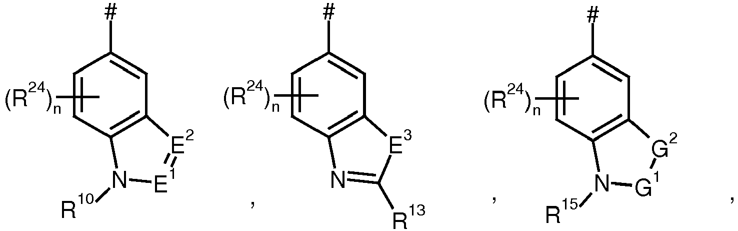 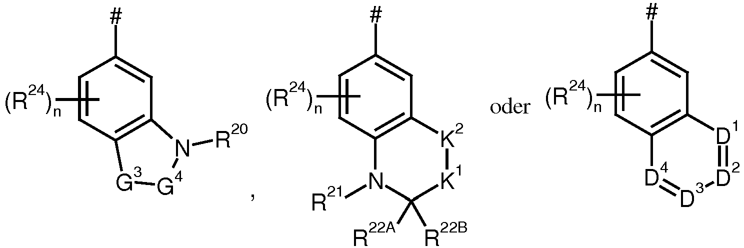 steht, wobei
# für die Anknüpfungsstelle an das Uracil-Stickstoffatom steht,
E¹ für CR¹¹ oder N steht,
worin
R¹¹ für Wasserstoff, (C₁-C₄)-Alkyl, (C₃-C₇)-Cycloalkyl oder Aminocarbonyl steht,
E² für CR¹² oder N steht,
worin
R¹² für Wasserstoff, (C₁-C₄)-Alkyl oder (C₃-C₇)-Cycloalkyl steht,
E³ für NR¹⁴ oder S steht,
worin
R¹⁴ für Wasserstoff, (C₁-C₄)-Alkyl oder (C₃-C₇)-Cycloalkyl steht,
G¹ für C=O oder SO₂ steht,
G² für CR^{16A}R^{16B}, NR¹⁷, O oder S steht,
worin
R^{16A} für Wasserstoff, Fluor, (C₁-C₄)-Alkyl oder Hydroxy steht,
R^{16B} für Wasserstoff, Fluor, Chlor, (C₁-C₄)-Alkyl oder Trifluormethyl steht,
oder
R^{16A} und R^{16B} bilden zusammen mit dem Kohlenstoffatom, an das sie gebunden sind, einen 3- bis 6-gliedrigen Carbocyclus,
R¹⁷ für Wasserstoff, (C₁-C₆)-Alkyl, (C₃-C₇)-Cycloalkyl oder (C₁-C₄)-Alkoxycarbonyl steht,
worin (C₁-C₆)-Alkyl mit 1 bis 3 Subsituenten unabhängig voneinander ausgewählt aus der Gruppe Fluor, Trifluormethyl, Cyano, (C₃-C₇)-Cycloalkyl, Hydroxy, Trifluormethoxy, (C₁-C₄)-Alkoxy, Azetidinyl, Oxetanyl, Tetrahydrofuranyl und Pyrrolidinyl substituiert sein kann,
G³ für CR^{18A}R^{18B}, NR¹⁹, O oder S steht,
worin
R^{18A} für Wasserstoff, Fluor, (C₁-C₄)-Alkyl oder Hydroxy steht,
R^{18B} für Wasserstoff, Fluor, Chlor, (C₁-C₄)-Alkyl oder Trifluormethyl steht,
oder
R^{18A} und R^{18B} zusammen mit dem Kohlenstoffatom, an das sie gebunden sind, einen 3- bis 6-gliedrigen Carbocyclus bilden,
R¹⁹ für Wasserstoff, (C₁-C₆)-Alkyl, (C₃-C₇)-Cycloalkyl oder (C₁-C₄)-Alkoxycarbonyl steht,
worin (C₁-C₆)-Alkyl mit 1 bis 3 Subsituenten unabhängig voneinander ausgewählt aus der Gruppe Fluor, Trifluormethyl, Cyano, (C₃-C₇)-Cycloalkyl, Hydroxy, Trifluormethoxy, (C₁-C₄)-Alkoxy, Azetidinyl, Oxetanyl, Tetrahydrofuranyl und Pyrrolidinyl substituiert sein kann,
G⁴ für CH₂, C=O oder SO₂ steht,
K¹ für CH₂ oder O steht,
K² für CH₂ oder O steht,
mit der Massgabe, dass nur eine der Gruppe K¹ und K² für O steht,
D¹, D², D³ und D⁴ unanhängig voneinander für CR²³ oder N stehen,
worin
R²³ für Wasserstoff, Halogen, (C₁-C₆)-Alkyl oder (C₃-C₇)-Cycloalkyl steht,
mit der Massgabe, dass maximal 2 der Gruppen D¹, D², D³ und D⁴ für N stehen,
R²⁴ für Fluor oder Methyl steht,
n für eine Zahl 0 oder 1 steht,
R¹⁰ für (C₁-C₄)-Alkyl oder (C₃-C₇)-Cycloalkyl steht,
worin (C₁-C₄)-Alkyl mit 1 oder 2 Substituenten unabhängig voneinander ausgewählt aus der Gruppe Fluor, Trifluormethyl, Cyclopropyl, Cyclobutyl, Hydroxy, Methoxy, Ethoxy, Azetidinyl, Oxetanyl, Tetrahydrofuranyl und Pyrrolidinyl substituiert sein kann,
R¹³ für Wasserstoff, (C₁-C₄)-Alkyl oder (C₃-C₇)-Cycloalkyl steht,
R¹⁵ für Wasserstoff, (C₁-C₆)-Alkyl oder (C₃-C₇)-Cycloalkyl steht,
worin (C₁-C₆)-Alkyl mit 1 oder 2 Substituenten unabhängig voneinander ausgewählt aus der Gruppe Fluor, Trifluormethyl, Cyclopropyl, Cyclobutyl, Hydroxy, Methoxy, Ethoxy, Azetidinyl, Oxetanyl, Tetrahydrofuranyl und Pyrrolidinyl substituiert sein kann,
R²⁰ für Wasserstoff, (C₁-C₆)-Alkyl, (C₃-C₇)-Cycloalkyl oder (C₁-C₄)-Alkylcarbonyl steht, worin (C₁-C₆)-Alkyl mit 1 oder 2 Substituenten unabhängig voneinander ausgewählt aus der Gruppe Fluor, Trifluormethyl, Cyclopropyl, Cyclobutyl, Hydroxy, Methoxy, Ethoxy, Azetidinyl, Oxetanyl, Tetrahydrofuranyl und Pyrrolidinyl substituiert sein kann,
R²¹ für Wasserstoff, (C₁-C₆)-Alkyl, (C₃-C₇)-Cycloalkyl oder (C₁-C₄)-Alkylsulfonyl steht,
R^{22A} für Wasserstoff oder (C₁-C₄)-Alkyl steht,
R^{22B} für Wasserstoff oder (C₁-C₄)-Alkyl steht,
oder
R^{22A} und R^{22B} bilden zusammen mit dem Kohlenstoffatom, an das sie gebunden sind, eine Carbonylgruppe,
sowie ihre Salze, Solvate und Solvate der Salze.

3. Verbindung der Formel (I) nach Anspruch 1 oder 2, in welcher
R¹ für Wasserstoff steht,
R² für eine Gruppe der Formel 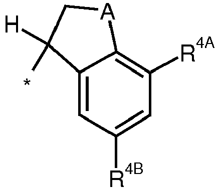 steht, wobei
* für die Anknüpfungsstelle an das Uracil-Stickstoffatom steht,
A für -CH₂- steht,
R^{4A} für Chlor oder Trifluormethyl steht,
R^{4B} für Wasserstoff steht,
R³ für eine Gruppe der Formel 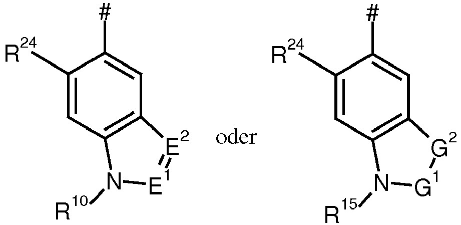 steht, wobei
# für die Anknüpfungsstelle an das Uracil-Stickstoffatom steht,
E¹ für CR¹¹ steht,
worin
R¹¹ für Wasserstoff steht,
E² für N steht,
G¹ für C=O steht,
G² für CR^{16A}R^{16B}, NR¹⁷, O oder S steht,
worin
R^{16A} für Wasserstoff, Fluor, Methyl oder Hydroxy steht,
R^{16B} für Wasserstoff, Fluor, Methyl oder Trifluormethyl steht,
oder
R^{16A} und R^{16B} zusammen mit dem Kohlenstoffatom, an das sie gebunden sind, einen Cyclopropyl-Ring bilden,
R¹⁷ für Wasserstoff, (C₁-C₄)-Alkyl oder (C₃-C₅)-Cycloalkyl steht,
worin (C₁-C₄)-Alkyl mit 1 bis 3 Substituenten unabhängig voneinander ausgewählt aus der Gruppe Fluor, Trifluormethyl, Cyano, Cyclopropyl, Cyclobutyl, Hydroxy, Trifluormethoxy, Methoxy, Ethoxy, Azetidinyl, Oxetanyl, Tetrahydrofuranyl und Pyrrolidinyl substituiert sein kann,
R²⁴ für Wasserstoff oder Fluor steht,
R¹⁰ für (C₁C₄)-Alkyl steht,
R¹⁵ für Wasserstoff, Methyl oder Ethyl steht, worin Methyl und Ethyl mit 1 Substituenten ausgewählt aus der Gruppe Fluor, Trifluormethyl und Cyclopropyl substituiert sein können,
sowie ihre Salze, Solvate und Solvate der Salze.

4. Verbindung der Formel (I) nach Anspruch 1 oder 2, in welcher
R¹ für Wasserstoff steht,
R² für eine Gruppe der Formel 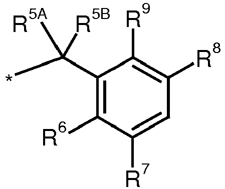 steht, wobei
* für die Anknüpfungsstelle an das Uracil-Stickstoffatom steht,
R^{5A} für Wasserstoff steht,
R^{5B} für Wasserstoff steht,
R⁶ für Wasserstoff steht,
R⁷ für Wasserstoff steht,
R⁸ für Fluor, Chlor oder Trifluormethyl steht,
R⁹ für Fluor, Chlor, Trifluormethyl oder Methyl steht,
R³ für eine Gruppe der Formel 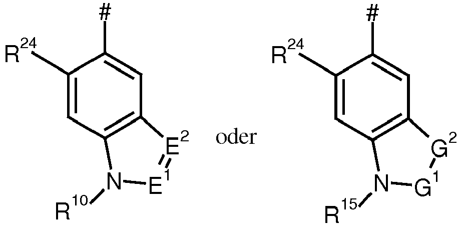 steht, wobei
# für die Anknüpfungsstelle an das Uracil-Stickstoffatom steht,
E¹ für CR¹¹ steht,
worin
R¹¹ für Wasserstoff steht,
E² für N steht,
G¹ für C=O steht,
G² für CR^{16A}R^{16B}, NR¹⁷, O oder S steht,
worin
R^{16A} für Wasserstoff, Fluor, Methyl oder Hydroxy steht,
R^{16B} für Wasserstoff, Fluor, Methyl oder Trifluormethyl steht,
oder
R^{16A} und R^{16B} zusammen mit dem Kohlenstoffatom, an das sie gebunden sind, einen Cyclopropyl-Ring bilden,
R¹⁷ für Wasserstoff, (C₁-C₄)-Alkyl oder (C₃-C₅)-Cycloalkyl steht,
worin (C₁-C₄)-Alkyl mit 1 bis 3 Substituenten unabhängig voneinander ausgewählt aus der Gruppe Fluor, Trifluormethyl, Cyano, Cyclopropyl, Cyclobutyl, Hydroxy, Trifluormethoxy, Methoxy, Ethoxy, Azetidinyl, Oxetanyl, Tetrahydrofuranyl und Pyrrolidinyl substituiert sein kann,
R²⁴ für Wasserstoff oder Fluor steht,
R¹⁰ für (C₁-C₄)-Alkyl steht,
R¹⁵ für Wasserstoff, Methyl oder Ethyl steht,
worin Methyl und Ethyl mit 1 Substituenten ausgewählt aus der Gruppe Fluor, Trifluormethyl und Cyclopropyl substituiert sein können,
sowie ihre Salze, Solvate und Solvate der Salze.

5. Verbindung der Formel (I) gemäß Anspruch 1 mit dem systematischen Namen 1-(1,3-Dimethyl-2-oxo-2,3-dihydro-1H-benzimidazol-5-yl)-2,4-dioxo-3-[(1R)-4-(trifluormethyl)-2,3-dihydro-1H-inden-1-yl]-1,2,3,4-tetrahydropyrimidin-5-carbonsäure (R-Enantiomer) und der Strukturformel

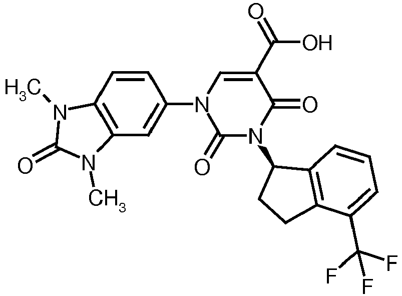

6. Verbindung der Formel (I) gemäß Anspruch 1 mit dem systematischen Namen 1-(6-Fluor-1,3-dimethyl-2-oxo-2,3-dihydro-1H-benzimidazol-5-yl)-2,4-dioxo-3-[(1R)-4-(trifluormethyl)-2,3-dihydro-1H-inden-1-yl]-1,2,3,4-tetrahydropyrimidin-5-carbonsäure (R-Enantiomer) und der Strukturformel

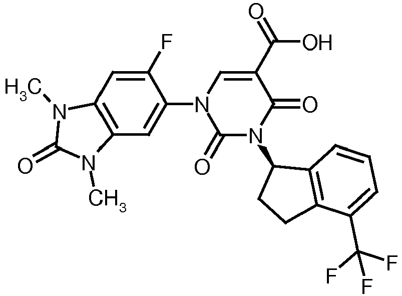

7. Verbindung der Formel (I) gemäß Anspruch 1 mit dem systematischen Namen 1-(3-Methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carbonsäure (R-Enantiomer) und der Strukturformel

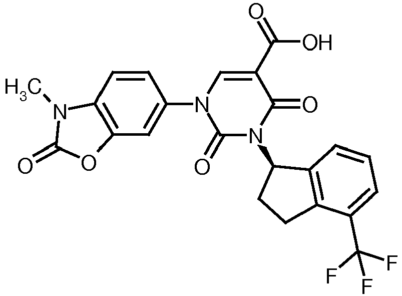

8. Verbindung der Formel (I) gemäß Anspruch 1 mit dem systematischen Namen 2,4-Dioxo-3-[(1R)-4-(trifluormethyl)-2,3-dihydro-1H-inden-1-yl]-1-(1,3,3-trimethyl-2-oxo-2,3-dihydro-1H-indol-5-yl)-1,2,3,4-tetrahydropyrimidin-5-carbonsäure (R-Enantiomer) und der Strukturformel

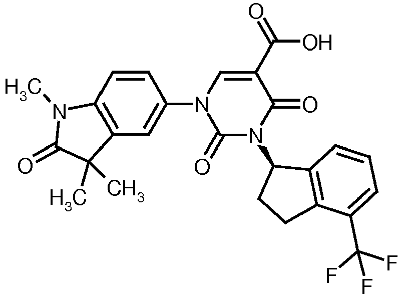

9. Verbindung der Formel (I) gemäß Anspruch 1 mit dem systematischen Namen 1-(1'-Methyl-2'-oxo-1',2'-dihydrospiro[cyclopropan-1,3'-indol]-5'-yl)-2,4-dioxo-3-[(1R)-4-(trifluormethyl)-2,3-dihydro-1H-inden-1-yl]-1,2,3,4-tetrahydropyrimidin-5-carbonsäure (R-Enantiomer) und der Strukturformel

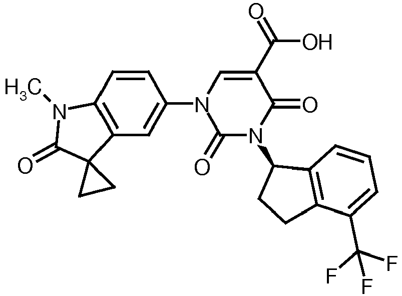

10. Verbindung der Formel (I) gemäß Anspruch 1 mit dem systematischen Namen 1-(3-Methyl-2-oxo-2,3-dihydro-1,3-benzothiazol-6-yl)-2,4-dioxo-3-[(1R)-4-(trifluormethyl)-2,3-dihydro-1H-inden-1-yl]-1,2,3,4-tetrahydropyrimidin-5-carbonsäure (R-Enantiomer) und der Strukturformel

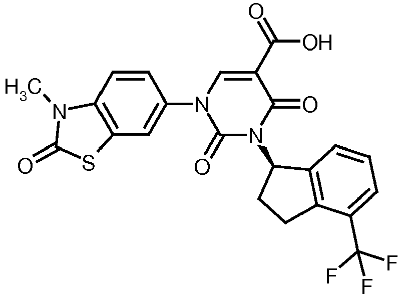

11. Verbindung der Formel (I) gemäß Anspruch 1 mit dem systematischen Namen Ethyl-1-(3-methyl-2-oxo-2,3-dihydro-1,3-benzoxazol-6-yl)-2,4-dioxo-3-[(1R)-4-(trifluormethyl)-2,3-dihydro-1H-inden-1-yl]-1,2,3,4-tetrahydropyrimidin-5-carboxylat (R-Enantiomer) und der Strukturformel

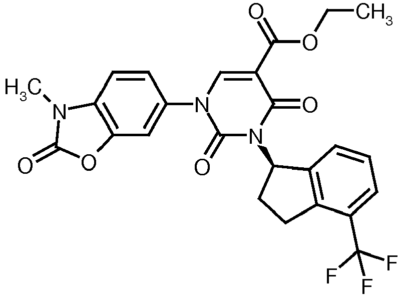

12. Verfahren zur Herstellung von Verbindungen der Formel (I), in welcher
[A] eine Verbindung der Formel (II) 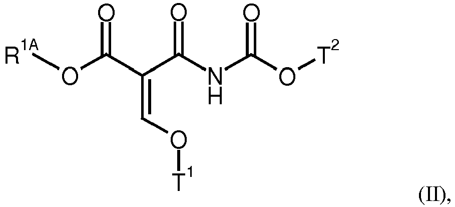 in welcher
R^{1A} für (C₁-C₄)-Alkyl steht,
T¹ für (C₁-C₄)-Alkyl steht,
T² für (C₁-C₄)-Alkyl steht,
in einem inerten Lösungsmittel gegebenenfalls in Gegenwart einer geeigneten Base mit einer Verbindung der Formel (III)
H₂N-R³ (III),
in welcher R³ die in den Ansprüchen 1 bis 4 angegebene Bedeutung hat,
zu einer Verbindung der Formel (IV) 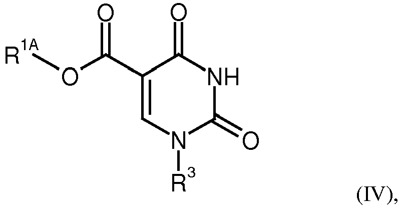 in welcher R^{1A} und R³ jeweils die zuvor angegebenen Bedeutungen haben, umsetzt, diese anschließend in einem inerten Lösungsmittel in Gegenwart einer geeigneten Base mit einer Verbindung der Formel (V)
X¹-R² (V),
in welcher R² die in den Ansprüchen 1 bis 4 angegebene Bedeutung hat, und
X¹ für Hydroxy oder eine geeignete Abgangsgruppe, insbesondere Chlor, Brom oder Iod, steht
zu einer Verbindung der Formel (I-1) 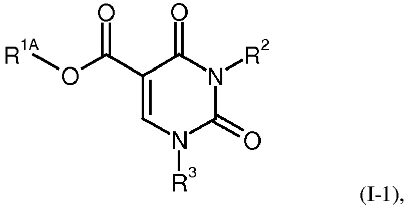 in welcher R^{1A}, R² und R³ jeAnsprücheweils die zuvor angegebenen Bedeutungen haben, umsetzt
oder
[B] eine Verbindung der Formel (VI) 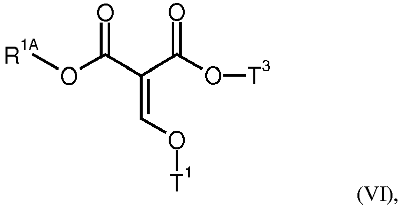 in welcher R^{1A} und T¹ jeweils die zuvor genannten Bedeutungen haben, und
T³ für (C₁-C₄)-Alkyl steht,
in einem inerten Lösungsmittel oder auch ohne Lösungsmittel mit einer Verbindung der Formel (III) in eine Verbindung der Formel (VII) 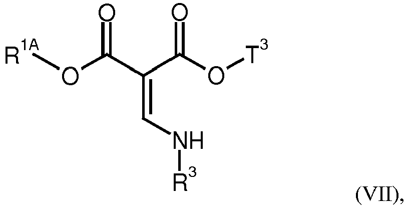 in welcher R^{1A}, R³ und T³ jeweils die zuvor angegebenen Bedeutungen haben, überführt, diese im Folgenden in einem inerten Lösungsmittel mit Chlorsulfonylisocyanat zu einer Verbindung der Formel (IV) umsetzt und diese anschließend analog zu Verfahren [A] in eine Verbindung der Formel (I-1) überführt,
oder
[C] eine Verbindung der Formel (VIII) 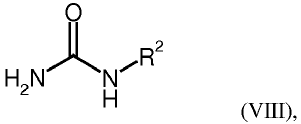 in welcher R² die in den Ansprüchen 1 bis 4 genannte Bedeutung hat, in einem inerten Lösungsmittel mit einer Verbindung der Formel (IX) 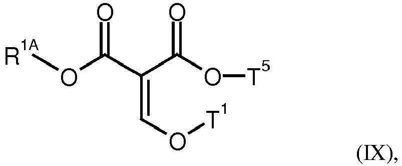 in welcher R^{1A} und T¹ jeweils die zuvor angegebenen Bedeutungen haben, und
T⁵ für (C₁-C₄)-Alkyl steht,
umsetzt und in Gegenwart einer geeigneten Base zu einer Verbindung der Formel (X) 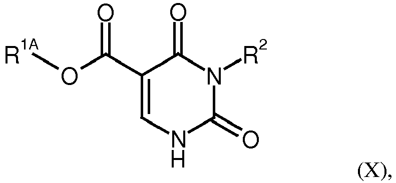 in welcher R^{1A} und R² jeweils die zuvor genannten Bedeutungen haben, cyclisiert, und diese anschließend in einem inerten Lösungsmittel in Gegenwart eines geeigneten Katalysators und einer geeigneten Base mit einer Verbindung der Formel (XI) 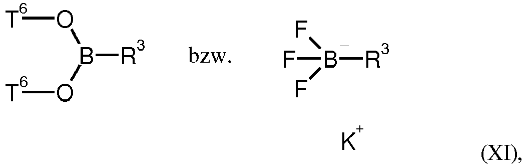 in welcher R³ die oben angegebene Bedeutung hat, und
T⁶ für Wasserstoff, (C₁-C₄)-Alkyl steht oder beide Reste T⁶ zusammen eine -C(CH₃)₂-C(CH₃)₂--Brücke bilden,
zu einer Verbindung der Formel (I-1) umsetzt,
oder
[D] eine Verbindung der Formel (I-1) in einem inerten Lösungsmittel in Gegenwart einer geeigneten Säure oder Base zu einer Verbindung der Formel (I-2) 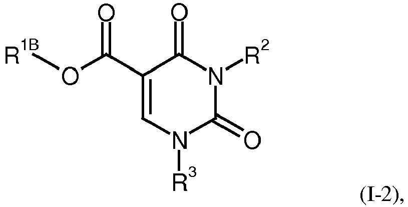 in welcher R² und R³ jeweils die in den Anspruchen 1 bis 4 genannten Bedeutungen haben, und
R^{1B} für Wasserstoff steht,
verseift, gegebenenfalls vorhandene Schutzgruppen abspaltet und/ oder die Verbindungen der Formeln (I-1) und (I-2) gegebenenfalls mit den entsprechenden (i) Lösungsmitteln und/oder (*ii*) Basen oder Säuren in ihre Solvate, Salze und/oder Solvate der Salze überführt.

13. Verbindung, wie in einem der Ansprüche 1 bis 11 definiert, zur Verwendung bei der Behandlung und/oder Prophylaxe von Krankheiten.

14. Verbindung, wie in einem der Ansprüche 1 bis 11 definiert, zur Verwendung in einem Verfahren zur Behandlung und/oder Prophylaxe von Herzinsuffizienz, pulmonaler Hypertonie, chronisch-obstruktiver Lungenerkrankung, Asthma, Niereninsuffizienz, Nephropathien, fibrotischen Erkrankungen der inneren Organe und dermatologischen Fibrosen.

15. Arzneimittel enthaltend eine Verbindung, wie in einem der Ansprüche 1 bis 11 definiert, in Kombination mit einem oder mehreren inerten, nicht-toxischen, pharmazeutisch geeigneten Hilfsstoffen.

16. Arzneimittel enthaltend eine Verbindung, wie in einem der Ansprüche 1 bis 11 definiert, in Kombination mit einem oder mehreren weiteren Wirkstoffen ausgewählt aus der Gruppe bestehend aus Calcium-Antagonisten, Angiotensin AII-Antagonisten, ACE-Inhibitoren, Vasopeptidase-Inhibitoren, Endothelin-Antagonisten, Renin-Inhibitoren, alpha-Rezeptoren-Blocker, beta-Rezeptoren-Blocker, Mineralocorti-coid-Rezeptor-Antagonisten, Rho-Kinase-Inhibitoren, Diuretika, Kinase-Inhibitoren, Matrixmetalloprotease-Inhibitoren, Stimulatoren und Aktivatoren der löslichen Guanylatcyclase und Phosphodiesterase-Inhibitoren.

17. Arzneimittel nach Anspruch 15 oder 16 zur Verwendung bei der Behandlung und/oder Prophylaxe von Herzinsuffizienz,
pulmonaler Hypertonie, chronisch-obstruktiver Lungenerkrankung, Asthma, Niereninsuffizienz, Nephropathien, fibrotischen Erkrankungen der inneren Organe und dermatologischen Fibrosen.

## Claims

1. Compound of the formula (I) 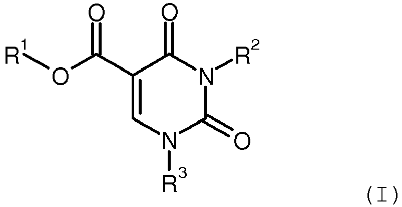 in which
R¹ is hydrogen or (C₁-C₄)-alkyl,
R² is a group of the formula 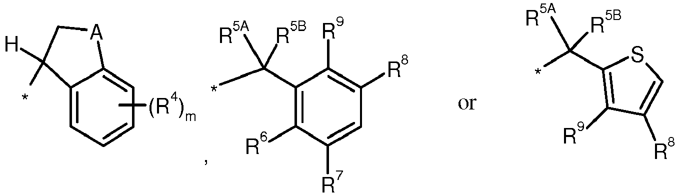 where
* is the attachment site to the uracil nitrogen atom,
A is -CH₂-, -CH₂-CH₂-, -O-CH₂-## or oxygen, in which
## is the attachment site to the phenyl ring,
m is a number 0, 1 or 2,
R⁴ is halogen, difluoromethyl, trifluoromethyl, (C₁-C₄)-alkyl, difluoromethoxy, trifluoromethoxy or (C₁-C₄)-alkoxy,
R^{5A} is hydrogen or deuterium,
R^{5B} is hydrogen, deuterium or (C₁-C₄)-alkyl,
R⁶ is hydrogen or fluorine,
R⁷ is hydrogen or fluorine,
R⁸ is halogen, difluoromethyl, trifluoromethyl, (C₁-C₄)-alkyl or nitro,
R⁹ is hydrogen, halogen, difluoromethyl, trifluoromethyl, (C₁-C₄)-alkyl, nitro or (C₁-C₄)-alkylthio,
R³ is a group of the formula 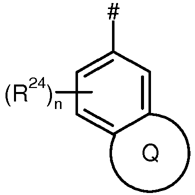 where
# is the attachment site to the uracil nitrogen atom,
the ring Q is 5- to 7-membered heterocyclyl or 5- or 6-membered heteroaryl,
in which 5- to 7-membered heterocyclyl and 5- or 6-membered heteroaryl may be substituted by 1 to 4 substituents independently selected from the group of halogen, difluoromethyl, trifluoromethyl, trideuteromethyl, (C₁-C₆)-alkyl, (C₃-C₇)-cycloalkyl, oxo, hydroxyl, (C₁-C₄)-alkylcarbonyl, (C₁-C₄)-alkoxycarbonyl, aminocarbonyl and (C₁-C₄)-alkylsulphonyl,
in which (C₁-C₆)-alkyl and (C₃-C₇)-cycloalkyl may in turn be substituted by 1 to 3 substituents independently selected from the group of halogen, cyano, trifluoromethyl, (C₃-C₇)-cycloalkyl, hydroxyl, (C₁-C₄)-alkoxy and 4- to 7-membered heterocyclyl,
and
in which two (C₁-C₆)-alkyl radicals bonded to a carbon atom of 5- to 7-membered heterocyclyl and 5- or 6-membered heteroaryl, together with the carbon atom to which they are bonded, may form a 3- to 6-membered carbocycle,
R²⁴ is halogen, (C₁-C₄)-alkyl or (C₁-C₄)-alkoxy,
n is a number 0, 1, 2 or 3,
and the salts, solvates and solvates of the salts thereof.

2. Compound of the formula (I) according to Claim 1 in which
R¹ is hydrogen, methyl or ethyl,
R² is a group of the formula 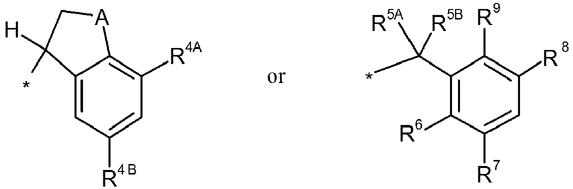 where
* is the attachment site to the uracil nitrogen atom,
A is -CH₂-, -CH₂-CH₂-, -O-CH₂-## or oxygen, in which
## is the attachment site to the phenyl ring,
R^{4A} is hydrogen, fluorine, chlorine, trifluoromethyl or methyl,
R^{4B} is hydrogen, fluorine, chlorine, trifluoromethyl or methyl,
with the proviso that at least one of the R^{4A} and
R^{4B} radicals is not hydrogen,
R^{5A} is hydrogen,
R^{5B} is hydrogen,
R⁶ is hydrogen,
R⁷ is hydrogen,
R⁸ is fluorine, chlorine, difluoromethyl, trifluoromethyl or methyl,
R⁹ is fluorine, chlorine, difluoromethyl, trifluoromethyl or methyl,
R³ is a group of the formula 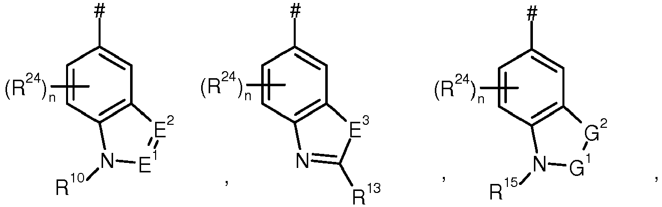 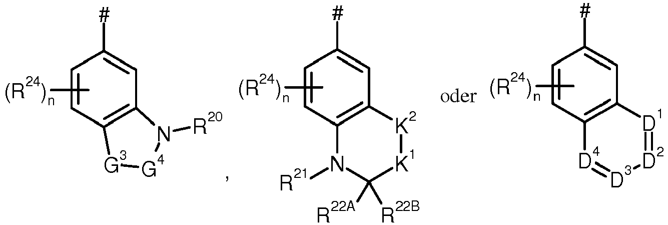 where
# is the attachment site to the uracil nitrogen atom,
E¹ is CR¹¹ or N, in which
R¹¹ is hydrogen, (C₁-C₄)-alkyl, (C₃-C₇)-cycloalkyl or aminocarbonyl,
E² is CR¹² or N, in which
R¹² is hydrogen, (C₁-C₄)-alkyl or (C₃-C₇)-cycloalkyl,
E³ is NR¹⁴ or S, in which
R¹⁴ is hydrogen, (C₁-C₄)-alkyl or (C₃-C₇)-cycloalkyl,
G¹ is C=O or SO₂,
G² is CR^{16A}R^{16B}, NR¹⁷, O or S, in which
R^{16A} is hydrogen, fluorine, (C₁-C₄)-alkyl or hydroxyl,
R^{16B} is hydrogen, fluorine, chlorine, (C₁-C₄)-alkyl or trifluoromethyl,
or
R^{16A} and R^{16B} together with the carbon atom to which they are bonded form a 3- to 6-membered carbocycle,
R¹⁷ is hydrogen, (C₁-C₆)-alkyl, (C₃-C₇)-cycloalkyl or (C₁-C₄)-alkoxycarbonyl, in which (C₁-C₆)-alkyl may be substituted by 1 to 3 substituents independently selected from the group of fluorine, trifluoromethyl, cyano, (C₃-C₇)-cycloalkyl, hydroxyl, trifluoromethoxy, (C₁-C₄)-alkoxy, azetidinyl, oxetanyl, tetrahydrofuranyl and pyrrolidinyl,
G³ is CR^{18A}R^{18B}, NR¹⁹, O or S, in which
R^{18A} is hydrogen, fluorine, (C₁-C₄)-alkyl or hydroxyl,
R^{18B} is hydrogen, fluorine, chlorine, (C₁-C₄)-alkyl or trifluoromethyl,
or
R^{18A} and R^{18B} together with the carbon atom to which they are bonded form a 3- to 6-membered carbocycle,
R¹⁹ is hydrogen, (C₁-C₆)-alkyl, (C₃-C₇)-cycloalkyl or (C₁-C₄)-alkoxycarbonyl, in which (C₁-C₆)-alkyl may be substituted by 1 to 3 substituents independently selected from the group of fluorine, trifluoromethyl, cyano, (C₃-C₇)-cycloalkyl, hydroxyl, trifluoromethoxy, (C₁-C₄)-alkoxy, azetidinyl, oxetanyl, tetrahydrofuranyl and pyrrolidinyl,
G⁴ is CH₂, C=O or SO₂,
Kⁱ is CH₂ or O,
K² is CH₂ or O,
with the proviso that only one of the K¹ and K² groups is O,
D¹, D², D³ and D⁴ are each independently CR²³ or N,
in which
R²³ is hydrogen, halogen, (C₁-C₆)-alkyl or (C₃-C₇)-cycloalkyl,
with the proviso that not more than 2 of the D¹, D², D³ and D⁴ groups are N,
R²⁴ is fluorine or methyl,
n is a number 0 or 1,
R¹⁰ is (C₁-C₄)-alkyl or (C₃-C₇)-cycloalkyl,
in which (C₁-C₄)-alkyl may be substituted by 1 or 2 substituents independently selected from the group of fluorine, trifluoromethyl, cyclopropyl, cyclobutyl, hydroxyl, methoxy, ethoxy, azetidinyl, oxetanyl, tetrahydrofuranyl and pyrrolidinyl,
R¹³ is hydrogen, (C₁-C₄)-alkyl or (C₃-C₇)-cycloalkyl,
R¹⁵ is hydrogen, (C₁-C₆)-alkyl or (C₃-C₇)-cycloalkyl,
in which (C₁-C₆)-alkyl may be substituted by 1 or 2 substituents independently selected from the group of fluorine, trifluoromethyl, cyclopropyl, cyclobutyl, hydroxyl, methoxy, ethoxy, azetidinyl, oxetanyl, tetrahydrofuranyl and pyrrolidinyl,
R²⁰ is hydrogen, (C₁-C₆)-alkyl, (C₃-C₇)-cycloalkyl or (C₁-C₄)-alkylcarbonyl,
in which (C₁-C₆)-alkyl may be substituted by 1 or 2 substituents independently selected from the group of fluorine, trifluoromethyl, cyclopropyl, cyclobutyl, hydroxyl, methoxy, ethoxy, azetidinyl, oxetanyl, tetrahydrofuranyl and pyrrolidinyl,
R²¹ is hydrogen, (C₁-C₆)-alkyl, (C₃-C₇)-cycloalkyl or (C₁-C₄)-alkylsulphonyl,
R^{22A} is hydrogen or (C₁-C₄)-alkyl,
R^{22B} is hydrogen or (C₁-C₄)-alkyl, or
R^{22A} and R^{22B} together with the carbon atom to which they are bonded form a carbonyl group,
and the salts, solvates and solvates of the salts thereof.

3. Compound of the formula (I) according to Claim 1 or 2 in which
R¹ is hydrogen,
R² is a group of the formula 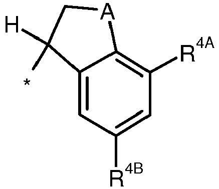 where
* is the attachment site to the uracil nitrogen atom,
A is -CH₂-,
R^{4A} is chlorine or trifluoromethyl,
R^{4B} is hydrogen,
R³ is a group of the formula 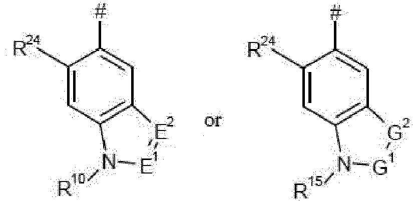 where
# is the attachment site to the uracil nitrogen atom,
E¹ is CR¹¹
in which
R¹¹ is hydrogen,
E² is N,
G¹ is C=O,
G² is CR^{16A}R^{16B}, NR¹⁷, O or S,
in which
R^{16A} is hydrogen, fluorine, methyl or hydroxyl,
R^{16B} is hydrogen, fluorine, methyl or trifluoromethyl,
or
R^{16A} and R^{16B} together with the carbon atom to which they are bonded form a cyclopropyl ring,
R¹⁷ is hydrogen, (C₁-C₄)-alkyl or (C₃-C₅) cycloalkyl,
in which (C₁-C₄)-alkyl may be substituted by 1 to 3 substituents independently selected from the group of fluorine, trifluoromethyl, cyano, cyclopropyl, cyclobutyl, hydroxyl, trifluoromethoxy, methoxy, ethoxy, azetidinyl, oxetanyl, tetrahydrofuranyl and pyrrolidinyl,
R²⁴ is hydrogen or fluorine,
R¹⁰ is (C₁-C₄)-alkyl,
R¹⁵ is hydrogen, methyl or ethyl,
in which methyl and ethyl may be substituted by 1 substituent selected from the group of fluorine, trifluoromethyl and cyclopropyl,
and the salts, solvates and solvates of the salts thereof.

4. Compound of the formula (I) according to Claim 1 or 2 in which
R¹ is hydrogen,
R² is a group of the formula 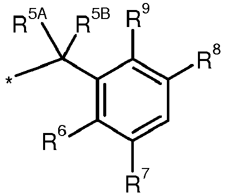 where
* is the attachment site to the uracil nitrogen atom,
R^{5A} is hydrogen,
R^{5B} is hydrogen,
R⁶ is hydrogen,
R⁷ is hydrogen,
R⁸ is fluorine, chlorine or trifluoromethyl,
R⁹ is fluorine, chlorine, trifluoromethyl or methyl,
R³ is a group of the formula 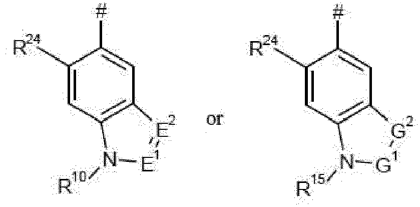 where
# is the attachment site to the uracil nitrogen atom,
E¹ is CR¹¹
in which
R¹¹ is hydrogen,
E² is N,
G¹ is C=O,
G² is CR^{16A}R^{16B}, NR¹⁷, O or S, in which
R^{16A} is hydrogen, fluorine, methyl or hydroxyl,
R^{16B} is hydrogen, fluorine, methyl or trifluoromethyl,
or
R^{16A} and R^{16B} together with the carbon atom to which they are bonded form a cyclopropyl ring,
R¹⁷ is hydrogen, (C₁-C₄)-alkyl or (C₃-C₅)-cycloalkyl,
in which (C₁-C₄)-alkyl may be substituted by 1 to 3 substituents independently selected from the group of fluorine, trifluoromethyl, cyano, cyclopropyl, cyclobutyl, hydroxyl, trifluoromethoxy, methoxy, ethoxy, azetidinyl, oxetanyl, tetrahydrofuranyl and pyrrolidinyl,
R²⁴ is hydrogen or fluorine,
R¹⁰ is (C₁-C₄)-alkyl,
R¹⁵ is hydrogen, methyl or ethyl, in which methyl and ethyl may be substituted by 1 substituent selected from the group of fluorine, trifluoromethyl and cyclopropyl,
and the salts, solvates and solvates of the salts thereof.

5. Compound of the formula (I) according to Claim 1 having the systematic name 1-(1,3-dimethyl-2-oxo-2,3-dihydro-1H-benzimidazol-5-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) and the structural formula

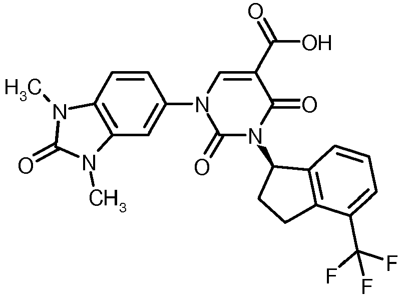

6. Compound of the formula (I) according to Claim 1 having the systematic name 1-(6-fluoro-1,3-dimethyl-2-oxo-2,3-dihydro-1H-benzimidazol-5-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) and the structural formula

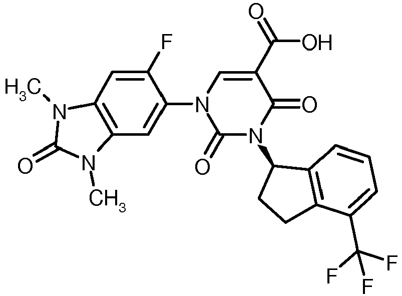

7. Compound of the formula (I) according to Claim 1 having the systematic name 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) and the structural formula

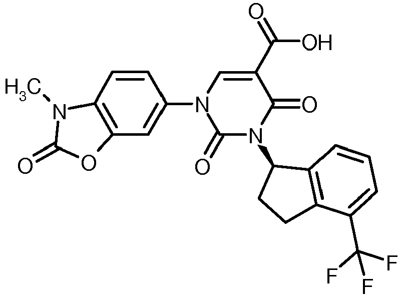

8. Compound of the formula (I) according to Claim 1 having the systematic name 2,4-dioxo-3-[(1R)-4-(trifluoromethyl)-2,3-dihydro-1H-inden-1-yl]-1-(1,3,3-trimethyl-2-oxo-2,3-dihydro-1H-indol-5-yl)-1,2,3,4-tetrahydropyrimidine-5-carboxylic acid (R enantiomer) and the structural formula

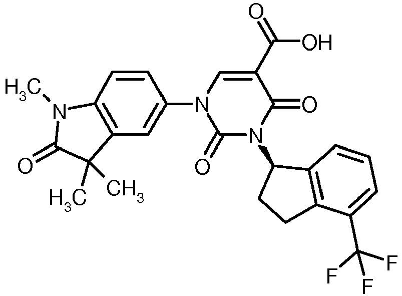

9. Compound of the formula (I) according to Claim 1 having the systematic name 1-(1'-methyl-2'-oxo-1',2'-dihydrospiro[cyclopropane-1,3'-indol]-5'-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) and the structural formula

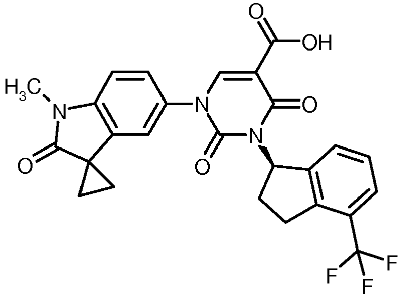

10. Compound of the formula (I) according to Claim 1 having the systematic name 1-(3-methyl-2-oxo-2,3-dihydro-1,3-benzothiazol-6-yl)-2,4-dioxo-3-[(1R)-4-(trifluoromethyl)-2,3-dihydro-1H-inden-1-yl]-1,2,3,4-tetrahydropyrimidine-5-carboxylic acid (R enantiomer) and the structural formula

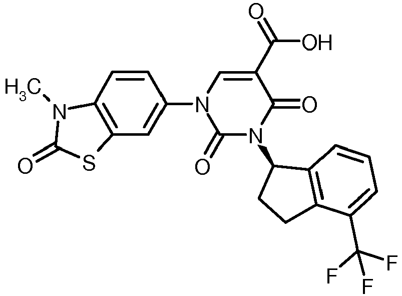

11. Compound of the formula (I) according to Claim 1 having the systematic name ethyl 1-(3-methyl-2-oxo-2,3-dihydro-1,3-benzoxazol-6-yl)-2,4-dioxo-3-[(1R)-4-(trifluoromethyl)-2,3-dihydro-1H-inden-1-yl]-1,2,3,4-tetrahydropyrimidine-5-carboxylate (R enantiomer) and the structural formula

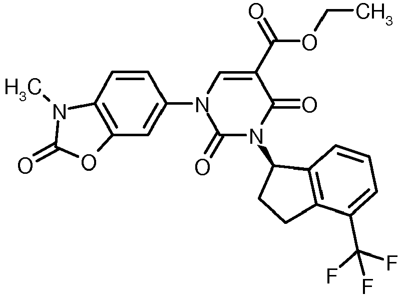

12. Process for preparing compounds of the formula (I), in which
[A] a compound of the formula (II) 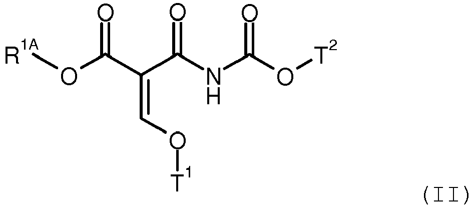 in which
R^{1A} is (C₁-C₄)-alkyl,
T¹ is (C₁-C₄)-alkyl,
T² is (C₁-C₄)-alkyl,
is reacted in an inert solvent, optionally in the presence of a suitable base, with a compound of the formula (III)
H₂N-R³ (III)
in which R³ is as defined in Claims 1 to 4
to give a compound of the formula (IV) 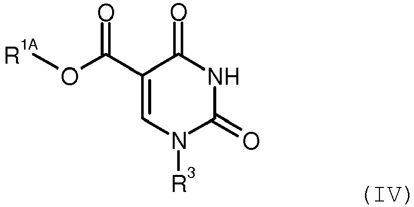 in which R^{1A} and R³ are each as defined above, this is then reacted in an inert solvent, in the presence of a suitable base, with a compound of the formula (V)
X¹-R² (V)
in which R² is as defined in Claims 1 to 4 and
X¹ is hydroxyl or a suitable leaving group, especially chlorine, bromine or iodine
to give a compound of the formula (I-1) 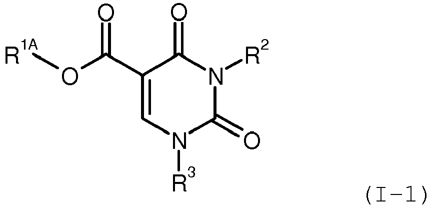 in which R^{1A}, R² and R³ are each claims as defined above,
or
[B] a compound of the formula (VI) 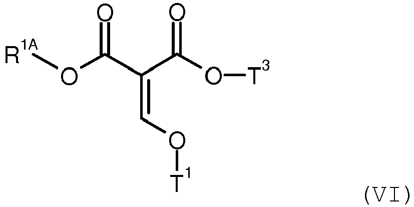 in which R^{1A} and T¹ are each as defined above, and
T³ is (C₁-C₄)-alkyl
is converted in an inert solvent or else without solvent with a compound of the formula (III) to a compound of the formula (VII) 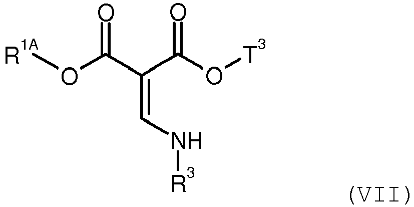 in which R^{1A}, R³ and T³ are each as defined above,
this is subsequently reacted in an inert solvent with chlorosulphonyl isocyanate to give a compound of the formula (IV) and this is subsequently converted analogously to process [A] to a compound of the formula (I-1),
or
[C] a compound of the formula (VIII) 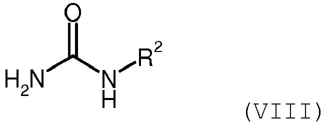 in which R² is as defined in Claims 1 to 4 is reacted in an inert solvent with a compound of the formula (IX) 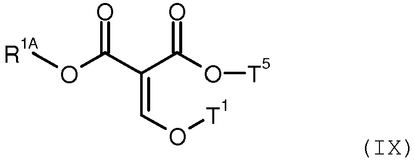 in which R^{1A} and T¹ are each as defined above and T⁵ is (C₁-C₄)-alkyl
and cyclized in the presence of a suitable base to give a compound of the formula (X) 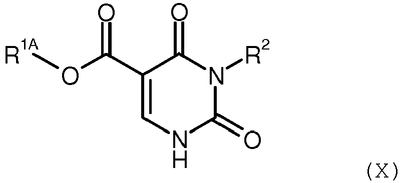 in which R^{1A} and R² are each as defined above, and this is then reacted in an inert solvent, in the presence of a suitable catalyst and a suitable base, with a compound of the formula (XI) 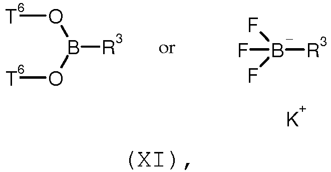 in which R³ is as defined above and
T⁶ is hydrogen, (C₁-C₄)-alkyl, or the two T⁶ radicals together form a -C(CH₃₎₂-C(CH₃)₂-bridge
to give a compound of the formula (I-1),
or
[D] a compound of the formula (I-1) is hydrolysed in an inert solvent in the presence of a suitable acid or base to give a compound of the formula (I-2) 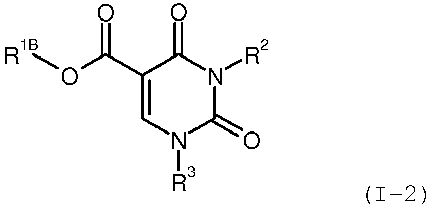
in which R² and R³ are each as defined in Claims 1 to 4 and
R^{1B} is hydrogen,
any protecting groups are detached and/or the compounds of the formulae (I-1) and (I-2) are, where appropriate, converted with the appropriate (i) solvents and/or (ii) bases or acids to the solvates, salts and/or solvates of the salts thereof.

13. Compound as defined in any of Claims 1 to 11 for use in the treatment and/or prophylaxis of diseases.

14. Compound as defined in any of Claims 1 to 11 for use in a method for treatment and/or prophylaxis of heart failure, pulmonary hypertension, chronic obstructive pulmonary disease, asthma, kidney failure, nephropathy, fibrotic disorders of the internal organs and dermatological fibroses.

15. Medicament comprising a compound as defined in any of Claims 1 to 11 in combination with one or more inert nontoxic pharmaceutically suitable excipients.

16. Medicament comprising a compound as defined in any of Claims 1 to 11 in combination with one or more further active ingredients selected from the group consisting of calcium antagonists, angiotensin AII antagonists, ACE inhibitors, vasopeptidase inhibitors, endothelin antagonists, renin inhibitors, alpha-receptor blockers, beta-receptor blockers, mineralocorticoid receptor antagonists, rho-kinase inhibitors, diuretics, kinase inhibitors, matrix metalloprotease inhibitors, stimulators and activators of soluble guanylate cyclase and phosphodiesterase inhibitors.

17. Medicament according to Claim 15 or 16 for use in the treatment and/or prophylaxis of heart failure, pulmonary hypertension, chronic obstructive pulmonary disease, asthma, kidney failure, nephropathy, fibrotic disorders of the internal organs and dermatological fibroses.

## Revendications

1. Composé de formule (I) 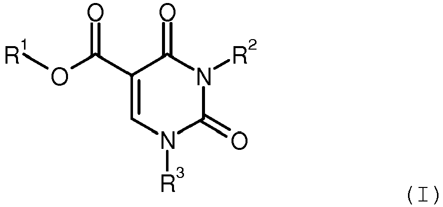 dans lequel
R¹ représente hydrogène ou alkyle en (C₁-C₄),
R² représente un groupe de formule 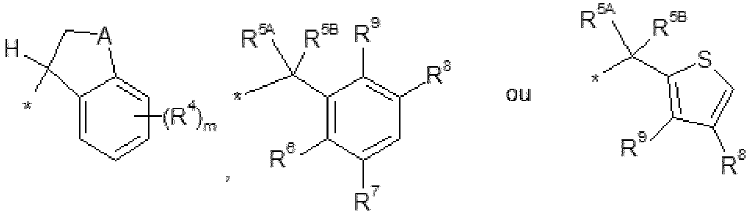
dans lesquelles
* représente l'emplacement de liaison à l'atome d'azote de l'uracile,
A représente -CH₂-, -CH₂-CH₂-, -O-CH₂-## ou oxygène,
## représentant l'emplacement de liaison au cycle phényle,
m représente un nombre 0, 1 ou 2,
R⁴ représente halogène, difluorométhyle, trifluorométhyle, alkyle en (C₁-C₄), difluorométhoxy, trifluorométhoxy ou alcoxy en (C₁-C₄),
R^{5A} représente hydrogène ou deutérium,
R^{5B} représente hydrogène, deutérium ou alkyle en (C₁-C₄),
R⁶ représente hydrogène ou fluor,
R⁷ représente hydrogène ou fluor,
R⁸ représente halogène, difluorométhyle, trifluorométhyle, alkyle en (C₁-C₄) ou nitro,
R⁹ représente hydrogène, halogène, difluorométhyle, trifluorométhyle, alkyle en C₁-C₄, nitro ou alkylthio en C₁-C₄,
R³ représente un groupe de formule 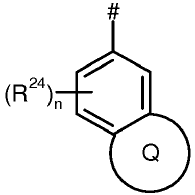 dans laquelle
# représente l'emplacement de liaison à l'atome d'azote de l'uracile,
le cycle Q représente hétérocyclyle de 5 à 7 éléments ou hétéroaryle à 5 ou 6 éléments,
l'hétérocyclyle de 5 à 7 éléments ou l'hétéroaryle à 5 ou 6 éléments pouvant être substitué avec 1 à 4 substituants choisis indépendamment les uns des autres dans le groupe constitué par halogène, difluorométhyle, trifluorométhyle, trideutérométhyle, alkyle en (C₁-C₆), cycloalkyle en (C₃-C₇), oxo, hydroxy, alkylcarbonyle en (C₁-C₄), alcoxycarbonyle en (C₁-C₄), aminocarbonyle et alkylsulfonyle en (C₁-C₄),
l'alkyle en (C₁-C₆) et le cycloalkyle en (C₃-C₇) pouvant eux-mêmes être substitués avec 1 à 3 substituants choisis indépendamment les uns des autres dans le groupe constitué par halogène, cyano, trifluorométhyle, cycloalkyle en (C₃-C₇), hydroxy, alcoxy en (C₁-C₄) et hétérocyclyle de 4 à 7 éléments,
et
deux radicaux alkyle en (C₁-C₆) reliés à un atome de carbone de l'hétérocycle de 5 à 7 éléments et l'hétéroaryle à 5 ou 6 éléments pouvant former ensemble avec l'atome de carbone auquel ils sont reliés un carbocycle de 3 à 6 éléments,
R²⁴ représente halogène, alkyle en C₁-C₄ ou alcoxy en C₁-C₄ ;
n représente un nombre 0, 1, 2 ou 3,
ainsi que leurs sels, solvates et solvates des sels.

2. Composé de formule (I) selon la revendication 1, dans lequel
R¹ représente hydrogène, méthyle ou éthyle,
R² représente un groupe de formule 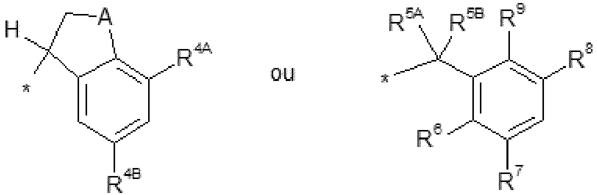 dans lesquelles
* représente l'emplacement de liaison à l'atome d'azote de l'uracile,
A représente -CH₂-, -CH₂-CH₂-, -O-CH₂-## ou oxygène,
## représentant l'emplacement de liaison au cycle phényle,
R^{4A} représente hydrogène, fluor, chlore, trifluorométhyle ou méthyle,
R^{4B} représente hydrogène, fluor, chlore, trifluorométhyle ou méthyle,
à condition qu'au moins un des radicaux R^{4A} et R^{4B} soit différent de l'hydrogène,
R^{5A} représente hydrogène,
R^{5B} représente hydrogène,
R⁶ représente hydrogène,
R⁷ représente hydrogène,
R⁸ représente fluor, chlore, difluorométhyle, trifluorométhyle ou méthyle,
R⁹ représente fluor, chlore, difluorométhyle, trifluorométhyle ou méthyle,
R³ représente un groupe de formule 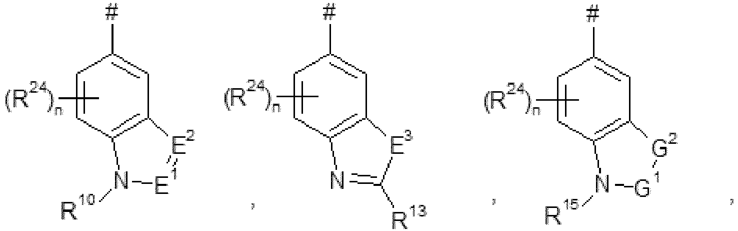 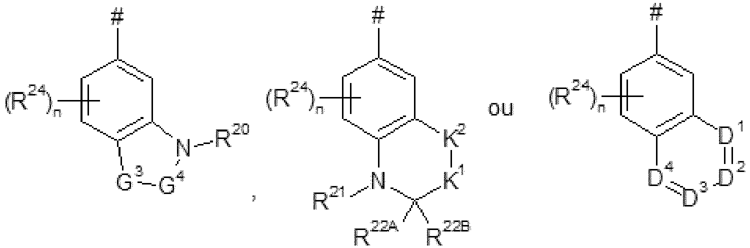 dans lesquelles
# représente l'emplacement de liaison à l'atome d'azote de l'uracile,
E¹ représente CR¹¹ ou N,
R¹¹ représentant hydrogène, alkyle en C₁-C₄, cycloalkyle en C₃-C₇ ou aminocarbonyle,
E² représente CR¹² ou N,
R¹² représentant hydrogène, alkyle en C₁-C₄ ou cycloalkyle en C₃-C₇,
E³ représente NR¹⁴ ou S,
R¹⁴ représentant hydrogène, alkyle en C₁-C₄ ou cycloalkyle en C₃-C₇,
G¹ représente C=O ou SO₂,
G² représente CR^{16A}R^{16B}, NR¹⁷, O ou S,
R^{16A} représentant hydrogène, fluor, alkyle en (C₁-C₄) ou hydroxy,
R^{16B} représentant hydrogène, fluor, chlore, alkyle en (C₁-C₄) ou trifluorométhyle,
ou R^{16A} et R^{16B}
formant ensemble avec l'atome de carbone auquel ils sont reliés un carbocycle de 3 à 6 éléments,
R¹⁷ représentant hydrogène, alkyle en (C₁-C₆), cycloalkyle en (C₃-C₇) ou alcoxycarbonyle en (C₁-C₄),
l'allyle en (C₁-C₆) pouvant être substitué avec 1 à 3 substituants choisis indépendamment les uns des autres dans le groupe constitué par fluor, trifluorométhyle, cyano, cycloalkyle en (C₃-C₇), hydroxy, trifluorométhoxy, alcoxy en (C₁-C₄), azétidinyle, oxétanyle, tétrahydrofuranyle et pyrrolidinyle,
G³ représente CR^{18A}R^{18B}, NR¹⁹, O ou S,
R^{18A} représentant hydrogène, fluor, alkyle en (C₁-C₄) ou hydroxy,
R^{18B} représentant hydrogène, fluor, chlore, alkyle en (C₁-C₄) ou trifluorométhyle,
ou
R^{18A} et R^{18B} formant ensemble avec l'atome de carbone auquel ils sont reliés un carbocycle de 3 à 6 éléments,
R¹⁹ représente hydrogène, alkyle en (C₁-C₆), cycloalkyle en (C₃-C₇) ou alcoxycarbonyle en (C₁-C₄),
l'allyle en (C₁-C₆) pouvant être substitué avec 1 à 3 substituants choisis indépendamment les uns des autres dans le groupe constitué par fluor, trifluorométhyle, cyano, cycloalkyle en (C₃-C₇), hydroxy, trifluorométhoxy, alcoxy en (C₁-C₄), azétidinyle, oxétanyle, tétrahydrofuranyle et pyrrolidinyle,
G⁴ représente CH₂, C=O ou SO₂,
K¹ représente CH₂ ou O,
K² représente CH₂ ou O,
à condition qu'uniquement un des groupes K¹ et K² représente O,
D¹, D², D³ et D⁴ représentent indépendamment les uns des autres CR²³ ou N,
R²³ représentant hydrogène, halogène, alkyle en C₁-C₆ ou cycloalkyle en C₃-C₇,
à condition qu'au plus 2 des groupes D¹, D², D³ et D⁴ représentent N,
R²⁴ représente fluor ou méthyle,
n représente un nombre 0 ou 1,
R¹⁰ représente alkyle en (C₁-C₄) ou cycloalkyle en (C₃-C₇),
l'alkyle en (C₁-C₄) pouvant être substitué avec 1 ou 2 substituants choisis indépendamment l'un de l'autre dans le groupe constitué par fluor, trifluorométhyle, cyclopropyle, cyclobutyle, hydroxy, méthoxy, éthoxy, azétidinyle, oxétanyle, tétrahydrofuranyle et pyrrolidinyle,
R¹³ représente hydrogène, alkyle en C₁-C₄ ou cycloalkyle en C₃-C₇,
R¹⁵ représente hydrogène, alkyle en C₁-C₆ ou cycloalkyle en C₃-C₇,
l'alkyle en (C₁-C₆) pouvant être substitué avec 1 ou 2 substituants choisis indépendamment l'un de l'autre dans le groupe constitué par fluor, trifluorométhyle, cyclopropyle, cyclobutyle, hydroxy, méthoxy, éthoxy, azétidinyle, oxétanyle, tétrahydrofuranyle et pyrrolidinyle,
R²⁰ représente hydrogène, alkyle en (C₁-C₆), cycloalkyle en (C₃-C₇) ou alkylcarbonyle en (C₁-C₄),
l'alkyle en (C₁-C₆) pouvant être substitué avec 1 ou 2 substituants choisis indépendamment l'un de l'autre dans le groupe constitué par fluor, trifluorométhyle, cyclopropyle, cyclobutyle, hydroxy, méthoxy, éthoxy, azétidinyle, oxétanyle, tétrahydrofuranyle et pyrrolidinyle,
R²¹ représente hydrogène, alkyle en (C₁-C₆), cycloalkyle en (C₃-C₇) ou alkylsulfonyle en (C₁-C₄),
R^{22A} représente hydrogène ou alkyle en (C₁-C₄),
R^{22B} représente hydrogène ou alkyle en (C₁-C₄),
ou
R^{22A} et R^{22B} forment ensemble avec l'atome de carbone auquel ils sont reliés un groupe carbonyle,
ainsi que leurs sels, solvates et solvates des sels.

3. Composé de formule (I) selon la revendication 1 ou 2, dans lequel
R¹ représente hydrogène,
R² représente un groupe de formule 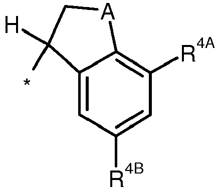 dans laquelle
* représente l'emplacement de liaison à l'atome d'azote de l'uracile,
A représente -CH₂-,
R^{4A} représente chlore ou trifluorométhyle,
R^{4B} représente hydrogène,
R³ représente un groupe de formule 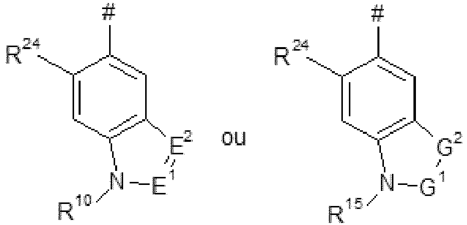 dans lesquelles
# représente l'emplacement de liaison à l'atome d'azote de l'uracile,
E¹ représente CR¹¹,
R¹¹ représentant hydrogène,
E² représente N,
G¹ représente C=O,
G² représente CR^{16A}R^{16B}, NR¹⁷, O ou S,
R^{16A} représentant hydrogène, fluor, méthyle ou hydroxy,
R^{16B} représentant hydrogène, fluor, méthyle ou trifluorométhyle,
ou
R^{16A} et R^{16B} formant ensemble avec l'atome de carbone auquel ils sont reliés un cycle cyclopropyle,
R¹⁷ représentant hydrogène, alkyle en C₁-C₄ ou cycloalkyle en C₃-C₅,
l'alkyle en (C₁-C₄) pouvant être substitué avec 1 à 3 substituants choisis indépendamment les uns des autres dans le groupe constitué par fluor, trifluorométhyle, cyano, cyclopropyle, cyclobutyle, hydroxy, trifluorométhoxy, méthoxy, éthoxy, azétidinyle, oxétanyle, tétrahydrofuranyle et pyrrolidinyle,
R²⁴ représente hydrogène ou fluor,
R¹⁰ représente alkyle en (C₁-C₄),
R¹⁵ représente hydrogène, méthyle ou éthyle, le méthyle et l'éthyle pouvant être substitués avec 1 substituant choisi dans le groupe constitué par fluor, trifluorométhyle et cyclopropyle,
ainsi que leurs sels, solvates et solvates des sels.

4. Composé de formule (I) selon la revendication 1 ou 2, dans lequel
R¹ représente hydrogène,
R² représente un groupe de formule 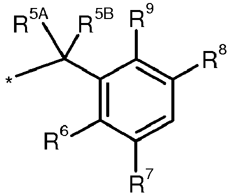 dans laquelle
* représente l'emplacement de liaison à l'atome d'azote de l'uracile,
R^{5A} représente hydrogène,
R^{5B} représente hydrogène,
R⁶ représente hydrogène,
R⁷ représente hydrogène,
R⁸ représente fluor, chlore ou trifluorométhyle,
R⁹ représente fluor, chlore, trifluorométhyle ou méthyle,
R³ représente un groupe de formule 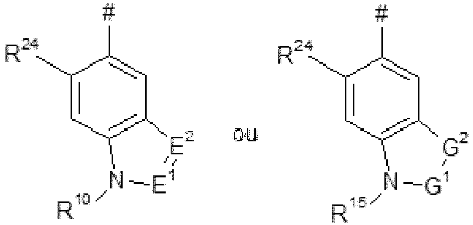 dans lesquelles
# représente l'emplacement de liaison à l'atome d'azote de l'uracile,
E¹ représente CR¹¹,
R¹¹ représentant hydrogène,
E² représente N,
G¹ représente C=O,
G² représente CR^{16A}R^{16B}, NR¹⁷, O ou S,
R^{16A} représentant hydrogène, fluor, méthyle ou hydroxy,
R^{16B} représentant hydrogène, fluor, méthyle ou trifluorométhyle,
ou
R^{16A} et R^{16B} formant ensemble avec l'atome de carbone auquel ils sont reliés un cycle cyclopropyle,
R¹⁷ représentant hydrogène, alkyle en C₁-C₄ ou cycloalkyle en C₃-C₅,
l'alkyle en (C₁-C₄) pouvant être substitué avec 1 à 3 substituants choisis indépendamment les uns des autres dans le groupe constitué par fluor, trifluorométhyle, cyano, cyclopropyle, cyclobutyle, hydroxy, trifluorométhoxy, méthoxy, éthoxy, azétidinyle, oxétanyle, tétrahydrofuranyle et pyrrolidinyle,
R²⁴ représente hydrogène ou fluor,
R¹⁰ représente alkyle en (C₁-C₄),
R¹⁵ représente hydrogène, méthyle ou éthyle,
le méthyle et l'éthyle pouvant être substitués avec 1 substituant choisi dans le groupe constitué par fluor, trifluorométhyle et cyclopropyle,
ainsi que leurs sels, solvates et solvates des sels.

5. Composé de formule (I) selon la revendication 1, ayant le nom systématique acide 1-(1,3-diméthyl-2-oxo-2,3-dihydro-1H-benzimidazol-5-yl)-2,4-dioxo-3-[(1R)-4-(trifluorométhyl)-2,3-dihydro-1H-indén-1-yl]-1,2,3,4-tétrahydropyrimidine-5-carboxylique (énantiomère R) et la formule structurale

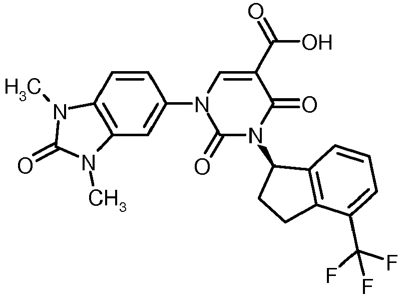

6. Composé de formule (I) selon la revendication 1, ayant le nom systématique acide 1-(6-fluoro-1,3-diméthyl-2-oxo-2,3-dihydro-1H-benzimidazol-5-yl)-2,4-dioxo-3-[(1R)-4-(trifluorométhyl)-2,3-dihydro-1H-indén-1-yl]-1,2,3,4-tétrahydropyrimidine-5-carboxylique (énantiomère R) et la formule structurale

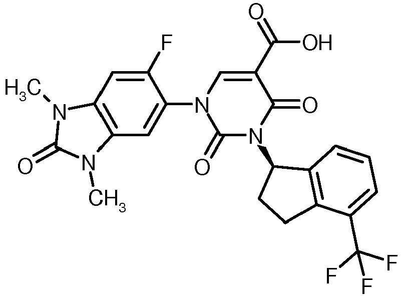

7. Composé de formule (I) selon la revendication 1, ayant le nom systématique acide 1-(3-méthyl-2-oxo-2,3-dihydro-1,3-benzoxazol-6-yl)-2,4-dioxo-3-[(1R)-4-(trifluorométhyl)-2,3-dihydro-1H-indén-1-yl]-1,2,3,4-tétrahydropyrimidine-5-carboxylique (énantiomère R) et la formule structurale

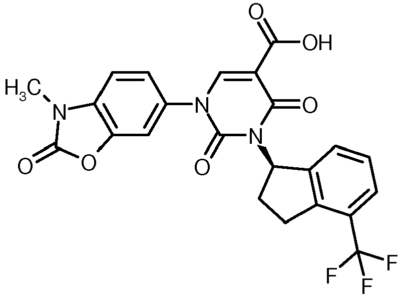

8. Composé de formule (I) selon la revendication 1, ayant le nom systématique acide 2,4-dioxo-3-[(1R)-4-(trifluorométhyl)-2,3-dihydro-1H-indén-1-yl]-1-(1,3,3-triméthyl-2-oxo-2,3-dihydro-1H-indol-5-yl)-1,2,3,4-tétrahydropyrimidine-5-carboxylique (énantiomère R) et la formule structurale

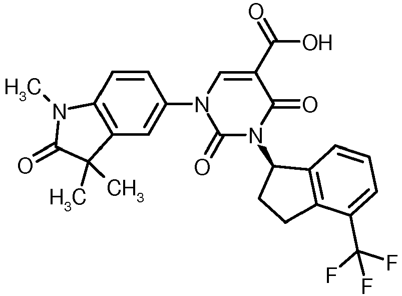

9. Composé de formule (I) selon la revendication 1, ayant le nom systématique acide 1-(1'-méthyl-2'-oxo-1',2'-dihydro-spiro-[cyclo-propane-1,3'-indol]-5'-yl)-2,4-dioxo-3-[(1R)-4-(trifluorométhyl)-2,3-dihydro-1H-indén-1-yl]-1,2,3,4-tétrahydropyrimidine-5-carboxylique (énantiomère R) et la formule structurale

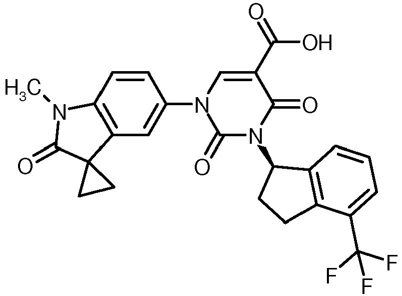

10. Composé de formule (I) selon la revendication 1, ayant le nom systématique acide 1-(3-méthyl-2-oxo-2,3-dihydro-1,3-benzothiazol-6-yl)-2,4-dioxo-3-[(1R)-4-(trifluorométhyl)-2,3-dihydro-1H-indén-1-yl]-1,2,3,4-tétrahydropyrimidine-5-carboxylique (énantiomère R) et la formule structurale

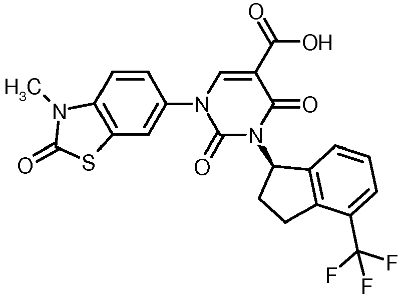

11. Composé de formule (I) selon la revendication 1, ayant le nom systématique 1-(3-méthyl-2-oxo-2,3-dihydro-1,3-benzoxazol-6-yl)-2,4-dioxo-3-[(1R)-4-(trifluorométhyl)-2,3-dihydro-1H-indén-1-yl]-1,2,3,4-tétrahydropyrimidine-5-carboxylate d'éthyle (énantiomère R) et la formule structurale

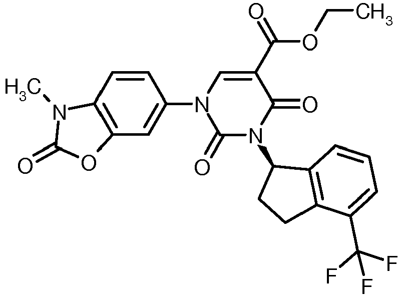

12. Procédé de fabrication de composés de formule (I), selon lequel
[A] un composé de formule (II) 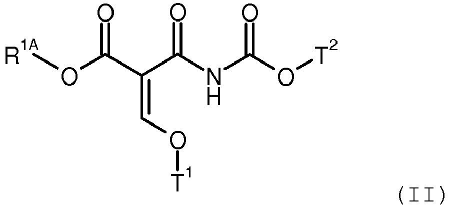 dans laquelle
R^{1A} représente alkyle en (C₁-C₄),
T¹ représente alkyle en (C₁-C₄),
T² représente alkyle en (C₁-C₄),
est mis en réaction dans un solvant inerte, éventuellement en présence d'une base appropriée, avec un composé de formule (III)
H₂N-R³ (III)
dans laquelle R³ a la signification indiquée dans les revendications 1 à 4,
pour former un composé de formule (IV) 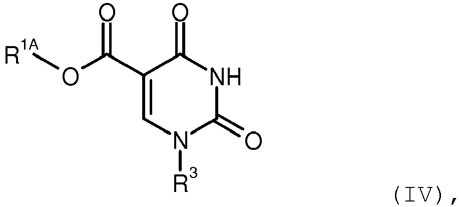 dans laquelle R^{1A} et R³ ont chacun les significations indiquées précédemment,
puis celui-ci est mis en réaction dans un solvant inerte, en présence d'une base appropriée, avec un composé de formule (V)
X¹-R² (V),
dans laquelle R² a la signification indiquée dans les revendications 1 à 4,
X¹ représente hydroxy ou un groupe partant approprié, notamment chlore, brome ou iode,
pour former un composé de formule (I-1) 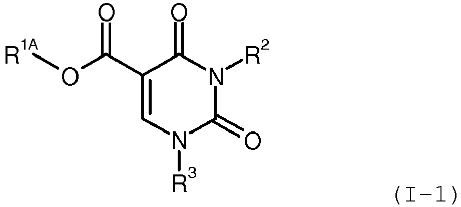 dans laquelle R^{1A}, R² et R³ ont chacun les significations indiquées précédemment,
ou
[B] un composé de formule (VI) 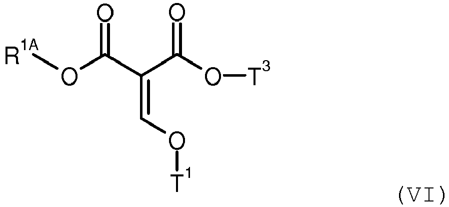
dans laquelle R^{1A} et T¹ ont chacun les significations indiquées précédemment,
et
T³ représente alkyle en (C₁-C₄),
est transformé dans un solvant inerte ou également sans solvant avec un composé de formule (III) en un composé de formule (VII) 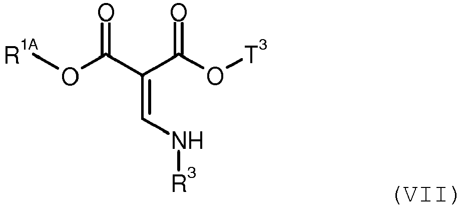 dans laquelle R^{1A}, R³ et T³ ont chacun les significations indiquées précédemment,
puis celui-ci est mis en réaction dans un solvant inerte avec de l'isocyanate de chlorosulfonyle pour former un composé de formule (IV), puis celui-ci est transformé de manière analogue au procédé [A] en un composé de formule (I-1),
ou
[C] un composé de formule (VIII) 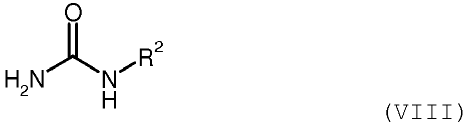 dans laquelle R² a la signification indiquée dans les revendications 1 à 4,
est mis en réaction dans un solvant inerte avec un composé de formule (IX) 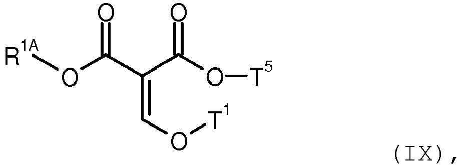
dans laquelle R^{1A} et T¹ ont chacun les significations indiquées précédemment, et
T⁵ représente alkyle en (C₁-C₄),
et cyclisé en présence d'une base appropriée en un composé de formule (X) 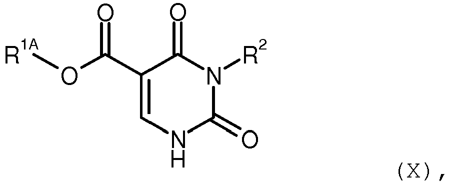 dans laquelle R^{1A} et R² ont chacun les significations indiquées précédemment,
puis celui-ci est mis en réaction dans un solvant inerte en présence d'un catalyseur approprié et d'une base appropriée avec un composé de formule (XI) 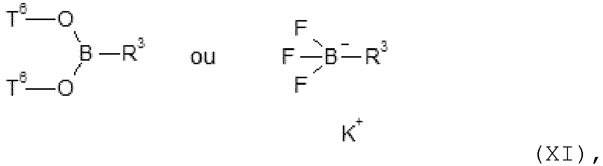
dans laquelle R³ a la signification indiquée précédemment,
et
T⁶ représente hydrogène, alkyle en (C₁-C₄) ou deux radicaux T⁶ forment ensemble un pont -C(CH₃)₂-C(CH₃)₂-, pour former un composé de formule (I-1),
ou
[D] un composé de formule (I-1) est saponifié dans un solvant inerte en présence d'un acide ou d'une base approprié pour former un composé de formule (I-2) 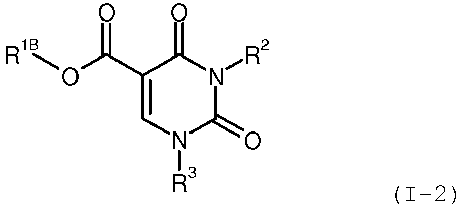
dans laquelle R² et R³ ont les significations indiquées dans les revendications 1 à 4, et
R^{1B} représente hydrogène,
les groupes protecteurs éventuellement présents sont clivés et/ou les composés de formules (I-1) et (I-2) sont éventuellement transformés avec les (i) solvants et/ou (ii) bases ou acides appropriés en leurs solvates, sels et/ou solvates des sels.

13. Composé, tel que défini dans l'une quelconque des revendications 1 à 11, destiné à une utilisation pour le traitement et/ou la prophylaxie de maladies.

14. Composé, tel que défini dans l'une quelconque des revendications 1 à 11, destiné à une utilisation dans un procédé de traitement et/ou de prophylaxie de l'insuffisance cardiaque, l'hypertension pulmonaire, la pneumopathie obstructive chronique, l'asthme, l'insuffisance rénale, les néphropathies, les maladies fibrotiques des organes internes et les fibroses dermatologiques.

15. Médicament contenant un composé tel que défini dans l'une quelconque des revendications 1 à 11 en combinaison avec un ou plusieurs adjuvants inertes, non toxiques, pharmaceutiquement appropriés.

16. Médicament contenant un composé tel que défini dans l'une quelconque des revendications 1 à 11 en combinaison avec un ou plusieurs agents actifs supplémentaires choisis dans le groupe constitué par les antagonistes de calcium, les antagonistes d'angiotensine AII, les inhibiteurs d'ACE, les inhibiteurs de vasopeptidase, les antagonistes d'endothéline, les inhibiteurs de rénine, les bloquants des récepteurs alpha, les bloquants des récepteurs bêta, les antagonistes des récepteurs minéralocorticoïdes, les inhibiteurs de kinase rho, les diurétiques, les inhibiteurs de kinases, les inhibiteurs de métalloprotéase de la matrice, les stimulateurs et les activateurs de guanylate cyclase soluble et les inhibiteurs de phosphodiestérase.

17. Médicament selon la revendication 15 ou 16, destiné à une utilisation pour le traitement et/ou la prophylaxie de l'insuffisance cardiaque, l'hypertension pulmonaire, la pneumopathie obstructive chronique, l'asthme, l'insuffisance rénale, les néphropathies, les maladies fibrotiques des organes internes et les fibroses dermatologiques.
